# EUROPEAN PATENT APPLICATION

(11) **EP 2 145 960 A1**
(43) Date of publication of application: **20.01.2010**
(21) Application number: 09168583.4
(22) Date of filing: 03.08.2006
(51) Int. Cl.: C12N 15/82, C12N 15/31, C12N 5/04, C07K 14/245, A01H 5/00, A01H 5/10

(54) **Nucleic acid sequences encoding proteins associated with abiotic stress response and plant cells and plants with in-creased tolerance to environmental stress**

(30) Priority: 12.08.2005 US 707841 P
(62) Divisional of application: 06792673.3
(71) Applicant: BASF Plant Science GmbH, 67056 Ludwigshafen (DE); Metanomics GmbH, 10589 Berlin (DE)
(72) Inventor: Taman-Chardonnens, Agnes A., 1611, DS Bovenkarspel (NL); Puzio, Piotr, 9030, Mariakerke (Gent) (BE); McKersie, Bryan, Raleigh, NC 27617 (US)
(74) Representative: Krieger, Stephan Gerhard

(57) **Abstract**

This invention relates generally to nucleic acid sequences encoding proteins that are associated with abiotic stress responses and abiotic stress tolerance in plants. This invention further relates to transformed plant cells with altered metabolic activity compared to a corresponding non-transformed, wild-type plant cell, wherein the metabolic activity is altered by transformation with a Stress-Related Protein (SRP) coding nucleic acid and results in increased tolerance and/or resistance to an environmental stress as compared to a corresponding non-transformed, wild-type plant cell.

## Description

The instant application contains a "lengthy" Sequence Listing, which has been submitted via CD-R in lieu of a printed, paper copy, and is hereby incorporated by reference in its entirety. Said CD-Rs, recorded on July 29, 2005, are labeled CRF, "Copy 1," and "Copy 2," respectively, and each contains only one identical 664 Kb file (00015127.APP).

This invention relates generally to nucleic acid sequences encoding proteins that are associated with abiotic stress responses and abiotic stress tolerance in plants.

This invention further relates to transformed plant cells with altered metabolic activity, compared to a corresponding non-transformed wild-type plant cell, wherein the metabolic activity is altered by transformation with a Stress-Related Protein (SRP) coding nucleic acid and results in increased tolerance and/or resistance to an environmental stress, as compared to a corresponding non-transformed wild-type plant cell.

In particular, this invention relates to nucleic acid sequences encoding proteins that confer drought, heat, cold, and/or salt tolerance and/or resistance to plants, especially by altering the metabolic activity leading to drought, heat, cold, and/or salt tolerance and/or resistance to plants. The invention also deals with methods of producing, screening for, and breeding such plant cells or plants and method of detecting stress in plants cells or plants.

Abiotic environmental stresses, such as drought stress, salinity stress, heat stress, and cold stress, are major limiting factors of plant growth and productivity (Boyer. 1982. Science 218, 443-448). Crop losses and crop yield losses of major crops, such as rice, maize (corn), and wheat caused by these stresses represent a significant economic and political factor and contribute to food shortages in many underdeveloped countries.

Plants are typically exposed, during their life cycle, to conditions of reduced environmental water content. Most plants have evolved strategies to protect themselves against these conditions of low water or desiccation (drought). However, if the severity and duration of the drought conditions are too great, the effects on plant development, growth, and yield of most crop plants are profound:
Continuous exposure to drought causes major alterations in the plant metabolism. These great changes in metabolism ultimately lead to cell death and consequently yield losses.

Developing stress-tolerant plants is a strategy that has the potential to solve or mediate at least some of these problems (McKersie and Leshem, 1994, Stress and Stress Coping in Cultivated Plants, Kluwer Academic Publishers). However, traditional plant breeding strategies to develop new lines of plants that exhibit resistance (tolerance) to these types of stresses are relatively slow and require specific resistant lines for crossing with the desired line. Limited germplasm resources for stress tolerance and incompatibility in crosses between distantly related plant species represent significant problems encountered in conventional breeding. Additionally, the cellular processes leading to drought, cold, and salt tolerance are complex in nature and involve multiple mechanisms of cellular adaptation and numerous metabolic pathways (McKersie and Leshem, 1994, Stress and Stress Coping in Cultivated Plants, Kluwer Academic Publishers). This multicomponent nature of stress tolerance has not only made breeding for tolerance largely unsuccessful, but has also limited the ability to genetically engineer stress-tolerance plants using biotechnological methods.

Drought, heat, cold, and salt stresses have a common theme important for plant growth and that is water availability. Plants are exposed during their entire life cycle to conditions of reduced environmental water content. Most plants have evolved strategies to protect themselves against these conditions. However, if the severity and duration of the drought conditions are too great, the effects on plant development, growth, and yield of most crop plants are profound. Since high salt content in some soils result in less available water for cell intake, its effect is similar to those observed under drought conditions. Additionally, under freezing temperatures, plant cells lose water as a result of ice formation that starts in the apoplast and withdraws water from the symplast (McKersie and Leshem, 1994, Stress and Stress Coping in Cultivated Plants, Kluwer Academic Publishers). Commonly, a plant's molecular response mechanisms to each of these stress conditions are similar.

The results of current research indicate that drought tolerance is a complex quantitative trait and that no real diagnostic marker is yet available. High salt concentrations, or dehydration, may cause damage at the cellular level during drought stress, but the precise injury is not entirely clear (Bray, 1997, Trends Plant Sci. 2, 48-54). This lack of a mechanistic understanding makes it difficult to design a transgenic approach to improve drought tolerance. However, an important consequence of damage may be the production of reactive oxygen radicals that cause cellular injury, such as lipid peroxidation or protein and nucleic acid modification. Details of oxygen-free, radical chemistry, and their reaction with cellular components such as cell membranes, have been described (McKersie and Leshem, 1994, Stress and Stress Coping in Cultivated Plants, Kluwer Academic Publishers).

It is the object of this invention to identify new, unique genes capable of conferring stress tolerance to plants upon expression, under expression, or over expression.

It is a further object of this invention to identify, produce, and breed new, unique stress tolerant and/or resistant plant cells or plants and methods of inducing and detecting stress tolerance and/or resistance in plants or plant cells.

It is a further object to identify new methods to detect stress tolerance and/or resistance in plants or plant cells. It is also the object of this invention to identify new, unique genes capable of conferring stress tolerance to plants upon expression or over expression.

The present invention provides a transformed plant cell with altered metabolic activity, compared to a corresponding non-transformed wild-type plant cell, wherein the metabolic activity is altered by transformation with a Stress-Related Protein (SRP) coding nucleic acid and results in increased tolerance and/or resistance to an environmental stress, as compared to a corresponding non-transformed wild-type plant cell.

The present invention provides a transgenic plant cell transformed by Stress-Related Protein (SRP) coding nucleic acid selected from the group consisting of: a) a nucleic acid molecule encoding one of the polypeptides disclosed in the Sequence Listing or a fragment thereof, which confers an altered metabolic activity in an organism or a part thereof; b) a nucleic acid molecule comprising one of the nucleic acid molecule disclosed in the Sequence Listing; c) a nucleic acid molecule whose sequence can be deduced from a polypeptide sequence encoded by a nucleic acid molecule of (a) or (b) as a result of the degeneracy of the genetic code and conferring an altered metabolic activity in an organism or a part thereof; d) a nucleic acid molecule which encodes a polypeptide, which has at least 50% identity with the amino acid sequence of the polypeptide encoded by the nucleic acid molecule of (a) to (c) and conferring an altered metabolic activity in an organism or a part thereof; e) a nucleic acid molecule which hybridizes with a nucleic acid molecule of (a) to (c) under stringent hybridization conditions and conferring an altered metabolic activity in an organism or a part thereof; f) a nucleic acid molecule which is obtained by amplifying nucleic acid molecules from a cDNA library or a genomic library using the primers as shown in table 2 and conferring an altered metabolic activity in an organism or a part thereof; g) a nucleic acid molecule encoding a polypeptide which is isolated with the aid of monoclonal antibodies against a polypeptide encoded by one of the nucleic acid molecules of (a) to (f) and conferring an altered metabolic activity in an organism or a part thereof; and h) a nucleic acid molecule which is obtainable by screening a suitable nucleic acid library under stringent hybridization conditions with a probe comprising one of the sequences of the nucleic acid molecule of (a) to (k) or with a fragment thereof having at least 15 nt, preferably 20 nt, 30 nt, 50 nt, 100 nt, 200 nt or 500 nt of the nucleic acid molecule characterized in (a) to (g) and conferring an altered metabolic activity in an organism or a part thereof.

As used herein, the term "metabolite" refers to intermediate substances, preferably such of low molecular weight, which occur during anabolism and catabolism in a cell or plant, in other words, a substance produced or consumed by metabolism.

The term "altered metabolic activity" refers to the change (increase or decrease) of the amount, concentration, or activity (meaning here the effective concentration for the purposes of chemical reactions and/or other mass action) of a metabolite in a specific volume relative to a corresponding volume (e.g. in an organism, a tissue, a cell or a cell compartment) of a control, reference, or wild type, including the de novo creation of the activity or expression, measured, for example, by one of the methods described herein below, which is changed or altered (increased or decreased) as compared to a corresponding non-transformed wild-type plant cell.

The terms "increased," "raised," "extended," "enhanced," "improved," or "amplified" relate to a corresponding change of a property in an organism, a part of an organism such as a tissue, seed, root, leave, flower, etc. or in a cell and are interchangeable. Preferably, the overall activity in the volume is increased or enhanced in cases if the increase or enhancement is related to the increase or enhancement of an activity of a gene product, independent of whether the amount of gene product or the specific activity of the gene product, or both, is increased or enhanced or whether the amount, stability, or translation efficacy of the nucleic acid sequence or gene encoding for the gene product is increased or enhanced. The terms "reduction," "decrease," or "deletion" relate to a corresponding change of a property in an organism, a part of an organism such as a tissue, seed, root, leave, flower, etc. or in a cell. Preferably, the overall activity in the volume is reduced, decreased, or deleted in cases if the reduction, decrease, or deletion is related to the reduction, decrease, or deletion of an activity of a gene product, independent whether the amount of gene product or the specific activity of the gene product or both is reduced, decreased, or deleted, or whether the amount, stability, or translation efficacy of the nucleic acid sequence or gene encoding for the gene product is reduced, decreased, or deleted.

The terms "increase" or "decrease" relate to a corresponding change of a property in an organism or in a part of an organism, such as a tissue, seed, root, leave, flower, etc. or in a cell. Preferably, the overall activity in the volume is increased in cases the increase relates to the increase of an activity of a gene product, independent of whether the amount of gene product or the specific activity of the gene product, or both, is increased or generated, or whether the amount, stability, or translation efficacy of the nucleic acid sequence or gene encoding for the gene product is increased.

Under "change of a property" it is understood that the activity, expression level, or amount of a gene product or the metabolite content is changed in a specific volume relative to a corresponding volume of a control, reference, or wild type, including the de novo creation of the activity or expression.

The terms "increase" or "decrease" include the change of said property in only parts of the subject of the present invention, for example, the modification can be found in compartment of a cell, like a organelle, or in a part of a plant, like tissue, seed, root, leave, flower, etc., but is not detectable if the overall subject, i.e. complete cell or plant, is tested. Preferably, the increase or decrease is found cellular, thus the term "increase of an activity" or "increase of a metabolite content" relates to the cellular increase compared to the wild-type cell.

Accordingly, the term "increase" or "decrease" means that the specific activity of an enzyme, as well as the amount of a compound or metabolite, e.g. of a polypeptide, a nucleic acid molecule, or of the fine chemical of the invention, or an encoding mRNA or DNA, can be increased or decreased in a volume.

The terms "wild type," "control," or "reference" are exchangeable and can be a cell or a part of organisms such as an organelle or a tissue, or an organism, in particular a microorganism or a plant, which was not modified or treated according to the herein described process according to the invention. Accordingly, the cell or a part of organisms, such as an organelle or a tissue, or an organism, in particular a microorganism or a plant used as wild type, control, or reference corresponds to the cell, organism, or part thereof as much as possible and is in any other property but in the result of the process of the invention as identical to the subject matter of the invention as possible. Thus, the wild type, control, or reference is treated identically or as identical as possible, saying that only conditions or properties might be different, which do not influence the quality of the tested property.

Preferably, any comparison is carried out under analogous conditions. The term "analogous conditions" means that all conditions such as, for example, culture or growing conditions, or assay conditions (such as buffer composition, temperature, substrates, pathogen strain, concentrations and the like) are kept identical between the experiments to be compared.

The "reference," "control," or "wild type" is preferably a subject, e.g., an organelle, a cell, a tissue, an organism, in particular a plant or a microorganism, which was not modified or treated according to the herein-described process of the invention and is in any other property as similar to the subject matter of the invention as possible. The reference, control, or wild type is in its genome, transcriptome, proteome, or metabolome as similar as possible to the subject of the present invention. Preferably, the term "reference-" "control-" or "wild type-"-organelle, -cell, -tissue, or -organism, in particular plant or microorganism, relates to an organelle, cell, tissue, or organism, in particular plant or microorganism, which is nearly genetically identical to the organelle, cell, tissue, or organism, in particular microorganism or plant, of the present invention or a part thereof preferably 95%, more preferred are 98%, even more preferred are 99.00%, in particular 99.10%, 99.30%, 99.50%, 99.70%, 99.90%, 99.99%, 99.999%, or more. Most preferable the "reference," "control," or "wild type" is a subject, e.g. an organelle, a cell, a tissue, an organism, which is genetically identical to the organism, cell, or organelle used according to the process of the invention except that the responsible or activity conferring nucleic acid molecules or the gene product encoded by them are amended, manipulated, exchanged, or introduced, according to the inventive process.

Preferably, the reference, control or wild type differs form the subject of the present invention only in the cellular activity of the polypeptide of the invention, e.g. as result of an increase in the level of the nucleic acid molecule of the present invention or an increase of the specific activity of the polypeptide of the invention, e.g. by or in the expression level or activity of an protein having the activity of an Stress-Related Protein (SRP) or its homologs, its biochemical or genetic causes and the altered metabolic activity.

In case a control, reference, or wild type differing from the subject of the present invention, only by not being subject of the process of the invention, cannot be provided, a control, reference, or wild type can be an organism in which the cause for the modulation of an activity conferring the altered metabolic activity or expression of the nucleic acid molecule of the invention, as described herein, has been switched back or off, e.g. by knocking out the expression of responsible gene product, e.g. by antisense inhibition, by inactivation of an activator or agonist, by activation of an inhibitor or antagonist, by inhibition through adding inhibitory antibodies, by adding active compounds as e.g. hormones, by introducing negative dominant mutants, etc. A gene production can, for example, be knocked out by introducing inactivating point mutations, which lead to an enzymatic activity inhibition or a destabilization or an inhibition of the ability to bind to cofactors etc.

Accordingly, preferred reference subject is the starting subject of the present process of the invention. Preferably, the reference and the subject matter of the invention are compared after standardization and normalization, e.g. to the amount of total RNA, DNA, or protein or activity, or expression of reference genes, like housekeeping genes, such as ubiquitin, actin, or ribosomal proteins.

A series of mechanisms exists via which a modification of the a protein, e.g. the polypeptide of the invention, can directly or indirectly affect the yield, production, and/or production efficiency of the amino acid.

For example, the molecule number or the specific activity of the polypeptide, or the nucleic acid molecule, may be increased. Larger amounts of the fine chemical can be produced if the polypeptide or the nucleic acid of the fine chemical can be produced if the polypeptide or the nucleic acid of the invention is expressed de novo in an organism lacking the activity of said protein. However, it is also possible to increase the expression of the gene, which is naturally present in the organisms, for example, by modifying the regulation of the gene, or by increasing the stability of the corresponding mRNA, or of the corresponding gene product encoded by the nucleic acid molecule of the invention, or by introducing homologous genes from other organisms, which are differently regulated, e.g. not feedback sensitive.

This also applies analogously to the combined, increased expression of the nucleic acid molecule of the present invention or its gene product with that of further enzymes of the amino acid biosynthesis pathways, e.g., which are useful for the synthesis of the fine chemicals.

The increase, decrease, or modulation, according to this invention, can be constitutive, e.g., due to a stable permanent transgenic expression or to a stable mutation in the corresponding endogenous gene encoding the nucleic acid molecule of the invention, or to a modulation of the expression or of the behavior of a gene conferring the expression of the polypeptide of the invention, or transient, e.g. due to an transient transformation or temporary addition of a modulator such as a agonist or antagonist or inducible, e.g. after transformation with a inducible construct carrying the nucleic acid molecule of the invention under control of a inducible promoter and adding the inducer, e.g. tetracycline or as described herein below.

The increase in activity of the polypeptide amounts in a cell, a tissue, a organelle, an organ, or an organism or a part thereof, preferably to at least 5%, preferably to at least 20%, or at to least 50%, especially preferably to at least 70%, 80%, 90%, or more, very especially preferably are to at least 200%, most preferably are to at least 500%, or more in comparison to the control, reference, or wild type.

The specific activity of a polypeptide encoded by a nucleic acid molecule of the present invention, or of the polypeptide of the present invention, can be tested as described in the examples. In particular, the expression of a protein in question in a cell, e.g., a plant cell or a microorganism, and the detection of an increase the fine chemical level in comparison to a control is an easy test and can be performed as described in the state of the art.

The term "increase" includes that a compound or an activity is introduced into a cell de novo or that the compound or the activity has not been detectable before, in other words, it is "generated".

Accordingly, in the following, the term "increasing" also comprises the term "generating" or "stimulating". The increased activity manifests itself in an increase of the fine chemical.

The transformed plant cells are compared to the corresponding non-transformed wild type of the same genus and species under otherwise identical conditions (such as, for example, culture conditions, age of the plants, and the like). In this context, a change in metabolic activity of at least 10%, advantageously of at least 20%, preferably at least 30%, especially preferably of at least 40%, 50%, or 60%, very especially preferably of at least 70%, 80%, 90%, 95%, or even 100%, or more, in comparison with the non-transformed organism is advantageous.

Preferably, the change in metabolite concentration of the transformed plant cells is the changed compared to the corresponding non-transformed wild type. Preferably, the change in metabolite concentration is measured by HPLC and calculated by dividing the peak height or peak area of each analyte (metabolite) through the peak area of the respective internal standards. Data is normalized using the individual sample fresh weight. The resulting values are divided by the mean values found for wild-type plants grown under control conditions and analyzed in the same sequence, resulting in the so-called ratios, which represent values independent of the analytical sequence. These ratios indicate the behavior of the metabolite concentration of the transformed plants in comparison to the concentration in the wild-type control plants.

According to this method, the change in at least one metabolite concentration of the transformed plant cells, compared to the corresponding non-transformed, wild type, is at least 10%, advantageously of at least 20%, preferably at least 40%, 60%, or 80%, especially preferably of at least 90%, 100%, or 200%, very especially preferably of at least 700%, 800%, 900%, 1000% or more.

Data significance can be determined by all statistical methods known by a person skilled in the art, preferably by a t-test, more preferably by the student t-test.

Altered metabolic activity also refers to metabolites that, compared to a corresponding non-transformed, wild-type plant cell, are not produced after transformation or are only produced after transformation.

Preferred metabolites of the invention are 2,3-dimethyl-5-phytylquinol or 2-hydroxy-palmitic acid or 3,4-dihydroxyphenylalanine (= dopa) or 3-hydroxy-palmitic acid or 5-oxoproline or alanine or alpha linolenic acid (c18:3 (c9, c12, c15)) or alpha-tocopherol or aminoadipic acid or anhydroglucose or arginine or aspartic acid or beta-apo-8' carotenal or beta-carotene or beta-sitosterol or beta-tocopherol or (delta-7-cis,10-cis)-hexadecadienic acid or hexadecatrienic acid or margaric acid or delta-15-cis-tetracosenic acid or ferulic acid or campesterol or cerotic acid (c26:0) or citrulline or cryptoxanthine or eicosenoic acid (20: 1) or fructose or fumarate or galactose or gamma-aminobutyric acid or gamma-tocopherol or gluconic acid or glucose or glutamic acid or glutamine or glycerate or glycerinaldehyd or glycerol or glycerol-3-phosphate or glycine or homoserine or inositol or isoleucine or iso-maltose or isopentenyl pyrophosphate or leucine or lignoceric acid (c24:0) or linoleic acid (c18:2 (c9, c12)) or luteine or lycopene or malate or mannose or methionine or methylgalactofuranoside or methylgalactopyranoside or methylgalactopyranoside or palmitic acid (c16:0) or phenylalanine or phosphate or proline or putrescine or pyruvate or raffinose or ribonic acid or serine or shikimate or sinapine acid or stearic acid (c18:0) or succinate or sucrose or threonine or triacontanoic acid or tryptophane or tyrosine or ubichinone or udp-glucose or valine or zeaxanthine.

Metabolic activity may also be altered concerning one or more derivates of one or more of the above metabolites.

Preferably, metabolic activity is altered concerning one or more metabolites selected from the group consisting of all of the above metabolites.

Alternatively, metabolic activity may be altered concerning one or more metabolites selected from the group consisting of mannose, inositol, phosphate, aspartic acid, isoleucine, leucine, gamma-aminobutyric acid, glycerinaldehyd, sucrose, campesterol, valine, beta-tocopherol, ubichinone, palmitic acid (c16:0), 2-hydroxy-palmitic acid, 2,3-dimethyl-5-phytylquinol, beta-carotene, alphalinolenic acid (c18:3 (c9, c12, c15)), lycopene.

Alternatively, metabolic activity may be altered concerning one or more metabolites selected from the group consisting of methylgalactofuranoside, beta-sitosterol, delta-15-cis-tetracosenic acid (c24:1 me), margaric acid (c17:0 me), stearic acid (c18:0), methylgalactopyranoside, gamma-tocopherol, linoleic acid (c18:2 (c9, c12)), hexadecatrienic acid (c16:3 me), shikimate, raffinose, glutamic acid, glutamine, udp-glucose, proline, threonine, isopentenyl pyrophosphate, 5-oxoproline, ferulic acid, sinapine acid.

Alternatively metabolic activity may be altered concerning one or more metabolites selected from the group consisting of tryptophane, citrulline, serine, alanine, glycerate, arginine, 3-hydroxy-palmitic acid, putrescine, 3,4-dihydroxyphenylalanine (=dopa), alpha-tocopherol, aminoadipic acid, anhydroglucose, beta-apo-8' carotenal, delta-7-cis,10-cis-hexadecadienic acid (c16:2 me), cerotic acid (c26:0), cryptoxanthine, eicosenoic acid (20:1), fructose, fumarate.

Alternatively, metabolic activity may be altered concerning one or more metabolites selected from the group consisting of galactose, gluconic acid, glucose, glycerol, glycerol-3-phosphate, glycine, homoserine, iso-maltose, lignoceric acid (c24:0), luteine, malate, triacontanoic acid, methionine, phenylalanine, pyruvate, ribonic acid, succinate, tyrosine, zeaxanthine.

The present invention provides a transgenic plant cell, wherein expression of said nucleic acid sequence in the plant cell results altered metabolic activity leading to increased tolerance and/or resistance to environmental stress, as compared to a corresponding non-transformed, wild-type plant cell. One preferred wild-type plant cell is a non-transformed Arabidopsis plant cell. An example here is the Arabidopsis wild type C24 (Nottingham Arabidopsis Stock Centre, UK; NASC Stock N906).

Other preferred, wild-type plant cells are a non-transformed from plants selected from the group consisting of maize, wheat, rye, oat, triticale, rice, barley, soybean, peanut, cotton, rapeseed, canola, manihot, pepper, sunflower, flax, borage, safflower, linseed, primrose, rapeseed, turnip rape, tagetes, solanaceous plants, potato, tobacco, eggplant, tomato, Vicia species, pea, alfalfa, coffee, cacao, tea, Salix species, oil palm, coconut, perennial grass, and forage crops.

More preferred wild-type plant cells are a non-transformed Linum plant cell, preferably Linum usitatissimum, more preferably the variety Brigitta, Golda, Gold Merchant, Helle, Juliel, Olpina, Livia, Marlin, Maedgold, Sporpion, Serenade, Linus, Taunus, Lifax or Liviola, a non-transformed Heliantus plant cell, preferably Heliantus annuus, more preferably the variety Aurasol, Capella, Flavia, Flores, Jazzy, Palulo, Pegasol, PIR64A54, Rigasol, Sariuca, Sideral, Sunny, Alenka, Candisol or Floyd, or a non-transformed Brassica plant cell, preferably Brassica napus, more preferably the variety Dorothy, Evita, Heros, Hyola, Kimbar, Lambada, Licolly, Liconira, Licosmos, Lisonne, Mistral, Passat, Serator, Siapula, Sponsor, Star, Caviar, Hybridol, Baical, Olga, Lara, Doublol, Karola, Falcon, Spirit, Olymp, Zeus, Libero, Kyola, Licord, Lion, Lirajet, Lisbeth, Magnum, Maja, Mendel, Mica, Mohican, Olpop, Ontarion, Panthar, Prinoe, Pronio, Susanna, Talani, Titan, Transfer, Wiking, Woltan, Zeniah, Artus, Contact or Smart.

The expression of said nucleic acid sequence in the plant cell may directly or indirectly influence the metabolic activity of the transformed plant cells. Preferably, they influence the activity of the above metabolites.

Preferably, metabolic activity may be altered by transformation with one or more Stress-Related Protein (SRP) coding nucleic acid selected from the group comprising the nucleic acid of the Sequence Listingb homologs of the afore mentioned sequences.

It is within the scope of the invention to identify the genes encoded by a nucleic acid sequence selected from the group consisting of the nucleic acid of the Sequence Listing and/or homologs thereof in target plants, especially crop plants, and then express the corresponding gene to achieve the altered metabolic activity resulting in increased tolerance and/or resistance to environmental stress. Consequently, the invention is not limited to a specific plant.

A protein having an activity conferring an altered metabolic activity preferably has the structure of the polypeptide described herein, in particular of the polypeptides comprising a consensus sequence of the polypeptide disclosed in the Sequence Listing or the functional homologues thereof as described herein, or is encoded by the nucleic acid molecule characterized herein or the nucleic acid molecule according to the invention, for example by the nucleic acid molecule as shown in the Sequence Listing or its herein described functional homologues and has the herein mentioned activity.

It is further possible to detect environmental stress in plant cells or plants by screening the plant cells for altered metabolic activity as compared to non-stress conditions. This allows for monitoring of stress levels in plants, even when no symptoms are visible. Therefore, counter action can be taken earlier and, e.g., crop losses minimized by timely watering.

It is also within the scope of the invention to screen plant cells or plants for increased tolerance and/or resistance to environmental stress by screening the plant cells under stress conditions for altered metabolic activity as compared to non-stress conditions. This allows selection of plants with increased tolerance and/or resistance to environmental stress without the identification of genes or visual symptoms.

With the invention, it is further possible to breed plant cells or plants towards increased tolerance and/or resistance to environmental stress by screening the plant cells under stress conditions for altered metabolic activity as compared to non-stress conditions and selecting those with increased tolerance and/or resistance to environmental stress. The screening for metabolite activity is faster and easier than e.g. screening for.genes.

Screening is well known to those skilled in the art and generally refers to the search for a particular attribute or trait. In the invention this trait in a plant or plant cell is preferably the concentration of a metabolite, especially preferred the concentration of the above metabolites. The methods and devices for screening are familiar to those skilled in the art and include GC (gas chromatography), LC (liquid chromatography), HPLC (high performance (pressure) liquid chromatography), MS (mass spectrometry), NMR (nuclear magnetic resonance) spectroscopy, IR (infra red) spectroscopy, photometric methods, etc., and combinations of these methods.

Breeding is also customary knowledge for those skilled in the art. It is understood as the directed and stable incorporation of a particular attribute or trait into a plant or plant cell.

The various breeding steps are characterized by well-defined human intervention, such as selecting the lines to be crossed, directing pollination of the parental lines, or selecting appropriate progeny plants. Different breeding measures can be taken, depending on the desired properties. All the techniques are well known by a person skilled in the art and include, for example, but are not limited to, hybridization, inbreeding, backcross breeding, multiline breeding, variety blend, interspecific hybridization, aneuploid techniques, etc. Hybridization techniques also can include the sterilization of plants to yield male or female sterile plants by mechanical, chemical, or biochemical means. Cross pollination of a male sterile plant with pollen of a different line assures that the genome of the male sterile but female fertile plant will uniformly obtain properties of both of the parental lines. The transgenic seeds and plants according to the invention can therefore be used for the breeding of improved plant lines, which can increase the effectiveness of conventional methods, such as herbicide or pesticide treatment, or which allow one to dispense with said methods due to their modified genetic properties. Alternatively new crops with improved stress tolerance, preferably drought and temperature, can be obtained, which, due to their optimized genetic "equipment," yield harvested product of better quality than products that were not able to tolerate comparable adverse developmental conditions.

The invention provides that the environmental stress can be salinity, drought, temperature, metal, chemical, pathogenic, and oxidative stresses, or combinations thereof, preferably drought and/or temperature.

The object of the invention is a transgenic plant cell, wherein the SRP (=stress related protein) is selected preferably from yeast, preferably Saccharomyces cerevisiae, or E. coli or a plant, preferably Brassica napus, Glycine max, or Oryza sativa.

Object of the invention is also a transgenic plant cell, wherein the SRP coding nucleic acid is at least about 50 % homologous to one of the nucleic acid of the Sequence Listing.

In the transgenic plant cell of the invention, the expression of said nucleic acid results in increased tolerance to an environmental stress, which is preferably achieved by altering metabolic activity, as compared to a corresponding non-transformed, wild-type plant cell. Herein, the environmental stress is selected from the group consisting of salinity, drought, temperature, metal, chemical, pathogenic, and oxidative stresses, or combinations thereof, preferably drought and/or temperature.

The term "expression" refers to the transcription and/or translation of a codogenic gene segment or gene. As a rule, the resulting product is an mRNA or a protein. However, expression products can also include functional RNAs such as, for example, antisense, nucleic acids, tRNAs, snRNAs, rRNAs, RNAi, siRNA, ribozymes etc. Expression may be systemic, local or temporal, for example limited to certain cell types, tissue, organs or time periods.

Unless otherwise specified, the terms "polynucleotides," "nucleic acid," and "nucleic acid molecule" are interchangeably in the present context. Unless otherwise specified, the terms "peptide," "polypeptide," and "protein" are interchangeably in the present context. The term "sequence" may relate to polynucleotides, nucleic acids, nucleic acid molecules, peptides, polypeptides and proteins, depending on the context in which the term "sequence" is used. The terms "gene(s)," "polynucleotide," "nucleic acid sequence," "nucleotide sequence," or "nucleic acid molecule(s)," as used herein, refer to a polymeric form of nucleotides of any length, either ribonucleotides or deoxyribonucleotides. The terms refer only to the primary structure of the molecule.

Thus, the terms "gene(s)," "polynucleotide," "nucleic acid sequence," "nucleotide sequence", or "nucleic acid molecule(s)" as used herein, include double- and single-stranded DNA and RNA. They also include known types of modifications, for example, methylation, "caps" substitutions of one or more of the naturally occurring nucleotides with an analog. Preferably, the DNA or RNA sequence of the invention comprises a coding sequence encoding the herein defined polypeptide.

A "coding sequence" is a nucleotide sequence, which is transcribed into mRNA and/or translated into a polypeptide when placed under the control of appropriate regulatory sequences. The boundaries of the coding sequence are determined by a translation start codon at the 5'-terminus and a translation stop codon at the 3'-terminus. A coding sequence can include, but is not limited to, mRNA, cDNA, recombinant nucleotide sequences, or genomic DNA, while introns may be present as well under certain circumstances.

For the purposes of the invention, as a rule, the plural is intended to encompass the singular and vice versa.

Further, the transgenic plant cell is derived from a monocotyledonous plant. Alternative, the transgenic plant cell is derived from a dicotyledonous plant. Preferably, the transgenic plant cell is selected from the group consisting of maize, wheat, rye, oat, triticale, rice, barley, soybean, peanut, cotton, rapeseed, canola, manihot, pepper, sunflower, flax, borage, safflower, linseed, primrose, rapeseed, turnip rape, tagetes, solanaceous plants, potato, tobacco, eggplant, tomato, Vicia species, pea, alfalfa, coffee, cacao, tea, Salix species, oil palm, coconut, perennial grass, forage crops, and Arabidopsis thaliana. Moreover, the transgenic plant cell of the present invention can be derived from a gymnosperm plant. Preferably, the plant is selected from the group of spruce, pine, and fir.

The invention further provides a seed produced by a transgenic plant transformed by a SRP coding nucleic acid, wherein the plant is true breeding for increased tolerance to environmental stress, which is preferably achieved by altering metabolic activity, as compared to a wild-type plant cell. The transgenic plant might be a monocot, a dicot, or a gymnosperm plant. The invention further provides a seed produced by a transgenic plant expressing an SRP, wherein the plant is true breeding for increased tolerance to environmental stress, which is preferably achieved by altering metabolic activity, as compared to a wild-type plant cell. The invention pertains to a seed produced by a transgenic plant, wherein the seed is genetically homozygous for a transgene conferring an increased tolerance to environmental stress, which is preferably achieved by altering metabolic activity, as compared to a wild-type plant.

The invention further provides an agricultural product produced by any of the below-described transgenic plants, plant parts such as leafs, petal, anther, roots, tubers, stems, buds, flowers, or seeds. The invention further provides an isolated recombinant expression vector comprising a SRP encoding nucleic acid.

The invention further provides a method of producing a transgenic plant with a SRP coding nucleic acid, wherein expression of the nucleic acid in the plant results in increased tolerance and/or resistance to an environmental stress, which is preferably achieved by altering metabolic activity, as compared to a corresponding non-transformed, wild-type plant cell, comprising: a) transforming a plant cell with an expression vector including a SRP encoding nucleic acid selected from the group comprising a nucleic acid of the Sequence Listing and/or homologs or parts thereof; and b) generating from the plant cell a transgenic plant with an increased tolerance to environmental stress as compared to a corresponding non-transformed wild-type plant.

With regard to invention described herein, "transformed or transgene" means all those plants or parts thereof which have been brought about by genetic manipulation methods where: a) one or more genes, preferably encoded by one or more nucleic acid sequences as disclosed in the Sequence Listing and/or a homolog thereof; or b) a genetic regulatory element, for example a promoter, which is functionally linked e.g. to the nucleic acid sequence as disclosed in the Sequence Listing and/or a homolog thereof; or c) "a)" and "b)" is/are not present in its/their natural genetic environment or has/have been modified by means of genetic manipulation methods, it being possible for the modification to be, by way of example, a substitution, addition, deletion, inversion or insertion of one or more nucleotide radicals.

"Natural genetic environment" means the natural chromosomal locus in the organism of origin or the presence in a genomic library. In the case of a genomic library, the natural, genetic environment of the nucleic acid sequence is preferably at least partially still preserved. The environment flanks the nucleic acid sequence at least on one side and has a sequence length of at least 50 bp, preferably at least 500 bp, particularly preferably at least 1000 bp, very particularly preferably at least 5000 bp.

In said method for producing a transgenic plant comprising an SRP, the SRP coding nucleic acid is selected from the group comprising a nucleic acid as disclosed in the Sequence Listing and/or homologs of the aforementioned sequences. Further, the SRP coding nucleic acid used in the said method is at least about 50% homologous to one of the nucleic acid sequences as disclosed in the Sequence Listing.

A plant or plant cell is considered "true breeding" for a particular trait if it is genetically homozygous for that trait to the extent that, when the true- breeding plant is self-pollinated, a significant amount of independent segregation of the trait among the progeny is not observed. In the present invention, the trait arises from the transgenic expression of one or more DNA sequences introduced into a plant cell or plant.

The present invention also provides methods of modifying stress tolerance of a plant, comprising modifying the level of expression of a SRP nucleic acid in the plant. The invention provides one method of producing a transgenic plant with a synthetic, novel, or modified transcription factor that acts by increasing the transcription of a SRP gene. Theoretically it is also possible to obtain a decrease in expression of the gene.

A method of detecting environmental stress in plant cells or plants comprising screening the plant cells for altered metabolic activity, as compared to non-stress conditions, is also in the scope of the invention,

Further, a method of screening plant cells or plants for increased tolerance and/or resistance to environmental stress, comprising screening the plant cells under stress conditions for altered metabolic activity, as compared to non-stress conditions, is encompassed in the invention.

The present invention also encloses a method of breeding plant cells or plants towards increased tolerance and/or resistance to environmental stress, comprising screening the plant cells under stress conditions for altered metabolic activity, as compared to non-stress conditions, and selecting those with increased tolerance and/or resistance to environmental stress.

In these methods, metabolite activity is preferably altered concerning the above metabolites and groups of metabolites.

The present invention also encompasses the use of altered metabolic activity and/or a SRP encoding nucleic acid as disclosed in the Sequence Listing and/or homologs of the aforementioned sequences or parts thereof as markers for selection of plants or plant cells with increased tolerance to environmental stress.

The present invention further encompasses the use of altered metabolic activity and/or a SRP encoding nucleic acid as disclosed in the Sequence Listing and/or homologs of the aforementioned sequences or parts thereof as markers for detection of stress in plants or plant cells.

The present invention also provides methods of modifying stress tolerance of a crop plant comprising utilizing a SRP coding nucleic acid sequence to identify individual plants in populations segregating for either increased or decreased environmental stress tolerance (DNA marker).

In the said method of modifying stress tolerance of a plant the SRP encoding nucleic acid may be selected from the nucleic acids as disclosed in the Sequence Listing and/or homologs of the afore mentioned sequences. Further, the SRP coding nucleic acid used therein may at least about 50% homologous to one of the nucleic acids as disclosed in the Sequence Listing. Also an expression vector as described in the present invention might be used in the said method.

In a variant method of said method of modifying stress tolerance, the plant is transformed with an inducible promoter that directs expression of the SRP. For example, the promoter is tissue specific. In a variant method, the used promoter is developmentally regulated.

In a further embodiment, the method of modifying stress tolerance comprises one or more of the following steps: a) stabilizing a protein conferring the increased expression of a protein encoded by the nucleic acid molecule of the invention or of the polypeptide of the invention having the herein-mentioned activity of altering the metabolic activity; b) stabilizing a mRNA conferring the increased expression of a protein encoded by the nucleic acid molecule of the invention or its homologs or of a mRNA encoding the polypeptide of the present invention having the herein-mentioned activity of altering the metabolic activity; c) increasing the specific activity of a protein conferring the increased expression of a protein encoded by the nucleic acid molecule of the invention or of the polypeptide of the present invention or decreasing the inhibitory regulation of the polypeptide of the invention; d) generating or increasing the expression of an endogenous or artificial transcription factor mediating the expression of a protein conferring the increased expression of a protein encoded by the nucleic acid molecule of the invention or of the polypeptide of the invention having the herein-mentioned activity of altering the metabolic activity; e) stimulating activity of a protein conferring the increased expression of a protein encoded by the nucleic acid molecule of the present invention or a polypeptide of the present invention having the herein-mentioned activity of altering the metabolic activity by adding one or more exogenous inducing factors to the organisms or parts thereof; f) expressing a transgenic gene encoding a protein conferring the increased expression of a polypeptide encoded by the nucleic acid molecule of the present invention or a polypeptide of the present invention, having the herein-mentioned activity of altering the metabolic activity; and/or g) increasing the copy number of a gene conferring the increased expression of a nucleic acid molecule encoding a polypeptide encoded by the nucleic acid molecule of the invention or the polypeptide of the invention having the herein-mentioned activity of altering the metabolic activity; h) increasing the expression of the endogenous gene encoding the polypeptide of the invention or its homologs by adding positive expression or removing negative expression elements, e.g. homologous recombination can be used to either introduce positive regulatory elements like for plants the 35S enhancer into the promoter or to remove repressor elements form regulatory regions. Further gene conversion methods can be used to disrupt repressor elements or to enhance to activity of positive elements- positive elements can be randomly introduced in plants by T-DNA or transposon mutagenesis and lines can be identified in which the positive elements have be integrated near to a gene of the invention, the expression of which is thereby enhanced; and/or i) modulating growth conditions of the plant in such a manner, that the expression or activity of the gene encoding the protein of the invention or the protein itself is enhanced; and j) selecting of organisms with especially high activity of the proteins of the invention from natural or from mutagenized resources and breeding them into the target organisms, e.g. the elite crops.

Preferably, said mRNA is the nucleic acid molecule of the present invention and/or the protein conferring the increased expression of a protein encoded by the nucleic acid molecule of the present invention or the polypeptide having the herein mentioned activity is the polypeptide of the present invention, e.g. conferring increased tolerance to environmental stress by altering the metabolic activity.

In general, the amount of mRNA, polynucleotide, or nucleic acid molecule in a cell or a compartment of an organism correlates with the amount of encoded protein, and thus with the overall activity of the encoded protein in said volume. Said correlation is not always linear, the activity in the volume is dependent on the stability of the molecules, the degradation of the molecules or the presence of activating or inhibiting co-factors. Further, product inhibitions of enzymes are well known, e.g. Zinser et al. "Enzyminhibitoren / Enzyme inhibitors".

The activity of the abovementioned proteins and/or polypeptide encoded by the nucleic acid molecule of the present invention can be increased in various ways. For example, the activity in an organism or in a part thereof, like a cell, is increased via increasing the gene product number, e.g. by increasing the expression rate, like introducing a stronger promoter, or by increasing the stability of the mRNA expressed, thus increasing the translation rate, and/or increasing the stability of the gene product, thus reducing the proteins decayed. Further, the activity or turnover of enzymes can be influenced in such a way that a reduction or increase of the reaction rate or a modification (reduction or increase) of the affinity to the substrate results, is reached. A mutation in the catalytic centre of an polypeptide of the invention, e.g. as enzyme, can modulate the turn over rate of the enzyme, e.g. a knock out of an essential amino acid can lead to a reduced or completely knock out activity of the enzyme, or the deletion or mutation of regulator binding sites can reduce a negative regulation like a feedback inhibition (or a substrate inhibition, if the substrate level is also increased). The specific activity of an enzyme of the present invention can be increased such that the turn over rate is increased or the binding of a co-factor is improved. Improving the stability of the encoding mRNA or the protein can also increase the activity of a gene product. The stimulation of the activity is also under the scope of the term "increased activity."

Moreover, the regulation of the abovementioned nucleic acid sequences may be modified so that gene expression is increased. This can be achieved advantageously by means of heterologous regulatory sequences or by modifying, for example mutating, the natural regulatory sequences which are present. The advantageous methods may also be combined with each other.

In general, an activity of a gene product in an organism or part thereof, in particular in a plant cell, a plant, or a plant tissue or a part thereof or in a microorganism can be increased by increasing the amount of the specific encoding mRNA or the corresponding protein in said organism or part thereof. "Amount of protein or mRNA" is understood as meaning the molecule number of polypeptides or mRNA molecules in an organism, a tissue, a cell, or a cell compartment. "Increase" in the amount of a protein means the quantitative increase of the molecule number of said protein in an organism, a tissue, a cell or a cell compartment or part thereof - for example by one of the methods described herein below - in comparison to a wild type, control, or reference.

The increase in molecule number amounts preferably to at least 1%, preferably to more than 10%, more preferably to 30% or more, especially preferably to 50%, 70% or more, very especially preferably to 100%, most preferably to 500% or more. However, a de novo expression is also regarded as subject of the present invention.

A modification, i.e. an increase or decrease, can be caused by endogenous or exogenous factors. For example, an increase in activity in an organism or a part thereof can be caused by adding a gene product or a precursor or an activator or an agonist to the media or nutrition or can be caused by introducing said subjects into a organism, transient or stable.

In one embodiment the increase or decrease in metabolic activity in the plant or a part thereof, e.g. in a cell, a tissue, an organ, an organelle, etc., is achieved by increasing the endogenous level of the polypeptide of the invention. Accordingly, in an embodiment of the present invention, the present invention relates to a process, wherein the gene copy number of a gene encoding the polynucleotide or nucleic acid molecule of the invention is increased. Further, the endogenous level of the polypeptide of the invention can, for example, be increased by modifying the transcriptional or translational regulation of the polypeptide.

In one embodiment the metabolic activity in the plant or part thereof can be altered by targeted or random mutagenesis of the endogenous genes of the invention. For example homologous recombination can be used to either introduce positive regulatory elements like for plants the 35S enhancer into the promoter or to remove repressor elements form regulatory regions. In addition gene conversion like methods described by Kochevenko and Willmitzer (Plant Physiol. 2003 May;132(1):174-84) and citations therein can be used to disrupt repressor elements or to enhance to activity of positive regulatory elements. Furthermore positive elements can be randomly introduced in (plant) genomes by T-DNA or transposon mutagenesis and lines can be screened for, in which the positive elements has be integrated near to a gene of the invention, the expression of which is thereby enhanced. The activation of plant genes by random integrations of enhancer elements has been described by Hayashi et al., 1992 (Science 258:1350-1353) or Weigel et al., 2000 (Plant Physiol. 122, 1003-1013) and others citied therein.

Reverse genetic strategies to identify insertions (which eventually carrying the activation elements) near in genes of interest have been described for various cases e.g. Krysan et al., 1999 (Plant Cell 1999, 11, 2283-2290); Sessions et al., 2002 (Plant Cell 2002, 14, 2985-2994); Young et al., 2001, (Plant Physiol. 2001, 125, 513-518); Koprek et al., 2000 (Plant J. 2000, 24, 253-263); Jeon et al., 2000 (Plant J. 2000, 22, 561-570); Tissier et al., 1999 (Plant Cell 1999, 11, 1841-1852); Speulmann et al., 1999 (Plant Cell 1999 ,11 , 1853-1866). Briefly material from all plants of a large T-DNA or transposon mutagenized plant population is harvested and genomic DNA prepared. Then the genomic DNA is pooled following specific architectures as described for example in Krysan et al., 1999 (Plant Cell 1999, 11, 2283-2290). Pools of genomics DNAs are then screened by specific multiplex PCR reactions detecting the combination of the insertional mutagen (e.g. T-DNA or Transposon) and the gene of interest. Therefore PCR reactions are run on the DNA pools with specific combinations of T-DNA or transposon border primers and gene specific primers. General rules for primer design can again be taken from Krysan et al., 1999 (Plant Cell 1999, 11, 2283-2290) Rescreening of lower levels DNA pools lead to the identification of individual plants in which the gene of interest is activated by the insertional mutagen.

The enhancement of positive regulatory elements or the disruption or weakening of negative regulatory elements can also be achieved through common mutagenesis techniques: The production of chemically or radiation mutated populations is a common technique and known to the skilled worker. Methods for plants are described by Koorneef et al. 1982 and the citations therein and by Lightner and Caspar in "Methods in Molecular Biology" Vol 82. These techniques usually induce point mutations that can be identified in any known gene using methods such as TILLING (Colbert et al. 2001).

Accordingly, the expression level can be increased if the endogenous genes encoding a polypeptide conferring an increased expression of the polypeptide of the present invention, in particular genes comprising the nucleic acid molecule of the present invention, are modified via homologous recombination, Tilling approaches or gene conversion

Regulatory sequences can be operatively linked to the coding region of an endogenous protein and control its transcription and translation or the stability or decay of the encoding mRNA or the expressed protein. In order to modify and control the expression, promoter, UTRs, splicing sites, processing signals, polyadenylation sites, terminators, enhancers, repressors, post transcriptional, or posttranslational modification sites can be changed, added, or amended For example, the activation of plant genes by random integrations of enhancer elements has been described by Hayashi et al., 1992 (Science 258:1350-1353) or Weigel et al., 2000 (Plant Physiol. 122, 1003-1013) and others citied therein. For example, the expression level of the endogenous protein can be modulated by replacing the endogenous promoter with a stronger transgenic promoter or by replacing the endogenous 3'UTR with a 3'UTR, which provides more stability without amending the coding region. Further, the transcriptional regulation can be modulated by introduction of an artificial transcription factor as described in the examples. Alternative promoters, terminators, and UTRs are described below.

The activation of an endogenous polypeptide having above*-mentioned activity, e.g., conferring an increased tolerance to environmental stress after altering the metabolic activity, can also be increased by introducing a synthetic transcription factor, which binds close to the coding region of the protein of the invention-encoding gene and activates its transcription. A chimeric zinc finger protein can be construed, which comprises a specific DNA-binding domain and an activation domain as e.g. the VP16 domain of Herpes Simplex virus. The specific binding domain can bind to the regulatory region of the protein-coding region. The expression of the chimeric transcription factor in a plant leads to a specific expression of the protein of the invention, see e.g. in WO01/52620, Oriz, Proc. Natl. Acad. Sci. USA, 2002, Vol. 99, 13290 or Guan, Proc. Natl. Acad. Sci. USA, 2002, Vol. 99, 13296.

In one further embodiment of the method according to the invention, plants are used in which one of the abovementioned genes, or one of the abovementioned nucleic acids, is mutated in a way that the activity of the encoded gene products is less influenced by cellular factors, or not at all, in comparison with the unmutated proteins. For example, well known regulation mechanism of enzymatic activity are substrate inhibition or feed back regulation mechanisms. Ways and techniques for the introduction of substitutions, deletions and additions of one or more bases, nucleotides or amino acids of a corresponding sequence are described herein below in the corresponding paragraphs and the references listed there, e.g. in Sambrook et al., Molecular Cloning, Cold Spring Harbor, NY, 1989. The person skilled in the art will be able to identify regulation domains and binding sites of regulators by comparing the sequence of the nucleic acid molecule of the present invention or the expression product thereof with the state of the art by computer software means which comprise algorithms for the identifying of binding sites and regulation domains or by introducing into a nucleic acid molecule or in a protein systematically mutations and assaying for those mutations which will lead to an increased specific activity or an increased activity per volume, in particular per cell.

It is therefore advantageously to express in a plant a nucleic acid molecule of the invention or a polypeptide of the invention derived from a evolutionary distantly related organism, as e.g. using a prokaryotic gene in a eukaryotic host, as in these cases the regulation mechanism of the host cell may not weaken the activity (cellular or specific) of the gene or its expression product

The mutation is introduced in such a way that the production of the amino acids is not adversely affected.

Less influence on the regulation of a gene or its gene product is understood as meaning a reduced regulation of the enzymatic activity leading to an increased specific or cellular activity of the gene or its product. An increase of the enzymatic activity is understood as meaning an enzymatic activity, which is increased by at least 10%, advantageously at least 20, 30, or 40%, especially advantageously by at least 50, 60 or 70% in comparison with the starting organism.

The invention provides that the above methods can be performed such that the stress tolerance is increased. It is also possible to obtain a decrease in stress tolerance.

The invention is not limited to specific nucleic acids, specific polypeptides, specific cell types, specific host cells, specific conditions, or specific methods etc., as such, but may vary and numerous modifications and variations therein will be apparent to those skilled in the art. It is also to be understood that the terminology used herein is for the purpose of describing specific embodiments only and is not intended to be limiting.

The present invention also relates to isolated Stress Related Proteins (SRP), which are as disclosed in the Sequence Listing homologs thereof. Preferably, the isolated Stress Related Proteins (SRP) of the present invention are selected from yeast or E. coli. Further, the present invention is related to isolated Stress Related Protein (SRP) encoding nucleic acids selected from the group comprising the nucleic acid of the Sequence Listing and/or homologs thereof. Here, preferably, an isolated Stress Related Protein (SRP) encoding nucleic acid encodes an SRP, which is selected from yeast or E. coli.

The present invention provides stress-related gene sequences selected from the group consisting of the nucleic acid, as disclosed in the Sequence Listing of yeast, preferably from Saccharomyces cerevisiae or E. coli.

Homologs of the aforementioned sequences can be isolated advantageously from yeast, fungi, viruses, algae, bacteria, such as Acetobacter (subgen. Acetobacter) aceti; Acidithiobacillus ferrooxidans; Acinetobacter sp.; Actinobacillus sp; Aeromonas salmonicida; Agrobacterium tumefaciens; Aquifex aeolicus; Arcanobacterium pyogenes; Aster yellows phytoplasma; Bacillus sp.; Bifidobacterium sp.; Borrelia burgdorferi; Brevibacterium linens; Brucella melitensis; Buchnera sp.; Butyrivibrio fibrisolvens; Campylobacter jejuni; Caulobacter crescentus; Chlamydia sp.; Chlamydophila sp.; Chlorobium limicola; Citrobacter rodentium; Clostridium sp.; Comamonas testosterbni; Corynebacterium sp.; Coxiella burnetii; Deinococcus radiodurans; Dichelobacter nodosus; Edwardsiella ictaluri; Enterobacter sp.; Erysipelothrix rhusiopathiae; Escherichia coli; Flavobacterium sp.; Francisella tularensis; Frankia sp. Cpl1; Fusobacterium nucleatum; Geobacillus stearothermophilus; Gluconobacter oxydans; Haemophilus sp.; Helicobacter pylori; Klebsiella pneumoniae; Lactobacillus sp.; Lactococcus lactis; Listeria sp.; Mannheimia haemolytica; Mesorhizobium loti; Methylophaga thalassica; Microcystis aeruginosa; Microscilia sp. PRE1; Moraxella sp. TA144; Mycobacterium sp.; Mycoplasma sp.; Neisseria sp.; Nitrosomonas sp.; Nostoc sp. PCC 7120; Novosphingobium aromaticivorans; Oenococcus oeni; Pantoea citrea; Pasteurella multocida; Pediococcus pentosaceus; Phormidium foveolarum; Phytoplasma sp.; Plectonema boryanum; Prevotella ruminicola; Propionibacterium sp.; Proteus vulgaris; Pseudomonas sp.; Ralstonia sp.; Rhizobium sp.; Rhodococcus equi; Rhodothermus marinus; Rickettsia sp.; Riemerella anatipestifer; Ruminococcus flavefaciens; Salmonella sp.; Selenomonas ruminantium; Serratia entomophila; Shigella sp.; Sinorhizobium meliloti; Staphylococcus sp.; Streptococcus sp.; Streptomyces sp.; Synechococcus sp.; Synechocystis sp. PCC 6803; Thermotoga maritima; Treponema sp.; Ureaplasma urealyticum; Vibrio cholerae; Vibrio parahaemolyticus; Xylella fastidiosa; Yersinia sp.; Zymomonas mobilis, preferably Salmonella sp. or Escherichia coli or plants, preferably from yeasts such as from the genera Saccharomyces, Pichia, Candida, Hansenula, Torulopsis or Schizosaccharomyces or plants such as Arabidopsis thaliana, maize, wheat, rye, oat, triticale, rice, barley, soybean, peanut, cotton, borage, sufflower, linseed, primrose, rapeseed, canola and turnip rape, manihot, pepper, sunflower, tagetes, solanaceous plant such as potato, tobacco, eggplant and tomato, Vicia species, pea, alfalfa, bushy plants such as coffee, cacao, tea, Salix species, trees such as oil palm, coconut, perennial grass, such as ryegrass and fescue, and forage crops, such as alfalfa and clover, and from spruce, pine, or fir, for example. More preferably homologs of aforementioned sequences can be isolated from Saccharomyces cerevisiae, E. coli or plants, preferably Brassica napus, Glycine max, or Oryza sativa.

The stress-related proteins of the present invention are preferably produced by recombinant DNA techniques. For example, a nucleic acid molecule encoding the protein is cloned into an expression vector, for example, into a binary vector, the expression vector is introduced into a host cell, for example, the Arabidopsis thaliana, wild-type NASC N906, or any other plant cell as described in the examples (see below), and the stress-related protein is expressed in said host cell. Examples for binary vectors are pBIN19, pBI101, pBinAR, pGPTV, pCAMBIA, pBIB-HYG, pBecks, pGreen or pPZP (Hajukiewicz, P. et al., 1994, Plant Mol. Biol., 25: 989-994 and Hellens et al, Trends in Plant Science (2000) 5, 446-451.).

Advantageously, the nucleic acid sequences, according to the invention, or the gene construct, together with at least one reporter gene, are cloned into an expression cassette, which is introduced into the organism via a vector or directly into the genome. This reporter gene should allow easy detection via a growth, fluorescence, chemical, bioluminescence, or resistance assay or via a photometric measurement. Examples of reporter genes, which may be mentioned, are antibiotic- or herbicide-resistance genes, hydrolase genes, fluorescence protein genes, bioluminescence genes, sugar or nucleotide metabolic genes or biosynthesis genes such as the Ura3 gene, the IIv2 gene, the luciferase gene, the β-galactosidase gene, the gfp gene, the 2-desoxyglucose-6-phosphate phosphatase gene, the β-glucuronidase gene, β-lactamase gene, the neomycin phosphotransferase gene, the hygromycin phosphotransferase gene or the BASTA (= gluphosinate-resistance) gene. These genes permit easy measurement and quantification of the transcription activity and hence of the expression of the genes. In this way genome positions may be identified which exhibit differing productivity.

In a preferred embodiment, a nucleic acid construct, for example an expression cassette, comprises upstream, i.e. at the 5' end of the encoding sequence, a promoter and downstream, i.e. at the 3' end, a polyadenylation signal and optionally other regulatory elements, which are operably linked to the intervening encoding sequence with the nucleic acid of as disclosed in the Sequence Listing. By an operable linkage is meant the sequential arrangement of promoter, encoding sequence, terminator, and optionally other regulatory elements in such a way that each of the regulatory elements can fulfill its function in the expression of the encoding sequence in due manner. The sequences preferred for operable linkage are targeting sequences for ensuring subcellular localization in plastids. However, targeting sequences for ensuring subcellular localization in the mitochondrion, in the endoplasmic reticulum (= ER), in the nucleus, in oil corpuscles or other compartments may also be employed as well as translation promoters such as the 5' lead sequence in tobacco mosaic virus (Gallie et al., Nucl. Acids Res. 15 (1987), 8693-8711).

A nucleic acid construct, for example an expression cassette may, for example, contain a constitutive promoter or a tissue-specific promoter (preferably the USP or napin promoter) the gene to be expressed and the ER retention signal. For the ER retention signal the KDEL (SEQ ID NO: 531) amino acid sequence (lysine, aspartic acid, glutamic acid, leucine) or the KKX amino acid sequence (lysinelysine-X-stop, wherein X means every other known amino acid) is preferably employed.

For expression in a prokaryotic or eukaryotic host organism, for example, a microorganism, such as a fungus or a plant the expression cassette, is advantageously inserted into a vector such as by way of example a plasmid, a phage, or other DNA which allows optimum expression of the genes in the host organism. Examples of suitable plasmids are: in E. coli pLG338, pACYC184, pBR series such as e.g. pBR322, pUC series such as pUC18 or pUC19, M113mp series, pKC30, pRep4, pHS1, pHS2, pPLc236, pMBL24, pLG200, pUR290, pIN-III¹¹³-B1, λgt11 or pBdCl; in Streptomyces pIJ101, pIJ364, pIJ702 or pIJ361; in Bacillus pUB110, pC194 or pBD214; in Corynebacterium pSA77 or pAJ667; in fungi pALS1, pIL2 or pBB116; other advantageous fungal vectors are described by Romanos, M.A. et al., ((1992) "Foreign gene expression in yeast: a review," Yeast 8: 423-488) and by van den Hondel, C.A.M.J.J. et al. ((1991) "Heterologous gene expression in filamentous fungi,') as well as in More Gene Manipulations in Fungi (J.W. Bennet & L.L. Lasure, eds., pp. 396-428: Academic Press: San Diego) and in "Gene transfer systems and vector development for filamentous fungi" (van den Hondel, C.A.M.J.J. & Punt, P.J. (1991) in: Applied Molecular Genetics of Fungi, Peberdy, J.F. et al., eds., pp. 1-28, Cambridge University Press: Cambridge). Examples of advantageous yeast promoters are 2µM, pAG-1, YEp6, YEp13, or pEMBLYe23. Examples of algal or plant promoters are pLGV23, pGHlac⁺, pBIN19, pAK2004, pVKH or pDH51 (see Schmidt, R. and Willmitzer, L., 1988). The vectors identified above, or derivatives of the vectors identified above, are a small selection of the possible plasmids. Further plasmids are well known to those skilled in the art and may be found, for example, in the book Cloning Vectors (Eds. Pouwels P.H. et al. Elsevier, Amsterdam-New York-Oxford, 1985, ISBN 0 444 904018. Suitable plant vectors are described inter alia in "Methods in Plant Molecular Biology and Biotechnology" (CRC Press, Ch. 6/7, pp. 71-119). Advantageous vectors are known as shuttle vectors or binary vectors, which replicate in E. coli and Agrobacterium.

By vectors is meant, with the exception of plasmids, all other vectors known to those skilled in the art, such as by way of example phages, viruses such as SV40, CMV, baculovirus, adenovirus, transposons, IS elements, phasmids, phagemids, cosmids, linear, or circular DNA. These vectors can be replicated autonomously in the host organism or be chromosomally replicated, chromosomal replication being preferred.

In a further embodiment of the vector, the expression cassette, according to the invention, may also advantageously be introduced into the organisms in the form of a linear DNA and be integrated into the genome of the host organism by way of heterologous or homologous recombination. This linear DNA may be composed of a linearized plasmid or only of the expression cassette as vector or the nucleic acid sequences according to the invention.

In a further advantageous embodiment, the nucleic acid sequence, according to the invention, can also be introduced into an organism on its own.

If, in addition to the nucleic acid sequence according to the invention, further genes are to be introduced into the organism, all together with a reporter gene in a single vector, or each single gene with a reporter gene in a vector, in each case can be introduced into the organism, whereby the different vectors can be introduced simultaneously or successively.

The vector advantageously contains at least one copy of the nucleic acid sequences according to the invention and/or the expression cassette (= gene construct) according to the invention.

The invention further provides an isolated recombinant expression vector comprising a SRP nucleic acid as described above, wherein expression of the vector in a host cell results in increased tolerance to environmental stress as compared to a wild-type variety of the host cell. As used herein, the term "vector" refers to a nucleic acid molecule capable of transporting another nucleic acid, to which it has been linked. One type of vector is a "plasmid," which refers to a circular double stranded DNA loop, into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors." In general, expression vectors of utility in recombinant DNA techniques are often in the form of plasmids. In the present specification, "plasmid" and "vector" can be used interchangeably, as the plasmid is the most commonly used form of vector. However, the invention is intended to include such other forms of expression vectors, such as viral vectors (e.g., replication defective retroviruses, adenoviruses, and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. As used herein, with respect to a recombinant expression vector, "operatively linked" is intended to mean that the nucleotide sequence of interest is linked to the regulatory sequence(s) in a manner which allows for expression of the nucleotide sequence (e.g., in an in vitro transcription/translation system or in a host cell when the vector is introduced into the host cell). The term "regulatory sequence" is intended to include promoters, enhancers, and other expression control elements (e.g., polyadenylation signals). Such regulatory sequences are described, for example, in Goeddel, Gene Expression Technology: Methods in Enzymology 185, Academic Press, San Diego, CA (1990) and Gruber and Crosby, in: Methods in Plant Molecular Biology and Biotechnology, eds. Glick and Thompson, Chapter 7, 89-108, CRC Press: Boca Raton, Florida, including the references therein. Regulatory sequences include those that direct constitutive expression of a nucleotide sequence in many types of host cells and those that direct expression of the nucleotide sequence only in certain host cells or under certain conditions. It will be appreciated by those skilled in the art that the design of the expression vector can depend on such factors as the choice of the host cell to be transformed, the level of expression of polypeptide desired, etc. The expression vectors of the invention can be introduced into host cells to thereby produce polypeptides or peptides, including fusion polypeptides or peptides, encoded by nucleic acids as described herein (e.g., SRPs, mutant forms of SRPs, fusion polypeptides, etc.)

The recombinant expression vectors of the invention can be designed for expression of SRPs in prokaryotic or eukaryotic cells. For example, SRP genes can be expressed in bacterial cells such as C. glutamicum, insect cells (using baculovirus expression vectors), yeast and other fungal cells (See Romanos, M.A. et al., 1992, Foreign gene expression in yeast: a review, Yeast 8:423-488; van den Hondel, C.A.M.J.J. et al., 1991, Heterologous gene expression in filamentous fungi, in: More Gene Manipulations in Fungi, J.W. Bennet & L.L. Lasure, eds., p. 396-428: Academic Press: San Diego; and van den Hondel, C.A.M.J.J. & Punt, P.J., 1991, Gene transfer systems and vector development for filamentous fungi, in: Applied Molecular Genetics of Fungi, Peberdy, J.F. et al., eds., p. 1-28, Cambridge University Press: Cambridge), algae (Falciatore et al., 1999, Marine Biotechnology 1 (3):239-251), ciliates of the types: Holotrichia, Peritrichia, Spirotrichia, Suctoria, Tetrahymena, Paramecium, Colpidium, Glaucoma, Platyophrya, Potomacus, Pseudocohnilembus, Euplotes, Engelmaniella, and Stylonychia, especially of the genus Stylonychia lemnae with vectors following a transformation method as described in PCT Application No. WO 98/01572, and multicellular plant cells (See Schmidt, R. and Willmitzer, L., 1988, High efficiency Agrobacterium tumefaciens-mediated transformation of Arabidopsis thaliana leaf and cotyledon explants, Plant Cell Rep. 583-586; Plant Molecular Biology and Biotechnology, C Press, Boca Raton, Florida, chapter 6/7, S.71-119 (1993); F.F. White, B. Jenes et al., Techniques for Gene Transfer, in: Transgenic Plants, Vol. 1, Engineering and Utilization, eds. Kung und R. Wu, 128-43, Academic Press: 1993; Potrykus, 1991, Annu. Rev. Plant Physiol. Plant Molec. Biol. 42:205-225 and references cited therein) or mammalian cells. Suitable host cells are discussed further in Goeddel, Gene Expression Technology: Methods in Enzymology 185, Academic Press: San Diego, CA (1990). Alternatively, the recombinant expression vector can be transcribed and translated in vitro, for example using T7 promoter regulatory sequences and T7 polymerase.

Expression of polypeptides in prokaryotes is most often carried out with vectors containing constitutive or inducible promoters directing the expression of either fusion or non-fusion polypeptides. Fusion vectors add a number of amino acids to a polypeptide encoded therein, usually to the amino terminus of the recombinant polypeptide but also to the C-terminus or fused within suitable regions in the polypeptides. Such fusion vectors typically serve three purposes: 1) to increase expression of a recombinant polypeptide; 2) to increase the solubility of a recombinant polypeptide; and 3) to aid in the purification of a recombinant polypeptide by acting as a ligand in affinity purification. Often, in fusion expression vectors, a proteolytic cleavage site is introduced at the junction of the fusion moiety and the recombinant polypeptide to enable separation of the recombinant polypeptide from the fusion moiety subsequent to purification of the fusion polypeptide. Such enzymes, and their cognate recognition sequences, include Factor Xa, thrombin, and enterokinase.

By way of example the plant expression cassette can be installed in the pRT transformation vector ((a) Toepfer et al., 1993, Methods Enzymol., 217: 66-78; (b) Toepfer et al. 1987, Nucl. Acids. Res. 15: 5890 ff.).

Alternatively, a recombinant vector (= expression vector) can also be transcribed and translated in vitro, e.g. by using the T7 promoter and the T7 RNA polymerase.

Expression vectors employed in prokaryotes frequently make use of inducible systems with and without fusion proteins or fusion oligopeptides, wherein these fusions can ensue in both N-terminal and C-terminal manner or in other useful domains of a protein. Such fusion vectors usually have the following purposes: i) to increase the RNA expression rate; ii) to increase the achievable protein synthesis rate; iii) to increase the solubility of the protein; or iv) to simplify purification by means of a binding sequence usable for affinity chromatography. Proteolytic cleavage points are also frequently introduced via fusion proteins, which allow cleavage of a portion of the fusion protein and purification. Such recognition sequences for proteases are recognized, e.g. factor Xa, thrombin, and enterokinase.

Typical advantageous fusion and expression vectors are pGEX [Pharmacia Biotech Inc; Smith, D.B. and Johnson, K.S. (1988) Gene 67: 31-40], pMAL (New England Biolabs, Beverly, MA) and pRIT5 (Pharmacia, Piscataway, NJ), which contains glutathione S-transferase (GST), maltose binding protein or protein A.

In one embodiment, the coding sequence of the SRP is cloned into a pGEX expression vector to create a vector encoding a fusion polypeptide comprising, from the N-terminus to the C-terminus, GST-thrombin cleavage site-X polypeptide. The fusion polypeptide can be purified by affinity chromatography using glutathione-agarose resin. Recombinant PKSRP unfused to GST can be recovered by cleavage of the fusion polypeptide with thrombin.

Other examples of E. coli expression vectors are pTrc (Amann et al., (1988) Gene 69:301-315) and pET vectors (Studier et al., Gene Expression Technology: Methods in Enzymology 185, Academic Press, San Diego, California (1990) 60-89; Stratagene, Amsterdam, The Netherlands).

Target gene expression from the pTrc vector relies on host RNA polymerase transcription from a hybrid trp-lac fusion promoter. Target gene expression from the pET 11d vector relies on transcription from a T7 gn10-lac fusion promoter mediated by a co-expressed viral RNA polymerase (T7 gn1). This viral polymerase is supplied by host strains BL21(DE3) or HMS174(DE3) from a resident λ prophage harboring a T7 gn1 gene under the transcriptional control of the lacUV 5 promoter.

One strategy to maximize recombinant polypeptide expression is to express the polypeptide in a host bacteria with an impaired capacity to proteolytically cleave the recombinant polypeptide (Gottesman, S., Gene Expression Technology: Methods in Enzymology 185, Academic Press, San Diego, California (1990) 119-128). Another strategy is to alter the sequence of the nucleic acid to be inserted into an expression vector so that the individual codons for each amino acid are those preferentially utilized in the bacterium chosen for expression, such as C. glutamicum (Wada et al., 1992, Nucleic Acids Res. 20:2111-2118). Such alteration of nucleic acid sequences of the invention can be carried out by standard DNA synthesis techniques.

Other advantageous vectors for use in yeast are pYepSec1 (Baldari, et al., (1987) Embo J. 6:229-234), pMFa (Kurjan and Herskowitz, (1982) Cell 30:933-943), pJRY88 (Schultz et al., (1987) Gene 54:113-123), and pYES derivatives (Invitrogen Corporation, San Diego, CA). Vectors for use in filamentous fungi are described in: van den Hondel, C.A.M.J.J. & Punt, P.J. (1991) "Gene transfer systems and vector development for filamentous fungi," in: Applied Molecular Genetics of Fungi, J.F. Peberdy, et al., eds., pp. 1-28, Cambridge University Press: Cambridge.

Alternatively, insect cell expression vectors can also be advantageously utilized, e.g. for expression in Sf 9 cells. These are e.g. the vectors of the pAc series (Smith et al. (1983) Mol. Cell Biol. 3:2156-2165) and the pVL series (Lucklow and Summers (1989) Virology 170:31-39).

Furthermore, plant cells or algal cells can advantageously be used for gene expression. Examples of plant expression vectors may be found in Becker, D., et al. (1992) "New plant binary vectors with selectable markers located proximal to the left border," Plant Mol. Biol. 20: 1195-1197 or in Bevan, M.W. (1984) "Binary Agrobacterium vectors for plant transformation," Nucl. Acid. Res. 12: 8711-8721.

Furthermore, the nucleic acid sequences may also be expressed in mammalian cells, advantageously in nonhuman mammalian cells. Examples of corresponding expression vectors are pCDM8 and pMT2PC referred to in: Seed, B. (1987) Nature 329:840 or Kaufman et al. (1987) EMBO J. 6: 187-195). At the same time promoters preferred for use are of viral origin, such as by way of example promoters of polyoma, adenovirus 2, cytomegalovirus, or simian virus 40. Other prokaryotic and eukaryotic expression systems are referred to in chapters 16 and 17 of Sambrook et al., Molecular Cloning: A Laboratory Manual, 2nd ed., Cold Spring Harbor Laboratory, Cold Spring Harbor Laboratory Press, Cold Spring Harbor, NY, 1989.

In a preferred embodiment of the present invention, the SRPs are expressed in plants and plants cells such as unicellular plant cells (e.g. algae) (See Falciatore et al., 1999, Marine Biotechnology 1 (3):239-251 and references therein) and plant cells from higher plants (e.g., the spermatophytes, such as crop plants). A SRP may be "introduced" into a plant cell by any means, including transfection, transformation or transduction, electroporation, particle bombardment, agroinfection, and the like. One transformation method known to those of skill in the art is the dipping of a flowering plant into an Agrobacteria solution, wherein the Agrobacteria contains the SRP nucleic acid, followed by breeding of the transformed gametes.

Other suitable methods for transforming or transfecting host cells including plant cells can be found in Sambrook, et al., Molecular Cloning: A Laboratory Manual, 2nd ed., Cold Spring Harbor Laboratory, Cold Spring Harbor Laboratory Press, Cold Spring Harbor, NY, 1989, and other laboratory manuals such as Methods in Molecular Biology, 1995, Vol. 44, Agrobacterium protocols, ed: Gartland and Davey, Humana Press, Totowa, New Jersey. As biotic and abiotic stress tolerance is a general trait wished to be inherited into a wide variety of plants like maize, wheat, rye, oat, triticale, rice, barley, soybean, peanut, cotton, rapeseed and canola, manihot, pepper, sunflower and tagetes, solanaceous plants like potato, tobacco, eggplant, and tomato, Vicia species, pea, alfalfa, bushy plants (coffee, cacao, tea), Salix species, trees (oil palm, coconut), perennial grasses, and forage crops, these crop plants are also preferred target plants for a genetic engineering as one further embodiment of the present invention. Forage crops include, but are not limited to, Wheatgrass, Canarygrass, Bromegrass, Wildrye Grass, Bluegrass, Orchardgrass, Alfalfa, Salfoin, Birdsfoot Trefoil, Alsike Clover, Red Clover, and Sweet Clover.

In one embodiment of the present invention, transfection of an SRP into a plant is achieved by Agrobacterium-mediated gene transfer. Agrobacterium-mediated plant transformation can be performed using, for example, the GV3101(pMP90) (Koncz and Schell, 1986, Mol. Gen. Genet. 204:383-396) or LBA4404 (Clontech) Agrobacterium tumefaciens strain. Transformation can be performed by standard transformation and regeneration techniques (Deblaere et al., 1994, Nucl. Acids Res. 13:4777-4788; Gelvin, Stanton B. and Schilperoort, Robert A, Plant Molecular Biology Manual, 2nd Ed. - Dordrecht: Kluwer Academic Publ., 1995. - in Sect., Ringbuc Zentrale Signatur: BT11-P ISBN 0-7923-2731-4; Glick, Bernard R.; Thompson, John E., Methods in Plant Molecular Biology and Biotechnology, Boca Raton : CRC Press, 1993 360 S., ISBN 0-8493-5164-2). For example, rapeseed can be transformed via cotyledon or hypocotyl transformation (Moloney et al., 1989, Plant cell Report 8:238-242; De Block et al., 1989, Plant Physiol. 91:694-701). Use of antibiotics for Agrobacterium and plant selection depends on the binary vector and the Agrobacterium strain used for transformation. Rapeseed selection is normally performed using kanamycin as selectable plant marker. Agrobacterium-mediated gene transfer to flax can be performed using, for example, a technique described by Mlynarova et al., 1994, Plant Cell Report 13:282-285. Additionally, transformation of soybean can be performed using, for example, a technique described in European Patent No. 0424 047, U.S. Patent No. 5,322,783, European Patent No. 0397 687, U.S. Patent No. 5,376,543, or U.S. Patent No. 5,169,770. Transformation of maize can be achieved by particle bombardment, polyethylene-glycol-mediated DNA uptake, or via the silicon carbide fiber technique. (See, for example, Freeling and Walbot, "The maize handbook" Springer Verlag: New York (1993) ISBN 3-540-97826-7). A specific example of maize transformation is found in U.S. Patent No. 5,990,387, and a specific example of wheat transformation can be found in PCT Application No. WO 93/07256.

According to the present invention, the introduced SRP may be maintained in the plant cell stably if it is incorporated into a non-chromosomal autonomous replicon or integrated into the plant chromosomes. Alternatively, the introduced SRP may be present on an extra-chromosomal non-replicating vector and be transiently expressed or transiently active.

In one embodiment, a homologous recombinant microorganism can be created wherein the SRP is integrated into a chromosome, a vector is prepared, which contains at least a portion of a SRP gene into which a deletion, addition, or substitution has been introduced to thereby alter, e.g., functionally disrupt, the SRP gene. Preferably, the SRP gene is a yeast, E.coli, Brassica napus, Glycine max, or Oryza sativa SRP gene, but it can be a homolog from a related plant or even from a mammalian or insect source. In one embodiment, the vector is designed such that, upon homologous recombination, the endogenous SRP gene is functionally disrupted (i.e., no longer encodes a functional polypeptide; also referred to as a knock-out vector). Alternatively, the vector can be designed such that, upon homologous recombination, the endogenous SRP gene is mutated or otherwise altered but still encodes a functional polypeptide (e.g., the upstream regulatory region can be altered to thereby alter the expression of the endogenous SRP). To create a point mutation via homologous recombination, DNA-RNA hybrids can be used in a technique known as chimeraplasty (Cole-Strauss et al., 1999, Nucleic Acids Research 27(5):1323-1330 and Kmiec, 1999 Gene therapy American Scientist. 87(3):240-247). Homologous recombination procedures in Physcomitrella patens are also well known in the art and are contemplated for use herein.

Whereas in the homologous recombination vector, the altered portion of the SRP gene is flanked at its 5' and 3' ends by an additional nucleic acid molecule of the SRP gene to allow for homologous recombination to occur between the exogenous SRP gene carried by the vector and an endogenous SRP gene, in a microorganism or plant. The additional flanking SRP nucleic acid molecule is of sufficient length for successful homologous recombination with the endogenous gene. Typically, several hundreds of base pairs up to kilobases of flanking DNA (both at the 5' and 3' ends) are included in the vector. See, e.g., Thomas, K.R., and Capecchi, M.R., 1987, Cell 51:503 for a description of homologous recombination vectors or Strepp et al., 1998, PNAS, 95 (8):4368-4373 for cDNA based recombination in Physcomitrella patens). The vector is introduced into a microorganism or plant cell (e.g., via polyethylene glycol mediated DNA), and cells in which the introduced PKSRP gene has homologously recombined with the endogenous PKSRP gene are selected using art-known techniques.

In another embodiment, recombinant microorganisms can be produced that contain selected systems, which allow for regulated expression of the introduced gene. For example, inclusion of a SRP gene on a vector placing it under control of the lac operon permits expression of the SRP gene only in the presence of IPTG. Such regulatory systems are well known in the art.

Whether present in an extra-chromosomal non-replicating vector or a vector that is integrated into a chromosome, the SRP polynucleotide preferably resides in a plant expression cassette. A plant expression cassette preferably contains regulatory sequences capable of driving gene expression in plant cells that are operatively linked so that each sequence can fulfill its function, for example, termination of transcription by polyadenylation signals. Preferred polyadenylation signals are those originating from Agrobacterium tumefaciens t-DNA such as the gene 3 known as octopine synthase of the Ti-plasmid pTiACH5 (Gielen et al., 1984, EMBO J. 3:835) or functional equivalents thereof but also all other terminators functionally active in plants are suitable. As plant gene expression is very often not limited on transcriptional levels, a plant expression cassette preferably contains other operatively linked sequences like translational enhancers such as the overdrive-sequence containing the 5'-untranslated leader sequence from tobacco mosaic virus enhancing the polypeptide per RNA ratio (Gallie et al., 1987, Nucl. Acids Research 15:8693-8711). Examples of plant expression vectors include those detailed in: Becker, D. et al., 1992, New plant binary vectors with selectable markers located proximal to the left border, Plant Mol. Biol. 20: 1195-1197; and Bevan, M.W., 1984, Binary Agrobacterium vectors for plant transformation, Nucl. Acid. Res. 12:8711-8721; and Vectors for Gene Transfer in Higher Plants; in: Transgenic Plants, Vol. 1, Engineering and Utilization, eds.: Kung and R. Wu, Academic Press, 1993, S. 15-38.

"Transformation" is defined herein as a process for introducing heterologous DNA into a plant cell, plant tissue, or plant. It may occur under natural or artificial conditions using various methods well known in the art. Transformation may rely on any known method for the insertion of foreign nucleic acid sequences into a prokaryotic or eukaryotic host cell. The method is selected based on the host cell being transformed and may include, but is not limited to, viral infection, electroporation, lipofection, and particle bombardment. Such "transformed" cells include stably transformed cells in which the inserted DNA is capable of replication either as an autonomously replicating plasmid or as part of the host chromosome. They also include cells, which transiently express the inserted DNA or RNA for limited periods of time. Transformed plant cells, plant tissue, or plants are understood to encompass not only the end product of a transformation process, but also transgenic progeny thereof.

The terms "transformed," "transgenic," and "recombinant" refer to a host organism such as a bacterium or a plant into which a heterologous nucleic acid molecule has been introduced. The nucleic acid molecule can be stably integrated into the genome of the host or the nucleic acid molecule can also be present as an extrachromosomal molecule. Such an extrachromosomal molecule can be auto-replicating. Transformed cells, tissues, or plants are understood to encompass not only the end product of a transformation process, but also transgenic progeny thereof. A "non-transformed," "non-transgenic," or "non-recombinant" host refers to a wild-type organism, e.g., a bacterium or plant, which does not contain the heterologous nucleic acid molecule.

A "transgenic plant", as used herein, refers to a plant, which contains a foreign nucleotide sequence inserted into either its nuclear genome or organelle genome. It encompasses further the offspring generations i.e. the T1-, T2- and consecutively generations or BC1-, BC2- and consecutively generation as well as crossbreeds thereof with non-transgenic or other transgenic plants.

The host organism (= transgenic organism) advantageously contains at least one copy of the nucleic acid according to the invention and/or of the nucleic acid construct according to the invention.

In principle all plants can be used as host organism. Preferred transgenic plants are, for example, selected from the families Aceraceae, Anacardiaceae, Apiaceae, Asteraceae, Brassicaceae, Cactaceae, Cucurbitaceae, Euphorbiaceae, Fabaceae, Malvaceae, Nymphaeaceae, Papaveraceae, Rosaceae, Salicaceae, Solanaceae, Arecaceae, Bromeliaceae, Cyperaceae, Iridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae or Poaceae and preferably from a plant selected from the group of the families Apiaceae, Asteraceae, Brassicaceae, Cucurbitaceae, Fabaceae, Papaveraceae, Rosaceae, Solanaceae, Liliaceae or Poaceae. Preferred are crop plants such as plants advantageously selected from the group of the genus peanut, oilseed rape, canola, sunflower, safflower, olive, sesame, hazelnut, almond, avocado, bay, pumpkin/squash, linseed, soya, pistachio, borage, maize, wheat, rye, oats, sorghum and miller, triticale, rice, barley, cassava, potato, sugarbeet, egg plant, alfalfa, and perennial grasses and forage plants, oil palm, vegetables (brassicas, root vegetables, tuber vegetables, pod vegetables, fruiting vegetables, onion vegetables, leafy vegetables and stem vegetables), buckwheat, Jerusalem artichoke, broad bean, vetches, lentil, dwarf bean, lupin, clover and Lucerne for mentioning only some of them.

In one prefered embodiment, the host plant is selected from the families Aceraceae, Anacardiaceae, Apiaceae, Asteraceae, Brassicaceae, Cactaceae, Cucurbitaceae, Euphorbiaceae, Fabaceae, Malvaceae, Nymphaeaceae, Papaveraceae, Rosaceae, Salicaceae, Solanaceae, Arecaceae, Bromeliaceae, Cyperaceae, Iridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae, or Poaceae, and preferably from a plant selected from the group of the families Apiaceae, Asteraceae, Brassicaceae, Cucurbitaceae, Fabaceae, Papaveraceae, Rosaceae, Solanaceae, Liliaceae or Poaceae. Preferred are crop plants and in particular plants mentioned herein above as host plants, such as the families and genera mentioned above, for example, preferred the species Anacardium occidentale, Calendula officinalis, Carthamus tinctorius, Cichorium intybus, Cynara scolymus, Helianthus annus, Tagetes lucida, Tagetes erecta, Tagetes tenuifolia; Daucus carota; Corylus avellana, Corylus colurna, Borago officinalis; Brassica napus, Brassica rapa ssp., Sinapis arvensis Brassica juncea, Brassica juncea var. juncea, Brassica juncea var. crispifolia, Brassica juncea var. foliosa, Brassica nigra, Brassica sinapioides, Melanosinapis communis, Brassica oleracea, Arabidopsis thaliana, Anana comosus, Ananas ananas, Bromelia comosa, Carica papaya, Cannabis sative, Ipomoea batatus, Ipomoea pandurata, Convolvulus batatas, Convolvulus tiliaceus, Ipomoea fastigiata, Ipomoea tiliacea, Ipomoea triloba, Convolvulus panduratus, Beta vulgaris, Beta vulgaris var. altissima, Beta vulgaris var. vulgaris, Beta maritima, Beta vulgaris var. perennis, Beta vulgaris var. conditiva, Beta vulgaris var. esculenta, Cucurbita maxima, Cucurbita mixta, Cucurbita pepo, Cucurbita moschata, Olea europaea, Manihot utilissima, Janipha manihot, Jatropha manihot, Manihot aipil, Manihot dulcis, Manihot manihot, Manihot melanobasis, Manihot esculenta, Ricinus communis, Pisum sativum, Pisum arvense, Pisum humile, Medicago sativa, Medicago falcata, Medicago varia, Glycine max Dolichos soja, Glycine gracilis, Glycine hispida, Phaseolus max, Soja hispida, Soja max, Cocos nucifera, Pelargonium grossularioides, Oleum cocoas, Laurus nobilis, Persea americana, Arachis hypogaea, Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense, Linum trigynum, Punica granatum, Gossypium hirsutum, Gossypium arboreum, Gossypium barbadense, Gossypium herbaceum, Gossypium thurberi, Musa nana, Musa acuminata, Musa paradisiaca, Musa spp., Elaeis guineensis, Papaver orientale, Papaver rhoeas, Papaver dubium, Sesamum indicum, Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata, Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum, Avena sativa. Avena fatua, Avena byzantina, Avena fatua var. sativa, Avena hybrida, Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum millet, Panicum militaceum, Zea mays, Triticum aestivum, Triticum durum, Triticum turgidum, Triticum hybernum, Triticum macha, Triticum sativum or Triticum vulgare, Cofea spp., Coffea arabica, Coffea canephora, Coffea liberica, Capsicum annuum, Capsicum annuum var. glabriusculum, Capsicum frutescens, Capsicum annuum, Nicotiana tabacum, Solanum tuberosum, Solanum melongena, Lycopersicon esculentum, Lycopersicon lycopersicum, Lycopersicon pyriforme, Solanum integrifolium, Solanum lycopersicum Theobroma cacao or Camellia sinensis.

Anacardiaceae such as the genera Pistacia, Mangifera, Anacardium e.g. the species Pistacia vera (pistachios, Pistazie), Mangifer indica (Mango) or Anacardium occidentale (Cashew); Asteraceae, such as the genera Calendula, Carthamus, Centaurea, Cichorium, Cynara, Helianthus, Lactuca, Locusta, Tagetes, Valeriana, e.g., the species Calendula officinalis (Marigold), Carthamus tinctorius (safflower), Centaurea cyanus (cornflower), Cichorium intybus (blue daisy), Cynara scolymus (Artichoke), Helianthus annus (sunflower), Lactuca sativa, Lactuca crispa, Lactuca esculenta, Lactuca scariola L. ssp. sativa, Lactuca scariola L. var. integrata, Lactuca scariola L. var. integrifolia, Lactuca sativa subsp. romana, Locusta communis, Valeriana locusta (lettuce), Tagetes lucida, Tagetes erecta or Tagetes tenuifolia (Marigold); Apiaceae, such as the genera Daucus, e.g., the species Daucus carota (carrot); Betulaceae, such as the genera Corylus, e.g., the species Corylus avellana or Corylus colurna (hazelnut); Boraginaceae, such as the genera Borago, e.g., the species Borago officinalis (borage); Brassicaceae, such as the genera Brassica, Melanosinapis, Sinapis, Arabadopsis, e.g., the species Brassica napus, Brassica rapa ssp. (canola, oilseed rape, turnip rape), Sinapis arvensis Brassica juncea, Brassica juncea var. juncea, Brassica juncea var. crispifolia, Brassica juncea var. foliosa, Brassica nigra, Brassica sinapioides, Melanosinapis communis (mustard), Brassica oleracea (fodder beet) or Arabidopsis thaliana; Bromeliaceae, such as the genera Anana, Bromelia, e.g., the species Anana comosus, Ananas ananas or Bromelia comosa (pineapple); Caricaceae, such as the genera Carica, e.g., the species Carica papaya (papaya); Cannabaceae, such as the genera Cannabis, e.g., the species Cannabis sative (hemp); Convolvulaceae, such as the genera Ipomea, Convolvulus, e.g., the species Ipomoea batatus, Ipomoea pandurata, Convolvulus batatas, Convolvulus tiliaceus, Ipomoea fastigiata, Ipomoea tiliacea, Ipomoea triloba or Convolvulus panduratus (sweet potato, Man of the Earth, wild potato); Chenopodiaceae, such as the genera Beta, i.e., the species Beta vulgaris, Beta vulgaris var. altissima, Beta vulgaris var. Vulgaris, Beta maritima, Beta vulgaris var. perennis, Beta vulgaris var. conditiva or Beta vulgaris var. esculenta (sugar beet); Cucurbitaceae, such as the genera Cucubita, e.g., the species Cucurbita maxima, Cucurbita mixta, Cucurbita pepo or Cucurbita moschata (pumpkin, squash); Elaeagnaceae, such as the genera Elaeagnus, e.g., the species Olea europaea (olive); Ericaceae such as the genera Kalmia e.g. the species Kalmia latifolia, Kalmia angustifolia, Kalmia microphylla, Kalmia polifolia, Kalmia occidentalis, Cistus chamaerhodendros or Kalmia lucida (American laurel, broadleafed laurel, calico bush, spoon wood, sheep laurel, alpine laurel, bog laurel, western bog-laurel, swamp-laurel); Euphorbiaceae, such as the genera Manihot, Janipha, Jatropha, Ricinus, e.g., the species Manihot utilissima, Janipha manihot, Jatropha manihot, Manihot aipil, Manihot dulcis, Manihot manihot, Manihot melanobasis, Manihot esculenta (manihot, arrowroot, tapioca, cassava) or Ricinus communis (castor bean, Castor Oil Bush, Castor Oil Plant, Palma Christi, Wonder Tree); Fabaceae, such as the genera Pisum, Albizia, Cathormion, Feuillea, Inga, Pithecolobium, Acacia, Mimosa, Medicajo, Glycine, Dolichos, Phaseolus, Soja, e.g., the species Pisum sativum, Pisum arvense, Pisum humile (pea), Albizia berteriana, Albizia julibrissin, Albizia lebbeck, Acacia berteriana, Acacia littoralis, Albizia berteriana, Albizzia berteriana, Cathormion berteriana, Feuillea berteriana, Inga fragrans, Pithecellobium berterianum, Pithecellobium fragrans, Pithecolobium berterianum, Pseudalbizzia berteriana, Acacia julibrissin, Acacia nemu, Albizia nemu, Feuilleea julibrissin, Mimosa julibrissin, Mimosa speciosa, Sericanrda julibrissin, Acacia lebbeck, Acacia macrophylla, Albizia lebbek, Feuilleea lebbeck, Mimosa lebbeck, Mimosa speciosa (bastard logwood, silk tree, East Indian Walnut), Medicago sativa, Medicago falcata, Medicago varia (alfalfa) Glycine max Dolichos soja, Glycine gracilis, Glycine hispida, Phaseolus max, Soja hispida or Soja max (soybean); Geraniaceae, such as the genera Pelargonium, Cocos, Oleum, e.g., the species Cocos nucifera, Pelargonium grossularioides or Oleum cocois (coconut); Gramineae, such as the genera Saccharum, e.g., the species Saccharum officinarum; Juglandaceae, such as the genera Juglans, Wallia, e.g., the species Juglans regia, Juglans ailanthifolia, Juglans sieboldiana, Juglans cinerea, Wallia cinerea, Juglans bixbyi, Juglans californica, Juglans hindsii, Juglans intermedia, Juglans jamaicensis, Juglans major, Juglans microcarpa, Juglans nigra or Wallia nigra (walnut, black walnut, common walnut, persian walnut, white walnut, butternut, black walnut); Lauraceae, such as the genera Persea, Laurus, e.g., the species laurel Laurus nobilis (bay, laurel, bay laurel, sweet bay), Persea americana Persea americana, Persea gratissima or Persea persea (avocado); Leguminosae, such as the genera Arachis e.g. the species Arachis hypogaea (peanut); Linaceae, such as the genera Linum, Adenolinum, e.g., the species Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pretense, or Linum trigynum (flax, linseed); Lythrarieae, such as the genera Punica, e.g., the species Punica granatum (pomegranate); Malvaceae, such as the genera Gossypium, e.g., the species Gossypium hirsutum, Gossypium arboreum, Gossypium barbadense, Gossypium herbaceum or Gossypium thurberi (cotton); Musaceae, such as the genera Musa, e.g., the species Musa nana, Musa acuminata, Musa paradisiaca, Musa spp. (banana); Onagraceae, such as the genera Camissonia, Oenothera, e.g., the species Oenothera biennis or Camissonia brevipes (primrose, evening primrose); Palmae, such as the genera Elacis, e.g., the species Elaeis guineensis (oil palm); Papaveraceae, such as the genera Papaver, e.g., the species Papaver orientale, Papaver rhoeas, Papaver dubium (poppy, oriental poppy, corn poppy, field poppy, shirley poppies, field poppy, long-headed poppy, long-pod poppy); Pedaliaceae, such as the genera Sesamum, e.g., the species Sesamum indicum (sesame); Piperaceae, such as the genera Piper, Artanthe, Peperomia, Steffensia, e.g., the species Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata (Cayenne pepper, wild pepper); Poaceae, such as the genera Hordeum, Secale, Avena, Sorghum, Andropogon, Holcus, Panicum, Oryza, Zea, Triticum, e.g. ,the species Hordeum vulgare, Hordeum jubatum, Hordeum murinum, Hordeum secalinum, Hordeum distichon, Hordeum aegiceras, Hordeum hexastichon, Hordeum hexastichum, Hordeum irregulare, Hordeum sativum, Hordeum secalinum (barley, pearl barley, foxtail barley, wall barley, meadow barley), Secale cereale (rye), Avena sativa, Avena fatua, Avena byzantina, Avena fatua var. sativa, Avena hybrida (oat), Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum millet, Panicum militaceum (Sorghum, millet), Oryza sativa, Oryza latifolia (rice), Zea mays (corn, maize) Triticum aestivum, Triticum durum, Triticum turgidum, Triticum hybernum, Triticum macha, Triticum sativum or Triticum vulgare (wheat, bread wheat, common wheat), Proteaceae, such as the genera Macadamia, e.g., the species Macadamia intergrifolia (macadamia); Rubiaceae, such as the genera Coffea, e.g., the species Cofea spp., Coffea arabica, Coffea canephora or Coffea liberica (coffee); Scrophulariaceae, such as the genera Verbascum, e.g., the species Verbascum blattaria, Verbascum chaixii, Verbascum densiflorum, Verbascum lagurus, Verbascum longifolium, Verbascum lychnitis, Verbascum nigrum, Verbascum olympicum, Verbascum phlomoides, Verbascum phoenicum, Verbascum pulverulentum, or Verbascum thapsus (mullein, white moth mullein, nettle-leaved mullein, dense-flowered mullein, silver mullein, long-leaved mullein, white mullein, dark mullein, greek mullein, orange mullein, purple mullein, hoary mullein, great mullein); Solanaceae, such as the genera Capsicum, Nicotiana, Solanum, Lycopersicon, e.g., the species Capsicum annuum, Capsicum annuum var. glabriusculum, Capsicum frutescens (pepper), Capsicum annuum (paprika), Nicotiana tabacum, Nicotiana alata, Nicotiana attenuata, Nicotiana glauca, Nicotiana langsdorffii, Nicotiana obtusifolia, Nicotiana quadrivalvis, Nicotiana repanda, Nicotiana rustica, Nicotiana sylvestris (tobacco), Solanum tuberosum (potato), Solanum melongena (egg-plant) (Lycopersicon esculentum, Lycopersicon lycopersicum, Lycopersicon pyriforme, Solanum integrifolium, or Solanum lycopersicum (tomato); Sterculiaceae, such as the genera Theobroma, e.g., the species Theobroma cacao (cacao); Theaceae, such as the genera Camellia, e.g., the species Camellia sinensis) (tea). The introduction of the nucleic acids according to the invention, the expression cassette, or the vector into organisms, plants for example, can in principle be done by all of the methods known to those skilled in the art. The introduction of the nucleic acid sequences gives rise to recombinant or transgenic organisms.

In the case of microorganisms, those skilled in the art can find appropriate methods in the textbooks by Sambrook, J. et al. (1989) Molecular cloning: A laboratory manual, Cold Spring Harbor Laboratory Press, by F.M. Ausubel et al. (1994) Current protocols in molecular biology, John Wiley and Sons, by D.M. Glover et al., DNA Cloning Vol.1, (1995), IRL Press (ISBN 019-963476-9), by Kaiser et al. (1994) Methods in Yeast Genetics, Cold Spring Harbor Laboratory Press or Guthrie et al. Guide to Yeast Genetics and Molecular Biology, Methods in Enzymology, 1994, Academic Press.

The transfer of foreign genes into the genome of a plant is called transformation. In doing this the methods described for the transformation and regeneration of plants from plant tissues or plant cells are utilized for transient or stable transformation. Suitable methods are protoplast transformation by poly(ethylene glycol)-induced DNA uptake, the "biolistic" method using the gene cannon - referred to as the particle bombardment method, electroporation, the incubation of dry embryos in DNA solution, microinjection and gene transfer mediated by Agrobacterium. Said methods are described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming Agrobacterium tumefaciens, for example pBin19 (Bevan et al., Nucl. Acids Res. 12 (1984) 8711). Agrobacteria transformed by such a vector can then be used in known manner for the transformation of plants, in particular of crop plants such as by way of example tobacco plants, for example by bathing bruised leaves or chopped leaves in an agrobacterial solution and then culturing them in suitable media. The transformation of plants by means of Agrobacterium tumefaciens is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds., S.D. Kung and R. Wu, Academic Press, 1993, pp. 15-38.

Agrobacteria transformed by an expression vector according to the invention may likewise be used in known manner for the transformation of plants such as test plants like Arabidopsis or crop plants such as cereal crops, corn, oats, rye, barley, wheat, soybean, rice, cotton, sugar beet, canola, sunflower, flax, hemp, potatoes, tobacco, tomatoes, carrots, paprika, oilseed rape, tapioca, cassava, arrowroot, tagetes, alfalfa, lettuce and the various tree, nut and vine species, in particular of oil-containing crop plants such as soybean, peanut, castor oil plant, sunflower, corn, cotton, flax, oilseed rape, coconut, oil palm, safflower (Carthamus tinctorius) or cocoa bean, e.g. by bathing bruised leaves or chopped leaves in an agrobacterial solution and then culturing them in suitable media.

The genetically modified plant cells may be regenerated by all of the methods known to those skilled in the art. Appropriate methods can be found in the publications referred to above by S.D. Kung and R. Wu, Potrykus or Höfgen and Willmitzer.

Accordingly, a further aspect of the invention relates to transgenic organisms transformed by at least one nucleic acid sequence, expression cassette or vector according to the invention as well as cells, cell cultures, tissue, parts - such as, for example, leaves, roots, etc. in the case of plant organisms - or reproductive material derived from such organisms. The terms "host organism," "host cell," "recombinant (host) organism," and "transgenic (host) cell" are used here interchangeably. Of course, these terms relate not only to the particular host organism or the particular target cell, but also to the descendants or potential descendants of these organisms or cells. Since, due to mutation or environmental effects, certain modifications may arise in successive generations, these descendants need not necessarily be identical with the parental cell but, nevertheless, are still encompassed by the term as used here.

For the purposes of the invention "transgenic" or "recombinant" means with regard for example to a nucleic acid sequence, an expression cassette (= gene construct, nucleic acid construct) or a vector containing the nucleic acid sequence according to the invention or an organism transformed by the nucleic acid sequences, expression cassette, or vector according to the invention all those constructions produced by genetic engineering methods in which either: a) the nucleic acid sequence as disclosed in the Sequence Listing or its derivatives or parts thereof; or b) a genetic control sequence functionally linked to the nucleic acid sequence described under (a), for example a 3'- and/or 5'- genetic control sequence such as a promoter or terminator, or c) (a) and (b), are not found in their natural, genetic environment or have been modified by genetic engineering methods, wherein the modification may by way of example be a substitution, addition, deletion, inversion or insertion of one or more nucleotide residues. Natural genetic environment means the natural genomic or chromosomal locus in the organism of origin or inside the host organism or presence in a genomic library. In the case of a genomic library the natural genetic environment of the nucleic acid sequence is preferably retained at least in part. The environment borders the nucleic acid sequence at least on one side and has a sequence length of at least 50 bp, preferably at least 500 bp, particularly preferably at least 1,000 bp, most particularly preferably at least 5,000 bp. A naturally occurring expression cassette - for example, the naturally occurring combination of the natural promoter of the nucleic acid sequence according to the invention with the corresponding Δ-8-desaturase, Δ-9-elongase and/or Δ-5-desaturase gene - turns into a transgenic expression cassette when the latter is modified by unnatural, synthetic ("artificial') methods, such as, by way of example, a mutagenation. Appropriate methods are described by way of example in US 5,565,350 or WO 00/15815.

Suitable organisms or host organisms for the nucleic acid, expression cassette, or vector according to the invention are advantageously in principle all organisms, which are suitable for the expression of recombinant genes as described above. Further examples which may be mentioned are plants such as Arabidopsis, Asteraceae such as Calendula, or crop plants such as soybean, peanut, castor oil plant, sunflower, flax, corn, cotton, flax, oilseed rape, coconut, oil palm, safflower (Carthamus tinctorius) or cocoa bean.

A further object of the invention relates to the use of a nucleic acid construct, e.g. an expression cassette, containing DNA sequences encoding polypeptides disclosed in the Sequence Listing or DNA sequences hybridizing therewith for the transformation of plant cells, tissues or parts of plants.

In doing so, depending on the choice of promoter, the sequences as disclosed in the Sequence Listing can be expressed specifically in the leaves, in the seeds, the nodules, in roots, in the stem or other parts of the plant. Those transgenic plants overproducing sequences disclosed in the Sequence Listing, the reproductive material thereof, together with the plant cells, tissues, or parts thereof are a further object of the present invention.

The expression cassette or the nucleic acid sequences or construct according to the invention containing sequences as disclosed in the Sequence Listing can, moreover, also be employed for the transformation of the organisms identified by way of example above such as bacteria, yeasts, filamentous fungi and plants.

Within the framework of the present invention, altering metabolic activity means, for example, the artificially acquired trait of increased biosynthetic performance due to functional over expression of sequences disclosed in the Sequence Listing in the organisms according to the invention, advantageously in the transgenic plants according to the invention, by comparison with the nongenetically modified initial plants at least for the duration of at least one plant generation.

A constitutive expression of the exogenous sequences disclosed in the Sequence Listing is, moreover, advantageous. On the other hand, however, an inducible expression may also appear desirable.

The efficiency of the expression of the sequences disclosed in the Sequence Listing can be determined, for example, in vitro by shoot meristem propagation. In addition, an expression of the sequences disclosed in the Sequence Listing modified in nature and level and its effect on the metabolic pathways performance can be tested on test plants in greenhouse trials.

An additional object of the invention comprises transgenic organisms such as transgenic plants transformed by an expression cassette containing sequences disclosed in the Sequence Listing according to the invention or DNA sequences hybridizing therewith, as well as transgenic cells, tissue, parts and reproduction material of such plants. Particular preference is given in this case to transgenic crop plants, such as by way of example barley, wheat, rye, oats, corn, soybean, rice, cotton, sugar beet, oilseed rape and canola, sunflower, flax, hemp, thistle, potatoes, tobacco, tomatoes, tapioca, cassava, arrowroot, alfalfa, lettuce and the various tree, nut, and vine species.

For the purposes of the invention, plants are mono- and dicotyledonous plants, mosses or algae.

A further refinement, according to the invention, is transgenic plants, as described above, which contain a nucleic acid sequence or construct according to the invention or an expression cassette according to the invention.

Furthermore, by derivatives is meant homologues of the sequences disclosed in the Sequence Listing, for example eukaryotic homologues, truncated sequences, single-stranded DNA of the encoding and nonencoding DNA sequence or RNA of the encoding and nonencoding DNA sequence.

In addition, by homologues of the sequences disclosed in the Sequence Listing is meant derivatives such as by way of example promoter variants. These variants may be modified by one or more nucleotide exchanges, by insertion(s) and/or deletion(s) without, however, adversely affecting the functionality or efficiency of the promoters. Furthermore, the promoters can have their efficiency increased by altering their sequence or be completely replaced by more effective promoters even of foreign organisms.

By derivatives is also advantageously meant variants whose nucleotide sequence has been altered in the region from -1 to -2000 ahead of the start codon in such a way that the gene expression and/or the protein expression is modified, preferably increased. Furthermore, by derivatives is also meant variants, which have been modified at the 3' end.

Suitable promoters in the expression cassette are in principle all promoters which can control the expression of foreign genes in organisms such as microorganisms like protozoa such as ciliates, algae such as green, brown, red or blue algae such as Euglenia, bacteria such as gram-positive or gram-negative bacteria, yeasts such as Saccharomyces, Pichia or Schizosaccharomyces or fungi such as Mortierella, Thraustochytrium or Schizochytrium or plants, advantageously in plants or fungi. Use is preferably made in particular of plant promoters or promoters derived from a plant virus. Advantageous regulation sequences for the method according to the invention are found for example in promoters, such as cos, tac, trp, tet, trp-tet, lpp, lac, lpp-lac, laclq-, T7, T5, T3, gal, trc, ara, SP6, λ-PR or in λ-PL promoters which are employed advantageously in gram-negative bacteria. Other advantageous regulation sequences are found, for example, in the gram-positive promoters amy and SPO2, in the yeast or fungal promoters ADC1, MFα, AC, P-60, CYC1, GAPDH, TEF, rp28, ADH or in the plant promoters CaMV/35S [Franck et al., Cell 21(1980) 285-294], SSU, OCS, lib4, STLS1, B33, nos (= Nopalin Synthase Promoter) or in the ubiquintin or phaseolin promoter. The expression cassette may also contain a chemically inducible promoter by means of which the expression of the sequences disclosed in the Sequence Listing transformed into the organisms can be controlled advantageously in the plants at a particular time. Advantageous plant promoters of this type are by way of example the PRP1 promoter (Ward et al., Plant.Mol. Biol.22(1993), 361-366), a promoter inducible by benzenesulfonamide (EP 388186), a promoter inducible by tetracycline [Gatz et al., (1992) Plant J. 2,397-404], a promoter inducible by salicylic acid (WO 95/19443), a promoter inducible by abscisic acid (EP 335528) and a promoter inducible by ethanol or cyclohexanone (WO93/21334). Other examples of plant promoters, which can advantageously be used, are the promoter of cytosolic FBPase from potato, the ST-LSI promoter from potato (Stockhaus et al., EMBO J. 8 (1989) 2445-245), the promoter of phosphoribosyl pyrophosphate amidotransferase from Glycine max (see also gene bank accession number U87999) or a nodiene-specific promoter as described in EP 249676. Particularly advantageous are those plant promoters, which ensure expression in tissues, or plant parts/organs, in which fatty acid biosynthesis or the precursor stages thereof occurs, as in endosperm or in the developing embryo for example. Particularly noteworthy are advantageous promoters, which ensure seed-specific expression, such as, by way of example, the USP promoter or derivatives thereof, the LEB4 promoter, the phaseolin promoter, or the napin promoter. The particularly advantageous USP promoter cited according to the invention or its derivatives mediate very early gene expression in seed development (Baeumlein et al., Mol Gen Genet, 1991, 225 (3): 459-67). Other advantageous seed-specific promoters which may be used for monocotylodonous or dicotylodonous plants are the promoters suitable for dicots such as napin gene promoters, likewise cited by way of example, from oilseed rape (US 5,608,152), the oleosin promoter from Arabidopsis (WO 98/45461), the phaseolin promoter from Phaseolus vulgaris (US 5,504,200), the Bce4 promoter from Brassica (WO 91/13980) or the leguminous B4 promoter (LeB4, Baeumlein et al., Plant J., 2, 2, 1992: 233 - 239) or promoters suitable for monocotylodons such as the promoters of the lpt2 or lpt1 gene in barley (WO 95/15389 and WO 95/23230) or the promoters of the barley hordeine gene, the rice glutelin gene, the rice oryzin gene, the rice prolamin gene, the wheat gliadin gene, the white glutelin gene, the corn zein gene, the oats glutelin gene, the sorghum kasirin gene or the rye secalin gene which are described in WO99/16890.

Furthermore, particularly preferred are those promoters, which ensure the expression in tissues, or plant parts in which, for example, the biosynthesis of fatty acids, oils and lipids or the precursor stages thereof takes place. Particularly noteworthy are promoters, which ensure a seed-specific expression. Noteworthy are the promoter of the napin gene from oilseed rape (US 5,608,152), the USP promoter from Vicia faba (USP = unknown seed protein, Baeumlein et al., Mol Gen Genet, 1991, 225 (3): 459-67), the promoter of the oleosin gene from Arabidopsis (WO98/45461), the phaseolin promoter (US 5,504,200) or the promoter of the legumin B4 gene (LeB4; Baeumlein et al., 1992, Plant Journal, 2 (2): 233-9). Other promoters to be mentioned are that of the lpt2 or lpt1 gene from barley (WO95/15389 and WO95/23230), which mediate seed-specific expression in monocotyledonous plants. Other advantageous seed specific promoters are promoters such as the promoters from rice, corn or wheat disclosed in WO 99/16890 or Amy32b, Amy6-6 or aleurain (US 5,677,474), Bce4 (rape, US 5,530,149), glycinin (soy bean, EP 571741), phosphoenol pyruvat carboxylase (soy bean, JP 06/62870), ADR12-2 (soy bean, WO 98/08962), isocitratlyase (rape, US 5,689,040) or β-amylase (barley, EP 781 849).

As described above, the expression construct (= gene construct, nucleic acid construct) may contain yet other genes, which are to be introduced into the organisms. These genes can be subject to separate regulation or be subject to the same regulation region as sequences disclosed in the Sequence Listing. These genes are by way of example other biosynthesis genes, advantageously for fatty acid biosynthesis, vitamin biosynthesis etc. that allow increased synthesis.

In principle all natural promoters with their regulation sequences can be used like those named above for the expression cassette according to the invention and the method according to the invention. Over and above this, synthetic promoters may also advantageously be used.

In the preparation of an expression cassette various DNA fragments can be manipulated in order to obtain a nucleotide sequence, which usefully reads in the correct direction and is equipped with a correct reading raster. To connect the DNA fragments (=nucleic acids according to the invention) to one another adaptors or linkers may be attached to the fragments.

The promoter and the terminator regions can usefully be provided in the transcription direction with a linker or polylinker containing one or more restriction points for the insertion of this sequence. Generally, the linker has 1 to 10, mostly 1 to 8, preferably 2 to 6, restriction points. In general the size of the linker inside the regulatory region is less than 100 bp, frequently less than 60 bp, but at least 5 bp. The promoter may be both native or homologous, as well as foreign or heterologous to the host organism, for example to the host plant. In the 5'-3' transcription direction the expression cassette contains the promoter, a DNA sequence which encodes a nucleic acid disclosed herein and a region for transcription termination. Different termination regions can be exchanged for one another in any desired fashion.

Furthermore, manipulations which provide suitable restriction interfaces, or which remove excess DNA or restriction interfaces can be employed. Where insertions, deletions, or substitutions, such as transitions and transversions, come into consideration, in vitro mutagenesis, primer repair, restriction or ligation may be used. In suitable manipulations such as restriction, chewing back or filling of overhangs for blunt ends complementary ends of the fragments can be provided for the ligation.

For an advantageous high expression the attachment of the specific ER retention signal SEKDEL (SEQ ID NO: 532) inter alia can be of importance (Schouten, A. et al., Plant Mol. Biol. 30 (1996), 781-792). In this way the average expression level is tripled or even quadrupled. Other retention signals, which occur naturally in plant and animal proteins located in the ER may also be employed for the construction of the cassette. In another preferred embodiment a plastidial targeting sequence is used as described by Napier J.A. (Targeting of foreign proteins to the chloroplast, Methods Mol. Biol., 49, 1995: 369 - 376). A preferred used vector comprising said plastidial targeting sequence is disclosed by Colin Lazarus [Guerineau F., Woolston S., Brooks L., Mullineaux P. "An expression cassette for targeting foreign proteins into chloroplast; Nucleic. Acids Res., Dec 9, 16 (23), 1988: 11380].

Preferred polyadenylation signals are plant polyadenylation signals, preferably those which substantially correspond to T-DNA polyadenylation signals from Agrobacterium tumefaciens, in particular gene 3 of the T-DNA (octopin synthase) of the Ti plasmid pTiACH5 (Gielen et al., EMBO J.3 (1984), 835 et seq.) or corresponding functional equivalents.

An expression cassette is produced by fusion of a suitable promoter with suitable sequences disclosed in the Sequence Listing together with a polyadenylation signal by common recombination and cloning techniques as described, for example, in T. Maniatis, E.F. Fritsch and J. Sambrook, Molecular Cloning: A Laboratory Manual, Cold Spring Harbor Laboratory, Cold Spring Harbor, NY (1989) as well as in T.J. Silhavy, M.L. Berman and L.W. Enquist, Experiments with Gene Fusions, Cold Spring Harbor Laboratory, Cold Spring Harbor, NY (1984) and in Ausubel, F.M. et al., Current Protocols in Molecular Biology, Greene Publishing Assoc. and Wiley-Interscience (1987).

In the preparation of an expression cassette various DNA fragments can be manipulated to produce a nucleotide sequence, which usefully reads in the correct direction and is equipped with a correct reading raster. Adapters or linkers can be attached to the fragments for joining the DNA fragments.

The promoter and the terminator regions can usefully be provided in the transcription direction with a linker or polylinker containing one or more restriction points for the insertion of this sequence. Generally, the linker has 1 to 10, mostly 1 to 8, preferably 2 to 6, restriction points. In general the size of the linker inside the regulatory region is less than 100 bp, frequently less than 60 bp, but at least 5 bp. The promoter may be both native or homologous as well as foreign or heterologous to the host organism, for example to the host plant. In the 5'-3' transcription direction the expression cassette contains the promoter, a DNA sequence, which either encodes a nucleic acid disclosed in the Sequence Listing and a region for transcription termination. Different termination regions can be exchanged for one another in any desired fashion.

In the preparation of an expression cassette various DNA fragments can be manipulated to produce a nucleotide sequence, which usefully reads in the correct direction and is equipped with a correct reading raster. Adapters or linkers can be attached to the fragments for joining the DNA fragments.

The DNA sequences encoding the nucleic acid sequences used in the inventive processes such as the sequences disclosed in the Sequence Listing contain all the sequence characteristics needed to achieve correct localization of respective biosynthesis. Accordingly, no further targeting sequences are needed per se. However, such localization may be desirable and advantageous and hence artificially modified or reinforced so that such fusion constructs are also a preferred advantageous embodiment of the invention.

Particularly preferred are sequences, which ensure targeting in plastids. Under certain circumstances targeting into other compartments (reported in: Kermode, Crit. Rev. Plant Sci. 15, 4 (1996), 285-423) may also be desirable, e.g. into vacuoles, the mitochondria, the endoplasmic reticulum (ER), peroxisomes, lipid structures or due to lack of corresponding operative sequences retention in the compartment of origin, the cytosol.

As used herein, the term "environmental stress" refers to any sub-optimal growing condition and includes, but is not limited to, sub-optimal conditions associated with salinity, drought, temperature, metal, chemical, pathogenic and oxidative stresses, or combinations thereof. In preferred embodiments, the environmental stress can be salinity, drought, heat, or low temperature, or combinations thereof, and in particular, can be low water content or low temperature. Wherein drought stress means any environmental stress which leads to a lack of water in plants or reduction of water supply to plants, wherein low temperature stress means freezing of plants below + 4°C as well as chilling of plants below 15°C and
wherein high temperature stress means for example a temperature above 35°C. The range of stress and stress response depends on the different plants, which are used for the invention, i.e. it differs for example between a plant such as wheat and a plant such as Arabidopsis. A common response of plants to environmental stress is the loss of yield or the loss of quality. It is also to be understood that as used in the specification and in the claims, "a" or "an" can mean one or more, depending upon the context in which it is used. Thus, for example, reference to "a cell" can mean that at least one cell can be utilized.

As also used herein, the terms "nucleic acid" and "nucleic acid molecule" are intended to include DNA molecules (e.g., cDNA or genomic DNA) and RNA molecules (e.g., mRNA) and analogs of the DNA or RNA generated using nucleotide analogs. This term also encompasses untranslated sequence located at both the 3' and 5' ends of the coding region of the gene: at least about 1000 nucleotides of sequence upstream from the 5' end of the coding region and at least about 200 nucleotides of sequence downstream from the 3' end of the coding region of the gene. The nucleic acid molecule can be single stranded or double stranded, but preferably is double-stranded DNA.

An "isolated" nucleic acid molecule is one that is substantially separated from other nucleic acid molecules, which are present in the natural source of the nucleic acid. That means other nucleic acid molecules are present in an amount less than 5% based on weight of the amount of the desired nucleic acid, preferably less than 2% by weight, more preferably less than 1% by weight, and most preferably less than 0.5% by weight. Preferably, an "isolated" nucleic acid is free of some of the sequences that naturally flank the nucleic acid (i.e., sequences located at the 5' and 3' ends of the nucleic acid) in the genomic DNA of the organism, from which the nucleic acid is derived. For example, in various embodiments, the isolated stress related protein encoding nucleic acid molecule can contain less than about 5 kb, 4 kb, 3 kb, 2 kb, 1 kb, 0.5 kb, or 0.1 kb of nucleotide sequences, which naturally flank the nucleic acid molecule in genomic DNA of the cell from which the nucleic acid is derived. Moreover, an "isolated" nucleic acid molecule, such as a cDNA molecule, can be free from some of the other cellular material with which it is naturally associated, or culture medium when produced by recombinant techniques, or chemical precursors or other chemicals when chemically synthesized.

A nucleic acid molecule of the present invention, e.g., a nucleic acid molecule encoding an SRP or a portion thereof, which confers tolerance and/or resistance to environmental stress in plants, can be isolated using standard molecular biological techniques and the sequence information provided herein. For example, an Arabidopsis thaliana stress related protein-encoding cDNA can be isolated from a A. thaliana c-DNA library using all or portion of one of the sequences disclosed in the Sequence Listing. Moreover, a nucleic acid molecule encompassing all or a portion of one of the sequences disclosed in the Sequence Listing can be isolated by the polymerase chain reaction using oligonucleotide primers designed based upon this sequence. For example, mRNA can be isolated from plant cells (e.g., by the guanidinium-thiocyanate extraction procedure of Chirgwin et al., 1979 Biochemistry 18:5294-5299) and cDNA can be prepared using reverse transcriptase (e.g., Moloney MLV reverse transcriptase, available from Gibco/BRL, Bethesda, MD; or AMV reverse transcriptase, available from Seikagaku America, Inc., St. Petersburg, FL). Synthetic oligonucleotide primers for polymerase chain reaction amplification can be designed based upon one of the nucleotide sequences disclosed in the Sequence Listing. A nucleic acid molecule of the invention can be amplified using cDNA or, alternatively, genomic DNA, as a template and appropriate oligonucleotide primers according to standard PCR amplification techniques. The nucleic acid molecule, so amplified, can be cloned into an appropriate vector and characterized by DNA sequence analysis. Furthermore, oligonucleotides corresponding to a SRP encoding nucleotide sequence can be prepared by standard synthetic techniques, e.g., using an automated DNA synthesizer.

In a preferred embodiment, an isolated nucleic acid molecule of the invention comprises one of the nucleotide sequences disclosed in the Sequence Listing encoding the SRP (i.e., the "coding region'), as well as 5'untranslated sequences and 3' untranslated sequences.

Moreover, the nucleic acid molecule of the invention can comprise only a portion of the coding region of one of the sequences disclosed in the Sequence Listing, for example, a fragment, which can be used as a probe or primer or a fragment encoding a biologically active portion of an SRP.

Portions of proteins encoded by the SRP encoding nucleic acid molecules of the invention are preferably biologically active portions described herein. As used herein, the term "biologically-active portion of" a SRP is intended to include a portion, e.g., a domain/motif, of stress related protein that participates in a stress tolerance and/or resistance response in a plant. To determine whether a SRP, or a biologically active portion thereof, results in increased stress tolerance in a plant, a stress analysis of a plant comprising the SRP may be performed. Such analysis methods are well known to those skilled in the art, as detailed in the Examples. More specifically, nucleic acid fragments encoding biologically active portions of a SRP can be prepared by isolating a portion of one of the sequences disclosed in the Sequence Listing expressing the encoded portion of the SRP or peptide (e.g., by recombinant expression in vitro) and assessing the activity of the encoded portion of the SRP or peptide.

Biologically active portions of a SRP are encompassed by the present invention and include peptides comprising amino acid sequences derived from the amino acid sequence of a SRP encoding gene, or the amino acid sequence of a protein homologous to a SRP, which include fewer amino acids than a full length SRP or the full length protein, which is homologous to a SRP, and exhibits at least some enzymatic activity of a SRP. Typically, biologically active portions (e.g., peptides which are, for example, 5, 10, 15, 20, 30, 35, 36, 37, 38, 39, 40, 50, 100, or more amino acids in length) comprise a domain or motif with at least one activity of a SRP. Moreover, other biologically active portions in which other regions of the protein are deleted, can be prepared by recombinant techniques, and evaluated for one or more of the activities described herein. Preferably, the biologically active portions of a SRP include one or more selected domains/motifs or portions thereof having biological activity.

The term "biological active portion" or "biological activity" means a SRP or a portion of a SRP which still has at least 10 % or 20 %, preferably 20 %, 30 %, 40 %, or 50 %, especially preferably 60 %, 70 %, or 80 % of the enzymatic activity of the natural or starting enzyme.

A nucleic acid molecule encompassing a complete sequence of the nucleic acid molecules used in the process, for example the polynucleotide of the invention, or a part thereof may additionally be isolated by polymerase chain reaction, oligonucleotide primers based on this sequence or on parts thereof being used. For example, a nucleic acid molecule comprising the complete sequence or part thereof can be isolated by polymerase chain reaction using oligonucleotide primers, which have been generated on the basis of this sequence. For example, mRNA can be isolated from cells (for example by means of the guanidinium thiocyanate extraction method of Chirgwin et al. (1979) Biochemistry 18:5294-5299) and cDNA can be generated by means of reverse transcriptase (for example Moloney MLV reverse transcriptase, available from Gibco/BRL, Bethesda, MD, or AMV reverse transcriptase, obtainable from Seikagaku America, Inc., St.Petersburg, FL).

Synthetic oligonucleotide primers for the amplification, e.g. as shown in Table 2, by means of polymerase chain reaction can be generated on the basis of a sequence disclosed in the Sequence Listing or the sequences derived from polypeptides as disclosed in the Sequence Listing.

Moreover, it is possible to identify conserved regions from various organisms by carrying out protein sequence alignments with the polypeptide used in the process of the invention, in particular with sequences of the polypeptide of the invention, from which conserved regions, and in turn, degenerate primers can be derived.

Degenerated primers can then be utilized by PCR for the amplification of fragments of novel proteins having above-mentioned activity, e.g. having an SPR activity or further functional homologs of the polypeptide of the invention from other organisms.

These fragments can then be utilized as hybridization probe for isolating the complete gene sequence. As an alternative, the missing 5' and 3' sequences can be isolated by means of RACE-PCR (rapid amplification of cDNA ends). A nucleic acid molecule according to the invention can be amplified using cDNA or, as an alternative, genomic DNA as template and suitable oligonucleotide primers, following standard PCR amplification techniques. The nucleic acid molecule amplified thus can be cloned into a suitable vector and characterized by means of DNA sequence analysis. Oligonucleotides, which correspond to one of the nucleic acid molecules used in the process can be generated by standard synthesis methods, for example using an automatic DNA synthesizer.

Nucleic acid molecules which are advantageously for the process according to the invention can be isolated based on their homology to the nucleic acid molecules disclosed herein using the sequences or part thereof as hybridization probe and following standard hybridization techniques under stringent hybridization conditions. In this context, it is possible to use, for example, isolated nucleic acid molecules of at least 15, 20, 25, 30, 35, 40, 50, 60, or more nucleotides, preferably of at least 15, 20, or 25 nucleotides in length, which hybridize under stringent conditions with the above-described nucleic acid molecules, in particular with those which encompass a nucleotide sequence of the present invention, a nucleic acid molecule used in the present invention or in a process of the invention, or encoding a protein used in the invention or of the nucleic acid molecule of the invention. Nucleic acid molecules with 30, 50, 100, 250, or more nucleotides may also be used.

In addition to fragments of the SRP described herein, the present invention includes homologs and analogs of naturally occurring SRP and SRP encoding nucleic acids in a plant.

"Homologs" are defined herein as two nucleic acids or proteins that have similar, or "homologous," nucleotide or amino acid sequences, respectively. Homologs include allelic variants, orthologs, paralogs, agonists, and antagonists of SRP, as defined hereafter. The term "homolog" further encompasses nucleic acid molecules that differ from one of the nucleotide sequences disclosed in the Sequence Listing (and portions thereof) due to degeneracy of the genetic code and thus encode the same SRP as that encoded by the amino acid sequences disclosed in the Sequence Listing. As used herein a "naturally-occurring" SRP refers to a SRP amino acid sequence that occurs in nature.

The term "homology" means that the respective nucleic acid molecules or encoded proteins are functionally and/or structurally equivalent. The nucleic acid molecules that are homologous to the nucleic acid molecules described above and that are derivatives of said nucleic acid molecules are, for example, variations of said nucleic acid molecules which represent modifications having the same biological function, in particular encoding proteins with the same or substantially the same biological function. They may be naturally occurring variations, such as sequences from other plant varieties or species, or mutations. These mutations may occur naturally or may be obtained by mutagenesis techniques. The allelic variations may be naturally occurring allelic variants as well as synthetically produced or genetically engineered variants. Structural equivalents can, for example, be identified by testing the binding of said polypeptide to antibodies or computer-based predictions. Structurally equivalent has the similar immunological characteristic, e.g. comprise similar epitopes.

Functional equivalents derived from one of the polypeptides disclosed in the Sequence Listing according to the invention by substitution, insertion or deletion have at least 30%, 35%, 40%, 45%, or 50%, preferably at least 55%, 60%, 65%, or 70% by preference at least 80%, especially preferably at least 85% or 90%, 91 %, 92%, 93%, or 94%, very especially preferably at least 95%, 97%, 98%, or 99% homology with one of the polypeptides disclosed in the Sequence Listing according to the invention and are distinguished by essentially the same properties as a polypeptide disclosed in the Sequence Listing.

Functional equivalents derived from a nucleic acid sequence disclosed in the Sequence Listing by substitution, insertion or deletion have at least 30%, 35%, 40%, 45%, or 50%, preferably at least 55%, 60%, 65%, or 70% by preference at least 80%, especially preferably at least 85% or 90%, 91%, 92%, 93%, or 94%, very especially preferably at least 95%, 97%, 98%, or 99% homology with one of the polypeptides disclosed in the Sequence Listing according to the invention and encode polypeptides having essentially the same properties as a polypeptide disclosed in the Sequence Listing.

"Essentially the same properties" of a functional equivalent is above all understood as meaning that the functional equivalent has above mentioned activity, e.g. conferring an increase in the fine chemical amount while increasing the amount of protein, activity or function of said functional equivalent in an organism, e.g., a microorgansim, a plant, or plant or animal tissue, plant or animal cells, or a part of the same.

By "hybridizing" it is meant that such nucleic acid molecules hybridize under conventional hybridization conditions, preferably under stringent conditions such as described by, e.g., Sambrook (Molecular Cloning; A Laboratory Manual, 2nd Edition, Cold Spring Harbor Laboratory Press, Cold Spring Harbor, NY (1989)) or in Current Protocols in Molecular Biology, John Wiley & Sons, N. Y. (1989), 6.3.1-6.3.6.

According to the invention, DNA as well as RNA molecules of the nucleic acid of the invention can be used as probes. Further, as template for the identification of functional homologues Northern blot assays as well as Southern blot assays can be performed. The Northern blot assay advantageously provides further information about the expressed gene product: e.g. expression pattern, occurrence of processing steps, like splicing and capping, etc. The Southern blot assay provides additional information about the chromosomal localization and organization of the gene encoding the nucleic acid molecule of the invention.

A preferred, nonlimiting example of stringent hybridization conditions are hybridizations in 6 x sodium chloride/sodium citrate (= SSC) at approximately 45°C, followed by one or more wash steps in 0.2 x SSC, 0.1 % SDS at 50°C to 65°C, for example at 50°C, 55°C, or 60°C. The skilled worker knows that these hybridization conditions differ as a function of the type of the nucleic acid and, for example when organic solvents are present, with regard to the temperature and concentration of the buffer. The temperature under "standard hybridization conditions" differs for example as a function of the type of the nucleic acid between 42°C and 58°C, preferably between 45°C and 50°C in an aqueous buffer with a concentration of 0.1 x 0.5 x, 1 x, 2x, 3x, 4x or 5 x SSC (pH 7.2). If organic solvent(s) is/are present in the abovementioned buffer, for example 50% formamide, the temperature under standard conditions is approximately 40°C, 42°C, or 45°C. The hybridization conditions for DNA:DNA hybrids are preferably for example 0.1 x SSC and 20°C, 25°C, 30°C, 35°C, 40°C, or 45°C, preferably between 30°C and 45°C. The hybridization conditions for DNA:RNA hybrids are preferably for example 0.1 x SSC and 30°C, 35°C, 40°C, 45°C, 50°C or 55°C, preferably between 45°C and 55°C. The abovementioned hybridization temperatures are determined for example for a nucleic acid approximately 100 bp (= base pairs) in length and a G + C content of 50% in the absence of formamide. The skilled worker knows to determine the hybridization conditions required with the aid of textbooks, for example the ones mentioned above, or from the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach," IRL Press at Oxford University Press, Oxford.

A further example of one such stringent hybridization condition is hybridization at 4XSSC at 65°C, followed by a washing in 0.1XSSC at 65°C for one hour. Alternatively, an exemplary stringent hybridization condition is in 50 % formamide, 4XSSC at 42°C. Further, the conditions during the wash step can be selected from the range of conditions delimited by low-stringency conditions (approximately 2X SSC at 50°C) and high-stringency conditions (approximately 0.2X SSC at 50°C, preferably at 65°C) (20X SSC: 0.3M sodium citrate, 3M NaCl, pH 7.0). In addition, the temperature during the wash step can be raised from low-stringency conditions at room temperature, approximately 22°C, to higherstringency conditions at approximately 65°C. Both of the parameters salt concentration and temperature can be varied simultaneously, or else one of the two parameters can be kept constant while only the other is varied. Denaturants, for example formamide or SDS, may also be employed during the hybridization. In the presence of 50% formamide, hybridization is preferably effected at 42°C. Relevant factors like i) length of treatment, ii) salt conditions, iii) detergent conditions, iv) competitor DNAs, v) temperature, and vi) probe selection can be combined case by case so that not all possibilities can be mentioned herein.

Thus, in a preferred embodiment, Northern blots are prehybridized with Rothi-Hybri-Quick buffer (Roth, Karlsruhe) at 68°C for 2h. Hybridization with radioactive labelled probe is done overnight at 68°C. Subsequent washing steps are performed at 68°C with 1xSSC.

For Southern blot assays the membrane is prehybridized with Rothi-Hybri-Quick buffer (Roth, Karlsruhe) at 68°C for 2h. The hybridization with radioactive labelled probe is conducted over night at 68°C. Subsequently the hybridization buffer is discarded and the filter shortly washed using 2xSSC; 0,1% SDS. After discarding the washing buffer new 2xSSC; 0,1 % SDS buffer is added and incubated at 68°C for 15 minutes. This washing step is performed twice followed by an additional washing step using 1xSSC; 0,1 % SDS at 68°C for 10 min.

Some further examples of conditions for DNA hybridization (Southern blot assays) and wash step are shown hereinbelow:

Hybridization conditions can be selected, for example, from the following conditions: a) 4X SSC at 65°C; b) 6X SSC at 45°C; c) 6X SSC, 100 mg/ml denatured fragmented fish sperm DNA at 68°C; d) 6X SSC, 0.5% SDS, 100 mg/ml denatured salmon sperm DNA at 68°C; e) 6X SSC, 0.5% SDS, 100 mg/ml denatured fragmented salmon sperm DNA, 50% formamide at 42°C; f) 50% formamide, 4X SSC at 42°C; g) 50% (vol/vol) formamide, 0.1% bovine serum albumin, 0.1% Ficoll, 0.1% polyvinylpyrrolidone, 50 mM sodium phosphate buffer pH 6.5, 750 mM NaCl, 75 mM sodium citrate at 42°C; h) 2X or 4X SSC at 50°C (low-stringency condition; or i) 30 to 40% formamide, 2X or 4X SSC at 42°C (low-stringency condition).

Wash steps can be selected, for example, from the following conditions: a) 0.015 M NaCI/0.0015 M sodium citrate/0.1% SDS at 50°C; b) 0.1X SSC at 65°C; c) 0.1X SSC, 0.5% SDS at 68°C; d) 0.1X SSC, 0.5% SDS, 50% formamide at 42°C; e) 0.2X SSC, 0.1 % SDS at 42°C; f) 2X SSC at 65°C (low-stringency condition).

In an other embodiment is meant by standard conditions, for example, depending on the nucleic acid in question temperatures between 42°C and 58°C in an aqueous buffer solution having a concentration of between 0.1 and 5 x SSC (1 X SSC = 0.15 M NaCI, 15 mM sodium citrate, pH 7.2) or additionally in the presence of 50 % formamide, such as by way of example 42°C in 5 x SSC, 50 % formamide. Hybridization conditions for DNA:DNA hybrids are advantageously 0.1 x SSC and temperatures between approximately 20°C and 45°C, preferably between approximately 30°C and 45°C. For DNA:RNA hybrids the hybridization conditions are advantageously 0.1 x SSC and temperatures between approximately 30°C and 55°C, preferably between approximately 45°C and 55°C. These specified temperatures for hybridization are melting temperature values calculated by way of example for a nucleic acid having a length of approximately 100 nucleotides and a G + C content of 50 % in the absence of formamide. The experimental conditions for DNA hybridization are described in relevant genetics textbooks such as by way of example Sambrook et al., "Molecular Cloning," Cold Spring Harbor Laboratory, 1989, and may be calculated by formulae known to those skilled in the art, for example as a function of the length of the nucleic acids, the nature of the hybrids or the G + C content. Those skilled in the art may draw on the following textbooks for further information on hybridization: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Polypeptides having above-mentioned activity, i.e. conferring the altered metabolic activity, derived from other organisms, can be encoded by other DNA sequences, which hybridize to the sequences disclosed in the Sequence Listing under relaxed hybridization conditions and which code on expression for peptides conferring an altered metabolic activity.

Further, some applications have to be performed at low stringency hybridization conditions, without any consequences for the specificity of the hybridization. For example, a Southern blot analysis of total DNA could be probed with a nucleic acid molecule of the present invention and washed at low stringency (55°C in 2xSSPE0, 1 % SDS). The hybridization analysis could reveal a simple pattern of only genes encoding polypeptides of the present invention or used in the process of the invention, e.g. having herein-mentioned activity of increasing the fine chemical. A further example of such low-stringent hybridization conditions is 4XSSC at 50°C or hybridization with 30 to 40% formamide at 42°C. Such molecules comprise those which are fragments, analogues or derivatives of the polypeptide of the invention or used in the process of the invention and differ, for example, by way of amino acid and/or nucleotide deletion(s), insertion(s), substitution (s), addition(s) and/or recombination (s) or any other modification(s) known in the art either alone or in combination from the above-described amino acid sequences or their underlying nucleotide sequence(s). However, it is preferred to use high stringency hybridization conditions.

Hybridization should advantageously be carried out with fragments of at least 5, 10, 15, 20, 25, 30, 35, or 40 bp, advantageously at least 50, 60, 70, or 80 bp, preferably at least 90, 100, or 110 bp. Most preferably are fragments of at least 15, 20, 25, or 30 bp. Preferably are also hybridizations with at least 100 bp or 200, very especially preferably at least 400 bp in length. In an especially preferred embodiment, the hybridization should be carried out with the entire nucleic acid sequence with conditions described above.

The terms "fragment," "fragment of a sequence," or "part of a sequence" mean a truncated sequence of the original sequence referred to. The truncated sequence (nucleic acid or protein sequence) can vary widely in length; the minimum size being a sequence of sufficient size to provide a sequence with at least a comparable function and/or activity of the original sequence referred to or hybridizing with the nucleic acid molecule of the invention or used in the process of the invention under stringent conditions, while the maximum size is not critical. In some applications, the maximum size usually is not substantially greater than that required to provide the desired activity and/or function(s) of the original sequence.

In addition to fragments and fusion polypeptides of the SRPs described herein, the present invention includes homologs and analogs of naturally occurring SRPs and SRP encoding nucleic acids in a plant. "Homologs" are defined herein as two nucleic acids or polypeptides that have similar, or substantially identical, nucleotide or amino acid sequences, respectively. Homologs include allelic variants, orthologs, paralogs, agonists, and antagonists of SRPs as defined hereafter. The term "homolog" further encompasses nucleic acid molecules that differ from one of the nucleotide sequences disclosed in the Sequence Listing (and portions thereof) due to degeneracy of the genetic code and thus encode the same SRP as that encoded by the nucleotide sequences disclosed in the Sequence Listing. As used herein a "naturally occurring" SRP refers to a SRP amino acid sequence that occurs in nature. Preferably, a naturally occurring SRP comprises an amino acid sequence selected from the group consisting of polypeptides disclosed in the Sequence Listing.

An agonist of the SRP can retain substantially the same, or a subset, of the biological activities of the SRP. An antagonist of the SRP can inhibit one or more of the activities of the naturally occurring form of the SRP. For example, the SRP antagonist can competitively bind to a downstream or upstream member of the cell membrane component metabolic cascade that includes the SRP, or bind to a SRP that mediates transport of compounds across such membranes, thereby preventing translocation from taking place.

Nucleic acid molecules corresponding to natural allelic variants and analogs, orthologs and paralogs of a SRP cDNA can be isolated based on their identity to the Saccharomyces cerevisiae, E.coli, Brassica napus, Glycine max, or Oryza sativa SRP nucleic acids described herein using SRP cDNAs, or a portion thereof, as a hybridization probe according to standard hybridization techniques under stringent hybridization conditions. In an alternative embodiment, homologs of the SRP can be identified by screening combinatorial libraries of mutants, e.g., truncation mutants, of the SRP for SRP agonist or antagonist activity. In one embodiment, a variegated library of SRP variants is generated by combinatorial mutagenesis at the nucleic acid level and is encoded by a variegated gene library. A variegated library of SRP variants can be produced by, for example, enzymatically ligating a mixture of synthetic oligonucleotides into gene sequences such that a degenerate set of potential SRP sequences is expressible as individual polypeptides, or alternatively, as a set of larger fusion polypeptides (e.g., for phage display) containing the set of SRP sequences therein. There are a variety of methods that can be used to produce libraries of potential SRP homologs from a degenerate oligonucleotide sequence. Chemical synthesis of a degenerate gene sequence can be performed in an automatic DNA synthesizer, and the synthetic gene is then ligated into an appropriate expression vector. Use of a degenerate set of genes allows for the provision, in one mixture, of all of the sequences encoding the desired set of potential SRP sequences. Methods for synthesizing degenerate oligonucleotides are known in the art. See, e.g., Narang, S.A., 1983, Tetrahedron 39:3; Itakura et al., 1984, Annu. Rev. Biochem. 53:323; Itakura et al., 1984, Science 198:1056; lke et al., 1983, Nucleic Acid Res. 11:477.

In addition, libraries of fragments of the SRP coding regions can be used to generate a variegated population of SRP fragments for screening and subsequent selection of homologs of a SRP. In one embodiment, a library of coding sequence fragments can be generated by treating a double stranded PCR fragment of a SRP coding sequence with a nuclease under conditions wherein nicking occurs only about once per molecule, denaturing the double stranded DNA, renaturing the DNA to form double stranded DNA, which can include sense/antisense pairs from different nicked products, removing single stranded portions from reformed duplexes by treatment with S1 nuclease, and ligating the resulting fragment library into an expression vector. By this method, an expression library can be derived which encodes N-terminal, C-terminal, and internal fragments of various sizes of the SRP.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of SRP homologs. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique that enhances the frequency of functional mutants in the libraries, can be used in combination with the screening assays to identify SRP homologs (Arkin and Yourvan, 1992, PNAS 89:7811-7815; Delgrave et al., 1993, Polypeptide Engineering 6(3):327-331). In another embodiment, cell based assays can be exploited to analyze a variegated SRP library, using methods well known in the art. The present invention further provides a method of identifying a novel SRP, comprising (a) raising a specific antibody response to a SRP, or a fragment thereof, as described herein; (b) screening putative SRP material with the antibody, wherein specific binding of the antibody to the material indicates the presence of a potentially novel SRP; and (c) analyzing the bound material in comparison to known SRP, to determine its novelty.

As stated above, the present invention includes SRPs and homologs thereof. To determine the percent sequence identity of two amino acid sequences (e.g., one of the sequences disclosed in the Sequence Listing, and a mutant form thereof), the sequences are aligned for optimal comparison purposes (e.g., gaps can be introduced in the sequence of one polypeptide for optimal alignment with the other polypeptide or nucleic acid). The amino acid residues at corresponding amino acid positions are then compared. When a position in one sequence (e.g., one of the sequences disclosed in the Sequence Listing) is occupied by the same amino acid residue as the corresponding position in the other sequence (e.g., a mutant form of the sequence selected from a polypeptide disclosed in the Sequence Listing), then the molecules are identical at that position. The same type of comparison can be made between two nucleic acid sequences.

The percent sequence identity between the two sequences is a function of the number of identical positions shared by the sequences (i.e., percent sequence identity = numbers of identical positions/total numbers of positions x 100). Preferably, the isolated amino acid homologs included in the present invention are at least about 50-60%, preferably at least about 60-70%, and more preferably at least about 70-75%, 75-80%, 80-85%, 85-90%, or 90-95%, and most preferably at least about 96%, 97%, 98%, 99%, or more identical to an entire amino acid sequence disclosed in the Sequence Listing. In yet another embodiment, the isolated amino acid homologs included in the present invention are at least about 50-60%, preferably at least about 60-70%, and more preferably at least about 70-75%, 75-80%, 80-85%, 85-90%, or 90-95%, and most preferably at least about 96%, 97%, 98%, 99%, or more identical to an entire amino acid sequence encoded by a nucleic acid sequence disclosed in the Sequence Listing. In other embodiments, the SRP amino acid homologs have sequence identity over at least 15 contiguous amino acid residues, more preferably at least 25 contiguous amino acid residues, and most preferably at least 35 contiguous amino acid residues of a sequence disclosed in the Sequence Listing.

In another preferred embodiment, an isolated nucleic acid homolog of the invention comprises a nucleotide sequence which is at least about 50-60%, preferably at least about 60-70%, more preferably at least about 70-75%, 75-80%, 80-85%, 85-90%, or 90-95%, and even more preferably at least about 95%, 96%, 97%, 98%, 99%, or more identical to a nucleotide sequence disclosed in the Sequence Listing, or to a portion comprising at least 20, 30, 40, 50, or 60 consecutive nucleotides thereof. The preferable length of sequence comparison for nucleic acids is at least 75 nucleotides, more preferably at least 100 nucleotides and most preferably the entire length of the coding region.

It is further preferred that the isolated nucleic acid homolog of the invention encodes a SRP, or portion thereof, that is at least 85% identical to an amino acid sequence disclosed in the Sequence Listing and that functions as a modulator of an environmental stress response in a plant. In a more preferred embodiment, overexpression of the nucleic acid homolog in a plant increases the tolerance of the plant to an environmental stress.

For the purposes of the invention, the percent sequence identity between two nucleic acid or polypeptide sequences is determined using the Vector NTI 6.0 (PC) software package (InforMax, 7600 Wisconsin Ave., Bethesda, MD 20814). A gap-opening penalty of 15 and a gap extension penalty of 6.66 are used for determining the percent identity of two nucleic acids. A gap-opening penalty of 10 and a gap-extension penalty of 0.1 are used for determining the percent identity of two polypeptides. All other parameters are set at the default settings. For purposes of a multiple alignment (Clustal W algorithm), the gap-opening penalty is 10, and the gap-extension penalty is 0.05 with blosum62 matrix. It is to be understood that for the purposes of determining sequence identity when comparing a DNA sequence to an RNA sequence, a thymidine nucleotide is equivalent to a uracil nucleotide.

In another aspect, the invention provides an isolated nucleic acid comprising a polynucleotide that hybridizes to a polynucleotide disclosed in the Sequence Listing under stringent conditions. More particularly, an isolated nucleic acid molecule of the invention is at least 15 nucleotides in length and hybridizes under stringent conditions to the nucleic acid molecule comprising a nucleotide sequence disclosed in the Sequence Listing. In other embodiments, the nucleic acid is at least 30, 50, 100, 250, or more nucleotides in length. Preferably, an isolated nucleic acid homolog of the invention comprises a nucleotide sequence, which hybridizes under highly stringent conditions to a nucleotide sequence disclosed in the Sequence Listing, and functions as a modulator of stress tolerance in a plant. In a further preferred embodiment, overexpression of the isolated nucleic acid homolog in a plant increases a plant's tolerance to an environmental stress.

As used herein with regard to hybridization for DNA to DNA blot, the term "stringent conditions" refers in one embodiment to hybridization overnight at 60°C in 10X Denharts solution, 6X SSC, 0.5% SDS and 100 µg/ml denatured salmon sperm DNA. Blots are washed sequentially at 62°C for 30 minutes each time in 3X SSC/0.1% SDS, followed by 1X SSC/0.1% SDS and finally 0.1X SSC/0.1 % SDS. As also used herein, "highly stringent conditions" refers to hybridization overnight at 65°C in 10X Denharts solution, 6X SSC, 0.5% SDS and 100 µg/ml denatured salmon sperm DNA. Blots are washed sequentially at 65°C for 30 minutes each time in 3X SSC/0.1% SDS, followed by 1X SSC/0.1% SDS and finally 0.1X SSC/0.1% SDS. Methods for nucleic acid hybridizations are described in Meinkoth and Wahl, 1984, Anal. Biochem. 138:267-284; Ausubel et al. eds, 1995, Current Protocols in Molecular Biology, Chapter 2, Greene Publishing and Wiley-Interscience, New York; and Tijssen, 1993, Laboratory Techniques in Biochemistry and Molecular Biology: Hybridization with Nucleic Acid Probes, Part 1, Chapter 2, Elsevier, New York. Preferably, an isolated nucleic acid molecule of the invention that hybridizes under stringent or highly stringent conditions to a sequence disclosed in the Sequence Listing corresponds to a naturally occurring nucleic acid molecule. As used herein, a "naturally occurring" nucleic acid molecule refers to an RNA or DNA molecule having a nucleotide sequence that occurs in nature (e.g., encodes a natural polypeptide). In one embodiment, the nucleic acid encodes a naturally occurring Saccharomyces cerevisiae, E.coli, Brassica napus, Glycine max, or Oryza sativa SRP.

Using the above-described methods, and others known to those of skill in the art, one of ordinary skill in the art can isolate homologs of the SRPs comprising amino acid sequences disclosed in the Sequence Listing. One subset of these homologs are allelic variants. As used herein, the term "allelic variant" refers to a nucleotide sequence containing polymorphisms that lead to changes in the amino acid sequences of a SRP and that exist within a natural population (e.g., a plant species or variety). Such natural allelic variations can typically result in 1-5% variance in a SRP nucleic acid. Allelic variants can be identified by sequencing the nucleic acid sequence of interest in a number of different plants, which can be readily carried out by using hybridization probes to identify the same SRP genetic locus in those plants. Any and all such nucleic acid variations and resulting amino acid polymorphisms or variations in a SRP that are the result of natural allelic variation and that do not alter the functional activity of a SRP, are intended to be within the scope of the invention.

An isolated nucleic acid molecule encoding a SRP having sequence identity with a polypeptide sequence disclosed in the Sequence Listing can be created by introducing one or more nucleotide substitutions, additions or deletions into a nucleotide sequence disclosed in the Sequence Listing, respectively, such that one or more amino acid substitutions, additions, or deletions are introduced into the encoded polypeptide. Mutations can be introduced into one of the sequences disclosed in the Sequence Listing by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain.

Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine), and aromatic side chains (e.g., tyrosine, phenylalanine, tryptophan, histidine). Thus, a predicted nonessential amino acid residue in a SRP is preferably replaced with another amino acid residue from the same side chain family. Alternatively, in another embodiment, mutations can be introduced randomly along all or part of a SRP coding sequence, such as by saturation mutagenesis, and the resultant mutants can be screened for a SRP activity described herein to identify mutants that retain SRP activity. Following mutagenesis of one of the sequences disclosed in the Sequence Listing, the encoded polypeptide can be expressed recombinantly and the activity of the polypeptide can be determined by analyzing the stress tolerance of a plant expressing the polypeptide as described herein.

Additionally, optimized SRP nucleic acids can be created. As used herein, "optimized" refers to a nucleic acid that is genetically engineered to increase its expression in a given plant or animal. To provide plant optimized SRP nucleic acids, the DNA sequence of the gene can be modified to 1) comprise codons preferred by highly expressed plant genes; 2) comprise an A+T content in nucleotide base composition to that substantially found in plants; 3) form a plant initiation sequence; or 4) eliminate sequences that cause destabilization, inappropriate polyadenylation, degradation, and termination of RNA, or that form secondary structure hairpins or RNA splice sites. Increased expression of SRP nucleic acids in plants can be achieved by utilizing the distribution frequency of codon usage in plants in general or a particular plant. Methods for optimizing nucleic acid expression in plants can be found in EPA 0359472; EPA 0385962; PCT Application No. WO 91/16432; U.S. Patent No. 5,380,831; U.S. Patent No. 5,436,391; Perlack et al., 1991, Proc. Natl. Acad. Sci. USA 88:3324-3328; and Murray et al., 1989, Nucleic Acids Res. 17:477-498.

As used herein, "frequency of preferred codon usage" refers to the preference exhibited by a specific host cell in usage of nucleotide codons to specify a given amino acid. To determine the frequency of usage of a particular codon in a gene, the number of occurrences of that codon in the gene is divided by the total number of occurrences of all codons specifying the same amino acid in the gene. Similarly, the frequency of preferred codon usage exhibited by a host cell can be calculated by averaging frequency of preferred codon usage in a large number of genes expressed by the host cell. It is preferable that this analysis be limited to genes that are highly expressed by the host cell. The percent deviation of the frequency of preferred codon usage for a synthetic gene from that employed by a host cell is calculated first by determining the percent deviation of the frequency of usage of a single codon from that of the host cell followed by obtaining the average deviation over all codons. As defined herein, this calculation includes unique codons (i.e., ATG and TGG). In general terms, the overall average deviation of the codon usage of an optimized gene from that of a host cell is calculated using the equation 1A=n=1ZXₙ-YₙXₙ times 100 Z where Xₙ = frequency of usage for codon n in the host cell; Yₙ = frequency of usage for codon n in the synthetic gene; n represents an individual codon that specifies an amino acid; and the total number of codons is Z. The overall deviation of the frequency of codon usage, A, for all amino acids should preferably be less than about 25%, and more preferably less than about 10%.

Hence, a SRP nucleic acid can be optimized such that its distribution frequency of codon usage deviates, preferably, no more than 25% from that of highly expressed plant genes and, more preferably, no more than about 10%. In addition, consideration is given to the percentage G+C content of the degenerate third base (monocotyledons appear to favor G+C in this position, whereas dicotyledons do not). It is also recognized that the XCG (where X is A, T, C, or G) nucleotide is the least preferred codon in dicots whereas the XTA codon is avoided in both monocots and dicots. Optimized SRP nucleic acids of this invention also preferably have CG and TA doublet avoidance indices closely approximating those of the chosen host plant (i.e., Brassica napus, Glycine max, or Oryza sativa). More preferably these indices deviate from that of the host by no more than about 10-15%.

In addition to the nucleic acid molecules encoding the SRPs described above, another aspect of the invention pertains to isolated nucleic acid molecules that are antisense thereto. Antisense polynucleotides are thought to inhibit gene expression of a target polynucleotide by specifically binding the target polynucleotide and interfering with transcription, splicing, transport, translation, and/or stability of the target polynucleotide. Methods are described in the prior art for targeting the antisense polynucleotide to the chromosomal DNA, to a primary RNA transcript, or to a processed mRNA. Preferably, the target regions include splice sites, translation initiation codons, translation termination codons, and other sequences within the open reading frame.

The term "antisense," for the purposes of the invention, refers to a nucleic acid comprising a polynucleotide that is sufficiently complementary to all or a portion of a gene, primary transcript, or processed mRNA, so as to interfere with expression of the endogenous gene. "Complementary" polynucleotides are those that are capable of base pairing according to the standard Watson-Crick complementarily rules. Specifically, purines will base pair with pyrimidines to form a combination of guanine paired with cytosine (G:C) and adenine paired with either thymine (A:T) in the case of DNA, or adenine paired with uracil (A:U) in the case of RNA. It is understood that two polynucleotides may hybridize to each other even if they are not completely complementary to each other, provided that each has at least one region that is substantially complementary to the other. The term "antisense nucleic acid" includes single stranded RNA as well as double-stranded DNA expression cassettes that can be transcribed to produce an antisense RNA. "Active" antisense nucleic acids are antisense RNA molecules that are capable of selectively hybridizing with a primary transcript or mRNA encoding a polypeptide having at least 80% sequence identity with a polypeptide disclosed in the Sequence Listing.

The antisense nucleic acid can be complementary to an entire SRP coding strand, or to only a portion thereof. In one embodiment, an antisense nucleic acid molecule is antisense to a "coding region" of the coding strand of a nucleotide sequence encoding a SRP. The term "coding region" refers to the region of the nucleotide sequence comprising codons that are translated into amino acid residues. In another embodiment, the antisense nucleic acid molecule is antisense to a "noncoding region" of the coding strand of a nucleotide sequence encoding a SRP. The term "noncoding region" refers to 5' and 3' sequences that flank the coding region that are not translated into amino acids (i.e., also referred to as 5' and 3' untranslated regions). The antisense nucleic acid molecule can be complementary to the entire coding region of SRP mRNA, but more preferably is an oligonucleotide, which is antisense to only a portion of the coding or noncoding region of SRP mRNA. For example, the antisense oligonucleotide can be complementary to the region surrounding the translation start site of PKSRP mRNA. An antisense oligonucleotide can be, for example, about 5, 10, 15, 20, 25, 30, 35, 40, 45, or 50 nucleotides in length. Typically, the antisense molecules of the present invention comprise an RNA having 60-100% sequence identity with at least 14 consecutive nucleotides of one of a nucleic acid disclosed in the Sequence Listing. Preferably, the sequence identity will be at least 70%, more preferably at least 75%, 80%, 85%, 90%, 95%, 98%, and most preferably 99%.

An antisense nucleic acid of the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier et al., 1987, Nucleic Acids. Res. 15:6625-6641). The antisense nucleic acid molecule can also comprise a 2'-o-methylribonucleotide (Inoue et al., 1987, Nucleic Acids Res. 15:6131-6148) or a chimeric RNA-DNA analogue (Inoue et al., 1987, FEBS Lett. 215:327-330).

The antisense nucleic acid molecules of the invention are typically administered to a cell or generated in situ such that they hybridize with or bind to cellular mRNA and/or genomic DNA encoding a SRP to thereby inhibit expression of the polypeptide, e.g., by inhibiting transcription and/or translation. The hybridization can be by conventional nucleotide complementarily to form a stable duplex, or, for example, in the case of an antisense nucleic acid molecule which binds to DNA duplexes, through specific interactions in the major groove of the double helix. The antisense molecule can be modified such that it specifically binds to a receptor or an antigen expressed on a selected cell surface, e.g., by linking the antisense nucleic acid molecule to a peptide or an antibody which binds to a cell surface receptor or antigen. The antisense nucleic acid molecule can also be delivered to cells using the vectors described herein. To achieve suffcient intracellular concentrations of the antisense molecules, vector constructs in which the antisense nucleic acid molecule is placed under the control of a strong prokaryotic, viral, or eukaryotic (including plant) promoter are preferred.

As an alternative to antisense polynucleotides, ribozymes, sense polynucleotides, or double stranded RNA (dsRNA) can be used to reduce expression of a SRP polypeptide. By "ribozyme" is meant a catalytic RNA-based enzyme with ribonuclease activity, which is capable of cleaving a single-stranded nucleic acid, such as an mRNA, to which it has a complementary region. Ribozymes (e.g., hammerhead ribozymes described in Haselhoff and Gerlach, 1988, Nature 334:585-591) can be used to catalytically cleave SRP mRNA transcripts to thereby inhibit translation of SRP mRNA. A ribozyme having specificity for a SRP-encoding nucleic acid can be designed based upon the nucleotide sequence of a SRP cDNA, as disclosed herein (i.e., disclosed in the Sequence Listing or otherwise in the specification) or on the basis of a heterologous sequence to be isolated according to methods taught in this invention. For example, a derivative of a Tetrahymena L-19 IVS RNA can be constructed in which the nucleotide sequence of the active site is complementary to the nucleotide sequence to be cleaved in a SRP-encoding mRNA. See, e.g., U.S. Patent Nos. 4,987,071 and 5,116,742 to Cech et al. Alternatively, SRP mRNA can be used to select a catalytic RNA having a specific ribonuclease activity from a pool of RNA molecules. See, e.g., Bartel, D. and Szostak, J.W., 1993, Science 261:1411-1418. In preferred embodiments, the ribozyme will contain a portion having at least 7, 8, 9, 10, 12, 14, 16, 18 or 20 nucleotides, and more preferably 7 or 8 nucleotides, that have 100% complementarity to a portion of the target RNA. Methods for making ribozymes are known to those skilled in the art. See, e.g., U.S. Patent Nos. 6,025,167; 5,773,260; and 5,496,698.

The term "dsRNA," as used herein, refers to RNA hybrids comprising two strands of RNA. The dsRNAs can be linear or circular in structure. In a preferred embodiment, dsRNA is specific for a polynucleotide encoding either a polypeptide disclosed in the Sequence Listing or a polypeptide having at least 70% sequence identity with a polypeptide disclosed in the Sequence Listing. The hybridizing RNAs may be substantially or completely complementary. By "substantially complementary," is meant that when the two hybridizing RNAs are optimally aligned using the BLAST program as described above, the hybridizing portions are at least 95% complementary. Preferably, the dsRNA will be at least 100 base pairs in length. Typically, the hybridizing RNAs will be of identical length with no over hanging 5' or 3' ends and no gaps. However, dsRNAs having 5' or 3' overhangs of up to 100 nucleotides may be used in the methods of the invention.

The dsRNA may comprise ribonucleotides or ribonucleotide analogs, such as 2'-O-methyl ribosyl residues, or combinations thereof. See, e.g., U.S. Patent Nos. 4,130,641 and 4,024,222. A dsRNA polyriboinosinic acid:polyribocytidylic acid is described in U.S. patent 4,283,393. Methods for making and using dsRNA are known in the art. One method comprises the simultaneous transcription of two complementary DNA strands, either in vivo, or in a single in vitro reaction mixture. See, e.g., U.S. Patent No. 5,795,715. In one embodiment, dsRNA can be introduced into a plant or plant cell directly by standard transformation procedures. Alternatively, dsRNA can be expressed in a plant cell by transcribing two complementary RNAs.

Other methods for the inhibition of endogenous gene expression, such as triple helix formation (Moser et al., 1987, Science 238:645-650 and Cooney et al., 1988, Science 241:456-459) and cosuppression (Napoli et al., 1990, The Plant Cell 2:279-289) are known in the art. Partial and full-length cDNAs have been used for the cosuppression of endogenous plant genes. See, e.g., U.S. Patent Nos. 4,801,340, 5,034,323, 5,231,020, and 5,283,184; Van der Kroll et al., 1990, The Plant Cell 2:291-299; Smith et al., 1990, Mol. Gen. Genetics 224:477-481 and Napoli et al., 1990, The Plant Cell 2:279-289.

For sense suppression, it is believed that introduction of a sense polynucleotide blocks transcription of the corresponding target gene. The sense polynucleotide will have at least 65% sequence identity with the target plant gene or RNA. Preferably, the percent identity is at least 80%, 90%, 95%, or more. The introduced sense polynucleotide need not be full length relative to the target gene or transcript. Preferably, the sense polynucleotide will have at least 65% sequence identity with at least 100 consecutive nucleotides of one of the nucleic acids disclosed in the Sequence Listing. The regions of identity can comprise introns and and/or exons and untranslated regions. The introduced sense polynucleotide may be present in the plant cell transiently, or may be stably integrated into a plant chromosome or extrachromosomal replicon.

Moreover, nucleic acid molecules encoding SRP from the same or other species such as SRP analogs, orthologs, and paralogs, are intended to be within the scope of the present invention. As used herein, the term "analogs" refers to two nucleic acids that have the same or similar function, but that have evolved separately in unrelated organisms. As used herein, the term "orthologs" refers to two nucleic acids from different species that have evolved from a common ancestral gene by speciation. Normally, orthologs encode proteins having the same or similar functions. As also used herein, the term "paralogs" refers to two nucleic acids that are related by duplication within a genome. Paralogs usually have different functions, but these functions may be related (Tatusov, R.L. et al. 1997 Science 278(5338):631-637). Analogs, orthologs, and paralogs of a naturally occurring stress related protein can differ from the naturally occurring stress related protein by post-translational modifications, by amino acid sequence differences, or by both. Post-translational modifications include in vivo and in vitro chemical derivatization of polypeptides e.g., acetylation, carboxylation, phosphorylation or glycosylation, and such modifications may occur during polypeptide synthesis or processing or following treatment with isolated modifying enzymes. In particular, orthologs of the invention will generally exhibit at least 80-85%, more preferably 90%, 91%, 92%, 93%, 94%, and most preferably 95%, 96%, 97%, 98%, or even 99% identity or homology with all or part of a naturally occurring stress related protein amino acid sequence and will exhibit a function similar to a stress related protein. Orthologs of the present invention are also preferably capable of participating in the stress response in plants.

In addition to naturally-occurring variants of a stress related protein sequence that may exist in the population, the skilled artisan will further appreciate that changes can be introduced by mutation into a nucleotide sequence disclosed in the Sequence Listing, thereby leading to changes in the amino acid sequence of the encoded stress related protein, without altering the functional ability of the stress related protein or enhancing the functional ability of the stress related protein. For example, nucleotide substitutions leading to amino acid substitutions at "non-essential" amino acid residues can be made in a sequence disclosed in the Sequence Listing. A "non-essential" amino acid residue is a residue that can be altered from the wild-type sequence of one of stress related proteins without altering the activity thereof, whereas an "essential" amino acid residue is required for stress related protein activity. Other amino acid residues, however, (e.g., those that are not conserved or only semi-conserved in the domain having SRP activity) may not be essential for activity and thus are likely to be amenable to alteration without altering SRP activity.

Accordingly, another aspect of the invention pertains to nucleic acid molecules encoding stress related proteins that contain changes in amino acid residues that are not essential for stress related protein activity. Such SRP differ in amino acid sequence from a sequence disclosed in the Sequence Listing, yet retain at least one of the stress related protein activities described herein. In one embodiment, the isolated nucleic acid molecule comprises a nucleotide sequence encoding a protein, wherein the protein comprises an amino acid sequence at least about 50% homologous to an amino acid sequence disclosed in the Sequence Listing. Preferably, the protein encoded by the nucleic acid molecule is at least about 50-60% homologous to one of the sequences disclosed in the Sequence Listing, more preferably at least about 60 - 70% homologous to one of the sequences disclosed in the Sequence Listing, even more preferably at least about 70 - 80%, 80 - 90%, more preferably 90%, 91%, 92%, 93%, or 94% homologous to one of the sequences disclosed in the Sequence Listing and most preferably at least about 96%, 97%, 98%, or 99% homologous to one of the sequences disclosed in the Sequence Listing. The preferred stress related protein homologs of the present invention are preferably capable of participating in the stress tolerance response in a plant. The homology (= identity) was calculated over the entire amino acid range. The program used was PileUp (J. Mol. Evolution, 25 (1987), 351-360, Higgins et al., CABIOS, 5 1989: 151-153).

Homologs of the sequences disclosed in the Sequence Listing are furthermore to be understood as meaning, for example, homologs, analogs, orthologs and paralogs which have at least 30% homology (= identity) at the derived amino acid level, preferably at least 50 %, 60 %, 70 %, or 80 % homology, especially preferably at least 85 % homology, very especially preferably 90 % 91 %, 92%, 93%, 94% homology, most preferably 95 %, 96 %, 97 %, 98 % or 99 % homology. The homology (= identity) was calculated over the entire amino acid range. The program used was PileUp (J. Mol. Evolution, 25 (1987), 351 - 360, Higgens et al., CABIOS, 5 1989: 151 - 153) or the program Gap and BestFit (Needleman and Wunsch (J. Mol. Biol. 48; 443 - 453 (1970) and Smith and Waterman respectively (Adv. Appl. Math. 2; 482-489 (1981)), which are part of the GCG software package (Genetics Computer Group, 575 Science Drive, Madison, Wisconsin, USA 53711 (1991)). The above mentioned percentages of sequence homology are calculated with the program BestFit or Gap, preferably Gap, over the total sequence length with the following parameters used: Gap Weight: 8, Length Weight: 2.

Variants shall also be encompassed, in particular, functional variants, which can be obtained from a sequence disclosed in the Sequence Listing by means of deletion, insertion or substitution of nucleotides, the enzymatic activity of the derived synthetic proteins being retained or enhanced.

An isolated nucleic acid molecule encoding a stress related protein homologous to a protein sequence disclosed in the Sequence Listing can be created by introducing one or more nucleotide substitutions, additions or deletions into a nucleotide sequence disclosed in the Sequence Listing such that one or more amino acid substitutions, additions or deletions are introduced into the encoded protein. Mutations can be introduced into one of the sequences disclosed in the Sequence Listing by standard techniques, such as site-directed mutagenesis and PCR-mediated mutagenesis. Another route to the mutagenesis of enzymes, disclosed in the European Publication EP-A-0 909 821, is a method using the specific Escherichia coli strain XL1-Red to generate mutants and altered the enzyme activity.

Preferably, conservative amino acid substitutions are made at one or more predicted non-essential amino acid residues. A "conservative amino acid substitution" is one in which the amino acid residue is replaced with an amino acid residue having a similar side chain.

Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in a stress related protein is preferably replaced with another amino acid residue from the same side chain family. Alternatively, in another embodiment, mutations can be introduced randomly along all or part of a stress related protein coding sequence, such as by saturation mutagenesis, and the resultant mutants can be screened for a stress related protein activity as described herein to identify mutants that retain stress related protein activity or show enhanced stress related protein activity. Following mutagenesis of one of the sequences of a nucleic acid disclosed in the Sequence Listing, the encoded protein can be expressed recombinantly and the activity of the protein can be determined by analyzing the stress tolerance of a plant expressing the protein as described in the examples below.

A useful method to ascertain the level of transcription of the gene (an indicator of the amount of mRNA available for translation to the gene product) is to perform a Northern blot (for reference see, for example, Ausubel et al., 1988 Current Protocols in Molecular Biology, Wiley: New York). This information at least partially demonstrates the degree of transcription of the gene. Total cellular RNA can be prepared from cells, tissues or organs by several methods, all well-known in the art, such as that described in Bormann, E.R. et al., 1992 Mol. Microbiol. 6:317-326. To assess the presence or relative quantity of protein translated from this mRNA, standard techniques, such as a Western blot, may be employed. These techniques are well known to one of ordinary skill in the art (see, for example, Ausubel et al., 1988 Current Protocols in Molecular Biology, Wiley: New York).

The present invention also relates to a plant expression cassette comprising a SRP coding nucleic acid selected from the group comprising sequences disclosed in the Sequence Listing and/or homologs or parts thereof operatively linked to regulatory sequences and/or targeting sequences.

Further, object of the invention is an expression vector comprising a SRP encoding nucleic acid selected from the group comprising sequences of the nucleic acids disclosed in the Sequence Listing and/or homologs or parts thereof or a plant expression cassette as described above, whereby expression of the SRP coding nucleic acid in a host cell results in increased tolerance to environmental stress, which is preferably achieved by altering metabolic activity, as compared to a corresponding non-transformed, wild-type host cell.

The invention further provides an isolated recombinant expression vector comprising a stress related protein encoding nucleic acid as described above, wherein expression of the vector or stress related protein encoding nucleic acid, respectively in a host cell results in increased tolerance and/or resistance to environmental stress, which is preferably achieved by altering metabolic activity, as compared to the corresponding non-transformed, wild-type of the host cell. As used herein, the term "vector" refers to a nucleic acid molecule capable of transporting another nucleic acid to which it has been linked. One type of vector is a "plasmid," which refers to a circular double stranded DNA loop into which additional DNA segments can be ligated. Another type of vector is a viral vector, wherein additional DNA segments can be ligated into the viral genome. Further types of vectors can be linearized nucleic acid sequences, such as transposons, which are pieces of DNA, which can copy and insert themselves. There have been 2 types of transposons found: simple transposons, known as Insertion Sequences and composite transposons, which can have several genes as well as the genes that are required for transposition.

Certain vectors are capable of autonomous replication in a host cell, into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors." In, general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions.

A plant expression cassette preferably contains regulatory sequences capable of driving gene expression in plant cells and operably linked so that each sequence can fulfill its function, for example, termination of transcription by polyadenylation signals. Preferred polyadenylation signals are those originating from Agrobacterium tumefaciens T-DNA such as the gene 3 known as octopine synthase of the Ti-plasmid pTiACH5 (Gielen et al., 1984 EMBO J. 3:835) or functional equivalents thereof but also all other terminators functionally active in plants are suitable.

As plant gene expression is very often not limited on transcriptional levels, a plant expression cassette preferably contains other operably linked sequences like translational enhancers such as the overdrive-sequence containing the 5'-untranslated leader sequence from tobacco mosaic virus enhancing the protein per RNA ratio (Gallie et al., 1987 Nucl. Acids Research 15:8693-8711).

Plant gene expression has to be operably linked to an appropriate promoter conferring gene expression in a timely, cell or tissue specific manner. Preferred are promoters driving constitutive expression (Benfey et al., 1989 EMBO J. 8:2195-2202) like those derived from plant viruses like the 35S CaMV (Franck et al., 1980 Cell 21:285-294), the 19S CaMV (see also U.S. Patent No. 5352605 and PCT Application No. WO 8402913) or plant promoters like those from Rubisco small subunit described in U.S. Patent No. 4,962,028.

Additional advantageous regulatory sequences are, for example, included in the plant promoters such as CaMV/35S [Franck et al., Cell 21 (1980) 285 - 294], PRP1 (Ward et al., Plant. Mol. Biol. 22 (1993)), SSU, OCS, lib4, usp, STLS1, B33, LEB4, nos or in the ubiquitin, napin or phaseolin promoter. Also advantageous in this connection are inducible promoters such as the promoters described in EP-A-0 388 186 (benzyl sulfonamide inducible), Plant J. 2, 1992: 397 - 404 (Gatz et al., Tetracyclin inducible), EP-A-0 335 528 (abscisic acid inducible) or WO 93/21334 (ethanol or cyclohexenol inducible). Additional useful plant promoters are the cytosolic FBPase promoter or ST-LSI promoter of the potato (Stockhaus et al., EMBO J. 8, 1989, 2445), the phosphorybosyl phyrophoshate amido transferase promoter of Glycine max (gene bank accession No. U87999) or the noden specific promoter described in EP-A-0 249 676. Additional particularly advantageous promoters are seed specific promoters which can be used for monokotyledones or dikotyledones and are described in US 5,608,152 (napin promoter from rapeseed), WO 98/45461 (phaseolin promoter from Arobidopsis), US 5,504,200 (phaseolin promoter from Phaseolus vulgaris), WO 91/13980 (Bce4 promoter from Brassica) and Baeumlein et al., Plant J., 2, 2, 1992: 233-239 (LEB4 promoter from leguminosa). Said promoters are useful in dikotyledones. The following promoters are useful for example in monokotyledones lpt-2- or lpt-1- promoter from barley (WO 95/15389 and WO 95/23230) or hordein promoter from barley. Other useful promoters are described in WO 99/16890.

It is possible in principle to use all natural promoters with their regulatory sequences like those mentioned above for the novel process. It is also possible and advantageous in addition to use synthetic promoters.

The gene construct may also comprise further genes, which are to be inserted into the organisms, and which are for example involved in stress resistance. It is possible and advantageous to insert and express in host organisms regulatory genes such as genes for inducers, repressors or enzymes, which intervene by their enzymatic activity in the regulation, or one or more or all genes of a biosynthetic pathway. These genes can be heterologous or homologous in origin. The inserted genes may have their own promoter or else be under the control of same promoter as a sequence disclosed in the Sequence Listing or their homologs.

The gene construct advantageously comprises, for expression of the other genes present, additionally 3' and/or 5' terminal regulatory sequences to enhance expression, which are selected for optimal expression depending on the selected host organism and gene or genes.

These regulatory sequences are intended to make specific expression of the genes and protein expression possible as mentioned above. This may mean, depending on the host organism, for example that the gene is expressed or overexpressed only after induction, or that it is immediately expressed and/or overexpressed.

The regulatory sequences or factors may moreover preferably have a beneficial effect on expression of the introduced genes, and thus increase it. It is possible in this way for the regulatory elements to be enhanced advantageously at the transcription level by using strong transcription signals such as promoters and/or enhancers. However, in addition, it is also possible to enhance translation by, for example, improving the stability of the mRNA.

Other preferred sequences for use in plant gene expression cassettes are targeting-sequences necessary to direct the gene product in its appropriate cell compartment (for review see Kermode, 1996 Crit. Rev. Plant Sci. 15(4):285-423 and references cited therein) such as the vacuole, the nucleus, all types of plastids like amyloplasts, chloroplasts, chromoplasts, the extracellular space, mitochondria, the endoplasmic reticulum, oil bodies, peroxisomes and other compartments of plant cells.

Plant gene expression can also be facilitated via an inducible promoter (for review, see Gatz, 1997 Annu. Rev. Plant Physiol. Plant Mol. Biol. 48:89-108). Chemically inducible promoters are especially suitable if gene expression is wanted to occur in a time specific manner.

Table 1 lists several examples of promoters that may be used to regulate transcription of the stress related protein nucleic acid coding sequences.

**Table 1: Examples of Tissue-specific and Stress inducible promoters in plants.**

| Expression | Reference |
|---|---|
| Cor78- Cold, drought, salt, ABA, wounding-inducible | lshitani, et al., Plant Cell 9:1935-1949 (1997). Yamaguchi-Shinozaki and Shinozaki, Plant Cell 6:251-264 (1994). |
| Rci2A - Cold, dehydra-tion-inducible | Capel et al., Plant Physiol 115:569-576 (1997) |
| Rd22 - Drought, salt | Yamaguchi-Shinozaki and Shinozaki, Mol Gen Genet 238:17-25 (1993). |
| Cor15A - Cold, dehy-dration, ABA | Baker et al., Plant Mol. Biol. 24:701-713 (1994). |
| GH3- Auxin inducible | Liu et al., Plant Cell 6:645-657 (1994) |
| ARSK1-Root, salt inducible | Hwang and Goodman, Plant J 8:37-43 (1995). |
| PtxA - Root, salt inducible | GenBank accession X67427 |
| SbHRGP3 - Root specific | Ahn et al., Plant Cell 8:1477-1490 (1998). |
| KST1 - Guard cell specific | Plesch et al., Plant Journal. 28(4):455-64, (2001) |
| KAT1 - Guard cell specific | Plesch et al., Gene 249:83-89 (2000) Nakamura et al., Plant Physiol. 109:371-374 (1995) |
| salicylic acid inducible | PCT Application No. WO 95/19443 |
| tetracycline inducible | Gatz et al. Plant J. 2:397-404 (1992) |
| Ethanol inducible | PCT Application No. WO 93/21334 |
| pathogen inducible PRP1 | Ward et al., 1993 Plant. Mol. Biol. 22:361-366 |
| heat inducible hsp80 | U.S. Patent No. 5187267 |
| cold inducible alphaamylase | PCT Application No. WO 96/12814 |
| Wound-inducible pinll | European Patent No. 375091 |
| RD29A - salt-inducible | Yamaguchi-Shinozalei et al. (1993) Mol. Gen. Genet. 236:331-340 |
| plastid-specific viral RNA-polymerase | PCT Application No. WO 95/16783 and. WO 97/06250 |

Other promoters, e.g. superpromoter (Ni et al., Plant Journal 7, 1995: 661-676), Ubiquitin promoter (Callis et al., J. Biol. Chem., 1990, 265: 12486-12493; US 5,510,474; US 6,020,190; Kawalleck et al., Plant. Molecular Biology, 1993, 21: 673-684) or 34S promoter (GenBank Accession numbers M59930 and X16673) were similar useful for the present invention and are known to a person skilled in the art.

Developmental stage-preferred promoters are preferentially expressed at certain stages of development. Tissue and organ preferred promoters include those that are preferentially expressed in certain tissues or organs, such as leaves, roots, seeds, or xylem. Examples of tissue preferred and organ preferred promoters include, but are not limited to fruit-preferred, ovule-preferred, male tissue-preferred, seed-preferred, integument-preferred, tuber-preferred, stalkpreferred, pericarp-preferred, and leaf-preferred, stigma-preferred, pollen-preferred, anther-preferred, a petal-preferred, sepal-preferred, pedicel-preferred, silique-preferred, stem-preferred, root-preferred promoters, and the like. Seed preferred promoters are preferentially expressed during seed development and/or germination. For example, seed preferred promoters can be embryo-preferred, endosperm preferred, and seed coat-preferred. See Thompson et al., 1989, BioEssays 10:108. Examples of seed preferred promoters include, but are not limited to, cellulose synthase (celA), Cim1, gamma-zein, globulin-1, maize 19 kD zein (cZ19B1), and the like.

Other promoters useful in the expression cassettes of the invention include, but are not limited to, the major chlorophyll a/b binding protein promoter, histone promoters, the Ap3 promoter, the β-conglycin promoter, the napin promoter, the soybean lectin promoter, the maize 15kD zein promoter, the 22kD zein promoter, the 27kD zein promoter, the g-zein promoter, the waxy, shrunken 1, shrunken 2 and bronze promoters, the Zm13 promoter (U.S. Patent No. 5,086,169), the maize polygalacturonase promoters (PG) (U.S. Patent Nos. 5,412,085 and 5,545,546), and the SGB6 promoter (U.S. Patent No. 5,470,359), as well as synthetic or other natural promoters.

Additional flexibility in controlling heterologous gene expression in plants may be obtained by using DNA binding domains and response elements from heterologous sources (i.e., DNA binding domains from non-plant sources). An example of such a heterologous DNA binding domain is the LexA DNA binding domain (Brent and Ptashne, 1985, Cell 43:729-736).

The invention further provides a recombinant expression vector comprising a SRP DNA molecule of the invention cloned into the expression vector in an antisense orientation. That is, the DNA molecule is operatively linked to a regulatory sequence in a manner that allows for expression (by transcription of the DNA molecule) of an RNA molecule that is antisense to a SRP mRNA. Regulatory sequences operatively linked to a nucleic acid molecule cloned in the antisense orientation can be chosen which direct the continuous expression of the antisense RNA molecule in a variety of cell types. For instance, viral promoters and/or enhancers, or regulatory sequences can be chosen which direct constitutive, tissue specific, or cell type specific expression of antisense RNA. The antisense expression vector can be in the form of a recombinant plasmid, phagemid, or attenuated virus wherein antisense nucleic acids are produced under the control of a high efficiency regulatory region. The activity of the regulatory region can be determined by the cell type, into which the vector is introduced. For a discussion of the regulation of gene expression using antisense genes, see Weintraub, H. et al., 1986, Antisense RNA as a molecular tool for genetic analysis, Reviews - Trends in Genetics, Vol. 1(1), and Mol et al., 1990, FEBS Letters 268:427-430.

Another aspect of the invention pertains to host cells into which a recombinant expression vector of the invention has been introduced. The terms "host cell" and "recombinant host cell" are used interchangeably herein. It is understood that such terms refer not only to the particular subject cell but they also apply to the progeny or potential progeny of such a cell. Because certain modifications may occur in succeeding generations due to either mutation or environmental influences, such progeny may not, in fact, be identical to the parent cell, but are still included within the scope of the term as used herein. A host cell can be any prokaryotic or eukaryotic cell. For example, a SRP can be expressed in bacterial cells such as C. glutamicum, yeast, E.coli, insect cells, fungal cells, or mammalian cells (such as Chinese hamster ovary cells (CHO) or COS cells), algae, ciliates, plant cells, fungi, or other microorganisms like C. glutamicum. Other suitable host cells are known to those skilled in the art.

A host cell of the invention, such as a prokaryotic or eukaryotic host cell in culture, can be used to produce (i.e., express) a SRP. Accordingly, the invention further provides methods for producing SRPs using the host cells of the invention. In one embodiment, the method comprises culturing the host cell of invention (into which a recombinant expression vector encoding a SRP has been introduced, or into which genome has been introduced a gene encoding a wild-type or altered SRP) in a suitable medium until SRP is produced. In another embodiment, the method further comprises isolating SRPs from the medium or the host cell.

Another aspect of the invention pertains to isolated SRPs, and biologically active portions thereof. An "isolated" or "purified" polypeptide or biologically active portion thereof is free of some of the cellular material when produced by recombinant DNA techniques, or chemical precursors or other chemicals when chemically synthesized. The language "substantially free of cellular material" includes preparations of SRP, in which the polypeptide is separated from some of the cellular components of the cells in which it is naturally or recombinantly produced. In one embodiment, the language "substantially free of cellular material" includes preparations of a SRP having less than about 30% (by dry weight) of non-SRP material (also referred to herein as a "contaminating polypeptide'), more preferably less than about 20% of non-SRP material, still more preferably less than about 10% of non-SRP material, and most preferably less than about 5% non-PKSRP material.

When the SRP or biologically active portion thereof is recombinantly produced, it is also preferably substantially free of culture medium, i.e., culture medium represents less than about 20%, more preferably less than about 10%, and most preferably less than about 5% of the volume of the polypeptide preparation. The language "substantially free of chemical precursors or other chemicals" includes preparations of SRP in which the polypeptide is separated from chemical precursors or other chemicals that are involved in the synthesis of the polypeptide. In one embodiment, the language "substantially free of chemical precursors or other chemicals" includes preparations of a SRP having less than about 30% (by dry weight) of chemical precursors or non-SRP chemicals, more preferably less than about 20% chemical precursors or non-SRP chemicals, still more preferably less than about 10% chemical precursors or non-SRP chemicals, and most preferably less than about 5% chemical precursors or non-SRP chemicals. In preferred embodiments, isolated polypeptides, or biologically active portions thereof, lack contaminating polypeptides from the same organism from which the SRP is derived. Typically, such polypeptides are produced by recombinant expression of, for example, a Saccharomyces cerevisiae, E.coli, Brassica napus, Glycine max, or Oryza sativa SRP in plants other than Saccharomyces cerevisiae, E.coli, Brassica napus, Glycine max, or Oryza sativa, or microorganisms such as C. glutamicum, ciliates, algae or fungi.

The nucleic acid molecules, polypeptides, polypeptide homologs, fusion polypeptides, primers, vectors, and host cells described herein can be used in one or more of the following methods: identification of Saccharomyces cerevisiae, E.coli, Brassica napus, Glycine max, or Oryza sativa and related organisms; mapping of genomes of organisms related to Saccharomyces cerevisiae, E.coli, Brassica napus, Glycine max, or Oryza sativa; identification and localization of Saccharomyces cerevisiae, E.coli, Brassica napus, Glycine max, or Oryza sativa sequences of interest; evolutionary studies; determination of SRP regions required for function; modulation of a SRP activity; modulation of the metabolism of one or more cell functions; modulation of the transmembrane transport of one or more compounds; modulation of stress resistance; and modulation of expression of SRP nucleic acids.

The SRP nucleic acid molecules of the invention are also useful for evolutionary and polypeptide structural studies. The metabolic and transport processes in which the molecules of the invention participate are utilized by a wide variety of prokaryotic and eukaryotic cells; by comparing the sequences of the nucleic acid molecules of the present invention to those encoding similar enzymes from other organisms, the evolutionary relatedness of the organisms can be assessed. Similarly, such a comparison permits an assessment of which regions of the sequence are conserved and which are not, which may aid in determining those regions of the polypeptide that are essential for the functioning of the enzyme. This type of determination is of value for polypeptide engineering studies and may give an indication of what the polypeptide can tolerate in terms of mutagenesis without losing function.

Manipulation of the SRP nucleic acid molecules of the invention may result in the production of SRPs having functional differences from the wild-type SRPs. These polypeptides may be improved in efficiency or activity, may be present in greater numbers in the cell than is usual, or may be decreased in efficiency or activity.

There are a number of mechanisms by which the alteration of a SRP of the invention may directly affect stress response and/or stress tolerance. In the case of plants expressing SRPs, increased transport can lead to improved salt and/or solute partitioning within the plant tissue and organs. By either increasing the number or the activity of transporter molecules, which export ionic molecules from the cell, it may be possible to affect the salt tolerance of the cell.

The effect of the genetic modification in plants, C. glutamicum, fungi, algae, or ciliates on stress tolerance can be assessed by growing the modified microorganism or plant under less than suitable conditions and then analyzing the growth characteristics and/or metabolism of the plant. Such analysis techniques are well known to one skilled in the art, and include dry weight, wet weight, polypeptide synthesis, carbohydrate synthesis, lipid synthesis, evapotranspiration rates, general plant and/or crop yield, flowering, reproduction, seed setting, root growth, respiration rates, photosynthesis rates, etc. (Applications of HPLC in Biochemistry in: Laboratory Techniques in Biochemistry and Molecular Biology, vol. 17; Rehm et al., 1993 Biotechnology, vol. 3, Chapter III: Product recovery and purification, page 469-714, VCH: Weinheim; Belter, P.A. et al., 1988, Bioseparations: downstream processing for biotechnology, John Wiley and Sons; Kennedy, J.F. and Cabral, J.M.S., 1992, Recovery processes for biological materials, John Wiley and Sons; Shaeiwitz, J.A. and Henry, J.D., 1988, Biochemical separations, in: Ulmann's Encyclopedia of Industrial Chemistry, vol. B3, Chapter 11, page 1-27, VCH: Weinheim; and Dechow, F.J., 1989, Separation and purification techniques in biotechnology, Noyes Publications).

For example, yeast expression vectors comprising the nucleic acids disclosed herein, or fragments thereof, can be constructed and transformed into Saccharomyces cerevisiae using standard protocols. The resulting transgenic cells can then be assayed for fail or alteration of their tolerance to drought, salt, and temperature stress. Similarly, plant expression vectors comprising the nucleic acids disclosed herein, or fragments thereof, can be constructed and transformed into an appropriate plant cell such as Arabidopsis, soy, rape, maize, wheat, Medicago truncatula, etc., using standard protocols. The resulting transgenic cells and/or plants derived therefrom can then be assayed for fail or alteration of their tolerance to drought, salt, temperature stress, and lodging.

The engineering of one or more SRP genes of the invention may also result in SRPs having altered activities, which indirectly impact the stress response and/or stress tolerance of algae, plants, ciliates, or fungi, or other microorganisms like C. glutamicum. For example, the normal biochemical processes of metabolism result in the production of a variety of products (e.g., hydrogen peroxide and other reactive oxygen species), which may actively interfere with these same metabolic processes. For example, peroxynitrite is known to nitrate tyrosine side chains, thereby inactivating some enzymes having tyrosine in the active site (Groves, J.T., 1999, Curr. Opin. Chem. Biol. 3(2):226-235). While these products are typically excreted, cells can be genetically altered to transport more products than is typical for a wild-type cell. By optimizing the activity of one or more PKSRPs of the invention, which are involved in the export of specific molecules, such as salt molecules, it may be possible to improve the stress tolerance of the cell.

Additionally, the sequences disclosed herein, or fragments thereof, can be used to generate knockout mutations in the genomes of various organisms, such as bacteria, mammalian cells, yeast cells, and plant cells (Girke, T., 1998, The Plant Journal 15:39-48). The resultant knockout cells can then be evaluated for their ability or capacity to tolerate various stress conditions, their response to various stress conditions, and the effect on the phenotype and/or genotype of the mutation. For other methods of gene inactivation, see U.S. Patent No. 6,004,804 "Non-Chimeric Mutational Vectors" and Puttaraju et al., 1999, Spliceosome-mediated RNA trans-splicing as a tool for gene therapy, Nature Biotechnology 17:246-252.

The aforementioned mutagenesis strategies for SRPs resulting in increased stress resistance are not meant to be limiting; variations on these strategies will be readily apparent to one skilled in the art. Using such strategies, and incorporating the mechanisms disclosed herein, the nucleic acid and polypeptide molecules of the invention may be utilized to generate algae, ciliates, plants, fungi, or other microorganisms like C. glutamicum expressing mutated PKSRP nucleic acid and polypeptide molecules such that the stress tolerance is improved.

The present invention also provides antibodies that specifically bind to a SRP, or a portion thereof, as encoded by a nucleic acid described herein. Antibodies can be made by many well-known methods (See, e.g. Harlow and Lane, "Antibodies; A Laboratory Manual," Cold Spring Harbor Laboratory, Cold Spring Harbor, New York, (1988)). Briefly, purified antigen can be injected into an animal in an amount and in intervals sufficient to elicit an immune response. Antibodies can either be purified directly, or spleen cells can be obtained from the animal. The cells can then fused with an immortal cell line and screened for antibody secretion. The antibodies can be used to screen nucleic acid clone libraries for cells secreting the antigen. Those positive clones can then be sequenced. See, for example, Kelly et al., 1992, Bio/Technology 10:163-167; Bebbington et al., 1992, Bio/Technology 10:169-175.

The phrases "selectively binds" and "specifically binds" with the polypeptide refer to a binding reaction that is determinative of the presence of the polypeptide in a heterogeneous population of polypeptides and other biologics. Thus, under designated immunoassay conditions, the specified antibodies bound to a particular polypeptide do not bind in a significant amount to other polypeptides present in the sample. Selective binding of an antibody under such conditions may require an antibody that is selected for its specificity for a particular polypeptide. A variety of immunoassay formats may be used to select antibodies that selectively bind with a particular polypeptide. For example, solid-phase ELISA immunoassays are routinely used to select antibodies selectively immunoreactive with a polypeptide. See Harlow and Lane, "Antibodies, A Laboratory Manual," Cold Spring Harbor Publications, New York, (1988), for a description of immunoassay formats and conditions that could be used to determine selective binding.

In some instances, it is desirable to prepare monoclonal antibodies from various hosts. A description of techniques for preparing such monoclonal antibodies may be found in Stites et al., eds., "Basic and Clinical Immunology," (Lange Medical Publications, Los Altos, Calif., Fourth Edition) and references cited therein, and in Harlow and Lane, "Antibodies, A Laboratory Manual," Cold Spring Harbor Publications, New York, (1988).

Gene expression in plants is regulated by the interaction of protein transcription factors with specific nucleotide sequences within the regulatory region of a gene. A common type of transcription factor contains zinc finger (ZF) motifs. Each ZF module is approximately 30 amino acids long folded around a zinc ion. The DNA recognition domain of a ZF protein is a α-helical structure that inserts into the major grove of the DNA double helix. The module contains three amino acids that bind to the DNA with each amino acid contacting a single base pair in the target DNA sequence. ZF motifs are arranged in a modular repeating fashion to form a set of fingers that recognize a contiguous DNA sequence. For example, a threefingered ZF motif will recognize 9 bp of DNA. Hundreds of proteins have been shown to contain ZF motifs with between 2 and 37 ZF modules in each protein (Isalan M, et al., 1998 Biochemistry 37(35):12026-33; Moore M, et al., 2001 Proc. Natl. Acad. Sci. USA 98(4):1432-1436 and 1437-1441; US patents US 6007988 and US 6013453).

The regulatory region of a plant gene contains many short DNA sequences (cis-acting elements) that serve as recognition domains for transcription factors, including ZF proteins. Similar recognition domains in different genes allow the coordinate expression of several genes encoding enzymes in a metabolic pathway by common transcription factors. Variation in the recognition domains among members of a gene family facilitates differences in gene expression within the same gene family, for example, among tissues and stages of development and in response to environmental conditions.

Typical ZF proteins contain not only a DNA recognition domain but also a functional domain that enables the ZF protein to activate or repress transcription of a specific gene. Experimentally, an activation domain has been used to activate transcription of the target gene (US patent 5789538 and patent application WO9519431), but it is also possible to link a transcription repressor domain to the ZF and thereby inhibit transcription (patent applications WO00/47754 and WO2001002019). It has been reported that an enzymatic function such as nucleic acid cleavage can be linked to the ZF (patent application WO00/20622)

The invention provides a method that allows one skilled in the art to isolate the regulatory region of one or more stress related protein encoding genes from the genome of a plant cell and to design zinc finger transcription factors linked to a functional domain that will interact with the regulatory region of the gene. The interaction of the zinc finger protein with the plant gene can be designed in such a manner as to alter expression of the gene and preferably thereby alter metabolic activity to confer increased (or decreased) tolerance of abiotic stress such as drought. The invention provides a method of producing a transgenic plant with a transgene encoding this designed transcription factor, or alternatively a natural transcription factor, that modifies transcription of the Stress-Related Protein, particularly stress related protein gene to provide increased tolerance of environmental stress, which is preferably achieved by altering metabolic activity. Such a regulation of plant genes by artificial polydactyl zinc fingers has been demonstrated by Ordiz et al. (Regulation of transgene Expression in plants with polydactyl zinc finger transcription factors, Ordiz et al., PNAS, 99 (20) 13290-13295, 2002) or Guan et al. (Hertiable endogenous gene regulation in plants with designed polydactyl zinc finger transcription factos, PNAS, Vol. 99 (20), 13296-13301 (2002)).

In particular, the invention provides a method of producing a transgenic plant with a stress related protein coding nucleic acid, wherein expression of the nucleic acid(s) in the plant results in increased tolerance to environmental stress, which is preferably achieved by altering metabolic activity, as compared to a wild-type plant comprising: (a) transforming a plant cell with an expression vector comprising a stress related protein encoding nucleic acid, and (b) generating from the plant cell a transgenic plant with an increased tolerance to environmental stress as compared to a wild-type plant. For such plant transformation, binary vectors such as pBinAR can be used (Höfgen and Willmitzer, 1990 Plant Science 66:221-230). Moreover suitable binary vectors are for example pBIN19, pBI101, pGPTV or pPZP (Hajukiewicz, P. et al., 1994, Plant Mol. Biol., 25: 989-994).

Construction of the binary vectors can be performed by ligation of the cDNA into the T-DNA. 5' to the cDNA a plant promoter activates transcription of the cDNA. A polyadenylation sequence is located 3' to the cDNA. Tissue-specific expression can be achieved by using a tissue specific promoter as listed above. Also, any other promoter element can be used. For constitutive expression within the whole plant, the CaMV 35S promoter can be used. The expressed protein can be targeted to a cellular compartment using a signal peptide, for example for plastids, mitochondria or endoplasmic reticulum (Kermode, 1996 Crit. Rev. Plant Sci. 4(15):285-423). The signal peptide is cloned 5' in frame to the cDNA to archive subcellular localization of the fusion protein. Additionally, promoters that are responsive to abiotic stresses can be used with, such as the Arabidopsis promoter RD29A. One skilled in the art will recognize that the promoter used should be operatively linked to the nucleic acid such that the promoter causes transcription of the nucleic acid, which results in the synthesis of a mRNA, which encodes a polypeptide.

Alternate methods of transfection include the direct transfer of DNA into developing flowers via electroporation or Agrobacterium mediated gene transfer. Agrobacterium mediated plant transformation can be performed using for example the GV3101(pMP90) (Koncz and Schell, 1986 Mol. Gen. Genet. 204:383-396) or LBA4404 (Ooms et al., Plasmid, 1982, 7: 15-29; Hoekema et al., Nature, 1983, 303: 179-180) Agrobacterium tumefaciens strain. Transformation can be performed by standard transformation and regeneration techniques (Deblaere et al., 1994 Nucl. Acids. Res. 13:4777-4788; Gelvin and Schilperoort, Plant Molecular Biology Manual, 2nd Ed. - Dordrecht : Kluwer Academic Publ., 1995. - in Sect., Ringbuc Zentrale Signatur: BT11-P ISBN 0-7923-2731-4; Glick, B R and Thompson, J E, Methods in Plant Molecular Biology and Biotechnology, Boca Raton : CRC Press, 1993.- 360 S., ISBN 0-8493-5164-2). For example, rapeseed can be transformed via cotyledon or hypocotyl transformation (Moloney et al., 1989 Plant Cell Reports 8:238-242; De Block et al., 1989 Plant Physiol. 91:694-701). Use of antibiotics for Agrobacterium and plant selection depends on the binary vector and the Agrobacterium strain used for transformation. Rapeseed selection is normally performed using kanamycin as selectable plant marker. Agrobacterium mediated gene transfer to flax can be performed using, for example, a technique described by Mlynarova et al., 1994 Plant Cell Report 13:282-285. Additionally, transformation of soybean can be performed using for example a technique described in European Patent No. 0424 047, U.S. Patent No. 5,322,783, European Patent No. 0397 687, U.S. Patent No. 5,376,543, or U.S. Patent No. 5,169,770. Transformation of maize can be achieved by particle bombardment, polyethylene glycol mediated DNA uptake or via the silicon carbide fiber technique (see, for example, Freeling and Walbot "The maize handbook" Springer Verlag: New York (1993) ISBN 3-540-97826-7). A specific example of maize transformation is found in U.S. Patent No. 5,990,387 and a specific example of wheat transformation can be found in PCT Application No. WO 93/07256.

The stress related protein encoding nucleic acid molecules of the invention have a variety of uses. Most importantly, the nucleic acid and amino acid sequences of the present invention can be used to transform plant cells or plants, thereby inducing tolerance to stresses such as drought, high salinity, and cold. The present invention therefore provides a transgenic plant transformed by a stress related protein encoding nucleic acid (coding or antisense), wherein expression of the nucleic acid sequence in the plant results in increased tolerance to environmental stress as compared to a wild-type plant. The increased stress tolerance is apparent as an increase in the yield or quality of the plant. The transgenic plant can be a monocot or a dicot or a gymnosperm plant. The invention further provides that the transgenic plant can be selected from maize, wheat, rye, oat, triticale, rice, barley, soybean, peanut, cotton, borage, safflower, linseed, primrose, rapeseed, canola and turnip rape, manihot, pepper, sunflower, tagetes, solanaceous plant such as potato, tobacco, eggplant and tomato, Vicia species, pea, alfalfa, bushy plants, such as coffee, cacao, tea, Salix species, trees, such as oil palm, coconut, perennial grass, such as ryegrass and fescue, and forage crops, such as alfalfa and clover and Arabidopsis thaliana. Further the transgenic plant can be selected from spruce, pine, or fir for example.

In particular, the present invention describes using the expression of stress related proteins to engineer drought-tolerant, salt-tolerant, and/or coldtolerant plants. This strategy has herein been demonstrated for Arabidopsis thaliana, Ryegrass, Alfalfa, Rapeseed/Canola, Soybean, Corn and Wheat but its application is not restricted to these plants. Accordingly, the invention provides a transgenic plant containing a stress related protein encoding gene selected from the nucleic acids disclosed in the Sequence Listing and/or homologs of the afore mentioned sequences, wherein the environmental stress is drought, increased salt or decreased or increased temperature but its application is not restricted to these adverse environments. Protection against other adverse conditions such as heat, air pollution, heavy metals, and chemical toxicants, for example, may be obtained. In preferred embodiments, the environmental stress is drought.

The present invention also provides methods of modifying stress tolerance of a plant comprising, modifying the expression of a stress-related, protein-encoding gene in the plant. The invention provides that this method can be performed such that the stress tolerance is increased. This can for example be done by the use of transcription factors. In particular, the present invention provides methods of producing a transgenic plant having an increased tolerance to environmental stress as compared to a wild-type plant due to increased expression of a stress related protein in the plant.

Growing the modified plants under stress conditions and then screening and analyzing the growth characteristics and/or metabolic activity assess the effect of the genetic modification in plants on stress tolerance and/or resistance. Such analysis techniques are well known to one skilled in the art. They include next to screening (Römpp Lexikon Biotechnologie, Stuttgart/New York: Georg Thieme Verlag 1992, "screening" p. 701) dry weight, wet weight, protein synthesis, carbohydrate synthesis, lipid synthesis, evapotranspiration rates, general plant and/or crop yield, flowering, reproduction, seed setting, root growth, respiration rates, photosynthesis rates, etc. (Applications of HPLC in Biochemistry in: Laboratory Techniques in Biochemistry and Molecular Biology, vol. 17; Rehm et al., 1993 Biotechnology, vol. 3, Chapter III: Product recovery and purification, page 469-714, VCH: Weinheim; Belter, P.A. et al., 1988 Bioseparations: downstream processing for biotechnology, John Wiley and Sons; Kennedy, J.F. and Cabral, J.M.S., 1992 Recovery processes for biological materials, John Wiley and Sons; Shaeiwitz, J.A. and Henry, J.D., 1988 Biochemical separations, in: Ulmann's Encyclopedia of Industrial Chemistry, vol. B3, Chapter 11, page 1-27, VCH: Weinheim; and Dechow, F.J. (1989) Separation and purification techniques in biotechnology, Noyes Publications).

The engineering of one or more stress-related, protein-encoding genes of the invention may also result in stress-related proteins having altered activities, which indirectly impact the stress response and/or stress tolerance of plants. For example, the normal biochemical processes of metabolism result in the production of a variety of products (e.g., hydrogen peroxide and other reactive oxygen species), which may actively interfere with these same metabolic processes (for example, peroxynitrite is known to react with tyrosine side chains, thereby inactivating some enzymes having tyrosine in the active site (Groves, J.T., 1999 Curr. Opin. Chem. Biol. 3(2):226-235). By optimizing the activity of one or more stress related proteins (enzymes) of the invention, it may be possible to improve the stress tolerance of the cell.

Throughout this application, various publications are referenced. The disclosures of all of these publications and those references cited within those publications in their entireties are hereby incorporated by reference into this application in order to more fully describe the state of the art to which this invention pertains.

It should also be understood that the foregoing relates to preferred embodiments of the present invention and that numerous changes and variations may be made therein without departing from the scope of the invention. The invention is further illustrated by the following examples, which are not to be construed in any way as limiting. On the contrary, it is to be clearly understood that various other embodiments, modifications and equivalents thereof, which, after reading the description herein, may suggest themselves to those skilled in the art without departing from the spirit of the present invention and/or the scope of the claims.

The invention also pertains to the use of SRP encoding nucleic acid selected form the group comprising a nucleic acid disclosed in the Sequence Listing and/or homologs of the afore mentioned sequences for preparing a plant cell with increased environmental stress tolerance, which is preferably achieved by altering metabolic activity. The said sequences can also be used for preparing a plant with increased environmental stress tolerance.

Object of the invention is further the use of altered metabolic activity and/or a SRP encoding nucleic acid selected form the group of sequences disclosed in the Sequence Listing and/or homologs of the afore mentioned sequences or parts thereof as markers for selection of plants with increased tolerance to environmental stress or as markers for detection of stress in plants or plant cells.

Example 1: Engineering stress-tolerant Arabidopsis plants by overexpressing stress related protein genes.

Gene cloning and transformation of Arabidopsis thaliana - Amplification. The standard protocol of Pfu DNA polymerase or a Pfu/Taq DNA polymerase mix (Herculase) was used for the amplification procedure. Amplified ORF fragments were analyzed by gel electrophoresis. Each primer consists of a universal 5' end and ORF specific 3' end, whereby the universal sequences differ for the forward and reverse primers (forward primer sequence contains an EcoRl for yeast or Smal for E.coli, and the reverse primer sequence a Smal for yeast or Sacl for E.coli restriction site) allowing generally a unidirectional cloning success.

Amplification using the protocol of Pfu or Herculase DNA polymerase (Stratagene). Conditions: 1x PCR buffer, 0.2 mM dNTP, 100 ng genomic DNA Saccharomyces cerevisiae (S288C) or 60 ng genomic DNA Escherichia coli K-12 (MG1655), 25 pmol forward primer, 25 pmol reverse primer, 2.5 u Pfu or Herculase DNA polymerase. 1st cycle for 3' for yeast of 2' for E.coli at 94°C, followed by 25 cycles for 30" at 94°C, 30" 55°C for yeast or 60°C for E.coli and 5-6' 72°C, followed by 1 cycle for 610' at 72°C, final for 4°C at ∞.

**Table 2: Forward and reverse primer sequences used for ORF amplification (SEQ ID NOS 257-508, in order of appearance).**

| Gene | Forward Seq |
|---|---|
| b0025 | ATGAAGCTGATACGCGGCATAC |
| b0061 | ATGTTAGAAGATCTCAAACGCCA |
| b0141 | ATGTCTAAAAAATTAGGTTTTGCCCT |
| b0144 | ATGCTCCCGCCTTATTTTTTGTTC |
| b0148 | ATGCTACAATGTGGCGCGAAGAA |
| b0318 | ATGAGTATAAAAAATCTACCTGCCG |
| b0328 | ATGATGCAGTTAGTTCACTTATTTATG |
| b0361 | ATGGATAGCGCACGCGCCCTTA |
| b0723 | ATGAAATTGCCAGTCAGAGAATTTG |
| b0726 | ATGCAGAACAGCGCTTTGAAAGC |
| b0880 | ATGGAAAAGGGTACTGTTAAGTG |
| b1069 | ATGAATTTATTAAAATCGCTGGCCG |
| b1080 | ATGATTAAATTTCTCTCTGCATTAATTC |
| b1130 | ATGCGCGTACTGGTTGTTGAAGA |
| b1221 | ATGAGTAATCAGGAACCGGC |
| b1350 | ATGAGCACAAAACCACTCTTCCTG |
| b1392 | ATGACAACGTTTCATTCCTTAAC |
| b1557 | ATGTCAAATAAAATGACTGGTTTAGTA |
| b1650 | ATGTCATCTGAAAAACTGTATTCCC |
| b1717 | ATGCCAAAAATTAAGACCGTACGC |
| b1779 | ATGACTATCAAAGTAGGTATCAACG |
| b2164 | ATGGATATAATGAGAAGTGTTGTGG |
| b2194 | ATGAGGTTTTTATTGGGCGTGCTG |
| b2197 | ATGAATATTCGCCGTAAAAACCG |
| b2231 | ATGAGCGACCTTGCGAGAGAAAT |
| b2246 | ATGACAACGGTAACGCCCACATAT |
| b2287 | ATGGATTATACGCTCACCCGCAT |
| b2488 | ATGCTGAAGTTACTGAAAACTATTATG |
| b2699 | ATGGCTATCGACGAAAACAAACAG |
| b2733 | ATGAGTGCAATAGAAAATTTCGA |
| b2886 | ATGATGAAATCGTTAATTATCGTTAATC |
| b2926 | ATGTCTGTAATTAAGATGACCG |
| b2980 | ATGAAAGATGAACGTCGCCCTATT |
| b2987 | ATGCTAAATTTATTTGTTGGCCTTGA |
| b3030 | ATGACGCAAACTTATAACGCTG |
| b3066 | ATGGCTGGACGAATCCCACGC |
| b3067 | ATGGAGCAAAACCCGCAGTCAC |
| b3358 | ATGTGGCGCAGACTGATTTATCAC |
| b3604 | ATGATTGTTTTACCCAGACGCCTG |
| b3687 | ATGCGTAACTTTGATTTATCCCCG |
| b3811 | ATGACCGATTTACACACCGATGTA |
| b4074 | ATGTTGACCCCGTTGACGGCCT |
| b4090 | ATGAAAAAGATTGCATTTGGCTGTG |
| b4351 | ATGACGGTTCCTACCTATGACAAA |
| YAL005C | ATGTCAAAAGCTGTCGGTATTGATT |
| YAL025C | ATGTCCGACGAAATTGTTTGGCAA |
| YAL047C | ATGGTACGTCGATGGATTCCTAGT |
| YAR028W | ATGCAAACACCTTCAGAAAATACCG |
| YAR068W | ATGCCACAAGTACAGTCGTGGTTT |
| YBL021C | ATGAATACCAACGAGTCCGAACAT |
| YBL100C | ATGTTGTTCAAACCAAAAACACGAG |
| YBL109W | ATGTCCCTACGGCCTTGTCTAAC |
| YBR004C | ATGATTGTGGGGTTGACACTTTATT |
| YBR101C | ATGGAAAAGCTATTACAGTGGTCTA |
| YBR128C | ATGCATTGCCCAATTTGCCACCAT |
| YBR176W | ATGAATATAATGAAAAGACAATTATGCA |
| YBR204C | ATGAATATGGCAGAACGTGCAGAA |
| YBR243C | ATGTTGCGACTTTTTTCACTGGCA |
| YDL199C | ATGAAACCTCCGTTAAACATGTCAC |
| YDL212W | ATGTTCTCATATTCAGATTTCTGTTC |
| YDL242W | ATGAATTTAGAAGAGAGCCAATCAAA |
| YDL248W | ATGAAAGAGAATGAAGTCAAAGATGA |
| YDR446W | ATGACTGTTATAAAGACAGAACCAAC |
| YDR467C | ATGATTTATATGTTGGTTTTTCTGGAT |
| YDR476C | ATGTGGGATTCGTTAATTGTAAGCA |
| YDR524C | ATGGATTTTTATACTACTGATATCAAC |
| YDR526C | ATGCCTTGCTTGTTGCCACCTAC |
| YEL020W-A | ATGGACGCATTGAACTCCAAAGAAC |
| YEL059W | ATGAGTCTTTCTTTCCTTCTCTTTTC |
| YER075C | ATGAAGGACAGTGTAGACTGCCC |
| YER085C | ATGAATTTCAAGGAGCCGCTAGTG |
| YER148W | ATGGCCGATGAGGAACGTTTAAAG |
| YER159C | ATGGCAGATCAAGTACCAGTTACAA |
| YER185W | ATGTCCACAACTGATTCGGGGTT |
| YFL029C | ATGAAACTGGATAGTATAGACATTAC |
| YFL038C | ATGAATAGCGAGTACGATTACCTGT |
| YGL028C | ATGATTTCACCCATAAGCTTTCTATC |
| YGL186C | ATGAATAGGGACAACATGGACACAA |
| YGL196W | ATGGAAGAAGTGAAAGCAGTCAATT |
| YGL217C | ATGAGCATTCTATCATCCACACAAT |
| YGR006W | ATGGACCTAGATCTAGCCAGTATC |
| YGR070W | ATGAATAGTAATGAACTGGATCTAAG |
| YGR111W | ATGACAGCCTCATCAAATGACGAT |
| YGR182C | ATGTCTCGCCAAAGCGCATTCAAA |
| YHR005C-A | ATGTCTTTCTTAGGTTTCGGTGGT |
| YHR169W | ATGGCAGACTTTAAATCTTTAGGTC |
| YHR203C | ATGGCTAGAGGACCGTATGTTGAT |
| YIL012W | ATGTGCCTAGGTAAGCTGTACTTC |
| YIL046W | ATGAGGAGAGAGAGGCAAAGGAT |
| YIL059C | ATGAATTTCTCCACAGTTTTTCAAGC |
| YIL096C | ATGGCCAGGAAATTGAAGGGTAAG |
| YIL100W | ATGTATATATATGTTGAAGTGTGTACA |
| YIL128W | ATGACACCAGACGAACTAAATTCAG |
| YIL166C | ATGTCCGTACAAAAAGAAGAATACG |
| YIL172C | ATGACTATTTCTTCTGCACATCCAG |
| YJL010C | ATGGGAAAGACTAAAACAAGAGGCA |
| YJL021C | ATGCCAAATACTGCTCCGCCACT |
| YJL038C | ATGAGGTTCCAGCTTTTCATATATTT |
| YJL151C | ATGGACAGAGACCATATTAATGACC |
| YJR034W | ATGGTAGCTAGTTGTAAAGATCAGA |
| YJR078W | ATGAACAACACTTCCATAACCGGA |
| YJR153W . | ATGATTTCTGCTAATTCATTACTTATTT |
| YKL026C | ATGCAAGAATTTTATTCTTTTTCACCA |
| YKL047W | ATGAATTCCGGAGGTGAAGAACCA |
| YKL075C | ATGGCTAAAGATTTATTGCCCAAGC |
| YKL081W | ATGTCCCAAGGTACTTTATACATTAA |
| YKL096W-A | ATGCAATTCTCTACTGTCGCTTCC |
| YLR104W | ATGAGTCAAAGTCGCTGGAGTATC |
| YLR128W | ATGGTATGTTTTTAATTTCGTTTCTTTA |
| YLR354C | ATGTCTGAACCAGCTCAAAAGAAAC |
| YLR406C | ATGGCCGGTTTAAAAGACGTTGTC |
| YLR414C | ATGAGGAATTTTTTCACGTTATTTTTTG |
| YML090W | ATGCTTGTTTTTTCTTTTCTGTTTGTT |
| YMR217W | ATGGCTGCCGGTGAACAAGTTTC |
| YNL010W | ATGGTCAAAGCTGTTATTTTTACCG |
| YNL065W | ATGTCACGAAGTAACAGTATATACAC |
| YNL206C | ATGTCAAAATTGTTCTTAGATGAACTG |
| YNL276C | ATGTGTGGTATCTATCCCTACCAG |
| YNL279W | ATGAGCGGTTTTAAATGCTATTTGC |
| YNR015W | ATGGTTACATATGCTGGAAAACTGG |
| YOL055C | ATGACCTATTCTACAGTTAGCATCA |
| YPL091 W | ATGCTTTCTGCAACCAAACAAACAT |
| YPL151C | ATGGACGGAAATGATCACAAAGTC |
| YPL197C | ATGAAGAGAAATATTATTTATTATACATTG |
| YPL224C | ATGCTACGGATAAGTATTGACTCTA |
| YPR055W | ATGGATTACCTAAAACCAGCGCAG |
| | |

| **Gene** | Reverse Seq |
|---|---|
| b0025 | TTAAGCCGGTTTTGTTAGCCCAAA |
| b0061 | TTACTGCCCGTAATATGCCTTCGC |
| b0141 | TTACTGATAAGTAATGGTGTATGCAG |
| b0144 | TTAGCATGACGCATTTGAGAATGTTG |
| b0148 | TTACGAATACTTTTTCGTCCGTCGC |
| b0318 | TTATGCACCTGCGGCAATCAGTA |
| b0328 | TTACCGCTGCGTCACCCCTTCG |
| b0361 | TTATATTTCCAGACATCTGTTATCACT |
| b0723 | TTAGTAAGTACGAATCTTCGGCGG |
| b0726 | TTATTCGACGTTCAGCGCGTCAT |
| b0880 | TTATGCGACTGCCGCTTCTACTTC |
| b1069 | TTACACCGTCCGGCGGGCA |
| b1080 | TTAGATGATTTCCAGTTTTGCCCGC |
| b1130 | TTAGCGCAATTCGAACAGATAGCCC |
| b1221 | TTAGAAAATGCGCTCCTGATGCACC |
| b1350 | TTAGTCATTTGCATATTCCTTAGCC |
| b1392 | TTATGCCATCCCCTTCGCGTCAAA |
| b1557 | TTAATCAGTAATGATGACATTTGCTG |
| b1650 | TTACAACGTCGGGTAATCGGTATA |
| b1717 | TTATGCGTACGGCAGGCACGC |
| b1779 | TTATTTGGAGATGTGAGCGATCAG |
| b2164 | TTACGCCAGACCAATAAAGAACCC |
| b2194 | TTATTTACTCTCCTGCGGCGACA |
| b2197 | TTATGATGCTGGGTCCTTATAAACAC |
| b2231 | TTATTCTTCTTCTGGCTCGTCGTC |
| b2246 | TTAATGATGTGCCACGTCGGTCT |
| b2287 | TTAAATCTCGTCAGGTGTACGCAG |
| b2488 | TTATAGCTGCTCCTTAGCCACCAG |
| b2699 | TTAAAAATCTTCGTTAGTTTCTGCTAC |
| b2733 | TTACACCAGGCTCTTCAAGCGAT |
| b2886 | TTAGAGAGGATTCTCACCGCTGGC |
| b2926 | TTACTTCTTAGCGCGCTCTTCGA |
| b2980 | TTAACTCAGGTTCATCTCCAGCGG |
| b2987 | TTAAATCAACTGCAATGCTATCCA |
| b3030 | TTAAACCTCAATCTCCGCCATGTC |
| b3066 | TTACTTTTTCGCCAGCTCCTGGTTT |
| b3067 | TTAATCGTCCAGGAAGCTACGCA |
| b3358 | TTACGCCTTCGAATCCCGCAAC |
| b3604 | TTATGCGTTTTTCTCCCTCGAATGC |
| b3687 | TTAGTTGATTTCGATACGGCGCG |
| b3811 | TTATTTCCCCCGTTTGGCGCGT |
| b4074 | TTATTCATCGCGCTTCTTCCCCC |
| b4090 | TTAATTTCTCCGCTGCTCTATTGCC |
| b4351 | TTACTCAAAATAGTCCATATCCAGTTTC |
| YAL005C | TTAATCAACTTCTTCAACGGTTGGA |
| YAL025C | TTATTGTGCCACTTCTTGCTCAGC |
| YAL047C | TTAGGGATTGTTGATTGATAGGTTG |
| YAR028W | TCAAGGTAAATCAGCGTCAGGATAT |
| YAR068W | TCAAACCAGCAGCCATACCTTCG |
| YBL021C | TCAAGGCACCTCTTCGTCGTCC |
| YBL100C | TCAGCTCTCATAACTATCGCAAGAA |
| YBL109W | TCAATATGGAGGGTAGAACAACAGT |
| YBR004C | TCAGGCTGGTGGTAAAAAAGCTG |
| YBR101C | TCATAATACATACTTTACGGCTAAATA |
| YBR128C | CTAGCCTACCACGTACCATCGG |
| YBR176W | TTACTTTTCGTTAATTGAAGAGAGGA |
| YBR204C | CTACAATTTGGAATTATCAATCACCT |
| YBR243C | TCAACGTACTGTCCATAGGTTGTC |
| YDL199C | CTAGGCAACTGCCATGTCTCCA |
| YDL212W | CTATTTTTTGGCGTTCTTCCTCTTT |
| YDL242W | TCAAATTTTGAAGCCGAAGTTGGTA |
| YDL248W | TCATTTCTTCATTAAAGACTCTTCATT |
| YDR446W | TTATATAATATTGAGAATTTCATTAGCC |
| YDR467C | TCAAATCAAAACAACAGGATATCGAC |
| YDR476C | CTAATTAACTGACCCTTCGCCAAG |
| YDR524C | TTATTTTTTGTCCTTTTTGGCCAATTT |
| YDR526C | TCACTCTGCCACCATCGTAATGG |
| YEL020W-A | TTATCGGCCCAAGCCTTGTCCCA |
| YEL059W | TTAACTGTAGGCTGTGCCGACAT |
| YER075C | CTATTGTGGCAATTCTTTCAACTTAT |
| YER085C | TTAATTAATAGTCTTCTTCTTTCTTATC |
| YER148W | TCACATTTTTCTAAATTCACTTAGCAC |
| YER159C | TCAGGCACTCTCTTCCTCCGGT |
| YER185W | CTAATTACTTAACGTTTGACACTCC |
| YFL029C | TTATGGCTTTTCTAATTCTTGCAAGA |
| YFL038C | TCAACAGCAGCCCCCACCGGT |
| YGL028C | TTATGGAAAATATTCAATCACACCGA |
| YGL186C | TTAACGACCAAATTTACGTAGCTCT |
| YGL196W | CTAATTTAATGGAGTAGTTTCGTTCC |
| YGL217C | TTAACTACTTGAGTTTTCTTTCCAGC |
| YGR006W | TTATGTTAGCAGCATTCCGGCCT |
| YGR070W | TCAAAAATTTAACAGTTCGATAATTTCA |
| YGR111 W | TTAAAACCAACAGAATTTGGTATTGTT |
| YGR182C | TCAAAGGCTTACATCTCCAGAATC |
| YHR005C-A | CTAAAACTTACCGGCTGCGTTAAAT |
| YHR169W | TCAAGAGCGCAAACTTTTCCCGT |
| YHR203C | TTACAAACCTTGTTGAGCTCTTCTT |
| YIL012W | CTACACACGTTTCCAACCTCTGC |
| YIL046W | CTAATCATTGAGATCGAATTTGTACA |
| YIL059C | TCACATACATACCTGCGGTAGCAA |
| YIL096C | TTAGTCTGAATCAGAATCTGTGTCT |
| YIL100W | TTATTCACCCTCACCCCTAATACC |
| YIL128W | TTACTCGAACGGGATTTGGCCTAA |
| YIL166C | CTAGTGAATAAACCTATAATCAAGAC |
| YIL172C | TCATTCAGATATGTAAATTCTGCCC |
| YJL010C | TTATCTATAGTGCTTTTGCTTTTTGAA |
| YJL021C | TTACCAACCTACGTATCCTCTCG |
| YJL038C | TTATTGAATAGCATTATATATTAGTGCC |
| YJL151C | CTAAGTACGGCCGGAAGAGAGC |
| YJR034W | TCAGTCCTTCTGCTGGCTGTTCA |
| YJR078W | TCAATTTTTATCTTCATTTTTAATGTCC |
| YJR153W | TTAACAGCTTGCACCAGATCCAGA |
| YKL026C | TTAAATCTGTTCTTCTGGTGGTTGA |
| YKL047W | TTAACAGAGATTTCCCAAACATTCTG |
| YKL075C | TTATATCCATTTCTGTCTTGCAGCA |
| YKL081 W | TTATTTCAAAACCTTACCGTCAACAA |
| YKL096W-A | TTATAACAACATAGCAGCAGCAGCT |
| YLR104W | TCAATGGTTTTTGTAAGCATCTGATA |
| YLR128W | TTATTGCTGATCCTGTAAGCACTCA |
| YLR354C | TTAAGCGGTAACTTTCTTTTCAATCA |
| YLR406C | TTAAGCATCTTCTTCAACGACAACG |
| YLR414C | TCAAATCAATGGTTTTTCCTCAATTG |
| YML090W | TCAATTACAAATCCATGGATTAAGTTG |
| YMR217W | TCATTCCCATTCAACAGTTGCAGG |
| YNL010W | CTAATTTTCCATCAATTCAGCGACT |
| YNL065W | TTAATTATGATTATCGTTCTGGTCTC |
| YNL206C | TCAATCGTATTCTACTCCGGATCC |
| YNL276C | TTACATTGCCATCACACGTAAACTG |
| YNL279W | TCAGTCAAAGGTGGCTTTGCGAA |
| YNR015W | TTATATATCTGTGGGAAGGGGTACA |
| YOL055C | CTACTCGTATTCTAGCGCAGCG |
| YPL091W | TCATCTCATAGTAACCAATTCTTCTG |
| YPL151C | CTAAAATCTTTTGGCGCTTAAGTTG |
| YPL197C | CTATTGAAGTTGATTGAACCATACGT |
| YPL224C | TTACATCAACAAACTCGACGTCCA |
| YPR055W | TCATTTTTCGTTTGCAGTATGGACA |

Vector preparation. The preferred binary vector 1bxbigResgen for yeast and 1bxSuperCoLic for E.coli, which is based on the modified pPZP binary vector backbone (comprising the kanamycin-gene for bacterial selection; Hajukiewicz, P. et al., 1994, Plant Mol. Biol., 25: 989-994) carried the selection marker bar-gene (De Block et al., 1987, EMBO J. 6, 2513-2518) driven by the mas1 promoter (Velten et al., 1984, EMBO J. 3, 2723-2730; Mengiste, Amedeo and Paszkowski, 1997, Plant J., 12, 945-948) on its T-DNA. In addition the T-DNA contained the strong double 35S(Kay et al., 1987, Science 236, 1299-1302) for yeast or Super promoter (Ni et al., 1995, Plant Journal 7, 661-676) for E.coli in front of a cloning cassette followed by the nos-terminator (Depicker A. Stachel S. Dhaese P. Zambryski P. Goodman HM. Nopaline synthase: transcript mapping and DNA sequence. Journal of Molecular & Applied Genetics. 1(6):561-73, 1982.). The cloning cassette consists of the following sequence:

'Yeast: 5'-GGAATTCCAGCTGACCACCATGGCAATTCCCGGGGATC-3 (SEQ ID NO: 533) or

E.coli: 5'-TTG CTC TTC CAT GGC AAT GAT TAA TTA ACG AAG AGC AA-3' (SEQ ID NO: 534), respectively.

Other selection marker systems, like the AHAS marker or other promoters, e.g. superpromoter (see above), 35S promoter (see above), Ubiquitin promoter (Callis et al., J. Biol. Chem., 1990, 265: 12486-12493; US 5,510,474; US 6,020,190; Kawalleck et al., Plant. Molecular Biology, 1993, 21: 673-684) or 34S promoter (GenBank Accession numbers M59930 and X16673) were similar useful for the instant invention and are known to a person skilled in the art. The vector was linearised with EcoR and Smal for yeast or Smal and Sacl for E.coli using the standard protocol provided by the supplier (MBI Fermentas, Germany) and purified using Qiagen columns (Qiagen, Hilden, Germany).

Ligation and transformation. Present ORF fragments (- 100 ng) were digested by EcoRI and Smal for yeast and Smal and Sacl for E.coli using the standard protocol provided by the supplier (MBI Fermentas, Germany), purified using Qiagen columns (Qiagen, Hilden, Germany) and were ligated into the cloning cassette of the binary vector systems (- 30 ng) using standard procedures (Maniatis et al.).

In the case of internal EcoRI, Smal and Sacl restriction sites a blunt end cloning procedure was applied. The undigested ORF fragments were directly purified and ligated into the cloning cassette of the binary vector. In this case the EcoRI site was refilled by Klenow reaction and the Sacl site blunted Pfu DNA polymerase.

Ligation products were transformed into E.coli (DH5alpha) using a standard heat shock protocol (Maniatis et al.). Transformed colonies were grown on LB media and selected by respective antibiotica (Km) for 16h at 37°C. Positive clones were identified by control PCR reactions using a combination of a vector specific and the respective ORF-specific primers.

Plasmid preparation. Plasmid DNA was prepared from positive clones using standard protocols (Qiagen Hilden, Germany).

Transformation of Agrobacteria. Plasmids were transformed into Agrobacterium tumefaciens (GV3101pMP90; Koncz and Schell, 1986, Mol. Gen. Genet. 204: 383-396) using heat shock or electroporation protocols. Transformed colonies were grown on YEP media and selected by respective antibiotics (Rif/Gent/Km) for 2d at 28C. These Agrobacterium cultures were used for the plant transformation.

Arabidopsis thaliana was grown and transformed according to standard conditions Bechtold 1993 (Bechtold, N., Ellis, J., Pelletier, G. 1993. In planta Agrobacterium mediated gene transfer by infiltration of Arabidopsis thaliana plants C.R. Acad.Sci.Paris. 316:1194-1199); Bent et al. 1994 (Bent, A., Kunkel, B.N., Dahlbeck, D., Brown, K.L., Schmidt, R., Giraudat, J., Leung, J., and Staskawicz, B.J. 1994; PPCS2 of Arabidopsis thaliana: A leucin-rich repeat class of plant disease resistant genes; Science 265: 1856-1860).

Transgenic A. thaliana plants were grown individually in pots containing a 4:1 (v/v) mixture of soil and quartz sand in a York growth chamber. Standard growth conditions were: photoperiod of 16 h light and 8 h dark, 20°C, 60% relative humidity, and a photon flux density of 150 µE. To induce germination, sown seeds were kept at 4°C, in the dark, for 3 days. Plants were watered daily until they were approximately 3 weeks old at which time drought was imposed by withholding water. Concurrently, the relative humidity was reduced in 10% increments every second day to 20%. After approximately 12 days of withholding water, most plants showed visual symptoms of injury, such as wilting and leaf browning, whereas tolerant plants were identified as being visually turgid and healthy green in color. Plants were scored for symptoms of drought injury in comparison to neighbouring plants for 3 days in succession.

Three successive experiments were conducted. In the first experiment, 10 independent T2 lines were sown for each gene being tested. The percentage of plants not showing visual symptoms of injury was determined. In the second experiment, the lines that had been scored as tolerant in the first experiment were put through a confirmation screen according to the same experimental procedures. In this experiment, plants of each tolerant line were grown and treated as before. In the third experiment, at least 7 replicates of the most tolerant line were grown and treated as before. The average and maximum number of days of drought survival, after wild-type control had visually died, were determined.

In the first experiment, after 12 days of drought, the control, non-transgenic Arabidopsis thaliana and most transgenic lines expressing other transgenes in the test showed extreme visual symptoms of stress including necrosis and cell death. Several plants expressing the genes retained viability as shown by their turgid appearance and maintenance of green color.

The second experiment compared a smaller number of independent transgenic lines for each gene but a greater number of progeny within each independent transformation event. This experiment confirmed the previous results. Those lines containing the specific SRP encoding yeast genes survived longer than the controls. The results are summmarised in table 3.

In a third experiment, some lines were tested with multiple replicates. The average number of days the plants of the major line survived longer than wild type was measured, i.e., the number '1' means that, on average, the plants overexpressing this ORF, survived 1 day longer than wild-type. The value for WT in this column is '0'. The results are summmarised in table 4.

**Table 3: Drought tolerance of transgenic Arabidopsis thaliana expressing the various SRP encoding genes from Saccharomyces cerevisiae or E. coli after imposition of drought stress on 3week-old plants. Drought tolerance is measured for the indicated number of transgenic plants (Plants tested) as the average number of days (Average days of survival after WT) that the transgenic plants survived after the control (untransformed wild type) had died.**

| GENE | number of tested plants per line | number of tested lines per ORF | Average days of survival after WT |
|---|---|---|---|
| b0025 | 19 | 2 | 0.6 |
| b0061 | 19 | 2 | 0.9 |
| b0141 | 28 | 3 | 0.5 |
| b0144 | 30 | 3 | 0.8 |
| b0148 | 29 | 3 | 0.4 |
| b0318 | 10 | 1 | 0.5 |
| b0328 | 20 | 2 | 0.4 |
| b0361 | 10 | 1 | 0.5 |
| b0723 | 29 | 3 | 0.6 |
| b0726 | 29 | 3 | 0.4 |
| b0880 | 29 | 3 | 0.4 |
| b1069 | 29 | 3 | 0.4 |
| b1080 | 20 | 2 | 0.9 |
| b1130 | 29 | 3 | 1.1 |
| b1221 | 25 | 3 | 0.4 |
| b1350 | 30 | 3 | 0.6 |
| b1392 | 19 | 2 | 0.4 |
| b1557 | 30 | 3 | 0.8 |
| b1650 | 10 | 1 | 0.7 |
| b1717 | 30 | 3 | 0.4 |
| b1779 | 30 | 3 | 1.0 |
| b2164 | 30 | 3 | 0.5 |
| b2194 | 30 | 3 | 0.3 |
| b2197 | 10 | 1 | 0.3 |
| b2231 | 30 | 3 | 0.9 |
| b2246 | 30 | 3 | 0.5 |
| b2287 | 10 | 1 | 0.4 |
| b2488 | 10 | 1 | 0.2 |
| b2699 | 28 | 3 | 0.5 |
| b2733 | 30 | 3 | 0.7 |
| b2886 | 29 , | 3 | 0.3 |
| b2926 | 29 | 3 | 0.4 |
| b2980 | 20 | 2 | 0.7 |
| b3030 | 30 | 3 | 0.6 |
| b3066 | 30 | 3 | 0.3 |
| b3067 | 30 | 3 | 0.6 |
| b3604 | 20 | 3 | 0.7 |
| b3687 | 19 | 2 | 1.4 |
| b3811 | 30 | 3 | 0.4 |
| b4074 | 29 | 3 | 0.6 |
| b4090 | 10 | 1 | 1.5 |
| b4351 | 28 | 3 | 0.5 |
| YAL005C | 11 | 2 | 1.7 |
| YAL025C | 29 | 3 | 0.3 |
| YAL047C | 29 | 3 | 0.6 |
| YAR028W | 20 | 2 | 0.3 |
| YAR068W | 16 | 2 | 0.3 |
| YBL021C | 10 | 1 | 0.4 |
| YBR101C | 15 | 2 | 0.5 |
| YBR128C | 19 | 2 | 1.1 |
| YBR176W | 7 | 1 | 0.7 |
| YBR204C | 25 | 3 | 0.2 |
| YBR243C | 10 | 1 | 0.9 |
| YDL199C | 5 | 1 | 0.6 |
| YDL212W | 30 | 3 | 0.3 |
| YDL242W | 16 | 2 | 0.3 |
| YDL248W | 10 | 1 | 0.3 |
| YDR446W | 18 | 2 | 0.9 |
| YDR467C | 19 | 2 | 0.3 |
| YDR476C | 29 | 3 | 0.4 |
| YDR524C | 4 | 2 | 1.0 |
| YDR526C | 30 | 3 | 0.7 |
| YEL020W-A | 5 | 2 | 2.8 |
| YEL059W | 6 | 1 | 1.2 |
| YER075C | 27 | 3 | 0.6 |
| YER085C | 26 | 3 | 0.5 |
| YER148W | 31 | 4 | 0.5 |
| YER159C | 4 | 1 | 0.8 |
| YER185W | 27 | 2 | 0.7 |
| YFL029C | 10 | 1 | 0.7 |
| YFL038C | 30 | 3 | 0.3 |
| YGL028C | 29 | 3 | 1.0 |
| YGL186C | 10 | 1 | 0.2 |
| YGL196W | 10 | 1 | 0.3 |
| YGL217C | 10 | 1 | 1.3 |
| YGR006W | 7 | 1 | 0.4 |
| YGR070W | 20 | 2 | 0.3 |
| YGR111W | 20 | 2 | 0.4 |
| YGR182C | 9 | 1 | 0.2 |
| YHR005C-A | 30 | 3 | 0.3 |
| YHR169W | 18 | 2 | 0.8 |
| YHR203C | 23 | 3 | 0.4 |
| YIL012W | 20 | 3 | 0.6 |
| YIL046W | 22 | 3 | 0.7 |
| YIL059C | 17 | 2 | 1.9 |
| YIL096C | 3 | 1 | 1.3 |
| YIL100W | 28 | 4 | 1.2 |
| YIL128W | 27 | 3 | 0.7 |
| YIL166C | 37 | 4 | 0.4 |
| YIL172C | 20 | 2 | 0.9 |
| YJL021C | 25 | 3 | 0.4 |
| YJL038C | 30 | 3 | 0.2 |
| YJL151C | 27 | 3 | 0.5 |
| YJR034W | 28 | 3 | 0.4 |
| YJR078W | 17 | 2 | 0.2 |
| YJR153W | 18 | 3 | 0.8 |
| YKL026C | 16 | 2 | 0.3 |
| YKL047W | 40 | 5 | 0.3 |
| YKL075C | 10 | 2 | 0.9 |
| YKL081W | 13 | 2 | 0.4 |
| YKL096W-A | 24 | 3 | 0.3 |
| YLR104W | 35 | 4 | 0.9 |
| YLR128W | 27 | 3 | 0.6 |
| YLR354C | 25 | 3 | 1.0 |
| YLR406C | 18 | 3 | 0.4 |
| YLR414C | 23 | 3 | 0.5 |
| YML090W | 26 | 3 | 0.3 |
| YMR217W | 15 | 2 | 0.5 |
| YNL010W | 17 | 3 | 0.9 |
| YNL065W | 30 | 3 | 0.7 |
| YNL206C | 16 | 3 | 0.3 |
| YNL276C | 7 | 2 | 1.0 |
| YNL279W | 25 | 3 | 0.4 |
| YNR015W | 24 | 2 | 0.9 |
| YOL055C | 22 | 3 | 0.8 |
| YPL197C | 29 | 3 | 0.4 |
| YPL224C | 30 | 3 | 0.3 |
| YPR055W | 28 | 3 | 0.1 |

**Table 4: Drought tolerance of transgenic Arabidopsis thaliana expressing selected SRP encoding genes from Saccharomyces cerevisiae or E. coli after imposition of drought stress on 3 week old plants in a third experiment. Drought tolerance is measured for the indicated number of transgenic plants (Plants tested) as the average number of days (Average days of survival after WT) that the transgenic plants survived after the control (untransformed wild type) had died.**

| GENE | number of tested plants per line | number of tested lines per ORF | Average days of survival after WT |
|---|---|---|---|
| b2699 | 59 | 12 | 2.6 |
| b2987 | 122 | 12 | 1.5 |
| b3358 | 56 | 5 | 2.8 |
| YAL005C | 36 | 8 | 1.6 |
| YBL100C | 45 | 9 | 2.0 |
| YBL109W | 44 | 7 | 1.4 |
| YBR004C | 27 | 9 | 1.5 |
| YBR204C | 38 | 8 | 1.7 |
| YDR524C | 6 | 3 | 1.8 |
| YER185W | 31 | 7 | 1.6 |
| YFL029C | 68 | 5 | 0.9 |
| YGR070W | 72 | 10 | 1.0 |
| YIL172C | 46 | 10 | 1.4 |
| YJL010C | 40 | 7 | 2.6 |
| YJL151C | 46 | 9 | 2.8 |
| YJR078W | 6 | 3 | 2.2 |
| YJR153W | 26 | 2 | 1.5 |
| YMR217W | 39 | 8 | 1.2 |
| YNR015W | 39 | 7 | 2.4 |
| YPL091W | 37 | 6 | 1.7 |
| YPL151C | 26 | 8 | 2.0 |
| YPR055W | 47 | 10 | 0.8 |

## Claims

1. A transgenic plant with increased tolerance and/or resistance to an environmental stress as compared to a corresponding wild-type plant wherein the tolerance and/or resistance to an environmental stress is increased by transformation of a plant with a nucleic acid molecule, wherein the nucleic acid molecule expressed in the transgenic plant is selected from the group consisting of
a. a nucleic acid molecule encoding the polypeptide of SEQ ID NO: 27 ;
b. a nucleic acid molecule comprising the nucleic acid molecule SEQ ID NO. 153;
c. a nucleic acid molecule whose sequence can be deduced from a polypeptide sequence encoded by a nucleic acid molecule of (a) or (b) as a result of the degeneracy of the genetic code ;
d. a nucleic acid molecule which encodes a polypeptide which has at least 70% identity with the amino acid sequence of the polypeptide encoded by the nucleic acid molecule of (a) to (c) ;
e. a nucleic acid molecule which hybridizes with a nucleic acid molecule of (a) to (c) under stringent hybridisation conditions ;
f. a nucleic acid molecule which encompasses a nucleic acid molecule which is obtained by amplifying nucleic acid molecules from a cDNA library or a genomic library using the primers of SEQ ID NO. 279 and SEQ ID NO. 405 ;
g. a nucleic acid molecule encoding a polypeptide which is isolated with the aid of monoclonal antibodies against a polypeptide encoded by one of the nucleic acid molecules of (a) to (c) ; and
h. a nucleic acid molecule which is obtainable by screening a suitable nucleic acid library under stringent hybridization conditions with a probe comprising one of the sequences of the nucleic acid molecule of (a) to (g) or with a fragment thereof having 20 nt of the nucleic acid molecule **characterized in** (a) to (c) ;.

2. The transgenic plant of claim 1, wherein the environmental stress is selected from the group consisting of salinity, drought, temperature, metal, chemical, pathogenic and oxidative stresses, or combinations thereof.

3. The transgenic plant of any of claims 1 to 2 derived from a monocotyledonous plant or from a dicotyledonous plant.

4. The transgenic plant of any of claims 1 to 2, derived from a gymnosperm plant.

5. The transgenic plant of any of claims 1 to 4, wherein the plant is selected from the group consisting of maize, wheat, rye, oat, triticale, rice, barley, soybean, peanut, cotton, rapeseed, canola, manihot, pepper, sunflower, flax, borage, safflower, linseed, primrose, rapeseed, turnip rape, tagetes, solanaceous plants, potato, tobacco, eggplant, tomato, Vicia species, pea, alfalfa, coffee, cacao, tea, Salix species, oil palm, coconut, perennial grass, forage crops, Arabidopsis thaliana and spruce, pine and fir.

6. A seed produced by the transgenic plant of any of claims 1 to 3 or 5, wherein the seed is genetically homozygous for and transgenic for the nucleic acid molecule as defined in claim 1 conferring an increased tolerance to environmental stress as compared to a corresponding non-transformed, wild-type plant.

7. An nucleic acid molecule comprising a nucleic acid molecule selected from the group consisting of:
a. a nucleic acid molecule encoding the polypeptide of SEQ ID NO: 27;
b. a nucleic acid molecule comprising the nucleic acid molecule SEQ ID NO. 153;
c. a nucleic acid molecule whose sequence can be deduced from a polypeptide sequence encoded by the nucleic acid molecule of (a) or (b) as a result of the degeneracy of the genetic code ; and
d. a nucleic acid molecule encoding a polypeptide which is isolated with the aid of monoclonal antibodies against a polypeptide encoded by one of the nucleic acid molecules of (a) to (b).

8. A nucleic acid construct which confers the expression of the nucleic acid molecule of claim 7, comprising one or more regulatory elements operatively linked to the nucleic acid molecule.

9. A vector comprising the nucleic acid molecule as claimed in claim 6 or the nucleic acid construct of claim 8.

10. A host cell, which has been transformed stably or transiently with the vector as claimed in claim 9 or the nucleic acid molecule as claimed in claim 7 or the nucleic acid construct of claim 8.

11. The host cell of claim 10 wherein the cell is a plant cell.

12. An polypeptide encoded by the nucleic acid molecule of claim 7.

13. The polypeptide of claim 12 which is selected from yeast, preferably Saccharomyces cerevisiae, or E.coli, Brassica napus, Glycine max, or Oryza sativa.

14. A method of producing a transgenic plant with increased tolerance and/or resistance to an environmental stress compared to a corresponding non-transformed, wild-type plant, wherein tolerance and/or resistance to an environmental stress is increased by expression of the nucleic acid molecule as defined in claim 1, comprising transforming a plant cell with an expression vector comprising the nucleic acid molecule as defined in claim 1 and generating from the plant cell a transgenic plant with an increased tolerance to environmental stress as compared to a corresponding non-transformed, wild-type plant.

15. The method of claim 14, wherein the plant is transformed with regulatory sequences operatively linked to the nucleic acid molecule as defined in claim 1, wherein the regulatory sequences are active in plants

16. A method of increasing stress tolerance of a transgenic plant comprising, increasing the level of expression of the polypeptide encoded by the nucleic acid molecule as defined in claim 1 in the plant by transformation with a tissue specific, a developmentally regulated or an inducible transgenic promoter that directs expression of nucleic acid molecule as defined in claim 1.

17. The method of claims 14 to 16, wherein the expression is modified by administration of a targeting nucleic sequence complementary to the regulatory region of the nucleic acid molecule and/or by a transcription factor and/or by a zinc finger protein.

18. Use of the nucleic acid molecule as defined in claim 1 or parts of the nucleic acid molecule as markers for detection of stress in plants or plant cells, or as markers for selection of plants or plant cells with increased tolerance to environmental stress, or for preparing a plant cell with increased environmental stress tolerance.


SEQUENCE LISTING

<110> CHARDONNENS, AGNES
      PUZIO, PIOTR
      MCKERSIE, BRYAN

<120> NUCLEIC ACID SEQUENCES ENCODING PROTEINS ASSOCIATED
      WITH ABIOTIC STRESS RESPONSE AND PLANT CELLS AND PLANTS
      WITH INCREASED TOLERANCE TO ENVIRONMENTAL STRESS

<130> PF2063015127

<140>
<141>

<160> 534

<170> PatentIn Ver. 3.3

<210> 1
<211> 13
<212> PRT
<213> Saccharomyces cerevisiae

<220>
<221> MOD_RES
<222> (2)
<223> Variable amino acid

<220>
<221> MOD_RES
<222> (4)
<223> Variable amino acid

<220>
<221> MOD_RES
<222> (5)
<223> Variable amino acid

<220>
<221> MOD_RES
<222> (8)..(10)
<223> Variable amino acid

<400> 1
Leu Xaa Val Xaa Xaa Gly Asp Xaa Xaa Xaa Gly Lys Thr
  1               5                   10


<210> 2
<211> 9
<212> PRT
<213> Saccharomyces cerevisiae

<220>
<221> MOD_RES

<222> (2)..(4)
<223> Variable amino acid

<220>
<221> MOD_RES
<222> (6)
<223> Variable amino acid

<220>
<221> MOD_RES
<222> (7)
<223> Variable amino acid

<400> 2
Phe Xaa Xaa Xaa Tyr Xaa Xaa Thr Val
1               5

<210> 3
<211> 7
<212> PRT
<213> Saccharomyces cerevisiae

<400> 3
Trp Asp Thr Ala Gly Gln Glu
1               5

<210> 4
<211> 7
<212> PRT
<213> Saccharomyces cerevisiae

<220>
<221> MOD_RES
<222> (5)
<223> Variable amino acid

<400> 4
Val Gly Thr Lys Xaa Asp Leu
1               5

<210> 5
<211> 942
<212> DNA
<213> Escherichia coli

<400> 5
atgaagctga tacgcggcat acataatctc agccaggccc cgcaagaagg gtgtgtgctg 60
actattggta atttcgacgg cgtgcatcgc ggtcatcgcg cgctgttaca gggcttgcag 120
gaagaagggc gcaagcgcaa cttaccggtg atggtgatgc tttttgaacc tcaaccactg 180
gaactgtttg ctaccgataa agccccggca agactgaccc ggctgcggga aaaactgcgt 240
taccttgcag agtgtggcgt tgattacgtg ctgtgcgtgc gtttcgacag gcgtttcgcg 300
gcgttaaccg cgcaaaattt catcagcgat cttctggtga agcatttgcg cgtaaaattt 360
cttgccgtag gtgatgattt ccgctttggc gctggtcgtg aaggcgattt cttgttatta 420

cagaaagctg gcatggaata cggcttcgat atcaccagta cgcaaacttt ttgcgaaggt 480
ggcgtgcgca tcagcagcac cgccgtgcgt caggcccttg cggatgacaa tctggctctg 540
gcagagagtt tactggggca cccgtttgcc atctccgggc gtgtagtcca cggtgatgaa 600
ttagggcgca ctataggttt cccgacggcg aatgtaccgc tgcgccgtca ggtttccccg 660
gtgaaagggg tttatgcggt agaagtgctg ggcctcggtg aaaagccgtt acccggcgtg 720
gcaaacatcg gaacacgccc aacggttgcc ggtattcgcc agcagctgga agtgcatttg 780
ttagatgttg caatggacct ttacggtcgc catatacaag tagtgctgcg taaaaaaata 840
cgcaatgagc agcgatttgc gtcgctggac gaactgaaag cgcagattgc gcgtgatgaa 900
ttaaccgccc gcgaattttt tgggctaaca aaaccggctt aa 942

<210> 6
<211> 696
<212> DNA
<213> Escherichia coli

<400> 6
atgttagaag atctcaaacg ccaggtatta gaagccaacc tggcgctgcc aaaacacaac 60
ctggtcacgc tcacatgggg caacgtcagc gccgttgatc gcgagcgcgg cgtctttgtg 120
atcaaacctt ccggcgtcga ttacagcgtc atgaccgctg acgatatggt cgtggttagc 180
atcgaaaccg gtgaagtggt tgaaggtacg aaaaagccct cctccgacac gccaactcac 240
cggctgctct atcaggcatt cccctccatt ggcggcattg tgcatacgca ctcgcgccac 300
gccaccatct gggcgcaggc gggtcagtcg attccagcaa ccggcaccac ccacgccgac 360
tatttctacg gcaccattcc ctgcacccgc aaaatgaccg acgcagaaat caacggcgaa 420
tatgagtggg aaaccggtaa cgtcatcgta gaaacctttg aaaaacaggg tatcgatgca 480
gcgcaaatgc ccggcgttct ggtccattcc cacggcccgt ttgcatgggg caaaaatgcc 540
gaagatgcgg tgcataacgc catcgtgctg gaagaggtcg cttatatggg gatattctgc 600
cgtcagttag cgccgcagtt accggatatg cagcaaacgc tgctggataa acactatctg 660
cgtaagcatg gcgcgaaggc atattacggg cagtaa 696

<210> 7
<211> 585
<212> DNA
<213> Escherichia coli

<400> 7
atgtctaaaa aattaggttt tgccctgtct ggtcttatgc tggcgatggt tgcaggtact 60
gcttctgctg atatggacgg cggtcagtta aatatcagtg gtctggttgt tgataacacc 120
tgtgaaaccc gcgttgacgg cggtaacaag gatggtctga tcctgctgca aaccgcaacc 180
gttggtgaaa tcgatgctgg cgtactgaat gacaccgttg gcgctaaagc taaccttttc 240
agcatcaccg ttgactgcag caaagcaaat ccgaatccag gtagcacggc taaaatgacc 300
ttcggttctg ttttctttgg taacagcaaa gggactttga acaacgacat gtctattaac 360
aacccatctg acggcgttaa tatcgctcta cacaatattg atggttccac tatcaaacag 420
gttcaaatta acaaccctgg cgatgtgtat actaaagccc tggatgcaac gacaaaatct 480
gctgtttatg attttaaagc gtcttacgtt cgtgccgtcg cagaccaaac agcaactgct 540
ggttatgtaa aaactaacac tgcatacacc attacttatc agtaa 585

<210> 8
<211> 927
<212> DNA
<213> Escherichia coli

<400> 8
atgctcccgc cttatttttt gttcaaagag atgacagaca cacagtatat tggccgcttc 60

gccccctctc cttccggcga gcttcatttt ggctctctga tcgccgcgct cggcagctat 120
ttgcaggctc gcgcccggca aggtcgctgg ctggtacgca tagaagatat cgacccgcct 180
cgtgaagttc ccggtgccgc agaaactatc ctgcgccagc tggaacatta cggtctgcac 240
tgggacggcg atgttctctg gcaatcgcaa cgtcacgacg cctatcgtga agcactcgcc 300
tggttacatg aacaaggact aagttattac tgcacctgta cgcgtgcgcg tattcaaagc 360
attggcggta tttacgacgg tcattgccgg gtgttgcatc atggaccaga caacgccgca 420
gtgcgtatcc gccagcagca tccggtcacg caatttactg accagctgcg cggcattatt 480
cacgccgacg aaaaactggc acgggaagat tttatcattc atcgccgtga tgggttgttc 540
gcctacaacc tggctgttgt ggttgatgat catttccagg gcgttacaga aatagtgcgt 600
ggggctgatc tgattgaacc aacagtaagg caaatctcgc tgtaccagct ttttggctgg 660
aaagtgccag attacattca tctgccgctg gcgcttaatc cacaaggcgc taaactttcc 720
aagcagaatc atgcgcctgc gttgccgaaa ggcgatccac gcccggtact aatcgcggca 780
cttcaatttc tggggcagca ggcagaagca cactggcagg atttcagcgt cgagcaaatc 840
cttcagtcag ccgtcaaaaa ctggcggctt accgccgtgc ccgagtcggc aattgtaaat 900
tcaacattct caaatgcgtc atgctga 927

<210> 9
<211> 2475
<212> DNA
<213> Escherichia coli

<400> 9
atgctacaat gtggcgcgaa gaatgttaac cctctggagc gttttgtgtc gtcgttgccc 60
gttgctgccg tcttacctga attacttacc gctctcgatt gtgcgccgca ggtattatta 120
agtgcgccga ccggggccgg gaaatcaacc tggctgccgc tgcaactgct ggcgcatccc 180
ggcattaacg ggaaaattat cctgctggag ccgcgtcgtc tggcggcgcg taacgtcgcg 240
caacggctgg cggagctgct taacgaaaag ccaggcgata ccgttggcta ccggatgcgt 300
gcgcaaaact gcgtcggccc gaatacccgc ctggaagtgg ttaccgaagg cgtgctgacg 360
cgcatgatcc agcgtgaccc ggaactgagc ggtgttggac tggtgatcct tgatgaattt 420
catgagcgca gcttgcaggc ggatttggcg ttggcgctgt tactcgatgt gcaacaaggt 480
ctgcgtgatg accttaaact gctgattatg tcggctaccc tggacaacga ccgcttgcag 540
caaatgctgc cagaagcgcc tgtcgtcatc tcagaagggc gctcgtttcc ggttgaacgc 600
cgttatttac cgctgcccgc gcatcagcgt tttgacgatg ccgttgcggt agccaccgct 660
gaaatgctgc gtcaggaaag cggatcatta ctgttatttt tacctggcgt cggagaaatt 720
cagcgtgtgc aggaacaact ggcttcgcgc atcggcagtg atgtattgct ctgcccgctg 780
tatggcgcgt tgtcgctgaa cgatcagcga aaagcgatcc tcccggcacc gcaagggatg 840
cgcaaagtgg tgctggcgac caatattgct gaaaccagtt taaccattga aggtattcgt 900
ctggtggtgg attgtgccca ggagcgtgtg gcgcgttttg atccgcgcac ggggcttacg 960
cgactgatta ctcaacgcgt tagccaggca tccatgacgc agcgtgccgg gcgcgccggg 1020
cgtctggagc cgggtatcag cctgcattta atcgccaaag aacaagcaga acgcgccgcg 1080
gcgcaaagtg aaccggagat cttacaaagc gatctttccg gtttgctgat ggaattactg 1140
caatggggat gcagcgatcc ggcgcagatg agctggctgg atcaaccgcc agtagtgaat 1200
ctactggccg cgaaacgtct gttacaaatg ctgggggcac tggagggtga acggcttagt 1260
gcgcaagggc aaaaaatggc agcgctgggt aacgatccgc gtttagcggc aatgctggtt 1320
agcgcgaaga acgacgacga agctgctacc gcggcaaaaa ttgccgccat tctcgaagag 1380
ccgccacgga tgggcaatag tgacctgggc gtggcgtttt cgcgcaatca accagcctgg 1440
cagcaacgta gtcagcaact gttaaaacgc ttaaacgtac gtggcggtga ggcagacagt 1500
tcgcttatcg cgccgctact tgccggggcg tttgccgatc gcattgctcg tcgccgtggg 1560
caagatggac gctatcaact ggcaaacggc atgggagcga tgctcgatgc caacgacgcg 1620
ctaagccgcc acgaatggtt gatcgcaccg ttattattgc agggcagcgc ctcgccggat 1680
gcgcggattt tactggcgct gctggtcgat attgatgagt tagtacaacg ctgcccgcag 1740
ctggtacagc agtctgacac tgtggagtgg gatgacgcgc aaggtacgct gaaagcctgg 1800
cgtcggctac aaatcggtca gttgacggtg aaagtgcagc cgctggcgaa accgtcagaa 1860
gacgagttgc atcaggcgat gcttaatggc atccgtgata aaggtttaag cgtgctcaac 1920
tggacggcgg aagcggaaca gctacgcttg cgtttgttat gcgccgcaaa gtggttgccg 1980

gaatatgact ggccagcggt tgatgatgaa agtttattgg cagcgctgga aacgtggctg 2040
ctgccacata tgactggcgt acattcacta cgcggcctga aatcactcga catttatcag 2100
gcactacgcg gattacttga ttggggaatg cagcaacgtc tggatagtga attgcctgcg 2160
cattacactg tgccgacggg aagccggatc gccattcgtt atcatgaaga taacccgccc 2220
gcgctggcgg tgagaatgca agagatgttt ggcgaggcca ccaatccgac gatcgcccag 2280
gggcgcgtgc cgctggtgct ggagttgctt tcacctgccc aaaggccatt acaaatcaca 2340
cgagatttga gcgacttctg gaaaggagcg taccgtgagg tgcaaaaaga gatgaaaggg 2400
cgttatccca aacatgtctg gccggacgac ccggcaaata ctgcaccgac gcgacggacg 2460
aaaaagtatt cgtaa 2475

<210> 10
<211> 606
<212> DNA
<213> Escherichia coli

<400> 10
atgagtataa aaaatctacc tgccgactat ttattggctg cacaacaggg tgatatcgat 60
aaagtaaaaa cctgccttgc gctgggtgtc gatattaata cctgcgatcg tcaggggaaa 120
acggcaatta cgctggcaag tttatatcag caatatgctt gcgttcaggc attaattgat 180
gccggagcgg atattaataa gcaagatcat acctgtttaa atcctttttt aattagctgt 240
ctgaacgatg atttaacgct actacgaatt attttaccgg ctaaacccga tcttaattgc 300
gtaacccgtt ttggcggtgt cggcctgacg cctgcctgtg aaaaaggcca tttaagtatt 360
gtaaaagagc ttttggcgca tacggagatt aacgttaacc agaccaacca tgtaggctgg 420
acgccgctac tggaagcgat tgtgcttaat gatggtggta ttaaacagca ggcgattgtg 480
cagttattgc tggaacacgg tgccagcccg catctgaccg ataaatatgg caaaacgcca 540
ctggaactgg cgcgggaacg gggctttgaa gagattgcgc agttactgat tgccgcaggt 600
gcataa 606

<210> 11
<211> 672
<212> DNA
<213> Escherichia coli

<400> 11
atgatgcagt tagttcactt atttatggat gaaatcacta tggatccttt gcatgccgtt 60
tacctgaccg taggactgtt cgtgattact ttttttaatc cgggagccaa tctctttgtg 120
gtagtacaaa ccagcctggc ttccggtcga cgcgcagggg tgctgaccgg gctgggcgtg 180
gcgctgggcg atgcatttta ttccgggttg ggtttgtttg gtcttgcaac gctaattacg 240
cagtgtgagg agattttttc gcttatcaga atcgtcggcg gcgcttatct cttatggttt 300
gcgtggtgca gcatgcgccg ccagtcaaca ccgcaaatga gcacactaca acaaccgatt 360
agcgccccct ggtatgtctt ttttcgccgc ggattaatta ccgatctctc taacccgcaa 420
accgttttat tttttatcag tattttctca gtaacattaa atgccgaaac accaacatgg 480
gcacgtttaa tggcctgggc ggggattgtg ctcgcatcaa ttatctggcg agtttttctt 540
agtcaggcgt tttctttgcc cgctgtgcgt cgtgcttatg ggcgtatgca acgcgttgcc 600
agtcgggtta ttggtgcaat tattggtgta ttcgcgctac gcctgattta cgaaggggtg 660
acgcagcggt ga 672

<210> 12
<211> 906
<212> DNA
<213> Escherichia coli

<400> 12

gtggatagcg cacgcgccct tattgcccgg ggatggggag taagcttagt cagccgttgt 60
ctccgggtgt cgcgtgcgca gttgcacgtc attctcagac gaaccgatga ctggatggat 120
ggccgccgca gtcgtcacac tgatgatacg gatgtgcttc tccgtataca ccatgttatc 180
ggagagctgc caacgtatgg ttatcgtcgg gtatgggcgc tgcttcgcag acaggcagaa 240
cttgatggta tgcctgcgat caatgccaaa cgtgtttacc ggatcatgcg ccagaatgcg 300
ctgttgcttg agcgaaaacc tgctgtaccg ccatcgaaac gggcacatac aggcagagtg 360
gccgtgaaag aaagcaatca gcgatggtgc tctgacgggt tcgagttctg ctgtgataac 420
ggagagagac tgcgtgtcac gttcgcgctg gactgctgtg atcgtgaggc actgcactgg 480
gcggtcacta ccggcggctt caacagtgaa acagtacagg acgtcatgct gggagcggtg 540
gaacgccgct tcggcaacga tcttccgtcg tctccagtgg agtggctgac ggataatggt 600
tcatgctacc gggctaatga aacacgccag ttcgcccgga tgttgggact tgaaccgaag 660
aacacggcgg tgcggagtcc ggagagtaac ggaatagcag agagcttcgt gaaaacgata 720
aagcgtgact acatcagtat catgcccaaa ccagacgggt taacggcagc aaagaacctt 780
gcagaggcgt tcgagcatta taacgaatgg catccgcata gtgcgctggg ttatcgctcg 840
ccacgggaat atctgcggca gcgggcttgt aatgggttaa gtgataacag atgtctggaa 900
atatag 906

<210> 13
<211> 1767
<212> DNA
<213> Escherichia coli

<400> 13
atgaaattgc cagtcagaga atttgatgca gttgtgattg gtgccggtgg cgcaggtatg 60
cgcgcggcgc tgcaaatttc ccagagcggc cagacctgtg cgctgctctc taaagtcttc 120
ccgacccgtt cccataccgt ttctgcgcaa ggcggcatta ccgttgcgct gggtaatacc 180
catgaagata actgggaatg gcatatgtac gacaccgtga aagggtcgga ctatatcggt 240
gaccaggacg cgattgaata tatgtgtaaa accgggccgg aagcgattct ggaactcgaa 300
cacatgggcc tgccgttctc gcgtctcgat gatggtcgta tctatcaacg tccgtttggc 360
ggtcagtcga aaaacttcgg cggcgagcag gcggcacgca ctgcggcagc agctgaccgt 420
accggtcacg cactgttgca cacgctttat cagcagaacc tgaaaaacca caccaccatt 480
ttctccgagt ggtatgcgct ggatctggtg aaaaaccagg atggcgcggt ggtgggttgt 540
accgcactgt gcatcgaaac cggtgaagtg gtttatttca aagcccgcgc taccgtgctg 600
gcgactggcg gagcagggcg tatttatcag tccaccacca acgcccacat taacaccggc 660
gacggtgtcg gcatggctat ccgtgccggc gtaccggtgc aggatatgga aatgtggcag 720
ttccacccga ccggcattgc cggtgcgggc gtactggtca ccgaaggttg ccgtggtgaa 780
ggcggttatc tgctgaacaa acatggcgaa cgttttatgg agcgttatgc gccgaacgcc 840
aaagacctgg cgggccgtga cgtggttgcg cgttccatca tgatcgaaat ccgtgaaggt 900
cgcggctgtg atggtccgtg ggggccacac gcgaaactga aactcgatca cctgggtaaa 960
gaagttctcg aatcccgtct gccgggtatc ctggagcttt cccgtacctt cgctcacgtc 1020
gatccggtga aagagccgat tccggttatc ccaacctgtc actacatgat gggcggtatt 1080
ccgaccaaag ttaccggtca ggcactgact gtgaatgaga aaggcgaaga tgtggttgtt 1140
ccgggactgt ttgccgttgg tgaaatcgct tgtgtatcgg tacacggcgc taaccgtctg 1200
ggcggcaact cgctgctgga cctggtggtc tttggtcgcg cggcaggtct gcatctgcaa 1260
gagtctatcg ccgagcaggg cgcactgcgc gatgccagcg agtctgatgt tgaagcgtct 1320
ctggatcgcc tgaaccgctg gaacaataat cgtaacggtg aagatccggt ggcgatccgt 1380
aaagcgctgc aagaatgtat gcagcataac ttctcggtct tccgtgaagg tgatgcgatg 1440
gcgaaagggc ttgagcagtt gaaagtgatc cgcgagcgtc tgaaaaatgc ccgtctggat 1500
gacacttcca gcgagttcaa cacccagcgc gttgagtgcc tggaactgga taacctgatg 1560
gaaacggcgt atgcaacggc tgtttctgcc aacttccgta ccgaaagccg tggcgcgcat 1620
agccgcttcg acttcccgga tcgtgatgat gaaaactggc tgtgccactc cctgtatctg 1680
ccagagtcgg aatccatgac gcgccgaagc gtcaacatgg aaccgaaact gcgcccggca 1740
ttcccgccga agattcgtac ttactaa 1767

<210> 14
<211> 2802
<212> DNA
<213> Escherichia coli

<400> 14
atgcagaaca gcgctttgaa agcctggttg gactcttctt acctctctgg cgcaaaccag 60
agctggatag aacagctcta tgaagacttc ttaaccgatc ctgactcggt tgacgctaac 120
tggcgttcga cgttccagca gttacctggt acgggagtca aaccggatca attccactct 180
caaacgcgtg aatatttccg ccgcctggcg aaagacgctt cacgttactc ttcaacgatc 240
tccgaccctg acaccaatgt gaagcaggtt aaagtcctgc agctcattaa cgcataccgc 300
ttccgtggtc accagcatgc gaatctcgat ccgctgggac tgtggcagca agataaagtg 360
gccgatctgg atccgtcttt ccacgatctg accgaagcag acttccagga gaccttcaac 420
gtcggttcat ttgccagcgg caaagaaacc atgaaactcg gcgagctgct ggaagccctc 480
aagcaaacct actgcggccc gattggtgcc gagtatatgc acattaccag caccgaagaa 540
aaacgctgga tccaacagcg tatcgagtct ggtcgcgcga ctttcaatag cgaagagaaa 600
aaacgcttct taagcgaact gaccgccgct gaaggtcttg aacgttacct cggcgcaaaa 660
ttccctggcg caaaacgctt ctcgctggaa ggcggtgacg cgttaatccc gatgcttaaa 720
gagatgatcc gccacgctgg caacagcggc acccgcgaag tggttctcgg gatggcgcac 780
cgtggtcgtc tgaacgtgct ggtgaacgtg ctgggtaaaa aaccgcaaga cttgttcgac 840
gagttcgccg gtaaacataa agaacacctc ggcacgggtg acgtgaaata ccacatgggc 900
ttctcgtctg acttccagac cgatggcggc ctggtgcacc tggcgctggc gtttaacccg 960
tctcaccttg agattgtaag cccggtagtt atcggttctg ttcgtgcccg tctggacaga 1020
cttgatgagc cgagcagcaa caaagtgctg ccaatcacca tccacggtga cgccgcagtg 1080
accgggcagg gcgtggttca ggaaaccctg aacatgtcga aagcgcgtgg ttatgaagtt 1140
ggcggtacgg tacgtatcgt tatcaacaac caggttggtt tcaccacctc taatccgctg 1200
gatgcccgtt ctacgccgta ctgtactgat atcggtaaga tggttcaggc cccgattttc 1260
cacgttaacg cggacgatcc ggaagccgtt gcctttgtga cccgtctggc gctcgatttc 1320
cgtaacacct ttaaacgtga tgtcttcatc gacctggtgt gctaccgccg tcacggccac 1380
aacgaagccg acgagccgag cgcaacccag ccgctgatgt atcagaaaat caaaaaacat 1440
ccgacaccgc gcaaaatcta cgctgacaag ctggagcagg aaaaagtggc gacgctggaa 1500
gatgccaccg agatggttaa cctgtaccgc gatgcgctgg atgctggcga ttgcgtagtg 1560
gcagagtggc gtccgatgaa catgcactct ttcacctggt cgccgtacct caaccacgaa 1620
tgggacgaag agtacccgaa caaagttgag atgaagcgcc tgcaggagct ggcgaaacgc 1680
atcagcacgg tgccggaagc agttgaaatg cagtctcgcg ttgccaagat ttatggcgat 1740
cgccaggcga tggctgccgg tgagaaactg ttcgactggg gcggtgcgga aaacctcgct 1800
tacgccacgc tggttgatga aggcattccg gttcgcctgt cgggtgaaga ctccggtcgc 1860
ggtaccttct tccaccgcca cgcggtgatc cacaaccagt ctaacggttc cacttacacg 1920
ccgctgcaac atatccataa cgggcagggc gcgttccgtg tctgggactc cgtactgtct 1980
gaagaagcag tgctggcgtt tgaatatggt tatgccaccg cagaaccacg cactctgacc 2040
atctgggaag cgcagttcgg tgacttcgcc aacggtgcgc aggtggttat cgaccagttc 2100
atctcctctg gcgaacagaa atggggccgg atgtgtggtc tggtgatgtt gctgccgcac 2160
ggttacgaag ggcaggggcc ggagcactcc tccgcgcgtc tggaacgtta tctgcaactt 2220
tgtgctgagc aaaacatgca ggtttgcgta ccgtctaccc cggcacaggt ttaccacatg 2280
ctgcgtcgtc aggcgctgcg cgggatgcgt cgtccgctgg tcgtgatgtc gccgaaatcc 2340
ctgctgcgtc atccgctggc ggtttccagc ctcgaagaac tggcgaacgg caccttcctg 2400
ccagccatcg gtgaaatcga cgagcttgat ccgaagggcg tgaagcgcgt agtgatgtgt 2460
tctggtaagg tttattacga cctgctggaa cagcgtcgta agaacaatca acacgatgtc 2520
gccattgtgc gtatcgagca actctacccg ttcccgcata aagcgatgca ggaagtgttg 2580
cagcagtttg ctcacgtcaa ggattttgtc tggtgccagg aagagccgct caaccagggc 2640
gcatggtact gcagccagca tcatttccgt gaagtgattc cgtttggggc ttctctgcgt 2700
tatgcaggcc gcccggcctc cgcctctccg gcggtagggt atatgtccgt tcaccagaaa 2760
cagcaacaag atctggttaa tgacgcgctg aacgtcgaat aa 2802

<210> 15

<211> 225
<212> DNA
<213> Escherichia coli

<400> 15
atggaaaagg gtactgttaa gtggttcaac aatgccaaag ggtttggttt catctgccct 60
gaaggcggcg gcgaagatat tttcgctcat tattccacca ttcagatgga tggttacaga 120
acgctaaaag ctggacaatc cgttcagttt gatgtccacc aggggccaaa aggcaatcac 180
gccagtgtta ttgtgcccgt cgaagtagaa gcggcagtcg catag 225

<210> 16
<211> 1536
<212> DNA
<213> Escherichia coli

<400> 16
atgaatttat taaaatcgct ggccgccgtc agctcgatga ccatgttttc gcgtgtgctt 60
ggcttcgcac gagacgcaat tgtcgccaga atctttggcg cagggatggc aaccgacgcc 120
tttttcgtcg cttttaaact tcctaacttg ttacgccgta tctttgccga aggggcattt 180
tcccaggcat ttgtaccgat tctggcggaa tataaaagta agcagggtga agacgccacg 240
cgggtctttg tctcttatgt ttctggcctg ctgacacttg cgctggcggt tgtgacggtc 300
gctggcatgc tcgccgcacc gtgggtgatc atggtgaccg cgccaggctt cgctgacaca 360
gctgacaaat ttgccctgac cagccagcta ctaaagatta cctttcccta tatcttgctg 420
atctccctgg cgtcgctggt gggagcgatt ctgaatacgt ggaaccgctt ctcgattccg 480
gcgtttgctc caacactgct taacatcagc atgattggtt tcgcgctgtt tgccgcaccg 540
tactttaacc caccggtgct ggcgctggcg tgggctgtta cggtcggcgg cgtcctgcag 600
ctggtgtatc agctaccgca cctgaagaag atcggcatgc tggtcctgcc gcgcattaac 660
ttccacgatg ccggagcaat gcgcgtggtg aaacagatgg gaccggcgat ccttggcgtc 720
tctgtgagcc agatctcctt aatcatcaac accatttttg cctcgtttct tgcttccggt 780
tcggtgtctt ggatgtatta cgccgaccgc ttaatggagt ttccgtccgg tgtgctgggc 840
gtggcgcttg gcaccatttt gctgccgtcg ctgtcgaaaa gttttgccag tggcaatcat 900
gatgaataca accgtttgat ggactggggg ttgcgtcttt gtttcctgtt ggcgctgccg 960
agtgcggttg cgttgggcat tctttccggt ccgttgaccg tttcgctgtt ccagtacggt 1020
aaatttaccg cgtttgatgc gctgatgacc cagcgggcgt taattgccta ctcggtgggt 1080
ttgattggcc tgattgtagt gaaagtgttg gctcctggct tttattcccg ccaggacatt 1140
aaaacgccag tgaaaattgc catcgttacg ctgattttaa cgcaattgat gaacctggcg 1200
tttattggtc cgttgaaaca tgccgggctg tcactttcta ttggtctggc ggcgtgtctg 1260
aatgcttcgc tgctttactg gcagttgcgt aagcagaaaa tctttacccc gcaacccggc 1320
tggatggcgt ttctgttgcg tctggtggtg gcggtactgg tgatgtctgg cgtgctttta 1380
ggtatgttac atatcatgcc ggagtggtca ttgggtacca tgccctggcg tttactgcgt 1440
ttaatggcgg tcgtgctggc ggggattgcc gcgtacttcg ctgcactggc ggtactgggc 1500
ttcaaagtta aagaatttgc ccgccggacg gtgtaa 1536

<210> 17
<211> 1098
<212> DNA
<213> Escherichia coli

<400> 17
gtgattaaat ttctctctgc attaattctt ctactggtca cgacggcggc tcaggctgag 60
cgtattcgcg atctcaccag tgttcagggg gtaaggcaaa actcactgat tggctatggt 120
ctggtggtgg ggctggatgg caccggtgac cagacaaccc agacgccgtt taccacacaa 180
acgcttaata acatgctctc acagctggga attaccgttc cgacgggcac caatatgcag 240
ctaaaaaacg tcgctgcggt aatggtgaca gcgtcacttc ctccgtttgg acgtcagggg 300

caaaccatcg atgtggtggt ttcttccatg ggaaatgcca aaagcttgcg tggaggtacg 360
ttgttgatga caccgcttaa gggcgttgac agtcaggtgt atgcgctggc gcagggcaat 420
attctggttg gcggcgcagg agcctccgct ggcggtagca gtgttcaggt taaccaactg 480
aacggtggac ggatcaccaa tggtgcggtt attgaacgtg aattgcccag ccagtttggc 540
gtcgggaata cccttaattt gcaacttaac gacgaagatt tcagcatggc gcagcaaatc 600
gctgacacca tcaaccgcgt gcgtggatat ggcagcgcca ccgcgttaga tgcgcggact 660
attcaggtgc gcgtaccgag tggcaacagt tcccaggtcc gcttccttgc cgatattcag 720
aatatgcagg ttaatgtcac cccgcaggac gctaaagtag tgattaactc gcgcaccggt 780
tcggtggtga tgaatcgcga agtgaccctc gacagctgcg cggtagcgca ggggaatctc 840
tcagtaacag ttaatcgtca ggccaatgtc agccagccag atacaccgtt tggtggtgga 900
cagactgtgg ttactccaca aacgcagatc gatttacgcc agagcggcgg ttcgctgcaa 960
agcgtacgtt ccagcgccag cctcaataac gtggtgcgcg cgctcaatgc gctgggcgct 1020
acgccgatgg atctgatgtc catactgcaa tcaatgcaaa gtgcgggatg tctgcgggca 1080
aaactggaaa tcatctga 1098

<210> 18
<211> 672
<212> DNA
<213> Escherichia coli

<400> 18
atgcgcgtac tggttgttga agacaatgcg ttgttacgtc accaccttaa agttcagatt 60
caggatgctg gtcatcaggt cgatgacgca gaagatgcca aagaagccga ttattatctc 120
aatgaacata taccggatat tgcgattgtc gatctcggat tgccagacga ggacggtctg 180
tcactgattc gccgctggcg tagcaacgat gtttcactgc cgattctggt attaaccgcc 240
cgtgaaagct ggcaggacaa agtcgaagta ttaagtgccg gtgctgatga ttatgtgact 300
aaaccgtttc atattgaaga ggtgatggcg cgaatgcagg cattaatgcg gcgtaatagc 360
ggtctggctt cacaggtcat ttcgctcccc ccgtttcagg ttgatctctc tcgccgtgaa 420
ttatctatta atgacgaagt gatcaaactg accgcgttcg aatacactat tatggaaacg 480
ttgatacgca ataatggcaa agtggtcagc aaagattcgt taatgctcca actctatccg 540
gatgcggagc tgcgggaaag ccataccatt gatgtactga tgggacgtct gcgcaaaaaa 600
attcaggcac aatatcccca agaagtgatt accaccgttc gcggccaggg ctatctgttc 660
gaattgcgct ga 672

<210> 19
<211> 651
<212> DNA
<213> Escherichia coli

<400> 19
atgagtaatc aggaaccggc tactatcctg ctgattgacg atcacccgat gctgcgaact 60
ggcgtaaaac agcttatcag tatggcacca gatatcaccg tggttggcga agcgagtaat 120
ggcgaacagg gtattgaact ggcggagtct cttgatcccg atctgatcct gttagatctc 180
aatatgcccg gcatgaacgg tctggaaacg ctggataaac tgcgcgaaaa gtccctctca 240
gggcgcattg tggtattcag cgtctctaac catgaagaag atgtggtcac cgcactgaaa 300
cgcggcgcgg atggctatct gttaaaagat atggaaccgg aagatctgct gaaagcattg 360
catcaggcag ctgctggcga aatggtatta agcgaagcat taacgcctgt tctggccgcc 420
agcttgcgcg ctaaccgtgc cactactgag cgcgatgtta accagttaac cccacgcgag 480
cgcgatattc tcaagctgat tgcccagggt ttgccgaaca agatgattgc ccgccgcctg 540
gatatcaccg aaagcacagt aaaagtgcac gtcaagcaca tgctgaagaa aatgaagctc 600
aagtctcgcg tggaagcagc ggtatgggtg catcaggagc gcatttctga a 651

<210> 20

<211> 2601
<212> DNA
<213> Escherichia coli

<400> 20
atgagcacaa aaccactctt cctgttacgg aaagcgaaaa aatcatccgg tgaacctgac 60
gtcgtcctgt gggcaagcaa cgattttgaa tcgacctgtg ccactctgga ctacctgatc 120
gttaagtcag gtaaaaaact gagcagctat tttaaagctg ttgccacgaa ttttcctgtc 180
gttaatgacc tgcccgctga aggtgagatc gattttacct ggagtgaacg ctatcaactc 240
agcaaagact ccatgacatg ggaactaaaa ccgggagcag caccagacaa cgctcactat 300
caaggcaata ccaacgtcaa cggcgaagac atgactgaga ttgaggagaa tatgctactc 360
ccaatttctg gccaggaact gcccattcgt tggcttgctc aacacggcag cgaaaaaccg 420
gtaacgcacg tttcacgcga cggactccag gcattacaca ttgctcgggc tgaagaacta 480
ccggctgtta ctgccctggc tgtttcccac aaaaccagcc tgctcgaccc gctggaaatt 540
cgcgaactcc acaaactggt tcgtgacact gacaaagttt tccctaatcc tggtaattca 600
aacctgggac tgataactgc ttttttcgaa gcatacctga acgctgacta caccgatcga 660
ggactgctga caaaagagtg gatgaagggt aatcgtgttt cacacatcac tcgcacggct 720
tccggtgcta atgctggcgg cggaaacctc accgatcgcg gcgaaggttt cgtacacgat 780
ctgacgtcac tggcgcgcga cgtagccact ggcgtactgg cccgttcaat ggatctggac 840
atctataacc ttcatccggc acacgctaaa cgcattgagg aaattatcgc tgaaaataaa 900
ccgccctttt ctgttttccg cgacaaattc atcaccatgc ctggcgggct ggattattcc 960
cgcgccatcg tggttgcgtc cgtaaaagaa gcaccaattg ggatcgaggt catccccgcg 1020
cacgtcactg aatatctgaa caaagtactg actgaaaccg atcatgccaa ccctgatccg 1080
gaaatcgtgg atattgcctg cggtcgctcc tctgccccga tgccgcagcg agtaacagaa 1140
gaaggaaaac aggatgatga agaaaaaccg caaccatctg gaacaacggc agttgaacag 1200
ggagaggctg aaacaatgga accggacgca actgaacatc atcaggacac gcagccgctg 1260
gatgctcagt cacaggtaaa ttctgttgat gcgaaatatc aggaactgcg ggcagaactc 1320
catgaagccc ggaaaaacat tccatcaaaa aatcctgtcg atgacgataa attgcttgct 1380
gcatcacgtg gtgaatttgt tgacggaatt agcgacccga acgatccgaa atgggtaaag 1440
gggatccaga ctcgcgattg tgtgtaccag aaccagccag aaacggaaaa aaccagccca 1500
gatatgaatc aacctgagcc agtagtgcaa caggaaccgg aaatagcctg caatgcctgc 1560
ggccagactg gcggggataa ctgccctgac tgtggtgcgg tgatgggcga cgcaacatac 1620
caggaaacat tcgatgaaga gagtcaggtt gaagctaagg aaaatgatcc ggaggaaatg 1680
gaaggcgctg aacatccgca caatgagaat gctggcagcg atccgcatcg cgattgcagt 1740
gatgaaactg gcgaagtcgc agatcccgta atcgtagaag acatagagcc aggtatttat 1800
tacggaattt cgaatgagaa ttaccacgcg ggtcccggta tcagtaagtc tcagctcgat 1860
gacattgctg atactccggc actatatttg tggcgtaaaa atgcccccgt ggacaccaca 1920
aagacaaaaa cgctcgattt aggaactgct ttccactgcc gggtacttga accggaagaa 1980
ttcagtaacc gctttatcgt agcacctgaa tttaaccgcc gtacaaacgc cggaaaagaa 2040
gaagagaaag cgtttctgat ggaatgcgca agcacaggaa aaacggttat cactgcggaa 2100
gaaggccgga aaattgaact catgtatcaa agcgttatgg ctttgccgct ggggcaatgg 2160
cttgttgaaa gcgccggaca cgctgaatca tcaatttact gggaagatcc tgaaacagga 2220
attttgtgtc ggtgccgtcc ggacaaaatt atccctgaat ttcactggat catggacgtg 2280
aaaactacgg cggatattca acgattcaaa accgcttatt acgactaccg ctatcacgtt 2340
caggatgcat tctacagtga cggttatgaa gcacagtttg gagtgcagcc aactttcgtt 2400
tttctggttg ccagcacaac tattgaatgc ggacgttatc cggttgaaat tttcatgatg 2460
ggcgaagaag caaaactggc aggtcaacag gaatatcacc gcaatctgcg aaccctgtct 2520
gactgcctga ataccgatga atggccagct attaagacat tatcactgcc ccgctgggct 2580
aaggaatatg caaatgacta a 2601

<210> 21
<211> 1071
<212> DNA
<213> Escherichia coli

<400> 21
atgacaacgt ttcattcctt aacagtggca aaagtcgagt cggaaacccg tgatgcggtg 60
accattacct ttgcggtgcc ccagcctttg caggaggcgt atcgctttcg ccccggtcaa 120
catttgacct taaaagccag ctttgatggt gaagaattac gccgttgtta ctccatttgc 180
cgcagctatc tgcctggcga aattagtgtg gcggtgaaag ccattgaagg cggacgtttc 240
tcccgctatg cccgcgaaca catccgccag ggtatgacgc tggaggtcat ggtgccgcag 300
gggcatttcg gctatcagcc gcaggccgaa cgccaggggc gctatctggc aattgcagca 360
ggatcaggta ttacgccaat gctggcgatt atcgccacca ctttacaaac cgagcctgaa 420
agtcagttca ccctgatcta cggtaaccgt accagccaga gcatgatgtt tcgccaggca 480
ctggcagacc tgaaagacaa atatcctcag cgtttacagt tgttgtgcat tttcagtcag 540
gaaaccctcg acagcgatct gcttcacggg cgtattgacg gtgaaaaatt acagtcactt 600
ggggcctcgc tcattaattt tcgtctttat gatgaggcat ttatttgtgg tccggcggcg 660
atgatggatg acgcggaaac cgccttaaaa gcactgggaa tgccagataa aaccattcat 720
ctggagcggt ttaatacgcc tggcacgcgc gtcaaacgta gcgttaacgt gcaaagtgac 780
ggacaaaaag tgactgtacg tcaggatggg cgggatcggg aaatcgtgct taatgccgac 840
gatgaaagca ttctcgatgc ggcattgcgc cagggggcgg atctgcccta tgcctgcaaa 900
ggcggcgtct gtgcgacctg caaatgcaaa gtgctgcgtg gcaaagtggc gatggaaacc 960
aattacagtc tggaaccgga tgaactggcc gcaggttatg tgttgagttg ccaggcactg 1020
ccgctgacca gcgatgtggt ggttgacttt gacgcgaagg ggatggcatg a 1071

<210> 22
<211> 216
<212> DNA
<213> Escherichia coli

<400> 22
atgtcaaata aaatgactgg tttagtaaaa tggtttaacg ctgataaagg tttcggcttt 60
atttctcctg ttgatggtag taaagatgtg tttgtgcatt tttctgcgat tcagaatgat 120
aattatcgaa ccttatttga aggtcaaaag gttaccttct ctatagagag tggtgctaaa 180
ggtcctgcag cagcaaatgt catcattact gattaa 216

<210> 23
<211> 1098
<212> DNA
<213> Escherichia coli

<400> 23
atgtcatctg aaaaactgta ttccccactg aaagtgggcg cgatcacggc ggcaaaccgt 60
atttttatgg caccgctgac gcgtctgcgc agtattgaac cgggtgacat tcctaccccg 120
ttgatggcgg aatactatcg ccaacgtgcc agtgccggtt tgattattag tgaagccacg 180
caaatttctg cccaggcaaa aggatatgca ggtgcgcctg gcatccatag tccggagcaa 240
attgccgcat ggaaaaaaat caccgctggc gttcatgctg aaaatggtca tatggccgtg 300
cagctgtggc acaccggacg catttctcac gccagcctgc aacctggcgg tcaggcaccg 360
gtagcgcctt cagcacttag cgcgggaaca cgtacttctc tgcgcgatga aaatggtcag 420
gcgatccgtg ttgaaacatc catgccgcgt gcgcttgaac tggaagagat tccaggtatc 480
gtcaatgatt tccgtcaggc cattgctaac gcgcgtgaag ccggttttga tctggtagag 540
ctccactctg ctcacggtta tttgctgcat cagttccttt ctccttcttc aaaccatcgt 600
accgatcagt acggcggcag cgtggaaaat cgcgcacgtt tggtactgga agtggtcgat 660
gccgggattg aagaatgggg tgccgatcgc attggcattc gcgtttcacc aatcggtact 720
ttccagaaca cagataacgg cccgaatgaa gaagccgatg cactgtatct gattgaacaa 780
ctgggtaaac gcggcattgc ttatctgcat atgtcagaac cagattgggc ggggggtgaa 840
ccgtatactg atgcgttccg cgaaaaagta cgcgcccgtt tccacggtcc gattatcggc 900
gcaggtgcat acacagtaga aaaagctgaa acgctgatcg gcaaagggtt aattgatgcg 960
gtggcatttg gtcgtgactg gattgcgaac ccggatctgg tcgcccgctt gcagcgcaaa 1020

gctgagctta acccacagcg tgccgaaagt ttctacggtg gcggcgcgga aggctatacc 1080
gattacccga cgttgtaa 1098

<210> 24
<211> 198
<212> DNA
<213> Escherichia coli

<400> 24
atgccaaaaa ttaagaccgt acgcggtgct gctaagcgct tcaaaaaaac cggtaaaggt 60
ggttttaagc acaagcacgc taacctgcgt cacattctga ccaaaaaagc gaccaaacgt 120
aaacgtcacc tgcgtccgaa agccatggtt tccaaaggcg atctgggcct ggtaatcgcg 180
tgcctgccgt acgcataa 198

<210> 25
<211> 996
<212> DNA
<213> Escherichia coli

<400> 25
atgactatca aagtaggtat caacggtttt ggccgtatcg gtcgcattgt tttccgtgct 60
gctcagaaac gttctgacat cgagatcgtt gcaatcaacg acctgttaga cgctgattac 120
atggcataca tgctgaaata tgactccact cacggccgtt tcgacggtac cgttgaagtg 180
aaagacggtc atctgatcgt taacggtaaa aaaatccgtg ttaccgctga acgtgatccg 240
gctaacctga aatgggacga agttggtgtt gacgttgtcg ctgaagcaac tggtctgttc 300
ctgactgacg aaactgctcg taaacacatc accgctggtg cgaagaaagt ggttatgact 360
ggtccgtcta aagacaacac tccgatgttc gttaaaggcg ctaacttcga caaatatgct 420
ggccaggaca tcgtttccaa cgcttcctgc accaccaact gcctggctcc gctggctaaa 480
gttatcaacg ataacttcgg catcatcgaa ggtctgatga ccaccgttca cgctactacc 540
gctactcaga aaaccgttga tggcccgtct cacaaagact ggcgcggcgg ccgcggcgct 600
tcccagaaca tcatcccgtc ctctaccggt gctgctaaag ctgtaggtaa agtactgcca 660
gaactgaatg gcaaactgac tggtatggcg ttccgcgttc cgaccccgaa cgtatctgta 720
gttgacctga ccgttcgtct ggaaaaagct gcaacttacg agcagatcaa agctgccgtt 780
aaagctgctg ctgaaggcga aatgaaaggc gttctgggct acaccgaaga tgacgtagta 840
tctaccgatt tcaacggcga agtttgcact tccgtgttcg atgctaaagc tggtatcgct 900
ctgaacgaca acttcgtgaa actggtatcc tggtacgaca acgaaaccgg ttactccaac 960
aaagttctgg acctgatcgc tcacatctcc aaataa 996

<210> 26
<211> 1251
<212> DNA
<213> Escherichia coli

<400> 26
atggatataa tgagaagtgt tgtggggatg gtggtgttac tggcaatagc atttctgttg 60
tcagtgaata aaaagagcat cagtttgcgc acggttggag ccgcactgct gctgcaaatc 120
gctattggtg gcatcatgct ctacttccca ccgggaaaat gggcagtaga acaggcggca 180
ttaggcgttc ataaagtgat gtcttacagt gatgccggta gcgccttcat ttttggttcg 240
ctggttgggc cgaaaatgga tgtcctgttt gacggtgcgg gttttatctt cgcctttcgc 300
gtacttccgg cgattatttt cgttactgcg ctcatcagtc tgctgtacta cattggcgtg 360
atgggcctgc tgattcgcat ccttggcagc attttccaga aagccctcaa catcagcaaa 420
atcgaatctt ttgttgcggt tactactatt ttcctcgggc aaaatgagat cccggcgatc 480
gttaaaccgt ttatcgatcg catgaatcgc aacgagttgt ttaccgcaat ttgtagcggg 540

atggcgtcca ttgctggttc gatgatgatt ggttatgccg gaatgggcgt accaattgac 600
tacctgttag cggcatcgct gatggcgatc cctggcggga ttttgtttgc acgtattctt 660
agcccggcaa ccgagccttc gcaggtcaca tttgaaaatc tgtcgttcag cgaaacgccg 720
ccaaaaagct ttatcgaagc ggcggcgagc ggtgcgatga ccgggctaaa aatcgccgct 780
ggtgtggcga cggtggtaat ggcgtttgtc gcaattattg cgctgatcaa cggcattatc 840
ggcggaattg gcggctggtt tggtttcgcc aatgcctctc tggaaagtat ttttggctat 900
gtgctggcac cgctggcgtg gatcatgggt gtggactgga gtgatgccaa tcttgcgggt 960
agcctgattg ggcagaaact ggcgattaac gaattcgtcg cttacctgag tttctcccca 1020
tacctgcaaa cgggcggcac gctggaagtg aaaaccattg cgattatctc ctttgcgctt 1080
tgtggttttg ctaactttgg ttctatcggt gttgtcgttg gcgcattttc ggctatttcg 1140
ccaaaacgcg cgccggaaat cgcccagctt ggtttacggg cgctggcagc agcaacgctt 1200
tccaacctga tgagtgcgac tattgccggg ttctttattg gtctggcgta a 1251

<210> 27
<211> 1053
<212> DNA
<213> Escherichia coli

<400> 27
atgaggtttt tattgggcgt gctgatgctg atgatctccg gctcagcgct ggcgaccatc 60
gatgtgttgc agtttaaaga tgaagcacag gaacaacagt tccgtcagct cactgaagaa 120
ctgcgctgcc cgaaatgcca gaacaacagc attgccgatt ccaactcgat gattgccacc 180
gacctgcgtc agaaagtgta tgaactgatg caggaaggta aaagtaaaaa agagattgtc 240
gattatatgg tggcgcgtta cggcaacttc gtcacttacg atccgccgtt aacgccgctg 300
accgtgctgc tgtgggtgct gccagtagtg gctattggca ttggcggttg ggtcatatac 360
gcccgctcgc ggcgtcgggt acgcgtggtg ccggaagcgt ttcctgaaca aagcgtgccg 420
gaaggtaagc gtgccggata tgttgtttat ctgccgggta ttgtggtggc gttaattgtg 480
gctggcgtca gctactacca gactggcaat tatcagcagg tgaaaatctg gcagcaggcc 540
acggcacagg ctccggcgtt gctggacagg gcgctggatc cgaaagccga tccgctcaac 600
gaagaagaga tgtcgcgtct tgcgctgggg atgcgtactc aactgcaaaa aaatccggga 660
gatatagaag gctggattat gttgggccgc gttggcatgg cgctgggtaa cgccagtatc 720
gccaccgatg catacgccac tgcgtatcgc ctcgatccga aaaacagtga tgctgcactg 780
ggatacgctg aagcgttgac tcgttcatct gatcccaacg acaaccgcct cggcggtgaa 840
ctgctacgtc agctggtgag aacggaccat agcaatatcc gtgtgctaag catgtatgcg 900
tttaatgcct ttgagcagca gcgatttggc gaagccgttg ccgcgtggga gatgatgttg 960
aaactcttac ctgccaacga tactcgccgt gcggtgattg aacgtagtat cgcgcaggcg 1020
atgcaacatt tgtcgccgca ggagagtaaa taa 1053

<210> 28
<211> 480
<212> DNA
<213> Escherichia coli

<400> 28
atgaatattc gccgtaaaaa ccgcttgtgg attgcctgtg ccgtgttggc agggctggcg 60
ctgactatcg gtctggtgct atatgcgctg cgctcgaata tcgatctctt ttatacgccg 120
ggggaaattc tctacggcaa gcgtgaaact cagcaaatgc cggaagtcgg tcagcgtctg 180
cgcgttggcg ggatggtgat gccgggtagt gtgcagcgcg atcccaattc gctgaaagtg 240
accttcacca tttacgatgc tgaaggctca gtggatgtct cttacgaagg cattttgccg 300
gatctgttcc gtgaagggca gggcgttgtg gtgcagggcg aactggaaaa aggcaatcat 360
atcctcgcga aagaagtgct ggcgaaacac gatgaaaact atacgccgcc agaagttgag 420
aaagcgatgg aagctaacca ccgtcgcccg gcgagtgttt ataaggaccc agcatcatga 480

<210> 29
<211> 2628
<212> DNA
<213> Escherichia coli

<400> 29
atgagcgacc ttgcgagaga aattacaccg gtcaacattg aggaagagct gaagagctcc 60
tatctggatt atgcgatgtc ggtcattgtt ggccgtgcgc tgccagatgt ccgagatggc 120
ctgaagccgg tacaccgtcg cgtactttac gccatgaacg tactaggcaa tgactggaac 180
aaagcctata aaaaatctgc ccgtgtcgtt ggtgacgtaa tcggtaaata ccatccccat 240
ggtgactcgg cggtctatga cacgatcgtc cgcatggcgc agccattctc gctgcgttat 300
atgctggtag acggtcaggg taacttcggt tctatcgacg gcgactctgc ggcggcaatg 360
cgttatacgg aaatccgtct ggcgaaaatt gcccatgaac tgatggccga tctcgaaaaa 420
gagacggtcg atttcgttga taactatgac ggcacggaaa aaattccgga cgtcatgcca 480
accaaaattc ctaacctgct ggtgaacggt tcttccggta tcgccgtagg tatggcaacc 540
aacatcccgc cgcacaacct gacggaagtc atcaacggtt gtctggcgta tattgatgat 600
gaagacatca gcattgaagg gctgatggaa cacatcccgg ggccggactt cccgacggcg 660
gcaatcatta acggtcgtcg cggtattgaa gaagcttacc gtaccggtcg cggcaaggtg 720
tatatccgcg ctcgcgcaga agtggaagtt gacgccaaaa ccggtcgtga aaccattatc 780
gtccacgaaa ttccgtatca ggtaaacaaa gcgcgcctga tcgagaagat tgcggaactg 840
gtaaaagaaa aacgcgtgga aggcatcagc gcgctgcgtg acgagtctga caaagacggt 900
atgcgcatcg tgattgaagt gaaacgcgat gcggtcggtg aagttgtgct caacaacctc 960
tactcccaga cccagttgca ggtttctttc ggtatcaaca tggtggcatt gcaccatggt 1020
cagccgaaga tcatgaacct gaaagacatc atcgcggcgt ttgttcgtca ccgccgtgaa 1080
gtggtgaccc gtcgtactat tttcgaactg cgtaaagctc gcgatcgtgc tcatatcctt 1140
gaagcattag ccgtggcgct ggcgaacatc gacccgatca tcgaactgat ccgtcatgcg 1200
ccgacgcctg cagaagcgaa aactgcgctg gttgctaatc cgtggcagct gggcaacgtt 1260
gccgcgatgc tcgaacgtgc tggcgacgat gctgcgcgtc cggaatggct ggagccagag 1320
ttcggcgtgc gtgatggtct gtactacctg accgaacagc aagctcaggc gattctggat 1380
ctgcgtttgc agaaactgac cggtcttgag cacgaaaaac tgctcgacga atacaaagag 1440
ctgctggatc agatcgcgga actgttgcgt attcttggta gcgccgatcg tctgatggaa 1500
gtgatccgtg aagagctgga gctggttcgt gaacagttcg gtgacaaacg tcgtactgaa 1560
atcaccgcca acagcgcaga catcaacctg gaagatctga tcacccagga agatgtggtc 1620
gtgacgctct ctcaccaggg ctacgttaag tatcagccgc tttctgaata cgaagcgcag 1680
cgtcgtggcg ggaaaggtaa atctgccgca cgtattaaag aagaagactt tatcgaccga 1740
ctgctggtgg cgaacactca cgaccatatt ctgtgcttct ccagccgtgg tcgcgtctat 1800
tcgatgaaag tttatcagtt gccggaagcc actcgtggcg cgcgcggtcg tccgatcgtc 1860
aacctgctgc cgctggagca ggacgaacgt atcactgcga tcctgccagt gaccgagttt 1920
gaagaaggcg tgaaagtctt catggcgacc gctaacggta ccgtgaagaa aactgtcctc 1980
accgagttca accgtctgcg taccgccggt aaagtggcga tcaaactggt tgacggcgat 2040
gagctgatcg gcgttgacct gaccagcggc gaagacgaag taatgctgtt ctccgctgaa 2100
ggtaaagtgg tgcgctttaa agagtcttct gtccgtgcga tgggctgcaa caccaccggt 2160
gttcgcggta ttcgcttagg tgaaggcgat aaagtcgtct ctctgatcgt gcctcgtggc 2220
gatggcgcaa tcctcaccgc aacgcaaaac ggttacggta aacgtaccgc agtggcggaa 2280
tacccaacca agtcgcgtgc gacgaaaggg gttatctcca tcaaggttac cgaacgtaac 2340
ggtttagttg ttggcgcggt acaggtagat gactgcgacc agatcatgat gatcaccgat 2400
gccggtacgc tggtacgtac tcgcgtttcg gaaatcagca tcgtgggccg taacacccag 2460
ggcgtgatcc tcatccgtac tgcggaagat gaaaacgtag tgggtctgca acgtgttgct 2520
gaaccggttg acgaggaaga tctggatacc atcgacggca gtgccgcgga aggggacgat 2580
gaaatcgctc cggaagtgga cgttgacgac gagccagaag aagaataa 2628

<210> 30
<211> 1329
<212> DNA
<213> Escherichia coli

<400> 30
gtgacaacgg taacgcccac atattctaag gagcttttta tgagcaccgc tttgcttgac 60
gccgtggtga agaaaaaccg tgtgcgttta attccgttta tgttggcgct gtatgtgctg 120
gcgtttctcg accgttcgaa tatcggtttt gccaaacaga cctaccagat tgataccggg 180
ttgagtaatg aagcttatgc gctgggagca ggcattttct ttgtggtata tgcgtttctg 240
ggtgttccgg cgaatctttt gatgcgcaaa ctgggggcca gaacctggat tggtacgaca 300
acactgctgt ggggatttct ttcggcagcc atggcatggg ccgatactga agcgaaattt 360
ctgatagttc gcactctgct tcgtgctgcg gaggccggat ttttccctgg tatgatttat 420
ctcacctcgc aatggtttcc gcagcgtaat cgcgccagca ttatggggct gttctatatg 480
ggcgcaccgc tggcgttaac actgggatca ccgctttccg gcgcgctgtt ggagatgcat 540
ggatttatgg ggcatcccgg ctggttctgg atgtttgtga ttgaaggatt gttggcagtc 600
ggtgctgggg tattcacatt cttttggctt gatgacacac cggagcaggc acgttttctg 660
agtaaacaag aaaaaacgtt gcttatcaat caactggcaa gtgaagaaca acagaaagtg 720
acttctcggc tgagcgatgc gctgcgtaat ggccgagtct ggcaactagc gattatctac 780
ctgaccattc aggtggcggt ttacggatta attttcttcc tgccgaccca ggttgcggca 840
ttgctgggaa caaaagtggg ctttacagcg tcggtggtca ccgccattcc gtgggttgcg 900
gccttgtttg ggacctggct tattccgcgc tattccgata aaaccggcga acggcgtaat 960
gtcgcagcgc tgacattact ggcggcgggc attggtattg gtctgtccgg gctgctttct 1020
ccagtaatgg cgatcgtagc gctgtgtgtt gcagctatcg ggtttattgc cgtgcagcca 1080
gtgttctgga cgatgccgac acaactgctt tccggtacgg cgctggctgc gggaattggt 1140
tttgtaaacc tgtttggtgc agtgggcggg tttattgccc cgatcctgcg cgtgaaagca 1200
gaaacgttat ttgccagcga tgcggcggga ttactgacgc tggcagcggt ggcggtcatc 1260
ggttcgctga ttattttcac tctgcgtgta aatcgcactg ttgcgcagac cgacgtggca 1320
catcattaa 1329

<210> 31
<211> 663
<212> DNA
<213> Escherichia coli

<400> 31
atggattata cgctcacccg catagatccc aacggtgaga acgaccgtta ccccctgcaa 60
aagcaggaga tcgtaaccga ccctctggag caagaagtta acaaaaacgt gtttatgggc 120
aagctcaatg acatggttaa ctggggtcgt aaaaactcaa tttggccgta taacttcggt 180
ctttcctgct gttacgttga gatggtgact tcgtttaccg cggtgcatga cgtggcgcgt 240
tttggcgcag aagtattgcg tgcttcgccg cgtcaggctg acctgatggt ggttgcagga 300
acctgcttta ccaaaatggc accggttatt cagcgtctgt atgaccagat gctggaacca 360
aaatgggtta tctcaatggg tgcctgtgcc aactctggtg gtatgtacga tatttattcc 420
gttgtgcagg gcgtcgataa attcatcccg gttgatgtgt atatcccggg ctgcccgccg 480
cgtcctgaag cgtacatgca ggcactgatg ctgttgcagg aatctatcgg caaagaacgt 540
cgtccgctct cctgggtggt tggcgatcag ggcgtttatc gcgccaatat gcaatcagag 600
cgcgaacgca agcgcggtga acgcattgcc gtaactaacc tgcgtacacc tgacgagatt 660
taa 663

<210> 32
<211> 546
<212> DNA
<213> Escherichia coli

<400> 32
atgctgaagt tactgaaaac tattatgcgc gccggaaccg cgacggtgaa atatcccttc 60
gcgccactgg aggtcagccc tggctttcgc ggaaaaccgg acctgatgcc cagccaatgt 120
attgcctgcg gtgcctgcgc ctgtgcttgt ccggcaaatg cgctgactat ccagaccgac 180

gaccagcaaa attcgcgcac ctggcagctc tatctggggc gttgtattta ctgcggacgt 240
tgtgaagaag tgtgcccgac cagagccatc cagcttacca ataactttga actgaccgtc 300
accaataaag ccgatctcta tacccgcgcg acgttccatc tacaacgttg cagccgttgc 360
gaacgcccgt ttgccccgca aaaaaccatc gcactggctg ctgaattgtt agcacagcaa 420
caaaatgcgc cacaaaaccg cgaaatgttg tgggcgcaag cgagcgtctg cccggaatgc 480
aaacaacgcg cgacgctgat caacgacgat acagatgtac tgctggtggc taaggagcag 540
ctatga 546

<210> 33
<211> 1062
<212> DNA
<213> Escherichia coli

<400> 33
atggctatcg acgaaaacaa acagaaagcg ttggcggcag cactgggcca gattgagaaa 60
caatttggta aaggctccat catgcgcctg ggtgaagacc gttccatgga tgtggaaacc 120
atctctaccg gttcgctttc actggatatc gcgcttgggg caggtggtct gccgatgggc 180
cgtatcgtcg aaatctacgg accggaatct tccggtaaaa ccacgctgac gctgcaggtg 240
atcgccgcag cgcagcgtga aggtaaaacc tgtgcgttta tcgatgctga acacgcgctg 300
gacccaatct acgcacgtaa actgggcgtc gatatcgaca acctgctgtg ctcccagccg 360
gacaccggcg agcaggcact ggaaatctgt gacgccctgg cgcgttctgg cgcagtagac 420
gttatcgtcg ttgactccgt ggcggcactg acgccgaaag cggaaatcga aggcgaaatc 480
ggcgactctc acatgggcct tgcggcacgt atgatgagcc aggcgatgcg taagctggcg 540
ggtaacctga agcagtccaa cacgctgctg atcttcatca accagatccg tatgaaaatt 600
ggtgtgatgt tcggtaaccc ggaaaccact accggtggta acgcgctgaa attctacgcc 660
tctgttcgtc tcgacatccg tcgtatcggc gcggtgaaag agggcgaaaa cgtggtgggt 720
agcgaaaccc gcgtgaaagt ggtgaagaac aaaatcgctg cgccgtttaa acaggctgaa 780
ttccagatcc tctacggcga aggtatcaac ttctacggcg aactggttga cctgggcgta 840
aaagagaagc tgatcgagaa agcaggcgcg tggtacagct acaaaggtga gaagatcggt 900
cagggtaaag cgaatgcgac tgcctggctg aaagataacc cggaaaccgc gaaagagatc 960
gagaagaaag tacgtgagtt gctgctgagc aacccgaact caacgccgga tttctctgta 1020
gatgatagcg aaggcgtagc agaaactaac gaagattttt aa 1062

<210> 34
<211> 2562
<212> DNA
<213> Escherichia coli

<400> 34
atgagtgcaa tagaaaattt cgacgcccat acgcccatga tgcagcagta tctcaggctg 60
aaagcccagc atcccgagat cctgctgttt taccggatgg gtgattttta tgaactgttt 120
tatgacgacg caaaacgcgc gtcgcaactg ctggatattt cactgaccaa acgcggtgct 180
tcggcgggag agccgatccc gatggcgggg attccctacc atgcggtgga aaactatctc 240
gccaaactgg tgaatcaggg agagtccgtt gccatctgcg aacaaattgg cgatccggcg 300
accagcaaag gtccggttga gcgcaaagtt gtgcgtatcg ttacgccagg caccatcagc 360
gatgaagccc tgttgcagga gcgtcaggac aacctgctgg cggctatctg gcaggacagc 420
aaaggtttcg gctacgcgac gctggatatc agttccgggc gttttcgcct gagcgaaccg 480
gctgaccgcg aaacgatggc ggcagaactg caacgcacta atcctgcgga actgctgtat 540
gcagaagatt ttgctgaaat gtcgttaatt gaaggccgtc gcggcctgcg ccgtcgcccg 600
ctgtgggagt ttgaaatcga caccgcgcgc cagcagttga atctgcaatt tgggacccgc 660
gatctggtcg gttttggcgt cgagaacgcg ccgcgcggac tttgtgctgc cggttgtctg 720
ttgcagtatg cgaaagatac ccaacgtacg actctgccgc atattcgttc catcaccatg 780
gaacgtgagc aggacagcat cattatggat gccgcgacgc gtcgtaatct ggaaatcacc 840
cagaacctgg cgggtggtgc ggaaaatacg ctggcttctg tgctcgactg caccgtcacg 900

ccgatgggca gccgtatgct gaaacgctgg ctgcatatgc cagtgcgcga tacccgcgtg 960
ttgcttgagc gccagcaaac tattggcgca ttgcaggatt tcaccgccgg gctacagccg 1020
gtactgcgtc aggtcggcga cctggaacgt attctggcac gtctggcttt acgaactgct 1080
cgcccacgcg atctggcccg tatgcgccac gctttccagc aactgccgga gctgcgtgcg 1140
cagttagaaa ctgtcgatag tgcaccggta caggcgctac gtgagaagat gggcgagttt 1200
gccgagctgc gcgatctgct ggagcgagca atcatcgaca caccgccggt gctggtacgc 1260
gacggtggtg ttatcgcatc gggctataac gaagagctgg atgagtggcg cgcgctggct 1320
gacggcgcga ccgattatct ggagcgtctg gaagtccgcg agcgtgaacg taccggcctg 1380
gacacgctga aagttggctt taatgcggtg cacggctact acattcaaat cagccgtggg 1440
caaagccatc tggcacccat caactacatg cgtcgccaga cgctgaaaaa cgccgagcgc 1500
tacatcattc cagagctaaa agagtacgaa gataaagttc tcacctcaaa aggcaaagca 1560
ctggcactgg aaaaacagct ttatgaagag ctgttcgacc tgctgttgcc gcatctggaa 1620
gcgttgcaac agagcgcgag cgcgctggcg gaactcgacg tgctggttaa cctggcggaa 1680
cgggcctata ccctgaacta cacctgcccg accttcattg ataaaccggg cattcgcatt 1740
accgaaggtc gccatccggt agttgaacaa gtactgaatg agccatttat cgccaacccg 1800
ctgaatctgt cgccgcagcg ccgcatgttg atcatcaccg gtccgaacat gggcggtaaa 1860
agtacctata tgcgccagac cgcactgatt gcgctgatgg cctacatcgg cagctatgta 1920
ccggcacaaa aagtcgagat tggacctatc gatcgcatct ttacccgcgt aggcgcggca 1980
gatgacctgg cgtccgggcg ctcaaccttt atggtggaga tgactgaaac cgccaatatt 2040
ttacataacg ccaccgaata cagtctggtg ttaatggatg agatcgggcg tggaacgtcc 2100
acctacgatg gtctgtcgct ggcgtgggcg tgcgcggaaa atctggcgaa taagattaag 2160
gcattgacgt tatttgctac ccactatttc gagctgaccc agttaccgga gaaaatggaa 2220
ggcgtcgcta acgtgcatct cgatgcactg gagcacggcg acaccattgc ctttatgcac 2280
agcgtgcagg atggcgcggc gagcaaaagc tacggcctgg cggttgcagc tctggcaggc 2340
gtgccaaaag aggttattaa gcgcgcacgg caaaagctgc gtgagctgga aagcatttcg 2400
ccgaacgccg ccgctacgca agtggatggt acgcaaatgt ctttgctgtc agtaccagaa 2460
gaaacttcgc ctgcggtcga agctctggaa aatcttgatc cggattcact caccccgcgt 2520
caggcgctgg agtggattta tcgcttgaag agcctggtgt aa 2562

<210> 35
<211> 492
<212> DNA
<213> Escherichia coli

<400> 35
gtgatgaaat cgttaattat cgttaatccg gctgactgta ttggctgccg tacttgtgaa 60
gtagcctgtg tggtcgctca cccttcagaa caggagttga atgccgatgt ctttttaccc 120
cggctgaagg tacagcgact ggacagcatc agtgcgccgg tgatgtgcca tcagtgtgaa 180
aacgcccctt gtgttggcgc ttgccccgtg ggggcgctga cgatggcgga gcaggtggtg 240
cagaccaatt ctgcccgttg tattggctgt cagagttgcg tcagtgcgtg cccgtttgga 300
atgatcacca ttcagtcgtt gccgggggat acccgacaac aaattgtgaa atgcgatctc 360
tgtgaacagc gggaagaggg gccagcctgc gttgaatctt gcccaacaca ggcgctgcag 420
ttgcttactg aaagagaact caggcgagtc cgccagcagc gtattgttgc cagcggtgag 480
aatcctctct ga 492

<210> 36
<211> 1164
<212> DNA
<213> Escherichia coli

<400> 36
atgtctgtaa ttaagatgac cgatctggat cttgctggga aacgtgtatt tatccgtgcg 60
gatctgaacg taccagtaaa agacggaaaa gtaaccagcg acgcgcgtat ccgtgcttct 120
ctgccgacca tcgaactggc cctgaaacaa ggcgcaaaag tgatggtaac ttcccacctg 180

ggtcgtccta ccgaaggcga gtacaacgaa gaattctctc tgctgccggt tgttaactac 240
ctgaaagaca aactgtctaa cccggttcgt ctggttaaag attacctcga cggcgttgac 300
gttgctgaag gtgaactggt tgttctggaa aacgttcgct tcaacaaagg cgagaagaaa 360
gacgacgaaa ccctgtccaa aaaatacgct gcactgtgtg acgtgttcgt aatggacgca 420
ttcggtactg ctcaccgcgc gcaggcttct actcacggta tcggtaaatt cgctgacgtt 480
gcgtgcgcag gcccgctgct ggcagctgaa ctggacgcgc tgggtaaagc actgaaagaa 540
cctgctcgcc cgatggtggc tatcgttggt ggttctaaag tatctaccaa actgaccgtt 600
ctggactccc tgtctaaaat cgctgaccag ctgattgttg gtggtggtat cgctaacacc 660
tttatcgcgg cacaaggcca cgatgtgggt aaatccctgt acgaagctga cctggttgac 720
gaagctaaac gtctgctgac cacctgcaac atcccggttc cgtctgatgt tcgcgtagca 780
accgagttct ctgaaactgc accggctacc ctgaaatctg ttaacgatgt gaaagctgac 840
gagcagatcc tggatatcgg tgatgcttcc gctcaggaac tggctgaaat cctgaagaat 900
gcgaaaacca ttctgtggaa cggtccggtt ggcgtgttcg aattcccgaa cttccgcaaa 960
ggtactgaaa tcgtggctaa cgctatcgca gacagcgaag cgttctccat cgctggcggc 1020
ggcgacactc tggcagcaat cgacctgttc ggcattgctg acaaaatctc ctacatctcc 1080
actggcggcg gcgcattcct cgaattcgtg gaaggtaaag tactgcctgc agtagcgatg 1140
ctcgaagagc gcgctaagaa gtaa 1164

<210> 37
<211> 765
<212> DNA
<213> Escherichia coli

<400> 37
atgaaagatg aacgtcgccc tatttgcgaa gtggttgcag agagtatcga acggttaatt 60
atcgacggcg tactgaaggt cggtcagccg cttccctcgg aacgtcgact gtgtgaaaag 120
ctcggcttct cacgctccgc actgcgtgaa gggctgaccg tgctgcgcgg gcgcgggatt 180
attgaaacgg cgcagggtcg cgattctcgt gtcgcacggc ttaatcgggt gcaggacacc 240
agcccgctga tccatctgtt cagtacgcag ccgcgaacgc tgtacgatct gctcgacgtt 300
cgcgcattac tggagggcga atcggcaagg ctggcggcaa cgctgggaac gcaggctgat 360
tttgttgtga taacccgctg ttatgaaaaa atgctcgccg ccagtgagaa caacaaagag 420
atttcgctga tcgaacatgc gcagttggat cacgctttcc atctcgccat ttgtcaggct 480
tctcacaatc aggtgctggt gtttacgctg caatcattga ccgatctgat gtttaattca 540
gtgtttgcca gcgtaaataa tctctaccat cgaccacagc aaaaaaagca gatcgatcgc 600
cagcatgcgc ggatctacaa cgcggtgttg cagcggctgc cgcacgtcgc ccagcgcgca 660
gcacgcgatc atgtgcggac cgtgaaaaag aatctccacg atatcgagct ggaaggccac 720
catttgattc gctcggcggt gccgctggag atgaacctga gttag 765

<210> 38
<211> 1500
<212> DNA
<213> Escherichia coli

<400> 38
atgctaaatt tatttgttgg ccttgatata tacacagggc ttttgttatt gcttgctctg 60
gcatttgtgt tgttctacga agcaatcaat ggttttcatg acacggcgaa tgcggtggca 120
gccgttattt atactcgtgc catgcaacca caacttgctg tggtgatggc ggcatttttt 180
aactttttgg gcgtgttatt gggcggactt agcgttgcct atgccattgt ccatatgttg 240
ccaaccgatt tgttgctgaa tatggggtca acccacggcc tggcgatggt cttttccatg 300
ctgctggcgg cgattatctg gaacctggga acgtggttct tcggtttacc ggcctccagt 360
tcgcacacct tgattggtgc gattatcggc atcggtttaa ccaacgcgct gttaaccggc 420
tcatcggtga tggatgcgtt aaacctgcgt gaagtgacca aaattttctc ctcgctgatt 480
gtttccccta tcgtcggcct ggtcattgcg ggaggcctga tattcctgct gcgacgctac 540
tggagcggga cgaaaaagcg tgaccgtatt caccgcattc cggaagatcg caaaaagaaa 600

aaaggcaaac gtaaaccgcc attctggacg cgtattgcgc tgattgtttc cgctgcgggc 660
gtggcgtttt cgcacggcgc gaacgacgga caaaaaggga tcggcctggt aatgctggta 720
ctggtgggga ttgcccctgc tggcttcgtc gtcaatatga atgcgtccgg ctatgaaatt 780
acccgtaccc gcgatgccgt taccaacttc gaacactacc tgcaacagca tcctgaactg 840
ccgcagaagt tgattgcgat ggaacctcca ttgcctgcag catcgactga tggcacgcaa 900
gtaacagagt ttcactgtca tccggcaaat acctttgatg ctattgcgcg cgttaaaacg 960
atgctgccag gcaatatgga aagttacgag ccgttaagcg tgagtcagcg cagccagctg 1020
cgccgcatta tgctgtgcat ctctgatacc tccgcgaagc tagcgaaact gccaggcgtc 1080
agtaaagaag accagaacct gctgaaaaaa cttcgcagcg atatgttaag caccattgag 1140
tacgctccgg tgtggatcat catggcggta gcactggcgc tcggcattgg caccatgatt 1200
ggctggcgtc gtgtagcgat gaccatcggt gagaagattg gtaagcgcgg catgacgtat 1260
gcgcaaggca tggcggcaca aatgacggcg gcagtgtcta tcggtcttgc cagttatatt 1320
gggatgcccg tctccacaac acacgtcctc tcgtctgcag ttgcagggac gatggtggtg 1380
gacggcggtg ggttacagcg taaaacggta accagcatcc tgatggcgtg ggtatttact 1440
ttaccggcgg caatttttct ttctggtggg ctgtactgga tagcattgca gttgatttaa 1500

<210> 39
<211> 1893
<212> DNA
<213> Escherichia coli

<400> 39
atgacgcaaa cttataacgc tgatgccatt gaggtactca ccgggcttga gccggttcgc 60
cgccgtccgg ggatgtatac cgataccact cgccctaacc atttggggca agaagtcatt 120
gataacagtg tggatgaagc actggcgggt cacgcaaaac gcgtggacgt tattttacat 180
gctgaccagt cgttagaagt tattgacgat gggcgcggga tgccggtgga tattcacccg 240
gaagagggtg taccggcggt tgaactgatt ctttgccgtc tgcatgcagg cggtaaattc 300
tctaacaaaa attaccagtt ctctggcggc ctgcatggcg tggggatttc ggtggttaac 360
gccctgtcga agcgcgtaga agttaacgtg cgccgcgatg gtcaggttta taacatcgcc 420
tttgaaaatg gcgaaaaggt gcaggattta caggttgtcg gcacttgcgg taaacgcaat 480
actggtacca gtgtgcactt ctggccggat gaaaccttct ttgacagccc gcgattttct 540
gtttcacgcc tgacgcatgt gctgaaagcc aaagcggtat tgtgccctgg cgttgagatc 600
acttttaaag atgagatcaa caataccgaa caacgctggt gctatcagga cggtctgaat 660
gattacctgg cggaagcggt aaatggtctg ccgacgctgc cggaaaaacc gtttatcggt 720
aatttcgctg gtgatactga agctgtggac tgggcgctac tgtggctgcc ggaaggcggt 780
gaactgctga ccgaaagcta cgtcaacctt atcccaacga tgcagggcgg tacccatgtt 840
aatggtctgc gtcagggcct gttggacgcg atgcgtgagt tctgtgaata ccgcaatatt 900
ctgccgcgcg gtgtaaagct gtcggcggaa gatatctggg atcgctgcgc ctatgtgctg 960
tcagtaaaaa tgcaggatcc gcagtttgcc gggcagacga aagagcgtct ctcttcgcgt 1020
caatgcgcgg cattcgtttc tggcgtggtg aaagatgcct ttatcctgtg gctgaaccag 1080
aacgttcagg cggctgaact gctggcggag atggcgattt ccagcgccca gcgccgtatg 1140
cgtgcggcca aaaaagtggt gcgtaaaaag ctgaccagcg gcccggcgtt gcctggcaaa 1200
ctggctgatt gtaccgcgca ggaccttaac cgtaccgagc tgttccttgt ggaaggtgac 1260
tccgcaggcg gatctgccaa gcaggcgcgc gatcgcgaat atcaggcgat catgccactg 1320
aaaggtaaga tccttaacac ctgggaagtc tcttccgacg aagtgctggc ttcgcaggaa 1380
gtgcacgata tttcggtagc gatcggtatc gatcctgaca gcgacgatct gagccagctt 1440
cgttatggca aaatctgtat cctcgcggat gcggactctg atggtctgca cattgccacg 1500
ctgctctgcg ctttgttcgt aaaacatttc cgcgcgttgg tgaaacacgg tcacgtttac 1560
gtcgcactgc caccgctcta ccgtattgat ctcgggaaag aggtttatta cgcgctgacg 1620
gaagaagaga aagagggcgt acttgagcaa ttaaaacgca agaaaggcaa gccgaacgtc 1680
cagcgtttta aaggtctggg ggaaatgaac ccgatgcaat tgcgcgaaac cacgcttgat 1740
ccgaacactc gccgtctggt gcagttgact atcgatgatg aagacgatca gcgtactgac 1800
gcgatgatgg atatgctgct ggcgaagaaa cgctcggaag atcgccgcaa ctggttgcaa 1860
gagaaaggcg acatggcgga gattgaggtt taa 1893

<210> 40
<211> 1746
<212> DNA
<213> Escherichia coli

<400> 40
atggctggac gaatcccacg cgtattcatt aatgatctgc tggcacgcac tgacatcgtc 60
gatctgatcg atgcccgtgt gaagctgaaa aagcagggca agaatttcca cgcgtgttgt 120
ccattccaca acgagaaaac cccgtccttc accgttaacg gtgagaaaca gttttaccac 180
tgctttggat gtggcgcgca cggcaacgcg atcgacttcc tgatgaacta cgacaagctc 240
gagttcgtcg aaacggtcga agagctggca gcaatgcaca atcttgaagt gccatttgaa 300
gcaggcagcg gccccagcca gatcgagcgc catcagaggc aaacgcttta tcagttgatg 360
gacggtctga atacgtttta ccaacaatct ttacaacaac ctgttgccac gtctgcgcgc 420
cagtatctgg aaaaacgcgg attaagccac gaggttatcg ctcgctttgc gattggtttt 480
gcgccccccg gctgggacaa cgtcctgaag cggtttggcg gcaatccaga aaatcgccag 540
tcattgattg atgcggggat gttggtcact aacgatcagg gacgcagtta cgatcgtttc 600
cgcgagcggg tgatgttccc cattcgcgat aaacgcggtc gggtgattgg ttttggcggg 660
cgcgtgctgg gcaacgatac ccccaaatac ctgaactcgc cggaaacaga cattttccat 720
aaaggccgcc agctttacgg tctttatgaa gcgcagcagg ataacgctga acccaatcgt 780
ctgcttgtgg tcgaaggcta tatggacgtg gtggcgctgg cgcaatacgg cattaattac 840
gccgttgcgt cgttaggtac gtcaaccacc gccgatcaca tacaactgtt gttccgcgcg 900
accaacaatg tcatttgctg ttatgacggc gaccgtgcag gccgcgatgc cgcctggcga 960
gcgctggaaa cggcgctgcc ttacatgaca gacggccgtc agctacgctt tatgtttttg 1020
cctgatggcg aagaccctga cacgctagta cgaaaagaag gtaaagaagc gtttgaagcg 1080
cggatggagc aggcgatgcc actctccgca tttctgttta acagtctgat gccgcaagtt 1140
gatctgagta cccctgacgg gcgcgcacgt ttgagtacgc tggcactacc attgatatcg 1200
caagtgccgg gcgaaacgct gcgaatatat cttcgtcagg aattaggcaa caaattaggc 1260
atacttgatg acagccagct tgaacgatta atgccaaaag cggcagagag cggcgtttct 1320
cgccctgttc cgcagctaaa acgcacgacc atgcgtatac ttatagggtt gctggtgcaa 1380
aatccagaat tagcgacgtt ggtcccgccg cttgagaatc tggatgaaaa taagctccct 1440
ggacttggct tattcagaga actggtcaac acttgtctct cccagccagg tctgaccacc 1500
gggcaacttt tagagcacta tcgtggtaca aataatgctg ccacccttga aaaactgtcg 1560
atgtgggacg atatagcaga taagaatatt gctgagcaaa ccttcaccga ctcactcaac 1620
catatgtttg attcgctgct tgaactgcgc caggaagagt taatcgctcg tgagcgcacg 1680
catggtttaa gcaacgaaga acgcctggag ctctggacat taaaccagga gctggcgaaa 1740
aagtga 1746

<210> 41
<211> 1842
<212> DNA
<213> Escherichia coli

<400> 41
atggagcaaa acccgcagtc acagctgaaa cttcttgtca cccgtggtaa ggagcaaggc 60
tatctgacct atgccgaggt caatgaccat ctgccggaag atatcgtcga ttcagatcag 120
atcgaagaca tcatccaaat gatcaacgac atgggcattc aggtgatgga agaagcaccg 180
gatgccgatg atctgatgct ggctgaaaac accgcggacg aagatgctgc cgaagccgcc 240
gcgcaggtgc tttccagcgt ggaatctgaa atcgggcgca cgactgaccc ggtacgcatg 300
tacatgcgtg aaatgggcac cgttgaactg ttgacccgcg aaggcgaaat tgacatcgct 360
aagcgtattg aagacgggat caaccaggtt caatgctccg ttgctgaata tccggaagcg 420
atcacctatc tgctggaaca gtacgatcgt gttgaagcag aagaagcgcg tctgtccgat 480
ctgatcaccg gctttgttga cccgaacgca gaagaagatc tggcacctac cgccactcac 540
gtcggttctg agctttccca ggaagatctg gacgatgacg aagatgaaga cgaagaagat 600
ggcgatgacg acagcgccga tgatgacaac agcatcgacc cggaactggc tcgcgaaaaa 660

tttgcggaac tacgcgctca gtacgttgta acgcgtgaca ccatcaaagc gaaaggtcgc 720
agtcacgcta ccgctcagga agagatcctg aaactgtctg aagtattcaa acagttccgc 780
ctggtgccga agcagtttga ctacctggtc aacagcatgc gcgtcatgat ggaccgcgtt 840
cgtacgcaag aacgtctgat catgaagctc tgcgttgagc agtgcaaaat gccgaagaaa 900
aacttcatta ccctgtttac cggcaacgaa accagcgata cctggttcaa cgcggcaatt 960
gcgatgaaca agccgtggtc ggaaaaactg cacgatgtct ctgaagaagt gcatcgcgcc 1020
ctgcaaaaac tgcagcagat tgaagaagaa accggcctga ccatcgagca ggttaaagat 1080
atcaaccgtc gtatgtccat cggtgaagcg aaagcccgcc gtgcgaagaa agagatggtt 1140
gaagcgaact tacgtctggt tatttctatc gctaagaaat acaccaaccg tggcttgcag 1200
ttccttgacc tgattcagga aggcaacatc ggtctgatga aagcggttga taaattcgaa 1260
taccgccgtg gttacaagtt ctccacctac gcaacctggt ggatccgtca ggcgatcacc 1320
cgctctatcg cggatcaggc gcgcaccatc cgtattccgg tgcatatgat tgagaccatc 1380
aacaagctca accgtatttc tcgccagatg ctgcaagaga tgggccgtga accgacgccg 1440
gaagaactgg ctgaacgtat gctgatgccg gaagacaaga tccgcaaagt gctgaagatc 1500
gccaaagagc caatctccat ggaaacgccg atcggtgatg atgaagattc gcatctgggg 1560
gatttcatcg aggataccac cctcgagctg ccgctggatt ctgcgaccac cgaaagcctg 1620
cgtgcggcaa cgcacgacgt gctggctggc ctgaccgcgc gtgaagcaaa agttctgcgt 1680
atgcgtttcg gtatcgatat gaacaccgac tacacgctgg aagaagtggg taaacagttc 1740
gacgttaccc gcgaacgtat ccgtcagatc gaagcgaagg cgctgcgcaa actgcgtcac 1800
ccgagccgtt ctgaagtgct gcgtagcttc ctggacgatt aa 1842

<210> 42
<211> 2091
<212> DNA
<213> Escherichia coli

<400> 42
atgtggcgca gactgattta tcaccccgat atcaactatg cacttcgaca aacgctggtg 60
ctatgtttgc ccgtggccgt tgggttaatg cttggcgaat tacgattcgg tctgctcttc 120
tccctcgttc ctgcctgttg caatattgcg ggccttgata cgcctcataa acgttttttc 180
aaacgcttaa tcattggtgc gtcgctgttt gccacctgta gcttgctgac acagctacta 240
ctggcaaaag atgttcccct gccctttttg ctgaccggat taacgctggt acttggcgtc 300
actgctgagc tggggccatt gcacgcaaaa ttgcttcctg catcgctgct cgccgccatt 360
tttaccctca gtttggcggg atacatgccg gtctgggaac cgttgctcat ctatgcgttg 420
ggcactctct ggtacggatt gtttaactgg ttttggttct ggatctggcg cgaacaaccg 480
ctgcgcgagt cactaagtct gctgtaccgt gaactggcag attattgtga agccaaatac 540
agcctgctta cccagcacac cgaccctgaa aaagcgctgc cgccgctgct ggtgcgccag 600
caaaaagcgg tcgatctaat tacccagtgc tatcagcaaa tgcatatgct ttccgcgcaa 660
aataatactg actacaagcg gatgctgcgt attttccagg aggcgctgga tttacaggaa 720
catatttcgg tcagtttgca tcagccggaa gaggtgcaaa agctggtcga gcgtagccat 780
gcggaagaag ttatccgctg gaatgcgcaa accgtcgccg ctcgcctgcg cgtgctggct 840
gatgacattc tttaccatcg cctgccaacg cgttttacga tggaaaagca aattggcgca 900
ctggaaaaaa tcgcccgcca gcatccggat aatccggttg ggcaattctg ctactggcat 960
ttcagccgca tcgcccgcgt gctgcgcacc caaaaaccgc tctatgcccg tgacttactg 1020
gccgataaac agcggcgaat gccattactt ccggcgctga aaagttatct gtcactaaag 1080
tctccggcgc tacgcaatgc cggacgactc agtgtgatgt taagcgttgc cagcctgatg 1140
ggcaccgcgc tgcatctgcc gaagtcgtac tggatcctga tgacggtatt gctggtgaca 1200
caaaatggct atggcgcaac ccgtctgagg attgtgaatc gctccgtggg aaccgtggtc 1260
gggttaatca ttgcgggcgt ggcgctgcac tttaaaattc ccgaaggtta caccctgacg 1320
ttgatgctga ttaccaccct cgccagctac ctgatattgc gcaaaaacta cggctgggcg 1380
acggtcggtt ttactattac cgcagtgtat accctgcaac tattgtggtt gaacggcgag 1440
caatacatcc ttccgcgtct tatcgatacc attattggtt gtttaattgc tttcggcggt 1500
actgtctggc tgtggccgca gtggcagagc gggttattgc gtaaaaacgc ccatgatgct 1560
ttagaagcct atcaggaagc gattcgcttg attcttagcg aggatccgca acctacgcca 1620
ctggcctggc agcgaatgcg ggtaaatcag gcacataaca ctctgtataa ctcattgaat 1680

caggcgatgc aggaaccggc gtttaacagc cattatctgg cagatatgaa actgtgggta 1740
acgcacagcc agtttattgt tgagcatatt aatgccatga ccacgctggc gcgggaacac 1800
cgggcattgc cacctgaact ggcacaagag tatttacagt cttgtgaaat cgccattcag 1860
cgttgtcagc agcgactgga gtatgacgaa ccgggtagtt ctggcgatgc caatatcatg 1920
gatgcgccgg agatgcagcc gcacgaaggc gcggcaggta cgctggagca gcatttacag 1980
cgggttattg gtcatctgaa caccatgcac accatttcgt cgatggcatg gcgtcagcga 2040
ccgcatcacg ggatttggct gagtcgcaag ttgcgggatt cgaaggcgta a 2091

<210> 43
<211> 777
<212> DNA
<213> Escherichia coli

<400> 43
atgattgttt tacccagacg cctgtcagac gaggttgccg atcgtgtgcg ggcgctgatt 60
gatgaaaaaa acctggaagc gggcatgaag ttgcccgctg agcgccaact ggcgatgcaa 120
ctcggcgtat cacgtaattc actgcgcgag gcgctggcaa aactggtgag tgaaggcgtg 180
ctgctcagtc gacgcggcgg cgggacgttt attcgctggc gtcatgacac atggtcggag 240
caaaacatcg tccagccgct aaaaacactg atggccgatg atccggatta cagtttcgat 300
attctggaag cccgctacgc cattgaagcc agcaccgcat ggcatgcggc aatgcgcgcc 360
acacctggcg acaaagaaaa gattcagctt tgctttgaag caacgctaag tgaagacccg 420
gatatcgcct cacaagcgga cgttcgtttt catctggcga ttgccgaagc ctcacataac 480
atcgtgctgc tgcaaaccat gcgcggtttc ttcgatgtcc tgcaatcctc agtgaagcat 540
agccgtcagc ggatgtatct ggtgccaccg gttttttcac aactgaccga acaacatcag 600
gctgtcattg acgccatttt tgccggtgat gctgacgggg cgcgtaaagc aatgatggcg 660
caccttagtt ttgttcacac caccatgaaa cgattcgatg aagatcaggc tcgccacgca 720
cggattaccc gcctgcccgg tgagcataat gagcattcga gggagaaaaa cgcatga 777

<210> 44
<211> 414
<212> DNA
<213> Escherichia coli

<400> 44
atgcgtaact ttgatttatc cccgctttac cgttctgcta ttggatttga ccgtttgttt 60
aaccacttag aaaacaacca gagccagagt aatggcggct accctccgta taacgttgaa 120
ctggtagacg aaaaccatta ccgcattgct atcgctgtgg ctggttttgc tgagagcgaa 180
ctggaaatta ccgcccagga taatctgctg gtggtgaaag gtgctcacgc cgacgaacaa 240
aaagagcgca cctatctgta ccagggcatc gctgaacgca actttgaacg caaattccag 300
ttagctgaga acattcatgt tcgtggtgct aacctggtaa atggtttgct gtatatcgat 360
ctcgaacgcg tgattccgga agcgaaaaaa ccgcgccgta tcgaaatcaa ctaa 414

<210> 45
<211> 897
<212> DNA
<213> Escherichia coli

<400> 45
atgaccgatt tacacaccga tgtagaacgc tacctacgtt atctgagcgt ggagcgccag 60
cttagcccga taaccctgct taactaccag cgtcagcttg aggcgatcat caattttgcc 120
agcgaaaacg gcctgcaaag ctggcagcaa tgtgatgtga cgatggtgcg caattttgct 180
gtacgcagtc gccgtaaagg gctgggagca gcaagtctgg cgttacggct ttctgcgcta 240
cgtagctttt ttgactggct ggtcagccag aacgaactca aagctaaccc ggcgaaaggt 300

gtttcggcac cgaaagcgcc gcgtcatctg ccgaaaaaca tcgacgtcga cgatatgaat 360
cggctgctgg atattgatat caatgatccc ctcgctgtac gcgaccgtgc aatgctggaa 420
gtgatgtacg gcgcgggtct gcgtctttct gagctggtgg ggctggatat taaacacctc 480
gacctggagt ctggtgaagt gtgggttatg gggaaaggca gcaaagagcg ccgcctgccg 540
attggtcgca acgctgtggc gtggattgag cactggcttg atttgcgcga cctgtttggt 600
agcgaagacg acgcgctttt tctgtcgaaa ctgggcaagc gtatctccgc gcgtaatgtg 660
cagaaacgct ttgccgaatg gggcataaaa caagggctga ataatcacgt tcatccgcat 720
aaattacgtc actcgttcgc cacgcatatg ctggagtcga gcggcgatct tcgtggtgtg 780
caggagctgc tgggtcatgc caacctctcc accacgcaaa tctatactca tcttgatttt 840
caacaccttg cctcggtgta cgatgcggcg catccacgcg ccaaacgggg gaaataa 897

<210> 46
<211> 1659
<212> DNA
<213> Escherichia coli

<400> 46
gtgttgaccc cgttgacggc cttcgcggga gtgcggttgc gctggcctgc catgatgcga 60
ctcacttgca tcggcattct ggcgcagttc gcgctcctgc tgctcgcctt tggcgtactg 120
acgtattgtt ttctcatcag cgatttctcg gtcatttatg tcgcgcaaca tagctatagc 180
ctgctgtcgt gggaactcaa gctggcagcg gtgtggggcg gccatgaagg ttcgctgctg 240
ctttgggtgc tgctgctttc cgcctggagc gcgctgtttg cctggcatta tcggcagcaa 300
accgatccgc tatttccgct gacgttagcc gttttatctc tcatgctcgc cgcactgcta 360
ctgtttgtgg tgctgtggtc cgatcccttc gtgcggatat ttccaccagc aatcgaaggc 420
cgcgatctca atccgatgct gcaacatccc ggtcttatct ttcatccacc gctgctttac 480
cttggctatg gcggtttgat ggtagcggcg agcgtggcgc tggcgagtct actgcgcggc 540
gagtttgatg gtgcctgcgc ccgaatttgc tggcgctggg cgttacctgg ctggagtgca 600
ttaacggcgg ggatcatcct cggttcctgg tgggcctact gcgaactcgg ctggggcggc 660
tggtggttct gggatccggt ggaaaacgcc tctttattac cctggctttc tgccactgcg 720
ctgctgcaca gtttgtccct gacacgccag cgggggattt ttgccactgg gtcgctgttg 780
ctggcgatag ttactctgat gctgtcgctg ctgggcacct taattgtccg ttctggcatt 840
ctggtttcgg ttcatgcgtt cgcgctggat aacgtccgcg ccgtgccgtt gttcagcctg 900
tttgcactga ttagccttgc gtctctggct ctgtatggct ggcgagcgcg ggacggtggc 960
ccggcggtgc atttttcggg gttatcgcgg gaaatgttaa tcctcgctac gctgttgctg 1020
ttttgcgcag tgctactgat cgtgctggtg ggaacgcttt atccgatgat ttacggcctg 1080
ctgggctggg gacgcctctc cgttggcgcg ccgtatttta accgcgcgac gttaccgttt 1140
ggtctgttga tgctggtggt gattgtgctg gcgacgtttg tctctggcaa acgcgtgcag 1200
cttccggcgc tggtagctca tgcgggcgtg ctgttatttg ccgctggggt cgtggtctcc 1260
agcgtcagcc gtcaggagat cagcctgaat ttacagccgg gtcagcaggt gacgctggca 1320
ggatacacct tccgttttga gtgcctcgat ctacaagcca aaggcaatta caccagcgaa 1380
aaagcgatag tggcactgtt tgaccatcag caacgcattg gtgaactgac gccggagcgg 1440
cgtttttatg aagcacgccg tcaacaaatg atggaaccgt caattcgctg gaacggcatc 1500
catgactggt atgcggtcat gggggagaaa actgggccgg atcgttacgc ttttcgtttg 1560
tatgtacaaa gcggtgtgcg ctggatctgg gggggaggat tgttgatgat tgcgggcgca 1620
ttgttaagcg gatggcgggg gaagaagcgc gatgaataa 1659

<210> 47
<211> 450
<212> DNA
<213> Escherichia coli

<400> 47
atgaaaaaga ttgcatttgg ctgtgatcat gtcggtttca ttttaaaaca tgaaatagtg 60
gcacatttag ttgagcgtgg cgttgaagtg attgataaag gaacctggtc gtcagagcgt 120

actgattatc cacattacgc cagtcaagtc gcactggctg ttgctggcgg agaggttgat 180
ggcgggattt tgatttgtgg tactggcgtc ggtatttcga tagcggcgaa caagtttgcc 240
ggaattcgcg cggtcgtctg tagcgaacct tattccgcgc aactttcgcg gcagcataac 300
gacaccaacg tgctggcttt tggttcacga gtggttggcc tcgaactggc aaaaatgatt 360
gtggatgcgt ggctgggcgc acagtacgaa ggcggtcgtc atcaacaacg cgtggaggcg 420
attacggcaa tagagcagcg gagaaattga 450

<210> 48
<211> 915
<212> DNA
<213> Escherichia coli

<400> 48
atgacggttc ctacctatga caaatttatt gaacctgttc tgcgttatct ggcaacaaaa 60
ccggaaggtg cagccgcgcg tgatgttcat gaggctgccg cggatgcatt aggactggat 120
gacagccagc gagcgaaagt cattaccagc ggacaacttg tttataaaaa tcgtgcaggc 180
tgggcgcatg accgtttaaa acgtgccggg ttgtcgcaaa gtttgtcgcg tggcaaatgg 240
tgcctgactc ctgcgggttt tgactgggtt gcgtctcatc cccagccaat gacggagcag 300
gagacgaacc atctggcctt cgcttttgtg aatgtcaaac ttaagtcacg gccggatgcc 360
gtcgatttag atccgaaagc cgactctccc gatcatgaag aacttgcaaa gagcagcccg 420
gacgatcggt tagatcaggc gctaaaagag cttcgtgatg cggtggctga tgaggttctg 480
gaaaacttat tgcaggtttc tccttcgcgc tttgaagtca ttgttctgga tgttttgcat 540
cgcctggggt atggcggcca ccgtgatgat ttgcagcgtg ttggcggtac tggagatggt 600
ggcatcgatg gtgtgatatc gcttgataaa cttggcctgg agaaagttta tgttcaggca 660
aaacgttggc agaatactgt aggcaggcca gaattacagg catttacggg cgcactggct 720
gggcaaaaag cgaaacgtgg ggtgtttatt accacttctg gatttacttc tcaggcgcgt 780
gactttgccc aatccgtcga gggtatggtg ttggttgatg gggaacgcct ggtgcactta 840
atgatcgaaa acgaagtagg ggtttcttca cgtttgttga aggtgccgaa actggatatg 900
gactattttg agtga 915

<210> 49
<211> 1929
<212> DNA
<213> Saccharomyces cerevisiae

<400> 49
atgtcaaaag ctgtcggtat tgatttaggt acaacatact cgtgtgttgc tcactttgct 60
aatgatcgtg tggacattat tgccaacgat caaggtaaca gaaccactcc atcttttgtc 120
gctttcactg acactgaaag attgattggt gatgctgcta agaatcaagc tgctatgaat 180
ccttcgaata ccgttttcga cgctaagcgt ttgatcggta gaaacttcaa cgacccagaa 240
gtgcaggctg acatgaagca cttcccattc aagttgatcg atgttgacgg taagcctcaa 300
attcaagttg aatttaaggg tgaaaccaag aactttaccc cagaacaaat ctcctccatg 360
gtcttgggta agatgaagga aactgccgaa tcttacttgg gagccaaggt caatgacgct 420
gtcgtcactg tcccagctta cttcaacgat tctcaaagac aagctaccaa ggatgctggt 480
accattgctg gtttgaatgt cttgcgtatt attaacgaac ctaccgccgc tgccattgct 540
tacggtttgg acaagaaggg taaggaagaa cacgtcttga ttttcgactt gggtggtggt 600
actttcgatg tctctttgtt gttcattgaa gacggtatct ttgaagttaa ggccaccgct 660
ggtgacaccc atttgggtgg tgaagatttt gacaacagat tggtcaacca cttcatccaa 720
gaattcaaga gaaagaacaa gaaggacttg tctaccaacc aaagagcttt gagaagatta 780
agaaccgctt gtgaaagagc caagagaact ttgtcttcct ccgctcaaac ttccgttgaa 840
attgactctt tgttcgaagg tatcgatttc tacacttcca tcaccagagc cagattcgaa 900
gaattgtgtg ctgacttgtt cagatctact ttggacccag ttgaaaaggt cttgagagat 960
gctaaattgg acaaatctca agtcgatgaa attgtcttgg tcggtggttc taccagaatt 1020
ccaaaggtcc aaaaattggt cactgactac ttcaacggta aggaaccaaa cagatctatc 1080

aacccagatg aagctgttgc ttacggtgct gctgttcaag ctgctatttt gactggtgac 1140
gaatcttcca agactcaaga tctattgttg ttggatgtcg ctccattatc cttgggtatt 1200
gaaactgctg gtggtgtcat gaccaagttg attccaagaa actctaccat ttcaacaaag 1260
aagttcgaga tcttttccac ttatgctgat aaccaaccag gtgtcttgat tcaagtcttt 1320
gaaggtgaaa gagccaagac taaggacaac aacttgttgg gtaagttcga attgagtggt 1380
attccaccag ctccaagagg tgtcccacaa attgaagtca ctttcgatgt cgactctaac 1440
ggtattttga atgtttccgc cgtcgaaaag ggtactggta agtctaacaa gatcactatt 1500
accaacgaca agggtagatt gtccaaggaa gatatcgaaa agatggttgc tgaagccgaa 1560
aaattcaagg aagaagatga aaaggaatct caaagaattg cttccaagaa ccaattggaa 1620
tccattgctt actctttgaa gaacaccatt tctgaagctg gtgacaaatt ggaacaagct 1680
gacaaggaca ccgtcaccaa gaaggctgaa gagactattt cttggttaga cagcaacacc 1740
actgccagca aggaagaatt cgatgacaag ttgaaggagt tgcaagacat tgccaaccca 1800
atcatgtcta agttgtacca agctggtggt gctccaggtg gcgctgcagg tggtgctcca 1860
ggcggtttcc caggtggtgc tcctccagct ccagaggctg aaggtccaac cgttgaagaa 1920
gttgattaa 1929

<210> 50
<211> 921
<212> DNA
<213> Saccharomyces cerevisiae

<400> 50
atgtccgacg aaattgtttg gcaagtgatt aatcaaagtt tctgctctca tagaattaag 60
gcacctaatg gtcaaaattt ttgcagaaat gagtataacg tcactgggtt gtgtacaagg 120
caatcatgcc cacttgccaa ctccaagtat gcaacagtga agtgtgacaa tgggaaactg 180
tacttgtata tgaagacgcc tgaaagagcg cacactcctg ccaagttatg ggaaagaatc 240
aaactatcca aaaattacac gaaggcttta caacagatcg acgaacacct actacattgg 300
agcaagtttt tccgtcataa gtgtaaacag agatttacaa aattgacgca ggtcatgata 360
acagagagac gtttagcatt aagggaggag gagagacact acgttggtgt agcaccaaag 420
gtcaaaagaa gagaacaaaa cagggagaga aaggcattgg tagctgccaa gattgaaaaa 480
gccatcgaaa aagaattgat ggacagatta aagagtggtg cttacggtga taaaccatta 540
aatgttgacg aaaaggtctg gaagaagata atgggtcaaa tggaagagga gaattctcag 600
gacgaggaag aagactggga tgaagaagag gaaagtgatg atggggaagt tgaatacgtt 660
gcagatgatg gcgaaggtga atacgttgac gtggatgact tagaaaagtg gttagctgac 720
tctgacagag aagcttccag cgccagtcaa agcgaaagcg acagtgaaag cgagagcgac 780
agcgatagtg atgaagaaaa taagaattca gccaagagac gtaagaaggg aactagtgcc 840
aaaactaaac gtcctaaagt cgaaatcgaa tacgaggagg aacacgaagt tcaaaatgct 900
gagcaagaag tggcacaata a 921

<210> 51
<211> 1869
<212> DNA
<213> Saccharomyces cerevisiae

<400> 51
atggtacgtc gatggattcc tagtggcagg catcttcgca ataatgacaa cactggtgat 60
gatgacgaca gcgagttcac aaactcgatg gattctggga tgtccatacc atcactcagg 120
gactccatga ccacgaggtc atctcataac gatcccatca agcctgctct gatgaacgat 180
tccaacaaag tcaaaaattt ggagaaggag ttgactaatg ccaaaatcaa gattcaagta 240
ctctatgaat acattcgcag aatccctaat aaagacggca atgcaccatc gctgggcaat 300
gacactgatt ttagaaattc gattatcgag ggtctaaatc ttgaaataaa caaattgaaa 360
caggatttaa aggcgaagga agtcgagtac caagatacgc tacaattcgt tcaagagaat 420
ttagaaaatt cggaaagtat cgtgaatacg atcaatcacc tattgtcctt tatattgacg 480
cattttaatg agcaagatga aaatgcccat cttcttgata aagaagagag ggagaccctg 540

gaggaaactt tagagctgag ctcggattat gttcttgaaa aaatggatac tttgtccaag 600
ttcatcatcc aattcttgca ggactttttg cattccaaaa gtcgagcgga atcaaagcag 660
gataaggaag aatttctttc actggcccag tcctcaccag caggatcaca gttagaaagt 720
agggactcac catcaagtaa agaggagaat actgatggtg gataccagaa tgacgaaatt 780
catgacagta acaaccacat tgatacagaa aatgtaatgg caaacagtac ttccttacca 840
atttcagccg tcgagtctcg ttttgagaaa accttagaca ctcaattaga gattgtcata 900
gaaattttgc acaaagaata tgatcaattt ataaattcca ttagattgaa attcgagaaa 960
tcgcaaaaat tagaaaaaat aatagcgtcc aaactaaatg agcagtctca tctactagat 1020
tccttagaat tggaagaaaa ttctagttcc gttatagaaa aacaagatca tttgatttcc 1080
caactgaagg aaaagatcga atcgcaatct gttttgataa acaatttgga aaaattgaag 1140
gaagacatca ttaaaatgaa acaaaatgaa aaagttttaa ccaaagaact ggaaactcag 1200
accaaaatca ataaactaaa ggaaaataat tgggacagct acatcaatga cttggaaaaa 1260
caaatcaatg accttcaaat cgataaatca gaggaattcc acgtaataca aaatcagtta 1320
gacaaattag acttggagaa ttaccaatta aaaaatcagt taaatacttt ggacaaccaa 1380
aagttaatac tatcccaata tgaaagcaat tttatcaaat ttaatcagaa ccttttacta 1440
caccttgaca gtattttcaa tattctgcag aaaatcttac aagaaagttc tattgcccaa 1500
tttgacagga aaatgaaatc cataaaatct gtaccaaatg cgttgaaaaa cttgaatttg 1560
attcagccca aactagaatc gctctacact tttatagaga ctgcattaga gtccataata 1620
aactcatata tctcttcact gatttccatg gaaaccccag agcagccaca tcaacagggc 1680
aatgaattaa cggcaactcc aaataaagaa ctgaccttac gaatagagga gttacaaaga 1740
agatggatat ccgaaagaga aagacggaag ctcgatgcta acgcttcaga agctcgaatc 1800
aaggctttag aacaggaaaa tgagtcattg agatcgaaac ttttcaacct atcaatcaac 1860
aatccctaa 1869

<210> 52
<211> 705
<212> DNA
<213> Saccharomyces cerevisiae

<400> 52
atgcaaacac cttcagaaaa taccgacgta aagatggata ctctcgacga acccagtgca 60
catttaatcg aagagaatgt agctcttccc gaagacacat tcagttcaca tctgagttat 120
gtactttatg aaattgctca ttgtaaaccg atcatgttta tgatcatcat aatcgtgagt 180
ttgatctcat tgattgtgct ttttcatgat aacgacgggt gcactgtgat cttagtgatg 240
tcccttatag tagcctccat ggctttaatg gtggttgcag cattcacatt cgggaaagcg 300
atcactgaac aggagttcat gataaagctt ttagtggagg tgatcgcacg caagcctgcg 360
gggaaggaat ggggtactgt cgcatataat atgaaccaat atctattcat gaagagacta 420
tggtataccc cgtactattt ctatagcggc aagaagtgcc atgagttctt caccactctt 480
atcaaggaag tgaattctgg ttcgcactcg gattcctcat cgaatagtgc cgaggataca 540
caatcacctg tctcagcagg gaagacttca aatggtctaa acaactttta tagtattaga 600
tcagacccta ttttgatggc atatgttttg aaggcaacac aaatagaaaa ggaggctcaa 660
agtgaatact ggagaaagca atatcctgac gctgatttac cttga 705

<210> 53
<211> 486
<212> DNA
<213> Saccharomyces cerevisiae

<400> 53
atgccacaag tacagtcgtg gtttcctgtt cagaaacaac cgacgctagc ggtaacattt 60
acaccattac cacaactgtc ccatgctcat ctaccactgc caccatcaca tcttgtgacg 120
aaaacggatg ccatgttcca gcaccaactg ctaccgacgc aactgcaacc gtttcctcca 180
agtcatacac cactgttact gttactcact gtgacaacaa tggctgtaac accaagactg 240
tcacttctga atgttctaaa gaaactgcag caaccaccat ttctccaaaa tcatacacta 300

ctgttaccgt tactcactgt gacgacaacg gctgtaacac caagactgtc acttccgagg 360
cttccaaaca aacatcattg gccactagca cagtcaccaa gtctgctgct ccaacttctc 420
atactgctgc ttccagcacc ttcactggta ttgtcgttca atccgaaggt atggctgctg 480
gtttga 486

<210> 54
<211> 435
<212> DNA
<213> Saccharomyces cerevisiae

<400> 54
atgaatacca acgagtccga acatgttagc acaagcccag aggatactca ggagaacggt 60
ggaaacgcta gctccagcgg cagtttgcag caaatttcca cgctaagaga gcaggacaga 120
tggctaccca tcaacaatgt agcgcgactc atgaagaata ctctcccacc gagtgctaag 180
gtatcgaaag atgcgaaaga gtgcatgcag gagtgtgtca gtgagctcat ttcttttgtg 240
actagcgagg ccagcgatcg atgcgctgct gacaaaagaa agacgataaa cggggaagac 300
attctcatat cattgcacgc cttaggattc gagaactatg cagaggtgtt gaaaatctac 360
ttggctaaat acaggcaaca acaggcgctg aagaatcaac taatgtatga gcaggacgac 420
gaagaggtgc cttga 435

<210> 55
<211> 315
<212> DNA
<213> Saccharomyces cerevisiae

<400> 55
atgttgttca aaccaaaaac acgagcaata ccatcaccga ctgcaagaac tctaccagtt 60
tcgttcaaat tggcctcgtc ggacacaccc ttaattcttt cctctaagat ggaggaaact 120
tctgtgggtt gtgccttggt ggaagccaat cttctggtgg aagccaaagc agcagcggca 180
ggtcttgcgg ccttggtaga gttaattaga gttctcgata gagaacgaat agcagcagta 240
cgagccaaca ttattatatg tgcgtgtttt ttttatttat tttgttactg ttcttgcgat 300
agttatgaga gctga 315

<210> 56
<211> 336
<212> DNA
<213> Saccharomyces cerevisiae

<400> 56
atgtccctac ggccttgtct aacaccatcc agcatgcaat acagtgacat atatatatac 60
cctaacacta ccctaaccct accctatttc aacccttcca acctgtctct caacttaccc 120
tcacattacc ctacctctcc acttgttacc ctgtcccatt caaccatacc actcccaacc 180
accatccatc cctctactta ctaccaccaa tcaaccgtcc accataaccg ttaccctcca 240
attagccata ttcaacttca ctaccactta ccctgccatt actctaccat ccaccatctg 300
ctactcacca tactgttgtt ctaccctcca tattga 336

<210> 57
<211> 1302
<212> DNA
<213> Saccharomyces cerevisiae

<400> 57

atgattgtgg ggttgacact ttattttgta ttattccgtt caatacagta tttactggtt 60
tttttgactc caattaggca gtttgataca tcaacatcac ttttattaaa cgaattatgt 120
tcttctccct ctgaaatcaa cagttattgg aacaagtatt tttggaataa gctactatca 180
tgggacagtg ttttttttat caagaacata acttccaaaa acggaaaacc tcaatttgag 240
catgaatacg cgttttctca gttgtggact tttttcgtta ggctgtttat taaaagtaat 300
aacgatagca tctaccatgc cttaagggtt ggagttgcaa tagaaaatgt tttattttac 360
ttgtcaggta ttgttttata ttttctaaca aaaaaaattt tcagccaaaa tataaggcaa 420
tcacagtttg ctagaactat cgctaaaaaa acatctctgt tgtttttctt aacgagtgcc 480
gctggatttt taacaagcat atattctgaa ccattatctt ttttttttgc atttgttggt 540
atttggagtc gtgaatgcac catttccgtg cccgtattgg gtcaattcga tatttcgtgg 600
agatattggt ttccttactc ctttatcagc atggcttgct ttaccttagc atccttgaat 660
cgttcaaact gtgttttgtt agggatttac tttatttttg accttattga actaacaaag 720
aacaggaagt ttgtaaaagc aatatgtttc ccactattat caggatcatt aatgttttct 780
gctctactat atcaacaata ttacctacca tataagacat tttgtcctca aaggggtgaa 840
tggtgtaaat ctcaattgtt ttcaagcatt tttatcacga aaacttcttt atattcctac 900
attcagagtc attattgggg agttgggtta ttgaaatact ggaccccaaa caacatccca 960
aactttttgt ttgctgtccc aaatattatt attttaatct attcctccat atatttcagc 1020
aaaatttatc cctcctataa cttgaaggct ctcgtatgga tcaccagagc actggtcgtc 1080
atagtatgct tttttgccca tgtccaaatt ctgaatcgta tagcctcctt tttacccttg 1140
cacctttggt atttggctga tagattggtt aaaacttctg atccaaaaaa aatggaaaat 1200
ccgaaaggtg acgataagat agtcaagttt tacatatact ggttggcatt ctggatacct 1260
ttacagacta tcctatttgc agctttttta ccaccagcct ga 1302

<210> 58
<211> 873
<212> DNA
<213> Saccharomyces cerevisiae

<400> 58
atggaaaagc tattacagtg gtctattgcg aattctcaag gggacaaaga agctatggct 60
agggccggcc aacctgatcc taaattgcta cagcagttat tcggtggtgg tggtcctgac 120
gatccaacct taatgaaaga atccatggct gttattatga atccggaggt tgacttagaa 180
acaaaactcg ttgcatttga caactttgaa atgttgattg agaacttaga taatgctaat 240
aatatcgaaa atttaaaact gtgggagcca ttgttggatg ttcttgttca gacgaaggat 300
gaagaactac gtgctgctgc tttatccatt attggaacgg ctgtgcaaaa caacttggat 360
tcgcaaaata atttcatgaa atacgacaat ggtctgcgaa gccttatcga aatagctagt 420
gacaagacaa agccactcga cgtgagaaca aaagcttttt acgcactatc taatctaata 480
agaaaccaca aagatatctc agaaaagttt ttcaaattaa atgggctcga ctgcatagca 540
cctgtattaa gtgataacac cgccaaacca aaactgaaaa tgagagccat tgccttattg 600
accgcatatt tgtcatctgt taagattgat gaaaatataa tcagtgtgct gagaaaggat 660
ggagtaattg aaagtacgat tgagtgcttg tctgacgaga gtaacttgaa catcatagat 720
agagttctgt cttttctctc tcacctgata tcttccggaa taaaatttaa tgaacaggaa 780
ttgcacaaat tgaacgaagg ttacaaacat atcgagcctc taaaggacag acttaatgaa 840
gacgattatt tagccgtaaa gtatgtatta tga 873

<210> 59
<211> 1035
<212> DNA
<213> Saccharomyces cerevisiae

<400> 59
atgcattgcc caatttgcca ccatagagcg catgtagtgt actgtgcaca ttgtatcaat 60
acgagcccaa gtctgctact gaagctaaaa ctagatttaa ttttattaaa ggatgagaat 120
aaagaactta acgggaaagt cgaacaaata ttaaacgagg ccatgaacta tgaccagtta 180

gatatcaaga ggatggagaa aaagaaagat cccctgatga atagtctcat gaagttagat 240
gttttacgaa tgaaaaagaa taataacctg attaggcaca ggatagaaca gctaaatgag 300
cggatttata gtaaaaggaa tcacatcagc gaattgaaag tagaaattga caattataaa 360
tgttataagg tgggtactgg tacggacaaa ttaagagagc aagtggaaat tagtgatgca 420
aaaaataagc tggcacaagt atcgaagatt tgtgaatcgg ctagagatta caagctcaat 480
ctactaaata attggtttgt gatccagaag ctgcaagata atttccaaat acctttcgct 540
atagcttttc agccactaat atctttgaaa aacttccgta ttttacctct tgctataaca 600
aacgattcca tcaacatcat gtggaagtat attagcttct tctcagacat tcttatgatt 660
aaacttccct acacaaataa aatctgtgaa caacccatgt ttgagttttc cgatagcata 720
cagacagtag tacaaaggtt gatcaagctt atcataaata ttttacagat atgtagacat 780
ttgaaactcg taccttcaac acccatggat atcccatggc tactggacca gtacgatgtg 840
gatgggctgt tctataatat ggtgaaacgg aacaagatga agtgtaggtc cgtctcgcta 900
tattggactt ttgggatgtt gtactcgatg gttttggata acatgaataa tccacaaaga 960
ggacatcctg caaggcggac cgcaccacct ccaacagtca caggacctca tgaccgatgg 1020
tacgtggtag gctag 1035

<210> 60
<211> 939
<212> DNA
<213> Saccharomyces cerevisiae

<400> 60
atgaatataa tgaaaagaca attatgcacc tcctctaagc gatttttttc tacagcaaaa 60
aacgttgtca aatataatac gattcaggat atcaggaaca aatattttac tggtacgcct 120
ttgtcgatgt gtactgcgta tgacttcatt actgcaacat gggttaacaa ggctaattgt 180
gatttactct tagtcggaga ttctttggcg atgacttcat tggggtacga tagcacaatt 240
acactatcct tgaatgagtt taaatatcat gttgcctctg tgtgcagagc tgaaggttct 300
tccatggtag tggtagacat gccattcggt acgtttgaat cgggcatatc tgatggcttg 360
aagaatgcca tagatatcat gaaattggac agtaaagtca cctcagtcaa agttgaagtc 420
ggttcttaca ccaaagacaa atatgcaatg aaatttattg aagaactgtg ctcccggggt 480
atacctgtta tggctcatat aggactaact ccccaaaaag ttcattcact tggaggttat 540
aaagtgcaag gtagcaaaag tctattacaa atgcaggagc tgtatgaaac cgccatgcaa 600
ctacaaaaaa ttggctgttg gtctatcctg attgaatgtg tcccccataa gatggctcag 660
ttcataacat ctaaactctc agtaccaaca ataggtatcg gtgcaggtaa cggtaccagt 720
ggacaagttc tagttatatc tgaccttctc gggatgcaag gcgattctgt cccaaaattt 780
gtgaagcaag ctgtaaatat gacagatatt gccactcagg gcttgaagga gtacattgca 840
agtgtagagg ataggacatt tcctgagaga ggtactcata ctttcaaagt taaggaggac 900
ttatggaatg aattcctctc ttcaattaac gaaaagtaa 939

<210> 61
<211> 1128
<212> DNA
<213> Saccharomyces cerevisiae

<400> 61
atgaatatgg cagaacgtgc agaagcaaca aaaagttggt cctgtgagcc cctttctgga 60
aagacgctag aagagattgt tcaaaatgca gaaaatgcag ctgatttggt agcttacatt 120
cgcaaaccag aggtagattt ggactttagg ttgaagttta tcgcagaaca tgaggagttt 180
tttaatgaac aactatcgga tcgaaattca agaataagga cgtgtcacaa cttgagcgat 240
aaagggatac ggggtgatac tgttttcgtc ttcgtgcctg ggttggctgg caacttagaa 300
caatttgaac ccttactcga gcttgtggac tctgatcaaa aggcattttt gacgttagat 360
ctgcccggct ttggtcatag ttcagaatgg agcgactacc ccatgctgaa agtggtggaa 420
cttattttcg ttcttgtatg tgacgtctta agaaaatggt ctacagcggt gccgaataat 480
gataacgtaa atccatttaa cggtcataag atagtgcttg tagggcattc tatgggttgt 540

tttctggcat gtcatcttta cgagcagcac atggctgata caaaagcggt acaaacgcta 600
gttttgttga cgccccccaa agcacatatc gagcagcttt ctaaggataa acatattatc 660
cagtgggctc tctatggtgt gtttaaattg ccatggctat ttgacgttta tagaaacaaa 720
ttcgatcagg taaaaggtct acaaagttcc ggcatcaagc agtattttta tcaacaaggt 780
gatgatgtca agttgaaata caggaagttc tggcaattta aaaacaacat aagcaacaaa 840
agtagaacca tcataggtta tttactagga tgggaaactg ttgattgggt taaattcaat 900
ggtgtattga cgcaaacgga tatgaaacag aaaattataa tatttggcgc tgaaaaagat 960
cctatagctc ctattgaaaa tttggaattt tacaaacaaa ctataaataa agaatgtctt 1020
cgtaaagtga ttattttacc tgactgttct cataatttat gcttcgaccg tccagaattg 1080
gtgtgtgaaa actttcaacg agaggtgatt gataattcca aattgtag 1128

<210> 62
<211> 1347
<212> DNA
<213> Saccharomyces cerevisiae

<400> 62
atgttgcgac tttttttcact ggcacttatc acatgcttaa tctactattc caaaaatcag 60
ggcccatctg ctcttgttgc ggccgtggga tttggtatag caggctattt agctacagat 120
atgttgattc cacgtgtggg caaatccttc atcaaaatag gcctattcgg taaggacttg 180
agtaaacctg gccgtccggt gcttccagaa acaataggtg ctatccctgc tgcagtttat 240
ttatttgtaa tgttcattta cattcccttc attttctaca agtatatggt cataaccact 300
tcaggtgggg gccatcgcga tgtctcagtg gttgaagata acggtatgaa ttctaatatt 360
tttcctcatg ataaactatc tgaatatttg agcgctatcc tatgcttgga aagtacggtc 420
ctcttgggta tcgctgatga tttatttgat ttacgttgga gacataagtt tttcttgcct 480
gccattgcag ccatcccact gctaatggtt tattatgtgg attttggagt tacgcatgta 540
cttattcccg gattcatgga acgctggttg aaaaagacta gtgttgattt ggggctgtgg 600
tattatgttt atatggcatc gatggcaatt ttttgcccca actccatcaa catcctggca 660
ggtgttaatg gtttagaggt tggccaatgt atagtgttgg ccatcttagc gctattgaac 720
gatttgctgt acttctcgat ggggccatta gccacaagag actcccatag gttttccgct 780
gttttgatta tcccattttt gggtgtatcc ttggcgctat ggaaatggaa tcgttggccc 840
gccacagtgt ttgtgggaga tacatattgt tatttcgctg gaatggtatt cgcagtcgtt 900
ggtatactgg gtcatttttc aaagacgatg cttttgctat tcattcctca aatcgttaat 960
tttatttatt catgtcctca gctattcaaa ttggtcccct gcccgagaca taggttacct 1020
aaatttaatg aaaaagacgg tttaatgtat ccatcaagag caaacttaaa agaagaaccg 1080
ccaaagagca tttttaaacc gatcttgaag ttactatact gtctccattt gattgacctg 1140
gaatttgatg aaaataatga gattattagc acctctaata tgacgttaat aaacttgaca 1200
ttagtatggt ttggccctat gagggaagac aaattgtgca atacaatctt gaagttgcaa 1260
ttctgcattg gaattttggc tttacttgga agacacgcta taggcgctat catctttggc 1320
cacgacaacc tatggacagt acgttga 1347

<210> 63
<211> 2064
<212> DNA
<213> Saccharomyces cerevisiae

<400> 63
atgaaacctc cgttaaacat gtcacgttca aataaaccac ttactcagga agctaatagt 60
tcggcgcata ttgatagggc acatcagcta gcacaggatt ttaacagtaa acaggatgat 120
accgcactaa cttcactgcc ccacaagaac cccgacatat tcagatttga gaacaacatc 180
acagctcata gctcgcgtcg tggttctcta tatagagaca gcgatgctac tgtggttttg 240
ccgctttctg aacatacccc tagattatca atggatgatc cataccgcca attactacaa 300
caggctgaaa tctcacaact acgaagtaag aaaaaaaggc attccagtcg agttttgcgt 360
acttcgttta tatcttttgt ggtgctagtt tcctcgctgt caggttggga ccaaggcctc 420

atatctggta acgttatgac tttgtcgttc cagaagtatt ttcattaccc acttacttct 480
ccgctgggca atatagtgag cattgttaat ctgggtgcgt tcatggcgtc gttatttgtc 540
tattctggca tcctggagcc ttgtagcagg aaaaaaatgc tccaaataag cacaatgata 600
tactctttgg gtgccattgt tcaggtcttg gctctaaacc aatggtgttt actactgggt 660
agattcttac tcggagtagg tatgggcttt gccttttcta tggtaataat ttatcaattc 720
gagtttcccc ttccctgtat aagaaaacgg acattaattt ccattcaatg cgtctctagt 780
gtcattgcgt attcattcgg aatatggatt aattgtgctt tcagatattt gggctttgca 840
tggagatacc ctttgtcaac acatgtggca cttggaataa tactaaacct aatgtcgttc 900
tatcttattt tagagtcccc ttcgtggctg ttgaagcaaa agaacgacgt agaggcattg 960
gttttaatat cgaatgtttt cgatgatgga aatttcgaag aaaaccaaac tcagttgaaa 1020
tttagggtcc tgaaacgaga catattatta aagtcacacc ttcaaaaaaa ctcctatcca 1080
tatgcgtata tcttaaagga tttttcttca ataataaaat tgttgatagg tttccaactt 1140
ctcacacgaa caaatggagt ggatgctttc ttgtattact caccgttaat attacaacaa 1200
atggggagag gagaaagaaa atctatctat ttaacggggc ttaatgcttt aatttacagc 1260
atcgtaatat tagcatatgt tcccttggtg ttacggaaac gtaaagagaa aacgaatgta 1320
ctcttaggtt cgattgtgat gtgtgcattg ctatttacca tatcttttac agactggttt 1380
cccaagagca caacgaggta tatatcaata ctatttgctg tttttctttt tacgcacttc 1440
atcagttggg attctatagg atgggttatg accattgagc ttctacctca cttgtcacag 1500
gccccagtga tactactagt ctcaaacttt tattggattt ttaagtggtt cgtcagctta 1560
ataaccccta tacttattga ccgtctgtct tggaagtttt atcttattcc atctttatct 1620
tcctttattt ccattatatt tgtgctgaag attttcctta tagaaacccg tgacgaaagg 1680
ttagattctg atgatgattc taccggaaac ggtagcggta accatgatga tgtttttgac 1740
gataccggtt ctgaattctc ctcctcacca tctttttccg cgtatcagat aaacactcta 1800
ggaagtagta tcaaacaaaa taatcaagcc tattcatcta ttcaaaatga gcagattcta 1860
cccaaaaacg gaaacttatc gaaccagaca cacggttctg cacaaaatgt ttatttcatt 1920
acatctgatt caggaccatc aaggactgga gaatttttca gttttcataa cagaaccgat 1980
ccaaatatca gcgacaacat tgctgcgaat aaacccagtt caggtggagg ccagaattcg 2040
cctggagaca tggcagttgc ctag 2064

<210> 64
<211> 633
<212> DNA
<213> Saccharomyces cerevisiae

<400> 64
atgttctcat attcagattt ctgttctatc ggtacggcca tgatcttatc ggccacaacg 60
ttcctaatgg gtgttttctt cagtaacatg ccatacgatt atcatctttt atttaatcct 120
aactctactc aagagcattt cgatttggca ctgagacatt accaaatttt acatgagact 180
ccattgccgg tgattgttac tttgtgcgtt gttgccggta ttggcttagt tggtggtaca 240
attaaggttt tcaagccaaa ccctgaactg cagatgtttg agtattgttc attggggttg 300
tacgtgttgg ctatctgtgt gtttctcact aatgtgaaaa caggtatcga ctgttctgtg 360
agccataatt ggggagaagt tacggaaaat cagggtttgg cagttattgc ttcctccaac 420
ataattttat tagttatgtt tgccggtgtt atcatcctac aaattggttt gtggtacagt 480
aactgggatt tgcaaaaaag attaaaggag ttttatgctc aagaagaaag agaagctgcc 540
aatgccggta aaaagactga gaaagttgac aatgcgaaaa agaatgataa caaatctaaa 600
ggtgctcaaa agaggaagaa cgccaaaaaa tag 633

<210> 65
<211> 354
<212> DNA
<213> Saccharomyces cerevisiae

<400> 65
atgaatttag aagagagcca atcaaatagg ggtacgctta atattaaaaa aatattagta 60

attgtttgta tgacgaataa aatttgtaag cacctgatcc aatccttatt attgagcaag 120
agacgtcttg tttctgtact ctttgataac tatcaaggcg acatgaaact aaggtcatct 180
ccatctgcca ttcctctagt tccgtcaacg aaaattcttc aagctatgga ggcccggaac 240
gacattgttg acagtctgat catcaatagt cacgcaacgg aaagtgatcg ccggacttta 300
ggagaccatg aggttagaca atgtgaggat accaacttcg gcttcaaaat ttga 354

<210> 66
<211> 1152
<212> DNA
<213> Saccharomyces cerevisiae

<400> 66
atgaaagaga atgaagtcaa agatgagaaa agcgtagatg tgttatcctt caaacagctc 60
gaatcccaaa agattgttct acctcaagat cttttcagaa gcagctttac ctggttttgt 120
tatgaaattt acaagtcctt agcgtttcgc atctggatgc tattatggct accacttagc 180
gtctggtgga aactttccaa caattgtatt tacccactta tagtttcact tctggtcctg 240
tttctgggac caatatttgt ccttgttatt tgtggacttt ctcgtaagcg ttccttatcg 300
aaacaactca ttcagttttg caaagagatt actgaaaaca caccaagttc tgatcctcat 360
gattgggaag tcgttgtagc aaatctaaat tcgtacttat atgaaaataa ggcttggaat 420
actaagaatt tctttttcaa tgctacggat tgtgagaaaa tgttcagaac aactgttctt 480
gaaccatttt ctttgaaaaa agataaagct gccaaggtta agtcatttaa ggattctgtc 540
ccttatatcg aagaggcttt gcaagtgtat gctacaggat ttgacaaaca gtggaagttg 600
tttattactg aaaaatcatg gagccctgtt ggcctggaag acgttcagct tcccaaggat 660
attcatcggt ccaagcttac ttggttttta aagcggattt tcactattta ctctctacca 720
ttatggcttg cttttttgaa ctgtatctgc gtgtcacagc atttttgcct tgcatttcgt 780
atcttgtgtc ctggattgtt tttcctcatg atggtgtggc ttttccaaaa tatgaggacg 840
actgctttac tcgtgaaaat ggaacacaag atgcaattct tgttgactat tataaatgag 900
caagaaagtg gtgcgaatgg atgggacgaa attgcaagga aaatgaatag gtacttgttt 960
gagaaaaaag cctggaagaa tgaagagttt ttcttcgacg ggattgactg tgaatggttt 1020
tttagccact tcttctaccg ccttctatct gcaaagaaat ctatgtggct tttaccattg 1080
aatgtggaac tatggccata tattaaagaa gcgcaattgt cccgcaatga agagtcttta 1140
atgaagaaat ga 1152

<210> 67
<211> 909
<212> DNA
<213> Saccharomyces cerevisiae

<400> 67
atgactgtta taaagacaga accaacaaca gaagtgacat tatattctcc accatcaaaa 60
gaatctttaa gtaaagatga cgcccacaga aaaaaacaaa ataataaacc accttcgagt 120
ataaatagcc gctcaggacc aaataagcat aaacttgccg ccaaagcacc ggaaaagaaa 180
atcaataaca cagataaaca agacctctct gcctttttat taaatccttc ccttattgtt 240
aaaccttctg agagcaaaaa aaaagaaaat attgtggcct ataatgatac accaggtata 300
aaaacagaac atacagcgtt tcagccgctg acaccaattt cgaaaaaaag ggccctgaaa 360
gagaaagcag catcagaaaa atgtgatagc tttgatcttt ccagagacga gaagccttat 420
attcagaaaa agtcaaagac gctttcttcc gtcacagaga ttaattcctc agaatataaa 480
ctctcactaa atggcgaaaa tacatcatcc ccagctaagg aaaaatcgca agaacccata 540
gaaaatcctg gaagttacca aaaaacaaga aattaccttt ttgaaaagcc cgatccttta 600
gacacgtgcc ttcaggacta ctcgagtatg ctaccgtcaa atgtagcaga agaagatcaa 660
gaatatttta tcagcgtagc tgattcgaca ttggaggagt ggactaacaa gggccaggaa 720
attattgacc aacagttcca gctctaccaa gaaataatca agaaacgaat agaactaagc 780
tacaaattta agggtattat ttctgtaatc aatgataggg cagatgccct agaagaacag 840
ggtcaacaac tagaaggaaa aattaagaaa gttaaaactt tggctaatga aattctcaat 900

attatataa                                                          909

<210> 68
<211> 327
<212> DNA
<213> Saccharomyces cerevisiae

<400> 68
atgatttata tgttggtttt tctggatcgt caacaactcg tgcatatttt tttgtttcga 60
agtagaggaa ccacaaatat tattaaagcg tgttattttt ttttcttact cttctgtaaa 120
cttctaaatg ctgctgaggc acccctacta gcaatttccc tatcaaagtt tgtttggctg 180
ttattaaggg tatgtaagac ctcgtatctg ctgctactga tcaccatgtt agaaggagca 240
gaatactttt ctctcgtagt tgggaattcc atttgcggaa gtggaggtga aggtgttggc 300
tgtcgatatc ctgttgtttt gatttga                                     327

<210> 69
<211> 675
<212> DNA
<213> Saccharomyces cerevisiae

<400> 69
atgtgggatt cgttaattgt aagcatcaac gatacgcaca agttggggct ggaggattgt 60
ctcgccgttt ttggccacgt tcccattacc aaggccgtca agcatgtcag actgacagag 120
atcgacacgc agacgtccac gtttacgctc aagttccttc acacagaaac gggtcagaac 180
atcgagaaga ttatctactt cattgacaat gacacgggca acgatacccg cactgctacc 240
ggaatcaagc aaatcttcaa caagatgttc cgcatcgctg cggagaagag gaagctgtcg 300
ctcatccaga tcgacactgt tgagtacccc tgcactctag tagacttgtt gatcctggta 360
ggcgtggcgc taccgccatt gtgctacctc tatcgtcctg cactgcacgc tatctttttc 420
ctggtcccta atcctgtggg ctccacctta gaggcgtggc tggatagtga tcttgtgctg 480
cgactcatca ttgttgccga gttcctgact cacgccctcg agacgctcat tttcgtcgtc 540
cctaggctca agtactaccg tgttcctggc gagttcgtgc ccgagtggct tcttctaggt 600
ctcctcgaag gttacggtcc tgcgagacgt ctagatacta aggctcgcac tcttggcgaa 660
gggtcagtta attag                                                   675

<210> 70
<211> 1449
<212> DNA
<213> Saccharomyces cerevisiae

<400> 70
   atggattttt atactactga tatcaacaaa aatgttgtgc ctttgttcag taaaggaaca 60
   gtagcaagaa cagcatcaaa agcacagtat ccatcatggt gcaacaacgc gttaaagtta 120
   actaatattc ttcttaaatc tcttcgatgt aaatttcaaa cgaaccgctg cgaggatgat 180
   cgaggttttg aggtatattg tgttatttta aaaagcatcg ctttattaat ggccgcgaaa 240
   gaatcattga tattactaca aatacctcct tctttaccat caggatttcc atttcgttca 300
   ccccaactgt catttacata tttatcaacc cgtcttagtg ggagccaaca caaatctacg 360
   cattctcatc acatcaatca tcaaactcat cccattcata gtagcagtag caacagtaat 420
   agtaataacc gtattcctac aaaaacagat tcctccaaac agcatacaca gcatttctct 480
   tttgctaatg cgggagcctc caatagagac gaactgctat caatcgtgcg caaaattgat 540
   aaatctaatt tgaagtgttg tgactgcggt agcaccgcaa cagtagaatg ggtgtcaatc 600
   aatttattgt gtatattgtg tatcaaatgt tcgggcgttc atcgatctct gggttcacat 660
   atctccaaaa ttagatcttt aacgctggat aatttcacct cgttagagtt gatgcatctt 720
   ttacaaaata atgtttccaa tagtaatgtt aatgccattt atgaaagcaa tctcaggaac 780
   tttccagtta aaaaaataac tgcaaattct gatgactcgg aaagatccaa attcattatt 840
   gataagtatc aatttaaaaa gtttgttatc gacagcaacc aaggcagaga ggcttctttg 900
   aagtctttaa ttaaagctat ccatttagat agtgttttca tgatgcaaag agccatcgcc 960
   caaagtaaat attccctaag agaattgacg gcaagcgaaa aagagcagaa tgatctaaac 1020
   cattcttcca tttttcaata ttccctaaag cactatgaaa ttgttgatgg aacgcccatt 1080
   tttttattta cagaattttt attgtgcaat ggtattcata tagataattt accgaagatt 1140
   actacgaatt ggtctccaaa ggttttagaa tattgggaaa ctaagttgga gatgtacgga 1200
   acattccaag ctgtaaatac ttctcgtcca agatcagggc cgcatttaaa tatgcattca 1260
   aatgtagatt cagcttcgtc ctacaataag aaacacgatt tgagagttaa catacctgaa 1320
   agaagtgcct cagcaagtaa aaggtggagt ttgagctcca ttcctaagtc ttctcaaaat 1380
   ttgatgtccc caacaaactt attaacaatg cataagtctt taaaattggc caaaaaggac 1440
   aaaaaataa 1449

<210> 71
<211> 471
<212> DNA
<213> Saccharomyces cerevisiae

<400> 71
atgccttgct tgttgccacc tacccaggta ccagaagcgc cttctatttc tgcaaataat 60
ggagtgttgt tttcgtcctt cgctctttta tttatgtttt tcaatagctt ggcaatcagt 120
ttgggatcta aagagttata cagatcctca cgttcttgca cgatttgttc atcactcatt 180
ccctgcagaa ctttaatatt ttcattatgg atagattttg cctcggatag tggggcgtct 240
gtactcgttt gttgcttttc agcatccttg ccactagttt ttttcttttg ggctcttttt 300
tctcttagtc tttccttcca agatgatatt ttcttgggtt tgtacaactc gggaaatccc 360
gttccacagt tgttggtact aagagtgcca ttgtcattac tttcaacaga gtcagatgta 420
tctttctcca ctatatcgcc cagtaagtcc attacgatgg tggcagagtg a 471

<210> 72
<211> 264
<212> DNA
<213> Saccharomyces cerevisiae

<400> 72
atggacgcat tgaactccaa agaacaacaa gagttccaaa aagtagtgga acaaaagcaa 60
atgaaggatt tcatgcgttt gtactctaat ctggtagaaa gatgtttcac agactgtgtc 120
aatgacttca caacatcaaa gctaaccaat aaggaacaaa catgcatcat gaagtgctca 180
gaaaagttct tgaagcatag cgaacgtgta gggcagcgtt tccaagaaca aaacgctgcc 240
ttgggacaag gcttgggccg ataa 264

<210> 73
<211> 309
<212> DNA
<213> Saccharomyces cerevisiae

<400> 73
atgagtcttt ctttccttct cttttcccct tttctccccc cctgtttctc gtccatctct 60
atctgtctat ctgtcttatc tactgttagt ttttttttcg ctttcacgat accgcattac 120
gtactgcgct gcggaagtgt ggatgagtgg catatacact cttctgcaga ggattttcgt 180
acacaacggt gtgtgtgtgc tgtgaagctg tctgcttcct tacttggctg tttgcttgct 240
tgtgcgtcat ggtcactttt gttagaagtg agccgcataa aatggcatgt cggcacagcc 300
tacagttaa 309

<210> 74
<211> 2787
<212> DNA
<213> Saccharomyces cerevisiae

<400> 74
atgaaggaca gtgtagactg cccaagcatt ctacccaccg accgcacttc agtgctttcc 60
gagactagca ctttggtcgg atcgagttcg catgtatatt cgaggcatgc tcctatgaac 120
agttatcata acagcatgaa cagcaacata taccacagcc ccaaagccag tagccctttg 180
gtgagctata agacatcttc gccggtgtta ttgaagaggg cgaccgctcc tgtcctgcca 240
agcttcaagc ccaaggaaca gcgatataat aagccgcagg gttgttcgct aattactgct 300
gtagaactgg gcaagattat tgaaacctta ccagatgaaa aggtgctctt acttgatgtt 360
aggccgttta ccgaacatgc caaatccatt atcacaaact ccattcatgt atgtttgcct 420
tccacgctgc tgaggaggaa aaatttcacc ttttccaaac tcttggacaa cttaactcct 480
agcgagcaaa gcgtgttgaa atctaaactg gccatcgata accttcggat tatcatttat 540
gatagcaccg caaatcaaac ggaaagttct gtttctttac cttgttatgg aatagcgtcc 600
aagctaatag agtttgatac taatgttaaa aaaaccgtgt ctatactaat gtgtggtttt 660
cctcaattca aaattctatt tccagaccat ataaacacca acacatttaa ctcagattgc 720
attagttcgg ctgaacctaa aagccccaag acaaacctta tgaacagtct acacaacaca 780
gcgccgcata tgacggcgac aacaccacta tcgtctccgc aaatgaacct taagttgaaa 840
gttcctgacg atagtaggtc agatcattca aacttttcca gttctccctc tccgcgtaac 900
gtcctatcag attctcccat gtcttcttct tcccccatat ctgccttatt taagttccag 960
ctgcctgcac cacaaacaaa cataaaccaa atgttcaaat tttcgcaaaa tgaagaaata 1020
atgggtttag agacgtattt gagtgcagtc aacatcaaag aagaacatga aagatggtat 1080
aataacgatt ctgccaaaaa atctttacaa aacttccaat tcccaaagaa ccaaaattct 1140
ctagaaaagg acactaataa agacaaacta gggtttcaga tcagatacga aaacctgagc 1200
aaaaattacg aaaaggaggt cattgactcc gttatacctg aatggttcca gcatctgatg 1260
tcaataccca agatcgagtt ggtttctcag tttcaaaaat tagatttttt ggaaaaaagg 1320
cgcttgaacc attctgtctc tttcaggaag aaggaaaact cgtttatact tgaaaagcca 1380
tcatcatatc ctgaacagtt aacatcaact tcttcttcaa caatcatgcc tccaaaattt 1440
cctgacgtga acaaagtaca aaagaggtcg cattctcagc caatcttcac tcaatacagt 1500
aaatataaat cgatgctcag ccttgaatca gattccgatt cggagtcaga tgacgttata 1560
atatcatcag gcgttgaatt aggagccaag aacaggtaca aggatatttt tccctacgag 1620
cattcgagag tgattctaaa aaaaggtttg caatcatcaa aaggtatcaa gcattcacat 1680
tctacttccg atggtggcat cctcgacaat tatatcaacg caaactattt gtctctgcca 1740
aggttttctg tagagcaaaa ttcgtcattt caaacaacta caaccacaac aagaagagtg 1800
cgctatatag cgacgcaagc tccaatgccc tccactgtac atgactttta tacatgcata 1860
ttgaataatg gcgttccgtt ggttctctca ttgacaaatg atttcgaaaa tggcattgag 1920
aaatgttatc gatattggca agaaggtaat tataacggta ttcacgttaa attgttagaa 1980
aaaaaaatat taaagatgcc tagcactact agcatgagga aaatactatg ggaacacaaa 2040
aatagtagtt tatattcagc tggtgttcag ggaaatagta gtaactatag tactgataat 2100
gataatgata atgacaacaa taataataac aataacaata gtaatattcc tgttactgct 2160
gctgcttgtg atgatgatga tgatgatgat gatgacgcaa ttctcataag acaaattctg 2220
ctaacatatc atgatcaaga gaaaccttat gaattgttac agattcaggt gaaaaattgg 2280
cctgatttgg gcactttact caatcctatc tcaatcttac aagcaatcaa tgtcaagaat 2340
cacataatag atacactatt tgccagaaat tactatcaaa acgaccaact tcctaccatt 2400
ttggttcatt gttccgcagg ttgtggaagg actggtactt tgtgtaccat tgattccatt 2460
ctatccaatt tcgaaatgtt tgaaatgttg cagaaagagt ttgttaaatt aaaatacccg 2520
gcaaagttat tcgatccaat ttcatggaca attaatattt ttagaaagga acgaatatcc 2580
atggtccaga acattaatca atttattttc atttatgact gcttgctatt ttactttaga 2640
ttacgtcttg atgacataac agagagaacg gatggtgatg gaagtaacaa ggacaatata 2700
tcattaagtg cattaattga acaaatcgaa aaattagaga tcttgcagac attcgtggat 2760
gataagttga aagaattgcc acaatag 2787

<210> 75
<211> 522
<212> DNA
<213> Saccharomyces cerevisiae

<400> 75
atgaatttca aggagccgct agtgaatttt ttaaaatgcg tcttgaataa cataaactcg 60
gcattttcgc ataggattga tcaattgcaa ttgcagctgt tgcgggaaac aaatatcttg 120
agggttttga acaggggaat agagagactg tttagcgaga atccaaggac ccattctgtt 180
tcccgtatcg tggtggaaaa agatgtgggc tgcagaattg gacgatatgt ggaaggttct 240
aaatatgaac tggtagagtc cagggcgaaa gaaatacaaa tatattatga aaggatggtt 300
tttgaaataa cacaagaatt gaaaggggac aagcgggtgt ttgccttgat tgcaaatctt 360
tcccaaagat atagcgcaga gaggaaagaa atacgaaatg taaaggtgca tggtgggcgg 420
ccatgtaatg agaacgaatt ccaggtgatt ccgataagat tcaaacctat taatttggaa 480
agaagagcac gactgataag aaagaagaag actattaatt aa 522

<210> 76
<211> 723
<212> DNA
<213> Saccharomyces cerevisiae

<400> 76
atggccgatg aggaacgttt aaaggagttt aaagaggcaa acaagatagt gtttgatcca 60
aataccagac aagtatggga aaaccagaat cgagatggta caaaaccagc aactactttc 120
cagagtgaag aggacataaa aagagctgcc ccagaatctg aaaaagacac ctccgccaca 180
tcaggtattg ttccaacact acaaaacatt gtggcaactg tgactttggg gtgcaggtta 240
gatctgaaaa cagttgcgct acatgcccgt aatgcagaat ataaccccaa gcgttttgct 300
gctgtcatca tgcgtattag agagccaaaa actacagctt taatttttgc ctcagggaaa 360
atggttgtta ccggtgcaaa aagtgaggat gactcaaagc tggccagtag aaaatatgca 420
agaattatcc aaaaaatcgg gtttgctgct aaattcacag acttcaaaat acaaaatatt 480
gtcggttcgt gtgacgttaa attccctata cgtctagaag ggttagcatt cagtcatggt 540
actttctcct cctatgagcc agaattgttt cctggtttga tctatagaat ggtgaagccg 600
aaaattgtgt tgttaatttt tgtttcagga aagattgttc ttactggtgc aaagcaaagg 660
gaagaaattt accaagcttt tgaagctata taccctgtgc taagtgaatt tagaaaaatg 720
tga 723

<210> 77
<211> 429
<212> DNA
<213> Saccharomyces cerevisiae

<400> 77
atggcagatc aagtaccagt tacaacacaa ctaccaccaa taaaacctga acatgaggta 60
ccacttgatg ctggagggag tccagtaggt aacatgggta ccaactcgaa taacaacaac 120
gagctaggtg atgtattcga cagaataaag acacacttcc ctccggccaa ggtaaagaaa 180
ataatgcaga cagacgagga tataggaaaa gtttcacaag ccacgcccgt aatagcgggc 240
aggtccctag agttttttat agcgttattg gtgaaaaaaa gcggggagat ggcaagagga 300
caaggaacca agagaataac cgccgaaata ctaaaaaaaa caattttaaa cgacgaaaaa 360
ttcgatttct taagggaagg tctatgcgta gaagaaggcc aaacacaacc ggaggaagag 420
agtgcctga 429

<210> 78
<211> 912

<212> DNA
<213> Saccharomyces cerevisiae

<400> 78
atgtccacaa ctgattcggg gttcgtactt taccactata cgccaagtaa agcggctgca 60
atcgtatttg tcgtactctt cattataatg acagtcattt ttgccgttca aactttatat 120
gctgcgagaa aatcttctaa ggcattgaaa aataatccat ttgagtcatc tgatgataag 180
gtcgatagtc ttgaggatgc tgaatacaag caactaaaaa tcacacccac agtttttgcg 240
tttatacctt tcttcactgg ctgcatcatg gaagctgtcg ggtatatcgg gagggctcta 300
tccagttcca atcctgaaag aacgacccca tatattatcc aatctgttct tttattagtc 360
gctccagcac taatagctgc gacgatttat atgatttttg gaaggttatt acatgttatg 420
aggtgccaat ctttaatttt gatttctgca cgctttggta cgactttctt tgttgtggga 480
gatgtgttta gtttcttttt acaagctgca ggtggcggac taatgtctaa ggcaggatca 540
acgaagactg gttcaggact cataactgcg ggtctttttg tacaggtaat tttttttgga 600
tttttcatta tcaatgagat aagattcaca gtaaatgtta aaggagatgg cttattttac 660
gaagatattt ccagaaagtg gatctttgtg aatgctaccc tattattaag cagtatgttg 720
atcctactac gttcaattgt ccgaatagtt gaatttattc agggattcaa tggctatata 780
atctctcatg agtatttcat atatgtcttc gatgctgtac ctatgttatt ggtaataata 840
gcatttagtg tgggatcctt tttcggcaac gtattcgatg ttataaagga gtgtcaaacg 900
ttaagtaatt ag 912

<210> 79
<211> 1107
<212> DNA
<213> Saccharomyces cerevisiae

<400> 79
atgaaactgg atagtataga cattacacac tgtcagttgg tcaaatctac tagaactgct 60
aggatttata ggtcggatac atatgccatt aaatgtctag cactagattt cgatatcccg 120
ccacataacg ccaaattcga agtatcgata ttaaacaaac tgggcaacaa atgtaagcac 180
atcttacctc ttctagagtc taaggctacc gataataatg acctattgtt gttgtttccc 240
tttgaagaga tgaaccttta tgagttcatg caaatgcact ataaaagaga tagaagaaaa 300
aaaaatccct attacgattt gctaaatccc agtatcccaa ttgttgcgga cccccccgtt 360
cagaaatata ctaatcaatt ggacgtcaat cggtattctt tgtccttttt ccggcaaatg 420
gttgaaggga ttgcattctt acatgagaac aagatcattc accgcgacat caaaccgcaa 480
aatatcatgc taacaaacaa taccagcacc gtatccccaa agttgtacat aattgatttt 540
ggcatctctt atgacatggc aaataactca caaacaagtg cggaacccat ggatagcaag 600
gtgacggata taagcacagg aatttacaag gccccagaag tgctttttgg agtaaaatgc 660
tatgatggtg gcgtggacgt gtggtcgttg ttgataatta tttctcagtg gttccagaga 720
gaaacaagcc gtatggggca cgttccggcc atgattgatg acggcagcga cgacatgaac 780
tcagatggaa gcgatttcag actgatttgc tcaatattcg aaaagttggg cataccgtcc 840
attcagaaat gggaagaggt tgcgcaacac ggctcggttg atgcatttgt tggtatgttt 900
ggtgcagatg gcgatggcaa gtatgtactg gaccaggaga aagatgtaca gattagcatt 960
gttgagagga atatgcctcg actggacgag attgcggatg tcaaagtcaa gcagaagttc 1020
attaattgta tcctggggat ggtttcattt tcaccaaacg aaagatggag ctgtcaaaga 1080
atcttgcaag aattagaaaa gccataa 1107

<210> 80
<211> 621
<212> DNA
<213> Saccharomyces cerevisiae

<400> 80
atgaatagcg agtacgatta cctgttcaaa ctgctgttga tcgggaattc cggtgtcggg 60

aagtcctgtt tacttttgag gttttcggac gacacatata ccaacgacta catctccaca 120
attggagtgg acttcaagat taagactgta gaactggacg gcaagactgt aaagctacag 180
atttgggaca ctgcaggtca agaacgtttc cgtactatca cttcatctta ctaccgtggt 240
tcgcatggga tcattatcgt gtatgatgtc actgaccaag aatccttcaa cggcgtgaag 300
atgtggctgc aagagattga tcggtatgca acctcaacag tgttgaagct attggtaggt 360
aacaagtgtg atttaaagga caagcgtgtc gtggaatatg acgtggcaaa ggaatttgcg 420
gacgcgaata agatgccgtt cttagagact agtgctttgg actccaccaa cgtcgaggat 480
gcgtttttaa ccatggctag acaaatcaag gaaagtatgt cccaacaaaa cctgaacgaa 540
accactcaga agaaggaaga caaagggaac gtcaacctga agggacagag tttaaccaac 600
accggtgggg gctgctgttg a 621

<210> 81
<211> 1629
<212> DNA
<213> Saccharomyces cerevisiae

<400> 81
atgatttcac ccataagctt tctatcatcg ctgctatgct taacttatct aacctcagct 60
ttaccgattc ttcctaagag agaagttgta acgagagttc acacagcaag cacaactaat 120
gttgtgactg atttttactc cactactacc gaagttgtta ttgccccaac tgtcgaattc 180
ctaataagcg atagtgttac ctttacaaca accttaattc cacaaggtgt gaacccaaca 240
gctgaaccaa ctacgactat cactaaagtt ctcttaaaaa aggctgaaat gagtacatct 300
tcgcagccaa cctctacttt gcagccaagc actattccac aatcaacctc ctcttttcaa 360
gcagaatcta ctttgcaagc cgtctctacg caacaaactg ctatgtctgt ttctgcaggt 420
actagtgagg atgtgcaaca attagctact acttctacct ctatctcctc ctctccttcc 480
cctaccacta cgtctacttc cacacaaaat atcgtgagtg gtgtcggtga agccaatgtt 540
gaaggcaaca ctcaaagtca gcatcattct taccaagcag ctgcgacttc aactttgaat 600
caacaaacga gtacctcaat tgcttctcag gagtccaccg aatcaaccaa tacacctacg 660
tcgtcctcta cgtcgtcctc tacttcttct tctacttctt cttctacttc ttcctctact 720
tcttcttcta cttcttcctc tacttcgtcc tctacttcgt cctctacttc atccacacag 780
gaaacagccg ctaccacctc tgaaggttcc tctagtagca gcgctgctat aacttcttct 840
ccaaaggcta ttgcatattc accatataat gatgatggct cttgtaagtc tgcagacgct 900
gtctcttctg acttaacgtt gatcaaatct aaaggtattt ccaaaatccg tgtttatggc 960
accgactgta attcctttga aaccgtccaa ccagcagccg taaaactagg tattaagatt 1020
aaccaaggtt tgtatatcac cagtagcggc gttgattcca tagatgattc tgttaccaca 1080
ttgattcaat atggtcaaac taacggttgg gatgtctttg atttcattac tgttggaaat 1140
gaagctatca ataatggttg gtgttcagtt tctgatttga ttagtaaaat ttcctctgtc 1200
aaatccaaat taagcgaagc tggctattct ggccaaatta ctaccagtga gccccctgtc 1260
tcatttgaaa ataaccctga tttgtgcaaa aaatctgata ttgacttcgt tggtatcaac 1320
ccacatgcct actttgatac tagcgccagt gctgaaactg ccggtacatt tgttaagggt 1380
caagttgaat taattcaagg tgtttgtggt acttctaatg ttttcgtcac ggaaacaggt 1440
tacccatcgt ctggtattca aaatggaggc aacattccat ccactgctaa ccaaattact 1500
gcagtccaaa acatcttgaa tgaaatggac ttggatgtta caatcttatc gacttacaat 1560
gattactgga aggctcccgg tgattacggt atcgaacaat ctttcggtgt gattgaatat 1620
tttccataa 1629

<210> 82
<211> 1740
<212> DNA
<213> Saccharomyces cerevisiae

<400> 82
atgaataggg acaacatgga cacaacaaag cgcaaagaag atcatacaaa gcatactact 60
gatgtaatag aattttacga agaaggaacc gcagcttctt ctttaaacat agccacagaa 120

aaggccaact caagtccgtc cattttgaga agaattatca acagggcggc atggctttct 180
aagaaagtgg acgcaatggg agtagagtct accggtattc agaggatatc tccctatgaa 240
cgaggaacat caaagaaaca atttttgcat gtcgcggggc tatggttgag tgccacaggt 300
ggtttgtctt ccatgtcgtc gtttttatta ggccctctgc ttttcggttt aagctttaga 360
gaatccgtag cctccagttt aatatcggta acaattggtt gcttaattgc tgcctattgc 420
tcgatcatgg gaccgcagtc cggttgcaga cagatggtaa cagctagata cctgttcgga 480
tggtggtttg ttaaattagt tgcattagct tccattatcg gtgtcatggg ttggtctgtg 540
gttaattccg tggttggggg tgaaatgtta gctgctattt ctaatgataa agtaccccta 600
tgggtaggga ttgtcatagt gaccgtatgc tcctttttag ttgccatttt cggtatcaaa 660
caagtcatta aagtggaaac ttacttatcc gtacccgtcc tgacagcatt tttactactg 720
tacatttcat caagtgataa atacagcttc gtcaatgcat acgtttcaaa gggaaatttg 780
gactcttcca cccgaaaggg caactggatg agtttttttt cattatgcta tagtattacg 840
gctacttggg ggtctattac agcagactat tatatattgt tcccagaaga tacaccatat 900
attcaaattt tttgcctaac gtttttggaa acatttttgc ctacctgttt cgtcggaatc 960
ttaggtctct tactagcttc tgttgccatg tcgtataagc cttggtcagt cgagtacgat 1020
tcccatggaa tgggtggtct actttgggca ggttttcagc gttggaacgg cttcggaaag 1080
ttctgcgtgg tagtactggt attcagtttg gtatccaaca acatcatcaa tacgtattcg 1140
gcagcattct ccatccaact ttctagtgtg ttttgtgcaa aaatccctcg ctggttctgg 1200
tctattgttt gtactataat ttgtttggta tgcgcattaa taggtcgtaa tcattttagt 1260
actattttgg gaaatttcct accaatgata ggctactgga ttagcatgta ctttatctta 1320
ttattcgaag agaatttagt attcagaagg tttttcttac atttgtacac caaagagttt 1380
cctacagtta caggagaaat aaatggaccg gaacttgttg gatcttcaaa agaagtggag 1440
aaagatgcag ttactaatat ccatttgtta aaaagaaaac ataaggtgac taaacatcgt 1500
tacaattggg acaaatggga agattatgag gttttaacgc acggttatgc agcaacattt 1560
gcgtttattg taggagttgc tggtgtggta gttggtatgg cgcaagccta ttggattggc 1620
ccaattgctg ccaaatttgg tgaatatggt ggtgatgtcg caatgtggtt aagtatggcg 1680
ttctcaggtg ttgtataccc tccttgtaga tacttagagc tacgtaaatt tggtcgttaa 1740

<210> 83
<211> 498
<212> DNA
<213> Saccharomyces cerevisiae

<400> 83
atggaagaag tgaaagcagt caattctgcc gctaaagttt tgtgctctgt ggatcctcag 60
tttgatcctt ctaaattaac actttctgtg ggcgctactc cgacttccaa ttctttgaaa 120
ctcgataata aaagtaccct tgttaaattc attactactc aattagttag tacgcttgaa 180
atccactgcg gtaattactg catgtatgac ctgcaacagg tggcaacagg ctgtgtccaa 240
gatcacgaat tgtctggttt tgtattagga acagtactat catcttaccc ttctagaggt 300
gaattgttga gtaatacagg tgtaatgtgt ctaacgcgag aagcatcctc aataaaaggg 360
tttggaatat gtgctgattt ggaacatgtg ttaaaatccg agagtttcag tagggaatgg 420
tatgtagcaa gggtctctca agaacacggg atactgaggc caataagaaa ctggaacgaa 480
actactccat taaattag 498

<210> 84
<211> 342
<212> DNA
<213> Saccharomyces cerevisiae

<400> 84
atgagcattc tatcatccac acaatccaca attttacgta taccctccgg tctaattact 60
tttctcctca gcaagctatt tcttttgctc cgcgtagaac cttcttcagc gtctatgtct 120
atatcggagt cggagttatt actcatgggt aatattaacg acgaatcccc caaaccggga 180
aagttagctt ctgcaccact agcttcattg accaatcttg ttttttccat tgacgtaaag 240

ggccttactc ttatagctac gactatggag gattgtcttg tttcaggcac gttcatgtta 300
gtgtcaatag tatacagctg gaaagaaaac tcaagtagtt aa 342

<210> 85
<211> 660
<212> DNA
<213> Saccharomyces cerevisiae

<400> 85
atggacctag atctagccag tatcttaaaa ggtgaaattt ctaagaagaa gaaagagctg 60
gccaactcta aaggtgttca gccaccatgc actgagaaat tccagccaca tgaatccgca 120
aacatagacg aaacaccgcg acaggtagaa caagaaagta cagatgaaga aaacctgtca 180
gacaatcaga gtgacgatat taggaccacc attagcaaat tagaaaatcg gccagaaagg 240
atacaagaag cgatagctca agacaaaacc atctctgtaa tcattgatcc atcacaaatt 300
ggttctactg aaggaaaacc tcttttgtca atgaaatgta atttatatat tcacgaaata 360
ttatctcgtt ggaaggcgtc tttggaagct tatcatccgg agctgttctt agacactaaa 420
aaagcactct ttccactcct actgcaacta cgaaggaacc agcttgcccc ggatttactt 480
atttcccttg cgaccgttct ctatcattta caacaaccta aagaaataaa tttagccgtt 540
cagtcctata tgaaactcag tatcggcaat gtggcctggc ctattggtgt tactagcgta 600
gcattcatgc tcgtagtgca cattcgaaaa ttcaaggagg ccggaatgct gctaacataa 660

<210> 86
<211> 3468
<212> DNA
<213> Saccharomyces cerevisiae

<400> 86
atgaatagta atgaactgga tctaagaaat aaatattttt atgagatatt cggtaagaaa 60
agaaaatcag atacttcaac ccctactcag ttgttctccg gctccaaagt tcagacaaac 120
atcaatgaaa tttctatcac taacgatgag gatgaagata gtactgaaga tgaaaataag 180
gcttcattga aggattatac actagggcac gacaccggtg cacggtatag gatagcaccg 240
gactgttctt cccaccaatt aaaggcatct cctgtcctac atatttcaac aaaccttaat 300
tcaagtccac aatccttcac aggcgatcag atttcaccta ctaataaaaa aatttcaata 360
aatgattcga ccagacaaga taaaggtaac agttgcacta ccacttcatc accttctcaa 420
aaaagatcga atgttttgct tcctcacgta agaaaacatt catctccttc actattatca 480
ttttccaaaa acagtggcag tcatatgggg gatccaaacc agctatctac gcctccaact 540
cccaaaagtg caggtcacac gatggagtta cacagttcat tcaatggaaa acatagttct 600
tctagcacct cttctttatt tgcattagag tcactgaaaa cccaaaatag acgctcatca 660
aactcttcca atcattctag tcaatatcga cgccatacta atcaacacca acgtcatcat 720
tcaaggtcca aatcaagtcc tgtctctctg acggaaatat ccatgatcaa aggcacgcct 780
ttggtttatc ctgcactttt atcactaata gcaattaaat tcaaacagac catcaaattg 840
agcacgcata aaaagatggg gttactttac agagattcct ttacaggaaa acaagcaatt 900
gatactttat gcttgatcat aggaagctta gatcgtaatt tgggcatgtt gatcggaaaa 960
tcgctggaag ctcaaaaatt gttccatgac gtactttatg accatggcgt aagagattct 1020
gtactggaga tttacgagtt atcttcagaa tcaattttta tggcacatca gtcgcagagt 1080
tctacttcaa ttgccaacac attttcttca tcatcttctt cagttaattc gctccgtact 1140
aaaactgaaa tatatggtgt ttttgtccca ttgacacatt gttattcctc tacatgctct 1200
ctggaaaaac tttgctactc tatttcttgc cccaatcgtt tgcaacaaca ggctaattta 1260
catttaaaat taggtggtgg tcttaagaga aatatttcgt tagcactcga taaggaggat 1320
gatgaacgaa tttcctggac aaattctgta ccaaagagcg tatgggaatc attatccaaa 1380
caacaaatca aaaggcagga ggcaatatat gagttgttta ctacagaaaa gaagtttgta 1440
aaatctttgg aaatcatccg agatactttc atgaagaaat tattagaaac gaatattatt 1500
ccatctgatg taaggataaa ttttgtaaag cacgttttcg cacatatcaa tgaaatatat 1560
tctgtcaata gagaattttt gaaggcttta gcacaaaggc aatcattaag cccaatttgt 1620

cctggaattg cagatatatt tttgcagtat cttcctttct ttgatccttt tctgtcatac 1680
atagcatcaa gaccatacgc aaagtatcta attgaaaccc aaagatcagt taatcccaat 1740
tttgctcgtt ttgacgatga agtgtctaat tcttccctga ggcatgggat cgattcattc 1800
ctatctcagg gtgtttcaag acctggtaga tattcactgt tggtaagaga aataatacac 1860
ttctcggacc cagtaacaga caaagatgat ctacaaatgc taatgaaagt ccaagatctt 1920
ttaaaggatc taatgaaaag gattgataga gcaagcggtg cagcacaaga tcgttatgac 1980
gttaaagtgt taaagcagaa aattctattc aaaaatgaat acgttaatct gggtttgaat 2040
aacgaaaaaa ggaaaatcaa gcatgaaggt ttactctcaa ggaaggacgt gaacaaaaca 2100
gatgcgtcct tttcaggaga cattcaattt tacctactcg acaatatgct attattcttg 2160
aaatcaaaag ctgtaaacaa gtggcaccaa cacactgtat ttcagagacc aattccactc 2220
cctttactgt ttatttgtcc ggctgaggat atgccaccca taaaaagata tgtgacagaa 2280
aacccaaatt gctcagcggg tgtgctctta ccccaatatc aaacgagcaa tcccaagaat 2340
gctattgtat tcgcctatta cggtacgaaa caacaatatc aagttacttt gtacgcgccg 2400
cagccggccg gattacagac attaatagaa aaggtgaaac aagagcaaaa aaggctcctt 2460
gatgaaacta aacatattac ttttaagcaa atggtaggtc aattctttca ctcatacata 2520
aatactaatc gcgtcaacga tgtcctaatc tgtcatgctg gtaaaatttt attggttgca 2580
acaaatatgg gactctttgt tcttaattat gctacatcga tcaatcaaaa accagtgcac 2640
cttctgcaca aaatatcaat ttcacagatc tctgtattgg aagaatataa agttatgatt 2700
cttctaattg acaaaaaact gtacggctgt cctttagacg taatcgacga tgcagaaaat 2760
gcagattttc ttttcagaaa aaattctaaa gtgttattta aatatgttgc aatgttcaaa 2820
gacggtttct gtaatggtaa aagaatcatt atgattgcac atcatttttt gcacgccgca 2880
caattattga ttgttaatcc tttgatattt gattttaata gcggtaattt taaaaaaaac 2940
ctaaaggcag gcttggtaga ttttagcgtt gattctgaac ctctgtcctt ttcttttttg 3000
gagaataaga tctgcattgg ttgtaaaaaa aatatcaaaa tattaaacgt accggaagtg 3060
tgtgataaaa atggatttaa aatgagggag cttttaaatc tacatgataa caaagtttta 3120
gcgaacatgt ataaagagac gttcaaagta gtttccatgt ttccgataaa aaattcaact 3180
tttgcatgtt ttccagaact ctgctttttt ctcaataagc aagggaagag ggaggagaca 3240
aagggatgtt ttcattggga gggggaacca gaacagttcg cgtgttccta cccttatatt 3300
gtggcaatta atagtaactt tattgaaatt agacatatag aaaatggaga acttgtccgc 3360
tgtgtacttg gaaacaagat acgtatgtta aaatcatatg ccaagaagat cttatattgt 3420
tatgaggatc ctcaaggatt tgaaattatc gaactgttaa atttttga 3468

<210> 87
<211> 1203
<212> DNA
<213> Saccharomyces cerevisiae

<400> 87
atgacagcct catcaaatga cgatgaccta atattcgaat gttatagcga tcctgagtta 60
aaaagatgga cacaccttgc taatgcaaaa gcttggaaag gtattctgac cgtgcagcag 120
tacgctgata gggaacaatt gctagggtct tcagagattt ctcaaaagaa taaatcaaat 180
gaaatgatga caaaataccc aaaaagttac cagtggcttg ggcagaagta ctttgtttta 240
aaggatcgat ctttgccaga taatggaaaa tttagtcaag ttgtttccag ttgcgaaact 300
ttaaacagga ttggatattg tattcatccg ggttctaatg gaaaaatcga gccagctttg 360
attgtatgca ttgggggcgt ctttaccttt gaaaaccatc gcggcaaggg ctacgccaag 420
aagatgatta tcaaactgaa tgaattttat gacaagattc gtgatgacgc taacaccgta 480
ctagaattga aaaatttggt gattaacctt tacagcgaag tcggagaata ttattccgca 540
ttaggatacg agagtatgca tgttcccttg caccgcattt ctaaattaga tgaacttact 600
gagagatact gtggagaaga tgatgaccat gatggtaagt acttgggatt tgatgattac 660
aggggtttag taggactaca tgagacacag ttcaaagaaa gcttattaag tttacacaaa 720
gaaaacccag agaagtttgt ttttaccgtt gcgccagact ttgacatttt cacatggttt 780
cagtatcgtg atctttttat catgaataaa tcaggaagaa aagcccagca aaatctgttt 840
tttggatacg cgttgagtga taacagccac atcatatggc atcacaattg gaacggtgat 900
tctctgatta ttgtcaaaat ccatatacct gaagaaacat ttcaaaggaa agaattgaag 960
ctcaaaaaat tactaaggaa ggccatagaa gagacaaagc ttcatggatt gcaagaatta 1020

gaatttgggg acgaagaaat tcccatcaaa aagtacccac aattgtttca gttattaaca 1080
gagcttgaaa atgaatctaa agttttctca gaaaacggtt ccatcagtgc tgttcgtccc 1140
cccaaaggat atacagccga gcaggtaatt tgggataaca ataccaaatt ctgttggttt 1200
taa 1203

<210> 88
<211> 354
<212> DNA
<213> Saccharomyces cerevisiae

<400> 88
atgtctcgcc aaagcgcatt caaatttcag aatggaaata gacacgaacg agcctgtctg 60
tcagatgttc acaaaatcct tattataatt ttatattcta ctaaaggaaa aagagaatta 120
ggaaaaagaa taactcattt tatgtatata catatatttt gtacatatct ataccaagca 180
agtatagtgc aatactgttc ttcgacgtta ttaaacgtaa tagcattttc ttggtatcct 240
ttgaatctta tatacaagta cgagtacata ctgcgcagta aattgatcct gatggtgtgt 300
ttagatttcg ccagaagcgg aggcgttctg gattctggag atgtaagcct ttga 354

<210> 89
<211> 282
<212> DNA
<213> Saccharomyces cerevisiae

<400> 89
atgtctttct taggtttcgg tggtggtcag cctcaattat catctcaaca aaagattcaa 60
gctgcggaag ctgaactaga tttggtcaca gacatgttca ataaattggt taataactgt 120
tataaaaaat gtatcaatac ttcttattcc gagggtgagc tgaataagaa tgaatcttcg 180
tgcctagaca gatgtgtggc caaatatttt gagaccaatg ttcaagtcgg tgaaaacatg 240
cagaaaatgg gccaatcatt taacgcagcc ggtaagtttt ag 282

<210> 90
<211> 1296
<212> DNA
<213> Saccharomyces cerevisiae

<400> 90
atggcagact ttaaatcttt aggtctttca aaatggctta cggaatcctt aagagctatg 60
aaaattactc agccaaccgc tatccaaaag gcttgtattc ccaaaatctt agaaggaaga 120
gattgtattg gtggtgccaa gactggttct ggtaaaacta ttgcatttgc agggcctatg 180
ctgactaaat ggtccgaaga tccgtcaggg atgtttggag tggtcttgac cccaaccaga 240
gagttagcca tgcaaattgc cgaacaattt actgcacttg gtagcagtat gaacattcgc 300
gtgtcagtta tagttggtgg tgaaagcatc gttcagcagg cattggattt acaaaggaag 360
ccgcacttta ttattgccac gcctggacgt ctggcacatc atatcatgag cagcggagat 420
gataccgttg gagggctaat gagagctaaa tatttagttc tagatgaagc cgatattttg 480
ctaactagca cgtttgccga tcatttagca acatgtataa gtgctctacc tcccaaggat 540
aagagacaaa cacttctatt cactgcaact ataacggacc aagtaaagtc gttacaaaat 600
gcaccagtgc agaaagggaa acccccattg ttcgcttacc aagtagaaag tgtagataat 660
gtggctattc catcgacgtt gaaaatagag tatatcttgg ttccagaaca cgtcaaagaa 720
gcatatttgt accaattatt aacatgcgag gaatatgaga ataaaaccgc tataatattt 780
gtaaatagaa caatgaccgc ggagatcctt agaagaaccc tgaagcagtt ggaagtgagg 840
gtagcgtcgt tgcactctca aatgccccaa caggaaagaa caaattcctt acatagattt 900
cgagctaacg ctgcaagaat cctcatcgct acagatgtag catccagagg tttggatatc 960
ccaactgttg agcttgtagt gaattacgat ataccttcag acccagatgt attcatccat 1020

agatctggtc gtacggcccg tgctggcaga attggtgacg ccatatcatt cgttacccag 1080
cgagatgttt ctaggataca ggccattgag gaccgtatca acaagaagat gacagagaca 1140
aacaaggtac acgataccgc tgtaataaga aaagccttga ccaaggtaac caaggcgaag 1200
agggaatcgc tgatggcaat gcaaaaggaa aacttcggtg agagaaaaag acagcaaaag 1260
aagaagcaaa atgacgggaa aagtttgcgc tcttga 1296

<210> 91
<211> 786
<212> DNA
<213> Saccharomyces cerevisiae

<400> 91
atggctagag gaccaaagaa gcatctaaag agattagcag ctccacacca ttggttattg 60
gacaagttgt ccggttgtta cgccccaaga ccatctgctg gtccacacaa attgcgtgaa 120
tccttgccat tgattgtctt tctaagaaac agattaaagt atgctttgaa cggccgtgaa 180
gtcaaggcta tcttgatgca acgtcacgtc aaagttgacg gtaaggttag aactgacacc 240
acctacccag ctggtttcat ggacgtcatc actctagatg ccaccaatga aaacttcaga 300
ttggtctacg atgtcaaggg tagattcgct gtccaccgta tcaccgatga agaagcctct 360
tacaaattgg gtaaggtcaa gaaggtccaa ttaggtaaga agggtgttcc atacgttgtt 420
acccacgatg gtagaactat cagataccca gacccaaaca tcaaggtcaa tgacactgtt 480
aagattgatt tggcctctgg taagattact gatttcatca agttcgatgc cggtaagttg 540
gtttacgtta ctggtggtcg taacttgggt cgtatcggta ctatcgttca caaggaaaga 600
cacgatggtg gtttcgattt ggttcacatc aaggactcct tggacaacac tttcgtcact 660
agattgaaca atgtcttcgt cattggtgaa caaggtaagc cttacatttc tttgccaaag 720
ggtaagggta tcaagttgtc tattgctgaa gaacgtgaca gaagaagagc tcaacaaggt 780
ttgtaa 786

<210> 92
<211> 393
<212> DNA
<213> Saccharomyces cerevisiae

<400> 92
atgtgcctag gtaagctgta cttcgaacag ctaatattag taagatgcat aaaggggcgt 60
caaagtggaa atataacgac aggagaatct cggtgtgtaa gttggaatgt gtattgcacc 120
acttccatga ttgggctttt ttcctggcgc aaaatgttgc tttttcaaca tttttcttat 180
acgcagagag agaatcgtca gataggtggt aaaacttgga gcctaattag ctcattattt 240
catttgaacg agacattggc ttctgcccta catcacccat acgagacatt ggccttatac 300
gaggcgtact tcgcttgcgg gaaaaaaaat ttcttttgta agcgaggtta ctcagaaact 360
gtgactgaag gcagaggttg gaaacgtgtg tag 393

<210> 93
<211> 1923
<212> DNA
<213> Saccharomyces cerevisiae

<400> 93
atgaggagag agaggcaaag gatgatgagt ttcgaggaca aggacaagga cgaccttgac 60
aatagtaata gtaataacag cagtgaaatg acagatacgg cgatgatgcc accattaaag 120
agattgctta ttacgggcag tagcgatgat ttggcacaag gatcatcggg taagaagaag 180
atgacgatgg cgacgaggtc gccatcgtca tcacccgatt tggcgacaaa cgacagcggc 240
actagggtac agccattgcc agaatataac ttcaccaagt tctgctatcg gcataacccg 300
gacattcagt tctcaccaac tcatacagcg tgctacaagc aggatttgaa acgaacgcaa 360
gagattaatg ctaatatcgc gaagctaccc ctgcaggagc aatccgacat ccaccacatt 420

atctcgaagt acagcaattc caatgacaag atacggaagc tgattctgga tgggatccta 480
tcgacgagtt gcttcccaca gctttcctac atttcgtcac tcgttacaca catgatcaag 540
atcgacttca tcagcattct gccgcaggag ctgtcgctga agatcttgag ttatctggat 600
tgccaatctc tttgcaacgc cacgagagtg tgccgcaagt ggcagaagct cgcggatgac 660
gacagggtat ggtaccacat gtgcgagcag cacatagaca ggaaatgtcc caactgtggc 720
tgggggctgc ctcttttgca catgaaacgt gcgcggatac aacagaatag tacaggatct 780
agcagcaacg cagatatcca gacgcaaact acgcgacctt ggaaagtcat ctacagagaa 840
cggttcaaag tggagtcaaa ctggagaaag ggccactgca ggattcagga attcaagggc 900
cacatggatg gtgtgttaac gctccagttt aactacaggc ttttgttcac aggctcgtac 960
gactccacca taggtatatg ggacttattc acggggaagc taatacgaag gctcagcggc 1020
cattcggacg gcgtcaagac attatatttt gacgatagaa agctgattac gggctcgctc 1080
gacaagacga tccgtgtttg gaactacata accggtgaat gcatttccac gtatcgaggc 1140
cactcggata gcgttctgag cgtagattca taccagaagg ttatcgtttc cggcagtgct 1200
gacaagacgg tcaaggtatg gcacgtggag tccaggacat gctacacctt gagaggccac 1260
acggaatggg ttaattgcgt caaattgcat ccgaaaagct tttcatgttt tagttgcagt 1320
gacgatacca caatccgaat gtgggatatc aggaccaatt catgcctaaa agtgttcagg 1380
ggtcatgtag ggcaggtgca aaagatcata ccgcttacca ttaaggatgt agagaatcta 1440
gccaccgaca acacttctga tggcagctct ccgcaggatg acccaacaat gactgatggt 1500
gcagacgaat cagacacacc gtcgaacgag caagaaactg tcttagatga aaacatacct 1560
tatccaacac atctactatc ttgcggactg gataacacaa tcaaactatg ggacgtcaaa 1620
accggtaaat gcataagaac acagtttggg cacgtggaag gtgtttggga catcgccgct 1680
gacaacttca gaattataag tggttctcac gacggaagca ttaaggtctg ggacttgcaa 1740
agcgggaagt gtatgcacac gttcaacggt cgaagactac aaagagaaac tcagcacaca 1800
caaacacaat ccttgggtga taaagtcgcc cctatcgctt gtgtttgtat tggagattca 1860
gaatgcttta gtggtgatga atttgggtgc gtaaaaatgt acaaattcga tctcaatgat 1920
tag 1923

<210> 94
<211> 366
<212> DNA
<213> Saccharomyces cerevisiae

<400> 94
atgaatttct ccacagtttt tcaagcaata atcgcggttt taggtttaac tacagtcaca 60
gctttagcag aatttgattt cgacgtaggc tatgaagagt ttgtccgtac caatcctgat 120
acgattttct tagagagtga tattggcctg cacgttggat ataccgaggg tggcgaacga 180
cagatagcaa cgataccaca caattccacg ctagggactt cactacgtga gtatagtggc 240
tgtggtggta atggtacaga aacctccatc gctacccccg cacctacaat gagtgaggta 300
cctatcgcta ccttcgtgaa acgtagaaaa agtgtaccta tcttgctacc gcaggtatgt 360
atgtga 366

<210> 95
<211> 1011
<212> DNA
<213> Saccharomyces cerevisiae

<400> 95
atggccagga aattgaaggg taagattggg agtaaaggcc taaaaggtgc attattaagg 60
cacaaggcca aagtgaaatt agtgaggaat attgaaagta agcaaaaaca tgaactaaga 120
aagaaaaatt cgagcgctaa taacaaaacc gtgaagagaa accaagaatt ccaaaaactt 180
aatcaaggga aggttatgcc ctttgagaag gatgaaactt tgatgctatg tggtgaagga 240
gatttttctt ttgccagatc cattgttgaa caaaactata ttgaaagtga caatttaatc 300
ataacaagtt acgataattc tgtgaatgag ctgaaattga aatatccgca tacttttgag 360
gaaaattacc agtacttaaa agatctaaat attccgatat ttttccagat tgacgttacg 420

aagctcgtaa agagctttaa aatttccaaa aataatactt ggtttaaaat aatcaacagg 480
cttagcgatc atcgttgggg taacaaacct ttacaaaata tagttttcaa ttttccccat 540
aatggaaaag gtattaagga tcaagaaaga aatattagag agcatcagga cttaattttt 600
aactttttcc aaaattcctt acaattattt aatcttataa acacgaaaat tcaaaatgat 660
actcttcgtt atacgcaagg ctatgatctt aatgaggata ctccccaggc taaaaaattg 720
accgccgaag gttatggtaa catcatactg tcccttttcg atggagagcc ctatgattca 780
tggcaaatca aacttttagc caaaaaaaat ggactaactc tgagcagatc gagcaaattc 840
caatgggaaa attttccagg atatcatcac cggagaacga atagcgaaca agatactaca 900
aaacctgcga aagaacgtga tgctaggttc tacatcttta gtaaatatgt ctcgaatagt 960
tcaaaacata accgtaaaag caaaaaagac acagattctg attcagacta a 1011

<210> 96
<211> 354
<212> DNA
<213> Saccharomyces cerevisiae

<400> 96
atgtatatat atgttgaagt gtgtacacac gtacacgcac aagctacaga cgccacgcgg 60
cccggcattc atatatgtac atacatttat gcacatatat atatatatat atatgtatat 120
gtaaatgtat atatacagct tgtgatttcg cgcgccgaat gggctgaaaa gcacagtata 180
gtggagaaga agagagagga gaaggggaga ggggcaatag caatggatgt ggtagaatac 240
agggaaggcc aagctgttgt tcaatggatg cggttcgagg cccagcgcaa ggggggcgct 300
tcgaagcata gaacattatc cgcggaaacg ggtattaggg gtgagggtga ataa 354

<210> 97
<211> 3099
<212> DNA
<213> Saccharomyces cerevisiae

<400> 97
   atgacaccag acgaactaaa ttcagcagtc gtcaccttta tggccaatct gaatattgat 60
   gattcgaagg ccaacgaaac cgcttccaca gtaacggatt ctattgtaca ccgatcaata 120
   aaattgctgg aagttgtcgt tgcgttgaaa gattattttc tttcagaaaa tgaggtagaa 180
   aggaagaaag cgttgacatg tttaaccact atcttagcaa aaactcccaa ggaccacctc 240
   tccaaaaacg aatgctcagt aatatttcag ttttaccagt ctaagcttga tgatcaagcg 300
   cttgcaaaag aagtcttgga gggcttcgcc gcattagctc caatgaaata tgtctccatt 360
   aacgagatag cccaactttt gagattatta ttggacaact atcaacaagg ccagcatttg 420
   gcatcaacta gactgtggcc atttaaaatt ttgagaaaaa tatttgatag atttttcgtc 480
   aatggatcct cgaccgaaca agtcaaacga atcaacgatt tgtttattga aacatttctt 540
   catgttgcca atggtgaaaa agaccctaga aatttactct tgtcatttgc acttaataaa 600
   tctatcacat cgtccttgca aaacgtggag aattttaaag aagacttatt tgatgtctta 660
   ttttgctatt tcccaatcac tttcaagcca ccaaagcatg atccttacaa gatctccaac 720
   caagatttaa aaacagcttt gcgatcagca attactgcga cgccattatt tgccgaagat 780
   gcttatagca atttgctgga caaactcact gcttcttcac cggtagtgaa aaatgataca 840
   cttctgacgc tattagaatg cgttagaaaa ttcggcggct cttctatatt ggaaaattgg 900
   acgttacttt ggaacgcttt gaaatttgaa attatgcaaa acagcgaagg aaatgaaaac 960
   actctactaa atccgtacaa taaagaccaa cagagtgatg atgtgggcca atatactaac 1020
   tacgatgctt gcttgaaaat tattaatcta atggcattgc aactatacaa ttttgacaaa 1080
   gtatcatttg aaaagttctt cactcatgta ttagatgagt tgaaaccaaa ttttaaatat 1140
   gaaaaggatt tgaagcaaac ctgccagatt ttgtccgcta taggtagtgg gaatgtcgag 1200
   atatttaaca aggtcatttc gtcaactttc cccttatttt tgatcaatac atctgaagtt 1260
   gccaaactta agctgctgat tatgaatttt tctttttttg ttgactcgta cattgatttg 1320
   tttggaagaa catctaaaga atcgttggga acaccggtgc caaataataa aatggctgaa 1380
   tacaaagacg agatcataat gattttgagc atggccttga caagaagctc caaggcagag 1440
   gttaccataa ggactctatc tgtgattcaa ttcacaaaaa tgattaaaat gaagggcttt 1500
   ttaaccccag aggaagtttc actaattatt caatacttca cggaagaaat attaacggat 1560
   aacaacaaaa acatttacta tgcttgtttg gaagggttaa agacaatcag tgagatatat 1620
   gaggatttag tttttgaaat atcattgaag aaattattgg atttgttacc tgattgcttc 1680
   gaggaaaaaa ttcgagtaaa tgatgaggaa aatattcaca ttgaaacaat tttgaagatt 1740
   attcttgatt tcaccacttc gagacatatt ttggtcaaag aaagcattac atttttagcg 1800
   acaaaattga atagggtagc taaaatttcg aaatccagag aatattgctt tctacttatt 1860
   tcaaccatat attctctttt taataataat aatcaaaatg agaacgtgct aaatgaagaa 1920
   gatgctcttg cactcaagaa tgccattgag ccaaaattat ttgagataat tactcaggaa 1980
   tcggccattg taagtgacaa ttacaatttg acacttctat ctaacgttct tttcttcacc 2040
   aatttgaaaa ttccacaggc tgcacaccaa gaagaactag acagatataa cgagcttttc 2100
   atttctgaag gtaagataag aatcttagac acaccgaatg tattggctat ttcatatgct 2160
   aaaatattat ccgcactaaa taaaaactgc caattccccc aaaaatttac agtgcttttc 2220
   ggaacggttc aactactcaa aaaacatgct ccaagaatga cggagactga aaaattagga 2280
   tacctggaat tgctgttggt cttatcaaat aaattcgtct ctgaaaaaga tgtgatagga 2340
   ttattcgatt ggaaagactt atcagtaatt aatcttgaag tgatggtttg gttgactaaa 2400
   gggctgataa tgcaaaactc attagagtca tcagaaattg caaaaaagtt tattgattta 2460
   ctgtctaatg aagagattgg ctcacttgta tcgaagctat tcgaagtttt tgtcatggat 2520
   atcagttctt tgaagaaatt taaaggtatc agttggaata ataacgttaa aatcttgtac 2580
   aaacaaaaat tcttcggtga catttttcaa acactagtca gcaactataa aaataccgtg 2640
   gacatgacca ttaaatgcaa ttatttaact gcattatcct tagtgttgaa gcatactcca 2700
   agccaatcgg ttggtccttt tatcaacgat ttattcccgc ttttattgca ggctttggat 2760
   atgcctgatc ctgaagtaag ggtatccgct ttagaaactc tgaaggacac tacggataaa 2820
   catcacactt taattactga acacgtaagc acaatagttc cattattact atctctttcg 2880
   cttccgcaca aatataatag tgtcagtgtc aggttaatag ccttacagct tctagaaatg 2940
   attacgactg tagtacctct aaactattgt ttgagctatc aggacgatgt tctaagcgct 3000
   ttaataccag ttttatctga caaaaaaaga ataataagaa agcaatgcgt tgacacaaga 3060
   caggtttatt atgaattagg ccaaatcccg ttcgagtaa 3099

<210> 98
<211> 1629
<212> DNA
<213> Saccharomyces cerevisiae

<400> 98
   atgtccgtac aaaaagaaga atacgatatt gtagaaaagg cccaattatc tgtgtccgca 60
   gaaagcttaa catcagattc ggagagtatt tcacataacc catttgacga ttttcataaa 120
   gcagagcgtt ggagaaaagt ctatgaatcc agtggttatg aaggcttgtc taaattcgat 180
   cctgagttta catggacaaa ggatgaggaa aagaaattgg taagaaaaat ggacttgaag 240
   atttttttgt gggtttttat tatgtttgcc tttttggatt taataaggaa aaatattgca 300
   agggcagttt cagacaactt cattgttgac ttgaaaatga acacgaacga ctacaacctt 360
   ggccaaactg tttatctagt tatatttcta gcaagtgaac tacccggcaa tctgctgtcc 420
   aaaagatttg gtccagaaag ggtcattccg gttcaaattg tgttgtggag tgtaatttgt 480
   attactcaag ctggtctgaa aaatcgaggt caatttattg ctactagatg cttattagga 540
   atggtacagg gcgggttcat cccggacaat attctatact tgtcatatta ttatactgga 600
   gctgagctca cattccgcct aagtttcttt tggtgtgcta tacctctttt ccaaatttta 660
   ggctctcttt tagcttccgg aatcatagag atgaggggta ttcataactt agctggctgg 720
   cagtacctat ttataattga agggtttctg tccctttctg ttggcgtggc atccttttat 780
   ttaatgcgta gaggacctac gcaaactggt gagtctgcat ttcacaaagg aaaatcgtta 840
   ttcactgagt atgaggagaa aattatggtt aacagaattt taaggatgga cccatcaaag 900
   ggtgacatga gtaaccgaca accagttacc ttcaaggaga ttttgtacac tctaacagaa 960
   tttgatctat ggccattgtt tatccaaggt attacagcat tcatatctct tcaaacagtt 1020
   ggttcctatt tatctttgat attaaagagt ttaaattact ctacatttct ttcgaacatt 1080
   ttagcaattc caggccaagc cctgctgcta ataaatttac cattagcggc actattatcg 1140
   cgtaaattga aagaaaaatc actttgtgtg ggaattgcca acgtttgggt gctccctttc 1200
   atagtttctc tggtcgcttt accgactgac acaaacccct ggatcaagta tatattacta 1260
   accggtatac ttggtcttcc ctatacacat tccattcttg ctggttgggt ctccgagatt 1320
   tcaaattcgg taagatcgcg tacagtgggc acagcgttat acaatatgag tgcccaagtt 1380
   ggagcaatca ttgcttctaa catgtataga aatgatgata aaccttacta tactagaggt 1440
   aacaaaatac ttctaggatt cacttgcttc aacatttgta tggctgttgc tactaagttt 1500
   tactatatta gtaggaataa atacaaggac cgcaaatgga actctatgac aaaagaagag 1560
   cagatcaatt acttggacac aactaaagat aagggaatga agcgtcttga ttataggttt 1620
   attcactag 1629

<210> 99
<211> 1770
<212> DNA
<213> Saccharomyces cerevisiae

<400> 99
atgactattt cttctgcaca tccagaaaca gaacccaagt ggtggaaaga ggccacaatt 60
tatcaaattt atcccgcaag tttcaaagac tccaacaacg atggttgggg tgatatgaag 120
ggtattgcct ccaagttgga gtacatcaaa gagcttggta ccgatgccat ttggatctcg 180
ccattttacg actcgccaca agatgatatg ggttacgata ttgccaacta cgaaaaggtc 240
tggccaacct acggtacgaa cgaggactgc tttgccttga tcgaaaagac acataagctt 300
ggtatgaaat ttatcaccga cttagtcatc aaccattgct ccagcgaaca tgaatggttc 360
aaagagagca gatcctcaaa aaccaatcca aaacgtgact ggttcttctg gagacctcct 420
aagggttatg acgccgaagg caagccaatt cctccaaaca attggaggtc ttacttcggt 480
ggttctgcat ggacgttcga tgaaaagaca caagagtttt acttgcgttt gttttgctcc 540
acccaacctg atctaaactg ggagaacgaa gactgcagaa aggcaatcta cgaaagtgcc 600
gttggatact ggttagacca tggtgtagac ggctttagaa ttgatgtggg aagcttgtac 660
tccaaggttg ctggtctacc agacgctcct gtgattgacg aaaactcaaa gtggcaactc 720
agtgatcctt tcacaatgaa cggaccacgt atccatgagt ttcatcaaga aatgaacaag 780
ttcatcagaa acagagtgaa ggatggcaga gaaattatga cagttggtga aatgcgacat 840
gctactgatg agaccaagag gttgtataca agtgcgtcaa gacacgaact tagtgagtta 900
ttcaactttt cccacactga tgtcgggact tcgcccaagt tccgtcaaaa tttgatacca 960
tatgaactaa aggattggaa ggttgccctt gctgaacttt tcagatacgt taacggaact 1020
gattgttggt caacaattta tctggaaaat cacgaccaac ctcgttcaat taccagattt 1080
ggtgacgatt ctcccaaaaa ccgtgttatt tctggtaagt tgttgtctgt gttgttggtg 1140
tcactgagcg gtactctata tgtgtaccaa ggacaggaac tgggtgaaat caatttcaag 1200
aactggccta tcgaaaaata cgaggatgtc gaagttagaa ataactatga tgcgatcaag 1260
gaagagcatg gagaaaactc gaaggagatg aagaggtttt tggaagcaat tgcccttatt 1320
tccagagacc atgctagaac acctatgcaa tggtctcgtg aggagccaaa tgctggtttt 1380
tctggtccta atgctaaacc atggttttac ttgaacgagt ctttcaggga aggaattaac 1440
gctgaagacg aatccaagga ccccaactcg gttttgaact tctggaagga ggccttgaga 1500
tttagaaagg cacacaagga tattactgtg tatggatatg attttgagtt tattgatttg 1560
gacaataaga aactgttcag cttcacaaag aaatacgaca acaaaacatt gtttgctgct 1620
ttgaacttca gctctgattc gatcgacttc acgattccaa acaatagctc atcgtttaag 1680
ttggaattcg gaaactaccc aagaagtgaa gttgatgcgt cttccagaac attgaagcca 1740
tgggagggca gaatttacat atctgaatga 1770

<210> 100
<211> 2001
<212> DNA
<213> Saccharomyces cerevisiae

<400> 100
atgggaaaga ctaaaacaag aggcagaaga caccaagaca aacagagaaa ggacgaattt 60
gaacccagtt caaattctgc gaaggagcac atccagcaag aagagtctac ttacaatgac 120

gaagccgaaa ttaaagagac tcagccacaa atgttttttg gtgtgctaga tagagaagaa 180
ctggagtatt tcaagcaagc cgaatcgact ttgcaattag acgccttcga agcacctgag 240
gaaaagtttc aattcgtcac gagcattatt gaggaagcaa aaggtaaaga actgaaattg 300
gttacttccc aaataacttc gaaattgatg gaacgagtta ttcttgaatg tgatgagact 360
cagttaaaag acattttcca atctttcaat ggcgttttct tcgggttgtc atgtcataaa 420
tatgcttccc atgttttaga gactttattt gttagaagtg ccgccttggt agaaagagaa 480
cttttaacac caagttttga taacaatgaa aaagaagggc cttatgtgac tatggaaaat 540
atgtttctct ttatgttgaa tgaactgaaa ccgcacttga aaactatgat gaaccatcag 600
tatgcatcac atgttttgag attgctaata ttaattttgt cttccaagac attgcctaat 660
tcaactaaag cgaactctac tttacgttcc aaaaagtcaa aaattgcaag aaaaatgatt 720
gacattaaag ataatgacga tttcaataaa gtttaccaaa caccggaatc tttcaaatct 780
gaacttcgtg acataataac tactttatac aaaggattta ctaatggtgc cgagtcgcgt 840
tctgatattt cgcaatctac tatcaccaaa tttagagagt acagtgtgga taaagtggct 900
tcccctgtaa ttcagctcat tattcaagtc gaaggtattt ttgacaggga ccgttcattt 960
tggagattgg tcttcaatac agcagatgag aaggatccta aggaggaatc attcctggaa 1020
tatttactgt cagatcctgt cggttctcat ttcttagaaa atgtgattgg ctctgccagg 1080
ctaaaatatg ttgaaagact ttatcgttta tatatgaaag accgtattgt gaaactagca 1140
aaaagggata ctacaggtgc ctttgtagtg cgtgcattgc tggaacattt gaaggagaag 1200
gacgtaaaac agatattgga tgcagtagtt cctgaattaa gcatgctttt aaactcaaat 1260
atggattttg gtactgctat tatcaacgca agcaataaac aaggtggtta cttaagggat 1320
gatgtcattg ctcaattaat acaaaaatat tatccagaaa agtctgatgc aaagaatatt 1380
ttggaaagtt gcctgttgtt gagtgcatcg actttgggca ataccagaga tgactggcca 1440
acagcagaag aaagaagacg atcagttttc ttggaacaat taattgacta cgacgacaag 1500
tttttgaata ttaccattga cagcatgttg gctctgcctg aggaaaggtt gatacaaatg 1560
tgttaccatg gagtattttc gcatgttgtt gagcacgtac tacaaactac tagggtggac 1620
ataattaaga gaaaaatgct attgaacata ctgtctaaag agtctgtaaa tttggcatgc 1680
aatgtatatg gctctcatat aatggacaaa ttgtgggaat ttacggcaaa attaacatta 1740
tataaggagc gtattgctag agcattagtt ttagaaactg agaaggtgaa gaatagtatt 1800
tatggcaggc aagtgtggaa gaattggaaa ctggaattat atgttagaaa aatgtgggat 1860
tggaaaaaac taattaagga gcaagaattt gaaatatttc caaattctaa accgctacag 1920
ccaaagccag aaaagcactc aagagaaaga aataattcga aggaaggaag cgccttcaaa 1980
aagcaaaagc actatagata a 2001

<210> 101
<211> 1098
<212> DNA
<213> Saccharomyces cerevisiae

<400> 101
atgccaaata ctgctccgcc acttccacgt gcaccaccag taccaccggc tacttttgaa 60
tttgactctg aaccaacagc cactcattca catacagctc cttctcctcc accacatcaa 120
aatgtcacgg cctctacacc ttcaatgatg agtacgcaac aacgtgtgcc aacctcagta 180
ctatctgggg cagaaaaaga atcacgaacc ctaccgcccc atgtgccttc attgacgaat 240
cgtcctgtgg attcattcca tgagtcagat actacaccga aggtggcctc tataagaagg 300
tcaacgacgc atgatgtagg cgagatttcc aacaacgtca agatagagtt taatgcacaa 360
gaaagatggt ggattaataa aagcgcacct cctgctataa gcaatctgaa gttgaacttt 420
ttgatggaga tcgatgatca tttcatttcc aaaagattac atcaaaaatg ggtggtaagg 480
gatttttact tcttgtttga aaactattcg caactaagat tctccttgac ctttaatagt 540
acaagcccag agaaaacggt aacgactctt caagaacgct ttccatcgcc agtcgaaaca 600
caatcagcac gtattcttga tgaatacgct caaaggttta atgcgaaagt tgtagagaaa 660
tctcattcat tgataaattc acatattggg gctaagaatt tcgtgtcaca aattgtatcc 720
gaattcaaag acgaagttat acaacccata ggagcaagaa cgtttggcgc aactatattg 780
agttataaac ctgaagaagg aatcgaacag ttgatgaaga gcttacagaa gattaaacca 840
ggtgacattc ttgtaattag aaaggccaaa tttgaagctc ataaaaagat cggaaaaaac 900
gaaattatca acgttggaat ggactccgcc gctccgtatt cgagtgttgt tactgattat 960

gattttacaa agaataagtt tagagttatc gaaaatcacg agggaaagat tattcaaaac 1020
agctacaagc taagccatat gaaaagtggg aagttaaagg ttttcagaat tgttgcgaga 1080
ggatacgtag gttggtaa 1098

<210> 102
<211> 660
<212> DNA
<213> Saccharomyces cerevisiae

<400> 102
atgaggttcc agcttttcat atatttttat ttcaccattg ttgtgattgc aggaactaac 60
acaatacaac agttcagcga tgcgggcgat aggttaatca catcattgag aaatctggac 120
aacaatggaa catacgaaac attgacagca gagaaggtgc ctattatcga gggacaaatt 180
caaaacatta gtgcaaaata tgagcagcat acttttattt tgaagggctt agaagcggtg 240
ctaaattata aagtgaaaag tttggataat aatgagagag agtcgctcga aatagaatat 300
gaaaaggtgg aaaaggcact agatgcggct ttgaatgttt caccatttga atatatcaaa 360
aagtttaaag aagtctccag gggcaaggta gttaatgctt tagaaaacct cagtagagaa 420
cagaatcgaa ttactataaa tggtggtaga gaagacgaaa aagaaaaaga agcgagggaa 480
aaaaagaaac gactagacag aataaagaga atcctaactg taagtctgtt agaattgggt 540
ctagctcaag gagtggccga tttatgtgca gtagcacctt ttgcgtgtct tcttggggtg 600
acagttggaa gcatcggttt catattttgg ttggcactaa tatataatgc tattcaataa 660

<210> 103
<211> 402
<212> DNA
<213> Saccharomyces cerevisiae

<400> 103
atggacagag accatattaa tgaccatgac catcgaatga gctattccat caacaaggac 60
gacttgttgt taatggtttt ggcggttttc attcccccag tggccgtctg gaagcgtaag 120
ggtatgttca acagggatac actattgaac ttacttctct tcctactgtt attcttccca 180
gcaatcattc acgcttgcta cgttgtatat gaaacgagta gtgaacgttc gtacgatctt 240
tcacgcagac atgcgactgc gcccgccgta gaccgtgacc tggaagctca ccctgcagag 300
gaatctcaag cacagcctcc agcatatgat gaagacgatg aggccggtgc cgatgtgccc 360
ttgatggaca acaaacaaca gctctcttcc ggccgtactt ag 402

<210> 104
<211> 327
<212> DNA
<213> Saccharomyces cerevisiae

<400> 104
atggtagcta gttgtaaaga tcagaagaag gctgtcgcta tatgtttgca gagatcgccc 60
tgtgtcatga tagagagaca taaccctcaa gaatgtcttg acaatccaga gctgaataag 120
gatctgcccg aactttgtat tgctcagatg aaagcatttt tggattgtaa gcgaggaatc 180
gtcgacatga ctaagcggtt cacaggtaac gcacctctgt cgactggcaa gtacgatcaa 240
cagtacgaaa acttgtgcaa aggaaagttt gatccgaggg aggagatgga aaagctaaaa 300
cttctgaaca gccagcagaa ggactga 327

<210> 105
<211> 1362
<212> DNA

<213> Saccharomyces cerevisiae

<400> 105
atgaacaaca cttccataac cggaccacaa gtactacata gaacaaaaat gagaccacta 60
ccagtgctag aaaaatactg tatctcgcca catcatgggt ttttggacga ccggttaccc 120
ttgaccagac tgagcagcaa gaaatatatg aaatgggaag aaatcgttgc tgacctgccc 180
tctcttttgc aagaggataa caaggtgcgg agcgtcatcg atgggctgga tgtcttggac 240
ttggatgaga cgatcttggg cgacgtcagg gagctcagaa gagcatattc catttggggg 300
tttatggcgc acgcttatat ttgggccagc ggaactcccc gggatgtatt accggagtgt 360
attgcaaggc cactgttgga gacagcacat attttgggtg tgccaccgtt agctacgtac 420
tcctcgttgg tgttatggaa cttcaaagtg accgacgagt gcaagaaaac ggaaaccggg 480
tgtttggact tggaaaatat tacaacgata aacacattta cgggaaccgt tgacgaaagt 540
tggttttatc tggtcagcgt gcggtttgaa aaaatcggca gcgcttgttt aaaccatggg 600
ttgcagatat tgagagccat tagaagcggt gataaaggag acgctaacgt gatagacggg 660
ttggagggct tggctgcaac gatcgaaagg ttatcgaaag ccttgatgga aatggagctt 720
aagtgtgaac ccaatgtatt ttatttcaag ataagaccat ttttggccgg gtggacgaat 780
atgtcgcata tggggttacc acagggtgtt aggtatggag cggaaggaca gtatcggatc 840
ttttctggcg ggtccaatgc gcaaagctcg ttgatacaga cactggacat ccttttgggt 900
gtgaaacaca ctgcgaatgc tgcgcattct tcccaaggag acagcaagat caattatctg 960
gacgaaatga aaaaatacat gccaagagaa catcgtgaat ttctttacca tttggaatcg 1020
gtatgtaata ttcgcgaata cgtttctcgt aacgcttcta accgtgcatt gcaagaggcc 1080
tatggacgtt gcatttccat gctgaaaata tttagagata accacattca aattgttacc 1140
aagtacatta ttttgccctc caattcaaag caacacggct ccaataagcc gaacgtacta 1200
agtccaatcg agcccaatac caaagcaagt ggttgtttag ggcataaagt tgcctcatct 1260
aagactatcg gaaccggtgg tacaagatta atgcccttct taaagcagtg cagggacgag 1320
actgtcgcca ctgcggacat taaaaatgaa gataaaaatt ga 1362

<210> 106
<211> 1086
<212> DNA
<213> Saccharomyces cerevisiae

<400> 106
atgatttctg ctaattcatt acttatttcc actttgtgcg cttttgcgat cgcaacacct 60
ttgtcaaaaa gagattcctg taccctaaca ggatcttctt tgtcttcact ctcaaccgtg 120
aaaaaatgta gcagcatcgt tattaaagac ttaactgtcc cagctggaca gactttagat 180
ttaactgggt taagcagtgg tactactgtt acgtttgaag gcacaaccac atttcagtac 240
aaggaatgga gcggcccttt aatttcaatc tcagggtcta aaatcagcgt tgttggtgct 300
tcgggacata ccattgatgg tcaaggagca aaatggtggg atggcttagg tgatagcggt 360
aaagtcaaac cgaagtttgt aaagttggcg ttgacgggaa catctaaggt caccggattg 420
aatattaaaa atgctccaca ccaagtcttc agcatcaata aatgttcaga tttaaccatc 480
agcgacataa caattgatat cagagacggt gattcggctg gtggtcataa tacggatggg 540
tttgatgttg gtagttctag taacgtctta attcaaggat gtactgttta taatcaggat 600
gactgtattg ctgtgaattc cggttcaact attaaattta tgaacaacta ctgctacaat 660
ggccatggta tttctgtagg ttctgttggt ggccgttctg ataatacagt caatggtttc 720
tgggctgaaa ataaccatgt tatcaactct gacaacgggt tgagaataaa aaccgtagaa 780
ggtgcgacag gcacagtcac taatgtcaac tttatcagta ataaaattag cggcataaaa 840
agttatggta ttgttatcga aggcgattat ttgaatagta agactactgg aactgctaca 900
ggtggcgttc ccatttcgaa tttagtaatg aaggatatca ccgggagcgt gaactccaca 960
gcgaagaggg ttaaaatttt ggtgaaaaac gctactaact ggcaatggtc tggggtgtca 1020
attaccggtg gttcttccta ttctggatgt tctggaatcc catctggatc tggtgcaagc 1080
tgttaa 1086

<210> 107

<211> 504
<212> DNA
<213> Saccharomyces cerevisiae

<400> 107
atgcaagaat tttattcttt ttcaccaata gatgaaaacg gaaatccatt ccccttcaac 60
tccctgcgta acaaagtggt tttgatagtc aatgtagcat ctcattgtgc gttcacacct 120
caatacaagg aattagagta cttgtacgaa aaatacaaat cacatggtct agtgattgtg 180
gcctttccct gtggtcaatt cggaaatcaa gagttcgaga aggataaaga gattaataag 240
ttctgtcaag ataaatatgg tgtaaccttc cctattctac ataagatccg ttgcaacggg 300
caaaagcaag atcccgtcta caagttctta aagaattcag taagcgggaa gtctggaata 360
aaaatgataa aatggaattt tgaaaagttt gtggtagacc gaaatgggaa ggtggttaaa 420
aggttttcat gcatgactag gccactagag ctttgtccaa taatcgaaga attactgaat 480
caaccaccag aagaacagat ttaa 504

<210> 108
<211> 1551
<212> DNA
<213> Saccharomyces cerevisiae

<400> 108
atgaattccg gaggtgaaga accaactata aagccaaatg tatttaatat tactcaactt 60
ttaaactcaa atggtgaaaa accgggaatt gcttgcatct tcttaagtaa atttgacatg 120
aaaaagggaa atatcataat atggtctaaa agtattaatg gtgcggcaat tgacttaagc 180
aatattgaat ttaaatcgtt accagccggt attcacgaac aaactgacga tgttgtcaat 240
tttgtagttc ctaaagaatt agatgtctgt caaactgcca agacgacaac ttatgattac 300
ggcatcgcct atttcaaaca aaattcgttc gatattattg agaatgacaa tcgtattgat 360
aggagtaaag ttcaaatgtt ttctcttggt gtaattatcg atgtccaaaa tgcctcatca 420
gatagtaaaa agcactttta taaggaaata taccatgcgt atgctgccaa cagatactca 480
agctatctag aaagtttact cggtcaatgg ataaggcagc gtgatcttga taagtttgat 540
atttttgaaa agttttttga cgagaataac caagggcaca tggctgaaaa ttcagtagaa 600
gtctttgaac attcacccaa agaaagacgg catttggttg aatatttgcc atactggaca 660
aggaagttag ggcctctcat atttccactt tggaaagcaa gtctccttca gtcaaggata 720
ttgatactag ttcctcaagg agaatcgttc gaattgtgca attctttggc ctattgtgtc 780
ttcttaatat caatgcttcc aaaaaacttg ataggaaacc atgtaagcga tgaatatatt 840
aagcctattt tcacggtatc aacatcagat attccttttc tagagtcgtt caaaaagggg 900
aacggttatg tggctacgac cagcgaggaa attttgttgt ataagcctga aatttacgac 960
atagtagtca aacttacttc gagctcaact atagaagaat caccagaaaa agaagtggaa 1020
attcttaccg catctggtga acaaaacaaa gcaactcctc ttgacttaga ggtgtatgaa 1080
aaacttatac tgggggagtt acaagaggat gcctctacaa atgcgacatg tcgacatcat 1140
gaggtgacag aacccatatc atggctacag tttttaattg atgggttttt tcttttaact 1200
actgcaggct atctggtagc gccatatcac cttgcaaata atttcaagat tccgaggcac 1260
gtttctggcc cagaacccaa caattcagaa atacagatcg ctgaaaattt agttcgctat 1320
tttcaccgca gaacatcaaa tctgtacaac gatctgaagg atgtcataca aaaaagtgaa 1380
aatattgatt cagaacaacc gattactatc gcagcttcat ttttaaccaa gttaaatttg 1440
gattgcttta gcaaacaaga tcatcaattc gtcaaggata tcgcccttaa atggttccaa 1500
agaagcattg atattagcaa cctaccagaa tgtttgggaa atctctgtta a 1551

<210> 109
<211> 1353
<212> DNA
<213> Saccharomyces cerevisiae

<400> 109

atggctaaag atttattgcc caagcaagcg gccaacgagc catcgttaaa ggactgtacc 60
tgtaaaagat gtcttaaatt gggtgcttca aaagaaaaaa agattagaag aaagaagaaa 120
ggtgaggaga aaagagaaag acattatgga aatagacgaa agttaacatt caactttttg 180
aagcatacga acatggaaaa tacaaattac gacgtaatca cttcggttgg atatctgaat 240
gaaaaatacg gattgaaaaa atcacattac attgaaaaat ttatcaaatg catccataga 300
aaaataaata tcgatgttag caaaattacg gacgcctatg taaactcgct aaacccgtgg 360
gttaaagtaa aactcttcct tttattggtg acattgtctg aaaagggtgg tccggaatac 420
tggctagata agacagatgg tgaaaagaac tcagaggcgt cttcaacaga taatagtttg 480
gaaaattcta ctaaaggagc agatagcgct ggatctactg ctttgagaga tgaaatggtc 540
aaaagccata aaaacctttt cccaactctt acagagcaaa tcatacagca taacatcaac 600
caagatttta cggaatcgac ttacgacgag gattatgtat tttcgtcaat ttgggcaaac 660
ttcatggaag gactgatcaa tcactatttg gaaaaagtca ttgtacctta ctcggaaatg 720
aaagtctgtc agcaattata caagcctatg atgaaaatta tctccctcta taacgaatac 780
aatgagctta tggtcaaaag cgaaaagaac ggattcttgc cttctctaca agactccgaa 840
aatgtccaag gtgataaagg cgaaaaagag agcaaggatg atgccgttag ccaagaaaga 900
ctggaaagag cgcaaaagct gttatggcaa gctcgagaag atattccaaa aacaattagc 960
aaagaattaa cgttgttatc agaaatgtat tctacattat ctgctgatga gcaagattac 1020
gaattagatg aatttgtttg ttgcgcagag gagtacattg aattggaata cctacctgcc 1080
cttgtagatg tcttgtttgc taattgtggc acgaacaact tctggaaaat tatgttggtg 1140
ttggaaccct ttttctacta cattgaagac gtcggcggag atgatgatga agatgaagat 1200
aatgtggaca atagcgaggg cgacgaagaa agcttactta gcaggaatgt ggaaggagat 1260
gataatgtgg ttgagcgtca ttttaagcct gatccaaggg tcattacact ggaaaaaata 1320
tgtgaagttg ctgcaagaca gaaatggata taa 1353

<210> 110
<211> 1239
<212> DNA
<213> Saccharomyces cerevisiae

<400> 110
atgtcccaag gtactttata cattaataga tctccaagaa actacgcttc tgaagctttg 60
atttcttact ttaaactaga tgtcaagatc gttgatctag aacaatctag cgagtttgct 120
tctttgttcc cattgaagca agcccctgca tttttgggtc caaagggctt aaagctaact 180
gaagctttgg ctatccaatt ttatttggct aatcaagttg ccgatgaaaa agaaagagct 240
cgcttattag gttccgacgt tatcgaaaag tctcaaatcc ttagatgggc atctctagct 300
aattccgatg tcatgagcaa cattgctcgt ccattccttt ctttcaaagg tttgattcca 360
tacaacaaga aagacgttga tgcttgtttt gttaaaatcg acaacctagc tgctgtcttc 420
gatgctagat tgagagacta cacctttgtt gctaccgaaa acatttctct aggtgacctt 480
catgccgctg gttcttgggc ttttggtttg gccactattt tgggccctga atggagagct 540
aagcatcctc atttgatgag atggttcaac actgttgctg cttctccaat cgtgaagact 600
ccatttgctg aagtcaaatt ggctgaaaag gctctaacct acactccacc aaagaagcaa 660
aaggctgaaa agccaaaggc cgaaaagtca aaggctgaaa agaagaagga cgaagctaag 720
ccagcagatg atgctgctcc agctaaaaag ccaaagcacc cattggaagc tttaggaaag 780
tccacatttg tcttggatga ctggaagaga aagtactcca acgacgacac cagaccagtt 840
gctttgccat ggttctggga acactacaac cctgaagaat actccatctg gaaggttggt 900
tacaaataca acgacgaact taccttgact ttcatgtcca acaacttggt cggtggtttt 960
ttcaacagat tgtccgcttc tactaagtac atgttcggtt gtttagttgt ctatggtgaa 1020
aacaacaata atggtattgt tggtgccgtt atggtcagag gccaagattt cgctccagcc 1080
tttgatgtcg ctccagactg ggaatcttac gaatacacca agttggaccc aaccaaggaa 1140
gaagacaaag aatttgtcaa caacatgtgg gcttgggata agcctgttgt tgtcaatggc 1200
gaagataagg aaattgttga cggtaaggtt ttgaaataa 1239

<210> 111
<211> 279

<212> DNA
<213> Saccharomyces cerevisiae

<400> 111
atgcaattct ctactgtcgc ttccgttgct ttcgtcgctt tggctaactt tgttgccgct 60
gaatccgctg ccgccatttc tcaaatcact gacggtcaaa tccaagctac taccactgct 120
accaccgaag ctaccaccac tgctgcccca tcttccaccg ttgaaactgt ttctccatcc 180
agcaccgaaa ctatctctca acaaactgaa aatggtgctg ctaaggccgc tgtcggtatg 240
ggtgccggtg ctctagctgc tgctgctatg ttgttataa 279

<210> 112
<211> 396
<212> DNA
<213> Saccharomyces cerevisiae

<400> 112
atgagtcaaa gtcgctggag tatcgttctc atctttgcat tgtttatttt tggcagcact 60
ggagtgaatg ccttcttcaa tttcggtcat catcagcaac agcaacagca acagcaacaa 120
tcatatgaag atcaagtttt aaataaccct tgtgatgggt atttgtgtcc ggacacccta 180
acgtgcgttg cacagcagaa agactgtcct tgtcccttcc ctaaatctca gttgaaatgt 240
gtccttcctg ataataaatt tgtttgcata tccaaaccag ccacacacaa tgaaaagttt 300
agagcaatat atgatgaccc tgtcaagggc cccaaagcaa agaacaaggg gtttagggat 360
tgtggatggg tatcagatgc ttacaaaaac cattga 396

<210> 113
<211> 810
<212> DNA
<213> Saccharomyces cerevisiae

<400> 113
atgagtaata ataaaataaa aagaaaggat gcaagcccgg aacaagaggc aattgaatca 60
ttcacctcat taaccaaatg tgaccccaag gtatccagga agtacctgca gcgtaatcac 120
tggaacatca attacgctct caatgattat tatgacaagg aaatagggac gtttactgac 180
gaggtttcaa ctgtggctca tccacccgta tacccgaagg agctaacaca ggtatttgaa 240
cactatatca ataacaactt gtttgatata gactcactgg tcaagttcat tgaggagcta 300
ggctacaatc ttgaagattt agcgacatta tgcttagccc atttactggg gtacaagaaa 360
ctagaagaac ccttaaaacg agaggacttc ctgtcaacat ggtttatgca aggctgttcc 420
accatttcag acatgcaaga gtgcatcaag acgttagatg ttaaattaca tgaagactta 480
caatatttca cacagattta taactatgct ttcaatttga ttctggaccc aaatcgaaag 540
gatatagata cagacgaagg catccagtat tggaagctat ttttccaacc agagtaccca 600
gtgcgtatgg aaccagattt gcttgaggca tggttccgtt tccttcgcga tgaggggaaa 660
accactataa gtaaagacac ctggcgtatg ctgctcctct ttttcaaacg ataccccact 720
attcagaaaa taataagcga ttacgacgaa actgcagcct ggccatttat tatcgatgag 780
ttctatgagt gcttacagga tcagcaataa 810

<210> 114
<211> 1008
<212> DNA
<213> Saccharomyces cerevisiae

<400> 114
atgtctgaac cagctcaaaa gaaacaaaag gttgctaaca actctctaga acaattgaaa 60
gcctccggca ctgtcgttgt tgccgacact ggtgatttcg gctctattgc caagtttcaa 120

cctcaagact ccacaactaa cccatcattg atcttggctg ctgccaagca accaacttac 180
gccaagttga tcgatgttgc cgtggaatac ggtaagaagc atggtaagac caccgaagaa 240
caagtcgaaa atgctgtgga cagattgtta gtcgaattcg gtaaggagat cttaaagatt 300
gttccaggca gagtctccac cgaagttgat gctagattgt cttttgacac tcaagctacc 360
attgaaaagg ctagacatat cattaaattg tttgaacaag aaggtgtctc caaggaaaga 420
gtccttatta aaattgcttc cacttgggaa ggtattcaag ctgccaaaga attggaagaa 480
aaggacggta tccactgtaa tttgactcta ttattctcct tcgttcaagc agttgcctgt 540
gccgaggccc aagttacttt gatttcccca tttgttggta gaattctaga ctggtacaaa 600
tccagcactg gtaaagatta caagggtgaa gccgacccag gtgttatttc cgtcaagaaa 660
atctacaact actacaagaa gtacggttac aagactattg ttatgggtgc ttctttcaga 720
agcactgacg aaatcaaaaa cttggctggt gttgactatc taacaatttc tccagcttta 780
ttggacaagt tgatgaacag tactgaacct ttcccaagag ttttggaccc tgtctccgct 840
aagaaggaag ccggcgacaa gatttcttac atcagcgacg aatctaaatt cagattcgac 900
ttgaatgaag acgctatggc cactgaaaaa ttgtccgaag gtatcagaaa attctctgcc 960
gatattgtta ctctattcga cttgattgaa aagaaagtta ccgcttaa 1008

<210> 115
<211> 342
<212> DNA
<213> Saccharomyces cerevisiae

<400> 115
atggccggtt taaaagacgt tgtcactcgt gaatacacca tcaacttgca caaaagacta 60
catggtgtca gtttcaaaaa gagagctcca agagctgtga aggaaatcaa gaaattcgcc 120
aaattacaca tgggtactga agatgtccgt ctagccccag aattgaacca agctatctgg 180
aaaagaggtg ttaagggtgt tgaatacaga ttgagattga gaatctccag aaagagaaac 240
gaagaagaag atgccaagaa cccattgttt tcctacgtcg aaccagtttt ggttgcctct 300
gctaagggtc tacaaaccgt tgtcgttgaa gaagatgctt aa 342

<210> 116
<211> 792
<212> DNA
<213> Saccharomyces cerevisiae

<400> 116
atgaggaatt ttttcacgtt attttttgca gctatatttt cgctaggagc acttatatta 60
gccattgttg catgcgcagg atcaacgaaa aattacagtc ccataaataa aatttactgt 120
gcagaattgg atctgtcgca gatgaaggta tcgacggtgc tcccttcttt gagttctgct 180
acgctatctt cgttgggcct gccctcatat ataaatatag ggctttggtc gtactgtaca 240
gtggactcct cgcataacat ccaatcatgt tcttcgcctc acggtatcca gaattttaac 300
ctatcgtcat tagtgtatga caatatcaac aacaatgagg ctctggagct tatggattcc 360
gtggccagtg ttgttttgcc cgaaaaacta aaaagtaaaa tgacatacta caacaatttg 420
gtcaagtgta tgttcattac cattcttatt ggtattgtct tgacctttgt gaatctagtg 480
ttcaacgtat tgcgctggat catccacata aggccgctaa cgtggtttgg tgcctttttt 540
tcatttttcg cctttgccgc cctattagtc agtataggtt cgtgtttggg cacttactca 600
tacatcaaat acatcctaaa gcataactat agtgattacg gtatttcaat gagcattggt 660
aggaactacc agggtttgat gtggggggct gtcgttggag cattactgaa tttcattcta 720
tggtgtagcg tgagatcgag gcccaccgtc atctatgcga acgctccaat tgaggaaaaa 780
ccattgattt ga 792

<210> 117
<211> 387
<212> DNA

<213> Saccharomyces cerevisiae

<400> 117
atgcttgttt tttcttttct gtttgttgta gtctctataa atttaaatgc acttatcttt 60
ttatgcaaaa aaagctgggc gagttatttg tttctatatt tgtataattt ttttttttgt 120
gaagatgaat ataaactgac aaaaaattgt gtcagagttg aagctatagc tccttttatg 180
atgtgtcttg gttctttggg tgcgatatta gggaaacaac gcacagcaaa ttttcttctg 240
ctttcttata acgttataaa caatcctgtt gtcttggttt attatgtgga aaacttttca 300
cggatcaatt ttataaagca tacaactaaa gagaaaagtg taatatggac gaacgagaga 360
caacttaatc catggatttg taattga 387

<210> 118
<211> 1578
<212> DNA
<213> Saccharomyces cerevisiae

<400> 118
atggctgccg gtgaacaagt ttctaacatg tttgacacta tcttagtgtt agactttggt 60
tctcaatact ctcatttgat taccagaaga ttaagagagt ttaacatcta tgctgagatg 120
ttgccatgca ctcagaagat ctctgagttg ggctggactc caaagggtgt cattctttct 180
ggtggtccat attccgtata cgctgaagac gctccacacg tagaccatgc tatctttgat 240
ttgaacgttc caatcctggg tatctgttac ggtatgcaag aattggcttg gattaatggt 300
aaacaagttg gtcgtggtga caagagagag tacggtcctg ccactttgaa ggtgattgac 360
gattccaact ctctattcaa aggtatgaac gactctactg tctggatgtc tcacggtgat 420
aaattgcacg gcttgccaac tggttacaag accattgcca cctccgataa ctccccatac 480
tgtggtattg tccacgaaac taagccaatc tatggtattc aattccaccc agaagttaca 540
cattctaccc aaggtaagac tcttttgaaa aacttcgccg ttgatttgtg tcacgctaag 600
caaaactgga ctatggaaaa ctttatcgac actgaaataa acagaatcag aaaactggtc 660
ggtccaactg ctgaagtcat tggtgctgtt tccggtggtg ttgattctac tgttgcatcc 720
aaattgatga cagaagccat cggtgataga ttccatgcca tcttggtcga taacggtgtt 780
ttgagattaa atgaagctgc taacgttaaa aagacccttg tcgaaggttt aggtatcaac 840
ttgatggttg tcgatgcttc cgaagaattt ttaagtaaat tgaagggtgt cactgaccca 900
gaaaagaaga gaaagattat tggtaacaca tttatccacg tctttgaaag agaagctgaa 960
aaaattaagc caaaagatgg taaggagatt caattcctgt tacaaggtac tctttatcca 1020
gatgtcatcg aatccatttc tttcaaaggt ccttctcaaa ccattaaaac tcatcacaac 1080
gtcggtggtt tactagagaa catgaaatta aagctaattg aaccattgag agaactgttc 1140
aaagacgaag tcaggcatct aggtgaatta ttaggaatcc ctcatgatct agtctggaga 1200
caccctttcc ctggtccagg tattgctatt cgtgttctag gtgaagttac taaggaacaa 1260
gtcgaaatag caagaaaagc tgacaacatt tacattgaag aaatcaagaa ggccggctta 1320
tacaaccaaa tttcccaagc ctttgcctgt ttgttgcccg ttaaatccgt tggtgttatg 1380
ggtgaccaaa gaacctatga tcaagttatt gctttgaggg ccattgaaac tactgatttc 1440
atgactgctg actggttccc attcgaacac agtttcttga agaaagtcgc ctcaagaatt 1500
gtcaatgaag tcgatggggt tgctagggtc acttacgata taacttccaa acctcctgca 1560
actgttgaat gggaatga 1578

<210> 119
<211> 726
<212> DNA
<213> Saccharomyces cerevisiae

<400> 119
atggtcaaag ctgttatttt taccgatttc gacggtaccg ttactttgga agattctaac 60
gattatctga ccgatacttt aggtttcggt aaggaaaaaa gattaaaggt cttcgaaggc 120
gtgttagatg atactaagtc ttttaggcaa ggtttcatgg aaatgctgga atccatccac 180

acccctttcc ctgagtgtat caagatctta gaaaagaaaa ttcgattgga tcctggtttc 240
aaggatacat tcgaatgggc tcaagaaaat gatgtccctg tcatcgttgt ttccagcgga 300
atgaaaccaa ttatcaaggt tttattgacc agattagttg gacaagagtc tattcacaaa 360
attgacattg tttccaatga agtggaaatc gatgcacatg atcaatggaa aatcatctat 420
aaggatgaaa gtcccttcgg acatgacaaa tccagaagta tcgatgctta taaaaagaaa 480
tttgaatcta ccttgaaagc aggtgaacaa agacccgttt acttttactg tggtgacggt 540
gtttctgatc taagtgctgc aaaagaatgt gatttgctat ttgccaaaag aggtaaagat 600
ttggtcactt attgtaaaaa acaaaacgtc ccattccatg aattcgatac cttcaaagac 660
atcttggcta gcatgaaaca agttctggct ggtgaaaaga cagtcgctga attgatggaa 720
aattag 726

<210> 120
<211> 1761
<212> DNA
<213> Saccharomyces cerevisiae

<400> 120
atgtcacgaa gtaacagtat atacacagaa gatattgaga tgtatcctac tcacaatgag 60
cagcatctga cgagagaata cacaaaacca gatggtcaaa cgaaaagtga aaaactaaat 120
ttcgaaggcg cttatatcaa cagtcacggg actctatcca agactaccac aagggaaata 180
gaaggcgatt tagactctga gacttcttct cactccagtg atgataaagt tgatccaacc 240
caacagataa ctgcagagac aaaagcgcca tatactttac taagttacgg tcagaagtgg 300
ggaatggttg caattctaac aatgtgtggc ttttggtctt cattgggatc accgatctat 360
tatcccgctt tgaggcagtt ggaaaagcaa tttaatgtgg atgaaaatat ggttaacgtt 420
actgtggttg tgtatctgtt gtttcaaggt atatctccca cagttagcgg tggtctggcg 480
gattgctttg gacggagacc tataatttta gcaggtatgc tgatatacgt tattgcatcc 540
atcgggctag catgtgctcc atcatacggt gttattatct tcttgaggtg tattcagagt 600
attggtattt cccccacaat tgcaatcagt tctggtgttg tcggggactt cactttgaag 660
catgaaagag gtacatttgt tggcgccacc tcagggtttg ttttactggg tcaatgtttt 720
ggtagtctta ttggtgccgt cctgactgca aggtgggatt ggagagctat cttttggttt 780
ttgaccattg gttgtggaag ttgttttcta attgcctttc ttattttgcc agaaacaaag 840
cgaaccattg caggtaacct ttccattaag cctaaaagat tcatcaacag agcgccgatt 900
ttcctactgg gcccagttag aaggagattc aagtacgata atcctgatta tgagacatta 960
gatcccacca ttccgaaact agatttgtca tccgccggga agatccttgt attgccagaa 1020
attattctgt ctttatttcc atcagggctc ttatttgcta tgtggacgtt aatgctttcc 1080
tctatttcat ccggtttatc agtggcgcca tacaactatc atttggtcat tattggtgtg 1140
tgttatctac ccggtggtat tggcggttta atgggttcct tcttcacagg tagaattatt 1200
gacatgtatt tcaagagaaa gatcaagaaa ttcgagcaag acaaagctaa cgggttgatt 1260
ccacaagacg cagagattaa tatgttcaaa gtccgtttag tttgtcttct gccccaaaat 1320
ttcttggccg tcgtagccta tcttttattt ggttggagta tagataaggg ctggaggatc 1380
gaatctattt taattacttc atttgtctgt tcatactgtg ctatgagtac attatcaaca 1440
tcaactacgt tattggtcga tttgtaccca acgaaatcat ctacagcaag tagttgtttc 1500
aactttgtta ggtgcagttt gagtaccatc ttcatgggtt gctttgccaa aatgaaagct 1560
gcgatgactg taggtggtac ttttacattt ttatgtgcgc tggtattttt cttcaacttc 1620
ttaatgttta ttccgatgaa atatggtatg aagtggaggg aggatagatt attgaaacaa 1680
caaagacagt cttggttaaa caccttggca gtcaaagcca aaaagggaac aaaaagagac 1740
cagaacgata atcataatta a 1761

<210> 121
<211> 1368
<212> DNA
<213> Saccharomyces cerevisiae

<400> 121

atgtcaaaat tgttcttaga tgaactgcct gagtctctca gccgaaagat tggtaccgtg 60
gttagagtgc tgccaagctc attagaaata tttgaggaat tgtataaata tgcattgaat 120
gaaaatagca acgatagaag cggacgccat aaaaagccca gaatagatgt ctcttctgac 180
ctgcttaaaa cagatgaaat ttcggaaacc aatactattt tcaaattaga aggtgtttct 240
gtactgtcac cattaaggaa aaagcttgat ttggtgttct atctttccaa tgttgacggg 300
tctcccgtaa taacattact gaagggaaat gacagggaat taagcattta tcagctgaac 360
aaaaatatca aaatggcttc gttcttgcca gtgccagaaa agcctaatct aatatacctt 420
tttatgactt acacatcgtg cgaggataac aagttttctg aacctgtggt aatgaccttg 480
aacaaagaaa acacactaaa ccagtttaaa aatttagggc ttcttgattc caatgtaaca 540
gactttgaaa agtgcgtcga atatataaga aagcaagcca ttttgacggg ttttaaaatc 600
tccaacccat tcgttaacag tacactggtg gataccgatg ctgaaaaaat aaactcattt 660
cacctgcagt gtcatagagg tactaaagaa ggcacgctat atttttaccc agatcatata 720
atttttgggt ttaaaaagcc cattttgtta ttcgacgctt ctgacatcga gtctataacc 780
tattcgtcta ttaccagatt aacattcaat gcaagcttag tgacaaaaga tggtgaaaaa 840
tacgagtttt ccatgataga ccaaactgaa tacgctaaaa ttgacgacta cgttaaaagg 900
aaacaaatga aggataagtc tatgtctgag gaattgaaag ctaaatccaa gagcaaaggc 960
caagcaactg atggcacagc cgatcagcca tctattctac aggaagctac ccgtcaaatg 1020
caagacgaga aaaaggcagg tgtgttttca gacgatgatg aagaaaatga ccaaaatttc 1080
gaagctgaga gcgacctttc cgatgggagt ggccaagaat cctcagacgg tgcagaagac 1140
ggagaggaag cagaagaaga tgatgaagaa gacgatgaag aggaagacaa aaagggccaa 1200
agcgctttga atagagataa tagttttgct tctataaatg gtcaaccaga acaggaactc 1260
caatacaaag agtttaagga gccgttagaa ctcgaagata ttccgataga aattgataat 1320
gatgacgatg aagatgatga ggatggatcc ggagtagaat acgattga 1368

<210> 122
<211> 396
<212> DNA
<213> Saccharomyces cerevisiae

<400> 122
atgtgtggta tctatcccta ccagttggta acttcttcgg cctcaccaaa gacagacgtc 60
ttcgtttcat cgttaccaac gtcacctccc caagctcttg gagcggcatc ggtaatggcc 120
ttgcagttgg tttttaaaaa ttgtactatc agtttgttta tcagcttaaa ctccaatagg 180
aaggcatcgt ggccttcggg agattcaatt ttttctaatt gcgacttcgg tatgtgctca 240
gccaaaaatt cttgttctga atatgtgaac agtccatcag attggatacc gatgatcagg 300
gatggttgtt ggatggtaga aaggacctca gtgatgtcat ctactctgtc tcttgccaaa 360
tcgtgcgtat ccagtttacg tgtgatggca atgtaa 396

<210> 123
<211> 1986
<212> DNA
<213> Saccharomyces cerevisiae

<400> 123
   atgagcggtt ttaaatgcta tttgcaattg ggtgacaggc tctctcaaat atggctaaat 60
   aagtatactt tggttttgct gctagcaatg ctgaagcttc tgtttttctc caaatccata 120
   caacatgcga tagaagtctc ggaaacgtat attttgtcca attgttacag tattgattca 180
   ctatactcca agatgacaga caacacgccg cactatttag gtatcatggg gaattatctt 240
   atcgagaagg gtatggagga gactgttaaa gctacgctag agacgttatc acttatagta 300
   tatgcgagcg aggggctggt aaactttgcc attgacctgt atttgggcac ttatgcctgt 360
   ttgattgtta gtgccgttga tggtaccgtg gacgttgcta ctaacattac agaaaaactg 420
   attagcttag tcaatgatac agtttcaagt gtggctaatg aattggatac gggcttgaat 480
   gacatctcca aaataatcaa taaagtgatc aaggccgcat ccaaagtaga gaattttttc 540
   acaggtgatg acgatgacag taacatgacg tcgtcaatca aaagcgtcaa cttaaccata 600
   tctgcgcttc acaatttata cattccttcc tcaatcaacg ataagcttga agagttatcg 660
   gcaaagacgc cggactttgc ccaggttaag aatacaacca agaacctgat ctcggttccc 720
   ttcaatgaag ttcggaagaa tatcaaggcc gtgaatgcca gcaatataat cggagatacc 780
   tccgttttgt acgtacctcc cgtgtccctt gacaacagta ctgggatttg ctcatccaat 840
   caatcagaaa ttttggcctt ttattccatc ttgggacatg tcctgaaaat agccaccgta 900
   gtgtgcatta ccgtattgat atgcttcgct gttggtgcga tggcgcccgt tgcatggaat 960
   gaaatcaagc tctggaggcg cctttgcgga atgagagacc attacatgct gagcaggcaa 1020
   gattcgtata cgtccttttc cagtgaaaac acgcacgaat tgaaagatcc atttagagat 1080
   cctcctatac aaaatggcca atatgatgtc attgcaagct atcagcagtg ctttcaaaca 1140
   tggaacacaa gaatagcagg ctggatgaca aatcttgtta cctttggaaa atcaccagag 1200
   aacattgacc caaagactaa acaaaaaata gaatgggtag tggcttatat gacctccgaa 1260
   agagcactgt gtgttcttgg aattggactt ttgggaattt tagtgtgcat atgccaattt 1320
   gtcatgatag cactgttaaa acacaagata agccattcat tgacttctaa tgatggtgac 1380
   ggcgttcaaa atttgctgaa gtctagcact gccgtcgata tagagaacca aatgagcctt 1440
   tggagcgttc agactaataa atatataaat actacggaga ccaatatcaa tcaggaagta 1500
   ttcgggtgga taaacacgac aacactttct gtgaacaata cagtggccac catgatctct 1560
   gatatagaca caactttagc agatgtattc aatggaacac tgctatataa cccaatgaaa 1620
   accgtggtcg gatgtgccat tgaaaataag ctctacacaa tagagaaggc aatgacgtgg 1680
   attcacgaca aggctcagct gcatatcccg agaattaatg ggacacaaat caagcaagct 1740
   ctggcaaagc aaaccgacaa cagcactata cccactgcaa gctccacttc tgccgccaca 1800
   gaaaacttac tggagaacct tgtgaatgat atgagagaag gacttttaaa aattctccga 1860
   gcttaccacc gtataactct gggagaactc acggtagcct tggtcattct tgcggtgtgg 1920
   ctcgtacaat tgcccatagc tctggtaatt ctccgattac gtcttcgcaa agccaccttt 1980
   gactga 1986

<210> 124
<211> 1155
<212> DNA
<213> Saccharomyces cerevisiae

<400> 124
   atggttacat atgctggaaa actggttcta gctccaatgg taagagccgg cgaactgccc 60
   acgagactga tggcgctggc ccacggtgca gatctcgtct ggtccccaga aatcatcgac 120
   aaaaagctaa ttcagtgcgt caggaaggaa aacactgcac tccaaactgt cgactacgtc 180
   gtgccgtcca aggttcaaac gaggccggag acgctcgttt tcagaacata tccaaaacta 240
   gaatcctcta aattgatttt ccagatcggg tctgcatccc cggctctggc cacccaggct 300
   gctttgaaag tgatcaacga cgtcagcggg atagatatca atgcaggctg ccccaaacac 360
   ttctctatac actccggcat gggctctgcc ctcttgcgca ccccagacac actgtgtctc 420
   atcttgaaag aactggttaa gaacgtgggt aaccctcaca gcaaacccat cagtgtgaaa 480
   ataagactct tggacaccaa gcaagacact ctacagctcg tcaaacgtct gtgtgccacg 540
   ggcatcacaa atcttacagt tcactgcagg aagactgaaa tgagaaacag agagcagccc 600
   atcacagatt acattgcgga gatctacgag atatgccagg caaataacgt gtcgttgatt 660
   gtgaacggcg ctatccgcga ccgctctcat tttcatgatt tacaagcgaa tcattggaaa 720
   aacactaaca ttggtggtat gattgcagag tgtgctgaac gggaccctac agtttttgat 780
   cacacttcta agccttcaga agacggtcca tcttgggtcg ttgcttgtcg cgagtttatt 840
   cagtgggcaa cgaagtttga caaccacatc ggcaatacaa aatacatgtt aagcagaatt 900
   gtccccggaa aatcagtctt ttttcagtac tttgcccgtt gcaagagccc ggaagaagtt 960
   agtttcgtac taaagcaatt gaacgatgat ggaagcgctc aaacagatcc ttcagagtac 1020
   ttggaaaact gcagagctca agaaaaagct ttgaaaaacg ccaacgccat cgccaagcaa 1080
   aagaggaaac agaccgatca cataggcagt gacactaaaa agcaaaaggt tgtacccctt 1140
   cccacagata tataa 1155

<210> 125
<211> 1656

<212> DNA
<213> Saccharomyces cerevisiae

<400> 125
atgacctatt ctacagttag catcaacacg cctccaccat atcttacttt agcctgcaac 60
gaaaagttgc caacagtttt atccattgct ggaacagatc caagtggtgg agccggtatc 120
gaagctgatg tcaaaactat cacagcacac agatgttatg ccatgacatg tatcactgct 180
ttaaatgctc aaactccagt taaagtctac agcataaata acacaccaaa agaagtggtt 240
ttccaaactt tggaatccaa tttaaaggat atgaaatgta atgttatcaa aaccggtatg 300
cttaccgcag ctgctatcga agtcttgcac gaaaaactgc tgcaactcgg cgaaaataga 360
cctaagttgg ttgttgatcc agttcttgtc gctacttctg gttcttcttt agctggaaag 420
gatatagtta gtttgattac ggagaaggtt gcaccttttg ctgatatttt gactcctaac 480
atcccagagt gttacaaatt gttaggtgag gaaagaaagg ttaacggatt acaggatatt 540
ttccaaatcg caaaagacct tgctaaaata accaagtgtt cgaatatact agtgaagggt 600
ggccatattc catggaatga tgaaaaggaa aagtacatta ccgacgttct tttcttgggt 660
gctgaacaaa aattcattat ctttaaaggc aatttcgtta acaccacgca tacacatggt 720
actgggtgca cactagcttc tgccattgca tcaaacttgg ctcgtggtta ttctcttcct 780
cagtctgttt atggtggtat tgaatacgtc caaaatgccg tggcaattgg ttgtgatgtc 840
acaaaagaaa ctgtcaagga taatggacca attaatcatg tttacgccgt ggaaattcca 900
ttggagaaaa tgctcagtga tgaatgtttt actgcatcgg atgttatacc taaaaagccg 960
ctcaaaagtg ctgctgacaa aatcccaggc ggtaatttct atgaatacct gatcaatcac 1020
cctaaagtta agccacactg ggattcctat attaaccatg agtttgttaa aaaggttgcg 1080
gacggtacat tagaacgtaa gaagtttcag ttctttattg aacaagatta tgcatacttg 1140
gttgattatg ctagagttca ttgtatcgcg ggtagtaaag ctccatgcct agaagatatg 1200
gaaaaggagc tagtcattgt tggaggtgtt cgtactgaaa tgggtcaaca tgaaaagaga 1260
ttaaaagaag tatttggtgt taaagatcct gattactttc aaaagattaa gagaggacct 1320
gcattaagag cttactctcg ttactttaac gatgtttcca gaagaggtaa ctggcaagaa 1380
ttggttgcat cccttactcc ttgtttgatg ggttatggtg aagctttaac taaaatgaag 1440
ggtaaagtca ccgcacctga agggtccgtt taccacgaat ggtgtgaaac ttacgcatct 1500
tcatggtatc gtgaagccat ggatgaaggt gaaaaacttt tgaaccacat cttagaaaca 1560
taccctccag agcaacttga cactttagta acaatctacg ctgaagtttg tgaattagaa 1620
acaaattttt ggaccgctgc gctagaatac gagtag 1656

<210> 126
<211> 1452
<212> DNA
<213> Saccharomyces cerevisiae

<400> 126
atgctttctg caaccaaaca aacatttaga agtctacaga taagaactat gtccacgaac 60
accaagcatt acgattacct cgtcatcggg ggtggctcag ggggtgttgc ttccgcaaga 120
agagctgcat cttatggtgc gaagacatta ctagttgaag ctaaggctct tggtggtacc 180
tgtgttaacg tgggttgtgt tccgaagaaa gtcatgtggt atgcttctga cctcgctact 240
agagtatccc atgcaaatga atatggatta tatcagaatc ttccattaga taaagagcat 300
ttgactttta attggccaga atttaagcag aaaagggatg cttatgtcca taggttgaac 360
ggtatatacc agaagaattt agaaaaagaa aaagtggatg ttgtatttgg atgggctaga 420
ttcaataagg acggtaatgt tgaagttcag aaaagggata atactactga agtttactcc 480
gctaaccata ttttagttgc gaccggtgga aaggctattt tccccgaaaa cattccaggt 540
ttcgaattag gtactgattc tgatgggttc tttagattgg aagaacaacc taagaaagtt 600
gttgttgttg gcgctggtta tattggtatt gagctagcag gtgtgttcca tgggctggga 660
tccgaaacgc acttggtaat tagaggtgaa actgtcttga gaaaatttga tgaatgcatc 720
cagaacacta ttactgacca ttacgtaaag gaaggcatca acgttcataa actatccaaa 780
attgttaagg tggagaaaaa tgtagaaact gacaaactga aaatacatat gaatgactca 840
aagtccatcg atgacgttga cgaattaatt tggacaattg gacgtaaatc ccatctaggt 900
atgggttcag aaaatgtagg tataaagctg aactctcatg accaaataat tgctgatgaa 960

tatcagaaca ccaatgttcc aaacatttat tctctaggtg acgttgttgg aaaagttgaa 1020
ttgacacctg tcgctattgc agcgggcaga aagctgtcta atagactgtt tggtccagag 1080
aaattccgta atgacaaact agattacgag aacgtcccca gcgtaatttt ctcacatcct 1140
gaagccggtt ccattggtat ttctgagaag gaagccattg aaaagtacgg taaggagaat 1200
ataaaggtct acaattccaa atttaccgcc atgtactatg ctatgttgag tgagaaatca 1260
cccacaagat ataaaattgt ttgtgcggga ccaaatgaaa aggttgtcgg tctgcacatt 1320
gttggtgatt cctctgcaga aatcttgcaa gggttcggtg ttgctataaa gatgggtgcc 1380
actaaggctg atttcgataa ttgtgttgct attcatccga ctagcgcaga agaattggtt 1440
actatgagat ga 1452

<210> 127
<211> 1356
<212> DNA
<213> Saccharomyces cerevisiae

<400> 127
atggacggaa atgatcacaa agtcgaaaat ttaggagatg tagacaaatt ttattccaga 60
atacgctgga ataaccaatt ttcatatatg gccactctgc cgccccacct acaaagcgaa 120
atggaaggtc agaaatcatt gctaatgcga tatgatactt ataggaagga aagttcttct 180
tttagtggtg aaggcaagaa agttactctg cagcatgttc ctacagattt ttcagaagca 240
tcacaagcag tgattagcaa gaaagatcac gatacgcatg catctgcttt tgtgaataaa 300
attttccaac cagaggttgc tgaagaactt atagttaatc gatacgaaaa acttctgtca 360
caaaggccgg aatggcatgc accctggaaa ctttcacgcg ttatcaatgg ccatcttgga 420
tgggtacgat gcgttgcaat cgatcctgtt gacaacgaat ggttcatcac cggaagtaat 480
gatacgacaa tgaaagtttg ggatcttgca acaggaaaat taaaaactac cttagcaggg 540
catgtaatga cagtgagaga cgttgctgtg tcagatcgac atccttattt attttctgtt 600
agtgaagata agacggtcaa atgctgggac ctagagaaaa accaaattat tagagattac 660
tatggacatt tatcgggggt tcgtacggtg agcatacatc caacgctgga tctcatagct 720
accgcaggcc gagatagcgt tatcaaactc tgggatatga gaaccagaat acctgttatt 780
acactagttg ggcataaggg tccaatcaat caagtacagt gtactccagt agaccctcaa 840
gtggtgagtt catcgactga tgctacggta aggttatggg atgtagttgc tgggaaaaca 900
atgaaagttc taacacatca taagaggtct gtgagagcta cagcgttgca tcctaaggag 960
ttttcggtgg cttctgcgtg tactgatgac atcagatcat ggggattagc agaggggtct 1020
ttactcacca attttgagtc tgaaaagaca ggcataatca atactttaag cattaatcaa 1080
gatgatgtat tattcgctgg cggtgacaat ggtgtgcttt ccttttatga ttataagtct 1140
ggtcacaaat accaatcgtt ggccacgaga gaaatggtag gctctctgga aggtgaacgg 1200
agtgttcttt gtagcacttt cgataaaaca ggtttaagat taatcactgg agaagcagac 1260
aaaagcataa agatttggaa acaggatgag acggctacaa aagagtcaga accggggcta 1320
gcgtggaacc ccaacttaag cgccaaaaga ttttag 1356

<210> 128
<211> 414
<212> DNA
<213> Saccharomyces cerevisiae

<400> 128
atgaagagaa atattattta ttatacattg agattattgc cacacggaat aatgttagtt 60
catagcctta accaatttat tgatgaattc ttcacggtta ccgaaagaac caccttggat 120
gaaatgcttg aactttcttg ggacacccca accaccagat gggttggaca acttgaatgg 180
ccacaaaaag ttgttagctt gcttgaagtg tggaccaaca gtgatgattt cgtgaatcaa 240
atcgtcaatg gacaagatac catacttacc caagttggct tcgatgatag cattgtcgga 300
caatggaact ctttgcttgt tgatcttacc gaaacctctc ttgtagacca attgtctaat 360
agtagagtag gatgggtaac cgtaagcaac gtatggttca atcaacttca atag 414

<210> 129
<211> 1350
<212> DNA
<213> Saccharomyces cerevisiae

<400> 129
atgctacgga taagtattga ctctatcaag caattcggtt cctttgtgac aggttataac 60
aacactagtt accatgctgc cgggagggcc ataagaacat caagcctgta cagtacaatg 120
atctcagcca acccgagaag gtgcctgcat tcttcgaaac tgctaaataa agaaggtcaa 180
gaagagggct ataatgaaca gctcatttca aaaatgtctt cacagaatgg atcgaattcc 240
agacagaatg aaagtgaagg aaagaaggaa ggcaaggctt cctctgtgaa gtcgttgttg 300
caacatactc atagtcacag ccacacacat atgcatgaca atcctttatt gtcactgaac 360
gtacagcaga tcaagaagaa tcctggcgta agaataacat ggataggact tgcatcaaat 420
gtggggatgg cagtaggaaa atttgtagga ggaatcactt ttcattctca agcattactt 480
gctgattccg tacatgctct tagcgatttg gtatctgatt ttttaacact gttttctgtc 540
caatatgcgt ctcgtaaacc aacttcggag tacccatatg gctatggtaa agtggaaact 600
gtgggatctt tggcagtatc cacaatctta gcaatggctg gtatatctat cggttggtcg 660
tcgttatgcg ctattgtggg gcctgtcatt cctcatgcca ttctagaatc gatggccggt 720
ttaattggtg aaacgcattc ccattctcag tcgctaacac aacaggcaac taatgtaaat 780
gctgtatgga tagcagcggg ttctatcttg gttaaagaat gggtttttca ggcaacaaag 840
aaagttgcaa tccaaacaaa ttctaatgta ctaatggcaa acgcctggca tcatcgtgtt 900
gactccttga catcgttggt agcgttggtc gctatcacat ctagttattt tttcaacatt 960
caatccttag ataatttagg tgggcttgta gtgtcaggtt tgatcataaa aacaggtgga 1020
caaggtatct tatcctcact gaaggagtta gttgaccaat ctataccacc tactgatccg 1080
cgttatctag aaatcgaatc tgtcataaaa gacagcattg gcagtttgaa aacagactta 1140
gatctcaaac agtcgttaca tgttagagac cttactatat tagcttccgg tcctaatctt 1200
cgtgccacca caactttgga agtgccagta ttacattcag gtcaggaagt tgggataaga 1260
tttttggaaa acgctatttc gactataaga gaggatttac gtatgaaggt gccaaacgtg 1320
ggaaggtgga cgtcgagttt gttgatgtaa 1350

<210> 130
<211> 3198
<212> DNA
<213> Saccharomyces cerevisiae

<400> 130
atggattacc taaaaccagc gcagaagggg agaagacgtg gtctttccat aaacagtctt 60
tcggagactc agcaatctgc tatgaatagt tccctggacc atcttcagaa tgacttaaac 120
aggataaatc ttcaatggaa tagaatactg tcggataaca ctaatccctt agaactagcg 180
ctcgcatttt tggatgatac atctgtaggt ctgggccacc ggtatgaaga attcaatcaa 240
ttaaagtcac aaattggtag tcacttacaa gatgttgtta atgaacatag ccaagtgttc 300
aacactaatg tggcttctta cggaaaagca gtcagttcga tcatgcaggc ccaagaacag 360
actttaaatc tgaaaaattg tttaaaggaa gctaatgaga aaatcaccac tgataaaggt 420
tctttacagg aattaaatga taataacttg aagtatacaa aaatgattga tgttttagtc 480
aatattgagg agttattaca gatacccgaa aaaattgagg agaacattag aaaggaaaat 540
ttccatcagg tgcaaattct cctagaaagg ggtttcatat taatgaacaa caaatctttg 600
aagacagtgg agattttaaa gcctataaac caacaacttg agttacaaga acatttactt 660
ttcaacaatc tgattgaaga aattcacgac attatgtact ccaaatctaa caaaacaaat 720
tttactcgag taaccaataa tgatatattc aaaatcataa gcatttcaca taatggattt 780
acgagtttag aaaattacct gtacaacata gtcaatattg atattatgga acactcaaaa 840
acgataaaca agaaccttga acaattcatt catgaccaat cgttaaataa aggaaatatc 900
atgctacaag aaaatgctgc gactcaagca ccattggcac catctagaaa ccaagaaaac 960
gaaggattta acagaatagg gttcctacta aaaaccataa ataacatcaa taaattacct 1020
gttgcattca atataataac agaaagggct aaagaggaga ttcataatat aattgttaaa 1080

agtaccgaat caatacgttc gaagcaccct tctctgctta aaatggctac tagtttaaag 1140
aatgacaacc attttggcct acccgtacag gatatactat cgatcatttt aagggaatgc 1200
ttttgggaaa tatttttgaa attactgtat gctattcagt gccatagggc tatttttgaa 1260
atgtcaaaca ttttgcagcc aacgtcttcc gcaaagccag ctttcaagtt caataaaatt 1320
tggggcaaac tgttagatga aatagaatta ttacttgtga ggtacatcaa tgaccctgaa 1380
ttgatatcca gcaataacgg tagtattaaa ccaattaatg gcgcgacaaa taacgcaccc 1440
actttaccta aaaggaaaaa tcctaaaatt ttttctttgg agtataacat tgaggacaat 1500
tcttctgtaa aggatcaagc ttttgaacta aaggctttgt tgaaagatat attccctgga 1560
ttttctgtct cctcaaacat ggatttagac tctatttatg taaaagatga atccttcgaa 1620
caagatgagc ctctagtccc tccttctgtt ttcaacatga aggtaatttt agatccgttt 1680
ttgctgttta cgcaatcgac atctacaatt gttcccagtg tcttaacaca aaatactatt 1740
tcatccctaa cctttttcga tgattatatg aataaaagtt ttctcccaaa gattcagatg 1800
actatggatt atttattcac ggttgaagta gaatccaata atccgtacgc cttagagcta 1860
tccgatgaaa atcataacat ttttaaaaca gcattagatt ttcaaaggtt attttacaat 1920
ttattgaatg ttttcaacac agcaaacaca tttagagaaa aaatatcgta ctgcattcta 1980
gatcttttga atcattttta taattactac ttggggctat ttaattcctt gattggtact 2040
tctgatagac atttaactag gaaaataatt acggcatggt tgcagaatgg tattttgatg 2100
gatcaagaac aaaaaattct gaatggggat gagacactct ttcatgaaga atccatagag 2160
ttatttaagg aaatacctca tttctatcaa gcgggtaaag gtttgagcaa atctgattta 2220
ttcaataact tgacattgga cacaattctg cagttttcgg caagcgtgtt atggatatta 2280
aactggctgc caggcttaaa aaaggctatc aatattgatg aagtaagcca agaacccatg 2340
ttagatgctg atagattaag gagcagctgg acattttctg aatcaatgga tttaaactat 2400
tcgaacccta gttccagccc aaattcatta ggaaatctaa aaattttatt ggatgataaa 2460
gcctccaaaa aatttgatga gactatcgac ggattcaaaa ccttgaaatt caaactaatc 2520
actatactga ggttcaacat tagggccttg tgtatatacg acatcgggtc tttttttcaa 2580
aacaccaaaa tttggaatat ggatgtgggt agtattgaat tagatcaaaa tatagcttct 2640
ctaatttccg aattaagaag gactgaaagc aaattgaaac aacagttacc agaaaaggaa 2700
aaaaactcca tatttattgg tcttgatata gtcaacaact acgccttgat taagggtgcc 2760
aaatccataa aagttttgaa ccataacggg ataaagaaaa tgttgagaaa tgtaaatgtc 2820
ttacaacatg cctatagaaa tctttcttct gaaccatcaa aaattaatat gaacgtcaca 2880
atgaattttt actctttatg cggctccagt gaagctgaac tatttgaata tataaaagac 2940
aatgaattac cgcattgttc tgtcgaagat ttgaaaacca tattgaggct gcagtttagc 3000
gaggaaatgc atcgtcaatt gaagagacaa agcactagct caactaaggg ttccataaaa 3060
ccttccaata agaggtacac tgaagctttg gagaaactaa gtaacctcga aaaagagcag 3120
tcgaaagaag gagcccgcac caagattgga aaacttaaaa gcaaattgaa tgctgtccat 3180
actgcaaacg aaaaatga 3198

<210> 131
<211> 313
<212> PRT
<213> Escherichia coli

<400> 131
Met Lys Leu Ile Arg Gly Ile His Asn Leu Ser Gln Ala Pro Gln Glu
1               5                   10                  15

Gly Cys Val Leu Thr Ile Gly Asn Phe Asp Gly Val His Arg Gly His
            20                  25                  30

Arg Ala Leu Leu Gln Gly Leu Gln Glu Glu Gly Arg Lys Arg Asn Leu
        35                  40                  45

Pro Val Met Val Met Leu Phe Glu Pro Gln Pro Leu Glu Leu Phe Ala
    50                  55                  60

Thr Asp Lys Ala Pro Ala Arg Leu Thr Arg Leu Arg Glu Lys Leu Arg
65                  70                  75                  80

Tyr Leu Ala Glu Cys Gly Val Asp Tyr Val Leu Cys Val Arg Phe Asp
                85                  90                  95

Arg Arg Phe Ala Ala Leu Thr Ala Gln Asn Phe Ile Ser Asp Leu Leu
            100                 105                 110

Val Lys His Leu Arg Val Lys Phe Leu Ala Val Gly Asp Asp Phe Arg
        115                 120                 125

Phe Gly Ala Gly Arg Glu Gly Asp Phe Leu Leu Leu Gln Lys Ala Gly
    130                 135                 140

Met Glu Tyr Gly Phe Asp Ile Thr Ser Thr Gln Thr Phe Cys Glu Gly
145                 150                 155                 160

Gly Val Arg Ile Ser Ser Thr Ala Val Arg Gln Ala Leu Ala Asp Asp
                165                 170                 175

Asn Leu Ala Leu Ala Glu Ser Leu Leu Gly His Pro Phe Ala Ile Ser
            180                 185                 190

Gly Arg Val Val His Gly Asp Glu Leu Gly Arg Thr Ile Gly Phe Pro
        195                 200                 205

Thr Ala Asn Val Pro Leu Arg Arg Gln Val Ser Pro Val Lys Gly Val
    210                 215                 220

Tyr Ala Val Glu Val Leu Gly Leu Gly Glu Lys Pro Leu Pro Gly Val
225                 230                 235                 240

Ala Asn Ile Gly Thr Arg Pro Thr Val Ala Gly Ile Arg Gln Gln Leu
                245                 250                 255

Glu Val His Leu Leu Asp Val Ala Met Asp Leu Tyr Gly Arg His Ile
            260                 265                 270

Gln Val Val Leu Arg Lys Lys Ile Arg Asn Glu Gln Arg Phe Ala Ser
        275                 280                 285

Leu Asp Glu Leu Lys Ala Gln Ile Ala Arg Asp Glu Leu Thr Ala Arg
    290                 295                 300

Glu Phe Phe Gly Leu Thr Lys Pro Ala
305                 310


<210> 132
<211> 231
<212> PRT
<213> Escherichia coli

<400> 132
Met Leu Glu Asp Leu Lys Arg Gln Val Leu Glu Ala Asn Leu Ala Leu

1                 5                   10                  15

Pro Lys His Asn Leu Val Thr Leu Thr Trp Gly Asn Val Ser Ala Val
            20                  25                  30

Asp Arg Glu Arg Gly Val Phe Val Ile Lys Pro Ser Gly Val Asp Tyr
        35                  40                  45

Ser Val Met Thr Ala Asp Asp Met Val Val Val Ser Ile Glu Thr Gly
    50                  55                  60

Glu Val Val Glu Gly Thr Lys Lys Pro Ser Ser Asp Thr Pro Thr His
65                  70                  75                  80

Arg Leu Leu Tyr Gln Ala Phe Pro Ser Ile Gly Gly Ile Val His Thr
                85                  90                  95

His Ser Arg His Ala Thr Ile Trp Ala Gln Ala Gly Gln Ser Ile Pro
            100                 105                 110

Ala Thr Gly Thr Thr His Ala Asp Tyr Phe Tyr Gly Thr Ile Pro Cys
        115                 120                 125

Thr Arg Lys Met Thr Asp Ala Glu Ile Asn Gly Glu Tyr Glu Trp Glu
    130                 135                 140

Thr Gly Asn Val Ile Val Glu Thr Phe Glu Lys Gln Gly Ile Asp Ala
145                 150                 155                 160

Ala Gln Met Pro Gly Val Leu Val His Ser His Gly Pro Phe Ala Trp
                165                 170                 175

Gly Lys Asn Ala Glu Asp Ala Val His Asn Ala Ile Val Leu Glu Glu
            180                 185                 190

Val Ala Tyr Met Gly Ile Phe Cys Arg Gln Leu Ala Pro Gln Leu Pro
        195                 200                 205

Asp Met Gln Gln Thr Leu Leu Asp Lys His Tyr Leu Arg Lys His Gly
    210                 215                 220

Ala Lys Ala Tyr Tyr Gly Gln
225                 230

<210> 133
<211> 194
<212> PRT
<213> Escherichia coli

<400> 133
Met Ser Lys Lys Leu Gly Phe Ala Leu Ser Gly Leu Met Leu Ala Met
1               5                   10                  15

Val Ala Gly Thr Ala Ser Ala Asp Met Asp Gly Gly Gln Leu Asn Ile
            20                  25                  30

Ser Gly Leu Val Val Asp Asn Thr Cys Glu Thr Arg Val Asp Gly Gly
        35                  40                  45

Asn Lys Asp Gly Leu Ile Leu Leu Gln Thr Ala Thr Val Gly Glu Ile
    50                  55                  60

Asp Ala Gly Val Leu Asn Asp Thr Val Gly Ala Lys Ala Lys Pro Phe
65                  70                  75                  80

Ser Ile Thr Val Asp Cys Ser Lys Ala Asn Pro Asn Pro Gly Ser Thr
                85                  90                  95

Ala Lys Met Thr Phe Gly Ser Val Phe Phe Gly Asn Ser Lys Gly Thr
            100                 105                 110

Leu Asn Asn Asp Met Ser Ile Asn Asn Pro Ser Asp Gly Val Asn Ile
        115                 120                 125

Ala Leu His Asn Ile Asp Gly Ser Thr Ile Lys Gln Val Gln Ile Asn
    130                 135                 140

Asn Pro Gly Asp Val Tyr Thr Lys Ala Leu Asp Ala Thr Thr Lys Ser
145                 150                 155                 160

Ala Val Tyr Asp Phe Lys Ala Ser Tyr Val Arg Ala Val Ala Asp Gln
                165                 170                 175

Thr Ala Thr Ala Gly Tyr Val Lys Thr Asn Thr Ala Tyr Thr Ile Thr
            180                 185                 190

Tyr Gln

<210> 134
<211> 308
<212> PRT
<213> Escherichia coli

<400> 134
Met Leu Pro Pro Tyr Phe Leu Phe Lys Glu Met Thr Asp Thr Gln Tyr
  1               5                  10                  15

Ile Gly Arg Phe Ala Pro Ser Pro Ser Gly Glu Leu His Phe Gly Ser
             20                  25                  30

Leu Ile Ala Ala Leu Gly Ser Tyr Leu Gln Ala Arg Ala Arg Gln Gly
        35                  40                  45

Arg Trp Leu Val Arg Ile Glu Asp Ile Asp Pro Pro Arg Glu Val Pro
    50                  55                  60

Gly Ala Ala Glu Thr Ile Leu Arg Gln Leu Glu His Tyr Gly Leu His
 65                  70                  75                  80

Trp Asp Gly Asp Val Leu Trp Gln Ser Gln Arg His Asp Ala Tyr Arg
                85                  90                  95

Glu Ala Leu Ala Trp Leu His Glu Gln Gly Leu Ser Tyr Tyr Cys Thr
            100                 105                 110

Cys Thr Arg Ala Arg Ile Gln Ser Ile Gly Gly Ile Tyr Asp Gly His
        115                 120                 125

Cys Arg Val Leu His His Gly Pro Asp Asn Ala Ala Val Arg Ile Arg
    130                 135                 140

Gln Gln His Pro Val Thr Gln Phe Thr Asp Gln Leu Arg Gly Ile Ile
145                 150                 155                 160

His Ala Asp Glu Lys Leu Ala Arg Glu Asp Phe Ile Ile His Arg Arg
                165                 170                 175

Asp Gly Leu Phe Ala Tyr Asn Leu Ala Val Val Val Asp Asp His Phe
            180                 185                 190

Gln Gly Val Thr Glu Ile Val Arg Gly Ala Asp Leu Ile Glu Pro Thr
        195                 200                 205

Val Arg Gln Ile Ser Leu Tyr Gln Leu Phe Gly Trp Lys Val Pro Asp
    210                 215                 220

Tyr Ile His Leu Pro Leu Ala Leu Asn Pro Gln Gly Ala Lys Leu Ser
225                 230                 235                 240

Lys Gln Asn His Ala Pro Ala Leu Pro Lys Gly Asp Pro Arg Pro Val
                245                 250                 255

Leu Ile Ala Ala Leu Gln Phe Leu Gly Gln Gln Ala Glu Ala His Trp
            260                 265                 270

Gln Asp Phe Ser Val Glu Gln Ile Leu Gln Ser Ala Val Lys Asn Trp
        275                 280                 285

Arg Leu Thr Ala Val Pro Glu Ser Ala Ile Val Asn Ser Thr Phe Ser
    290                 295                 300

Asn Ala Ser Cys
305


<210> 135
<211> 824
<212> PRT
<213> Escherichia coli

<400> 135
Met Leu Gln Cys Gly Ala Lys Asn Val Asn Pro Leu Glu Arg Phe Val
  1               5                  10                  15

Ser Ser Leu Pro Val Ala Ala Val Leu Pro Glu Leu Leu Thr Ala Leu

            20                  25                  30

Asp Cys Ala Pro Gln Val Leu Leu Ser Ala Pro Thr Gly Ala Gly Lys
        35                  40                  45

Ser Thr Trp Leu Pro Leu Gln Leu Leu Ala His Pro Gly Ile Asn Gly
    50                  55                  60

Lys Ile Ile Leu Leu Glu Pro Arg Arg Leu Ala Ala Arg Asn Val Ala
65                  70                  75                  80

Gln Arg Leu Ala Glu Leu Leu Asn Glu Lys Pro Gly Asp Thr Val Gly
                85                  90                  95

Tyr Arg Met Arg Ala Gln Asn Cys Val Gly Pro Asn Thr Arg Leu Glu
            100                 105                 110

Val Val Thr Glu Gly Val Leu Thr Arg Met Ile Gln Arg Asp Pro Glu
        115                 120                 125

Leu Ser Gly Val Gly Leu Val Ile Leu Asp Glu Phe His Glu Arg Ser
    130                 135                 140

Leu Gln Ala Asp Leu Ala Leu Ala Leu Leu Leu Asp Val Gln Gln Gly
145                 150                 155                 160

Leu Arg Asp Asp Leu Lys Leu Leu Ile Met Ser Ala Thr Leu Asp Asn
                165                 170                 175

Asp Arg Leu Gln Gln Met Leu Pro Glu Ala Pro Val Val Ile Ser Glu
            180                 185                 190

Gly Arg Ser Phe Pro Val Glu Arg Arg Tyr Leu Pro Leu Pro Ala His
        195                 200                 205

Gln Arg Phe Asp Asp Ala Val Ala Val Ala Thr Ala Glu Met Leu Arg
    210                 215                 220

Gln Glu Ser Gly Ser Leu Leu Leu Phe Leu Pro Gly Val Gly Glu Ile
225                 230                 235                 240

Gln Arg Val Gln Glu Gln Leu Ala Ser Arg Ile Gly Ser Asp Val Leu
                245                 250                 255

Leu Cys Pro Leu Tyr Gly Ala Leu Ser Leu Asn Asp Gln Arg Lys Ala
            260                 265                 270

Ile Leu Pro Ala Pro Gln Gly Met Arg Lys Val Val Leu Ala Thr Asn
        275                 280                 285

Ile Ala Glu Thr Ser Leu Thr Ile Glu Gly Ile Arg Leu Val Val Asp
    290                 295                 300

Cys Ala Gln Glu Arg Val Ala Arg Phe Asp Pro Arg Thr Gly Leu Thr
305                 310                 315                 320

Arg Leu Ile Thr Gln Arg Val Ser Gln Ala Ser Met Thr Gln Arg Ala
                325                 330                 335

Gly Arg Ala Gly Arg Leu Glu Pro Gly Ile Ser Leu His Leu Ile Ala
            340                 345                 350

Lys Glu Gln Ala Glu Arg Ala Ala Ala Gln Ser Glu Pro Glu Ile Leu
        355                 360                 365

Gln Ser Asp Leu Ser Gly Leu Leu Met Glu Leu Leu Gln Trp Gly Cys
    370                 375                 380

Ser Asp Pro Ala Gln Met Ser Trp Leu Asp Gln Pro Pro Val Val Asn
385                 390                 395                 400

Leu Leu Ala Ala Lys Arg Leu Leu Gln Met Leu Gly Ala Leu Glu Gly
                405                 410                 415

Glu Arg Leu Ser Ala Gln Gly Gln Lys Met Ala Ala Leu Gly Asn Asp
            420                 425                 430

Pro Arg Leu Ala Ala Met Leu Val Ser Ala Lys Asn Asp Asp Glu Ala
        435                 440                 445

Ala Thr Ala Ala Lys Ile Ala Ala Ile Leu Glu Glu Pro Pro Arg Met
    450                 455                 460

Gly Asn Ser Asp Leu Gly Val Ala Phe Ser Arg Asn Gln Pro Ala Trp
465                 470                 475                 480

Gln Gln Arg Ser Gln Gln Leu Leu Lys Arg Leu Asn Val Arg Gly Gly
                485                 490                 495

Glu Ala Asp Ser Ser Leu Ile Ala Pro Leu Leu Ala Gly Ala Phe Ala
            500                 505                 510

Asp Arg Ile Ala Arg Arg Arg Gly Gln Asp Gly Arg Tyr Gln Leu Ala
        515                 520                 525

Asn Gly Met Gly Ala Met Leu Asp Ala Asn Asp Ala Leu Ser Arg His
    530                 535                 540

Glu Trp Leu Ile Ala Pro Leu Leu Leu Gln Gly Ser Ala Ser Pro Asp
545                 550                 555                 560

Ala Arg Ile Leu Leu Ala Leu Leu Val Asp Ile Asp Glu Leu Val Gln
                565                 570                 575

Arg Cys Pro Gln Leu Val Gln Gln Ser Asp Thr Val Glu Trp Asp Asp
            580                 585                 590

Ala Gln Gly Thr Leu Lys Ala Trp Arg Arg Leu Gln Ile Gly Gln Leu
        595                 600                 605

Thr Val Lys Val Gln Pro Leu Ala Lys Pro Ser Glu Asp Glu Leu His
    610                 615                 620

Gln Ala Met Leu Asn Gly Ile Arg Asp Lys Gly Leu Ser Val Leu Asn
625                 630                 635                 640

Trp Thr Ala Glu Ala Glu Gln Leu Arg Leu Arg Leu Leu Cys Ala Ala
                645                 650                 655

Lys Trp Leu Pro Glu Tyr Asp Trp Pro Ala Val Asp Asp Glu Ser Leu
            660                 665                 670

Leu Ala Ala Leu Glu Thr Trp Leu Leu Pro His Met Thr Gly Val His
        675                 680                 685

Ser Leu Arg Gly Leu Lys Ser Leu Asp Ile Tyr Gln Ala Leu Arg Gly
    690                 695                 700

Leu Leu Asp Trp Gly Met Gln Gln Arg Leu Asp Ser Glu Leu Pro Ala
705                 710                 715                 720

His Tyr Thr Val Pro Thr Gly Ser Arg Ile Ala Ile Arg Tyr His Glu
                725                 730                 735

Asp Asn Pro Pro Ala Leu Ala Val Arg Met Gln Glu Met Phe Gly Glu
            740                 745                 750

Ala Thr Asn Pro Thr Ile Ala Gln Gly Arg Val Pro Leu Val Leu Glu
        755                 760                 765

Leu Leu Ser Pro Ala Gln Arg Pro Leu Gln Ile Thr Arg Asp Leu Ser
    770                 775                 780

Asp Phe Trp Lys Gly Ala Tyr Arg Glu Val Gln Lys Glu Met Lys Gly
785                 790                 795                 800

Arg Tyr Pro Lys His Val Trp Pro Asp Asp Pro Ala Asn Thr Ala Pro
                805                 810                 815

Thr Arg Arg Thr Lys Lys Tyr Ser
            820

<210> 136
<211> 201
<212> PRT
<213> Escherichia coli

<400> 136
Met Ser Ile Lys Asn Leu Pro Ala Asp Tyr Leu Leu Ala Ala Gln Gln
1               5                   10                  15

Gly Asp Ile Asp Lys Val Lys Thr Cys Leu Ala Leu Gly Val Asp Ile
            20                  25                  30

Asn Thr Cys Asp Arg Gln Gly Lys Thr Ala Ile Thr Leu Ala Ser Leu
        35                  40                  45

Tyr Gln Gln Tyr Ala Cys Val Gln Ala Leu Ile Asp Ala Gly Ala Asp
    50                  55                  60

Ile Asn Lys Gln Asp His Thr Cys Leu Asn Pro Phe Leu Ile Ser Cys
65                  70                  75                  80

Leu Asn Asp Asp Leu Thr Leu Leu Arg Ile Ile Leu Pro Ala Lys Pro
                85                  90                  95

Asp Leu Asn Cys Val Thr Arg Phe Gly Gly Val Gly Leu Thr Pro Ala
            100                 105                 110

Cys Glu Lys Gly His Leu Ser Ile Val Lys Glu Leu Leu Ala His Thr
        115                 120                 125

Glu Ile Asn Val Asn Gln Thr Asn His Val Gly Trp Thr Pro Leu Leu
    130                 135                 140

Glu Ala Ile Val Leu Asn Asp Gly Gly Ile Lys Gln Gln Ala Ile Val
145                 150                 155                 160

Gln Leu Leu Leu Glu His Gly Ala Ser Pro His Leu Thr Asp Lys Tyr
                165                 170                 175

Gly Lys Thr Pro Leu Glu Leu Ala Arg Glu Arg Gly Phe Glu Glu Ile
            180                 185                 190

Ala Gln Leu Leu Ile Ala Ala Gly Ala
        195                 200


<210> 137
<211> 223
<212> PRT
<213> Escherichia coli

<400> 137
Met Met Gln Leu Val His Leu Phe Met Asp Glu Ile Thr Met Asp Pro
1               5                   10                  15

Leu His Ala Val Tyr Leu Thr Val Gly Leu Phe Val Ile Thr Phe Phe
            20                  25                  30

Asn Pro Gly Ala Asn Leu Phe Val Val Val Gln Thr Ser Leu Ala Ser
        35                  40                  45

Gly Arg Arg Ala Gly Val Leu Thr Gly Leu Gly Val Ala Leu Gly Asp
    50                  55                  60

Ala Phe Tyr Ser Gly Leu Gly Leu Phe Gly Leu Ala Thr Leu Ile Thr
65                  70                  75                  80

Gln Cys Glu Glu Ile Phe Ser Leu Ile Arg Ile Val Gly Gly Ala Tyr
                85                  90                  95

Leu Leu Trp Phe Ala Trp Cys Ser Met Arg Arg Gln Ser Thr Pro Gln

            100                 105                 110

Met Ser Thr Leu Gln Gln Pro Ile Ser Ala Pro Trp Tyr Val Phe Phe
        115                 120                 125

Arg Arg Gly Leu Ile Thr Asp Leu Ser Asn Pro Gln Thr Val Leu Phe
    130                 135                 140

Phe Ile Ser Ile Phe Ser Val Thr Leu Asn Ala Glu Thr Pro Thr Trp
145                 150                 155                 160

Ala Arg Leu Met Ala Trp Ala Gly Ile Val Leu Ala Ser Ile Ile Trp
                165                 170                 175

Arg Val Phe Leu Ser Gln Ala Phe Ser Leu Pro Ala Val Arg Arg Ala
            180                 185                 190

Tyr Gly Arg Met Gln Arg Val Ala Ser Arg Val Ile Gly Ala Ile Ile
        195                 200                 205

Gly Val Phe Ala Leu Arg Leu Ile Tyr Glu Gly Val Thr Gln Arg
    210                 215                 220

<210> 138
<211> 301
<212> PRT
<213> Escherichia coli

<400> 138
Met Asp Ser Ala Arg Ala Leu Ile Ala Arg Gly Trp Gly Val Ser Leu
1               5                   10                  15

Val Ser Arg Cys Leu Arg Val Ser Arg Ala Gln Leu His Val Ile Leu
            20                  25                  30

Arg Arg Thr Asp Asp Trp Met Asp Gly Arg Arg Ser Arg His Thr Asp
        35                  40                  45

Asp Thr Asp Val Leu Leu Arg Ile His His Val Ile Gly Glu Leu Pro
    50                  55                  60

Thr Tyr Gly Tyr Arg Arg Val Trp Ala Leu Leu Arg Arg Gln Ala Glu
65                  70                  75                  80

Leu Asp Gly Met Pro Ala Ile Asn Ala Lys Arg Val Tyr Arg Ile Met
                85                  90                  95

Arg Gln Asn Ala Leu Leu Leu Glu Arg Lys Pro Ala Val Pro Pro Ser
            100                 105                 110

Lys Arg Ala His Thr Gly Arg Val Ala Val Lys Glu Ser Asn Gln Arg
        115                 120                 125

Trp Cys Ser Asp Gly Phe Glu Phe Cys Cys Asp Asn Gly Glu Arg Leu
    130                 135                 140

Arg Val Thr Phe Ala Leu Asp Cys Cys Asp Arg Glu Ala Leu His Trp
145                 150                 155                 160

Ala Val Thr Thr Gly Gly Phe Asn Ser Glu Thr Val Gln Asp Val Met
                165                 170                 175

Leu Gly Ala Val Glu Arg Arg Phe Gly Asn Asp Leu Pro Ser Ser Pro
            180                 185                 190

Val Glu Trp Leu Thr Asp Asn Gly Ser Cys Tyr Arg Ala Asn Glu Thr
        195                 200                 205

Arg Gln Phe Ala Arg Met Leu Gly Leu Glu Pro Lys Asn Thr Ala Val
    210                 215                 220

Arg Ser Pro Glu Ser Asn Gly Ile Ala Glu Ser Phe Val Lys Thr Ile
225                 230                 235                 240

Lys Arg Asp Tyr Ile Ser Ile Met Pro Lys Pro Asp Gly Leu Thr Ala
                245                 250                 255

Ala Lys Asn Leu Ala Glu Ala Phe Glu His Tyr Asn Glu Trp His Pro
            260                 265                 270

His Ser Ala Leu Gly Tyr Arg Ser Pro Arg Glu Tyr Leu Arg Gln Arg
        275                 280                 285

Ala Cys Asn Gly Leu Ser Asp Asn Arg Cys Leu Glu Ile
    290                 295                 300

<210> 139
<211> 588
<212> PRT
<213> Escherichia coli

<400> 139
Met Lys Leu Pro Val Arg Glu Phe Asp Ala Val Val Ile Gly Ala Gly
  1               5                  10                  15

Gly Ala Gly Met Arg Ala Ala Leu Gln Ile Ser Gln Ser Gly Gln Thr
             20                  25                  30

Cys Ala Leu Leu Ser Lys Val Phe Pro Thr Arg Ser His Thr Val Ser
         35                  40                  45

Ala Gln Gly Gly Ile Thr Val Ala Leu Gly Asn Thr His Glu Asp Asn
     50                  55                  60

Trp Glu Trp His Met Tyr Asp Thr Val Lys Gly Ser Asp Tyr Ile Gly
 65                  70                  75                  80

Asp Gln Asp Ala Ile Glu Tyr Met Cys Lys Thr Gly Pro Glu Ala Ile
                 85                  90                  95

Leu Glu Leu Glu His Met Gly Leu Pro Phe Ser Arg Leu Asp Asp Gly
            100                 105                 110

Arg Ile Tyr Gln Arg Pro Phe Gly Gly Gln Ser Lys Asn Phe Gly Gly
        115                 120                 125

Glu Gln Ala Ala Arg Thr Ala Ala Ala Ala Asp Arg Thr Gly His Ala
    130                 135                 140

Leu Leu His Thr Leu Tyr Gln Gln Asn Leu Lys Asn His Thr Thr Ile
145                 150                 155                 160

Phe Ser Glu Trp Tyr Ala Leu Asp Leu Val Lys Asn Gln Asp Gly Ala
                165                 170                 175

Val Val Gly Cys Thr Ala Leu Cys Ile Glu Thr Gly Glu Val Val Tyr
            180                 185                 190

Phe Lys Ala Arg Ala Thr Val Leu Ala Thr Gly Gly Ala Gly Arg Ile
        195                 200                 205

Tyr Gln Ser Thr Thr Asn Ala His Ile Asn Thr Gly Asp Gly Val Gly
    210                 215                 220

Met Ala Ile Arg Ala Gly Val Pro Val Gln Asp Met Glu Met Trp Gln
225                 230                 235                 240

Phe His Pro Thr Gly Ile Ala Gly Ala Gly Val Leu Val Thr Glu Gly
                245                 250                 255

Cys Arg Gly Glu Gly Gly Tyr Leu Leu Asn Lys His Gly Glu Arg Phe
            260                 265                 270

Met Glu Arg Tyr Ala Pro Asn Ala Lys Asp Leu Ala Gly Arg Asp Val
        275                 280                 285

Val Ala Arg Ser Ile Met Ile Glu Ile Arg Glu Gly Arg Gly Cys Asp
    290                 295                 300

Gly Pro Trp Gly Pro His Ala Lys Leu Lys Leu Asp His Leu Gly Lys
305                 310                 315                 320

Glu Val Leu Glu Ser Arg Leu Pro Gly Ile Leu Glu Leu Ser Arg Thr
                325                 330                 335

Phe Ala His Val Asp Pro Val Lys Glu Pro Ile Pro Val Ile Pro Thr
            340                 345                 350

Cys His Tyr Met Met Gly Gly Ile Pro Thr Lys Val Thr Gly Gln Ala
        355                 360                 365

Leu Thr Val Asn Glu Lys Gly Glu Asp Val Val Val Pro Gly Leu Phe
    370                 375                 380

Ala Val Gly Glu Ile Ala Cys Val Ser Val His Gly Ala Asn Arg Leu
385                 390                 395                 400

Gly Gly Asn Ser Leu Leu Asp Leu Val Val Phe Gly Arg Ala Ala Gly
                405                 410                 415

Leu His Leu Gln Glu Ser Ile Ala Glu Gln Gly Ala Leu Arg Asp Ala
            420                 425                 430

Ser Glu Ser Asp Val Glu Ala Ser Leu Asp Arg Leu Asn Arg Trp Asn
        435                 440                 445

Asn Asn Arg Asn Gly Glu Asp Pro Val Ala Ile Arg Lys Ala Leu Gln
    450                 455                 460

Glu Cys Met Gln His Asn Phe Ser Val Phe Arg Glu Gly Asp Ala Met
465                 470                 475                 480

Ala Lys Gly Leu Glu Gln Leu Lys Val Ile Arg Glu Arg Leu Lys Asn
                485                 490                 495

Ala Arg Leu Asp Asp Thr Ser Ser Glu Phe Asn Thr Gln Arg Val Glu
            500                 505                 510

Cys Leu Glu Leu Asp Asn Leu Met Glu Thr Ala Tyr Ala Thr Ala Val
        515                 520                 525

Ser Ala Asn Phe Arg Thr Glu Ser Arg Gly Ala His Ser Arg Phe Asp
    530                 535                 540

Phe Pro Asp Arg Asp Asp Glu Asn Trp Leu Cys His Ser Leu Tyr Leu
545                 550                 555                 560

Pro Glu Ser Glu Ser Met Thr Arg Arg Ser Val Asn Met Glu Pro Lys
                565                 570                 575

Leu Arg Pro Ala Phe Pro Pro Lys Ile Arg Thr Tyr
            580                 585


<210> 140
<211> 933
<212> PRT
<213> Escherichia coli

<400> 140
Met Gln Asn Ser Ala Leu Lys Ala Trp Leu Asp Ser Ser Tyr Leu Ser
1               5                   10                  15

Gly Ala Asn Gln Ser Trp Ile Glu Gln Leu Tyr Glu Asp Phe Leu Thr
            20                  25                  30

Asp Pro Asp Ser Val Asp Ala Asn Trp Arg Ser Thr Phe Gln Gln Leu
        35                  40                  45

Pro Gly Thr Gly Val Lys Pro Asp Gln Phe His Ser Gln Thr Arg Glu
    50                  55                  60

Tyr Phe Arg Arg Leu Ala Lys Asp Ala Ser Arg Tyr Ser Ser Thr Ile
65                  70                  75                  80

Ser Asp Pro Asp Thr Asn Val Lys Gln Val Lys Val Leu Gln Leu Ile
                85                  90                  95

Asn Ala Tyr Arg Phe Arg Gly His Gln His Ala Asn Leu Asp Pro Leu
            100                 105                 110

Gly Leu Trp Gln Gln Asp Lys Val Ala Asp Leu Asp Pro Ser Phe His
        115                 120                 125

Asp Leu Thr Glu Ala Asp Phe Gln Glu Thr Phe Asn Val Gly Ser Phe
    130                 135                 140

Ala Ser Gly Lys Glu Thr Met Lys Leu Gly Glu Leu Leu Glu Ala Leu
145                 150                 155                 160

Lys Gln Thr Tyr Cys Gly Pro Ile Gly Ala Glu Tyr Met His Ile Thr
                165                 170                 175

Ser Thr Glu Glu Lys Arg Trp Ile Gln Gln Arg Ile Glu Ser Gly Arg
            180                 185                 190

Ala Thr Phe Asn Ser Glu Glu Lys Lys Arg Phe Leu Ser Glu Leu Thr
        195                 200                 205

Ala Ala Glu Gly Leu Glu Arg Tyr Leu Gly Ala Lys Phe Pro Gly Ala
    210                 215                 220

Lys Arg Phe Ser Leu Glu Gly Gly Asp Ala Leu Ile Pro Met Leu Lys
225                 230                 235                 240

Glu Met Ile Arg His Ala Gly Asn Ser Gly Thr Arg Glu Val Val Leu
                245                 250                 255

Gly Met Ala His Arg Gly Arg Leu Asn Val Leu Val Asn Val Leu Gly
            260                 265                 270

Lys Lys Pro Gln Asp Leu Phe Asp Glu Phe Ala Gly Lys His Lys Glu
        275                 280                 285

His Leu Gly Thr Gly Asp Val Lys Tyr His Met Gly Phe Ser Ser Asp
    290                 295                 300

Phe Gln Thr Asp Gly Gly Leu Val His Leu Ala Leu Ala Phe Asn Pro
305                 310                 315                 320

Ser His Leu Glu Ile Val Ser Pro Val Val Ile Gly Ser Val Arg Ala
                325                 330                 335

Arg Leu Asp Arg Leu Asp Glu Pro Ser Ser Asn Lys Val Leu Pro Ile
            340                 345                 350

Thr Ile His Gly Asp Ala Ala Val Thr Gly Gln Gly Val Val Gln Glu
        355                 360                 365

Thr Leu Asn Met Ser Lys Ala Arg Gly Tyr Glu Val Gly Gly Thr Val
    370                 375                 380

Arg Ile Val Ile Asn Asn Gln Val Gly Phe Thr Thr Ser Asn Pro Leu
385                 390                 395                 400

Asp Ala Arg Ser Thr Pro Tyr Cys Thr Asp Ile Gly Lys Met Val Gln
                405                 410                 415

Ala Pro Ile Phe His Val Asn Ala Asp Asp Pro Glu Ala Val Ala Phe
            420                 425                 430

Val Thr Arg Leu Ala Leu Asp Phe Arg Asn Thr Phe Lys Arg Asp Val
        435                 440                 445

Phe Ile Asp Leu Val Cys Tyr Arg Arg His Gly His Asn Glu Ala Asp
    450                 455                 460

Glu Pro Ser Ala Thr Gln Pro Leu Met Tyr Gln Lys Ile Lys Lys His
465                 470                 475                 480

Pro Thr Pro Arg Lys Ile Tyr Ala Asp Lys Leu Glu Gln Glu Lys Val
                485                 490                 495

Ala Thr Leu Glu Asp Ala Thr Glu Met Val Asn Leu Tyr Arg Asp Ala
            500                 505                 510

Leu Asp Ala Gly Asp Cys Val Val Ala Glu Trp Arg Pro Met Asn Met
        515                 520                 525

His Ser Phe Thr Trp Ser Pro Tyr Leu Asn His Glu Trp Asp Glu Glu
    530                 535                 540

Tyr Pro Asn Lys Val Glu Met Lys Arg Leu Gln Glu Leu Ala Lys Arg
545                 550                 555                 560

Ile Ser Thr Val Pro Glu Ala Val Glu Met Gln Ser Arg Val Ala Lys
                565                 570                 575

Ile Tyr Gly Asp Arg Gln Ala Met Ala Ala Gly Glu Lys Leu Phe Asp
            580                 585                 590

Trp Gly Gly Ala Glu Asn Leu Ala Tyr Ala Thr Leu Val Asp Glu Gly
        595                 600                 605

Ile Pro Val Arg Leu Ser Gly Glu Asp Ser Gly Arg Gly Thr Phe Phe
    610                 615                 620

His Arg His Ala Val Ile His Asn Gln Ser Asn Gly Ser Thr Tyr Thr
625                 630                 635                 640

Pro Leu Gln His Ile His Asn Gly Gln Gly Ala Phe Arg Val Trp Asp
                645                 650                 655

Ser Val Leu Ser Glu Glu Ala Val Leu Ala Phe Glu Tyr Gly Tyr Ala

660                 665                 670

Thr Ala Glu Pro Arg Thr Leu Thr Ile Trp Glu Ala Gln Phe Gly Asp
        675                 680                 685

Phe Ala Asn Gly Ala Gln Val Val Ile Asp Gln Phe Ile Ser Ser Gly
    690                 695                 700

Glu Gln Lys Trp Gly Arg Met Cys Gly Leu Val Met Leu Leu Pro His
705                 710                 715                 720

Gly Tyr Glu Gly Gln Gly Pro Glu His Ser Ser Ala Arg Leu Glu Arg
                725                 730                 735

Tyr Leu Gln Leu Cys Ala Glu Gln Asn Met Gln Val Cys Val Pro Ser
            740                 745                 750

Thr Pro Ala Gln Val Tyr His Met Leu Arg Arg Gln Ala Leu Arg Gly
        755                 760                 765

Met Arg Arg Pro Leu Val Val Met Ser Pro Lys Ser Leu Leu Arg His
    770                 775                 780

Pro Leu Ala Val Ser Ser Leu Glu Glu Leu Ala Asn Gly Thr Phe Leu
785                 790                 795                 800

Pro Ala Ile Gly Glu Ile Asp Glu Leu Asp Pro Lys Gly Val Lys Arg
                805                 810                 815

Val Val Met Cys Ser Gly Lys Val Tyr Tyr Asp Leu Leu Glu Gln Arg
            820                 825                 830

Arg Lys Asn Asn Gln His Asp Val Ala Ile Val Arg Ile Glu Gln Leu
        835                 840                 845

Tyr Pro Phe Pro His Lys Ala Met Gln Glu Val Leu Gln Gln Phe Ala
    850                 855                 860

His Val Lys Asp Phe Val Trp Cys Gln Glu Glu Pro Leu Asn Gln Gly
865                 870                 875                 880

Ala Trp Tyr Cys Ser Gln His His Phe Arg Glu Val Ile Pro Phe Gly
                885                 890                 895

Ala Ser Leu Arg Tyr Ala Gly Arg Pro Ala Ser Ala Ser Pro Ala Val
            900                 905                 910

Gly Tyr Met Ser Val His Gln Lys Gln Gln Gln Asp Leu Val Asn Asp
        915                 920                 925

Ala Leu Asn Val Glu
    930

<210> 141
<211> 74

<212> PRT
<213> Escherichia coli

<400> 141
Met Glu Lys Gly Thr Val Lys Trp Phe Asn Asn Ala Lys Gly Phe Gly
1               5                   10                  15

Phe Ile Cys Pro Glu Gly Gly Gly Glu Asp Ile Phe Ala His Tyr Ser
            20                  25                  30

Thr Ile Gln Met Asp Gly Tyr Arg Thr Leu Lys Ala Gly Gln Ser Val
        35                  40                  45

Gln Phe Asp Val His Gln Gly Pro Lys Gly Asn His Ala Ser Val Ile
    50                  55                  60

Val Pro Val Glu Val Glu Ala Ala Val Ala
65                  70


<210> 142
<211> 511
<212> PRT
<213> Escherichia coli

<400> 142
Met Asn Leu Leu Lys Ser Leu Ala Ala Val Ser Ser Met Thr Met Phe
1               5                   10                  15

Ser Arg Val Leu Gly Phe Ala Arg Asp Ala Ile Val Ala Arg Ile Phe
            20                  25                  30

Gly Ala Gly Met Ala Thr Asp Ala Phe Phe Val Ala Phe Lys Leu Pro
        35                  40                  45

Asn Leu Leu Arg Arg Ile Phe Ala Glu Gly Ala Phe Ser Gln Ala Phe
    50                  55                  60

Val Pro Ile Leu Ala Glu Tyr Lys Ser Lys Gln Gly Glu Asp Ala Thr
65                  70                  75                  80

Arg Val Phe Val Ser Tyr Val Ser Gly Leu Leu Thr Leu Ala Leu Ala
                85                  90                  95

Val Val Thr Val Ala Gly Met Leu Ala Ala Pro Trp Val Ile Met Val
            100                 105                 110

Thr Ala Pro Gly Phe Ala Asp Thr Ala Asp Lys Phe Ala Leu Thr Ser
        115                 120                 125

Gln Leu Leu Lys Ile Thr Phe Pro Tyr Ile Leu Leu Ile Ser Leu Ala
    130                 135                 140

Ser Leu Val Gly Ala Ile Leu Asn Thr Trp Asn Arg Phe Ser Ile Pro
145                 150                 155                 160

Ala Phe Ala Pro Thr Leu Leu Asn Ile Ser Met Ile Gly Phe Ala Leu
                165                 170                 175

Phe Ala Ala Pro Tyr Phe Asn Pro Pro Val Leu Ala Leu Ala Trp Ala
            180                 185                 190

Val Thr Val Gly Gly Val Leu Gln Leu Val Tyr Gln Leu Pro His Leu
        195                 200                 205

Lys Lys Ile Gly Met Leu Val Leu Pro Arg Ile Asn Phe His Asp Ala
    210                 215                 220

Gly Ala Met Arg Val Val Lys Gln Met Gly Pro Ala Ile Leu Gly Val
225                 230                 235                 240

Ser Val Ser Gln Ile Ser Leu Ile Ile Asn Thr Ile Phe Ala Ser Phe
                245                 250                 255

Leu Ala Ser Gly Ser Val Ser Trp Met Tyr Tyr Ala Asp Arg Leu Met
            260                 265                 270

Glu Phe Pro Ser Gly Val Leu Gly Val Ala Leu Gly Thr Ile Leu Leu
        275                 280                 285

Pro Ser Leu Ser Lys Ser Phe Ala Ser Gly Asn His Asp Glu Tyr Asn
    290                 295                 300

Arg Leu Met Asp Trp Gly Leu Arg Leu Cys Phe Leu Leu Ala Leu Pro
305                 310                 315                 320

Ser Ala Val Ala Leu Gly Ile Leu Ser Gly Pro Leu Thr Val Ser Leu
                325                 330                 335

Phe Gln Tyr Gly Lys Phe Thr Ala Phe Asp Ala Leu Met Thr Gln Arg
            340                 345                 350

Ala Leu Ile Ala Tyr Ser Val Gly Leu Ile Gly Leu Ile Val Val Lys
        355                 360                 365

Val Leu Ala Pro Gly Phe Tyr Ser Arg Gln Asp Ile Lys Thr Pro Val
    370                 375                 380

Lys Ile Ala Ile Val Thr Leu Ile Leu Thr Gln Leu Met Asn Leu Ala
385                 390                 395                 400

Phe Ile Gly Pro Leu Lys His Ala Gly Leu Ser Leu Ser Ile Gly Leu
                405                 410                 415

Ala Ala Cys Leu Asn Ala Ser Leu Leu Tyr Trp Gln Leu Arg Lys Gln
            420                 425                 430

Lys Ile Phe Thr Pro Gln Pro Gly Trp Met Ala Phe Leu Leu Arg Leu
        435                 440                 445

Val Val Ala Val Leu Val Met Ser Gly Val Leu Leu Gly Met Leu His
    450                 455                 460

Ile Met Pro Glu Trp Ser Leu Gly Thr Met Pro Trp Arg Leu Leu Arg
465                 470                 475                 480

Leu Met Ala Val Val Leu Ala Gly Ile Ala Ala Tyr Phe Ala Ala Leu
                485                 490                 495

Ala Val Leu Gly Phe Lys Val Lys Glu Phe Ala Arg Arg Thr Val
            500                 505                 510


<210> 143
<211> 365
<212> PRT
<213> Escherichia coli

<400> 143
Met Ile Lys Phe Leu Ser Ala Leu Ile Leu Leu Leu Val Thr Thr Ala
  1               5                  10                  15

Ala Gln Ala Glu Arg Ile Arg Asp Leu Thr Ser Val Gln Gly Val Arg
             20                  25                  30

Gln Asn Ser Leu Ile Gly Tyr Gly Leu Val Val Gly Leu Asp Gly Thr
         35                  40                  45

Gly Asp Gln Thr Thr Gln Thr Pro Phe Thr Thr Gln Thr Leu Asn Asn
     50                  55                  60

Met Leu Ser Gln Leu Gly Ile Thr Val Pro Thr Gly Thr Asn Met Gln
 65                  70                  75                  80

Leu Lys Asn Val Ala Ala Val Met Val Thr Ala Ser Leu Pro Pro Phe
                 85                  90                  95

Gly Arg Gln Gly Gln Thr Ile Asp Val Val Val Ser Ser Met Gly Asn
            100                 105                 110

Ala Lys Ser Leu Arg Gly Gly Thr Leu Leu Met Thr Pro Leu Lys Gly
        115                 120                 125

Val Asp Ser Gln Val Tyr Ala Leu Ala Gln Gly Asn Ile Leu Val Gly
    130                 135                 140

Gly Ala Gly Ala Ser Ala Gly Gly Ser Ser Val Gln Val Asn Gln Leu
145                 150                 155                 160

Asn Gly Gly Arg Ile Thr Asn Gly Ala Val Ile Glu Arg Glu Leu Pro
                165                 170                 175

Ser Gln Phe Gly Val Gly Asn Thr Leu Asn Leu Gln Leu Asn Asp Glu
            180                 185                 190

Asp Phe Ser Met Ala Gln Gln Ile Ala Asp Thr Ile Asn Arg Val Arg
        195                 200                 205

Gly Tyr Gly Ser Ala Thr Ala Leu Asp Ala Arg Thr Ile Gln Val Arg
    210                 215                 220

Val Pro Ser Gly Asn Ser Ser Gln Val Arg Phe Leu Ala Asp Ile Gln
225                 230                 235                 240

Asn Met Gln Val Asn Val Thr Pro Gln Asp Ala Lys Val Val Ile Asn
                245                 250                 255

Ser Arg Thr Gly Ser Val Val Met Asn Arg Glu Val Thr Leu Asp Ser
            260                 265                 270

Cys Ala Val Ala Gln Gly Asn Leu Ser Val Thr Val Asn Arg Gln Ala
        275                 280                 285

Asn Val Ser Gln Pro Asp Thr Pro Phe Gly Gly Gly Gln Thr Val Val
    290                 295                 300

Thr Pro Gln Thr Gln Ile Asp Leu Arg Gln Ser Gly Gly Ser Leu Gln
305                 310                 315                 320

Ser Val Arg Ser Ser Ala Ser Leu Asn Asn Val Val Arg Ala Leu Asn
                325                 330                 335

Ala Leu Gly Ala Thr Pro Met Asp Leu Met Ser Ile Leu Gln Ser Met
            340                 345                 350

Gln Ser Ala Gly Cys Leu Arg Ala Lys Leu Glu Ile Ile
        355                 360                 365


<210> 144
<211> 223
<212> PRT
<213> Escherichia coli

<400> 144
Met Arg Val Leu Val Val Glu Asp Asn Ala Leu Leu Arg His His Leu
1               5                   10                  15

Lys Val Gln Ile Gln Asp Ala Gly His Gln Val Asp Asp Ala Glu Asp
            20                  25                  30

Ala Lys Glu Ala Asp Tyr Tyr Leu Asn Glu His Ile Pro Asp Ile Ala
        35                  40                  45

Ile Val Asp Leu Gly Leu Pro Asp Glu Asp Gly Leu Ser Leu Ile Arg
    50                  55                  60

Arg Trp Arg Ser Asn Asp Val Ser Leu Pro Ile Leu Val Leu Thr Ala
65                  70                  75                  80

Arg Glu Ser Trp Gln Asp Lys Val Glu Val Leu Ser Ala Gly Ala Asp
                85                  90                  95

Asp Tyr Val Thr Lys Pro Phe His Ile Glu Glu Val Met Ala Arg Met

            100                 105                 110

Gln Ala Leu Met Arg Arg Asn Ser Gly Leu Ala Ser Gln Val Ile Ser
        115                 120                 125

Leu Pro Pro Phe Gln Val Asp Leu Ser Arg Arg Glu Leu Ser Ile Asn
    130                 135                 140

Asp Glu Val Ile Lys Leu Thr Ala Phe Glu Tyr Thr Ile Met Glu Thr
145                 150                 155                 160

Leu Ile Arg Asn Asn Gly Lys Val Val Ser Lys Asp Ser Leu Met Leu
                165                 170                 175

Gln Leu Tyr Pro Asp Ala Glu Leu Arg Glu Ser His Thr Ile Asp Val
            180                 185                 190

Leu Met Gly Arg Leu Arg Lys Lys Ile Gln Ala Gln Tyr Pro Gln Glu
        195                 200                 205

Val Ile Thr Thr Val Arg Gly Gln Gly Tyr Leu Phe Glu Leu Arg
    210                 215                 220

<210> 145
<211> 216
<212> PRT
<213> Escherichia coli

<400> 145
Met Ser Asn Gln Glu Pro Ala Thr Ile Leu Leu Ile Asp Asp His Pro
1               5                   10                  15

Met Leu Arg Thr Gly Val Lys Gln Leu Ile Ser Met Ala Pro Asp Ile
            20                  25                  30

Thr Val Val Gly Glu Ala Ser Asn Gly Glu Gln Gly Ile Glu Leu Ala
        35                  40                  45

Glu Ser Leu Asp Pro Asp Leu Ile Leu Leu Asp Leu Asn Met Pro Gly
    50                  55                  60

Met Asn Gly Leu Glu Thr Leu Asp Lys Leu Arg Glu Lys Ser Leu Ser
65                  70                  75                  80

Gly Arg Ile Val Val Phe Ser Val Ser Asn His Glu Glu Asp Val Val
                85                  90                  95

Thr Ala Leu Lys Arg Gly Ala Asp Gly Tyr Leu Leu Lys Asp Met Glu
            100                 105                 110

Pro Glu Asp Leu Leu Lys Ala Leu His Gln Ala Ala Ala Gly Glu Met
        115                 120                 125

Val Leu Ser Glu Ala Leu Thr Pro Val Leu Ala Ala Ser Leu Arg Ala
    130                 135                 140

Asn Arg Ala Thr Thr Glu Arg Asp Val Asn Gln Leu Thr Pro Arg Glu
145                 150                 155                 160

Arg Asp Ile Leu Lys Leu Ile Ala Gln Gly Leu Pro Asn Lys Met Ile
                165                 170                 175

Ala Arg Arg Leu Asp Ile Thr Glu Ser Thr Val Lys Val His Val Lys
            180                 185                 190

His Met Leu Lys Lys Met Lys Leu Lys Ser Arg Val Glu Ala Ala Val
        195                 200                 205

Trp Val His Gln Glu Arg Ile Phe
    210                 215


<210> 146
<211> 866
<212> PRT
<213> Escherichia coli

<400> 146
Met Ser Thr Lys Pro Leu Phe Leu Leu Arg Lys Ala Lys Lys Ser Ser
  1               5                  10                  15

Gly Glu Pro Asp Val Val Leu Trp Ala Ser Asn Asp Phe Glu Ser Thr
             20                  25                  30

Cys Ala Thr Leu Asp Tyr Leu Ile Val Lys Ser Gly Lys Lys Leu Ser
         35                  40                  45

Ser Tyr Phe Lys Ala Val Ala Thr Asn Phe Pro Val Val Asn Asp Leu
     50                  55                  60

Pro Ala Glu Gly Glu Ile Asp Phe Thr Trp Ser Glu Arg Tyr Gln Leu
 65                  70                  75                  80

Ser Lys Asp Ser Met Thr Trp Glu Leu Lys Pro Gly Ala Ala Pro Asp
                 85                  90                  95

Asn Ala His Tyr Gln Gly Asn Thr Asn Val Asn Gly Glu Asp Met Thr
            100                 105                 110

Glu Ile Glu Glu Asn Met Leu Leu Pro Ile Ser Gly Gln Glu Leu Pro
        115                 120                 125

Ile Arg Trp Leu Ala Gln His Gly Ser Glu Lys Pro Val Thr His Val
    130                 135                 140

Ser Arg Asp Gly Leu Gln Ala Leu His Ile Ala Arg Ala Glu Glu Leu
145                 150                 155                 160

Pro Ala Val Thr Ala Leu Ala Val Ser His Lys Thr Ser Leu Leu Asp
                165                 170                 175

Pro Leu Glu Ile Arg Glu Leu His Lys Leu Val Arg Asp Thr Asp Lys
            180                 185                 190

Val Phe Pro Asn Pro Gly Asn Ser Asn Leu Gly Leu Ile Thr Ala Phe
        195                 200                 205

Phe Glu Ala Tyr Leu Asn Ala Asp Tyr Thr Asp Arg Gly Leu Leu Thr
    210                 215                 220

Lys Glu Trp Met Lys Gly Asn Arg Val Ser His Ile Thr Arg Thr Ala
225                 230                 235                 240

Ser Gly Ala Asn Ala Gly Gly Gly Asn Leu Thr Asp Arg Gly Glu Gly
                245                 250                 255

Phe Val His Asp Leu Thr Ser Leu Ala Arg Asp Val Ala Thr Gly Val
            260                 265                 270

Leu Ala Arg Ser Met Asp Leu Asp Ile Tyr Asn Leu His Pro Ala His
        275                 280                 285

Ala Lys Arg Ile Glu Glu Ile Ile Ala Glu Asn Lys Pro Pro Phe Ser
    290                 295                 300

Val Phe Arg Asp Lys Phe Ile Thr Met Pro Gly Gly Leu Asp Tyr Ser
305                 310                 315                 320

Arg Ala Ile Val Val Ala Ser Val Lys Glu Ala Pro Ile Gly Ile Glu
                325                 330                 335

Val Ile Pro Ala His Val Thr Glu Tyr Leu Asn Lys Val Leu Thr Glu
            340                 345                 350

Thr Asp His Ala Asn Pro Asp Pro Glu Ile Val Asp Ile Ala Cys Gly
        355                 360                 365

Arg Ser Ser Ala Pro Met Pro Gln Arg Val Thr Glu Glu Gly Lys Gln
    370                 375                 380

Asp Asp Glu Glu Lys Pro Gln Pro Ser Gly Thr Thr Ala Val Glu Gln
385                 390                 395                 400

Gly Glu Ala Glu Thr Met Glu Pro Asp Ala Thr Glu His His Gln Asp
                405                 410                 415

Thr Gln Pro Leu Asp Ala Gln Ser Gln Val Asn Ser Val Asp Ala Lys
            420                 425                 430

Tyr Gln Glu Leu Arg Ala Glu Leu His Glu Ala Arg Lys Asn Ile Pro
        435                 440                 445

Ser Lys Asn Pro Val Asp Asp Asp Lys Leu Leu Ala Ala Ser Arg Gly
    450                 455                 460

Glu Phe Val Asp Gly Ile Ser Asp Pro Asn Asp Pro Lys Trp Val Lys
465                 470                 475                 480

Gly Ile Gln Thr Arg Asp Cys Val Tyr Gln Asn Gln Pro Glu Thr Glu
                485                 490                 495

Lys Thr Ser Pro Asp Met Asn Gln Pro Glu Pro Val Val Gln Gln Glu
            500                 505                 510

Pro Glu Ile Ala Cys Asn Ala Cys Gly Gln Thr Gly Gly Asp Asn Cys
        515                 520                 525

Pro Asp Cys Gly Ala Val Met Gly Asp Ala Thr Tyr Gln Glu Thr Phe
    530                 535                 540

Asp Glu Glu Ser Gln Val Glu Ala Lys Glu Asn Asp Pro Glu Glu Met
545                 550                 555                 560

Glu Gly Ala Glu His Pro His Asn Glu Asn Ala Gly Ser Asp Pro His
                565                 570                 575

Arg Asp Cys Ser Asp Glu Thr Gly Glu Val Ala Asp Pro Val Ile Val
            580                 585                 590

Glu Asp Ile Glu Pro Gly Ile Tyr Tyr Gly Ile Ser Asn Glu Asn Tyr
        595                 600                 605

His Ala Gly Pro Gly Ile Ser Lys Ser Gln Leu Asp Asp Ile Ala Asp
    610                 615                 620

Thr Pro Ala Leu Tyr Leu Trp Arg Lys Asn Ala Pro Val Asp Thr Thr
625                 630                 635                 640

Lys Thr Lys Thr Leu Asp Leu Gly Thr Ala Phe His Cys Arg Val Leu
                645                 650                 655

Glu Pro Glu Glu Phe Ser Asn Arg Phe Ile Val Ala Pro Glu Phe Asn
            660                 665                 670

Arg Arg Thr Asn Ala Gly Lys Glu Glu Glu Lys Ala Phe Leu Met Glu
        675                 680                 685

Cys Ala Ser Thr Gly Lys Thr Val Ile Thr Ala Glu Glu Gly Arg Lys
    690                 695                 700

Ile Glu Leu Met Tyr Gln Ser Val Met Ala Leu Pro Leu Gly Gln Trp
705                 710                 715                 720

Leu Val Glu Ser Ala Gly His Ala Glu Ser Ser Ile Tyr Trp Glu Asp
                725                 730                 735

Pro Glu Thr Gly Ile Leu Cys Arg Cys Arg Pro Asp Lys Ile Ile Pro
            740                 745                 750

Glu Phe His Trp Ile Met Asp Val Lys Thr Thr Ala Asp Ile Gln Arg
        755                 760                 765

Phe Lys Thr Ala Tyr Tyr Asp Tyr Arg Tyr His Val Gln Asp Ala Phe

770                 775                 780

Tyr Ser Asp Gly Tyr Glu Ala Gln Phe Gly Val Gln Pro Thr Phe Val
785                 790                 795                 800

Phe Leu Val Ala Ser Thr Thr Ile Glu Cys Gly Arg Tyr Pro Val Glu
                805                 810                 815

Ile Phe Met Met Gly Glu Glu Ala Lys Leu Ala Gly Gln Gln Glu Tyr
            820                 825                 830

His Arg Asn Leu Arg Thr Leu Ser Asp Cys Leu Asn Thr Asp Glu Trp
        835                 840                 845

Pro Ala Ile Lys Thr Leu Ser Leu Pro Arg Trp Ala Lys Glu Tyr Ala
    850                 855                 860

Asn Asp
865

<210> 147
<211> 356
<212> PRT
<213> Escherichia coli

<400> 147
Met Thr Thr Phe His Ser Leu Thr Val Ala Lys Val Glu Ser Glu Thr
1               5                   10                  15

Arg Asp Ala Val Thr Ile Thr Phe Ala Val Pro Gln Pro Leu Gln Glu
            20                  25                  30

Ala Tyr Arg Phe Arg Pro Gly Gln His Leu Thr Leu Lys Ala Ser Phe
        35                  40                  45

Asp Gly Glu Glu Leu Arg Arg Cys Tyr Ser Ile Cys Arg Ser Tyr Leu
    50                  55                  60

Pro Gly Glu Ile Ser Val Ala Val Lys Ala Ile Glu Gly Gly Arg Phe
65                  70                  75                  80

Ser Arg Tyr Ala Arg Glu His Ile Arg Gln Gly Met Thr Leu Glu Val
                85                  90                  95

Met Val Pro Gln Gly His Phe Gly Tyr Gln Pro Gln Ala Glu Arg Gln
            100                 105                 110

Gly Arg Tyr Leu Ala Ile Ala Ala Gly Ser Gly Ile Thr Pro Met Leu
        115                 120                 125

Ala Ile Ile Ala Thr Thr Leu Gln Thr Glu Pro Glu Ser Gln Phe Thr
    130                 135                 140

Leu Ile Tyr Gly Asn Arg Thr Ser Gln Ser Met Met Phe Arg Gln Ala
145                 150                 155                 160

Leu Ala Asp Leu Lys Asp Lys Tyr Pro Gln Arg Leu Gln Leu Leu Cys
                165                 170                 175

Ile Phe Ser Gln Glu Thr Leu Asp Ser Asp Leu Leu His Gly Arg Ile
            180                 185                 190

Asp Gly Glu Lys Leu Gln Ser Leu Gly Ala Ser Leu Ile Asn Phe Arg
        195                 200                 205

Leu Tyr Asp Glu Ala Phe Ile Cys Gly Pro Ala Ala Met Met Asp Asp
    210                 215                 220

Ala Glu Thr Ala Leu Lys Ala Leu Gly Met Pro Asp Lys Thr Ile His
225                 230                 235                 240

Leu Glu Arg Phe Asn Thr Pro Gly Thr Arg Val Lys Arg Ser Val Asn
                245                 250                 255

Val Gln Ser Asp Gly Gln Lys Val Thr Val Arg Gln Asp Gly Arg Asp
            260                 265                 270

Arg Glu Ile Val Leu Asn Ala Asp Asp Glu Ser Ile Leu Asp Ala Ala
        275                 280                 285

Leu Arg Gln Gly Ala Asp Leu Pro Tyr Ala Cys Lys Gly Gly Val Cys
    290                 295                 300

Ala Thr Cys Lys Cys Lys Val Leu Arg Gly Lys Val Ala Met Glu Thr
305                 310                 315                 320

Asn Tyr Ser Leu Glu Pro Asp Glu Leu Ala Ala Gly Tyr Val Leu Ser
                325                 330                 335

Cys Gln Ala Leu Pro Leu Thr Ser Asp Val Val Val Asp Phe Asp Ala
            340                 345                 350

Lys Gly Met Ala
        355


<210> 148
<211> 71
<212> PRT
<213> Escherichia coli

<400> 148
Met Ser Asn Lys Met Thr Gly Leu Val Lys Trp Phe Asn Ala Asp Lys
1               5                   10                  15

Gly Phe Gly Phe Ile Ser Pro Val Asp Gly Ser Lys Asp Val Phe Val
            20                  25                  30

His Phe Ser Ala Ile Gln Asn Asp Asn Tyr Arg Thr Leu Phe Glu Gly
        35                  40                  45

Gln Lys Val Thr Phe Ser Ile Glu Ser Gly Ala Lys Gly Pro Ala Ala
50                  55                  60

Ala Asn Val Ile Ile Thr Asp
65                  70

<210> 149
<211> 365
<212> PRT
<213> Escherichia coli

<400> 149
Met Ser Ser Glu Lys Leu Tyr Ser Pro Leu Lys Val Gly Ala Ile Thr
1               5                   10                  15

Ala Ala Asn Arg Ile Phe Met Ala Pro Leu Thr Arg Leu Arg Ser Ile
            20                  25                  30

Glu Pro Gly Asp Ile Pro Thr Pro Leu Met Ala Glu Tyr Tyr Arg Gln
        35                  40                  45

Arg Ala Ser Ala Gly Leu Ile Ile Ser Glu Ala Thr Gln Ile Ser Ala
    50                  55                  60

Gln Ala Lys Gly Tyr Ala Gly Ala Pro Gly Ile His Ser Pro Glu Gln
65                  70                  75                  80

Ile Ala Ala Trp Lys Lys Ile Thr Ala Gly Val His Ala Glu Asn Gly
                85                  90                  95

His Met Ala Val Gln Leu Trp His Thr Gly Arg Ile Ser His Ala Ser
            100                 105                 110

Leu Gln Pro Gly Gly Gln Ala Pro Val Ala Pro Ser Ala Leu Ser Ala
        115                 120                 125

Gly Thr Arg Thr Ser Leu Arg Asp Glu Asn Gly Gln Ala Ile Arg Val
    130                 135                 140

Glu Thr Ser Met Pro Arg Ala Leu Glu Leu Glu Glu Ile Pro Gly Ile
145                 150                 155                 160

Val Asn Asp Phe Arg Gln Ala Ile Ala Asn Ala Arg Glu Ala Gly Phe
                165                 170                 175

Asp Leu Val Glu Leu His Ser Ala His Gly Tyr Leu Leu His Gln Phe
            180                 185                 190

Leu Ser Pro Ser Ser Asn His Arg Thr Asp Gln Tyr Gly Gly Ser Val
        195                 200                 205

Glu Asn Arg Ala Arg Leu Val Leu Glu Val Val Asp Ala Gly Ile Glu
    210                 215                 220

Glu Trp Gly Ala Asp Arg Ile Gly Ile Arg Val Ser Pro Ile Gly Thr

225                 230                 235                 240

Phe Gln Asn Thr Asp Asn Gly Pro Asn Glu Glu Ala Asp Ala Leu Tyr
                245                 250                 255

Leu Ile Glu Gln Leu Gly Lys Arg Gly Ile Ala Tyr Leu His Met Ser
            260                 265                 270

Glu Pro Asp Trp Ala Gly Gly Glu Pro Tyr Thr Asp Ala Phe Arg Glu
        275                 280                 285

Lys Val Arg Ala Arg Phe His Gly Pro Ile Ile Gly Ala Gly Ala Tyr
    290                 295                 300

Thr Val Glu Lys Ala Glu Thr Leu Ile Gly Lys Gly Leu Ile Asp Ala
305                 310                 315                 320

Val Ala Phe Gly Arg Asp Trp Ile Ala Asn Pro Asp Leu Val Ala Arg
                325                 330                 335

Leu Gln Arg Lys Ala Glu Leu Asn Pro Gln Arg Ala Glu Ser Phe Tyr
            340                 345                 350

Gly Gly Gly Ala Glu Gly Tyr Thr Asp Tyr Pro Thr Leu
        355                 360                 365

<210> 150
<211> 65
<212> PRT
<213> Escherichia coli

<400> 150

<210> 151
<211> 331
<212> PRT
<213> Escherichia coli

<400> 151
Met Thr Ile Lys Val Gly Ile Asn Gly Phe Gly Arg Ile Gly Arg Ile

1               5                   10                  15

Val Phe Arg Ala Ala Gln Lys Arg Ser Asp Ile Glu Ile Val Ala Ile
            20                  25                  30

Asn Asp Leu Leu Asp Ala Asp Tyr Met Ala Tyr Met Leu Lys Tyr Asp
        35                  40                  45

Ser Thr His Gly Arg Phe Asp Gly Thr Val Glu Val Lys Asp Gly His
    50                  55                  60

Leu Ile Val Asn Gly Lys Lys Ile Arg Val Thr Ala Glu Arg Asp Pro
65                  70                  75                  80

Ala Asn Leu Lys Trp Asp Glu Val Gly Val Asp Val Val Ala Glu Ala
                85                  90                  95

Thr Gly Leu Phe Leu Thr Asp Glu Thr Ala Arg Lys His Ile Thr Ala
            100                 105                 110

Gly Ala Lys Lys Val Val Met Thr Gly Pro Ser Lys Asp Asn Thr Pro
        115                 120                 125

Met Phe Val Lys Gly Ala Asn Phe Asp Lys Tyr Ala Gly Gln Asp Ile
    130                 135                 140

Val Ser Asn Ala Ser Cys Thr Thr Asn Cys Leu Ala Pro Leu Ala Lys
145                 150                 155                 160

Val Ile Asn Asp Asn Phe Gly Ile Ile Glu Gly Leu Met Thr Thr Val
                165                 170                 175

His Ala Thr Thr Ala Thr Gln Lys Thr Val Asp Gly Pro Ser His Lys
            180                 185                 190

Asp Trp Arg Gly Gly Arg Gly Ala Ser Gln Asn Ile Ile Pro Ser Ser
        195                 200                 205

Thr Gly Ala Ala Lys Ala Val Gly Lys Val Leu Pro Glu Leu Asn Gly
    210                 215                 220

Lys Leu Thr Gly Met Ala Phe Arg Val Pro Thr Pro Asn Val Ser Val
225                 230                 235                 240

Val Asp Leu Thr Val Arg Leu Glu Lys Ala Ala Thr Tyr Glu Gln Ile
                245                 250                 255

Lys Ala Ala Val Lys Ala Ala Ala Glu Gly Glu Met Lys Gly Val Leu
            260                 265                 270

Gly Tyr Thr Glu Asp Asp Val Val Ser Thr Asp Phe Asn Gly Glu Val
        275                 280                 285

Cys Thr Ser Val Phe Asp Ala Lys Ala Gly Ile Ala Leu Asn Asp Asn
    290                 295                 300

Phe Val Lys Leu Val Ser Trp Tyr Asp Asn Glu Thr Gly Tyr Ser Asn
305                 310                 315                 320

Lys Val Leu Asp Leu Ile Ala His Ile Ser Lys
                325                 330

<210> 152
<211> 416
<212> PRT
<213> Escherichia coli

<400> 152
Met Asp Ile Met Arg Ser Val Val Gly Met Val Val Leu Leu Ala Ile
  1               5                  10                  15

Ala Phe Leu Leu Ser Val Asn Lys Lys Ser Ile Ser Leu Arg Thr Val
             20                  25                  30

Gly Ala Ala Leu Leu Leu Gln Ile Ala Ile Gly Gly Ile Met Leu Tyr
         35                  40                  45

Phe Pro Pro Gly Lys Trp Ala Val Glu Gln Ala Ala Leu Gly Val His
     50                  55                  60

Lys Val Met Ser Tyr Ser Asp Ala Gly Ser Ala Phe Ile Phe Gly Ser
 65                  70                  75                  80

Leu Val Gly Pro Lys Met Asp Val Leu Phe Asp Gly Ala Gly Phe Ile
                 85                  90                  95

Phe Ala Phe Arg Val Leu Pro Ala Ile Ile Phe Val Thr Ala Leu Ile
            100                 105                 110

Ser Leu Leu Tyr Tyr Ile Gly Val Met Gly Leu Leu Ile Arg Ile Leu
        115                 120                 125

Gly Ser Ile Phe Gln Lys Ala Leu Asn Ile Ser Lys Ile Glu Ser Phe
    130                 135                 140

Val Ala Val Thr Thr Ile Phe Leu Gly Gln Asn Glu Ile Pro Ala Ile
145                 150                 155                 160

Val Lys Pro Phe Ile Asp Arg Met Asn Arg Asn Glu Leu Phe Thr Ala
                165                 170                 175

Ile Cys Ser Gly Met Ala Ser Ile Ala Gly Ser Met Met Ile Gly Tyr
            180                 185                 190

Ala Gly Met Gly Val Pro Ile Asp Tyr Leu Leu Ala Ala Ser Leu Met
        195                 200                 205

Ala Ile Pro Gly Gly Ile Leu Phe Ala Arg Ile Leu Ser Pro Ala Thr
    210                 215                 220

Glu Pro Ser Gln Val Thr Phe Glu Asn Leu Ser Phe Ser Glu Thr Pro

225                 230                 235                 240

Pro Lys Ser Phe Ile Glu Ala Ala Ala Ser Gly Ala Met Thr Gly Leu
                245                 250                 255

Lys Ile Ala Ala Gly Val Ala Thr Val Val Met Ala Phe Val Ala Ile
            260                 265                 270

Ile Ala Leu Ile Asn Gly Ile Ile Gly Gly Ile Gly Gly Trp Phe Gly
        275                 280                 285

Phe Ala Asn Ala Ser Leu Glu Ser Ile Phe Gly Tyr Val Leu Ala Pro
    290                 295                 300

Leu Ala Trp Ile Met Gly Val Asp Trp Ser Asp Ala Asn Leu Ala Gly
305                 310                 315                 320

Ser Leu Ile Gly Gln Lys Leu Ala Ile Asn Glu Phe Val Ala Tyr Leu
                325                 330                 335

Ser Phe Ser Pro Tyr Leu Gln Thr Gly Gly Thr Leu Glu Val Lys Thr
            340                 345                 350

Ile Ala Ile Ile Ser Phe Ala Leu Cys Gly Phe Ala Asn Phe Gly Ser
        355                 360                 365

Ile Gly Val Val Val Gly Ala Phe Ser Ala Ile Ser Pro Lys Arg Ala
    370                 375                 380

Pro Glu Ile Ala Gln Leu Gly Leu Arg Ala Leu Ala Ala Ala Thr Leu
385                 390                 395                 400

Ser Asn Leu Met Ser Ala Thr Ile Ala Gly Phe Phe Ile Gly Leu Ala
                405                 410                 415

<210> 153
<211> 350
<212> PRT
<213> Escherichia coli

<400> 153
Met Arg Phe Leu Leu Gly Val Leu Met Leu Met Ile Ser Gly Ser Ala
1               5                   10                  15

Leu Ala Thr Ile Asp Val Leu Gln Phe Lys Asp Glu Ala Gln Glu Gln
            20                  25                  30

Gln Phe Arg Gln Leu Thr Glu Glu Leu Arg Cys Pro Lys Cys Gln Asn
        35                  40                  45

Asn Ser Ile Ala Asp Ser Asn Ser Met Ile Ala Thr Asp Leu Arg Gln
    50                  55                  60

Lys Val Tyr Glu Leu Met Gln Glu Gly Lys Ser Lys Lys Glu Ile Val
65                  70                  75                  80

Asp Tyr Met Val Ala Arg Tyr Gly Asn Phe Val Thr Tyr Asp Pro Pro
                85                  90                  95

Leu Thr Pro Leu Thr Val Leu Leu Trp Val Leu Pro Val Val Ala Ile
            100                 105                 110

Gly Ile Gly Gly Trp Val Ile Tyr Ala Arg Ser Arg Arg Arg Val Arg
        115                 120                 125

Val Val Pro Glu Ala Phe Pro Glu Gln Ser Val Pro Glu Gly Lys Arg
    130                 135                 140

Ala Gly Tyr Val Val Tyr Leu Pro Gly Ile Val Val Ala Leu Ile Val
145                 150                 155                 160

Ala Gly Val Ser Tyr Tyr Gln Thr Gly Asn Tyr Gln Gln Val Lys Ile
                165                 170                 175

Trp Gln Gln Ala Thr Ala Gln Ala Pro Ala Leu Leu Asp Arg Ala Leu
            180                 185                 190

Asp Pro Lys Ala Asp Pro Leu Asn Glu Glu Glu Met Ser Arg Leu Ala
        195                 200                 205

Leu Gly Met Arg Thr Gln Leu Gln Lys Asn Pro Gly Asp Ile Glu Gly
    210                 215                 220

Trp Ile Met Leu Gly Arg Val Gly Met Ala Leu Gly Asn Ala Ser Ile
225                 230                 235                 240

Ala Thr Asp Ala Tyr Ala Thr Ala Tyr Arg Leu Asp Pro Lys Asn Ser
                245                 250                 255

Asp Ala Ala Leu Gly Tyr Ala Glu Ala Leu Thr Arg Ser Ser Asp Pro
            260                 265                 270

Asn Asp Asn Arg Leu Gly Gly Glu Leu Leu Arg Gln Leu Val Arg Thr
        275                 280                 285

Asp His Ser Asn Ile Arg Val Leu Ser Met Tyr Ala Phe Asn Ala Phe
    290                 295                 300

Glu Gln Gln Arg Phe Gly Glu Ala Val Ala Ala Trp Glu Met Met Leu
305                 310                 315                 320

Lys Leu Leu Pro Ala Asn Asp Thr Arg Arg Ala Val Ile Glu Arg Ser
                325                 330                 335

Ile Ala Gln Ala Met Gln His Leu Ser Pro Gln Glu Ser Lys
            340                 345                 350

<210> 154
<211> 159
<212> PRT
<213> Escherichia coli

<400> 154
Met Asn Ile Arg Arg Lys Asn Arg Leu Trp Ile Ala Cys Ala Val Leu
1               5                   10                  15

Ala Gly Leu Ala Leu Thr Ile Gly Leu Val Leu Tyr Ala Leu Arg Ser
            20                  25                  30

Asn Ile Asp Leu Phe Tyr Thr Pro Gly Glu Ile Leu Tyr Gly Lys Arg
        35                  40                  45

Glu Thr Gln Gln Met Pro Glu Val Gly Gln Arg Leu Arg Val Gly Gly
    50                  55                  60

Met Val Met Pro Gly Ser Val Gln Arg Asp Pro Asn Ser Leu Lys Val
65                  70                  75                  80

Thr Phe Thr Ile Tyr Asp Ala Glu Gly Ser Val Asp Val Ser Tyr Glu
                85                  90                  95

Gly Ile Leu Pro Asp Leu Phe Arg Glu Gly Gln Gly Val Val Val Gln
            100                 105                 110

Gly Glu Leu Glu Lys Gly Asn His Ile Leu Ala Lys Glu Val Leu Ala
        115                 120                 125

Lys His Asp Glu Asn Tyr Thr Pro Pro Glu Val Glu Lys Ala Met Glu
    130                 135                 140

Ala Asn His Arg Arg Pro Ala Ser Val Tyr Lys Asp Pro Ala Ser
145                 150                 155


<210> 155
<211> 875
<212> PRT
<213> Escherichia coli

<400> 155
Met Ser Asp Leu Ala Arg Glu Ile Thr Pro Val Asn Ile Glu Glu Glu
1               5                   10                  15

Leu Lys Ser Ser Tyr Leu Asp Tyr Ala Met Ser Val Ile Val Gly Arg
            20                  25                  30

Ala Leu Pro Asp Val Arg Asp Gly Leu Lys Pro Val His Arg Arg Val
        35                  40                  45

Leu Tyr Ala Met Asn Val Leu Gly Asn Asp Trp Asn Lys Ala Tyr Lys
    50                  55                  60

Lys Ser Ala Arg Val Val Gly Asp Val Ile Gly Lys Tyr His Pro His

65                  70                  75                  80

Gly Asp Ser Ala Val Tyr Asp Thr Ile Val Arg Met Ala Gln Pro Phe
                85                  90                  95

Ser Leu Arg Tyr Met Leu Val Asp Gly Gln Gly Asn Phe Gly Ser Ile
            100                 105                 110

Asp Gly Asp Ser Ala Ala Ala Met Arg Tyr Thr Glu Ile Arg Leu Ala
        115                 120                 125

Lys Ile Ala His Glu Leu Met Ala Asp Leu Glu Lys Glu Thr Val Asp
    130                 135                 140

Phe Val Asp Asn Tyr Asp Gly Thr Glu Lys Ile Pro Asp Val Met Pro
145                 150                 155                 160

Thr Lys Ile Pro Asn Leu Leu Val Asn Gly Ser Ser Gly Ile Ala Val
                165                 170                 175

Gly Met Ala Thr Asn Ile Pro Pro His Asn Leu Thr Glu Val Ile Asn
            180                 185                 190

Gly Cys Leu Ala Tyr Ile Asp Asp Glu Asp Ile Ser Ile Glu Gly Leu
        195                 200                 205

Met Glu His Ile Pro Gly Pro Asp Phe Pro Thr Ala Ala Ile Ile Asn
    210                 215                 220

Gly Arg Arg Gly Ile Glu Glu Ala Tyr Arg Thr Gly Arg Gly Lys Val
225                 230                 235                 240

Tyr Ile Arg Ala Arg Ala Glu Val Glu Val Asp Ala Lys Thr Gly Arg
                245                 250                 255

Glu Thr Ile Ile Val His Glu Ile Pro Tyr Gln Val Asn Lys Ala Arg
            260                 265                 270

Leu Ile Glu Lys Ile Ala Glu Leu Val Lys Glu Lys Arg Val Glu Gly
        275                 280                 285

Ile Ser Ala Leu Arg Asp Glu Ser Asp Lys Asp Gly Met Arg Ile Val
    290                 295                 300

Ile Glu Val Lys Arg Asp Ala Val Gly Glu Val Val Leu Asn Asn Leu
305                 310                 315                 320

Tyr Ser Gln Thr Gln Leu Gln Val Ser Phe Gly Ile Asn Met Val Ala
                325                 330                 335

Leu His His Gly Gln Pro Lys Ile Met Asn Leu Lys Asp Ile Ile Ala
            340                 345                 350

Ala Phe Val Arg His Arg Arg Glu Val Val Thr Arg Arg Thr Ile Phe
        355                 360                 365

Glu Leu Arg Lys Ala Arg Asp Arg Ala His Ile Leu Glu Ala Leu Ala
    370                 375                 380

Val Ala Leu Ala Asn Ile Asp Pro Ile Ile Glu Leu Ile Arg His Ala
385                 390                 395                 400

Pro Thr Pro Ala Glu Ala Lys Thr Ala Leu Val Ala Asn Pro Trp Gln
                405                 410                 415

Leu Gly Asn Val Ala Ala Met Leu Glu Arg Ala Gly Asp Asp Ala Ala
            420                 425                 430

Arg Pro Glu Trp Leu Glu Pro Glu Phe Gly Val Arg Asp Gly Leu Tyr
        435                 440                 445

Tyr Leu Thr Glu Gln Gln Ala Gln Ala Ile Leu Asp Leu Arg Leu Gln
    450                 455                 460

Lys Leu Thr Gly Leu Glu His Glu Lys Leu Leu Asp Glu Tyr Lys Glu
465                 470                 475                 480

Leu Leu Asp Gln Ile Ala Glu Leu Leu Arg Ile Leu Gly Ser Ala Asp
                485                 490                 495

Arg Leu Met Glu Val Ile Arg Glu Glu Leu Glu Leu Val Arg Glu Gln
            500                 505                 510

Phe Gly Asp Lys Arg Arg Thr Glu Ile Thr Ala Asn Ser Ala Asp Ile
        515                 520                 525

Asn Leu Glu Asp Leu Ile Thr Gln Glu Asp Val Val Val Thr Leu Ser
    530                 535                 540

His Gln Gly Tyr Val Lys Tyr Gln Pro Leu Ser Glu Tyr Glu Ala Gln
545                 550                 555                 560

Arg Arg Gly Gly Lys Gly Lys Ser Ala Ala Arg Ile Lys Glu Glu Asp
                565                 570                 575

Phe Ile Asp Arg Leu Leu Val Ala Asn Thr His Asp His Ile Leu Cys
            580                 585                 590

Phe Ser Ser Arg Gly Arg Val Tyr Ser Met Lys Val Tyr Gln Leu Pro
        595                 600                 605

Glu Ala Thr Arg Gly Ala Arg Gly Arg Pro Ile Val Asn Leu Leu Pro
    610                 615                 620

Leu Glu Gln Asp Glu Arg Ile Thr Ala Ile Leu Pro Val Thr Glu Phe
625                 630                 635                 640

Glu Glu Gly Val Lys Val Phe Met Ala Thr Ala Asn Gly Thr Val Lys
                645                 650                 655

Lys Thr Val Leu Thr Glu Phe Asn Arg Leu Arg Thr Ala Gly Lys Val
            660                 665                 670

Ala Ile Lys Leu Val Asp Gly Asp Glu Leu Ile Gly Val Asp Leu Thr
        675                 680                 685

Ser Gly Glu Asp Glu Val Met Leu Phe Ser Ala Glu Gly Lys Val Val
    690                 695                 700

Arg Phe Lys Glu Ser Ser Val Arg Ala Met Gly Cys Asn Thr Thr Gly
705                 710                 715                 720

Val Arg Gly Ile Arg Leu Gly Glu Gly Asp Lys Val Val Ser Leu Ile
                725                 730                 735

Val Pro Arg Gly Asp Gly Ala Ile Leu Thr Ala Thr Gln Asn Gly Tyr
            740                 745                 750

Gly Lys Arg Thr Ala Val Ala Glu Tyr Pro Thr Lys Ser Arg Ala Thr
        755                 760                 765

Lys Gly Val Ile Ser Ile Lys Val Thr Glu Arg Asn Gly Leu Val Val
    770                 775                 780

Gly Ala Val Gln Val Asp Asp Cys Asp Gln Ile Met Met Ile Thr Asp
785                 790                 795                 800

Ala Gly Thr Leu Val Arg Thr Arg Val Ser Glu Ile Ser Ile Val Gly
                805                 810                 815

Arg Asn Thr Gln Gly Val Ile Leu Ile Arg Thr Ala Glu Asp Glu Asn
            820                 825                 830

Val Val Gly Leu Gln Arg Val Ala Glu Pro Val Asp Glu Glu Asp Leu
        835                 840                 845

Asp Thr Ile Asp Gly Ser Ala Ala Glu Gly Asp Asp Glu Ile Ala Pro
    850                 855                 860

Glu Val Asp Val Asp Asp Glu Pro Glu Glu Glu
865                 870                 875

<210> 156
<211> 442
<212> PRT
<213> Escherichia coli

<400> 156
Met Thr Thr Val Thr Pro Thr Tyr Ser Lys Glu Leu Phe Met Ser Thr
1               5                   10                  15

Ala Leu Leu Asp Ala Val Val Lys Lys Asn Arg Val Arg Leu Ile Pro
            20                  25                  30

Phe Met Leu Ala Leu Tyr Val Leu Ala Phe Leu Asp Arg Ser Asn Ile
        35                  40                  45

Gly Phe Ala Lys Gln Thr Tyr Gln Ile Asp Thr Gly Leu Ser Asn Glu
50                  55                  60

Ala Tyr Ala Leu Gly Ala Gly Ile Phe Phe Val Val Tyr Ala Phe Leu
65                  70                  75                  80

Gly Val Pro Ala Asn Leu Leu Met Arg Lys Leu Gly Ala Arg Thr Trp
                85                  90                  95

Ile Gly Thr Thr Thr Leu Leu Trp Gly Phe Leu Ser Ala Ala Met Ala
            100                 105                 110

Trp Ala Asp Thr Glu Ala Lys Phe Leu Ile Val Arg Thr Leu Leu Arg
        115                 120                 125

Ala Ala Glu Ala Gly Phe Phe Pro Gly Met Ile Tyr Leu Thr Ser Gln
    130                 135                 140

Trp Phe Pro Gln Arg Asn Arg Ala Ser Ile Met Gly Leu Phe Tyr Met
145                 150                 155                 160

Gly Ala Pro Leu Ala Leu Thr Leu Gly Ser Pro Leu Ser Gly Ala Leu
                165                 170                 175

Leu Glu Met His Gly Phe Met Gly His Pro Gly Trp Phe Trp Met Phe
            180                 185                 190

Val Ile Glu Gly Leu Leu Ala Val Gly Ala Gly Val Phe Thr Phe Phe
        195                 200                 205

Trp Leu Asp Asp Thr Pro Glu Gln Ala Arg Phe Leu Ser Lys Gln Glu
    210                 215                 220

Lys Thr Leu Leu Ile Asn Gln Leu Ala Ser Glu Glu Gln Gln Lys Val
225                 230                 235                 240

Thr Ser Arg Leu Ser Asp Ala Leu Arg Asn Gly Arg Val Trp Gln Leu
                245                 250                 255

Ala Ile Ile Tyr Leu Thr Ile Gln Val Ala Val Tyr Gly Leu Ile Phe
            260                 265                 270

Phe Leu Pro Thr Gln Val Ala Ala Leu Leu Gly Thr Lys Val Gly Phe
        275                 280                 285

Thr Ala Ser Val Val Thr Ala Ile Pro Trp Val Ala Ala Leu Phe Gly
    290                 295                 300

Thr Trp Leu Ile Pro Arg Tyr Ser Asp Lys Thr Gly Glu Arg Arg Asn
305                 310                 315                 320

Val Ala Ala Leu Thr Leu Leu Ala Ala Gly Ile Gly Ile Gly Leu Ser
                325                 330                 335

Gly Leu Leu Ser Pro Val Met Ala Ile Val Ala Leu Cys Val Ala Ala
            340                 345                 350

Ile Gly Phe Ile Ala Val Gln Pro Val Phe Trp Thr Met Pro Thr Gln
        355                 360                 365

Leu Leu Ser Gly Thr Ala Leu Ala Ala Gly Ile Gly Phe Val Asn Leu
    370                 375                 380

Phe Gly Ala Val Gly Gly Phe Ile Ala Pro Ile Leu Arg Val Lys Ala
385                 390                 395                 400

Glu Thr Leu Phe Ala Ser Asp Ala Ala Gly Leu Leu Thr Leu Ala Ala
                405                 410                 415

Val Ala Val Ile Gly Ser Leu Ile Ile Phe Thr Leu Arg Val Asn Arg
            420                 425                 430

Thr Val Ala Gln Thr Asp Val Ala His His
        435                 440


<210> 157
<211> 220
<212> PRT
<213> Escherichia coli

<400> 157
Met Asp Tyr Thr Leu Thr Arg Ile Asp Pro Asn Gly Glu Asn Asp Arg
1               5                   10                  15

Tyr Pro Leu Gln Lys Gln Glu Ile Val Thr Asp Pro Leu Glu Gln Glu
            20                  25                  30

Val Asn Lys Asn Val Phe Met Gly Lys Leu Asn Asp Met Val Asn Trp
        35                  40                  45

Gly Arg Lys Asn Ser Ile Trp Pro Tyr Asn Phe Gly Leu Ser Cys Cys
    50                  55                  60

Tyr Val Glu Met Val Thr Ser Phe Thr Ala Val His Asp Val Ala Arg
65                  70                  75                  80

Phe Gly Ala Glu Val Leu Arg Ala Ser Pro Arg Gln Ala Asp Leu Met
                85                  90                  95

Val Val Ala Gly Thr Cys Phe Thr Lys Met Ala Pro Val Ile Gln Arg
            100                 105                 110

Leu Tyr Asp Gln Met Leu Glu Pro Lys Trp Val Ile Ser Met Gly Ala
        115                 120                 125

Cys Ala Asn Ser Gly Gly Met Tyr Asp Ile Tyr Ser Val Val Gln Gly
    130                 135                 140

Val Asp Lys Phe Ile Pro Val Asp Val Tyr Ile Pro Gly Cys Pro Pro
145                 150                 155                 160

Arg Pro Glu Ala Tyr Met Gln Ala Leu Met Leu Leu Gln Glu Ser Ile
                165                 170                 175

Gly Lys Glu Arg Arg Pro Leu Ser Trp Val Val Gly Asp Gln Gly Val
            180                 185                 190

Tyr Arg Ala Asn Met Gln Ser Glu Arg Glu Arg Lys Arg Gly Glu Arg
        195                 200                 205

Ile Ala Val Thr Asn Leu Arg Thr Pro Asp Glu Ile
    210                 215                 220


<210> 158
<211> 181
<212> PRT
<213> Escherichia coli

<400> 158
Met Leu Lys Leu Leu Lys Thr Ile Met Arg Ala Gly Thr Ala Thr Val
  1               5                  10                  15

Lys Tyr Pro Phe Ala Pro Leu Glu Val Ser Pro Gly Phe Arg Gly Lys
             20                  25                  30

Pro Asp Leu Met Pro Ser Gln Cys Ile Ala Cys Gly Ala Cys Ala Cys
         35                  40                  45

Ala Cys Pro Ala Asn Ala Leu Thr Ile Gln Thr Asp Asp Gln Gln Asn
     50                  55                  60

Ser Arg Thr Trp Gln Leu Tyr Leu Gly Arg Cys Ile Tyr Cys Gly Arg
 65                  70                  75                  80

Cys Glu Glu Val Cys Pro Thr Arg Ala Ile Gln Leu Thr Asn Asn Phe
                 85                  90                  95

Glu Leu Thr Val Thr Asn Lys Ala Asp Leu Tyr Thr Arg Ala Thr Phe
            100                 105                 110

His Leu Gln Arg Cys Ser Arg Cys Glu Arg Pro Phe Ala Pro Gln Lys
        115                 120                 125

Thr Ile Ala Leu Ala Ala Glu Leu Leu Ala Gln Gln Gln Asn Ala Pro
    130                 135                 140

Gln Asn Arg Glu Met Leu Trp Ala Gln Ala Ser Val Cys Pro Glu Cys
145                 150                 155                 160

Lys Gln Arg Ala Thr Leu Ile Asn Asp Asp Thr Asp Val Leu Leu Val
                165                 170                 175

Ala Lys Glu Gln Leu
            180

<210> 159
<211> 353
<212> PRT
<213> Escherichia coli

<400> 159
Met Ala Ile Asp Glu Asn Lys Gln Lys Ala Leu Ala Ala Ala Leu Gly
1               5                   10                  15

Gln Ile Glu Lys Gln Phe Gly Lys Gly Ser Ile Met Arg Leu Gly Glu
            20                  25                  30

Asp Arg Ser Met Asp Val Glu Thr Ile Ser Thr Gly Ser Leu Ser Leu
        35                  40                  45

Asp Ile Ala Leu Gly Ala Gly Gly Leu Pro Met Gly Arg Ile Val Glu
    50                  55                  60

Ile Tyr Gly Pro Glu Ser Ser Gly Lys Thr Thr Leu Thr Leu Gln Val
65                  70                  75                  80

Ile Ala Ala Ala Gln Arg Glu Gly Lys Thr Cys Ala Phe Ile Asp Ala
                85                  90                  95

Glu His Ala Leu Asp Pro Ile Tyr Ala Arg Lys Leu Gly Val Asp Ile
            100                 105                 110

Asp Asn Leu Leu Cys Ser Gln Pro Asp Thr Gly Glu Gln Ala Leu Glu
        115                 120                 125

Ile Cys Asp Ala Leu Ala Arg Ser Gly Ala Val Asp Val Ile Val Val
    130                 135                 140

Asp Ser Val Ala Ala Leu Thr Pro Lys Ala Glu Ile Glu Gly Glu Ile
145                 150                 155                 160

Gly Asp Ser His Met Gly Leu Ala Ala Arg Met Met Ser Gln Ala Met
                165                 170                 175

Arg Lys Leu Ala Gly Asn Leu Lys Gln Ser Asn Thr Leu Leu Ile Phe
            180                 185                 190

Ile Asn Gln Ile Arg Met Lys Ile Gly Val Met Phe Gly Asn Pro Glu
        195                 200                 205

Thr Thr Thr Gly Gly Asn Ala Leu Lys Phe Tyr Ala Ser Val Arg Leu
    210                 215                 220

Asp Ile Arg Arg Ile Gly Ala Val Lys Glu Gly Glu Asn Val Val Gly
225                 230                 235                 240

Ser Glu Thr Arg Val Lys Val Val Lys Asn Lys Ile Ala Ala Pro Phe
                245                 250                 255

Lys Gln Ala Glu Phe Gln Ile Leu Tyr Gly Glu Gly Ile Asn Phe Tyr
            260                 265                 270

Gly Glu Leu Val Asp Leu Gly Val Lys Glu Lys Leu Ile Glu Lys Ala
        275                 280                 285

Gly Ala Trp Tyr Ser Tyr Lys Gly Glu Lys Ile Gly Gln Gly Lys Ala
    290                 295                 300

Asn Ala Thr Ala Trp Leu Lys Asp Asn Pro Glu Thr Ala Lys Glu Ile
305                 310                 315                 320

Glu Lys Lys Val Arg Glu Leu Leu Leu Ser Asn Pro Asn Ser Thr Pro
                325                 330                 335

Asp Phe Ser Val Asp Asp Ser Glu Gly Val Ala Glu Thr Asn Glu Asp
            340                 345                 350

Phe

<210> 160
<211> 853
<212> PRT
<213> Escherichia coli

<400> 160
Met Ser Ala Ile Glu Asn Phe Asp Ala His Thr Pro Met Met Gln Gln
  1               5                  10                  15

Tyr Leu Arg Leu Lys Ala Gln His Pro Glu Ile Leu Leu Phe Tyr Arg
             20                  25                  30

Met Gly Asp Phe Tyr Glu Leu Phe Tyr Asp Asp Ala Lys Arg Ala Ser
         35                  40                  45

Gln Leu Leu Asp Ile Ser Leu Thr Lys Arg Gly Ala Ser Ala Gly Glu
     50                  55                  60

Pro Ile Pro Met Ala Gly Ile Pro Tyr His Ala Val Glu Asn Tyr Leu
 65                  70                  75                  80

Ala Lys Leu Val Asn Gln Gly Glu Ser Val Ala Ile Cys Glu Gln Ile
                 85                  90                  95

Gly Asp Pro Ala Thr Ser Lys Gly Pro Val Glu Arg Lys Val Val Arg
            100                 105                 110

Ile Val Thr Pro Gly Thr Ile Ser Asp Glu Ala Leu Leu Gln Glu Arg
        115                 120                 125

Gln Asp Asn Leu Leu Ala Ala Ile Trp Gln Asp Ser Lys Gly Phe Gly
    130                 135                 140

Tyr Ala Thr Leu Asp Ile Ser Ser Gly Arg Phe Arg Leu Ser Glu Pro
145                 150                 155                 160

Ala Asp Arg Glu Thr Met Ala Ala Glu Leu Gln Arg Thr Asn Pro Ala
                165                 170                 175

Glu Leu Leu Tyr Ala Glu Asp Phe Ala Glu Met Ser Leu Ile Glu Gly
            180                 185                 190

Arg Arg Gly Leu Arg Arg Arg Pro Leu Trp Glu Phe Glu Ile Asp Thr
        195                 200                 205

Ala Arg Gln Gln Leu Asn Leu Gln Phe Gly Thr Arg Asp Leu Val Gly
    210                 215                 220

Phe Gly Val Glu Asn Ala Pro Arg Gly Leu Cys Ala Ala Gly Cys Leu
225                 230                 235                 240

Leu Gln Tyr Ala Lys Asp Thr Gln Arg Thr Thr Leu Pro His Ile Arg
                245                 250                 255

Ser Ile Thr Met Glu Arg Glu Gln Asp Ser Ile Ile Met Asp Ala Ala
            260                 265                 270

Thr Arg Arg Asn Leu Glu Ile Thr Gln Asn Leu Ala Gly Gly Ala Glu
        275                 280                 285

Asn Thr Leu Ala Ser Val Leu Asp Cys Thr Val Thr Pro Met Gly Ser
    290                 295                 300

Arg Met Leu Lys Arg Trp Leu His Met Pro Val Arg Asp Thr Arg Val
305                 310                 315                 320

Leu Leu Glu Arg Gln Gln Thr Ile Gly Ala Leu Gln Asp Phe Thr Ala
                325                 330                 335

Gly Leu Gln Pro Val Leu Arg Gln Val Gly Asp Leu Glu Arg Ile Leu
            340                 345                 350

Ala Arg Leu Ala Leu Arg Thr Ala Arg Pro Arg Asp Leu Ala Arg Met
        355                 360                 365

Arg His Ala Phe Gln Gln Leu Pro Glu Leu Arg Ala Gln Leu Glu Thr
    370                 375                 380

Val Asp Ser Ala Pro Val Gln Ala Leu Arg Glu Lys Met Gly Glu Phe
385                 390                 395                 400

Ala Glu Leu Arg Asp Leu Leu Glu Arg Ala Ile Ile Asp Thr Pro Pro
                405                 410                 415

Val Leu Val Arg Asp Gly Gly Val Ile Ala Ser Gly Tyr Asn Glu Glu
            420                 425                 430

Leu Asp Glu Trp Arg Ala Leu Ala Asp Gly Ala Thr Asp Tyr Leu Glu
        435                 440                 445

Arg Leu Glu Val Arg Glu Arg Glu Arg Thr Gly Leu Asp Thr Leu Lys
    450                 455                 460

Val Gly Phe Asn Ala Val His Gly Tyr Tyr Ile Gln Ile Ser Arg Gly
465                 470                 475                 480

Gln Ser His Leu Ala Pro Ile Asn Tyr Met Arg Arg Gln Thr Leu Lys
                485                 490                 495

Asn Ala Glu Arg Tyr Ile Ile Pro Glu Leu Lys Glu Tyr Glu Asp Lys
            500                 505                 510

Val Leu Thr Ser Lys Gly Lys Ala Leu Ala Leu Glu Lys Gln Leu Tyr
        515                 520                 525

Glu Glu Leu Phe Asp Leu Leu Leu Pro His Leu Glu Ala Leu Gln Gln
    530                 535                 540

Ser Ala Ser Ala Leu Ala Glu Leu Asp Val Leu Val Asn Leu Ala Glu
545                 550                 555                 560

Arg Ala Tyr Thr Leu Asn Tyr Thr Cys Pro Thr Phe Ile Asp Lys Pro
                565                 570                 575

Gly Ile Arg Ile Thr Glu Gly Arg His Pro Val Val Glu Gln Val Leu
            580                 585                 590

Asn Glu Pro Phe Ile Ala Asn Pro Leu Asn Leu Ser Pro Gln Arg Arg
        595                 600                 605

Met Leu Ile Ile Thr Gly Pro Asn Met Gly Gly Lys Ser Thr Tyr Met
    610                 615                 620

Arg Gln Thr Ala Leu Ile Ala Leu Met Ala Tyr Ile Gly Ser Tyr Val
625                 630                 635                 640

Pro Ala Gln Lys Val Glu Ile Gly Pro Ile Asp Arg Ile Phe Thr Arg
                645                 650                 655

Val Gly Ala Ala Asp Asp Leu Ala Ser Gly Arg Ser Thr Phe Met Val
            660                 665                 670

Glu Met Thr Glu Thr Ala Asn Ile Leu His Asn Ala Thr Glu Tyr Ser
        675                 680                 685

Leu Val Leu Met Asp Glu Ile Gly Arg Gly Thr Ser Thr Tyr Asp Gly
    690                 695                 700

Leu Ser Leu Ala Trp Ala Cys Ala Glu Asn Leu Ala Asn Lys Ile Lys
705                 710                 715                 720

Ala Leu Thr Leu Phe Ala Thr His Tyr Phe Glu Leu Thr Gln Leu Pro
                725                 730                 735

Glu Lys Met Glu Gly Val Ala Asn Val His Leu Asp Ala Leu Glu His
            740                 745                 750

Gly Asp Thr Ile Ala Phe Met His Ser Val Gln Asp Gly Ala Ala Ser

755                 760                 765

Lys Ser Tyr Gly Leu Ala Val Ala Ala Leu Ala Gly Val Pro Lys Glu
    770                 775                 780

Val Ile Lys Arg Ala Arg Gln Lys Leu Arg Glu Leu Glu Ser Ile Ser
785                 790                 795                 800

Pro Asn Ala Ala Ala Thr Gln Val Asp Gly Thr Gln Met Ser Leu Leu
                805                 810                 815

Ser Val Pro Glu Glu Thr Ser Pro Ala Val Glu Ala Leu Glu Asn Leu
            820                 825                 830

Asp Pro Asp Ser Leu Thr Pro Arg Gln Ala Leu Glu Trp Ile Tyr Arg
        835                 840                 845

Leu Lys Ser Leu Val
    850


<210> 161
<211> 163
<212> PRT
<213> Escherichia coli

<400> 161
Met Met Lys Ser Leu Ile Ile Val Asn Pro Ala Asp Cys Ile Gly Cys
1               5                   10                  15

Arg Thr Cys Glu Val Ala Cys Val Val Ala His Pro Ser Glu Gln Glu
            20                  25                  30

Leu Asn Ala Asp Val Phe Leu Pro Arg Leu Lys Val Gln Arg Leu Asp
        35                  40                  45

Ser Ile Ser Ala Pro Val Met Cys His Gln Cys Glu Asn Ala Pro Cys
    50                  55                  60

Val Gly Ala Cys Pro Val Gly Ala Leu Thr Met Gly Glu Gln Val Val
65                  70                  75                  80

Gln Thr Asn Ser Ala Arg Cys Ile Gly Cys Gln Ser Cys Val Ser Ala
                85                  90                  95

Cys Pro Phe Gly Met Ile Thr Ile Gln Ser Leu Pro Gly Asp Thr Arg
            100                 105                 110

Gln Gln Ile Val Lys Cys Asp Leu Cys Glu Gln Arg Glu Glu Gly Pro
        115                 120                 125

Ala Cys Val Glu Ser Cys Pro Thr Gln Ala Leu Gln Leu Leu Thr Glu
    130                 135                 140

Arg Glu Leu Arg Arg Val Arg Gln Gln Arg Ile Val Ala Ser Gly Glu
145                 150                 155                 160

Asn Pro Leu

<210> 162
<211> 387
<212> PRT
<213> Escherichia coli

<400> 162
Met Ser Val Ile Lys Met Thr Asp Leu Asp Leu Ala Gly Lys Arg Val
1               5                   10                  15

Phe Ile Arg Ala Asp Leu Asn Val Pro Val Lys Asp Gly Lys Val Thr
            20                  25                  30

Ser Asp Ala Arg Ile Arg Ala Ser Leu Pro Thr Ile Glu Leu Ala Leu
        35                  40                  45

Lys Gln Gly Ala Lys Val Met Val Thr Ser His Leu Gly Arg Pro Thr
    50                  55                  60

Glu Gly Glu Tyr Asn Glu Glu Phe Ser Leu Leu Pro Val Val Asn Tyr
65                  70                  75                  80

Leu Lys Asp Lys Leu Ser Asn Pro Val Arg Leu Val Lys Asp Tyr Leu
                85                  90                  95

Asp Gly Val Asp Val Ala Glu Gly Glu Leu Val Val Leu Glu Asn Val
            100                 105                 110

Arg Phe Asn Lys Gly Glu Lys Lys Asp Asp Glu Thr Leu Ser Lys Lys
        115                 120                 125

Tyr Ala Ala Leu Cys Asp Val Phe Val Met Asp Ala Phe Gly Thr Ala
    130                 135                 140

His Arg Ala Gln Ala Ser Thr His Gly Ile Gly Lys Phe Ala Asp Val
145                 150                 155                 160

Ala Cys Ala Gly Pro Leu Leu Ala Ala Glu Leu Asp Ala Leu Gly Lys
                165                 170                 175

Ala Leu Lys Glu Pro Ala Arg Pro Met Val Ala Ile Val Gly Gly Ser
            180                 185                 190

Lys Val Ser Thr Lys Leu Thr Val Leu Asp Ser Leu Ser Lys Ile Ala
        195                 200                 205

Asp Gln Leu Ile Val Gly Gly Gly Ile Ala Asn Thr Phe Ile Ala Ala
    210                 215                 220

Gln Gly His Asp Val Gly Lys Ser Leu Tyr Glu Ala Asp Leu Val Asp
225                 230                 235                 240

Glu Ala Lys Arg Leu Leu Thr Thr Cys Asn Ile Pro Val Pro Ser Asp
                245                 250                 255

Val Arg Val Ala Thr Glu Phe Ser Glu Thr Ala Pro Ala Thr Leu Lys
            260                 265                 270

Ser Val Asn Asp Val Lys Ala Asp Glu Gln Ile Leu Asp Ile Gly Asp
        275                 280                 285

Ala Ser Ala Gln Glu Leu Ala Glu Ile Leu Lys Asn Ala Lys Thr Ile
    290                 295                 300

Leu Trp Asn Gly Pro Val Gly Val Phe Glu Phe Pro Asn Phe Arg Lys
305                 310                 315                 320

Gly Thr Glu Ile Val Ala Asn Ala Ile Ala Asp Ser Glu Ala Phe Ser
                325                 330                 335

Ile Ala Gly Gly Gly Asp Thr Leu Ala Ala Ile Asp Leu Phe Gly Ile
            340                 345                 350

Ala Asp Lys Ile Ser Tyr Ile Ser Thr Gly Gly Gly Ala Phe Leu Glu
        355                 360                 365

Phe Val Glu Gly Lys Val Leu Pro Ala Val Ala Met Leu Glu Glu Arg
    370                 375                 380

Ala Lys Lys
385


<210> 163
<211> 254
<212> PRT
<213> Escherichia coli

<400> 163
Met Lys Asp Glu Arg Arg Pro Ile Cys Glu Val Val Ala Glu Ser Ile
  1               5                  10                  15

Glu Arg Leu Ile Ile Asp Gly Val Leu Lys Val Gly Gln Pro Leu Pro
             20                  25                  30

Ser Glu Arg Arg Leu Cys Glu Lys Leu Gly Phe Ser Arg Ser Ala Leu
         35                  40                  45

Arg Glu Gly Leu Thr Val Leu Arg Gly Arg Gly Ile Ile Glu Thr Ala
     50                  55                  60

Gln Gly Arg Asp Ser Arg Val Ala Arg Leu Asn Arg Val Gln Asp Thr
 65                  70                  75                  80

Ser Pro Leu Ile His Leu Phe Ser Thr Gln Pro Arg Thr Leu Tyr Asp
                 85                  90                  95

Leu Leu Asp Val Arg Ala Leu Leu Glu Gly Glu Ser Ala Arg Leu Ala

            100                 105                 110

Ala Thr Leu Gly Thr Gln Ala Asp Phe Val Val Ile Thr Arg Cys Tyr
        115                 120                 125

Glu Lys Met Leu Ala Ala Ser Glu Asn Asn Lys Glu Ile Ser Leu Ile
    130                 135                 140

Glu His Ala Gln Leu Asp His Ala Phe His Leu Ala Ile Cys Gln Ala
145                 150                 155                 160

Ser His Asn Gln Val Leu Val Phe Thr Leu Gln Ser Leu Thr Asp Leu
                165                 170                 175

Met Phe Asn Ser Val Phe Ala Ser Val Asn Asn Leu Tyr His Arg Pro
            180                 185                 190

Gln Gln Lys Lys Gln Ile Asp Arg Gln His Ala Arg Ile Tyr Asn Ala
        195                 200                 205

Val Leu Gln Arg Leu Pro His Val Ala Gln Arg Ala Ala Arg Asp His
    210                 215                 220

Val Arg Thr Val Lys Lys Asn Leu His Asp Ile Glu Leu Glu Gly His
225                 230                 235                 240

His Leu Ile Arg Ser Ala Val Pro Leu Glu Met Asn Leu Ser
                245                 250

<210> 164
<211> 499
<212> PRT
<213> Escherichia coli

<400> 164
Met Leu Asn Leu Phe Val Gly Leu Asp Ile Tyr Thr Gly Leu Leu Leu
1               5                   10                  15

Leu Leu Ala Leu Ala Phe Val Leu Phe Tyr Glu Ala Ile Asn Gly Phe
            20                  25                  30

His Asp Thr Ala Asn Ala Val Ala Ala Val Ile Tyr Thr Arg Ala Met
        35                  40                  45

Gln Pro Gln Leu Ala Val Val Met Ala Ala Phe Phe Asn Phe Phe Gly
    50                  55                  60

Val Leu Leu Gly Gly Leu Ser Val Ala Tyr Ala Ile Val His Met Leu
65                  70                  75                  80

Pro Thr Asp Leu Leu Leu Asn Met Gly Ser Thr His Gly Leu Ala Met
                85                  90                  95

Val Phe Ser Met Leu Leu Ala Ala Ile Ile Trp Asn Leu Gly Thr Trp
            100                 105                 110

Phe Phe Gly Leu Pro Ala Ser Ser Ser His Thr Leu Ile Gly Ala Ile
        115                 120                 125

Ile Gly Ile Gly Leu Thr Asn Ala Leu Leu Thr Gly Ser Ser Val Met
    130                 135                 140

Asp Ala Leu Asn Leu Arg Glu Val Thr Lys Ile Phe Ser Ser Leu Ile
145                 150                 155                 160

Val Ser Pro Ile Val Gly Leu Val Ile Ala Gly Gly Leu Ile Phe Leu
                165                 170                 175

Leu Arg Arg Tyr Trp Ser Gly Thr Lys Lys Arg Asp Arg Ile His Arg
            180                 185                 190

Ile Pro Glu Asp Arg Lys Lys Lys Lys Gly Lys Arg Lys Pro Pro Phe
        195                 200                 205

Trp Thr Arg Ile Ala Leu Ile Val Ser Ala Ala Gly Val Ala Phe Ser
    210                 215                 220

His Gly Ala Asn Asp Gly Gln Lys Gly Ile Gly Leu Val Met Leu Val
225                 230                 235                 240

Leu Val Gly Ile Ala Pro Ala Gly Phe Val Val Asn Met Asn Ala Ser
                245                 250                 255

Gly Tyr Glu Ile Thr Arg Thr Arg Asp Ala Val Thr Asn Phe Glu His
            260                 265                 270

Tyr Leu Gln Gln His Pro Glu Leu Pro Gln Lys Leu Ile Ala Met Glu
        275                 280                 285

Pro Pro Leu Pro Ala Ala Ser Thr Asp Gly Thr Gln Val Thr Glu Phe
    290                 295                 300

His Cys His Pro Ala Asn Thr Phe Asp Ala Ile Ala Arg Val Lys Thr
305                 310                 315                 320

Met Leu Pro Gly Asn Met Glu Ser Tyr Glu Pro Leu Ser Val Ser Gln
                325                 330                 335

Arg Ser Gln Leu Arg Arg Ile Met Leu Cys Ile Ser Asp Thr Ser Ala
            340                 345                 350

Lys Leu Ala Lys Leu Pro Gly Val Ser Lys Glu Asp Gln Asn Leu Leu
        355                 360                 365

Lys Lys Leu Arg Ser Asp Met Leu Ser Thr Ile Glu Tyr Ala Pro Val
    370                 375                 380

Trp Ile Ile Met Ala Val Ala Leu Ala Leu Gly Ile Gly Thr Met Ile
385                 390                 395                 400

Gly Trp Arg Arg Val Ala Met Thr Ile Gly Glu Lys Ile Gly Lys Arg

405                 410                 415

Gly Met Thr Tyr Ala Gln Gly Met Ala Ala Gln Met Thr Ala Ala Val
            420                 425                 430

Ser Ile Gly Leu Ala Ser Tyr Ile Gly Met Pro Val Ser Thr Thr His
        435                 440                 445

Val Leu Ser Ser Ala Val Ala Gly Thr Met Val Val Asp Gly Gly Gly
    450                 455                 460

Leu Gln Arg Lys Thr Val Thr Ser Ile Leu Met Ala Trp Val Phe Thr
465                 470                 475                 480

Leu Pro Ala Ala Ile Phe Leu Ser Gly Gly Leu Tyr Trp Ile Ala Leu
                485                 490                 495

Gln Leu Ile

<210> 165
<211> 630
<212> PRT
<213> Escherichia coli

<400> 165
Met Thr Gln Thr Tyr Asn Ala Asp Ala Ile Glu Val Leu Thr Gly Leu
1               5                   10                  15

Glu Pro Val Arg Arg Arg Pro Gly Met Tyr Thr Asp Thr Thr Arg Pro
            20                  25                  30

Asn His Leu Gly Gln Glu Val Ile Asp Asn Ser Val Asp Glu Ala Leu
        35                  40                  45

Ala Gly His Ala Lys Arg Val Asp Val Ile Leu His Ala Asp Gln Ser
    50                  55                  60

Leu Glu Val Ile Asp Asp Gly Arg Gly Met Pro Val Asp Ile His Pro
65                  70                  75                  80

Glu Glu Gly Val Pro Ala Val Glu Leu Ile Leu Cys Arg Leu His Ala
                85                  90                  95

Gly Gly Lys Phe Ser Asn Lys Asn Tyr Gln Phe Ser Gly Gly Leu His
            100                 105                 110

Gly Val Gly Ile Ser Val Val Asn Ala Leu Ser Lys Arg Val Glu Val
        115                 120                 125

Asn Val Arg Arg Asp Gly Gln Val Tyr Asn Ile Ala Phe Glu Asn Gly
    130                 135                 140

Glu Lys Val Gln Asp Leu Gln Val Val Gly Thr Cys Gly Lys Arg Asn
145                 150                 155                 160

Thr Gly Thr Ser Val His Phe Trp Pro Asp Glu Thr Phe Phe Asp Ser
                165                 170                 175

Pro Arg Phe Ser Val Ser Arg Leu Thr His Val Leu Lys Ala Lys Ala
            180                 185                 190

Val Leu Cys Pro Gly Val Glu Ile Thr Phe Lys Asp Glu Ile Asn Asn
        195                 200                 205

Thr Glu Gln Arg Trp Cys Tyr Gln Asp Gly Leu Asn Asp Tyr Leu Ala
    210                 215                 220

Glu Ala Val Asn Gly Leu Pro Thr Leu Pro Glu Lys Pro Phe Ile Gly
225                 230                 235                 240

Asn Phe Ala Gly Asp Thr Glu Ala Val Asp Trp Ala Leu Leu Trp Leu
                245                 250                 255

Pro Glu Gly Gly Glu Leu Leu Thr Glu Ser Tyr Val Asn Leu Ile Pro
            260                 265                 270

Thr Met Gln Gly Gly Thr His Val Asn Gly Leu Arg Gln Gly Leu Leu
        275                 280                 285

Asp Ala Met Arg Glu Phe Cys Glu Tyr Arg Asn Ile Leu Pro Arg Gly
    290                 295                 300

Val Lys Leu Ser Ala Glu Asp Ile Trp Asp Arg Cys Ala Tyr Val Leu
305                 310                 315                 320

Ser Val Lys Met Gln Asp Pro Gln Phe Ala Gly Gln Thr Lys Glu Arg
                325                 330                 335

Leu Ser Ser Arg Gln Cys Ala Ala Phe Val Ser Gly Val Val Lys Asp
            340                 345                 350

Ala Phe Ile Leu Trp Leu Asn Gln Asn Val Gln Ala Ala Glu Leu Leu
        355                 360                 365

Ala Glu Met Ala Ile Ser Ser Ala Gln Arg Arg Met Arg Ala Ala Lys
    370                 375                 380

Lys Val Val Arg Lys Lys Leu Thr Ser Gly Pro Ala Leu Pro Gly Lys
385                 390                 395                 400

Leu Ala Asp Cys Thr Ala Gln Asp Leu Asn Arg Thr Glu Leu Phe Leu
                405                 410                 415

Val Glu Gly Asp Ser Ala Gly Gly Ser Ala Lys Gln Ala Arg Asp Arg
            420                 425                 430

Glu Tyr Gln Ala Ile Met Pro Leu Lys Gly Lys Ile Leu Asn Thr Trp
        435                 440                 445

Glu Val Ser Ser Asp Glu Val Leu Ala Ser Gln Glu Val His Asp Ile

450                 455                 460

Ser Val Ala Ile Gly Ile Asp Pro Asp Ser Asp Asp Leu Ser Gln Leu
465                 470                 475                 480

Arg Tyr Gly Lys Ile Cys Ile Leu Ala Asp Ala Asp Ser Asp Gly Leu
                485                 490                 495

His Ile Ala Thr Leu Leu Cys Ala Leu Phe Val Lys His Phe Arg Ala
            500                 505                 510

Leu Val Lys His Gly His Val Tyr Val Ala Leu Pro Pro Leu Tyr Arg
        515                 520                 525

Ile Asp Leu Gly Lys Glu Val Tyr Tyr Ala Leu Thr Glu Glu Glu Lys
    530                 535                 540

Glu Gly Val Leu Glu Gln Leu Lys Arg Lys Lys Gly Lys Pro Asn Val
545                 550                 555                 560

Gln Arg Phe Lys Gly Leu Gly Glu Met Asn Pro Met Gln Leu Arg Glu
                565                 570                 575

Thr Thr Leu Asp Pro Asn Thr Arg Arg Leu Val Gln Leu Thr Ile Asp
            580                 585                 590

Asp Glu Asp Asp Gln Arg Thr Asp Ala Met Met Asp Met Leu Leu Ala
        595                 600                 605

Lys Lys Arg Ser Glu Asp Arg Arg Asn Trp Leu Gln Glu Lys Gly Asp
    610                 615                 620

Met Ala Glu Ile Glu Val
625                 630

<210> 166
<211> 581
<212> PRT
<213> Escherichia coli

<400> 166
Met Ala Gly Arg Ile Pro Arg Val Phe Ile Asn Asp Leu Leu Ala Arg
1               5                   10                  15

Thr Asp Ile Val Asp Leu Ile Asp Ala Arg Val Lys Leu Lys Lys Gln
            20                  25                  30

Gly Lys Asn Phe His Ala Cys Cys Pro Phe His Asn Glu Lys Thr Pro
        35                  40                  45

Ser Phe Thr Val Asn Gly Glu Lys Gln Phe Tyr His Cys Phe Gly Cys
    50                  55                  60

Gly Ala His Gly Asn Ala Ile Asp Phe Leu Met Asn Tyr Asp Lys Leu
65                  70                  75                  80

Glu Phe Val Glu Thr Val Glu Glu Leu Ala Ala Met His Asn Leu Glu
                85                  90                  95

Val Pro Phe Glu Ala Gly Ser Gly Pro Ser Gln Ile Glu Arg His Gln
            100                 105                 110

Arg Gln Thr Leu Tyr Gln Leu Met Asp Gly Leu Asn Thr Phe Tyr Gln
        115                 120                 125

Gln Ser Leu Gln Gln Pro Val Ala Thr Ser Ala Arg Gln Tyr Leu Glu
    130                 135                 140

Lys Arg Gly Leu Ser His Glu Val Ile Ala Arg Phe Ala Ile Gly Phe
145                 150                 155                 160

Ala Pro Pro Gly Trp Asp Asn Val Leu Lys Arg Phe Gly Gly Asn Pro
                165                 170                 175

Glu Asn Arg Gln Ser Leu Ile Asp Ala Gly Met Leu Val Thr Asn Asp
            180                 185                 190

Gln Gly Arg Ser Tyr Asp Arg Phe Arg Glu Arg Val Met Phe Pro Ile
        195                 200                 205

Arg Asp Lys Arg Gly Arg Val Ile Gly Phe Gly Gly Arg Val Leu Gly
    210                 215                 220

Asn Asp Thr Pro Lys Tyr Leu Asn Ser Pro Glu Thr Asp Ile Phe His
225                 230                 235                 240

Lys Gly Arg Gln Leu Tyr Gly Leu Tyr Glu Ala Gln Gln Asp Asn Ala
                245                 250                 255

Glu Pro Asn Arg Leu Leu Val Val Glu Gly Tyr Met Asp Val Val Ala
            260                 265                 270

Leu Ala Gln Tyr Gly Ile Asn Tyr Ala Val Ala Ser Leu Gly Thr Ser
        275                 280                 285

Thr Thr Ala Asp His Ile Gln Leu Leu Phe Arg Ala Thr Asn Asn Val
    290                 295                 300

Ile Cys Cys Tyr Asp Gly Asp Arg Ala Gly Arg Asp Ala Ala Trp Arg
305                 310                 315                 320

Ala Leu Glu Thr Ala Leu Pro Tyr Met Thr Asp Gly Arg Gln Leu Arg
                325                 330                 335

Phe Met Phe Leu Pro Asp Gly Glu Asp Pro Asp Thr Leu Val Arg Lys
            340                 345                 350

Glu Gly Lys Glu Ala Phe Glu Ala Arg Met Glu Gln Ala Met Pro Leu
        355                 360                 365

Ser Ala Phe Leu Phe Asn Ser Leu Met Pro Gln Val Asp Leu Ser Thr

370                 375                 380

Pro Asp Gly Arg Ala Arg Leu Ser Thr Leu Ala Leu Pro Leu Ile Ser
385                 390                 395                 400

Gln Val Pro Gly Glu Thr Leu Arg Ile Tyr Leu Arg Gln Glu Leu Gly
                405                 410                 415

Asn Lys Leu Gly Ile Leu Asp Asp Ser Gln Leu Glu Arg Leu Met Pro
            420                 425                 430

Lys Ala Ala Glu Ser Gly Val Ser Arg Pro Val Pro Gln Leu Lys Arg
        435                 440                 445

Thr Thr Met Arg Ile Leu Ile Gly Leu Leu Val Gln Asn Pro Glu Leu
    450                 455                 460

Ala Thr Leu Val Pro Pro Leu Glu Asn Leu Asp Glu Asn Lys Leu Pro
465                 470                 475                 480

Gly Leu Gly Leu Phe Arg Glu Leu Val Asn Thr Cys Leu Ser Gln Pro
                485                 490                 495

Gly Leu Thr Thr Gly Gln Leu Leu Glu His Tyr Arg Gly Thr Asn Asn
            500                 505                 510

Ala Ala Thr Leu Glu Lys Leu Ser Met Trp Asp Asp Ile Ala Asp Lys
        515                 520                 525

Asn Ile Ala Glu Gln Thr Phe Thr Asp Ser Leu Asn His Met Phe Asp
    530                 535                 540

Ser Leu Leu Glu Leu Arg Gln Glu Glu Leu Ile Ala Arg Glu Arg Thr
545                 550                 555                 560

His Gly Leu Ser Asn Glu Glu Arg Leu Glu Leu Trp Thr Leu Asn Gln
                565                 570                 575

Glu Leu Ala Lys Lys
            580

<210> 167
<211> 613
<212> PRT
<213> Escherichia coli

<400> 167
Met Glu Gln Asn Pro Gln Ser Gln Leu Lys Leu Leu Val Thr Arg Gly
1               5                   10                  15

Lys Glu Gln Gly Tyr Leu Thr Tyr Ala Glu Val Asn Asp His Leu Pro
            20                  25                  30

Glu Asp Ile Val Asp Ser Asp Gln Ile Glu Asp Ile Ile Gln Met Ile
        35                  40                  45

Asn Asp Met Gly Ile Gln Val Met Glu Glu Ala Pro Asp Ala Asp Asp
    50                  55                  60

Leu Met Leu Ala Glu Asn Thr Ala Asp Glu Asp Ala Ala Glu Ala Ala
65                  70                  75                  80

Ala Gln Val Leu Ser Ser Val Glu Ser Glu Ile Gly Arg Thr Thr Asp
                85                  90                  95

Pro Val Arg Met Tyr Met Arg Glu Met Gly Thr Val Glu Leu Leu Thr
            100                 105                 110

Arg Glu Gly Glu Ile Asp Ile Ala Lys Arg Ile Glu Asp Gly Ile Asn
        115                 120                 125

Gln Val Gln Cys Ser Val Ala Glu Tyr Pro Glu Ala Ile Thr Tyr Leu
    130                 135                 140

Leu Glu Gln Tyr Asp Arg Val Glu Ala Glu Glu Ala Arg Leu Ser Asp
145                 150                 155                 160

Leu Ile Thr Gly Phe Val Asp Pro Asn Ala Glu Glu Asp Leu Ala Pro
                165                 170                 175

Thr Ala Thr His Val Gly Ser Glu Leu Ser Gln Glu Asp Leu Asp Asp
            180                 185                 190

Asp Glu Asp Glu Asp Glu Glu Asp Gly Asp Asp Asp Ser Ala Asp Asp
        195                 200                 205

Asp Asn Ser Ile Asp Pro Glu Leu Ala Arg Glu Lys Phe Ala Glu Leu
    210                 215                 220

Arg Ala Gln Tyr Val Val Thr Arg Asp Thr Ile Lys Ala Lys Gly Arg
225                 230                 235                 240

Ser His Ala Thr Ala Gln Glu Glu Ile Leu Lys Leu Ser Glu Val Phe
                245                 250                 255

Lys Gln Phe Arg Leu Val Pro Lys Gln Phe Asp Tyr Leu Val Asn Ser
            260                 265                 270

Met Arg Val Met Met Asp Arg Val Arg Thr Gln Glu Arg Leu Ile Met
        275                 280                 285

Lys Leu Cys Val Glu Gln Cys Lys Met Pro Lys Lys Asn Phe Ile Thr
    290                 295                 300

Leu Phe Thr Gly Asn Glu Thr Ser Asp Thr Trp Phe Asn Ala Ala Ile
305                 310                 315                 320

Ala Met Asn Lys Pro Trp Ser Glu Lys Leu His Asp Val Ser Glu Glu
                325                 330                 335

Val His Arg Ala Leu Gln Lys Leu Gln Gln Ile Glu Glu Glu Thr Gly

340                 345                 350

Leu Thr Ile Glu Gln Val Lys Asp Ile Asn Arg Arg Met Ser Ile Gly
        355                 360                 365

Glu Ala Lys Ala Arg Arg Ala Lys Lys Glu Met Val Glu Ala Asn Leu
    370                 375                 380

Arg Leu Val Ile Ser Ile Ala Lys Lys Tyr Thr Asn Arg Gly Leu Gln
385                 390                 395                 400

Phe Leu Asp Leu Ile Gln Glu Gly Asn Ile Gly Leu Met Lys Ala Val
                405                 410                 415

Asp Lys Phe Glu Tyr Arg Arg Gly Tyr Lys Phe Ser Thr Tyr Ala Thr
            420                 425                 430

Trp Trp Ile Arg Gln Ala Ile Thr Arg Ser Ile Ala Asp Gln Ala Arg
        435                 440                 445

Thr Ile Arg Ile Pro Val His Met Ile Glu Thr Ile Asn Lys Leu Asn
    450                 455                 460

Arg Ile Ser Arg Gln Met Leu Gln Glu Met Gly Arg Glu Pro Thr Pro
465                 470                 475                 480

Glu Glu Leu Ala Glu Arg Met Leu Met Pro Glu Asp Lys Ile Arg Lys
                485                 490                 495

Val Leu Lys Ile Ala Lys Glu Pro Ile Ser Met Glu Thr Pro Ile Gly
            500                 505                 510

Asp Asp Glu Asp Ser His Leu Gly Asp Phe Ile Glu Asp Thr Thr Leu
        515                 520                 525

Glu Leu Pro Leu Asp Ser Ala Thr Thr Glu Ser Leu Arg Ala Ala Thr
    530                 535                 540

His Asp Val Leu Ala Gly Leu Thr Ala Arg Glu Ala Lys Val Leu Arg
545                 550                 555                 560

Met Arg Phe Gly Ile Asp Met Asn Thr Asp Tyr Thr Leu Glu Glu Val
                565                 570                 575

Gly Lys Gln Phe Asp Val Thr Arg Glu Arg Ile Arg Gln Ile Glu Ala
            580                 585                 590

Lys Ala Leu Arg Lys Leu Arg His Pro Ser Arg Ser Glu Val Leu Arg
        595                 600                 605

Ser Phe Leu Asp Asp
    610

<210> 168
<211> 696

<212> PRT
<213> Escherichia coli

<400> 168
Met Trp Arg Arg Leu Ile Tyr His Pro Asp Ile Asn Tyr Ala Leu Arg
1               5                   10                  15

Gln Thr Leu Val Leu Cys Leu Pro Val Ala Val Gly Leu Met Leu Gly
            20                  25                  30

Glu Leu Arg Phe Gly Leu Leu Phe Ser Leu Val Pro Ala Cys Cys Asn
        35                  40                  45

Ile Ala Gly Leu Asp Thr Pro His Lys Arg Phe Phe Lys Arg Leu Ile
    50                  55                  60

Ile Gly Ala Ser Leu Phe Ala Thr Cys Ser Leu Leu Thr Gln Leu Leu
65                  70                  75                  80

Leu Ala Lys Asp Val Pro Leu Pro Phe Leu Leu Thr Gly Leu Thr Leu
                85                  90                  95

Val Leu Gly Val Thr Ala Glu Leu Gly Pro Leu His Ala Lys Leu Leu
            100                 105                 110

Pro Ala Ser Leu Leu Ala Ala Ile Phe Thr Leu Ser Leu Ala Gly Tyr
        115                 120                 125

Met Pro Val Trp Glu Pro Leu Leu Ile Tyr Ala Leu Gly Thr Leu Trp
    130                 135                 140

Tyr Gly Leu Phe Asn Trp Phe Trp Phe Trp Ile Trp Arg Glu Gln Pro
145                 150                 155                 160

Leu Arg Glu Ser Leu Ser Leu Leu Tyr Arg Glu Leu Ala Asp Tyr Cys
                165                 170                 175

Glu Ala Lys Tyr Ser Leu Leu Thr Gln His Thr Asp Pro Glu Lys Ala
            180                 185                 190

Leu Pro Pro Leu Leu Val Arg Gln Gln Lys Ala Val Asp Leu Ile Thr
        195                 200                 205

Gln Cys Tyr Gln Gln Met His Met Leu Ser Ala Gln Asn Asn Thr Asp
    210                 215                 220

Tyr Lys Arg Met Leu Arg Ile Phe Gln Glu Ala Leu Asp Leu Gln Glu
225                 230                 235                 240

His Ile Ser Val Ser Leu His Gln Pro Glu Glu Val Gln Lys Leu Val
                245                 250                 255

Glu Arg Ser His Ala Glu Glu Val Ile Arg Trp Asn Ala Gln Thr Val
            260                 265                 270

Ala Ala Arg Leu Arg Val Leu Ala Asp Asp Ile Leu Tyr His Arg Leu

275                 280                 285

Pro Thr Arg Phe Thr Met Glu Lys Gln Ile Gly Ala Leu Glu Lys Ile
    290                 295                 300

Ala Arg Gln His Pro Asp Asn Pro Val Gly Gln Phe Cys Tyr Trp His
305                 310                 315                 320

Phe Ser Arg Ile Ala Arg Val Leu Arg Thr Gln Lys Pro Leu Tyr Ala
                325                 330                 335

Arg Asp Leu Leu Ala Asp Lys Gln Arg Arg Met Pro Leu Leu Pro Ala
            340                 345                 350

Leu Lys Ser Tyr Leu Ser Leu Lys Ser Pro Ala Leu Arg Asn Ala Gly
        355                 360                 365

Arg Leu Ser Val Met Leu Ser Val Ala Ser Leu Met Gly Thr Ala Leu
    370                 375                 380

His Leu Pro Lys Ser Tyr Trp Ile Leu Met Thr Val Leu Leu Val Thr
385                 390                 395                 400

Gln Asn Gly Tyr Gly Ala Thr Arg Leu Arg Ile Val Asn Arg Ser Val
                405                 410                 415

Gly Thr Val Val Gly Leu Ile Ile Ala Gly Val Ala Leu His Phe Lys
            420                 425                 430

Ile Pro Glu Gly Tyr Thr Leu Thr Leu Met Leu Ile Thr Thr Leu Ala
        435                 440                 445

Ser Tyr Leu Ile Leu Arg Lys Asn Tyr Gly Trp Ala Thr Val Gly Phe
    450                 455                 460

Thr Ile Thr Ala Val Tyr Thr Leu Gln Leu Leu Trp Leu Asn Gly Glu
465                 470                 475                 480

Gln Tyr Ile Leu Pro Arg Leu Ile Asp Thr Ile Ile Gly Cys Leu Ile
                485                 490                 495

Ala Phe Gly Gly Thr Val Trp Leu Trp Pro Gln Trp Gln Ser Gly Leu
            500                 505                 510

Leu Arg Lys Asn Ala His Asp Ala Leu Glu Ala Tyr Gln Glu Ala Ile
        515                 520                 525

Arg Leu Ile Leu Ser Glu Asp Pro Gln Pro Thr Pro Leu Ala Trp Gln
    530                 535                 540

Arg Met Arg Val Asn Gln Ala His Asn Thr Leu Tyr Asn Ser Leu Asn
545                 550                 555                 560

Gln Ala Met Gln Glu Pro Ala Phe Asn Ser His Tyr Leu Ala Asp Met
                565                 570                 575

Lys Leu Trp Val Thr His Ser Gln Phe Ile Val Glu His Ile Asn Ala
            580                 585                 590

Met Thr Thr Leu Ala Arg Glu His Arg Ala Leu Pro Pro Glu Leu Ala
        595                 600                 605

Gln Glu Tyr Leu Gln Ser Cys Glu Ile Ala Ile Gln Arg Cys Gln Gln
    610                 615                 620

Arg Leu Glu Tyr Asp Glu Pro Gly Ser Ser Gly Asp Ala Asn Ile Met
625                 630                 635                 640

Asp Ala Pro Glu Met Gln Pro His Glu Gly Ala Ala Gly Thr Leu Glu
                645                 650                 655

Gln His Leu Gln Arg Val Ile Gly His Leu Asn Thr Met His Thr Ile
            660                 665                 670

Ser Ser Met Ala Trp Arg Gln Arg Pro His His Gly Ile Trp Leu Ser
        675                 680                 685

Arg Lys Leu Arg Asp Ser Lys Ala
    690                 695

<210> 169
<211> 258
<212> PRT
<213> Escherichia coli

<400> 169
Met Ile Val Leu Pro Arg Arg Leu Ser Asp Glu Val Ala Asp Arg Val
1               5                   10                  15

Arg Ala Leu Ile Asp Glu Lys Asn Leu Glu Ala Gly Met Lys Leu Pro
            20                  25                  30

Ala Glu Arg Gln Leu Ala Met Gln Leu Gly Val Ser Arg Asn Ser Leu
        35                  40                  45

Arg Glu Ala Leu Ala Lys Leu Val Ser Glu Gly Val Leu Leu Ser Arg
    50                  55                  60

Arg Gly Gly Gly Thr Phe Ile Arg Trp Arg His Asp Thr Trp Ser Glu
65                  70                  75                  80

Gln Asn Ile Val Gln Pro Leu Lys Thr Leu Met Ala Asp Asp Pro Asp
                85                  90                  95

Tyr Ser Phe Asp Ile Leu Glu Ala Arg Tyr Ala Ile Glu Ala Ser Thr
            100                 105                 110

Ala Trp His Ala Ala Met Arg Ala Thr Pro Gly Asp Lys Glu Lys Ile
        115                 120                 125

Gln Leu Cys Phe Glu Ala Thr Leu Ser Glu Asp Pro Asp Ile Ala Ser

130                 135                 140

Gln Ala Asp Val Arg Phe His Leu Ala Ile Ala Glu Ala Ser His Asn
145                 150                 155                 160

Ile Val Leu Leu Gln Thr Met Arg Gly Phe Phe Asp Val Leu Gln Ser
                165                 170                 175

Ser Val Lys His Ser Arg Gln Arg Met Tyr Leu Val Pro Pro Val Phe
            180                 185                 190

Ser Gln Leu Thr Glu Gln His Gln Ala Val Ile Asp Ala Ile Phe Ala
        195                 200                 205

Gly Asp Ala Asp Gly Ala Arg Lys Ala Met Met Ala His Leu Ser Phe
    210                 215                 220

Val His Thr Thr Met Lys Arg Phe Asp Glu Asp Gln Ala Arg His Ala
225                 230                 235                 240

Arg Ile Thr Arg Leu Pro Gly Glu His Asn Glu His Ser Arg Glu Lys
                245                 250                 255

Asn Ala

<210> 170
<211> 137
<212> PRT
<213> Escherichia coli

<400> 170
Met Arg Asn Phe Asp Leu Ser Pro Leu Tyr Arg Ser Ala Ile Gly Phe
1               5                   10                  15

Asp Arg Leu Phe Asn His Leu Glu Asn Asn Gln Ser Gln Ser Asn Gly
            20                  25                  30

Gly Tyr Pro Pro Tyr Asn Val Glu Leu Val Asp Glu Asn His Tyr Arg
        35                  40                  45

Ile Ala Ile Ala Val Ala Gly Phe Ala Glu Ser Glu Leu Glu Ile Thr
    50                  55                  60

Ala Gln Asp Asn Leu Leu Val Val Lys Gly Ala His Ala Asp Glu Gln
65                  70                  75                  80

Lys Glu Arg Thr Tyr Leu Tyr Gln Gly Ile Ala Glu Arg Asn Phe Glu
                85                  90                  95

Arg Lys Phe Gln Leu Ala Glu Asn Ile His Val Arg Gly Ala Asn Leu
            100                 105                 110

Val Asn Gly Leu Leu Tyr Ile Asp Leu Glu Arg Val Ile Pro Glu Ala
        115                 120                 125

Lys Lys Pro Arg Arg Ile Glu Ile Asn
    130                 135

<210> 171
<211> 298
<212> PRT
<213> Escherichia coli

<400> 171
Met Thr Asp Leu His Thr Asp Val Glu Arg Tyr Leu Arg Tyr Leu Ser
  1               5                  10                  15

Val Glu Arg Gln Leu Ser Pro Ile Thr Leu Leu Asn Tyr Gln Arg Gln
             20                  25                  30

Leu Glu Ala Ile Ile Asn Phe Ala Ser Glu Asn Gly Leu Gln Ser Trp
         35                  40                  45

Gln Gln Cys Asp Val Thr Met Val Arg Asn Phe Ala Val Arg Ser Arg
     50                  55                  60

Arg Lys Gly Leu Gly Ala Ala Ser Leu Ala Leu Arg Leu Ser Ala Leu
 65                  70                  75                  80

Arg Ser Phe Phe Asp Trp Leu Val Ser Gln Asn Glu Leu Lys Ala Asn
                 85                  90                  95

Pro Ala Lys Gly Val Ser Ala Pro Lys Ala Pro Arg His Leu Pro Lys
            100                 105                 110

Asn Ile Asp Val Asp Asp Met Asn Arg Leu Leu Asp Ile Asp Ile Asn
        115                 120                 125

Asp Pro Leu Ala Val Arg Asp Arg Ala Met Leu Glu Val Met Tyr Gly
    130                 135                 140

Ala Gly Leu Arg Leu Ser Glu Leu Val Gly Leu Asp Ile Lys His Leu
145                 150                 155                 160

Asp Leu Glu Ser Gly Glu Val Trp Val Met Gly Lys Gly Ser Lys Glu
                165                 170                 175

Arg Arg Leu Pro Ile Gly Arg Asn Ala Val Ala Trp Ile Glu His Trp
            180                 185                 190

Leu Asp Leu Arg Asp Leu Phe Gly Ser Glu Asp Asp Ala Leu Phe Leu
        195                 200                 205

Ser Lys Leu Gly Lys Arg Ile Ser Ala Arg Asn Val Gln Lys Arg Phe
    210                 215                 220

Ala Glu Trp Gly Ile Lys Gln Gly Leu Asn Asn His Val His Pro His
225                 230                 235                 240

Lys Leu Arg His Ser Phe Ala Thr His Met Leu Glu Ser Ser Gly Asp
                245                 250                 255

Leu Arg Gly Val Gln Glu Leu Leu Gly His Ala Asn Leu Ser Thr Thr
            260                 265                 270

Gln Ile Tyr Thr His Leu Asp Phe Gln His Leu Ala Ser Val Tyr Asp
        275                 280                 285

Ala Ala His Pro Arg Ala Lys Arg Gly Lys
    290                 295

<210> 172
<211> 552
<212> PRT
<213> Escherichia coli

<400> 172
Met Leu Thr Pro Leu Thr Ala Phe Ala Gly Val Arg Leu Arg Trp Pro
1               5                   10                  15

Ala Met Met Arg Leu Thr Cys Ile Gly Ile Leu Ala Gln Phe Ala Leu
            20                  25                  30

Leu Leu Leu Ala Phe Gly Val Leu Thr Tyr Cys Phe Leu Ile Ser Asp
        35                  40                  45

Phe Ser Val Ile Tyr Val Ala Gln His Ser Tyr Ser Leu Leu Ser Trp
    50                  55                  60

Glu Leu Lys Leu Ala Ala Val Trp Gly Gly His Glu Gly Ser Leu Leu
65                  70                  75                  80

Leu Trp Val Leu Leu Leu Ser Ala Trp Ser Ala Leu Phe Ala Trp His
                85                  90                  95

Tyr Arg Gln Gln Thr Asp Pro Leu Phe Pro Leu Thr Leu Ala Val Leu
            100                 105                 110

Ser Leu Met Leu Ala Ala Leu Leu Leu Phe Val Val Leu Trp Ser Asp
        115                 120                 125

Pro Phe Val Arg Ile Phe Pro Pro Ala Ile Glu Gly Arg Asp Leu Asn
    130                 135                 140

Pro Met Leu Gln His Pro Gly Leu Ile Phe His Pro Pro Leu Leu Tyr
145                 150                 155                 160

Leu Gly Tyr Gly Gly Leu Met Val Ala Ala Ser Val Ala Leu Ala Ser
                165                 170                 175

Leu Leu Arg Gly Glu Phe Asp Gly Ala Cys Ala Arg Ile Cys Trp Arg
            180                 185                 190

Trp Ala Leu Pro Gly Trp Ser Ala Leu Thr Ala Gly Ile Ile Leu Gly

195                 200                 205

Ser Trp Trp Ala Tyr Cys Glu Leu Gly Trp Gly Gly Trp Trp Phe Trp
    210                 215                 220

Asp Pro Val Glu Asn Ala Ser Leu Leu Pro Trp Leu Ser Ala Thr Ala
225                 230                 235                 240

Leu Leu His Ser Leu Ser Leu Thr Arg Gln Arg Gly Ile Phe Cys His
                245                 250                 255

Trp Ser Leu Leu Leu Ala Ile Val Thr Leu Met Leu Ser Leu Leu Gly
            260                 265                 270

Thr Leu Ile Val Arg Ser Gly Ile Leu Val Ser Val His Ala Phe Ala
        275                 280                 285

Leu Asp Asn Val Arg Ala Val Pro Leu Phe Ser Leu Phe Ala Leu Ile
    290                 295                 300

Ser Leu Ala Ser Leu Ala Leu Tyr Gly Trp Arg Ala Arg Asp Gly Gly
305                 310                 315                 320

Pro Ala Val His Phe Ser Gly Leu Ser Arg Glu Met Leu Ile Leu Ala
                325                 330                 335

Thr Leu Leu Leu Phe Cys Ala Val Leu Leu Ile Val Leu Val Gly Thr
            340                 345                 350

Leu Tyr Pro Met Ile Tyr Gly Leu Leu Gly Trp Gly Arg Leu Ser Val
        355                 360                 365

Gly Ala Pro Tyr Phe Asn Arg Ala Thr Leu Pro Phe Gly Leu Leu Met
    370                 375                 380

Leu Val Val Ile Val Leu Ala Thr Phe Val Ser Gly Lys Arg Val Gln
385                 390                 395                 400

Leu Pro Ala Leu Val Ala His Ala Gly Val Leu Leu Phe Ala Ala Gly
                405                 410                 415

Val Val Val Ser Ser Val Ser Arg Gln Glu Ile Ser Leu Asn Leu Gln
            420                 425                 430

Pro Gly Gln Gln Val Thr Leu Ala Gly Tyr Thr Phe Arg Phe Glu Cys
        435                 440                 445

Leu Asp Leu Gln Ala Lys Gly Asn Tyr Thr Ser Glu Lys Ala Ile Val
    450                 455                 460

Ala Leu Phe Asp His Gln Gln Arg Ile Gly Glu Leu Thr Pro Glu Arg
465                 470                 475                 480

Arg Phe Tyr Glu Ala Arg Arg Gln Gln Met Met Glu Pro Ser Ile Arg
                485                 490                 495

Trp Asn Gly Ile His Asp Trp Tyr Ala Val Met Gly Glu Lys Thr Gly
            500                 505                 510

Pro Asp Arg Tyr Ala Phe Arg Leu Tyr Val Gln Ser Gly Val Arg Trp
        515                 520                 525

Ile Trp Gly Gly Gly Leu Leu Met Ile Ala Gly Ala Leu Leu Ser Gly
    530                 535                 540

Trp Arg Gly Lys Lys Arg Asp Glu
545                 550

<210> 173
<211> 149
<212> PRT
<213> Escherichia coli

<400> 173
Met Lys Lys Ile Ala Phe Gly Cys Asp His Val Gly Phe Ile Leu Lys
  1               5                  10                  15

His Glu Ile Val Ala His Leu Val Glu Arg Gly Val Glu Val Ile Asp
             20                  25                  30

Lys Gly Thr Trp Ser Ser Glu Arg Thr Asp Tyr Pro His Tyr Ala Ser
         35                  40                  45

Gln Val Ala Leu Ala Val Ala Gly Gly Glu Val Asp Gly Gly Ile Leu
     50                  55                  60

Ile Cys Gly Thr Gly Val Gly Ile Ser Ile Ala Ala Asn Lys Phe Ala
 65                  70                  75                  80

Gly Ile Arg Ala Val Val Cys Ser Glu Pro Tyr Ser Ala Gln Leu Ser
                 85                  90                  95

Arg Gln His Asn Asp Thr Asn Val Leu Ala Phe Gly Ser Arg Val Val
            100                 105                 110

Gly Leu Glu Leu Ala Lys Met Ile Val Asp Ala Trp Leu Gly Ala Gln
        115                 120                 125

Tyr Glu Gly Gly Arg His Gln Gln Arg Val Glu Ala Ile Thr Ala Ile
    130                 135                 140

Glu Gln Arg Arg Asn
145

<210> 174
<211> 304
<212> PRT
<213> Escherichia coli

<400> 174

Met Thr Val Pro Thr Tyr Asp Lys Phe Ile Glu Pro Val Leu Arg Tyr
1               5                   10                  15

Leu Ala Thr Lys Pro Glu Gly Ala Ala Ala Arg Asp Val His Glu Ala
            20                  25                  30

Ala Ala Asp Ala Leu Gly Leu Asp Asp Ser Gln Arg Ala Lys Val Ile
        35                  40                  45

Thr Ser Gly Gln Leu Val Tyr Lys Asn Arg Ala Gly Trp Ala His Asp
    50                  55                  60

Arg Leu Lys Arg Ala Gly Leu Ser Gln Ser Leu Ser Arg Gly Lys Trp
65                  70                  75                  80

Cys Leu Thr Pro Ala Gly Phe Asp Trp Val Ala Ser His Pro Gln Pro
                85                  90                  95

Met Thr Glu Gln Glu Thr Asn His Leu Ala Phe Ala Phe Val Asn Val
            100                 105                 110

Lys Leu Lys Ser Arg Pro Asp Ala Val Asp Leu Asp Pro Lys Ala Asp
        115                 120                 125

Ser Pro Asp His Glu Glu Leu Ala Lys Ser Ser Pro Asp Asp Arg Leu
    130                 135                 140

Asp Gln Ala Leu Lys Glu Leu Arg Asp Ala Val Ala Asp Glu Val Leu
145                 150                 155                 160

Glu Asn Leu Leu Gln Val Ser Pro Ser Arg Phe Glu Val Ile Val Leu
                165                 170                 175

Asp Val Leu His Arg Leu Gly Tyr Gly Gly His Arg Asp Asp Leu Gln
            180                 185                 190

Arg Val Gly Gly Thr Gly Asp Gly Gly Ile Asp Gly Val Ile Ser Leu
        195                 200                 205

Asp Lys Leu Gly Leu Glu Lys Val Tyr Val Gln Ala Lys Arg Trp Gln
    210                 215                 220

Asn Thr Val Gly Arg Pro Glu Leu Gln Ala Phe Tyr Gly Ala Leu Ala
225                 230                 235                 240

Gly Gln Lys Ala Lys Arg Gly Val Phe Ile Thr Thr Ser Gly Phe Thr
                245                 250                 255

Ser Gln Ala Arg Asp Phe Ala Gln Ser Val Glu Gly Met Val Leu Val
            260                 265                 270

Asp Gly Glu Arg Leu Val His Leu Met Ile Glu Asn Glu Val Gly Val
        275                 280                 285

Ser Ser Arg Leu Leu Lys Val Pro Lys Leu Asp Met Asp Tyr Phe Glu
    290                 295                 300

<210> 175
<211> 642
<212> PRT
<213> Saccharomyces cerevisiae

<400> 175
Met Ser Lys Ala Val Gly Ile Asp Leu Gly Thr Thr Tyr Ser Cys Val
1               5                   10                  15

Ala His Phe Ala Asn Asp Arg Val Asp Ile Ile Ala Asn Asp Gln Gly
            20                  25                  30

Asn Arg Thr Thr Pro Ser Phe Val Ala Phe Thr Asp Thr Glu Arg Leu
        35                  40                  45

Ile Gly Asp Ala Ala Lys Asn Gln Ala Ala Met Asn Pro Ser Asn Thr
    50                  55                  60

Val Phe Asp Ala Lys Arg Leu Ile Gly Arg Asn Phe Asn Asp Pro Glu
65                  70                  75                  80

Val Gln Ala Asp Met Lys His Phe Pro Phe Lys Leu Ile Asp Val Asp
                85                  90                  95

Gly Lys Pro Gln Ile Gln Val Glu Phe Lys Gly Glu Thr Lys Asn Phe
            100                 105                 110

Thr Pro Glu Gln Ile Ser Ser Met Val Leu Gly Lys Met Lys Glu Thr
        115                 120                 125

Ala Glu Ser Tyr Leu Gly Ala Lys Val Asn Asp Ala Val Val Thr Val
    130                 135                 140

Pro Ala Tyr Phe Asn Asp Ser Gln Arg Gln Ala Thr Lys Asp Ala Gly
145                 150                 155                 160

Thr Ile Ala Gly Leu Asn Val Leu Arg Ile Ile Asn Glu Pro Thr Ala
                165                 170                 175

Ala Ala Ile Ala Tyr Gly Leu Asp Lys Lys Gly Lys Glu Glu His Val
            180                 185                 190

Leu Ile Phe Asp Leu Gly Gly Gly Thr Phe Asp Val Ser Leu Leu Phe
        195                 200                 205

Ile Glu Asp Gly Ile Phe Glu Val Lys Ala Thr Ala Gly Asp Thr His
    210                 215                 220

Leu Gly Gly Glu Asp Phe Asp Asn Arg Leu Val Asn His Phe Ile Gln
225                 230                 235                 240

Glu Phe Lys Arg Lys Asn Lys Lys Asp Leu Ser Thr Asn Gln Arg Ala
                245                 250                 255

Leu Arg Arg Leu Arg Thr Ala Cys Glu Arg Ala Lys Arg Thr Leu Ser
            260                 265                 270

Ser Ser Ala Gln Thr Ser Val Glu Ile Asp Ser Leu Phe Glu Gly Ile
        275                 280                 285

Asp Phe Tyr Thr Ser Ile Thr Arg Ala Arg Phe Glu Glu Leu Cys Ala
    290                 295                 300

Asp Leu Phe Arg Ser Thr Leu Asp Pro Val Glu Lys Val Leu Arg Asp
305                 310                 315                 320

Ala Lys Leu Asp Lys Ser Gln Val Asp Glu Ile Val Leu Val Gly Gly
                325                 330                 335

Ser Thr Arg Ile Pro Lys Val Gln Lys Leu Val Thr Asp Tyr Phe Asn
            340                 345                 350

Gly Lys Glu Pro Asn Arg Ser Ile Asn Pro Asp Glu Ala Val Ala Tyr
        355                 360                 365

Gly Ala Ala Val Gln Ala Ala Ile Leu Thr Gly Asp Glu Ser Ser Lys
    370                 375                 380

Thr Gln Asp Leu Leu Leu Leu Asp Val Ala Pro Leu Ser Leu Gly Ile
385                 390                 395                 400

Glu Thr Ala Gly Gly Val Met Thr Lys Leu Ile Pro Arg Asn Ser Thr
                405                 410                 415

Ile Ser Thr Lys Lys Phe Glu Ile Phe Ser Thr Tyr Ala Asp Asn Gln
            420                 425                 430

Pro Gly Val Leu Ile Gln Val Phe Glu Gly Glu Arg Ala Lys Thr Lys
        435                 440                 445

Asp Asn Asn Leu Leu Gly Lys Phe Glu Leu Ser Gly Ile Pro Pro Ala
    450                 455                 460

Pro Arg Gly Val Pro Gln Ile Glu Val Thr Phe Asp Val Asp Ser Asn
465                 470                 475                 480

Gly Ile Leu Asn Val Ser Ala Val Glu Lys Gly Thr Gly Lys Ser Asn
                485                 490                 495

Lys Ile Thr Ile Thr Asn Asp Lys Gly Arg Leu Ser Lys Glu Asp Ile
            500                 505                 510

Glu Lys Met Val Ala Glu Ala Glu Lys Phe Lys Glu Glu Asp Glu Lys
        515                 520                 525

Glu Ser Gln Arg Ile Ala Ser Lys Asn Gln Leu Glu Ser Ile Ala Tyr
    530                 535                 540

Ser Leu Lys Asn Thr Ile Ser Glu Ala Gly Asp Lys Leu Glu Gln Ala
545                 550                 555                 560

Asp Lys Asp Thr Val Thr Lys Lys Ala Glu Glu Thr Ile Ser Trp Leu
                565                 570                 575

Asp Ser Asn Thr Thr Ala Ser Lys Glu Glu Phe Asp Asp Lys Leu Lys
            580                 585                 590

Glu Leu Gln Asp Ile Ala Asn Pro Ile Met Ser Lys Leu Tyr Gln Ala
        595                 600                 605

Gly Gly Ala Pro Gly Gly Ala Ala Gly Gly Ala Pro Gly Gly Phe Pro
    610                 615                 620

Gly Gly Ala Pro Pro Ala Pro Glu Ala Glu Gly Pro Thr Val Glu Glu
625                 630                 635                 640

Val Asp

<210> 176
<211> 306
<212> PRT
<213> Saccharomyces cerevisiae

<400> 176
Met Ser Asp Glu Ile Val Trp Gln Val Ile Asn Gln Ser Phe Cys Ser
  1               5                  10                  15

His Arg Ile Lys Ala Pro Asn Gly Gln Asn Phe Cys Arg Asn Glu Tyr
             20                  25                  30

Asn Val Thr Gly Leu Cys Thr Arg Gln Ser Cys Pro Leu Ala Asn Ser
         35                  40                  45

Lys Tyr Ala Thr Val Lys Cys Asp Asn Gly Lys Leu Tyr Leu Tyr Met
     50                  55                  60

Lys Thr Pro Glu Arg Ala His Thr Pro Ala Lys Leu Trp Glu Arg Ile
 65                  70                  75                  80

Lys Leu Ser Lys Asn Tyr Thr Lys Ala Leu Gln Gln Ile Asp Glu His
                 85                  90                  95

Leu Leu His Trp Ser Lys Phe Phe Arg His Lys Cys Lys Gln Arg Phe
            100                 105                 110

Thr Lys Leu Thr Gln Val Met Ile Thr Glu Arg Arg Leu Ala Leu Arg
        115                 120                 125

Glu Glu Glu Arg His Tyr Val Gly Val Ala Pro Lys Val Lys Arg Arg
    130                 135                 140

Glu Gln Asn Arg Glu Arg Lys Ala Leu Val Ala Ala Lys Ile Glu Lys
145                 150                 155                 160

Ala Ile Glu Lys Glu Leu Met Asp Arg Leu Lys Ser Gly Ala Tyr Gly
                165                 170                 175

Asp Lys Pro Leu Asn Val Asp Glu Lys Val Trp Lys Lys Ile Met Gly
            180                 185                 190

Gln Met Glu Glu Glu Asn Ser Gln Asp Glu Glu Glu Asp Trp Asp Glu
        195                 200                 205

Glu Glu Glu Ser Asp Asp Gly Glu Val Glu Tyr Val Ala Asp Asp Gly
    210                 215                 220

Glu Gly Glu Tyr Val Asp Val Asp Asp Leu Glu Lys Trp Leu Ala Asp
225                 230                 235                 240

Ser Asp Arg Glu Ala Ser Ser Ala Ser Gln Ser Glu Ser Asp Ser Glu
                245                 250                 255

Ser Glu Ser Asp Ser Asp Ser Asp Glu Glu Asn Lys Asn Ser Ala Lys
            260                 265                 270

Arg Arg Lys Lys Gly Thr Ser Ala Lys Thr Lys Arg Pro Lys Val Glu
        275                 280                 285

Ile Glu Tyr Glu Glu Glu His Glu Val Gln Asn Ala Glu Gln Glu Val
    290                 295                 300

Ala Gln
305


<210> 177
<211> 622
<212> PRT
<213> Saccharomyces cerevisiae

<400> 177
Met Val Arg Arg Trp Ile Pro Ser Gly Arg His Leu Arg Asn Asn Asp
1               5                   10                  15

Asn Thr Gly Asp Asp Asp Asp Ser Glu Phe Thr Asn Ser Met Asp Ser
            20                  25                  30

Gly Met Ser Ile Pro Ser Leu Arg Asp Ser Met Thr Thr Arg Ser Ser
        35                  40                  45

His Asn Asp Pro Ile Lys Pro Ala Leu Met Asn Asp Ser Asn Lys Val
    50                  55                  60

Lys Asn Leu Glu Lys Glu Leu Thr Asn Ala Lys Ile Lys Ile Gln Val
65                  70                  75                  80

Leu Tyr Glu Tyr Ile Arg Arg Ile Pro Asn Lys Asp Gly Asn Ala Pro

                85                  90                  95

Ser Leu Gly Asn Asp Thr Asp Phe Arg Asn Ser Ile Ile Glu Gly Leu
            100                 105                 110

Asn Leu Glu Ile Asn Lys Leu Lys Gln Asp Leu Lys Ala Lys Glu Val
        115                 120                 125

Glu Tyr Gln Asp Thr Leu Gln Phe Val Gln Glu Asn Leu Glu Asn Ser
    130                 135                 140

Glu Ser Ile Val Asn Thr Ile Asn His Leu Leu Ser Phe Ile Leu Thr
145                 150                 155                 160

His Phe Asn Glu Gln Asp Glu Asn Ala His Leu Leu Asp Lys Glu Glu
                165                 170                 175

Arg Glu Thr Leu Glu Glu Thr Leu Glu Leu Ser Ser Asp Tyr Val Leu
            180                 185                 190

Glu Lys Met Asp Thr Leu Ser Lys Phe Ile Ile Gln Phe Leu Gln Asp
        195                 200                 205

Phe Leu His Ser Lys Ser Arg Ala Glu Ser Lys Gln Asp Lys Glu Glu
    210                 215                 220

Phe Leu Ser Leu Ala Gln Ser Ser Pro Ala Gly Ser Gln Leu Glu Ser
225                 230                 235                 240

Arg Asp Ser Pro Ser Ser Lys Glu Glu Asn Thr Asp Gly Gly Tyr Gln
                245                 250                 255

Asn Asp Glu Ile His Asp Ser Asn Asn His Ile Asp Thr Glu Asn Val
            260                 265                 270

Met Ala Asn Ser Thr Ser Leu Pro Ile Ser Ala Val Glu Ser Arg Phe
        275                 280                 285

Glu Lys Thr Leu Asp Thr Gln Leu Glu Ile Val Ile Glu Ile Leu His
    290                 295                 300

Lys Glu Tyr Asp Gln Phe Ile Asn Ser Ile Arg Leu Lys Phe Glu Lys
305                 310                 315                 320

Ser Gln Lys Leu Glu Lys Ile Ile Ala Ser Lys Leu Asn Glu Gln Ser
                325                 330                 335

His Leu Leu Asp Ser Leu Glu Leu Glu Glu Asn Ser Ser Ser Val Ile
            340                 345                 350

Glu Lys Gln Asp His Leu Ile Ser Gln Leu Lys Glu Lys Ile Glu Ser
        355                 360                 365

Gln Ser Val Leu Ile Asn Asn Leu Glu Lys Leu Lys Glu Asp Ile Ile
    370                 375                 380

Lys Met Lys Gln Asn Glu Lys Val Leu Thr Lys Glu Leu Glu Thr Gln
385                 390                 395                 400

Thr Lys Ile Asn Lys Leu Lys Glu Asn Asn Trp Asp Ser Tyr Ile Asn
                405                 410                 415

Asp Leu Glu Lys Gln Ile Asn Asp Leu Gln Ile Asp Lys Ser Glu Glu
            420                 425                 430

Phe His Val Ile Gln Asn Gln Leu Asp Lys Leu Asp Leu Glu Asn Tyr
        435                 440                 445

Gln Leu Lys Asn Gln Leu Asn Thr Leu Asp Asn Gln Lys Leu Ile Leu
    450                 455                 460

Ser Gln Tyr Glu Ser Asn Phe Ile Lys Phe Asn Gln Asn Leu Leu Leu
465                 470                 475                 480

His Leu Asp Ser Ile Phe Asn Ile Leu Gln Lys Ile Leu Gln Glu Ser
                485                 490                 495

Ser Ile Ala Gln Phe Asp Arg Lys Met Lys Ser Ile Lys Ser Val Pro
            500                 505                 510

Asn Ala Leu Lys Asn Leu Asn Leu Ile Gln Pro Lys Leu Glu Ser Leu
        515                 520                 525

Tyr Thr Phe Ile Glu Thr Ala Leu Glu Ser Ile Ile Asn Ser Tyr Ile
    530                 535                 540

Ser Ser Leu Ile Ser Met Glu Thr Pro Glu Gln Pro His Gln Gln Gly
545                 550                 555                 560

Asn Glu Leu Thr Ala Thr Pro Asn Lys Glu Leu Thr Leu Arg Ile Glu
                565                 570                 575

Glu Leu Gln Arg Arg Trp Ile Ser Glu Arg Glu Arg Arg Lys Leu Asp
            580                 585                 590

Ala Asn Ala Ser Glu Ala Arg Ile Lys Ala Leu Glu Gln Glu Asn Glu
        595                 600                 605

Ser Leu Arg Ser Lys Leu Phe Asn Leu Ser Ile Asn Asn Pro
    610                 615                 620

<210> 178
<211> 234
<212> PRT
<213> Saccharomyces cerevisiae

<400> 178
Met Gln Thr Pro Ser Glu Asn Thr Asp Val Lys Met Asp Thr Leu Asp
1               5                   10                  15

Glu Pro Ser Ala His Leu Ile Glu Glu Asn Val Ala Leu Pro Glu Asp

20                  25                  30

Thr Phe Ser Ser His Leu Ser Tyr Val Leu Tyr Glu Ile Ala His Cys
        35                  40                  45

Lys Pro Ile Met Phe Met Ile Ile Ile Ile Val Ser Leu Ile Ser Leu
    50                  55                  60

Ile Val Leu Phe His Asp Asn Asp Gly Cys Thr Val Ile Leu Val Met
65                  70                  75                  80

Ser Leu Ile Val Ala Ser Met Ala Leu Met Val Val Ala Ala Phe Thr
                85                  90                  95

Phe Gly Lys Ala Ile Thr Glu Gln Glu Phe Met Ile Lys Leu Leu Val
            100                 105                 110

Glu Val Ile Ala Arg Lys Pro Ala Gly Lys Glu Trp Gly Thr Val Ala
        115                 120                 125

Tyr Asn Met Asn Gln Tyr Leu Phe Met Lys Arg Leu Trp Tyr Thr Pro
    130                 135                 140

Tyr Tyr Phe Tyr Ser Gly Lys Lys Cys His Glu Phe Phe Thr Thr Leu
145                 150                 155                 160

Ile Lys Glu Val Asn Ser Gly Ser His Ser Asp Ser Ser Ser Asn Ser
                165                 170                 175

Ala Glu Asp Thr Gln Ser Pro Val Ser Ala Gly Lys Thr Ser Asn Gly
            180                 185                 190

Leu Asn Asn Phe Tyr Ser Ile Arg Ser Asp Pro Ile Leu Met Ala Tyr
        195                 200                 205

Val Leu Lys Ala Thr Gln Ile Glu Lys Glu Ala Gln Ser Glu Tyr Trp
    210                 215                 220

Arg Lys Gln Tyr Pro Asp Ala Asp Leu Pro
225                 230

<210> 179
<211> 161
<212> PRT
<213> Saccharomyces cerevisiae

<400> 179
Ala Thr Ser Thr Val Val Val Ser Cys Ser Glu Thr Thr Asp Ala Ser
1               5                   10                  15

Gly Asn Ile Tyr Thr Ile Thr Thr Thr Val Pro Cys Ser Ser Thr Thr
            20                  25                  30

Ala Thr Ile Thr Ser Cys Asp Glu Asn Gly Cys His Val Pro Ala Pro
        35                  40                  45

Thr Ala Thr Asp Ala Thr Ala Thr Val Ser Ser Lys Ser Tyr Thr Thr
    50                  55                  60

Val Thr Val Thr His Cys Asp Asn Asn Gly Cys Asn Thr Lys Thr Val
65                  70                  75                  80

Thr Ser Glu Cys Ser Lys Glu Thr Ala Ala Thr Thr Ile Ser Pro Lys
                85                  90                  95

Ser Tyr Thr Thr Val Thr Val Thr His Cys Asp Asp Asn Gly Cys Asn
            100                 105                 110

Thr Lys Thr Val Thr Ser Glu Ala Ser Lys Gln Thr Ser Leu Ala Thr
        115                 120                 125

Ser Thr Val Thr Lys Ser Ala Ala Pro Thr Ser His Thr Ala Ala Ser
    130                 135                 140

Ser Thr Phe Thr Gly Ile Val Val Gln Ser Glu Gly Met Ala Ala Gly
145                 150                 155                 160

Leu

<210> 180
<211> 144
<212> PRT
<213> Saccharomyces cerevisiae

<400> 180

<210> 181
<211> 104
<212> PRT
<213> Saccharomyces cerevisiae

<400> 181
Met Leu Phe Lys Pro Lys Thr Arg Ala Ile Pro Ser Pro Thr Ala Arg
1               5                   10                  15

Thr Leu Pro Val Ser Phe Lys Leu Ala Ser Ser Asp Thr Pro Leu Ile
            20                  25                  30

Leu Ser Ser Lys Met Glu Glu Thr Ser Val Gly Cys Ala Leu Val Glu
        35                  40                  45

Ala Asn Leu Leu Val Glu Ala Lys Ala Ala Ala Ala Gly Leu Ala Ala
    50                  55                  60

Leu Val Glu Leu Ile Arg Val Leu Asp Arg Glu Arg Ile Ala Ala Val
65                  70                  75                  80

Arg Ala Asn Ile Ile Ile Cys Ala Cys Phe Phe Tyr Leu Phe Cys Tyr
                85                  90                  95

Cys Ser Cys Asp Ser Tyr Glu Ser
            100

<210> 182
<211> 111
<212> PRT
<213> Saccharomyces cerevisiae

<400> 182
Met Ser Leu Arg Pro Cys Leu Thr Pro Ser Ser Met Gln Tyr Ser Asp
1               5                   10                  15

Ile Tyr Ile Tyr Pro Asn Thr Thr Leu Thr Leu Pro Tyr Phe Asn Pro
            20                  25                  30

Ser Asn Leu Ser Leu Asn Leu Pro Ser His Tyr Pro Thr Ser Pro Leu
        35                  40                  45

Val Thr Leu Ser His Ser Thr Ile Pro Leu Pro Thr Thr Ile His Pro
    50                  55                  60

Ser Thr Tyr Tyr His Gln Ser Thr Val His His Asn Arg Tyr Pro Pro
65                  70                  75                  80

Ile Ser His Ile Gln Leu His Tyr His Leu Pro Cys His Tyr Ser Thr
                85                  90                  95

Ile His His Leu Leu Leu Thr Ile Leu Leu Phe Tyr Pro Pro Tyr
            100                 105                 110

<210> 183
<211> 433
<212> PRT
<213> Saccharomyces cerevisiae

<400> 183
Met Ile Val Gly Leu Thr Leu Tyr Phe Val Leu Phe Arg Ser Ile Gln
  1               5                  10                  15

Tyr Leu Leu Val Phe Leu Thr Pro Ile Arg Gln Phe Asp Thr Ser Thr
             20                  25                  30

Ser Leu Leu Leu Asn Glu Leu Cys Ser Ser Pro Ser Glu Ile Asn Ser
         35                  40                  45

Tyr Trp Asn Lys Tyr Phe Trp Asn Lys Leu Leu Ser Trp Asp Ser Val
     50                  55                  60

Phe Phe Ile Lys Asn Ile Thr Ser Lys Asn Gly Lys Pro Gln Phe Glu
 65                  70                  75                  80

His Glu Tyr Ala Phe Ser Gln Leu Trp Thr Phe Phe Val Arg Leu Phe
                 85                  90                  95

Ile Lys Ser Asn Asn Asp Ser Ile Tyr His Ala Leu Arg Val Gly Val
            100                 105                 110

Ala Ile Glu Asn Val Leu Phe Tyr Leu Ser Gly Ile Val Leu Tyr Phe
        115                 120                 125

Leu Thr Lys Lys Ile Phe Ser Gln Asn Ile Arg Gln Ser Gln Phe Ala
    130                 135                 140

Arg Thr Ile Ala Lys Lys Thr Ser Leu Leu Phe Phe Leu Thr Ser Ala
145                 150                 155                 160

Ala Gly Phe Leu Thr Ser Ile Tyr Ser Glu Pro Leu Ser Phe Phe Phe
                165                 170                 175

Ala Phe Val Gly Ile Trp Ser Arg Glu Cys Thr Ile Ser Val Pro Val
            180                 185                 190

Leu Gly Gln Phe Asp Ile Ser Trp Arg Tyr Trp Phe Pro Tyr Ser Phe
        195                 200                 205

Ile Ser Met Ala Cys Phe Thr Leu Ala Ser Leu Asn Arg Ser Asn Cys
    210                 215                 220

Val Leu Leu Gly Ile Tyr Phe Ile Phe Asp Leu Ile Glu Leu Thr Lys

225                 230                 235                 240

Asn Arg Lys Phe Val Lys Ala Ile Cys Phe Pro Leu Leu Ser Gly Ser
                245                 250                 255

Leu Met Phe Ser Ala Leu Leu Tyr Gln Gln Tyr Tyr Leu Pro Tyr Lys
            260                 265                 270

Thr Phe Cys Pro Gln Arg Gly Glu Trp Cys Lys Ser Gln Leu Phe Ser
        275                 280                 285

Ser Ile Phe Ile Thr Lys Thr Ser Leu Tyr Ser Tyr Ile Gln Ser His
    290                 295                 300

Tyr Trp Gly Val Gly Leu Leu Lys Tyr Trp Thr Pro Asn Asn Ile Pro
305                 310                 315                 320

Asn Phe Leu Phe Ala Val Pro Asn Ile Ile Ile Leu Ile Tyr Ser Ser
                325                 330                 335

Ile Tyr Phe Ser Lys Ile Tyr Pro Ser Tyr Asn Leu Lys Ala Leu Val
            340                 345                 350

Trp Ile Thr Arg Ala Leu Val Val Ile Val Cys Phe Phe Ala His Val
        355                 360                 365

Gln Ile Leu Asn Arg Ile Ala Ser Phe Leu Pro Leu His Leu Trp Tyr
    370                 375                 380

Leu Ala Asp Arg Leu Val Lys Thr Ser Asp Pro Lys Lys Met Glu Asn
385                 390                 395                 400

Pro Lys Gly Asp Asp Lys Ile Val Lys Phe Tyr Ile Tyr Trp Leu Ala
                405                 410                 415

Phe Trp Ile Pro Leu Gln Thr Ile Leu Phe Ala Ala Phe Leu Pro Pro
            420                 425                 430

Ala

<210> 184
<211> 290
<212> PRT
<213> Saccharomyces cerevisiae

<400> 184
Met Glu Lys Leu Leu Gln Trp Ser Ile Ala Asn Ser Gln Gly Asp Lys
1               5                   10                  15

Glu Ala Met Ala Arg Ala Gly Gln Pro Asp Pro Lys Leu Leu Gln Gln
            20                  25                  30

Leu Phe Gly Gly Gly Gly Pro Asp Asp Pro Thr Leu Met Lys Glu Ser
        35                  40                  45

Met Ala Val Ile Met Asn Pro Glu Val Asp Leu Glu Thr Lys Leu Val
    50                  55                  60

Ala Phe Asp Asn Phe Glu Met Leu Ile Glu Asn Leu Asp Asn Ala Asn
65                  70                  75                  80

Asn Ile Glu Asn Leu Lys Leu Trp Glu Pro Leu Leu Asp Val Leu Val
                85                  90                  95

Gln Thr Lys Asp Glu Glu Leu Arg Ala Ala Ala Leu Ser Ile Ile Gly
            100                 105                 110

Thr Ala Val Gln Asn Asn Leu Asp Ser Gln Asn Asn Phe Met Lys Tyr
        115                 120                 125

Asp Asn Gly Leu Arg Ser Leu Ile Glu Ile Ala Ser Asp Lys Thr Lys
    130                 135                 140

Pro Leu Asp Val Arg Thr Lys Ala Phe Tyr Ala Leu Ser Asn Leu Ile
145                 150                 155                 160

Arg Asn His Lys Asp Ile Ser Glu Lys Phe Phe Lys Leu Asn Gly Leu
                165                 170                 175

Asp Cys Ile Ala Pro Val Leu Ser Asp Asn Thr Ala Lys Pro Lys Leu
            180                 185                 190

Lys Met Arg Ala Ile Ala Leu Leu Thr Ala Tyr Leu Ser Ser Val Lys
        195                 200                 205

Ile Asp Glu Asn Ile Ile Ser Val Leu Arg Lys Asp Gly Val Ile Glu
    210                 215                 220

Ser Thr Ile Glu Cys Leu Ser Asp Glu Ser Asn Leu Asn Ile Ile Asp
225                 230                 235                 240

Arg Val Leu Ser Phe Leu Ser His Leu Ile Ser Ser Gly Ile Lys Phe
                245                 250                 255

Asn Glu Gln Glu Leu His Lys Leu Asn Glu Gly Tyr Lys His Ile Glu
            260                 265                 270

Pro Leu Lys Asp Arg Leu Asn Glu Asp Asp Tyr Leu Ala Val Lys Tyr
        275                 280                 285

Val Leu
    290

<210> 185
<211> 344
<212> PRT
<213> Saccharomyces cerevisiae

<400> 185

Met His Cys Pro Ile Cys His His Arg Ala His Val Val Tyr Cys Ala
1               5                   10                  15

His Cys Ile Asn Thr Ser Pro Ser Leu Leu Leu Lys Leu Lys Leu Asp
            20                  25                  30

Leu Ile Leu Leu Lys Asp Glu Asn Lys Glu Leu Asn Gly Lys Val Glu
        35                  40                  45

Gln Ile Leu Asn Glu Ala Met Asn Tyr Asp Gln Leu Asp Ile Lys Arg
    50                  55                  60

Met Glu Lys Lys Lys Asp Pro Leu Met Asn Ser Leu Met Lys Leu Asp
65                  70                  75                  80

Val Leu Arg Met Lys Lys Asn Asn Asn Leu Ile Arg His Arg Ile Glu
                85                  90                  95

Gln Leu Asn Glu Arg Ile Tyr Ser Lys Arg Asn His Ile Ser Glu Leu
            100                 105                 110

Lys Val Glu Ile Asp Asn Tyr Lys Cys Tyr Lys Val Gly Thr Gly Thr
        115                 120                 125

Asp Lys Leu Arg Glu Gln Val Glu Ile Ser Asp Ala Lys Asn Lys Leu
    130                 135                 140

Ala Gln Val Ser Lys Ile Cys Glu Ser Ala Arg Asp Tyr Lys Leu Asn
145                 150                 155                 160

Leu Leu Asn Asn Trp Phe Val Ile Gln Lys Leu Gln Asp Asn Phe Gln
                165                 170                 175

Ile Pro Phe Ala Ile Ala Phe Gln Pro Leu Ile Ser Leu Lys Asn Phe
            180                 185                 190

Arg Ile Leu Pro Leu Ala Ile Thr Asn Asp Ser Ile Asn Ile Met Trp
        195                 200                 205

Lys Tyr Ile Ser Phe Phe Ser Asp Ile Leu Met Ile Lys Leu Pro Tyr
    210                 215                 220

Thr Asn Lys Ile Cys Glu Gln Pro Met Phe Glu Phe Ser Asp Ser Ile
225                 230                 235                 240

Gln Thr Val Val Gln Arg Leu Ile Lys Leu Ile Ile Asn Ile Leu Gln
                245                 250                 255

Ile Cys Arg His Leu Lys Leu Val Pro Ser Thr Pro Met Asp Ile Pro
            260                 265                 270

Trp Leu Leu Asp Gln Tyr Asp Val Asp Gly Leu Phe Tyr Asn Met Val
        275                 280                 285

Lys Arg Asn Lys Met Lys Cys Arg Ser Val Ser Leu Tyr Trp Thr Phe
    290                 295                 300

Gly Met Leu Tyr Ser Met Val Leu Asp Asn Met Asn Asn Pro Gln Arg
305                 310                 315                 320

Gly His Pro Ala Arg Arg Thr Ala Pro Pro Pro Thr Val Thr Gly Pro
                325                 330                 335

His Asp Arg Trp Tyr Val Val Gly
            340

<210> 186
<211> 312
<212> PRT
<213> Saccharomyces cerevisiae

<400> 186
Met Asn Ile Met Lys Arg Gln Leu Cys Thr Ser Ser Lys Arg Phe Phe
1               5                   10                  15

Ser Thr Ala Lys Asn Val Val Lys Tyr Asn Thr Ile Gln Asp Ile Arg
            20                  25                  30

Asn Lys Tyr Phe Thr Gly Thr Pro Leu Ser Met Cys Thr Ala Tyr Asp
        35                  40                  45

Phe Ile Thr Ala Thr Trp Val Asn Lys Ala Asn Cys Asp Leu Leu Leu
    50                  55                  60

Val Gly Asp Ser Leu Ala Met Thr Ser Leu Gly Tyr Asp Ser Thr Ile
65                  70                  75                  80

Thr Leu Ser Leu Asn Glu Phe Lys Tyr His Val Ala Ser Val Cys Arg
                85                  90                  95

Ala Glu Gly Ser Ser Met Val Val Val Asp Met Pro Phe Gly Thr Phe
            100                 105                 110

Glu Ser Gly Ile Ser Asp Gly Leu Lys Asn Ala Ile Asp Ile Met Lys
        115                 120                 125

Leu Asp Ser Lys Val Thr Ser Val Lys Val Glu Val Gly Ser Tyr Thr
    130                 135                 140

Lys Asp Lys Tyr Ala Met Lys Phe Ile Glu Glu Leu Cys Ser Arg Gly
145                 150                 155                 160

Ile Pro Val Met Ala His Ile Gly Leu Thr Pro Gln Lys Val His Ser
                165                 170                 175

Leu Gly Gly Tyr Lys Val Gln Gly Ser Lys Ser Leu Leu Gln Met Gln
            180                 185                 190

Glu Leu Tyr Glu Thr Ala Met Gln Leu Gln Lys Ile Gly Cys Trp Ser
        195                 200                 205

Ile Leu Ile Glu Cys Val Pro His Lys Met Ala Gln Phe Ile Thr Ser
    210                 215                 220

Lys Leu Ser Val Pro Thr Ile Gly Ile Gly Ala Gly Asn Gly Thr Ser
225                 230                 235                 240

Gly Gln Val Leu Val Ile Ser Asp Leu Leu Gly Met Gln Gly Asp Ser
                245                 250                 255

Val Pro Lys Phe Val Lys Gln Ala Val Asn Met Thr Asp Ile Ala Thr
            260                 265                 270

Gln Gly Leu Lys Glu Tyr Ile Ala Ser Val Glu Asp Arg Thr Phe Pro
        275                 280                 285

Glu Arg Gly Thr His Thr Phe Lys Val Lys Glu Asp Leu Trp Asn Glu
    290                 295                 300

Phe Leu Ser Ser Ile Asn Glu Lys
305                 310

<210> 187
<211> 375
<212> PRT
<213> Saccharomyces cerevisiae

<400> 187
Met Asn Met Ala Glu Arg Ala Glu Ala Thr Lys Ser Trp Ser Cys Glu
  1               5                  10                  15

Pro Leu Ser Gly Lys Thr Leu Glu Glu Ile Val Gln Asn Ala Glu Asn
             20                  25                  30

Ala Ala Asp Leu Val Ala Tyr Ile Arg Lys Pro Glu Val Asp Leu Asp
         35                  40                  45

Phe Arg Leu Lys Phe Ile Ala Glu His Glu Glu Phe Phe Asn Glu Gln
     50                  55                  60

Leu Ser Asp Arg Asn Ser Arg Ile Arg Thr Cys His Asn Leu Ser Asp
 65                  70                  75                  80

Lys Gly Ile Arg Gly Asp Thr Val Phe Val Phe Val Pro Gly Leu Ala
                 85                  90                  95

Gly Asn Leu Glu Gln Phe Glu Pro Leu Leu Glu Leu Val Asp Ser Asp
            100                 105                 110

Gln Lys Ala Phe Leu Thr Leu Asp Leu Pro Gly Phe Gly His Ser Ser
        115                 120                 125

Glu Trp Ser Asp Tyr Pro Met Leu Lys Val Val Glu Leu Ile Phe Val
    130                 135                 140

Leu Val Cys Asp Val Leu Arg Lys Trp Ser Thr Ala Val Pro Asn Asn

145                 150                 155                 160

Asp Asn Val Asn Pro Phe Asn Gly His Lys Ile Val Leu Val Gly His
                165                 170                 175

Ser Met Gly Cys Phe Leu Ala Cys His Leu Tyr Glu Gln His Met Ala
            180                 185                 190

Asp Thr Lys Ala Val Gln Thr Leu Val Leu Leu Thr Pro Pro Lys Ala
        195                 200                 205

His Ile Glu Gln Leu Ser Lys Asp Lys His Ile Ile Gln Trp Ala Leu
    210                 215                 220

Tyr Gly Val Phe Lys Leu Pro Trp Leu Phe Asp Val Tyr Arg Asn Lys
225                 230                 235                 240

Phe Asp Gln Val Lys Gly Leu Gln Ser Ser Gly Ile Lys Gln Tyr Phe
                245                 250                 255

Tyr Gln Gln Gly Asp Asp Val Lys Leu Lys Tyr Arg Lys Phe Trp Gln
            260                 265                 270

Phe Lys Asn Asn Ile Ser Asn Lys Ser Arg Thr Ile Ile Gly Tyr Leu
        275                 280                 285

Leu Gly Trp Glu Thr Val Asp Trp Val Lys Phe Asn Gly Val Leu Thr
    290                 295                 300

Gln Thr Asp Met Lys Gln Lys Ile Ile Ile Phe Gly Ala Glu Lys Asp
305                 310                 315                 320

Pro Ile Ala Pro Ile Glu Asn Leu Glu Phe Tyr Lys Gln Thr Ile Asn
                325                 330                 335

Lys Glu Cys Leu Arg Lys Val Ile Ile Leu Pro Asp Cys Ser His Asn
            340                 345                 350

Leu Cys Phe Asp Arg Pro Glu Leu Val Cys Glu Asn Phe Gln Arg Glu
        355                 360                 365

Val Ile Asp Asn Ser Lys Leu
    370                 375

<210> 188
<211> 448
<212> PRT
<213> Saccharomyces cerevisiae

<400> 188
Met Leu Arg Leu Phe Ser Leu Ala Leu Ile Thr Cys Leu Ile Tyr Tyr
1               5                   10                  15

Ser Lys Asn Gln Gly Pro Ser Ala Leu Val Ala Ala Val Gly Phe Gly
            20                  25                  30

Ile Ala Gly Tyr Leu Ala Thr Asp Met Leu Ile Pro Arg Val Gly Lys
        35                  40                  45

Ser Phe Ile Lys Ile Gly Leu Phe Gly Lys Asp Leu Ser Lys Pro Gly
    50                  55                  60

Arg Pro Val Leu Pro Glu Thr Ile Gly Ala Ile Pro Ala Ala Val Tyr
65                  70                  75                  80

Leu Phe Val Met Phe Ile Tyr Ile Pro Phe Ile Phe Tyr Lys Tyr Met
                85                  90                  95

Val Ile Thr Thr Ser Gly Gly Gly His Arg Asp Val Ser Val Val Glu
            100                 105                 110

Asp Asn Gly Met Asn Ser Asn Ile Phe Pro His Asp Lys Leu Ser Glu
        115                 120                 125

Tyr Leu Ser Ala Ile Leu Cys Leu Glu Ser Thr Val Leu Leu Gly Ile
    130                 135                 140

Ala Asp Asp Leu Phe Asp Leu Arg Trp Arg His Lys Phe Phe Leu Pro
145                 150                 155                 160

Ala Ile Ala Ala Ile Pro Leu Leu Met Val Tyr Tyr Val Asp Phe Gly
                165                 170                 175

Val Thr His Val Leu Ile Pro Gly Phe Met Glu Arg Trp Leu Lys Lys
            180                 185                 190

Thr Ser Val Asp Leu Gly Leu Trp Tyr Tyr Val Tyr Met Ala Ser Met
        195                 200                 205

Ala Ile Phe Cys Pro Asn Ser Ile Asn Ile Leu Ala Gly Val Asn Gly
    210                 215                 220

Leu Glu Val Gly Gln Cys Ile Val Leu Ala Ile Leu Ala Leu Leu Asn
225                 230                 235                 240

Asp Leu Leu Tyr Phe Ser Met Gly Pro Leu Ala Thr Arg Asp Ser His
                245                 250                 255

Arg Phe Ser Ala Val Leu Ile Ile Pro Phe Leu Gly Val Ser Leu Ala
            260                 265                 270

Leu Trp Lys Trp Asn Arg Trp Pro Ala Thr Val Phe Val Gly Asp Thr
        275                 280                 285

Tyr Cys Tyr Phe Ala Gly Met Val Phe Ala Val Val Gly Ile Leu Gly
    290                 295                 300

His Phe Ser Lys Thr Met Leu Leu Leu Phe Ile Pro Gln Ile Val Asn
305                 310                 315                 320

Phe Ile Tyr Ser Cys Pro Gln Leu Phe Lys Leu Val Pro Cys Pro Arg

                325                 330                 335

His Arg Leu Pro Lys Phe Asn Glu Lys Asp Gly Leu Met Tyr Pro Ser
            340                 345                 350

Arg Ala Asn Leu Lys Glu Glu Pro Pro Lys Ser Ile Phe Lys Pro Ile
        355                 360                 365

Leu Lys Leu Leu Tyr Cys Leu His Leu Ile Asp Leu Glu Phe Asp Glu
    370                 375                 380

Asn Asn Glu Ile Ile Ser Thr Ser Asn Met Thr Leu Ile Asn Leu Thr
385                 390                 395                 400

Leu Val Trp Phe Gly Pro Met Arg Glu Asp Lys Leu Cys Asn Thr Ile
                405                 410                 415

Leu Lys Leu Gln Phe Cys Ile Gly Ile Leu Ala Leu Leu Gly Arg His
            420                 425                 430

Ala Ile Gly Ala Ile Ile Phe Gly His Asp Asn Leu Trp Thr Val Arg
        435                 440                 445

<210> 189
<211> 687
<212> PRT
<213> Saccharomyces cerevisiae

<400> 189
Met Lys Pro Pro Leu Asn Met Ser Arg Ser Asn Lys Pro Leu Thr Gln
1               5                   10                  15

Glu Ala Asn Ser Ser Ala His Ile Asp Arg Ala His Gln Leu Ala Gln
            20                  25                  30

Asp Phe Asn Ser Lys Gln Asp Asp Thr Ala Leu Thr Ser Leu Pro His
        35                  40                  45

Lys Asn Pro Asp Ile Phe Arg Phe Glu Asn Asn Ile Thr Ala His Ser
    50                  55                  60

Ser Arg Arg Gly Ser Leu Tyr Arg Asp Ser Asp Ala Thr Val Val Leu
65                  70                  75                  80

Pro Leu Ser Glu His Thr Pro Arg Leu Ser Met Asp Asp Pro Tyr Arg
                85                  90                  95

Gln Leu Leu Gln Gln Ala Glu Ile Ser Gln Leu Arg Ser Lys Lys Lys
            100                 105                 110

Arg His Ser Ser Arg Val Leu Arg Thr Ser Phe Ile Ser Phe Val Val
        115                 120                 125

Leu Val Ser Ser Leu Ser Gly Leu Asp Gln Gly Leu Ile Ser Gly Asn
    130                 135                 140

Val Met Thr Leu Ser Phe Gln Lys Tyr Phe His Tyr Pro Leu Thr Ser
145                 150                 155                 160

Pro Leu Gly Asn Ile Val Ser Ile Val Asn Leu Gly Ala Phe Met Ala
                165                 170                 175

Ser Leu Phe Val Tyr Ser Gly Ile Leu Glu Pro Cys Ser Arg Lys Lys
            180                 185                 190

Met Leu Gln Ile Ser Thr Met Ile Tyr Ser Leu Gly Ala Ile Val Gln
        195                 200                 205

Val Leu Ala Leu Asn Gln Trp Cys Leu Leu Leu Gly Arg Phe Leu Leu
    210                 215                 220

Gly Val Gly Met Gly Phe Ala Phe Ser Met Val Ile Ile Tyr Gln Phe
225                 230                 235                 240

Glu Phe Pro Leu Pro Cys Ile Arg Lys Arg Thr Leu Ile Ser Ile Gln
                245                 250                 255

Cys Val Ser Ser Val Ile Ala Tyr Ser Phe Gly Ile Trp Ile Asn Cys
            260                 265                 270

Ala Phe Arg Tyr Leu Gly Phe Ala Trp Arg Tyr Pro Leu Ser Thr His
        275                 280                 285

Val Ala Leu Gly Ile Ile Leu Asn Leu Met Ser Phe Tyr Leu Ile Leu
    290                 295                 300

Glu Ser Pro Ser Trp Leu Leu Lys Gln Lys Asn Asp Val Glu Ala Leu
305                 310                 315                 320

Val Leu Ile Ser Asn Val Phe Asp Asp Gly Asn Phe Glu Glu Asn Gln
                325                 330                 335

Thr Gln Leu Lys Phe Arg Val Leu Lys Arg Asp Ile Leu Leu Lys Ser
            340                 345                 350

His Leu Gln Lys Asn Ser Tyr Pro Tyr Ala Tyr Ile Leu Lys Asp Phe
        355                 360                 365

Ser Ser Ile Ile Lys Leu Leu Ile Gly Phe Gln Leu Leu Thr Arg Thr
    370                 375                 380

Asn Gly Val Asp Ala Phe Leu Tyr Tyr Ser Pro Leu Ile Leu Gln Gln
385                 390                 395                 400

Met Gly Arg Gly Glu Arg Lys Ser Ile Tyr Leu Thr Gly Leu Asn Ala
                405                 410                 415

Leu Ile Tyr Ser Ile Val Ile Leu Ala Tyr Val Pro Leu Val Leu Arg

            420                 425                 430

Lys Arg Lys Glu Lys Thr Asn Val Leu Leu Gly Ser Ile Val Met Cys
        435                 440                 445

Ala Leu Leu Phe Thr Ile Ser Phe Thr Asp Trp Phe Pro Lys Ser Thr
    450                 455                 460

Thr Arg Tyr Ile Ser Ile Leu Phe Ala Val Phe Leu Phe Thr His Phe
465                 470                 475                 480

Ile Ser Trp Asp Ser Ile Gly Trp Val Met Thr Ile Glu Leu Leu Pro
                485                 490                 495

His Leu Ser Gln Ala Pro Val Ile Leu Leu Val Ser Asn Phe Tyr Trp
            500                 505                 510

Ile Phe Lys Trp Phe Val Ser Leu Ile Thr Pro Ile Leu Ile Asp Arg
        515                 520                 525

Leu Ser Trp Lys Phe Tyr Leu Ile Pro Ser Leu Ser Ser Phe Ile Ser
    530                 535                 540

Ile Ile Phe Val Leu Lys Ile Phe Pro Ile Glu Thr Arg Asp Glu Arg
545                 550                 555                 560

Leu Asp Ser Asp Asp Asp Ser Thr Gly Asn Gly Ser Gly Asn His Asp
                565                 570                 575

Asp Val Phe Asp Asp Thr Gly Ser Glu Phe Ser Ser Ser Pro Ser Phe
            580                 585                 590

Ser Ala Tyr Gln Ile Asn Thr Leu Gly Ser Ser Ile Lys Gln Asn Asn
        595                 600                 605

Gln Ala Tyr Ser Ser Ile Gln Asn Glu Gln Ile Leu Pro Lys Asn Gly
    610                 615                 620

Asn Leu Ser Asn Gln Thr His Gly Ser Ala Gln Asn Val Tyr Phe Ile
625                 630                 635                 640

Thr Ser Asp Ser Gly Pro Ser Arg Thr Gly Glu Phe Phe Ser Phe His
                645                 650                 655

Asn Arg Thr Asp Pro Asn Ile Ser Asp Asn Ile Ala Ala Asn Lys Pro
            660                 665                 670

Ser Ser Gly Gly Gly Gln Asn Ser Pro Gly Asp Met Ala Val Ala
        675                 680                 685

<210> 190
<211> 210
<212> PRT
<213> Saccharomyces cerevisiae

<400> 190
Met Phe Ser Tyr Ser Asp Phe Cys Ser Ile Gly Thr Ala Met Ile Leu
1               5                   10                  15

Ser Ala Thr Thr Phe Leu Met Gly Val Phe Phe Ser Asn Met Pro Tyr
            20                  25                  30

Asp Tyr His Leu Leu Phe Asn Pro Asn Ser Thr Gln Glu His Phe Asp
        35                  40                  45

Leu Ala Leu Arg His Tyr Gln Ile Leu His Glu Thr Pro Leu Pro Val
    50                  55                  60

Ile Val Thr Leu Cys Val Val Ala Gly Ile Gly Leu Val Gly Gly Thr
65                  70                  75                  80

Ile Lys Val Phe Lys Pro Asn Pro Glu Leu Gln Met Phe Glu Tyr Cys
                85                  90                  95

Ser Leu Gly Leu Tyr Val Leu Ala Ile Cys Val Phe Leu Thr Asn Val
            100                 105                 110

Lys Thr Gly Ile Asp Cys Ser Val Ser His Asn Trp Gly Glu Val Thr
        115                 120                 125

Glu Asn Gln Gly Leu Ala Val Ile Ala Ser Ser Asn Ile Ile Leu Leu
    130                 135                 140

Val Met Phe Ala Gly Val Ile Ile Leu Gln Ile Gly Leu Trp Tyr Ser
145                 150                 155                 160

Asn Trp Asp Leu Gln Lys Arg Leu Lys Glu Phe Tyr Ala Gln Glu Glu
                165                 170                 175

Arg Glu Ala Ala Asn Ala Gly Lys Lys Thr Glu Lys Val Asp Asn Ala
            180                 185                 190

Lys Lys Asn Asp Asn Lys Ser Lys Gly Ala Gln Lys Arg Lys Asn Ala
        195                 200                 205

Lys Lys
    210


<210> 191
<211> 117
<212> PRT
<213> Saccharomyces cerevisiae

<400> 191
Met Asn Leu Glu Glu Ser Gln Ser Asn Arg Gly Thr Leu Asn Ile Lys
1               5                   10                  15

Lys Ile Leu Val Ile Val Cys Met Thr Asn Lys Ile Cys Lys His Leu
            20                  25                  30

Ile Gln Ser Leu Leu Leu Ser Lys Arg Arg Leu Val Ser Val Leu Phe
        35                  40                  45

Asp Asn Tyr Gln Gly Asp Met Lys Leu Arg Ser Ser Pro Ser Ala Ile
    50                  55                  60

Pro Leu Val Pro Ser Thr Lys Ile Leu Gln Ala Met Glu Ala Arg Asn
65                  70                  75                  80

Asp Ile Val Asp Ser Leu Ile Ile Asn Ser His Ala Thr Glu Ser Asp
                85                  90                  95

Arg Arg Thr Leu Gly Asp His Glu Val Arg Gln Cys Glu Asp Thr Asn
            100                 105                 110

Phe Gly Phe Lys Ile
        115

<210> 192
<211> 383
<212> PRT
<213> Saccharomyces cerevisiae

<400> 192
Met Lys Glu Asn Glu Val Lys Asp Glu Lys Ser Val Asp Val Leu Ser
1               5                   10                  15

Phe Lys Gln Leu Glu Ser Gln Lys Ile Val Leu Pro Gln Asp Leu Phe
            20                  25                  30

Arg Ser Ser Phe Thr Trp Phe Cys Tyr Glu Ile Tyr Lys Ser Leu Ala
        35                  40                  45

Phe Arg Ile Trp Met Leu Leu Trp Leu Pro Leu Ser Val Trp Trp Lys
    50                  55                  60

Leu Ser Asn Asn Cys Ile Tyr Pro Leu Ile Val Ser Leu Leu Val Leu
65                  70                  75                  80

Phe Leu Gly Pro Ile Phe Val Leu Val Ile Cys Gly Leu Ser Arg Lys
                85                  90                  95

Arg Ser Leu Ser Lys Gln Leu Ile Gln Phe Cys Lys Glu Ile Thr Glu
            100                 105                 110

Asn Thr Pro Ser Ser Asp Pro His Asp Trp Glu Val Val Ala Asn
        115                 120                 125

Leu Asn Ser Tyr Leu Tyr Glu Asn Lys Ala Trp Asn Thr Lys Asn Phe
        130                 135                 140

Phe Phe Asn Ala Thr Asp Cys Glu Lys Met Phe Arg Thr Thr Val Leu
145 150 155 160

Glu Pro Phe Ser Leu Lys Lys Asp Lys Ala Ala Lys Val Lys Ser Phe

165                 170                 175

Lys Asp Ser Val Pro Tyr Ile Glu Glu Ala Leu Gln Val Tyr Ala Thr
            180                 185                 190

Gly Phe Asp Lys Gln Trp Lys Leu Phe Ile Thr Glu Lys Ser Trp Ser
        195                 200                 205

Pro Val Gly Leu Glu Asp Val Gln Leu Pro Lys Asp Ile His Arg Ser
    210                 215                 220

Lys Leu Thr Trp Phe Leu Lys Arg Ile Phe Thr Ile Tyr Ser Leu Pro
225                 230                 235                 240

Leu Trp Leu Ala Phe Leu Asn Cys Ile Cys Val Ser Gln His Phe Cys
                245                 250                 255

Leu Ala Phe Arg Ile Leu Cys Pro Gly Leu Phe Phe Leu Met Met Val
            260                 265                 270

Trp Leu Phe Gln Asn Met Arg Thr Thr Ala Leu Leu Val Lys Met Glu
        275                 280                 285

His Lys Met Gln Phe Leu Leu Thr Ile Ile Asn Glu Gln Glu Ser Gly
    290                 295                 300

Ala Asn Gly Trp Asp Glu Ile Ala Arg Lys Met Asn Arg Tyr Leu Phe
305                 310                 315                 320

Glu Lys Lys Ala Trp Lys Asn Glu Glu Phe Phe Phe Asp Gly Ile Asp
                325                 330                 335

Cys Glu Trp Phe Phe Ser His Phe Phe Tyr Arg Leu Leu Ser Ala Lys
            340                 345                 350

Lys Ser Met Trp Leu Leu Pro Leu Asn Val Glu Leu Trp Pro Tyr Ile
        355                 360                 365

Lys Glu Ala Gln Leu Ser Arg Asn Glu Glu Ser Leu Met Lys Lys
    370                 375                 380


<210> 193
<211> 302
<212> PRT
<213> Saccharomyces cerevisiae

<400> 193
Met Thr Val Ile Lys Thr Glu Pro Thr Thr Glu Val Thr Leu Tyr Ser
1               5                   10                  15

Pro Pro Ser Lys Glu Ser Leu Ser Lys Asp Asp Ala His Arg Lys Lys
            20                  25                  30

Gln Asn Asn Lys Pro Pro Ser Ser Ile Asn Ser Arg Ser Gly Pro Asn
        35                  40                  45

Lys His Lys Leu Ala Ala Lys Ala Pro Glu Lys Lys Ile Asn Asn Thr
    50                  55                  60

Asp Lys Gln Asp Leu Ser Ala Phe Leu Leu Asn Pro Ser Leu Ile Val
65                  70                  75                  80

Lys Pro Ser Glu Ser Lys Lys Lys Glu Asn Ile Val Ala Tyr Asn Asp
                85                  90                  95

Thr Pro Gly Ile Lys Thr Glu His Thr Ala Phe Gln Pro Leu Thr Pro
            100                 105                 110

Ile Ser Lys Lys Arg Ala Leu Lys Glu Lys Ala Ala Ser Glu Lys Cys
        115                 120                 125

Asp Ser Phe Asp Leu Ser Arg Asp Glu Lys Pro Tyr Ile Gln Lys Lys
    130                 135                 140

Ser Lys Thr Leu Ser Ser Val Thr Glu Ile Asn Ser Ser Glu Tyr Lys
145                 150                 155                 160

Leu Ser Leu Asn Gly Glu Asn Thr Ser Ser Pro Ala Lys Glu Lys Ser
                165                 170                 175

Gln Glu Pro Ile Glu Asn Pro Gly Ser Tyr Gln Lys Thr Arg Asn Tyr
            180                 185                 190

Leu Phe Glu Lys Pro Asp Pro Leu Asp Thr Cys Leu Gln Asp Tyr Ser
        195                 200                 205

Ser Met Leu Pro Ser Asn Val Ala Glu Glu Asp Gln Glu Tyr Phe Ile
    210                 215                 220

Ser Val Ala Asp Ser Thr Leu Glu Glu Trp Thr Asn Lys Gly Gln Glu
225                 230                 235                 240

Ile Ile Asp Gln Gln Phe Gln Leu Tyr Gln Glu Ile Ile Lys Lys Arg
                245                 250                 255

Ile Glu Leu Ser Tyr Lys Phe Lys Gly Ile Ile Ser Val Ile Asn Asp
            260                 265                 270

Arg Ala Asp Ala Leu Glu Glu Gln Gly Gln Gln Leu Glu Gly Lys Ile
        275                 280                 285

Lys Lys Val Lys Thr Leu Ala Asn Glu Ile Leu Asn Ile Ile
    290                 295                 300

<210> 194
<211> 108
<212> PRT
<213> Saccharomyces cerevisiae

<400> 194

Met Ile Tyr Met Leu Val Phe Leu Asp Arg Gln Gln Leu Val His Ile
1               5                   10                  15

Phe Leu Phe Arg Ser Arg Gly Thr Thr Asn Ile Ile Lys Ala Cys Tyr
            20                  25                  30

Phe Phe Phe Leu Leu Phe Cys Lys Leu Leu Asn Ala Ala Glu Ala Pro
        35                  40                  45

Leu Leu Ala Ile Ser Leu Ser Lys Phe Val Trp Leu Leu Leu Arg Val
    50                  55                  60

Cys Lys Thr Ser Tyr Leu Leu Leu Leu Ile Thr Met Leu Glu Gly Ala
65                  70                  75                  80

Glu Tyr Phe Ser Leu Val Val Gly Asn Ser Ile Cys Gly Ser Gly Gly
                85                  90                  95

Glu Gly Val Gly Cys Arg Tyr Pro Val Val Leu Ile
            100                 105

<210> 195
<211> 224
<212> PRT
<213> Saccharomyces cerevisiae

<400> 195
Met Trp Asp Ser Leu Ile Val Ser Ile Asn Asp Thr His Lys Leu Gly
1               5                   10                  15

Leu Glu Asp Cys Leu Ala Val Phe Gly His Val Pro Ile Thr Lys Ala
            20                  25                  30

Val Lys His Val Arg Leu Thr Glu Ile Asp Thr Gln Thr Ser Thr Phe
        35                  40                  45

Thr Leu Lys Phe Leu His Thr Glu Thr Gly Gln Asn Ile Glu Lys Ile
    50                  55                  60

Ile Tyr Phe Ile Asp Asn Asp Thr Gly Asn Asp Thr Arg Thr Ala Thr
65                  70                  75                  80

Gly Ile Lys Gln Ile Phe Asn Lys Met Phe Arg Ile Ala Ala Glu Lys
                85                  90                  95

Arg Lys Leu Ser Leu Ile Gln Ile Asp Thr Val Glu Tyr Pro Cys Thr
            100                 105                 110

Leu Val Asp Leu Leu Ile Leu Val Gly Val Ala Leu Pro Pro Leu Cys
        115                 120                 125

Tyr Leu Tyr Arg Pro Ala Leu His Ala Ile Phe Phe Leu Val Pro Asn
    130                 135                 140

Pro Val Gly Ser Thr Leu Glu Ala Trp Leu Asp Ser Asp Leu Val Leu

145                 150                 155                 160

Arg Leu Ile Ile Val Ala Glu Phe Leu Thr His Ala Leu Glu Thr Leu
                165                 170                 175

Ile Phe Val Val Pro Arg Leu Lys Tyr Tyr Arg Val Pro Gly Glu Phe
            180                 185                 190

Val Pro Glu Trp Leu Leu Leu Gly Leu Leu Glu Gly Tyr Gly Pro Ala
        195                 200                 205

Arg Arg Leu Asp Thr Lys Ala Arg Thr Leu Gly Glu Gly Ser Val Asn
    210                 215                 220

<210> 196
<211> 482
<212> PRT
<213> Saccharomyces cerevisiae

<400> 196
   Met Asp Phe Tyr Thr Thr Asp Ile Asn Lys Asn Val Val Pro Leu Phe
     1               5                  10                  15
   Ser Lys Gly Thr Val Ala Arg Thr Ala Ser Lys Ala Gln Tyr Pro Ser
                20                  25                  30
   Trp Cys Asn Asn Ala Leu Lys Leu Thr Asn Ile Leu Leu Lys Ser Leu
            35                  40                  45
   Arg Cys Lys Phe Gln Thr Asn Arg Cys Glu Asp Asp Arg Gly Phe Glu
        50                  55                  60
   Val Tyr Cys Val Ile Leu Lys Ser Ile Ala Leu Leu Met Ala Ala Lys
    65                  70                  75                  80
   Glu Ser Leu Ile Leu Leu Gln Ile Pro Pro Ser Leu Pro Ser Gly Phe
                    85                  90                  95
   Pro Phe Arg Ser Pro Gln Leu Ser Phe Thr Tyr Leu Ser Thr Arg Leu
               100                 105                 110
   Ser Gly Ser Gln His Lys Ser Thr His Ser His His Ile Asn His Gln
           115                 120                 125
   Thr His Pro Ile His Ser Ser Ser Ser Asn Ser Asn Ser Asn Asn Arg
       130                 135                 140
   Ile Pro Thr Lys Thr Asp Ser Ser Lys Gln His Thr Gln His Phe Ser
   145                 150                 155                 160
   Phe Ala Asn Ala Gly Ala Ser Asn Arg Asp Glu Leu Leu Ser Ile Val
                   165                 170                 175
   Arg Lys Ile Asp Lys Ser Asn Leu Lys Cys Cys Asp Cys Gly Ser Thr
   180 185 190
   Ala Thr Val Glu Trp Val Ser Ile Asn Leu Leu Cys Ile Leu Cys Ile
   195 200 205
   Lys Cys Ser Gly Val His Arg Ser Leu Gly Ser His Ile Ser Lys Ile
   210 215 220
   Arg Ser Leu Thr Leu Asp Asn Phe Thr Ser Leu Glu Leu Met His Leu
   225 230 235 240
   Leu Gln Asn Asn Val Ser Asn Ser Asn Val Asn Ala Ile Tyr Glu Ser
   245 250 255
   Asn Leu Arg Asn Phe Pro Val Lys Lys Ile Thr Ala Asn Ser Asp Asp
   260 265 270
   Ser Glu Arg Ser Lys Phe Ile Ile Asp Lys Tyr Gln Phe Lys Lys Phe
   275 280 285
   Val Ile Asp Ser Asn Gln Gly Arg Glu Ala Ser Leu Lys Ser Leu Ile
   290 295 300
   Lys Ala Ile His Leu Asp Ser Val Phe Met Met Gln Arg Ala Ile Ala
   305 310 315 320
   Gln Ser Lys Tyr Ser Leu Arg Glu Leu Thr Ala Ser Glu Lys Glu Gln
   325 330 335
   Asn Asp Leu Asn His Ser Ser Ile Phe Gln Tyr Ser Leu Lys His Tyr
   340 345 350
   Glu Ile Val Asp Gly Thr Pro Ile Phe Phe Ile Thr Glu Phe Leu Leu
   355 360 365
   Cys Asn Gly Ile His Ile Asp Asn Leu Pro Lys Ile Thr Thr Asn Trp
   370 375 380
   Ser Pro Lys Val Leu Glu Tyr Trp Glu Thr Lys Leu Glu Met Tyr Gly
   385 390 395 400
   Thr Phe Gln Ala Val Asn Thr Ser Arg Pro Arg Ser Gly Pro His Leu
   405 410 415
   Asn Met His Ser Asn Val Asp Ser Ala Ser Ser Tyr Asn Lys Lys His
   420 425 430
   Asp Leu Arg Val Asn Ile Pro Glu Arg Ser Ala Ser Ala Ser Lys Arg
   435 440 445
   Trp Ser Leu Ser Ser Ile Pro Lys Ser Ser Gln Asn Leu Met Ser Pro
   450 455 460
   Thr Asn Leu Leu Thr Met His Lys Ser Leu Lys Leu Ala Lys Lys Asp
   465                 470                 475                 480
   Lys Lys

<210> 197
<211> 156
<212> PRT
<213> Saccharomyces cerevisiae

<400> 197
His Ser Ala Thr Ile Val Met Asp Leu Leu Gly Asp Ile Val Glu Lys
1               5                   10                  15

Asp Thr Ser Asp Ser Val Glu Ser Asn Asp Asn Gly Thr Leu Ser Thr
            20                  25                  30

Asn Asn Cys Gly Thr Gly Phe Pro Glu Leu Tyr Lys Pro Lys Lys Ile
        35                  40                  45

Ser Ser Trp Lys Glu Arg Leu Arg Glu Lys Arg Ala Gln Lys Lys Lys
    50                  55                  60

Thr Ser Gly Lys Asp Ala Glu Lys Gln Gln Thr Ser Thr Asp Ala Pro
65                  70                  75                  80

Leu Ser Glu Ala Lys Ser Ile His Asn Glu Asn Ile Lys Val Leu Gln
                85                  90                  95

Gly Met Ser Asp Glu Gln Ile Val Gln Glu Arg Glu Asp Leu Tyr Asn
            100                 105                 110

Ser Leu Asp Pro Lys Leu Ile Ala Lys Leu Leu Lys Asn Ile Asn Lys
        115                 120                 125

Arg Ala Lys Asp Glu Asn Asn Thr Pro Leu Phe Ala Glu Ile Glu Gly
    130                 135                 140

Ala Ser Gly Thr Trp Val Gly Gly Asn Lys Gln Gly
145                 150                 155

<210> 198
<211> 87
<212> PRT
<213> Saccharomyces cerevisiae

<400> 198
Tyr Arg Pro Lys Pro Cys Pro Lys Ala Ala Phe Cys Ser Trp Lys Arg
1               5                   10                  15

Cys Pro Thr Arg Ser Leu Cys Phe Lys Asn Phe Ser Glu His Phe Met
            20                  25                  30

Met His Val Cys Ser Leu Leu Val Ser Phe Asp Val Val Lys Ser Leu

35                  40                  45

Thr Gln Ser Val Lys His Leu Ser Thr Arg Leu Glu Tyr Lys Arg Met
    50                  55                  60

Lys Ser Phe Ile Cys Phe Cys Ser Thr Thr Phe Trp Asn Ser Cys Cys
65                  70                  75                  80

Ser Leu Glu Phe Asn Ala Ser
                85


<210> 199
<211> 102
<212> PRT
<213> Saccharomyces cerevisiae

<400> 199
Met Ser Leu Ser Phe Leu Leu Phe Ser Pro Phe Leu Pro Pro Cys Phe
1               5                   10                  15

Ser Ser Ile Ser Ile Cys Leu Ser Val Leu Ser Thr Val Ser Phe Phe
            20                  25                  30

Phe Ala Phe Thr Ile Pro His Tyr Val Leu Arg Cys Gly Ser Val Asp
        35                  40                  45

Glu Trp His Ile His Ser Ser Ala Glu Asp Phe Arg Thr Gln Arg Cys
    50                  55                  60

Val Cys Ala Val Lys Leu Ser Ala Ser Leu Leu Gly Cys Leu Leu Ala
65                  70                  75                  80

Cys Ala Ser Trp Ser Leu Leu Leu Glu Val Ser Arg Ile Lys Trp His
                85                  90                  95

Val Gly Thr Ala Tyr Ser
            100


<210> 200
<211> 928
<212> PRT
<213> Saccharomyces cerevisiae

<400> 200
Met Lys Asp Ser Val Asp Cys Pro Ser Ile Leu Pro Thr Asp Arg Thr
1               5                   10                  15

Ser Val Leu Ser Glu Thr Ser Thr Leu Val Gly Ser Ser Ser His Val
            20                  25                  30

Tyr Ser Arg His Ala Pro Met Asn Ser Tyr His Asn Ser Met Asn Ser
        35                  40                  45

Asn Ile Tyr His Ser Pro Lys Ala Ser Ser Pro Leu Val Ser Tyr Lys

    50                  55                  60

Thr Ser Ser Pro Val Leu Leu Lys Arg Ala Thr Ala Pro Val Leu Pro
65                  70                  75                  80

Ser Phe Lys Pro Lys Glu Gln Arg Tyr Asn Lys Pro Gln Gly Cys Ser
                85                  90                  95

Leu Ile Thr Ala Val Glu Leu Gly Lys Ile Ile Glu Thr Leu Pro Asp
            100                 105                 110

Glu Lys Val Leu Leu Leu Asp Val Arg Pro Phe Thr Glu His Ala Lys
        115                 120                 125

Ser Ile Ile Thr Asn Ser Ile His Val Cys Leu Pro Ser Thr Leu Leu
    130                 135                 140

Arg Arg Lys Asn Phe Thr Phe Ser Lys Leu Leu Asp Asn Leu Thr Pro
145                 150                 155                 160

Ser Glu Gln Ser Val Leu Lys Ser Lys Leu Ala Ile Asp Asn Leu Arg
                165                 170                 175

Ile Ile Ile Tyr Asp Ser Thr Ala Asn Gln Thr Glu Ser Ser Val Ser
            180                 185                 190

Leu Pro Cys Tyr Gly Ile Ala Ser Lys Leu Ile Glu Phe Asp Thr Asn
        195                 200                 205

Val Lys Lys Thr Val Ser Ile Leu Met Cys Gly Phe Pro Gln Phe Lys
    210                 215                 220

Ile Leu Phe Pro Asp His Ile Asn Thr Asn Thr Phe Asn Ser Asp Cys
225                 230                 235                 240

Ile Ser Ser Ala Glu Pro Lys Ser Pro Lys Thr Asn Leu Met Asn Ser
                245                 250                 255

Leu His Asn Thr Ala Pro His Met Thr Ala Thr Thr Pro Leu Ser Ser
            260                 265                 270

Pro Gln Met Asn Leu Lys Leu Lys Val Pro Asp Asp Ser Arg Ser Asp
        275                 280                 285

His Ser Asn Phe Ser Ser Ser Pro Ser Pro Arg Asn Val Leu Ser Asp
    290                 295                 300

Ser Pro Met Ser Ser Ser Ser Pro Ile Ser Ala Leu Phe Lys Phe Gln
305                 310                 315                 320

Leu Pro Ala Pro Gln Thr Asn Ile Asn Gln Met Phe Lys Phe Ser Gln
                325                 330                 335

Asn Glu Glu Ile Met Gly Leu Glu Thr Tyr Leu Ser Ala Val Asn Ile
            340                 345                 350

Lys Glu Glu His Glu Arg Trp Tyr Asn Asn Asp Ser Ala Lys Lys Ser
355 360 . 365

Leu Gln Asn Phe Gln Phe Pro Lys Asn Gln Asn Ser Leu Glu Lys Asp
370 375 380

Thr Asn Lys Asp Lys Leu Gly Phe Gln Ile Arg Tyr Glu Asn Leu Ser
385 390 395 400

Lys Asn Tyr Glu Lys Glu Val Ile Asp Ser Val Ile Pro Glu Trp Phe
405 410 415

Gln His Leu Met Ser Ile Pro Lys Ile Glu Leu Val Ser Gln Phe Gln
420 425 430

Lys Leu Asp Phe Leu Glu Lys Arg Arg Leu Asn His Ser Val Ser Phe
435 440 445

Arg Lys Lys Glu Asn Ser Phe Ile Leu Glu Lys Pro Ser Ser Tyr Pro
450 455 460

Glu Gln Leu Thr Ser Thr Ser Ser Ser Thr Ile Met Pro Pro Lys Phe
465 470 475 480

Pro Asp Val Asn Lys Val Gln Lys Arg Ser His Ser Gln Pro Ile Phe
485 490 495

Thr Gln Tyr Ser Lys Tyr Lys Ser Met Leu Ser Leu Glu Ser Asp Ser
500 505 510

Asp Ser Glu Ser Asp Asp Val Ile Ile Ser Ser Gly Val Glu Leu Gly
515 520 525

Ala Lys Asn Arg Tyr Lys Asp Ile Phe Pro Tyr Glu His Ser Arg Val
530 535 540

Ile Leu Lys Lys Gly Leu Gln Ser Ser Lys Gly Ile Lys His Ser His
545 550 555 560

Ser Thr Ser Asp Gly Gly Ile Leu Asp Asn Tyr Ile Asn Ala Asn Tyr
565 570 575

Leu Ser Leu Pro Arg Phe Ser Val Glu Gln Asn Ser Ser Phe Gln Thr
580 585 590

Thr Thr Thr Thr Thr Arg Arg Val Arg Tyr Ile Ala Thr Gln Ala Pro
595 600 605

Met Pro Ser Thr Val His Asp Phe Tyr Thr Cys Ile Leu Asn Asn Gly
610 615 620

Val Pro Leu Val Leu Ser Leu Thr Asn Asp Phe Glu Asn Gly Ile Glu
625 630 635 640

Lys Cys Tyr Arg Tyr Trp Gln Glu Gly Asn Tyr Asn Gly Ile His Val
645 650 655

Lys Leu Leu Glu Lys Lys Ile Leu Lys Met Pro Ser Thr Thr Ser Met
            660                 665                 670

Arg Lys Asn Thr Met Gly Thr Gln Asn Ser Ser Leu Tyr Ser Ala Gly
        675                 680                 685

Val Gln Gly Asn Ser Ser Asn Tyr Ser Thr Asp Asn Asp Asn Asp Asn
    690                 695                 700

Asp Asn Asn Asn Asn Asn Asn Asn Asn Ser Asn Ile Pro Val Thr Ala
705                 710                 715                 720

Ala Ala Cys Asp Asp Asp Asp Asp Asp Asp Asp Asp Ala Ile Leu Ile
                725                 730                 735

Arg Gln Ile Leu Leu Thr Tyr His Asp Gln Glu Lys Pro Tyr Glu Leu
            740                 745                 750

Leu Gln Ile Gln Val Lys Asn Trp Pro Asp Leu Gly Thr Leu Leu Asn
        755                 760                 765

Pro Ile Ser Ile Leu Gln Ala Ile Asn Val Lys Asn His Ile Ile Asp
    770                 775                 780

Thr Leu Phe Ala Arg Asn Tyr Tyr Gln Asn Asp Gln Leu Pro Thr Ile
785                 790                 795                 800

Leu Val His Cys Ser Ala Gly Cys Gly Arg Thr Gly Thr Leu Cys Thr
                805                 810                 815

Ile Asp Ser Ile Leu Ser Asn Phe Glu Met Phe Glu Met Leu Gln Lys
            820                 825                 830

Glu Phe Val Lys Leu Lys Tyr Pro Ala Lys Leu Phe Asp Pro Ile Ser
        835                 840                 845

Trp Thr Ile Asn Ile Phe Arg Lys Glu Arg Ile Ser Met Val Gln Asn
    850                 855                 860

Ile Asn Gln Phe Ile Phe Ile Tyr Asp Cys Leu Leu Phe Tyr Phe Arg
865                 870                 875                 880

Leu Arg Leu Asp Asp Ile Thr Glu Arg Thr Asp Gly Asp Gly Ser Asn
                885                 890                 895

Lys Asp Asn Ile Ser Leu Ser Ala Leu Ile Glu Gln Ile Glu Lys Leu
            900                 905                 910

Glu Ile Leu Gln Thr Phe Val Asp Asp Lys Leu Lys Glu Leu Pro Gln
        915                 920                 925

<210> 201
<211> 173
<212> PRT
<213> Saccharomyces cerevisiae

<400> 201
Met Asn Phe Lys Glu Pro Leu Val Asn Phe Leu Lys Cys Val Leu Asn
1               5                   10                  15

Asn Ile Asn Ser Ala Phe Ser His Arg Ile Asp Gln Leu Gln Leu Gln
            20                  25                  30

Leu Leu Arg Glu Thr Asn Ile Leu Arg Val Leu Asn Arg Gly Ile Glu
        35                  40                  45

Arg Leu Phe Ser Glu Asn Pro Arg Thr His Ser Val Ser Arg Ile Val
    50                  55                  60

Val Glu Lys Asp Val Gly Cys Arg Ile Gly Arg Tyr Val Glu Gly Ser
65                  70                  75                  80

Lys Tyr Glu Leu Val Glu Ser Arg Ala Lys Glu Ile Gln Ile Tyr Tyr
                85                  90                  95

Glu Arg Met Val Phe Glu Ile Thr Gln Glu Leu Lys Gly Asp Lys Arg
            100                 105                 110

Val Phe Ala Leu Ile Ala Asn Leu Ser Gln Arg Tyr Ser Ala Glu Arg
        115                 120                 125

Lys Glu Ile Arg Asn Val Lys Val His Gly Gly Arg Pro Cys Asn Glu
    130                 135                 140

Asn Glu Phe Gln Val Ile Pro Ile Arg Phe Lys Pro Ile Asn Leu Glu
145                 150                 155                 160

Arg Arg Ala Arg Leu Ile Arg Lys Lys Lys Thr Ile Asn
                165                 170


<210> 202
<211> 240
<212> PRT
<213> Saccharomyces cerevisiae

<400> 202
Met Ala Asp Glu Glu Arg Leu Lys Glu Phe Lys Glu Ala Asn Lys Ile
1               5                   10                  15

Val Phe Asp Pro Asn Thr Arg Gln Val Trp Glu Asn Gln Asn Arg Asp
            20                  25                  30

Gly Thr Lys Pro Ala Thr Thr Phe Gln Ser Glu Glu Asp Ile Lys Arg
        35                  40                  45

Ala Ala Pro Glu Ser Glu Lys Asp Thr Ser Ala Thr Ser Gly Ile Val

50                  55                  60

Pro Thr Leu Gln Asn Ile Val Ala Thr Val Thr Leu Gly Cys Arg Leu
65                  70                  75                  80

Asp Leu Lys Thr Val Ala Leu His Ala Arg Asn Ala Glu Tyr Asn Pro
                85                  90                  95

Lys Arg Phe Ala Ala Val Ile Met Arg Ile Arg Glu Pro Lys Thr Thr
            100                 105                 110

Ala Leu Ile Phe Ala Ser Gly Lys Met Val Val Thr Gly Ala Lys Ser
        115                 120                 125

Glu Asp Asp Ser Lys Leu Ala Ser Arg Lys Tyr Ala Arg Ile Ile Gln
    130                 135                 140

Lys Ile Gly Phe Ala Ala Lys Phe Thr Asp Phe Lys Ile Gln Asn Ile
145                 150                 155                 160

Val Gly Ser Cys Asp Val Lys Phe Pro Ile Arg Leu Glu Gly Leu Ala
                165                 170                 175

Phe Ser His Gly Thr Phe Ser Ser Tyr Glu Pro Glu Leu Phe Pro Gly
            180                 185                 190

Leu Ile Tyr Arg Met Val Lys Pro Lys Ile Val Leu Leu Ile Phe Val
        195                 200                 205

Ser Gly Lys Ile Val Leu Thr Gly Ala Lys Gln Arg Glu Glu Ile Tyr
    210                 215                 220

Gln Ala Phe Glu Ala Ile Tyr Pro Val Leu Ser Glu Phe Arg Lys Met
225                 230                 235                 240

<210> 203
<211> 142
<212> PRT
<213> Saccharomyces cerevisiae

<400> 203
Met Ala Asp Gln Val Pro Val Thr Thr Gln Leu Pro Pro Ile Lys Pro
1               5                   10                  15

Glu His Glu Val Pro Leu Asp Ala Gly Gly Ser Pro Val Gly Asn Met
            20                  25                  30

Gly Thr Asn Ser Asn Asn Asn Asn Glu Leu Gly Asp Val Phe Asp Arg
        35                  40                  45

Ile Lys Thr His Phe Pro Pro Ala Lys Val Lys Lys Ile Met Gln Thr
    50                  55                  60

Asp Glu Asp Ile Gly Lys Val Ser Gln Ala Thr Pro Val Ile Ala Gly
65                  70                  75                  80

Arg Ser Leu Glu Phe Phe Ile Ala Leu Leu Val Lys Lys Ser Gly Glu
                85                  90                  95

Met Ala Arg Gly Gln Gly Thr Lys Arg Ile Thr Ala Glu Ile Leu Lys
            100                 105                 110

Lys Thr Ile Leu Asn Asp Glu Lys Phe Asp Phe Leu Arg Glu Gly Leu
        115                 120                 125

Cys Val Glu Glu Gly Gln Thr Gln Pro Glu Glu Glu Ser Ala
    130                 135                 140

<210> 204
<211> 303
<212> PRT
<213> Saccharomyces cerevisiae

<400> 204
Met Ser Thr Thr Asp Ser Gly Phe Val Leu Tyr His Tyr Thr Pro Ser
1               5                   10                  15

Lys Ala Ala Ala Ile Val Phe Val Val Leu Phe Ile Ile Met Thr Val
            20                  25                  30

Ile Phe Ala Val Gln Thr Leu Tyr Ala Ala Arg Lys Ser Ser Lys Ala
        35                  40                  45

Leu Lys Asn Asn Pro Phe Glu Ser Ser Asp Asp Lys Val Asp Ser Leu
    50                  55                  60

Glu Asp Ala Glu Tyr Lys Gln Leu Lys Ile Thr Pro Thr Val Phe Ala
65                  70                  75                  80

Phe Ile Pro Phe Phe Thr Gly Cys Ile Met Glu Ala Val Gly Tyr Ile
                85                  90                  95

Gly Arg Ala Leu Ser Ser Ser Asn Pro Glu Arg Thr Thr Pro Tyr Ile
            100                 105                 110

Ile Gln Ser Val Leu Leu Leu Val Ala Pro Ala Leu Ile Ala Ala Thr
        115                 120                 125

Ile Tyr Met Ile Phe Gly Arg Leu Leu His Val Met Arg Cys Gln Ser
    130                 135                 140

Leu Ile Leu Ile Ser Ala Arg Phe Gly Thr Thr Phe Phe Val Val Gly
145                 150                 155                 160

Asp Val Phe Ser Phe Phe Leu Gln Ala Ala Gly Gly Gly Leu Met Ser
                165                 170                 175

Lys Ala Gly Ser Thr Lys Thr Gly Ser Gly Leu Ile Thr Ala Gly Leu
            180                 185                 190

Phe Val Gln Val Ile Phe Phe Gly Phe Phe Ile Ile Asn Glu Ile Arg
        195                 200                 205

Phe Thr Val Asn Val Lys Arg Arg Cys Leu Phe Tyr Glu Asp Ile Ser
    210                 215                 220

Arg Lys Trp Ile Phe Val Asn Ala Thr Leu Leu Leu Ser Ser Met Leu
225                 230                 235                 240

Ile Leu Leu Arg Ser Ile Val Arg Ile Val Glu Phe Ile Gln Gly Phe
                245                 250                 255

Asn Gly Tyr Ile Ile Ser His Glu Tyr Phe Ile Tyr Val Phe Asp Ala
            260                 265                 270

Val Pro Met Leu Leu Val Ile Ile Ala Phe Ser Val Gly Ser Phe Phe
        275                 280                 285

Gly Asn Val Phe Asp Val Ile Lys Glu Cys Gln Thr Leu Ser Asn
    290                 295                 300

<210> 205
<211> 368
<212> PRT
<213> Saccharomyces cerevisiae

<400> 205
Met Lys Leu Asp Ser Ile Asp Ile Thr His Cys Gln Leu Val Lys Ser
1               5                   10                  15

Thr Arg Thr Ala Arg Ile Tyr Arg Ser Asp Thr Tyr Ala Ile Lys Cys
            20                  25                  30

Leu Ala Leu Asp Phe Asp Ile Pro Pro His Asn Ala Lys Phe Glu Val
        35                  40                  45

Ser Ile Leu Asn Lys Leu Gly Asn Lys Cys Lys His Ile Leu Pro Leu
    50                  55                  60

Leu Glu Ser Lys Ala Thr Asp Asn Asn Asp Leu Leu Leu Leu Phe Pro
65                  70                  75                  80

Phe Glu Glu Met Asn Leu Tyr Glu Phe Met Gln Met His Tyr Lys Arg
                85                  90                  95

Asp Arg Arg Lys Lys Asn Pro Tyr Tyr Asp Leu Leu Asn Pro Ser Ile
            100                 105                 110

Pro Ile Val Ala Asp Pro Pro Val Gln Lys Tyr Thr Asn Gln Leu Asp
        115                 120                 125

Val Asn Arg Tyr Ser Leu Ser Phe Phe Arg Gln Met Val Glu Gly Ile

130                 135                 140

Ala Phe Leu His Glu Asn Lys Ile Ile His Arg Asp Ile Lys Pro Gln
145                 150                 155                 160

Asn Ile Met Leu Thr Asn Asn Thr Ser Thr Val Ser Pro Lys Leu Tyr
                165                 170                 175

Ile Ile Asp Phe Gly Ile Ser Tyr Asp Met Ala Asn Asn Ser Gln Thr
            180                 185                 190

Ser Ala Glu Pro Met Asp Ser Lys Val Thr Asp Ile Ser Thr Gly Ile
        195                 200                 205

Tyr Lys Ala Pro Glu Val Leu Phe Gly Val Lys Cys Tyr Asp Gly Gly
    210                 215                 220

Val Asp Val Trp Ser Leu Leu Ile Ile Ile Ser Gln Trp Phe Gln Arg
225                 230                 235                 240

Glu Thr Ser Arg Met Gly His Val Pro Ala Met Ile Asp Asp Gly Ser
                245                 250                 255

Asp Asp Met Asn Ser Asp Gly Ser Asp Phe Arg Leu Ile Cys Ser Ile
            260                 265                 270

Phe Glu Lys Leu Gly Ile Pro Ser Ile Gln Lys Trp Glu Glu Val Ala
        275                 280                 285

Gln His Gly Ser Val Asp Ala Phe Val Gly Met Phe Gly Ala Asp Gly
    290                 295                 300

Asp Gly Lys Tyr Val Leu Asp Gln Glu Lys Asp Val Gln Ile Ser Ile
305                 310                 315                 320

Val Glu Arg Asn Met Pro Arg Leu Asp Glu Ile Ala Asp Val Lys Val
                325                 330                 335

Lys Gln Lys Phe Ile Asn Cys Ile Leu Gly Met Val Ser Phe Ser Pro
            340                 345                 350

Asn Glu Arg Trp Ser Cys Gln Arg Ile Leu Gln Glu Leu Glu Lys Pro
        355                 360                 365

<210> 206
<211> 206
<212> PRT
<213> Saccharomyces cerevisiae

<400> 206
Met Asn Ser Glu Tyr Asp Tyr Leu Phe Lys Leu Leu Leu Ile Gly Asn
1               5                   10                  15

Ser Gly Val Gly Lys Ser Cys Leu Leu Leu Arg Phe Ser Asp Asp Thr
            20                  25                  30

Tyr Thr Asn Asp Tyr Ile Ser Thr Ile Gly Val Asp Phe Lys Ile Lys
        35                  40                  45

Thr Val Glu Leu Asp Gly Lys Thr Val Lys Leu Gln Ile Trp Asp Thr
    50                  55                  60

Ala Gly Gln Glu Arg Phe Arg Thr Ile Thr Ser Ser Tyr Tyr Arg Gly
65                  70                  75                  80

Ser His Gly Ile Ile Ile Val Tyr Asp Val Thr Asp Gln Glu Ser Phe
                85                  90                  95

Asn Gly Val Lys Met Trp Leu Gln Glu Ile Asp Arg Tyr Ala Thr Ser
            100                 105                 110

Thr Val Leu Lys Leu Leu Val Gly Asn Lys Cys Asp Leu Lys Asp Lys
        115                 120                 125

Arg Val Val Glu Tyr Asp Val Ala Lys Glu Phe Ala Asp Ala Asn Lys
    130                 135                 140

Met Pro Phe Leu Glu Thr Ser Ala Leu Asp Ser Thr Asn Val Glu Asp
145                 150                 155                 160

Ala Phe Leu Thr Met Ala Arg Gln Ile Lys Glu Ser Met Ser Gln Gln
                165                 170                 175

Asn Leu Asn Glu Thr Thr Gln Lys Lys Glu Asp Lys Gly Asn Val Asn
            180                 185                 190

Leu Lys Gly Gln Ser Leu Thr Asn Thr Gly Gly Gly Cys Cys
        195                 200                 205

<210> 207
<211> 542
<212> PRT
<213> Saccharomyces cerevisiae

<400> 207
Met Ile Ser Pro Ile Ser Phe Leu Ser Ser Leu Leu Cys Leu Thr Tyr
1               5                   10                  15

Leu Thr Ser Ala Leu Pro Ile Leu Pro Lys Arg Glu Val Val Thr Arg
            20                  25                  30

Val His Thr Ala Ser Thr Thr Asn Val Val Thr Asp Phe Tyr Ser Thr
        35                  40                  45

Thr Thr Glu Val Val Ile Ala Pro Thr Val Glu Phe Leu Ile Ser Asp
    50                  55                  60

Ser Val Thr Phe Thr Thr Thr Leu Ile Pro Gln Gly Val Asn Pro Thr
65                  70                  75                  80

Ala Glu Pro Thr Thr Thr Ile Thr Lys Val Leu Leu Lys Lys Ala Glu
                85                  90                  95

Met Ser Thr Ser Ser Gln Pro Thr Ser Thr Leu Gln Pro Ser Thr Ile
            100                 105                 110

Pro Gln Ser Thr Ser Ser Phe Gln Ala Glu Ser Thr Leu Gln Ala Val
        115                 120                 125

Ser Thr Gln Gln Thr Ala Met Ser Val Ser Ala Gly Thr Ser Glu Asp
    130                 135                 140

Val Gln Gln Leu Ala Thr Thr Ser Thr Ser Ile Ser Ser Ser Pro Ser
145                 150                 155                 160

Pro Thr Thr Thr Ser Thr Ser Thr Gln Asn Ile Val Ser Gly Val Gly
                165                 170                 175

Glu Ala Asn Val Glu Gly Asn Thr Gln Ser Gln His His Ser Tyr Gln
            180                 185                 190

Ala Ala Ala Thr Ser Thr Leu Asn Gln Gln Thr Ser Thr Ser Ile Ala
        195                 200                 205

Ser Gln Glu Ser Thr Glu Ser Thr Asn Thr Pro Thr Ser Ser Ser Thr
    210                 215                 220

Ser Ser Ser Thr Ser Ser Ser Thr Ser Ser Ser Thr Ser Ser Ser Thr
225                 230                 235                 240

Ser Ser Ser Thr Ser Ser Ser Thr Ser Ser Ser Thr Ser Ser Ser Thr
                245                 250                 255

Ser Ser Thr Gln Glu Thr Ala Ala Thr Thr Ser Glu Gly Ser Ser Ser
            260                 265                 270

Ser Ser Ala Ala Ile Thr Ser Ser Pro Lys Ala Ile Ala Tyr Ser Pro
        275                 280                 285

Tyr Asn Asp Asp Gly Ser Cys Lys Ser Ala Asp Ala Val Ser Ser Asp
    290                 295                 300

Leu Thr Leu Ile Lys Ser Lys Gly Ile Ser Lys Ile Arg Val Tyr Gly
305                 310                 315                 320

Thr Asp Cys Asn Ser Phe Glu Thr Val Gln Pro Ala Ala Val Lys Leu
                325                 330                 335

Gly Ile Lys Ile Asn Gln Gly Leu Tyr Ile Thr Ser Ser Gly Val Asp
            340                 345                 350

Ser Ile Asp Asp Ser Val Thr Thr Leu Ile Gln Tyr Gly Gln Thr Asn
        355                 360                 365

Gly Trp Asp Val Phe Asp Phe Ile Thr Val Gly Asn Glu Ala Ile Asn
    370                 375                 380

Asn Gly Trp Cys Ser Val Ser Asp Leu Ile Ser Lys Ile Ser Ser Val
385                 390                 395                 400

Lys Ser Lys Leu Ser Glu Ala Gly Tyr Ser Gly Gln Ile Thr Thr Ser
                405                 410                 415

Glu Pro Pro Val Ser Phe Glu Asn Asn Pro Asp Leu Cys Lys Lys Ser
            420                 425                 430

Asp Ile Asp Phe Val Gly Ile Asn Pro His Ala Tyr Phe Asp Thr Ser
        435                 440                 445

Ala Ser Ala Glu Thr Ala Gly Thr Phe Val Lys Gly Gln Val Glu Leu
    450                 455                 460

Ile Gln Gly Val Cys Gly Thr Ser Asn Val Phe Val Thr Glu Thr Gly
465                 470                 475                 480

Tyr Pro Ser Ser Gly Ile Gln Asn Gly Gly Asn Ile Pro Ser Thr Ala
                485                 490                 495

Asn Gln Ile Thr Ala Val Gln Asn Ile Leu Asn Glu Met Asp Leu Asp
            500                 505                 510

Val Thr Ile Leu Ser Thr Tyr Asn Asp Tyr Trp Lys Ala Pro Gly Asp
        515                 520                 525

Tyr Gly Ile Glu Gln Ser Phe Gly Val Ile Glu Tyr Phe Pro
    530                 535                 540


<210> 208
<211> 579
<212> PRT
<213> Saccharomyces cerevisiae

<400> 208
Met Asn Arg Asp Asn Met Asp Thr Thr Lys Arg Lys Glu Asp His Thr
1               5                   10                  15

Lys His Thr Thr Asp Val Ile Glu Phe Tyr Glu Glu Gly Thr Ala Ala
            20                  25                  30

Ser Ser Leu Asn Ile Ala Thr Glu Lys Ala Asn Ser Ser Pro Ser Ile
        35                  40                  45

Leu Arg Arg Ile Ile Asn Arg Ala Ala Trp Leu Ser Lys Lys Val Asp
    50                  55                  60

Ala Met Gly Val Glu Ser Thr Gly Ile Gln Arg Ile Ser Pro Tyr Glu
65                  70                  75                  80

Arg Gly Thr Ser Lys Lys Gln Phe Leu His Val Ala Gly Leu Trp Leu
                85                  90                  95

Ser Ala Thr Gly Gly Leu Ser Ser Met Ser Ser Phe Leu Leu Gly Pro
            100                 105                 110

Leu Leu Phe Gly Leu Ser Phe Arg Glu Ser Val Ala Ser Ser Leu Ile
        115                 120                 125

Ser Val Thr Ile Gly Cys Leu Ile Ala Ala Tyr Cys Ser Ile Met Gly
    130                 135                 140

Pro Gln Ser Gly Cys Arg Gln Met Val Thr Ala Arg Tyr Leu Phe Gly
145                 150                 155                 160

Trp Trp Phe Val Lys Leu Val Ala Leu Ala Ser Ile Ile Gly Val Met
                165                 170                 175

Gly Trp Ser Val Val Asn Ser Val Val Gly Gly Glu Met Leu Ala Ala
            180                 185                 190

Ile Ser Asn Asp Lys Val Pro Leu Trp Val Gly Ile Val Ile Val Thr
        195                 200                 205

Val Cys Ser Phe Leu Val Ala Ile Phe Gly Ile Lys Gln Val Ile Lys
    210                 215                 220

Val Glu Thr Tyr Leu Ser Val Pro Val Leu Thr Ala Phe Leu Leu Leu
225                 230                 235                 240

Tyr Ile Ser Ser Ser Asp Lys Tyr Ser Phe Val Asn Ala Tyr Val Ser
                245                 250                 255

Lys Gly Asn Leu Asp Ser Ser Thr Arg Lys Gly Asn Trp Met Ser Phe
            260                 265                 270

Phe Ser Leu Cys Tyr Ser Ile Thr Ala Thr Trp Gly Ser Ile Thr Ala
        275                 280                 285

Asp Tyr Tyr Ile Leu Phe Pro Glu Asp Thr Pro Tyr Ile Gln Ile Phe
    290                 295                 300

Cys Leu Thr Phe Phe Gly Thr Phe Leu Pro Thr Cys Phe Val Gly Ile
305                 310                 315                 320

Leu Gly Leu Leu Leu Ala Ser Val Ala Met Ser Tyr Lys Pro Trp Ser
                325                 330                 335

Val Glu Tyr Asp Ser His Gly Met Gly Gly Leu Leu Trp Ala Gly Phe
            340                 345                 350

Gln Arg Trp Asn Gly Phe Gly Lys Phe Cys Val Val Val Leu Val Phe
        355                 360                 365

Ser Leu Val Ser Asn Asn Ile Ile Asn Thr Tyr Ser Ala Ala Phe Ser
    370                 375                 380

Ile Gln Leu Ser Ser Val Phe Cys Ala Lys Ile Pro Arg Trp Phe Trp
385                 390                 395                 400

Ser Ile Val Cys Thr Ile Ile Cys Leu Val Cys Ala Leu Ile Gly Arg
                405                 410                 415

Asn His Phe Ser Thr Ile Leu Gly Asn Phe Leu Pro Met Ile Gly Tyr
            420                 425                 430

Trp Ile Ser Met Tyr Phe Ile Leu Leu Phe Glu Glu Asn Leu Val Phe
        435                 440                 445

Arg Arg Phe Phe Leu His Leu Tyr Thr Lys Glu Phe Pro Thr Val Thr
    450                 455                 460

Gly Glu Ile Asn Gly Pro Glu Leu Val Gly Ser Ser Lys Glu Val Glu
465                 470                 475                 480

Lys Asp Ala Val Thr Asn Ile His Leu Leu Lys Arg Lys His Lys Val
                485                 490                 495

Thr Lys His Arg Tyr Asn Trp Asp Lys Trp Glu Asp Tyr Glu Val Leu
            500                 505                 510

Thr His Gly Tyr Ala Ala Thr Phe Ala Phe Ile Val Gly Val Ala Gly
        515                 520                 525

Val Val Val Gly Met Ala Gln Ala Tyr Trp Ile Gly Pro Ile Ala Ala
    530                 535                 540

Lys Phe Gly Glu Tyr Gly Gly Asp Val Ala Met Trp Leu Ser Met Ala
545                 550                 555                 560

Phe Ser Gly Val Val Tyr Pro Pro Cys Arg Tyr Leu Glu Leu Arg Lys
                565                 570                 575

Phe Gly Arg

<210> 209
<211> 165
<212> PRT
<213> Saccharomyces cerevisiae

<400> 209
Met Glu Glu Val Lys Ala Val Asn Ser Ala Ala Lys Val Leu Cys Ser
1               5                   10                  15

Val Asp Pro Gln Phe Asp Pro Ser Lys Leu Thr Leu Ser Val Gly Ala
            20                  25                  30

Thr Pro Thr Ser Asn Ser Leu Lys Leu Asp Asn Lys Ser Thr Leu Val
        35                  40                  45

Lys Phe Ile Thr Thr Gln Leu Val Ser Thr Leu Glu Ile His Cys Gly
    50                  55                  60

Asn Tyr Cys Met Tyr Asp Leu Gln Gln Val Ala Thr Gly Cys Val Gln
65                  70                  75                  80

Asp His Glu Leu Ser Gly Phe Val Leu Gly Thr Val Leu Ser Ser Tyr
                85                  90                  95

Pro Ser Arg Gly Glu Leu Leu Ser Asn Thr Gly Val Met Cys Leu Thr
            100                 105                 110

Arg Glu Ala Ser Ser Ile Lys Gly Phe Gly Ile Cys Ala Asp Leu Glu
        115                 120                 125

His Val Leu Lys Ser Glu Ser Phe Ser Arg Glu Trp Tyr Val Ala Arg
    130                 135                 140

Val Ser Gln Glu His Gly Ile Leu Arg Pro Ile Arg Asn Trp Asn Glu
145                 150                 155                 160

Thr Thr Pro Leu Asn
                165

<210> 210
<211> 113
<212> PRT
<213> Saccharomyces cerevisiae

<400> 210
Met Ser Ile Leu Ser Ser Thr Gln Ser Thr Ile Leu Arg Ile Pro Ser
1               5                   10                  15

Gly Leu Ile Thr Phe Leu Leu Ser Lys Leu Phe Leu Leu Leu Arg Val
            20                  25                  30

Glu Pro Ser Ser Ala Ser Met Ser Ile Ser Glu Ser Glu Leu Leu Leu
        35                  40                  45

Met Gly Asn Ile Asn Asp Glu Ser Pro Lys Pro Gly Lys Leu Ala Ser
    50                  55                  60

Ala Pro Leu Ala Ser Leu Thr Asn Leu Val Phe Ser Ile Asp Val Lys
65                  70                  75                  80

Gly Leu Thr Leu Ile Ala Thr Thr Met Glu Asp Cys Leu Val Ser Gly
                85                  90                  95

Thr Phe Met Leu Val Ser Ile Val Tyr Ser Trp Lys Glu Asn Ser Ser
            100                 105                 110

Ser

<210> 211
<211> 219
<212> PRT
<213> Saccharomyces cerevisiae

<400> 211
Met Asp Leu Asp Leu Ala Ser Ile Leu Lys Gly Glu Ile Ser Lys Lys
1               5                   10                  15

Lys Lys Glu Leu Ala Asn Ser Lys Gly Val Gln Pro Pro Cys Thr Glu
            20                  25                  30

Lys Phe Gln Pro His Glu Ser Ala Asn Ile Asp Glu Thr Pro Arg Gln
        35                  40                  45

Val Glu Gln Glu Ser Thr Asp Glu Glu Asn Leu Ser Asp Asn Gln Ser
    50                  55                  60

Asp Asp Ile Arg Thr Thr Ile Ser Lys Leu Glu Asn Arg Pro Glu Arg
65                  70                  75                  80

Ile Gln Glu Ala Ile Ala Gln Asp Lys Thr Ile Ser Val Ile Ile Asp
                85                  90                  95

Pro Ser Gln Ile Gly Ser Thr Glu Gly Lys Pro Leu Leu Ser Met Lys
            100                 105                 110

Cys Asn Leu Tyr Ile His Glu Ile Leu Ser Arg Trp Lys Ala Ser Leu
        115                 120                 125

Glu Ala Tyr His Pro Glu Leu Phe Leu Asp Thr Lys Lys Ala Leu Phe
    130                 135                 140

Pro Leu Leu Leu Gln Leu Arg Arg Asn Gln Leu Ala Pro Asp Leu Leu
145                 150                 155                 160

Ile Ser Leu Ala Thr Val Leu Tyr His Leu Gln Gln Pro Lys Glu Ile
                165                 170                 175

Asn Leu Ala Val Gln Ser Tyr Met Lys Leu Ser Ile Gly Asn Val Ala
            180                 185                 190

Trp Pro Ile Gly Val Thr Ser Val Ala Phe Met Leu Val Val His Ile
        195                 200                 205

Arg Lys Phe Lys Glu Ala Gly Met Leu Leu Thr
    210                 215


<210> 212
<211> 1155
<212> PRT
<213> Saccharomyces cerevisiae

<400> 212
Met Asn Ser Asn Glu Leu Asp Leu Arg Asn Lys Tyr Phe Tyr Glu Ile

1               5                   10                  15

Phe Gly Lys Lys Arg Lys Ser Asp Thr Ser Thr Pro Thr Gln Leu Phe
            20                  25                  30

Ser Gly Ser Lys Val Gln Thr Asn Ile Asn Glu Ile Ser Ile Thr Asn
        35                  40                  45

Asp Glu Asp Glu Asp Ser Thr Glu Asp Glu Asn Lys Ala Ser Leu Lys
    50                  55                  60

Asp Tyr Thr Leu Gly His Asp Thr Gly Ala Arg Tyr Arg Ile Ala Pro
65                  70                  75                  80

Asp Cys Ser Ser His Gln Leu Lys Ala Ser Pro Val Leu His Ile Ser
                85                  90                  95

Thr Asn Leu Asn Ser Ser Pro Gln Ser Phe Thr Gly Asp Gln Ile Ser
            100                 105                 110

Pro Thr Asn Lys Lys Ile Ser Ile Asn Asp Ser Thr Arg Gln Asp Lys
        115                 120                 125

Gly Asn Ser Cys Thr Thr Thr Ser Ser Pro Ser Gln Lys Arg Ser Asn
    130                 135                 140

Val Leu Leu Pro His Val Arg Lys His Ser Ser Pro Ser Leu Leu Ser
145                 150                 155                 160

Phe Ser Lys Asn Ser Gly Ser His Met Gly Asp Pro Asn Gln Leu Ser
                165                 170                 175

Thr Pro Pro Thr Pro Lys Ser Ala Gly His Thr Met Glu Leu His Ser
            180                 185                 190

Ser Phe Asn Gly Lys His Ser Ser Ser Ser Thr Ser Ser Leu Phe Ala
        195                 200                 205

Leu Glu Ser Leu Lys Thr Gln Asn Arg Arg Ser Ser Asn Ser Ser Asn
    210                 215                 220

His Ser Ser Gln Tyr Arg Arg His Thr Asn Gln His Gln Arg His His
225                 230                 235                 240

Ser Arg Ser Lys Ser Ser Pro Val Ser Leu Thr Glu Ile Ser Met Ile
                245                 250                 255

Lys Gly Thr Pro Leu Val Tyr Pro Ala Leu Leu Ser Leu Ile Ala Ile
            260                 265                 270

Lys Phe Lys Gln Thr Ile Lys Leu Ser Thr His Lys Lys Met Gly Leu
        275                 280                 285

Leu Tyr Arg Asp Ser Phe Thr Gly Lys Gln Ala Ile Asp Thr Leu Cys
    290                 295                 300

Leu Ile Ile Gly Ser Leu Asp Arg Asn Leu Gly Met Leu Ile Gly Lys
305                 310                 315                 320

Ser Leu Glu Ala Gln Lys Leu Phe His Asp Val Leu Tyr Asp His Gly
                325                 330                 335

Val Arg Asp Ser Val Leu Glu Ile Tyr Glu Leu Ser Ser Glu Ser Ile
            340                 345                 350

Phe Met Ala His Gln Ser Gln Ser Ser Thr Ser Ile Ala Asn Thr Phe
        355                 360                 365

Ser Ser Ser Ser Ser Ser Val Asn Ser Leu Arg Thr Lys Thr Glu Ile
    370                 375                 380

Tyr Gly Val Phe Val Pro Leu Thr His Cys Tyr Ser Ser Thr Cys Ser
385                 390                 395                 400

Leu Glu Lys Leu Cys Tyr Ser Ile Ser Cys Pro Asn Arg Leu Gln Gln
                405                 410                 415

Gln Ala Asn Leu His Leu Lys Leu Gly Gly Gly Leu Lys Arg Asn Ile
            420                 425                 430

Ser Leu Ala Leu Asp Lys Glu Asp Asp Glu Arg Ile Ser Trp Thr Asn
        435                 440                 445

Ser Val Pro Lys Ser Val Trp Glu Ser Leu Ser Lys Gln Gln Ile Lys
    450                 455                 460

Arg Gln Glu Ala Ile Tyr Glu Leu Phe Thr Thr Glu Lys Lys Phe Val
465                 470                 475                 480

Lys Ser Leu Glu Ile Ile Arg Asp Thr Phe Met Lys Lys Leu Leu Glu
                485                 490                 495

Thr Asn Ile Ile Pro Ser Asp Val Arg Ile Asn Phe Val Lys His Val
            500                 505                 510

Phe Ala His Ile Asn Glu Ile Tyr Ser Val Asn Arg Glu Phe Leu Lys
        515                 520                 525

Ala Leu Ala Gln Arg Gln Ser Leu Ser Pro Ile Cys Pro Gly Ile Ala
    530                 535                 540

Asp Ile Phe Leu Gln Tyr Leu Pro Phe Phe Asp Pro Phe Leu Ser Tyr
545                 550                 555                 560

Ile Ala Ser Arg Pro Tyr Ala Lys Tyr Leu Ile Glu Thr Gln Arg Ser
                565                 570                 575

Val Asn Pro Asn Phe Ala Arg Phe Asp Asp Glu Val Ser Asn Ser Ser
            580                 585                 590

Leu Arg His Gly Ile Asp Ser Phe Leu Ser Gln Gly Val Ser Arg Pro
        595                 600                 605

Gly Arg Tyr Ser Leu Leu Val Arg Glu Ile Ile His Phe Ser Asp Pro
    610                 615                 620

Val Thr Asp Lys Asp Asp Leu Gln Met Leu Met Lys Val Gln Asp Leu
625                 630                 635                 640

Leu Lys Asp Leu Met Lys Arg Ile Asp Arg Ala Ser Gly Ala Ala Gln
                645                 650                 655

Asp Arg Tyr Asp Val Lys Val Leu Lys Gln Lys Ile Leu Phe Lys Asn
            660                 665                 670

Glu Tyr Val Asn Leu Gly Leu Asn Asn Glu Lys Arg Lys Ile Lys His
        675                 680                 685

Glu Gly Leu Leu Ser Arg Lys Asp Val Asn Lys Thr Asp Ala Ser Phe
    690                 695                 700

Ser Gly Asp Ile Gln Phe Tyr Leu Leu Asp Asn Met Leu Leu Phe Leu
705                 710                 715                 720

Lys Ser Lys Ala Val Asn Lys Trp His Gln His Thr Val Phe Gln Arg
                725                 730                 735

Pro Ile Pro Leu Pro Leu Leu Phe Ile Cys Pro Ala Glu Asp Met Pro
            740                 745                 750

Pro Ile Lys Arg Tyr Val Thr Glu Asn Pro Asn Cys Ser Ala Gly Val
        755                 760                 765

Leu Leu Pro Gln Tyr Gln Thr Ser Asn Pro Lys Asn Ala Ile Val Phe
    770                 775                 780

Ala Tyr Tyr Gly Thr Lys Gln Gln Tyr Gln Val Thr Leu Tyr Ala Pro
785                 790                 795                 800

Gln Pro Ala Gly Leu Gln Thr Leu Ile Glu Lys Val Lys Gln Glu Gln
                805                 810                 815

Lys Arg Leu Leu Asp Glu Thr Lys His Ile Thr Phe Lys Gln Met Val
            820                 825                 830

Gly Gln Phe Phe His Ser Tyr Ile Asn Thr Asn Arg Val Asn Asp Val
        835                 840                 845

Leu Ile Cys His Ala Gly Lys Ile Leu Leu Val Ala Thr Asn Met Gly
    850                 855                 860

Leu Phe Val Leu Asn Tyr Ala Thr Ser Ile Asn Gln Lys Pro Val His
865                 870                 875                 880

Leu Leu His Lys Ile Ser Ile Ser Gln Ile Ser Val Leu Glu Glu Tyr
                885                 890                 895

Lys Val Met Ile Leu Leu Ile Asp Lys Lys Leu Tyr Gly Cys Pro Leu

900                 905                 910

Asp Val Ile Asp Asp Ala Glu Asn Ala Asp Phe Leu Phe Arg Lys Asn
        915                 920                 925

Ser Lys Val Leu Phe Lys Tyr Val Ala Met Phe Lys Asp Gly Phe Cys
    930                 935                 940

Asn Gly Lys Arg Ile Ile Met Ile Ala His His Phe Leu His Ala Ala
945                 950                 955                 960

Gln Leu Leu Ile Val Asn Pro Leu Ile Phe Asp Phe Asn Ser Gly Asn
                965                 970                 975

Phe Lys Lys Asn Leu Lys Ala Gly Leu Val Asp Phe Ser Val Asp Ser
            980                 985                 990

Glu Pro Leu Ser Phe Ser Phe Leu Glu Asn Lys Ile Cys Ile Gly Cys
        995                 1000                1005

Lys Lys Asn Ile Lys Ile Leu Asn Val Pro Glu Val Cys Asp Lys Asn
    1010                1015                1020

Gly Phe Lys Met Arg Glu Leu Leu Asn Leu His Asp Asn Lys Val Leu
1025                1030                1035                1040

Ala Asn Met Tyr Lys Glu Thr Phe Lys Val Val Ser Met Phe Pro Ile
                1045                1050                1055

Lys Asn Ser Thr Phe Ala Cys Phe Pro Glu Leu Cys Phe Phe Leu Asn
            1060                1065                1070

Lys Gln Gly Lys Arg Glu Glu Thr Lys Gly Cys Phe His Trp Glu Gly
        1075                1080                1085

Glu Pro Glu Gln Phe Ala Cys Ser Tyr Pro Tyr Ile Val Ala Ile Asn
    1090                1095                1100

Ser Asn Phe Ile Glu Ile Arg His Ile Glu Asn Gly Glu Leu Val Arg
1105                1110                1115                1120

Cys Val Leu Gly Asn Lys Ile Arg Met Leu Lys Ser Tyr Ala Lys Lys
                1125                1130                1135

Ile Leu Tyr Cys Tyr Glu Asp Pro Gln Gly Phe Glu Ile Ile Glu Leu
            1140                1145                1150

Leu Asn Phe
        1155

<210> 213
<211> 400
<212> PRT
<213> Saccharomyces cerevisiae

<400> 213
Met Thr Ala Ser Ser Asn Asp Asp Asp Leu Ile Phe Glu Cys Tyr Ser
1               5                   10                  15

Asp Pro Glu Leu Lys Arg Trp Thr His Leu Ala Asn Ala Lys Ala Trp
            20                  25                  30

Lys Gly Ile Leu Thr Val Gln Gln Tyr Ala Asp Arg Glu Gln Leu Leu
        35                  40                  45

Gly Ser Ser Glu Ile Ser Gln Lys Asn Lys Ser Asn Glu Met Met Thr
    50                  55                  60

Lys Tyr Pro Lys Ser Tyr Gln Trp Leu Gly Gln Lys Tyr Phe Val Leu
65                  70                  75                  80

Lys Asp Arg Ser Leu Pro Asp Asn Gly Lys Phe Ser Gln Val Val Ser
                85                  90                  95

Ser Cys Glu Thr Leu Asn Arg Ile Gly Tyr Cys Ile His Pro Gly Ser
            100                 105                 110

Asn Gly Lys Ile Glu Pro Ala Leu Ile Val Cys Ile Gly Gly Val Phe
        115                 120                 125

Thr Phe Glu Asn His Arg Gly Lys Gly Tyr Ala Lys Lys Met Ile Ile
    130                 135                 140

Lys Leu Asn Glu Phe Tyr Asp Lys Ile Arg Asp Asp Ala Asn Thr Val
145                 150                 155                 160

Leu Glu Leu Lys Asn Leu Val Ile Asn Leu Tyr Ser Glu Val Gly Glu
                165                 170                 175

Tyr Tyr Ser Ala Leu Gly Tyr Glu Ser Met His Val Pro Leu His Arg
            180                 185                 190

Ile Ser Lys Leu Asp Glu Leu Thr Glu Arg Tyr Cys Gly Glu Asp Asp
        195                 200                 205

Asp His Asp Gly Lys Tyr Leu Gly Phe Asp Asp Tyr Arg Gly Leu Val
    210                 215                 220

Gly Leu His Glu Thr Gln Phe Lys Glu Ser Leu Leu Ser Leu His Lys
225                 230                 235                 240

Glu Asn Pro Glu Lys Phe Val Phe Thr Val Ala Pro Asp Phe Asp Ile
                245                 250                 255

Phe Thr Trp Phe Gln Tyr Arg Asp Leu Phe Ile Met Asn Lys Ser Gly
            260                 265                 270

Arg Lys Ala Gln Gln Asn Leu Phe Phe Gly Tyr Ala Leu Ser Asp Asn
        275                 280                 285

Ser His Ile Ile Trp His His Asn Trp Asn Gly Asp Ser Leu Ile Ile

290                 295                 300

Val Lys Ile His Ile Pro Glu Glu Thr Phe Gln Arg Lys Glu Leu Lys
305                 310                 315                 320

Leu Lys Lys Leu Leu Arg Lys Ala Ile Glu Glu Thr Lys Leu His Gly
                325                 330                 335

Leu Gln Glu Leu Glu Phe Trp Asp Glu Glu Ile Pro Ile Lys Lys Tyr
            340                 345                 350

Pro Gln Leu Phe Gln Leu Leu Thr Glu Leu Glu Asn Glu Ser Lys Val
        355                 360                 365

Phe Ser Glu Asn Gly Ser Ile Ser Ala Val Arg Pro Pro Lys Gly Tyr
    370                 375                 380

Thr Ala Glu Gln Val Ile Trp Asp Asn Asn Thr Lys Phe Cys Trp Phe
385                 390                 395                 400

<210> 214
<211> 117
<212> PRT
<213> Saccharomyces cerevisiae

<400> 214
Met Ser Arg Gln Ser Ala Phe Lys Phe Gln Asn Gly Asn Arg His Glu
1               5                   10                  15

Arg Ala Cys Leu Ser Asp Val His Lys Ile Leu Ile Ile Ile Leu Tyr
            20                  25                  30

Ser Thr Lys Gly Lys Arg Glu Leu Gly Lys Arg Ile Thr His Phe Met
        35                  40                  45

Tyr Ile His Ile Phe Cys Thr Tyr Leu Tyr Gln Ala Ser Ile Val Gln
    50                  55                  60

Tyr Cys Ser Ser Thr Leu Leu Asn Val Ile Ala Phe Ser Trp Tyr Pro
65                  70                  75                  80

Leu Asn Leu Ile Tyr Lys Tyr Glu Tyr Ile Leu Arg Ser Lys Leu Ile
                85                  90                  95

Leu Met Val Cys Leu Asp Phe Ala Arg Ser Gly Gly Val Leu Asp Ser
            100                 105                 110

Gly Asp Val Ser Leu
        115

<210> 215

<211> 93
<212> PRT
<213> Saccharomyces cerevisiae

<400> 215
Met Ser Phe Leu Gly Phe Gly Gly Gly Gln Pro Gln Leu Ser Ser Gln
1               5                   10                  15

Gln Lys Ile Gln Ala Ala Glu Ala Glu Leu Asp Leu Val Thr Asp Met
            20                  25                  30

Phe Asn Lys Leu Val Asn Asn Cys Tyr Lys Lys Cys Ile Asn Thr Ser
        35                  40                  45

Tyr Ser Glu Gly Glu Leu Asn Lys Asn Glu Ser Ser Cys Leu Asp Arg
    50                  55                  60

Cys Val Ala Lys Tyr Phe Glu Thr Asn Val Gln Val Gly Glu Asn Met
65                  70                  75                  80

Gln Lys Met Gly Gln Ser Phe Asn Ala Ala Gly Lys Phe
                85                  90


<210> 216
<211> 431
<212> PRT
<213> Saccharomyces cerevisiae

<400> 216
Met Ala Asp Phe Lys Ser Leu Gly Leu Ser Lys Trp Leu Thr Glu Ser
1               5                   10                  15

Leu Arg Ala Met Lys Ile Thr Gln Pro Thr Ala Ile Gln Lys Ala Cys
            20                  25                  30

Ile Pro Lys Ile Leu Glu Gly Arg Asp Cys Ile Gly Gly Ala Lys Thr
        35                  40                  45

Gly Ser Gly Lys Thr Ile Ala Phe Ala Gly Pro Met Leu Thr Lys Trp
    50                  55                  60

Ser Glu Asp Pro Ser Gly Met Phe Gly Val Val Leu Thr Pro Thr Arg
65                  70                  75                  80

Glu Leu Ala Met Gln Ile Ala Glu Gln Phe Thr Ala Leu Gly Ser Ser
                85                  90                  95

Met Asn Ile Arg Val Ser Val Ile Val Gly Gly Glu Ser Ile Val Gln
            100                 105                 110

Gln Ala Leu Asp Leu Gln Arg Lys Pro His Phe Ile Ile Ala Thr Pro
        115                 120                 125

Gly Arg Leu Ala His His Ile Met Ser Ser Gly Asp Asp Thr Val Gly
    130                 135                 140

Gly Leu Met Arg Ala Lys Tyr Leu Val Leu Asp Glu Ala Asp Ile Leu
145                 150                 155                 160

Leu Thr Ser Thr Phe Ala Asp His Leu Ala Thr Cys Ile Ser Ala Leu
                165                 170                 175

Pro Pro Lys Asp Lys Arg Gln Thr Leu Leu Phe Thr Ala Thr Ile Thr
            180                 185                 190

Asp Gln Val Lys Ser Leu Gln Asn Ala Pro Val Gln Lys Gly Lys Pro
        195                 200                 205

Pro Leu Phe Ala Tyr Gln Val Glu Ser Val Asp Asn Val Ala Ile Pro
    210                 215                 220

Ser Thr Leu Lys Ile Glu Tyr Ile Leu Val Pro Glu His Val Lys Glu
225                 230                 235                 240

Ala Tyr Leu Tyr Gln Leu Leu Thr Cys Glu Glu Tyr Glu Asn Lys Thr
                245                 250                 255

Ala Ile Ile Phe Val Asn Arg Thr Met Thr Ala Glu Ile Leu Arg Arg
            260                 265                 270

Thr Leu Lys Gln Leu Glu Val Arg Val Ala Ser Leu His Ser Gln Met
        275                 280                 285

Pro Gln Gln Glu Arg Thr Asn Ser Leu His Arg Phe Arg Ala Asn Ala
    290                 295                 300

Ala Arg Ile Leu Ile Ala Thr Asp Val Ala Ser Arg Gly Leu Asp Ile
305                 310                 315                 320

Pro Thr Val Glu Leu Val Val Asn Tyr Asp Ile Pro Ser Asp Pro Asp
                325                 330                 335

Val Phe Ile His Arg Ser Gly Arg Thr Ala Arg Ala Gly Arg Ile Gly
            340                 345                 350

Asp Ala Ile Ser Phe Val Thr Gln Arg Asp Val Ser Arg Ile Gln Ala
        355                 360                 365

Ile Glu Asp Arg Ile Asn Lys Lys Met Thr Glu Thr Asn Lys Val His
    370                 375                 380

Asp Thr Ala Val Ile Arg Lys Ala Leu Thr Lys Val Thr Lys Ala Lys
385                 390                 395                 400

Arg Glu Ser Leu Met Ala Met Gln Lys Glu Asn Phe Gly Glu Arg Lys
                405                 410                 415

Arg Gln Gln Lys Lys Lys Gln Asn Asp Gly Lys Ser Leu Arg Ser
            420                 425                 430

<210> 217
<211> 261
<212> PRT
<213> Saccharomyces cerevisiae

<400> 217
Met Ala Arg Gly Pro Lys Lys His Leu Lys Arg Leu Ala Ala Pro His
1               5                   10                  15

His Trp Leu Leu Asp Lys Leu Ser Gly Cys Tyr Ala Pro Arg Pro Ser
            20                  25                  30

Ala Gly Pro His Lys Leu Arg Glu Ser Leu Pro Leu Ile Val Phe Leu
        35                  40                  45

Arg Asn Arg Leu Lys Tyr Ala Leu Asn Gly Arg Glu Val Lys Ala Ile
    50                  55                  60

Leu Met Gln Arg His Val Lys Val Asp Gly Lys Val Arg Thr Asp Thr
65                  70                  75                  80

Thr Tyr Pro Ala Gly Phe Met Asp Val Ile Thr Leu Asp Ala Thr Asn
                85                  90                  95

Glu Asn Phe Arg Leu Val Tyr Asp Val Lys Gly Arg Phe Ala Val His
            100                 105                 110

Arg Ile Thr Asp Glu Glu Ala Ser Tyr Lys Leu Gly Lys Val Lys Lys
        115                 120                 125

Val Gln Leu Gly Lys Lys Gly Val Pro Tyr Val Val Thr His Asp Gly
    130                 135                 140

Arg Thr Ile Arg Tyr Pro Asp Pro Asn Ile Lys Val Asn Asp Thr Val
145                 150                 155                 160

Lys Ile Asp Leu Ala Ser Gly Lys Ile Thr Asp Phe Ile Lys Phe Asp
                165                 170                 175

Ala Gly Lys Leu Val Tyr Val Thr Gly Gly Arg Asn Leu Gly Arg Ile
            180                 185                 190

Gly Thr Ile Val His Lys Glu Arg His Asp Gly Gly Phe Asp Leu Val
        195                 200                 205

His Ile Lys Asp Ser Leu Asp Asn Thr Phe Val Thr Arg Leu Asn Asn
    210                 215                 220

Val Phe Val Ile Gly Glu Gln Gly Lys Pro Tyr Ile Ser Leu Pro Lys
225                 230                 235                 240

Gly Lys Gly Ile Lys Leu Ser Ile Ala Glu Glu Arg Asp Arg Arg Arg
                245                 250                 255

Ala Gln Gln Gly Leu
            260

<210> 218
<211> 130
<212> PRT
<213> Saccharomyces cerevisiae

<400> 218
Met Cys Leu Gly Lys Leu Tyr Phe Glu Gln Leu Ile Leu Val Arg Cys
1               5                   10                  15

Ile Lys Gly Arg Gln Ser Gly Asn Ile Thr Thr Gly Glu Ser Arg Cys
            20                  25                  30

Val Ser Trp Asn Val Tyr Cys Thr Thr Ser Met Ile Gly Leu Phe Ser
        35                  40                  45

Trp Arg Lys Met Leu Leu Phe Gln His Phe Ser Tyr Thr Gln Arg Glu
    50                  55                  60

Asn Arg Gln Ile Gly Gly Lys Thr Trp Ser Leu Ile Ser Ser Leu Phe
65                  70                  75                  80

His Leu Asn Glu Thr Leu Ala Ser Ala Leu His His Pro Tyr Glu Thr
                85                  90                  95

Leu Ala Leu Tyr Glu Ala Tyr Phe Ala Cys Gly Lys Lys Asn Phe Phe
            100                 105                 110

Cys Lys Arg Gly Tyr Ser Glu Thr Val Thr Glu Gly Arg Gly Trp Lys
        115                 120                 125

Arg Val
    130


<210> 219
<211> 640
<212> PRT
<213> Saccharomyces cerevisiae

<400> 219
Met Arg Arg Glu Arg Gln Arg Met Met Ser Phe Glu Asp Lys Asp Lys
1               5                   10                  15

Asp Asp Leu Asp Asn Ser Asn Ser Asn Asn Ser Ser Glu Met Thr Asp
            20                  25                  30

Thr Ala Met Met Pro Pro Leu Lys Arg Leu Leu Ile Thr Gly Ser Ser
        35                  40                  45

Asp Asp Leu Ala Gln Gly Ser Ser Gly Lys Lys Lys Met Thr Met Ala
    50                  55                  60

Thr Arg Ser Pro Ser Ser Ser Pro Asp Leu Ala Thr Asn Asp Ser Gly
65                  70                  75                  80

Thr Arg Val Gln Pro Leu Pro Glu Tyr Asn Phe Thr Lys Phe Cys Tyr
                85                  90                  95

Arg His Asn Pro Asp Ile Gln Phe Ser Pro Thr His Thr Ala Cys Tyr
            100                 105                 110

Lys Gln Asp Leu Lys Arg Thr Gln Glu Ile Asn Ala Asn Ile Ala Lys
        115                 120                 125

Leu Pro Leu Gln Glu Gln Ser Asp Ile His His Ile Ile Ser Lys Tyr
    130                 135                 140

Ser Asn Ser Asn Asp Lys Ile Arg Lys Leu Ile Leu Asp Gly Ile Leu
145                 150                 155                 160

Ser Thr Ser Cys Phe Pro Gln Leu Ser Tyr Ile Ser Ser Leu Val Thr
                165                 170                 175

His Met Ile Lys Ile Asp Phe Ile Ser Ile Leu Pro Gln Glu Leu Ser
            180                 185                 190

Leu Lys Ile Leu Ser Tyr Leu Asp Cys Gln Ser Leu Cys Asn Ala Thr
        195                 200                 205

Arg Val Cys Arg Lys Trp Gln Lys Leu Ala Asp Asp Asp Arg Val Trp
    210                 215                 220

Tyr His Met Cys Glu Gln His Ile Asp Arg Lys Cys Pro Asn Cys Gly
225                 230                 235                 240

Trp Gly Leu Pro Leu Leu His Met Lys Arg Ala Arg Ile Gln Gln Asn
                245                 250                 255

Ser Thr Gly Ser Ser Ser Asn Ala Asp Ile Gln Thr Gln Thr Thr Arg
            260                 265                 270

Pro Trp Lys Val Ile Tyr Arg Glu Arg Phe Lys Val Glu Ser Asn Trp
        275                 280                 285

Arg Lys Gly His Cys Arg Ile Gln Glu Phe Lys Gly His Met Asp Gly
    290                 295                 300

Val Leu Thr Leu Gln Phe Asn Tyr Arg Leu Leu Phe Thr Gly Ser Tyr
305                 310                 315                 320

Asp Ser Thr Ile Gly Ile Trp Asp Leu Phe Thr Gly Lys Leu Ile Arg
                325                 330                 335

Arg Leu Ser Gly His Ser Asp Gly Val Lys Thr Leu Tyr Phe Asp Asp
            340                 345                 350

Arg Lys Leu Ile Thr Gly Ser Leu Asp Lys Thr Ile Arg Val Trp Asn
        355                 360                 365

Tyr Ile Thr Gly Glu Cys Ile Ser Thr Tyr Arg Gly His Ser Asp Ser

370                 375                 380

Val Leu Ser Val Asp Ser Tyr Gln Lys Val Ile Val Ser Gly Ser Ala
385                 390                 395                 400

Asp Lys Thr Val Lys Val Trp His Val Glu Ser Arg Thr Cys Tyr Thr
                405                 410                 415

Leu Arg Gly His Thr Glu Trp Val Asn Cys Val Lys Leu His Pro Lys
            420                 425                 430

Ser Phe Ser Cys Phe Ser Cys Ser Asp Asp Thr Thr Ile Arg Met Trp
        435                 440                 445

Asp Ile Arg Thr Asn Ser Cys Leu Lys Val Phe Arg Gly His Val Gly
    450                 455                 460

Gln Val Gln Lys Ile Ile Pro Leu Thr Ile Lys Asp Val Glu Asn Leu
465                 470                 475                 480

Ala Thr Asp Asn Thr Ser Asp Gly Ser Ser Pro Gln Asp Asp Pro Thr
                485                 490                 495

Met Thr Asp Gly Ala Asp Glu Ser Asp Thr Pro Ser Asn Glu Gln Glu
            500                 505                 510

Thr Val Leu Asp Glu Asn Ile Pro Tyr Pro Thr His Leu Leu Ser Cys
        515                 520                 525

Gly Leu Asp Asn Thr Ile Lys Leu Trp Asp Val Lys Thr Gly Lys Cys
    530                 535                 540

Ile Arg Thr Gln Phe Gly His Val Glu Gly Val Trp Asp Ile Ala Ala
545                 550                 555                 560

Asp Asn Phe Arg Ile Ile Ser Gly Ser His Asp Gly Ser Ile Lys Val
                565                 570                 575

Trp Asp Leu Gln Ser Gly Lys Cys Met His Thr Phe Asn Gly Arg Arg
            580                 585                 590

Leu Gln Arg Glu Thr Gln His Thr Gln Thr Gln Ser Leu Gly Asp Lys
        595                 600                 605

Val Ala Pro Ile Ala Cys Val Cys Ile Gly Asp Ser Glu Cys Phe Ser
    610                 615                 620

Gly Asp Glu Phe Gly Cys Val Lys Met Tyr Lys Phe Asp Leu Asn Asp
625                 630                 635                 640

<210> 220
<211> 121

<212> PRT
<213> Saccharomyces cerevisiae

<400> 220
Met Asn Phe Ser Thr Val Phe Gln Ala Ile Ile Ala Val Leu Gly Leu
1               5                   10                  15

Thr Thr Val Thr Ala Leu Ala Glu Phe Asp Phe Asp Val Gly Tyr Glu
            20                  25                  30

Glu Phe Val Arg Thr Asn Pro Asp Thr Ile Phe Leu Glu Ser Asp Ile
        35                  40                  45

Gly Leu His Val Gly Tyr Thr Glu Gly Gly Glu Arg Gln Ile Ala Thr
    50                  55                  60

Ile Pro His Asn Ser Thr Leu Gly Thr Ser Leu Arg Glu Tyr Ser Gly
65                  70                  75                  80

Cys Gly Gly Asn Gly Thr Glu Thr Ser Ile Ala Thr Pro Ala Pro Thr
                85                  90                  95

Met Ser Glu Val Pro Ile Ala Thr Phe Val Lys Arg Arg Lys Ser Val
            100                 105                 110

Pro Ile Leu Leu Pro Gln Val Cys Met
        115                 120


<210> 221
<211> 336
<212> PRT
<213> Saccharomyces cerevisiae

<400> 221
Met Ala Arg Lys Leu Lys Gly Lys Ile Gly Ser Lys Gly Leu Lys Gly
1               5                   10                  15

Ala Leu Leu Arg His Lys Ala Lys Val Lys Leu Val Arg Asn Ile Glu
            20                  25                  30

Ser Lys Gln Lys His Glu Leu Arg Lys Lys Asn Ser Ser Ala Asn Asn
        35                  40                  45

Lys Thr Val Lys Arg Asn Gln Glu Phe Gln Lys Leu Asn Gln Gly Lys
    50                  55                  60

Val Met Pro Phe Glu Lys Asp Glu Thr Leu Met Leu Cys Gly Glu Gly
65                  70                  75                  80

Asp Phe Ser Phe Ala Arg Ser Ile Val Glu Gln Asn Tyr Ile Glu Ser
                85                  90                  95

Asp Asn Leu Ile Ile Thr Ser Tyr Asp Asn Ser Val Asn Glu Leu Lys
            100                 105                 110

Leu Lys Tyr Pro His Thr Phe Glu Glu Asn Tyr Gln Tyr Leu Lys Asp
        115                 120                 125

Leu Asn Ile Pro Ile Phe Phe Gln Ile Asp Val Thr Lys Leu Val Lys
    130                 135                 140

Ser Phe Lys Ile Ser Lys Asn Asn Thr Trp Phe Lys Ile Ile Asn Arg
145                 150                 155                 160

Leu Ser Asp His Arg Trp Gly Asn Lys Pro Leu Gln Asn Ile Val Phe
                165                 170                 175

Asn Phe Pro His Asn Gly Lys Gly Ile Lys Asp Gln Glu Arg Asn Ile
            180                 185                 190

Arg Glu His Gln Asp Leu Ile Phe Asn Phe Phe Gln Asn Ser Leu Gln
        195                 200                 205

Leu Phe Asn Leu Ile Asn Thr Lys Ile Gln Asn Asp Thr Leu Arg Tyr
    210                 215                 220

Thr Gln Gly Tyr Asp Leu Asn Glu Asp Thr Pro Gln Ala Lys Lys Leu
225                 230                 235                 240

Thr Ala Glu Gly Tyr Gly Asn Ile Ile Leu Ser Leu Phe Asp Gly Glu
                245                 250                 255

Pro Tyr Asp Ser Trp Gln Ile Lys Leu Leu Ala Lys Lys Asn Gly Leu
            260                 265                 270

Thr Leu Ser Arg Ser Ser Lys Phe Gln Trp Glu Asn Phe Pro Gly Tyr
        275                 280                 285

His His Arg Arg Thr Asn Ser Glu Gln Asp Thr Thr Lys Pro Ala Lys
    290                 295                 300

Glu Arg Asp Ala Arg Phe Tyr Ile Phe Ser Lys Tyr Val Ser Asn Ser
305                 310                 315                 320

Ser Lys His Asn Arg Lys Ser Lys Lys Asp Thr Asp Ser Asp Ser Asp
                325                 330                 335

<210> 222
<211> 117
<212> PRT
<213> Saccharomyces cerevisiae

<400> 222
Met Tyr Ile Tyr Val Glu Val Cys Thr His Val His Ala Gln Ala Thr
1               5                   10                  15

Asp Ala Thr Arg Pro Gly Ile His Ile Cys Thr Tyr Ile Tyr Ala His

20                  25                  30

Ile Tyr Ile Tyr Ile Tyr Val Tyr Val Asn Val Tyr Ile Gln Leu Val
        35                  40                  45

Ile Ser Arg Ala Glu Trp Ala Glu Lys His Ser Ile Val Glu Lys Lys
    50                  55                  60

Arg Glu Glu Lys Gly Arg Gly Ala Ile Ala Met Asp Val Val Glu Tyr
65                  70                  75                  80

Arg Glu Gly Gln Ala Val Val Gln Trp Met Arg Phe Glu Ala Gln Arg
                85                  90                  95

Lys Gly Gly Ala Ser Lys His Arg Thr Leu Ser Ala Glu Thr Gly Ile
            100                 105                 110

Arg Gly Glu Gly Glu
        115


<210> 223
<211> 1032
<212> PRT
<213> Saccharomyces cerevisiae

<400> 223
Met Thr Pro Asp Glu Leu Asn Ser Ala Val Val Thr Phe Met Ala Asn
1               5                   10                  15

Leu Asn Ile Asp Asp Ser Lys Ala Asn Glu Thr Ala Ser Thr Val Thr
            20                  25                  30

Asp Ser Ile Val His Arg Ser Ile Lys Leu Leu Glu Val Val Val Ala
        35                  40                  45

Leu Lys Asp Tyr Phe Leu Ser Glu Asn Glu Val Glu Arg Lys Lys Ala
    50                  55                  60

Leu Thr Cys Leu Thr Thr Ile Leu Ala Lys Thr Pro Lys Asp His Leu
65                  70                  75                  80

Ser Lys Asn Glu Cys Ser Val Ile Phe Gln Phe Tyr Gln Ser Lys Leu
                85                  90                  95

Asp Asp Gln Ala Leu Ala Lys Glu Val Leu Glu Gly Phe Ala Ala Leu
            100                 105                 110

Ala Pro Met Lys Tyr Val Ser Ile Asn Glu Ile Ala Gln Leu Leu Arg
        115                 120                 125

Leu Leu Leu Asp Asn Tyr Gln Gln Gly Gln His Leu Ala Ser Thr Arg
    130                 135                 140

Leu Trp Pro Phe Lys Ile Leu Arg Lys Ile Phe Asp Arg Phe Phe Val
145                 150                 155                 160

Asn Gly Ser Ser Thr Glu Gln Val Lys Arg Ile Asn Asp Leu Phe Ile
                165                 170                 175

Glu Thr Phe Leu His Val Ala Asn Gly Glu Lys Asp Pro Arg Asn Leu
            180                 185                 190

Leu Leu Ser Phe Ala Leu Asn Lys Ser Ile Thr Ser Ser Leu Gln Asn
        195                 200                 205

Val Glu Asn Phe Lys Glu Asp Leu Phe Asp Val Leu Phe Cys Tyr Phe
    210                 215                 220

Pro Ile Thr Phe Lys Pro Pro Lys His Asp Pro Tyr Lys Ile Ser Asn
225                 230                 235                 240

Gln Asp Leu Lys Thr Ala Leu Arg Ser Ala Ile Thr Ala Thr Pro Leu
                245                 250                 255

Phe Ala Glu Asp Ala Tyr Ser Asn Leu Leu Asp Lys Leu Thr Ala Ser
            260                 265                 270

Ser Pro Val Val Lys Asn Asp Thr Leu Leu Thr Leu Leu Glu Cys Val
        275                 280                 285

Arg Lys Phe Gly Gly Ser Ser Ile Leu Glu Asn Trp Thr Leu Leu Trp
    290                 295                 300

Asn Ala Leu Lys Phe Glu Ile Met Gln Asn Ser Glu Gly Asn Glu Asn
305                 310                 315                 320

Thr Leu Leu Asn Pro Tyr Asn Lys Asp Gln Gln Ser Asp Asp Val Gly
                325                 330                 335

Gln Tyr Thr Asn Tyr Asp Ala Cys Leu Lys Ile Ile Asn Leu Met Ala
            340                 345                 350

Leu Gln Leu Tyr Asn Phe Asp Lys Val Ser Phe Glu Lys Phe Phe Thr
        355                 360                 365

His Val Leu Asp Glu Leu Lys Pro Asn Phe Lys Tyr Glu Lys Asp Leu
    370                 375                 380

Lys Gln Thr Cys Gln Ile Leu Ser Ala Ile Gly Ser Gly Asn Val Glu
385                 390                 395                 400

Ile Phe Asn Lys Val Ile Ser Ser Thr Phe Pro Leu Phe Leu Ile Asn
                405                 410                 415

Thr Ser Glu Val Ala Lys Leu Lys Leu Leu Ile Met Asn Phe Ser Phe
            420                 425                 430

Phe Val Asp Ser Tyr Ile Asp Leu Phe Gly Arg Thr Ser Lys Glu Ser
        435                 440                 445

Leu Gly Thr Pro Val Pro Asn Asn Lys Met Ala Glu Tyr Lys Asp Glu

450                 455                 460

Ile Ile Met Ile Leu Ser Met Ala Leu Thr Arg Ser Ser Lys Ala Glu
465                 470                 475                 480

Val Thr Ile Arg Thr Leu Ser Val Ile Gln Phe Thr Lys Met Ile Lys
                485                 490                 495

Met Lys Gly Phe Leu Thr Pro Glu Glu Val Ser Leu Ile Ile Gln Tyr
            500                 505                 510

Phe Thr Glu Glu Ile Leu Thr Asp Asn Asn Lys Asn Ile Tyr Tyr Ala
        515                 520                 525

Cys Leu Glu Gly Leu Lys Thr Ile Ser Glu Ile Tyr Glu Asp Leu Val
    530                 535                 540

Phe Glu Ile Ser Leu Lys Lys Leu Leu Asp Leu Leu Pro Asp Cys Phe
545                 550                 555                 560

Glu Glu Lys Ile Arg Val Asn Asp Glu Glu Asn Ile His Ile Glu Thr
                565                 570                 575

Ile Leu Lys Ile Ile Leu Asp Phe Thr Thr Ser Arg His Ile Leu Val
            580                 585                 590

Lys Glu Ser Ile Thr Phe Leu Ala Thr Lys Leu Asn Arg Val Ala Lys
        595                 600                 605

Ile Ser Lys Ser Arg Glu Tyr Cys Phe Leu Leu Ile Ser Thr Ile Tyr
    610                 615                 620

Ser Leu Phe Asn Asn Asn Asn Gln Asn Glu Asn Val Leu Asn Glu Glu
625                 630                 635                 640

Asp Ala Leu Ala Leu Lys Asn Ala Ile Glu Pro Lys Leu Phe Glu Ile
                645                 650                 655

Ile Thr Gln Glu Ser Ala Ile Val Ser Asp Asn Tyr Asn Leu Thr Leu
            660                 665                 670

Leu Ser Asn Val Leu Phe Phe Thr Asn Leu Lys Ile Pro Gln Ala Ala
        675                 680                 685

His Gln Glu Glu Leu Asp Arg Tyr Asn Glu Leu Phe Ile Ser Glu Gly
    690                 695                 700

Lys Ile Arg Ile Leu Asp Thr Pro Asn Val Leu Ala Ile Ser Tyr Ala
705                 710                 715                 720

Lys Ile Leu Ser Ala Leu Asn Lys Asn Cys Gln Phe Pro Gln Lys Phe
                725                 730                 735

Thr Val Leu Phe Gly Thr Val Gln Leu Leu Lys Lys His Ala Pro Arg
            740                 745                 750

Met Thr Glu Thr Glu Lys Leu Gly Tyr Leu Glu Leu Leu Leu Val Leu
        755                 760                 765

Ser Asn Lys Phe Val Ser Glu Lys Asp Val Ile Gly Leu Phe Asp Trp
    770                 775                 780

Lys Asp Leu Ser Val Ile Asn Leu Glu Val Met Val Trp Leu Thr Lys
785                 790                 795                 800

Gly Leu Ile Met Gln Asn Ser Leu Glu Ser Ser Glu Ile Ala Lys Lys
                805                 810                 815

Phe Ile Asp Leu Leu Ser Asn Glu Glu Ile Gly Ser Leu Val Ser Lys
            820                 825                 830

Leu Phe Glu Val Phe Val Met Asp Ile Ser Ser Leu Lys Lys Phe Lys
        835                 840                 845

Gly Ile Ser Trp Asn Asn Asn Val Lys Ile Leu Tyr Lys Gln Lys Phe
    850                 855                 860

Phe Gly Asp Ile Phe Gln Thr Leu Val Ser Asn Tyr Lys Asn Thr Val
865                 870                 875                 880

Asp Met Thr Ile Lys Cys Asn Tyr Leu Thr Ala Leu Ser Leu Val Leu
                885                 890                 895

Lys His Thr Pro Ser Gln Ser Val Gly Pro Phe Ile Asn Asp Leu Phe
            900                 905                 910

Pro Leu Leu Leu Gln Ala Leu Asp Met Pro Asp Pro Glu Val Arg Val
        915                 920                 925

Ser Ala Leu Glu Thr Leu Lys Asp Thr Thr Asp Lys His His Thr Leu
    930                 935                 940

Ile Thr Glu His Val Ser Thr Ile Val Pro Leu Leu Leu Ser Leu Ser
945                 950                 955                 960

Leu Pro His Lys Tyr Asn Ser Val Ser Val Arg Leu Ile Ala Leu Gln
                965                 970                 975

Leu Leu Glu Met Ile Thr Thr Val Val Pro Leu Asn Tyr Cys Leu Ser
            980                 985                 990

Tyr Gln Asp Asp Val Leu Ser Ala Leu Ile Pro Val Leu Ser Asp Lys
        995                 1000                1005

Lys Arg Ile Ile Arg Lys Gln Cys Val Asp Thr Arg Gln Val Tyr Tyr
    1010                1015                1020

Glu Leu Gly Gln Ile Pro Phe Glu
1025                1030

<210> 224

<211> 542
<212> PRT
<213> Saccharomyces cerevisiae

<400> 224
Met Ser Val Gln Lys Glu Glu Tyr Asp Ile Val Glu Lys Ala Gln Leu
1               5                   10                  15

Ser Val Ser Ala Glu Ser Leu Thr Ser Asp Ser Glu Ser Ile Ser His
            20                  25                  30

Asn Pro Phe Asp Asp Phe His Lys Ala Glu Arg Trp Arg Lys Val Tyr
        35                  40                  45

Glu Ser Ser Gly Tyr Glu Gly Leu Ser Lys Phe Asp Pro Glu Phe Thr
    50                  55                  60

Trp Thr Lys Asp Glu Glu Lys Lys Leu Val Arg Lys Met Asp Leu Lys
65                  70                  75                  80

Ile Phe Leu Trp Val Phe Ile Met Phe Ala Phe Leu Asp Leu Ile Arg
                85                  90                  95

Lys Asn Ile Ala Arg Ala Val Ser Asp Asn Phe Ile Val Asp Leu Lys
            100                 105                 110

Met Asn Thr Asn Asp Tyr Asn Leu Gly Gln Thr Val Tyr Leu Val Ile
        115                 120                 125

Phe Leu Ala Ser Glu Leu Pro Gly Asn Leu Leu Ser Lys Arg Phe Gly
    130                 135                 140

Pro Glu Arg Val Ile Pro Val Gln Ile Val Leu Trp Ser Val Ile Cys
145                 150                 155                 160

Ile Thr Gln Ala Gly Leu Lys Asn Arg Gly Gln Phe Ile Ala Thr Arg
                165                 170                 175

Cys Leu Leu Gly Met Val Gln Gly Gly Phe Ile Pro Asp Asn Ile Leu
            180                 185                 190

Tyr Leu Ser Tyr Tyr Tyr Thr Gly Ala Glu Leu Thr Phe Arg Leu Ser
        195                 200                 205

Phe Phe Trp Cys Ala Ile Pro Leu Phe Gln Ile Leu Gly Ser Leu Leu
    210                 215                 220

Ala Ser Gly Ile Ile Glu Met Arg Gly Ile His Asn Leu Ala Gly Trp
225                 230                 235                 240

Gln Tyr Leu Phe Ile Ile Glu Gly Phe Leu Ser Leu Ser Val Gly Val
                245                 250                 255

Ala Ser Phe Tyr Leu Met Arg Arg Gly Pro Thr Gln Thr Gly Glu Ser
            260                 265                 270

Ala Phe His Lys Gly Lys Ser Leu Phe Thr Glu Tyr Glu Glu Lys Ile
        275                 280                 285

Met Val Asn Arg Ile Leu Arg Asp Asp Pro Ser Lys Gly Asp Met Ser
    290                 295                 300

Asn Arg Gln Pro Val Thr Phe Lys Glu Ile Leu Tyr Thr Leu Thr Glu
305                 310                 315                 320

Phe Asp Leu Trp Pro Leu Phe Ile Gln Gly Ile Thr Ala Phe Ile Ser
                325                 330                 335

Leu Gln Thr Val Gly Ser Tyr Leu Ser Leu Ile Leu Lys Ser Leu Asn
            340                 345                 350

Tyr Ser Thr Phe Leu Ser Asn Ile Leu Ala Ile Pro Gly Gln Ala Leu
        355                 360                 365

Leu Leu Ile Asn Leu Pro Leu Ala Ala Leu Leu Ser Arg Lys Leu Lys
    370                 375                 380

Glu Lys Ser Leu Cys Val Gly Ile Ala Asn Val Trp Val Leu Pro Phe
385                 390                 395                 400

Ile Val Ser Leu Val Ala Leu Pro Thr Asp Thr Asn Pro Trp Ile Lys
                405                 410                 415

Tyr Ile Leu Leu Thr Gly Ile Leu Gly Leu Pro Tyr Thr His Ser Ile
            420                 425                 430

Leu Ala Gly Trp Val Ser Glu Ile Ser Asn Ser Val Arg Ser Arg Thr
        435                 440                 445

Val Gly Thr Ala Leu Tyr Asn Met Ser Ala Gln Val Gly Ala Ile Ile
    450                 455                 460

Ala Ser Asn Met Tyr Arg Asn Asp Asp Lys Pro Tyr Tyr Thr Arg Gly
465                 470                 475                 480

Asn Lys Ile Leu Leu Gly Phe Thr Cys Phe Asn Ile Cys Met Ala Val
                485                 490                 495

Ala Thr Lys Phe Tyr Tyr Ile Ser Arg Asn Lys Tyr Lys Asp Arg Lys
            500                 505                 510

Trp Asn Ser Met Thr Lys Glu Glu Gln Ile Asn Tyr Leu Asp Thr Thr
        515                 520                 525

Lys Asp Lys Gly Met Lys Arg Leu Asp Tyr Arg Phe Ile His
    530                 535                 540

<210> 225
<211> 589
<212> PRT
<213> Saccharomyces cerevisiae

<400> 225
Met Thr Ile Ser Ser Ala His Pro Glu Thr Glu Pro Lys Trp Trp Lys
1               5                   10                  15

Glu Ala Thr Ile Tyr Gln Ile Tyr Pro Ala Ser Phe Lys Asp Ser Asn
            20                  25                  30

Asn Asp Gly Trp Gly Asp Met Lys Gly Ile Ala Ser Lys Leu Glu Tyr
        35                  40                  45

Ile Lys Glu Leu Gly Thr Asp Ala Ile Trp Ile Ser Pro Phe Tyr Asp
    50                  55                  60

Ser Pro Gln Asp Asp Met Gly Tyr Asp Ile Ala Asn Tyr Glu Lys Val
65                  70                  75                  80

Trp Pro Thr Tyr Gly Thr Asn Glu Asp Cys Phe Ala Leu Ile Glu Lys
                85                  90                  95

Thr His Lys Leu Gly Met Lys Phe Ile Thr Asp Leu Val Ile Asn His
            100                 105                 110

Cys Ser Ser Glu His Glu Trp Phe Lys Glu Ser Arg Ser Ser Lys Thr
        115                 120                 125

Asn Pro Lys Arg Asp Trp Phe Phe Trp Arg Pro Pro Lys Gly Tyr Asp
    130                 135                 140

Ala Glu Gly Lys Pro Ile Pro Pro Asn Asn Trp Arg Ser Tyr Phe Gly
145                 150                 155                 160

Gly Ser Ala Trp Thr Phe Asp Glu Lys Thr Gln Glu Phe Tyr Leu Arg
                165                 170                 175

Leu Phe Cys Ser Thr Gln Pro Asp Leu Asn Trp Glu Asn Glu Asp Cys
            180                 185                 190

Arg Lys Ala Ile Tyr Glu Ser Ala Val Gly Tyr Trp Leu Asp His Gly
        195                 200                 205

Val Asp Gly Phe Arg Ile Asp Val Gly Ser Leu Tyr Ser Lys Val Ala
    210                 215                 220

Gly Leu Pro Asp Ala Pro Val Ile Asp Glu Asn Ser Lys Trp Gln Leu
225                 230                 235                 240

Ser Asp Pro Phe Thr Met Asn Gly Pro Arg Ile His Glu Phe His Gln
                245                 250                 255

Glu Met Asn Lys Phe Ile Arg Asn Arg Val Lys Asp Gly Arg Glu Ile
            260                 265                 270

Met Thr Val Gly Glu Met Arg His Ala Thr Asp Glu Thr Lys Arg Leu
        275                 280                 285

Tyr Thr Ser Ala Ser Arg His Glu Leu Ser Glu Leu Phe Asn Phe Ser
    290                 295                 300

His Thr Asp Val Gly Thr Ser Pro Lys Phe Arg Gln Asn Leu Ile Pro
305                 310                 315                 320

Tyr Glu Leu Lys Asp Trp Lys Val Ala Leu Ala Glu Leu Phe Arg Tyr
                325                 330                 335

Val Asn Gly Thr Asp Cys Trp Ser Thr Ile Tyr Leu Glu Asn His Asp
            340                 345                 350

Gln Pro Arg Ser Ile Thr Arg Phe Gly Asp Asp Ser Pro Lys Asn Arg
        355                 360                 365

Val Ile Ser Gly Lys Leu Leu Ser Val Leu Leu Val Ser Leu Ser Gly
    370                 375                 380

Thr Leu Tyr Val Tyr Gln Gly Gln Glu Leu Gly Glu Ile Asn Phe Lys
385                 390                 395                 400

Asn Trp Pro Ile Glu Lys Tyr Glu Asp Val Glu Val Arg Asn Asn Tyr
                405                 410                 415

Asp Ala Ile Lys Glu Glu His Gly Glu Asn Ser Lys Glu Met Lys Arg
            420                 425                 430

Phe Leu Glu Ala Ile Ala Leu Ile Ser Arg Asp His Ala Arg Thr Pro
        435                 440                 445

Met Gln Trp Ser Arg Glu Glu Pro Asn Ala Gly Phe Ser Gly Pro Asn
    450                 455                 460

Ala Lys Pro Trp Phe Tyr Leu Asn Glu Ser Phe Arg Glu Gly Ile Asn
465                 470                 475                 480

Ala Glu Asp Glu Ser Lys Asp Pro Asn Ser Val Leu Asn Phe Trp Lys
                485                 490                 495

Glu Ala Leu Arg Phe Arg Lys Ala His Lys Asp Ile Thr Val Tyr Gly
            500                 505                 510

Tyr Asp Phe Glu Phe Ile Asp Leu Asp Asn Lys Lys Leu Phe Ser Phe
        515                 520                 525

Thr Lys Lys Tyr Asp Asn Lys Thr Leu Phe Ala Ala Leu Asn Phe Ser
    530                 535                 540

Ser Asp Ser Ile Asp Phe Thr Ile Pro Asn Asn Ser Ser Ser Phe Lys
545                 550                 555                 560

Leu Glu Phe Gly Asn Tyr Pro Arg Ser Glu Val Asp Ala Ser Ser Arg
                565                 570                 575

Thr Leu Lys Pro Trp Glu Gly Arg Ile Tyr Ile Ser Glu
            580                 585

<210> 226
<211> 666
<212> PRT
<213> Saccharomyces cerevisiae

<400> 226
Met Gly Lys Thr Lys Thr Arg Gly Arg Arg His Gln Asp Lys Gln Arg
1               5                   10                  15

Lys Asp Glu Phe Glu Pro Ser Ser Asn Ser Ala Lys Glu His Ile Gln
            20                  25                  30

Gln Glu Glu Ser Thr Tyr Asn Asp Glu Ala Glu Ile Lys Glu Thr Gln
        35                  40                  45

Pro Gln Met Phe Phe Gly Val Leu Asp Arg Glu Glu Leu Glu Tyr Phe
    50                  55                  60

Lys Gln Ala Glu Ser Thr Leu Gln Leu Asp Ala Phe Glu Ala Pro Glu
65                  70                  75                  80

Glu Lys Phe Gln Phe Val Thr Ser Ile Ile Glu Glu Ala Lys Gly Lys
                85                  90                  95

Glu Leu Lys Leu Val Thr Ser Gln Ile Thr Ser Lys Leu Met Glu Arg
            100                 105                 110

Val Ile Leu Glu Cys Asp Glu Thr Gln Leu Lys Asp Ile Phe Gln Ser
        115                 120                 125

Phe Asn Gly Val Phe Phe Gly Leu Ser Cys His Lys Tyr Ala Ser His
    130                 135                 140

Val Leu Glu Thr Leu Phe Val Arg Ser Ala Ala Leu Val Glu Arg Glu
145                 150                 155                 160

Leu Leu Thr Pro Ser Phe Asp Asn Asn Glu Lys Glu Gly Pro Tyr Val
                165                 170                 175

Thr Met Glu Asn Met Phe Leu Phe Met Leu Asn Glu Leu Lys Pro His
            180                 185                 190

Leu Lys Thr Met Met Asn His Gln Tyr Ala Ser His Val Leu Arg Leu
        195                 200                 205

Leu Ile Leu Ile Leu Ser Ser Lys Thr Leu Pro Asn Ser Thr Lys Ala
    210                 215                 220

Asn Ser Thr Leu Arg Ser Lys Lys Ser Lys Ile Ala Arg Lys Met Ile
225                 230                 235                 240

Asp Ile Lys Asp Asn Asp Asp Phe Asn Lys Val Tyr Gln Thr Pro Glu
                245                 250                 255

Ser Phe Lys Ser Glu Leu Arg Asp Ile Ile Thr Thr Leu Tyr Lys Gly
            260                 265                 270

Phe Thr Asn Gly Ala Glu Ser Arg Ser Asp Ile Ser Gln Ser Thr Ile
        275                 280                 285

Thr Lys Phe Arg Glu Tyr Ser Val Asp Lys Val Ala Ser Pro Val Ile
    290                 295                 300

Gln Leu Ile Ile Gln Val Glu Gly Ile Phe Asp Arg Asp Arg Ser Phe
305                 310                 315                 320

Trp Arg Leu Val Phe Asn Thr Ala Asp Glu Lys Asp Pro Lys Glu Glu
                325                 330                 335

Ser Phe Leu Glu Tyr Leu Leu Ser Asp Pro Val Gly Ser His Phe Leu
            340                 345                 350

Glu Asn Val Ile Gly Ser Ala Arg Leu Lys Tyr Val Glu Arg Leu Tyr
        355                 360                 365

Arg Leu Tyr Met Lys Asp Arg Ile Val Lys Leu Ala Lys Arg Asp Thr
    370                 375                 380

Thr Gly Ala Phe Val Val Arg Ala Leu Leu Glu His Leu Lys Glu Lys
385                 390                 395                 400

Asp Val Lys Gln Ile Leu Asp Ala Val Val Pro Glu Leu Ser Met Leu
                405                 410                 415

Leu Asn Ser Asn Met Asp Phe Gly Thr Ala Ile Ile Asn Ala Ser Asn
            420                 425                 430

Lys Gln Gly Gly Tyr Leu Arg Asp Asp Val Ile Ala Gln Leu Ile Gln
        435                 440                 445

Lys Tyr Tyr Pro Glu Lys Ser Asp Ala Lys Asn Ile Leu Glu Ser Cys
    450                 455                 460

Leu Leu Leu Ser Ala Ser Thr Leu Gly Asn Thr Arg Asp Asp Trp Pro
465                 470                 475                 480

Thr Ala Glu Glu Arg Arg Arg Ser Val Phe Leu Glu Gln Leu Ile Asp
                485                 490                 495

Tyr Asp Asp Lys Phe Leu Asn Ile Thr Ile Asp Ser Met Leu Ala Leu
            500                 505                 510

Pro Glu Glu Arg Leu Ile Gln Met Cys Tyr His Gly Val Phe Ser His
        515                 520                 525

Val Val Glu His Val Leu Gln Thr Thr Arg Val Asp Ile Ile Lys Arg
    530                 535                 540

Lys Met Leu Leu Asn Ile Leu Ser Lys Glu Ser Val Asn Leu Ala Cys
545                 550                 555                 560

Asn Val Tyr Gly Ser His Ile Met Asp Lys Leu Trp Glu Phe Thr Ala
                565                 570                 575

Lys Leu Thr Leu Tyr Lys Glu Arg Ile Ala Arg Ala Leu Val Leu Glu
            580                 585                 590

Thr Glu Lys Val Lys Asn Ser Ile Tyr Gly Arg Gln Val Trp Lys Asn
        595                 600                 605

Trp Lys Leu Glu Leu Tyr Val Arg Lys Met Trp Asp Trp Lys Lys Leu
    610                 615                 620

Ile Lys Glu Gln Glu Phe Glu Ile Phe Pro Asn Ser Lys Pro Leu Gln
625                 630                 635                 640

Pro Lys Pro Glu Lys His Ser Arg Glu Arg Asn Asn Ser Lys Glu Gly
                645                 650                 655

Ser Ala Phe Lys Lys Gln Lys His Tyr Arg
            660                 665

<210> 227
<211> 365
<212> PRT
<213> Saccharomyces cerevisiae

<400> 227
Met Pro Asn Thr Ala Pro Pro Leu Pro Arg Ala Pro Pro Val Pro Pro
  1               5                  10                  15

Ala Thr Phe Glu Phe Asp Ser Glu Pro Thr Ala Thr His Ser His Thr
            20                  25                  30

Ala Pro Ser Pro Pro Pro His Gln Asn Val Thr Ala Ser Thr Pro Ser
        35                  40                  45

Met Met Ser Thr Gln Gln Arg Val Pro Thr Ser Val Leu Ser Gly Ala
    50                  55                  60

Glu Lys Glu Ser Arg Thr Leu Pro Pro His Val Pro Ser Leu Thr Asn
65                  70                  75                  80

Arg Pro Val Asp Ser Phe His Glu Ser Asp Thr Thr Pro Lys Val Ala
                85                  90                  95

Ser Ile Arg Arg Ser Thr Thr His Asp Val Gly Glu Ile Ser Asn Asn
            100                 105                 110

Val Lys Ile Glu Phe Asn Ala Gln Glu Arg Trp Trp Ile Asn Lys Ser
        115                 120                 125

Ala Pro Pro Ala Ile Ser Asn Leu Lys Leu Asn Phe Leu Met Glu Ile
    130                 135                 140

Asp Asp His Phe Ile Ser Lys Arg Leu His Gln Lys Trp Val Val Arg
145                 150                 155                 160

Asp Phe Tyr Phe Leu Phe Glu Asn Tyr Ser Gln Leu Arg Phe Ser Leu
                165                 170                 175

Thr Phe Asn Ser Thr Ser Pro Glu Lys Thr Val Thr Thr Leu Gln Glu
            180                 185                 190

Arg Phe Pro Ser Pro Val Glu Thr Gln Ser Ala Arg Ile Leu Asp Glu
        195                 200                 205

Tyr Ala Gln Arg Phe Asn Ala Lys Val Val Glu Lys Ser His Ser Leu
    210                 215                 220

Ile Asn Ser His Ile Gly Ala Lys Asn Phe Val Ser Gln Ile Val Ser
225                 230                 235                 240

Glu Phe Lys Asp Glu Val Ile Gln Pro Ile Gly Ala Arg Thr Phe Gly
                245                 250                 255

Ala Thr Ile Leu Ser Tyr Lys Pro Glu Glu Gly Ile Glu Gln Leu Met
            260                 265                 270

Lys Ser Leu Gln Lys Ile Lys Pro Gly Asp Ile Leu Val Ile Arg Lys
        275                 280                 285

Ala Lys Phe Glu Ala His Lys Lys Ile Gly Lys Asn Glu Ile Ile Asn
    290                 295                 300

Val Gly Met Asp Ser Ala Ala Pro Tyr Ser Ser Val Val Thr Asp Tyr
305                 310                 315                 320

Asp Phe Thr Lys Asn Lys Phe Arg Val Ile Glu Asn His Glu Gly Lys
                325                 330                 335

Ile Ile Gln Asn Ser Tyr Lys Leu Ser His Met Lys Ser Gly Lys Leu
            340                 345                 350

Lys Val Phe Arg Ile Val Ala Arg Gly Tyr Val Gly Trp
        355                 360                 365


<210> 228
<211> 219
<212> PRT
<213> Saccharomyces cerevisiae

<400> 228
Met Arg Phe Gln Leu Phe Ile Tyr Phe Tyr Phe Thr Ile Val Val Ile
1               5                   10                  15

Ala Gly Thr Asn Thr Ile Gln Gln Phe Ser Asp Ala Gly Asp Arg Leu
            20                  25                  30

Ile Thr Ser Leu Arg Asn Leu Asp Asn Asn Gly Thr Tyr Glu Thr Leu

        35                  40                  45

Thr Ala Glu Lys Val Pro Ile Ile Glu Gly Gln Ile Gln Asn Ile Ser
    50                  55                  60

Ala Lys Tyr Glu Gln His Thr Phe Ile Leu Lys Gly Leu Glu Ala Val
65                  70                  75                  80

Leu Asn Tyr Lys Val Lys Ser Leu Asp Asn Asn Glu Arg Glu Ser Leu
                85                  90                  95

Glu Ile Glu Tyr Glu Lys Val Glu Lys Ala Leu Asp Ala Ala Leu Asn
            100                 105                 110

Val Ser Pro Phe Glu Tyr Ile Lys Lys Phe Lys Glu Val Ser Arg Gly
        115                 120                 125

Lys Val Val Asn Ala Leu Glu Asn Leu Ser Arg Glu Gln Asn Arg Ile
    130                 135                 140

Thr Ile Asn Gly Gly Arg Glu Asp Glu Lys Glu Lys Glu Ala Arg Glu
145                 150                 155                 160

Lys Lys Lys Arg Leu Asp Arg Ile Lys Arg Ile Leu Thr Val Ser Leu
                165                 170                 175

Leu Glu Leu Gly Leu Ala Gln Gly Val Ala Asp Leu Cys Ala Val Ala
            180                 185                 190

Pro Phe Ala Cys Leu Leu Gly Val Thr Val Gly Ser Ile Gly Phe Ile
        195                 200                 205

Phe Trp Leu Ala Leu Ile Tyr Asn Ala Ile Gln
    210                 215

<210> 229
<211> 133
<212> PRT
<213> Saccharomyces cerevisiae

<400> 229
Met Asp Arg Asp His Ile Asn Asp His Asp His Arg Met Ser Tyr Ser
1               5                   10                  15

Ile Asn Lys Asp Asp Leu Leu Leu Met Val Leu Ala Val Phe Ile Pro
            20                  25                  30

Pro Val Ala Val Trp Lys Arg Lys Gly Met Phe Asn Arg Asp Thr Leu
        35                  40                  45

Leu Asn Leu Leu Leu Phe Leu Leu Leu Phe Phe Pro Ala Ile Ile His
    50                  55                  60

Ala Cys Tyr Val Val Tyr Glu Thr Ser Ser Glu Arg Ser Tyr Asp Leu
65                  70                  75                  80

Ser Arg Arg His Ala Thr Ala Pro Ala Val Asp Arg Asp Leu Glu Ala
                85                  90                  95

His Pro Ala Glu Glu Ser Gln Ala Gln Pro Pro Ala Tyr Asp Glu Asp
            100                 105                 110

Asp Glu Ala Gly Ala Asp Val Pro Leu Met Asp Asn Lys Gln Gln Leu
        115                 120                 125

Ser Ser Gly Arg Thr
    130

<210> 230
<211> 108
<212> PRT
<213> Saccharomyces cerevisiae

<400> 230
Met Val Ala Ser Cys Lys Asp Gln Lys Lys Ala Val Ala Ile Cys Leu
  1               5                  10                  15

Gln Arg Ser Pro Cys Val Met Ile Glu Arg His Asn Pro Gln Glu Cys
             20                  25                  30

Leu Asp Asn Pro Glu Leu Asn Lys Asp Leu Pro Glu Leu Cys Ile Ala
         35                  40                  45

Gln Met Lys Ala Phe Leu Asp Cys Lys Arg Gly Ile Val Asp Met Thr
     50                  55                  60

Lys Arg Phe Thr Gly Asn Ala Pro Leu Ser Thr Gly Lys Tyr Asp Gln
 65                  70                  75                  80

Gln Tyr Glu Asn Leu Cys Lys Gly Lys Phe Asp Pro Arg Glu Glu Met
                 85                  90                  95

Glu Lys Leu Lys Leu Leu Asn Ser Gln Gln Lys Asp
            100                 105

<210> 231
<211> 453
<212> PRT
<213> Saccharomyces cerevisiae

<400> 231
Met Asn Asn Thr Ser Ile Thr Gly Pro Gln Val Leu His Arg Thr Lys
  1               5                  10                  15

Met Arg Pro Leu Pro Val Leu Glu Lys Tyr Cys Ile Ser Pro His His
             20                  25                  30

Gly Phe Leu Asp Asp Arg Leu Pro Leu Thr Arg Leu Ser Ser Lys Lys
         35                  40                  45

Tyr Met Lys Trp Glu Glu Ile Val Ala Asp Leu Pro Ser Leu Leu Gln
    50                  55                  60

Glu Asp Asn Lys Val Arg Ser Val Ile Asp Gly Leu Asp Val Leu Asp
65                  70                  75                  80

Leu Asp Glu Thr Ile Leu Gly Asp Val Arg Glu Leu Arg Arg Ala Tyr
                85                  90                  95

Ser Ile Leu Gly Phe Met Ala His Ala Tyr Ile Trp Ala Ser Gly Thr
            100                 105                 110

Pro Arg Asp Val Leu Pro Glu Cys Ile Ala Arg Pro Leu Leu Glu Thr
        115                 120                 125

Ala His Ile Leu Gly Val Pro Pro Leu Ala Thr Tyr Ser Ser Leu Val
    130                 135                 140

Leu Trp Asn Phe Lys Val Thr Asp Glu Cys Lys Lys Thr Glu Thr Gly
145                 150                 155                 160

Cys Leu Asp Leu Glu Asn Ile Thr Thr Ile Asn Thr Phe Thr Gly Thr
                165                 170                 175

Val Asp Glu Ser Trp Phe Tyr Leu Val Ser Val Arg Phe Glu Lys Ile
            180                 185                 190

Gly Ser Ala Cys Leu Asn His Gly Leu Gln Ile Leu Arg Ala Ile Arg
        195                 200                 205

Ser Gly Asp Lys Gly Asp Ala Asn Val Ile Asp Gly Leu Glu Gly Leu
    210                 215                 220

Ala Ala Thr Ile Glu Arg Leu Ser Lys Ala Leu Met Glu Met Glu Leu
225                 230                 235                 240

Lys Cys Glu Pro Asn Val Phe Tyr Phe Lys Ile Arg Pro Phe Leu Ala
                245                 250                 255

Gly Trp Thr Asn Met Ser His Met Gly Leu Pro Gln Gly Val Arg Tyr
            260                 265                 270

Gly Ala Glu Gly Gln Tyr Arg Ile Phe Ser Gly Gly Ser Asn Ala Gln
        275                 280                 285

Ser Ser Leu Ile Gln Thr Leu Asp Ile Leu Leu Gly Val Lys His Thr
    290                 295                 300

Ala Asn Ala Ala His Ser Ser Gln Gly Asp Ser Lys Ile Asn Tyr Leu
305                 310                 315                 320

Asp Glu Met Lys Lys Tyr Met Pro Arg Glu His Arg Glu Phe Leu Tyr
                325                 330                 335

His Leu Glu Ser Val Cys Asn Ile Arg Glu Tyr Val Ser Arg Asn Ala

340                 345                 350

Ser Asn Arg Ala Leu Gln Glu Ala Tyr Gly Arg Cys Ile Ser Met Leu
        355                 360                 365

Lys Ile Phe Arg Asp Asn His Ile Gln Ile Val Thr Lys Tyr Ile Ile
    370                 375                 380

Leu Pro Ser Asn Ser Lys Gln His Gly Ser Asn Lys Pro Asn Val Leu
385                 390                 395                 400

Ser Pro Ile Glu Pro Asn Thr Lys Ala Ser Gly Cys Leu Gly His Lys
                405                 410                 415

Val Ala Ser Ser Lys Thr Ile Gly Thr Gly Gly Thr Arg Leu Met Pro
            420                 425                 430

Phe Leu Lys Gln Cys Arg Asp Glu Thr Val Ala Thr Ala Asp Ile Lys
        435                 440                 445

Asn Glu Asp Lys Asn
    450


<210> 232
<211> 361
<212> PRT
<213> Saccharomyces cerevisiae

<400> 232
Met Ile Ser Ala Asn Ser Leu Leu Ile Ser Thr Leu Cys Ala Phe Ala
1               5                   10                  15

Ile Ala Thr Pro Leu Ser Lys Arg Asp Ser Cys Thr Leu Thr Gly Ser
            20                  25                  30

Ser Leu Ser Ser Leu Ser Thr Val Lys Lys Cys Ser Ser Ile Val Ile
        35                  40                  45

Lys Asp Leu Thr Val Pro Ala Gly Gln Thr Leu Asp Leu Thr Gly Leu
    50                  55                  60

Ser Ser Gly Thr Thr Val Thr Phe Glu Gly Thr Thr Thr Phe Gln Tyr
65                  70                  75                  80

Lys Glu Trp Ser Gly Pro Leu Ile Ser Ile Ser Gly Ser Lys Ile Ser
                85                  90                  95

Val Val Gly Ala Ser Gly His Thr Ile Asp Gly Gln Gly Ala Lys Trp
            100                 105                 110

Trp Asp Gly Leu Gly Asp Ser Gly Lys Val Lys Pro Lys Phe Val Lys
        115                 120                 125

Leu Ala Leu Thr Gly Thr Ser Lys Val Thr Gly Leu Asn Ile Lys Asn
    130                 135                 140

Ala Pro His Gln Val Phe Ser Ile Asn Lys Cys Ser Asp Leu Thr Ile
145                 150                 155                 160

Ser Asp Ile Thr Ile Asp Ile Arg Asp Gly Asp Ser Ala Gly Gly His
                165                 170                 175

Asn Thr Asp Gly Phe Asp Val Gly Ser Ser Ser Asn Val Leu Ile Gln
            180                 185                 190

Gly Cys Thr Val Tyr Asn Gln Asp Asp Cys Ile Ala Val Asn Ser Gly
        195                 200                 205

Ser Thr Ile Lys Phe Met Asn Asn Tyr Cys Tyr Asn Gly His Gly Ile
    210                 215                 220

Ser Val Gly Ser Val Gly Gly Arg Ser Asp Asn Thr Val Asn Gly Phe
225                 230                 235                 240

Trp Ala Glu Asn Asn His Val Ile Asn Ser Asp Asn Gly Leu Arg Ile
                245                 250                 255

Lys Thr Val Glu Gly Ala Thr Gly Thr Val Thr Asn Val Asn Phe Ile
            260                 265                 270

Ser Asn Lys Ile Ser Gly Ile Lys Ser Tyr Gly Ile Val Ile Glu Gly
        275                 280                 285

Asp Tyr Leu Asn Ser Lys Thr Thr Gly Thr Ala Thr Gly Gly Val Pro
    290                 295                 300

Ile Ser Asn Leu Val Met Lys Asp Ile Thr Gly Ser Val Asn Ser Thr
305                 310                 315                 320

Ala Lys Arg Val Lys Ile Leu Val Lys Asn Ala Thr Asn Trp Gln Trp
                325                 330                 335

Ser Gly Val Ser Ile Thr Gly Gly Ser Ser Tyr Ser Gly Cys Ser Gly
            340                 345                 350

Ile Pro Ser Gly Ser Gly Ala Ser Cys
        355                 360


<210> 233
<211> 167
<212> PRT
<213> Saccharomyces cerevisiae

<400> 233
Met Gln Glu Phe Tyr Ser Phe Ser Pro Ile Asp Glu Asn Gly Asn Pro
  1               5                  10                  15

Phe Pro Phe Asn Ser Leu Arg Asn Lys Val Val Leu Ile Val Asn Val
             20                  25                  30

Ala Ser His Cys Ala Phe Thr Pro Gln Tyr Lys Glu Leu Glu Tyr Leu
        35                  40                  45

Tyr Glu Lys Tyr Lys Ser His Gly Leu Val Ile Val Ala Phe Pro Cys
    50                  55                  60

Gly Gln Phe Gly Asn Gln Glu Phe Glu Lys Asp Lys Glu Ile Asn Lys
65                  70                  75                  80

Phe Cys Gln Asp Lys Tyr Gly Val Thr Phe Pro Ile Leu His Lys Ile
                85                  90                  95

Arg Cys Asn Gly Gln Lys Gln Asp Pro Val Tyr Lys Phe Leu Lys Asn
            100                 105                 110

Ser Val Ser Gly Lys Ser Gly Ile Lys Met Ile Lys Trp Asn Phe Glu
        115                 120                 125

Lys Phe Val Val Asp Arg Asn Gly Lys Val Val Lys Arg Phe Ser Cys
    130                 135                 140

Met Thr Arg Pro Leu Glu Leu Cys Pro Ile Ile Glu Glu Leu Leu Asn
145                 150                 155                 160

Gln Pro Pro Glu Glu Gln Ile
                165

<210> 234
<211> 516
<212> PRT
<213> Saccharomyces cerevisiae

<400> 234
Met Asn Ser Gly Gly Glu Glu Pro Thr Ile Lys Pro Asn Val Phe Asn
1               5                   10                  15

Ile Thr Gln Leu Leu Asn Ser Asn Gly Glu Lys Pro Gly Ile Ala Cys
            20                  25                  30

Ile Phe Leu Ser Lys Phe Asp Met Lys Lys Gly Asn Ile Ile Ile Trp
        35                  40                  45

Ser Lys Ser Ile Asn Gly Ala Ala Ile Asp Leu Ser Asn Ile Glu Phe
    50                  55                  60

Lys Ser Leu Pro Ala Gly Ile His Glu Gln Thr Asp Asp Val Val Asn
65                  70                  75                  80

Phe Val Val Pro Lys Glu Leu Asp Val Cys Gln Thr Ala Lys Thr Thr
                85                  90                  95

Thr Tyr Asp Tyr Gly Ile Ala Tyr Phe Lys Gln Asn Ser Phe Asp Ile
            100                 105                 110

Ile Glu Asn Asp Asn Arg Ile Asp Arg Ser Lys Val Gln Met Phe Ser

115                 120                 125

Leu Gly Val Ile Ile Asp Val Gln Asn Ala Ser Ser Asp Ser Lys Lys
    130                 135                 140

His Phe Tyr Lys Glu Ile Tyr His Ala Tyr Ala Ala Asn Arg Tyr Ser
145                 150                 155                 160

Ser Tyr Leu Glu Ser Leu Leu Gly Gln Trp Ile Arg Gln Arg Asp Leu
                165                 170                 175

Asp Lys Phe Asp Ile Phe Glu Lys Phe Phe Asp Glu Asn Asn Gln Gly
            180                 185                 190

His Met Ala Glu Asn Ser Val Glu Val Phe Glu His Ser Pro Lys Glu
        195                 200                 205

Arg Arg His Leu Val Glu Tyr Leu Pro Tyr Trp Thr Arg Lys Leu Gly
    210                 215                 220

Pro Leu Ile Phe Pro Leu Trp Lys Ala Ser Leu Leu Gln Ser Arg Ile
225                 230                 235                 240

Leu Ile Leu Val Pro Gln Gly Glu Ser Phe Glu Leu Cys Asn Ser Leu
                245                 250                 255

Ala Tyr Cys Val Phe Leu Ile Ser Met Leu Pro Lys Asn Leu Ile Gly
            260                 265                 270

Asn His Val Ser Asp Glu Tyr Ile Lys Pro Ile Phe Thr Val Ser Thr
        275                 280                 285

Ser Asp Ile Pro Phe Leu Glu Ser Phe Lys Lys Gly Asn Gly Tyr Val
    290                 295                 300

Ala Thr Thr Ser Glu Glu Ile Leu Leu Tyr Lys Pro Glu Ile Tyr Asp
305                 310                 315                 320

Ile Val Val Lys Leu Thr Ser Ser Ser Thr Ile Glu Glu Ser Pro Glu
                325                 330                 335

Lys Glu Val Glu Ile Leu Thr Ala Ser Gly Glu Gln Asn Lys Ala Thr
            340                 345                 350

Pro Leu Asp Leu Glu Val Tyr Glu Lys Leu Ile Leu Gly Glu Leu Gln
        355                 360                 365

Glu Asp Ala Ser Thr Asn Ala Thr Cys Arg His His Glu Val Thr Glu
    370                 375                 380

Pro Ile Ser Trp Leu Gln Phe Leu Ile Asp Gly Phe Phe Leu Leu Thr
385                 390                 395                 400

Thr Ala Gly Tyr Leu Val Ala Pro Tyr His Leu Ala Asn Asn Phe Lys
                405                 410                 415

Ile Pro Arg His Val Ser Gly Pro Glu Pro Asn Asn Ser Glu Ile Gln
            420                 425                 430

Ile Ala Glu Asn Leu Val Arg Tyr Phe His Arg Arg Thr Ser Asn Leu
        435                 440                 445

Tyr Asn Asp Leu Lys Asp Val Ile Gln Lys Ser Glu Asn Ile Asp Ser
    450                 455                 460

Glu Gln Pro Ile Thr Ile Ala Ala Ser Phe Leu Thr Lys Leu Asn Leu
465                 470                 475                 480

Asp Cys Phe Ser Lys Gln Asp His Gln Phe Val Lys Asp Ile Ala Leu
                485                 490                 495

Lys Trp Phe Gln Arg Ser Ile Asp Ile Ser Asn Leu Pro Glu Cys Leu
            500                 505                 510

Gly Asn Leu Cys
        515


<210> 235
<211> 450
<212> PRT
<213> Saccharomyces cerevisiae

<400> 235
Met Ala Lys Asp Leu Leu Pro Lys Gln Ala Ala Asn Glu Pro Ser Leu
1               5                   10                  15

Lys Asp Cys Thr Cys Lys Arg Cys Leu Lys Leu Gly Ala Ser Lys Glu
            20                  25                  30

Lys Lys Ile Arg Arg Lys Lys Lys Gly Glu Glu Lys Arg Glu Arg His
        35                  40                  45

Tyr Gly Asn Arg Arg Lys Leu Thr Phe Asn Phe Leu Lys His Thr Asn
    50                  55                  60

Met Glu Asn Thr Asn Tyr Asp Val Ile Thr Ser Val Gly Tyr Leu Asn
65                  70                  75                  80

Glu Lys Tyr Gly Leu Lys Lys Ser His Tyr Ile Glu Lys Phe Ile Lys
                85                  90                  95

Cys Ile His Arg Lys Ile Asn Ile Asp Val Ser Lys Ile Thr Asp Ala
            100                 105                 110

Tyr Val Asn Ser Leu Asn Pro Trp Val Lys Val Lys Leu Phe Leu Leu
        115                 120                 125

Leu Val Thr Leu Ser Glu Lys Gly Gly Pro Glu Tyr Trp Leu Asp Lys
    130                 135                 140

Thr Asp Gly Glu Lys Asn Ser Glu Ala Ser Ser Thr Asp Asn Ser Leu

145                 150                 155                 160

Glu Asn Ser Thr Lys Gly Ala Asp Ser Ala Gly Ser Thr Ala Leu Arg
                165                 170                 175

Asp Glu Met Val Lys Ser His Lys Asn Leu Phe Pro Thr Leu Thr Glu
            180                 185                 190

Gln Ile Ile Gln His Asn Ile Asn Gln Asp Phe Thr Glu Ser Thr Tyr
        195                 200                 205

Asp Glu Asp Tyr Val Phe Ser Ser Ile Trp Ala Asn Phe Met Glu Gly
    210                 215                 220

Leu Ile Asn His Tyr Leu Glu Lys Val Ile Val Pro Tyr Ser Glu Met
225                 230                 235                 240

Lys Val Cys Gln Gln Leu Tyr Lys Pro Met Met Lys Ile Ile Ser Leu
                245                 250                 255

Tyr Asn Glu Tyr Asn Glu Leu Met Val Lys Ser Glu Lys Asn Gly Phe
            260                 265                 270

Leu Pro Ser Leu Gln Asp Ser Glu Asn Val Gln Gly Asp Lys Gly Glu
        275                 280                 285

Lys Glu Ser Lys Asp Asp Ala Val Ser Gln Glu Arg Leu Glu Arg Ala
    290                 295                 300

Gln Lys Leu Leu Trp Gln Ala Arg Glu Asp Ile Pro Lys Thr Ile Ser
305                 310                 315                 320

Lys Glu Leu Thr Leu Leu Ser Glu Met Tyr Ser Thr Leu Ser Ala Asp
                325                 330                 335

Glu Gln Asp Tyr Glu Leu Asp Glu Phe Val Cys Cys Ala Glu Glu Tyr
            340                 345                 350

Ile Glu Leu Glu Tyr Leu Pro Ala Leu Val Asp Val Leu Phe Ala Asn
        355                 360                 365

Cys Gly Thr Asn Asn Phe Trp Lys Ile Met Leu Val Leu Glu Pro Phe
    370                 375                 380

Phe Tyr Tyr Ile Glu Asp Val Gly Gly Asp Asp Asp Glu Asp Glu Asp
385                 390                 395                 400

Asn Val Asp Asn Ser Glu Gly Asp Glu Glu Ser Leu Leu Ser Arg Asn
                405                 410                 415

Val Glu Gly Asp Asp Asn Val Val Glu Arg His Phe Lys Pro Asp Pro
            420                 425                 430

Arg Val Ile Thr Leu Glu Lys Ile Cys Glu Val Ala Ala Arg Gln Lys
        435                 440                 445

Trp Ile
    450

<210> 236
<211> 412
<212> PRT
<213> Saccharomyces cerevisiae

<400> 236
Met Ser Gln Gly Thr Leu Tyr Ile Asn Arg Ser Pro Arg Asn Tyr Ala
1               5                   10                  15

Ser Glu Ala Leu Ile Ser Tyr Phe Lys Leu Asp Val Lys Ile Val Asp
            20                  25                  30

Leu Glu Gln Ser Ser Glu Phe Ala Ser Leu Phe Pro Leu Lys Gln Ala
        35                  40                  45

Pro Ala Phe Leu Gly Pro Lys Gly Leu Lys Leu Thr Glu Ala Leu Ala
    50                  55                  60

Ile Gln Phe Tyr Leu Ala Asn Gln Val Ala Asp Glu Lys Glu Arg Ala
65                  70                  75                  80

Arg Leu Leu Gly Ser Asp Val Ile Glu Lys Ser Gln Ile Leu Arg Trp
                85                  90                  95

Ala Ser Leu Ala Asn Ser Asp Val Met Ser Asn Ile Ala Arg Pro Phe
            100                 105                 110

Leu Ser Phe Lys Gly Leu Ile Pro Tyr Asn Lys Lys Asp Val Asp Ala
        115                 120                 125

Cys Phe Val Lys Ile Asp Asn Leu Ala Ala Val Phe Asp Ala Arg Leu
    130                 135                 140

Arg Asp Tyr Thr Phe Val Ala Thr Glu Asn Ile Ser Leu Gly Asp Leu
145                 150                 155                 160

His Ala Ala Gly Ser Trp Ala Phe Gly Leu Ala Thr Ile Leu Gly Pro
                165                 170                 175

Glu Trp Arg Ala Lys His Pro His Leu Met Arg Trp Phe Asn Thr Val
            180                 185                 190

Ala Ala Ser Pro Ile Val Lys Thr Pro Phe Ala Glu Val Lys Leu Ala
        195                 200                 205

Glu Lys Ala Leu Thr Tyr Thr Pro Pro Lys Lys Gln Lys Ala Glu Lys
    210                 215                 220

Pro Lys Ala Glu Lys Ser Lys Ala Glu Lys Lys Lys Asp Glu Ala Lys
225                 230                 235                 240

Pro Ala Asp Asp Ala Ala Pro Ala Lys Lys Pro Lys His Pro Leu Glu

245                 250                 255

Ala Leu Gly Lys Ser Thr Phe Val Leu Asp Asp Trp Lys Arg Lys Tyr
            260                 265                 270

Ser Asn Asp Asp Thr Arg Pro Val Ala Leu Pro Trp Phe Trp Glu His
        275                 280                 285

Tyr Asn Pro Glu Glu Tyr Ser Ile Trp Lys Val Gly Tyr Lys Tyr Asn
    290                 295                 300

Asp Glu Leu Thr Leu Thr Phe Met Ser Asn Asn Leu Val Gly Gly Phe
305                 310                 315                 320

Phe Asn Arg Leu Ser Ala Ser Thr Lys Tyr Met Phe Gly Cys Leu Val
                325                 330                 335

Val Tyr Gly Glu Asn Asn Asn Asn Gly Ile Val Gly Ala Val Met Val
            340                 345                 350

Arg Gly Gln Asp Phe Ala Pro Ala Phe Asp Val Ala Pro Asp Trp Glu
        355                 360                 365

Ser Tyr Glu Tyr Thr Lys Leu Asp Pro Thr Lys Glu Glu Asp Lys Glu
    370                 375                 380

Phe Val Asn Asn Met Trp Ala Trp Asp Lys Pro Val Val Val Asn Gly
385                 390                 395                 400

Glu Asp Lys Glu Ile Val Asp Gly Lys Val Leu Lys
                405                 410


<210> 237
<211> 92
<212> PRT
<213> Saccharomyces cerevisiae

<400> 237
Met Gln Phe Ser Thr Val Ala Ser Val Ala Phe Val Ala Leu Ala Asn
1               5                   10                  15

Phe Val Ala Ala Glu Ser Ala Ala Ala Ile Ser Gln Ile Thr Asp Gly
            20                  25                  30

Gln Ile Gln Ala Thr Thr Thr Ala Thr Thr Glu Ala Thr Thr Thr Ala
        35                  40                  45

Ala Pro Ser Ser Thr Val Glu Thr Val Ser Pro Ser Ser Thr Glu Thr
    50                  55                  60

Ile Ser Gln Gln Thr Glu Asn Gly Ala Ala Lys Ala Ala Val Gly Met
65                  70                  75                  80

Gly Ala Gly Ala Leu Ala Ala Ala Ala Met Leu Leu
                85                  90

<210> 238
<211> 131
<212> PRT
<213> Saccharomyces cerevisiae

<400> 238
Met Ser Gln Ser Arg Trp Ser Ile Val Leu Ile Phe Ala Leu Phe Ile
1               5                   10                  15

Phe Gly Ser Thr Gly Val Asn Ala Phe Phe Asn Phe Gly His His Gln
            20                  25                  30

Gln Gln Gln Gln Gln Gln Gln Gln Ser Tyr Glu Asp Gln Val Leu Asn
        35                  40                  45

Asn Pro Cys Asp Gly Tyr Leu Cys Pro Asp Thr Leu Thr Cys Val Ala
    50                  55                  60

Gln Gln Lys Asp Cys Pro Cys Pro Phe Pro Lys Ser Gln Leu Lys Cys
65                  70                  75                  80

Val Leu Pro Asp Asn Lys Phe Val Cys Ile Ser Lys Pro Ala Thr His
                85                  90                  95

Asn Glu Lys Phe Arg Ala Ile Tyr Asp Asp Pro Val Lys Gly Pro Lys
            100                 105                 110

Ala Lys Asn Lys Gly Phe Arg Asp Cys Gly Trp Val Ser Asp Ala Tyr
        115                 120                 125

Lys Asn His
    130


<210> 239
<211> 269
<212> PRT
<213> Saccharomyces cerevisiae

<400> 239
Met Ser Asn Asn Lys Ile Lys Arg Lys Asp Ala Ser Pro Glu Gln Glu
1               5                   10                  15

Ala Ile Glu Ser Phe Thr Ser Leu Thr Lys Cys Asp Pro Lys Val Ser
            20                  25                  30

Arg Lys Tyr Leu Gln Arg Asn His Trp Asn Ile Asn Tyr Ala Leu Asn
        35                  40                  45

Asp Tyr Tyr Asp Lys Glu Ile Gly Thr Phe Thr Asp Glu Val Ser Thr
    50                  55                  60

Val Ala His Pro Pro Val Tyr Pro Lys Glu Leu Thr Gln Val Phe Glu
65                  70                  75                  80

His Tyr Ile Asn Asn Asn Leu Phe Asp Ile Asp Ser Leu Val Lys Phe
                85                  90                  95

Ile Glu Glu Leu Gly Tyr Asn Leu Glu Asp Leu Ala Thr Leu Cys Leu
            100                 105                 110

Ala His Leu Leu Gly Tyr Lys Lys Leu Glu Glu Pro Leu Lys Arg Glu
        115                 120                 125

Asp Phe Leu Ser Thr Trp Phe Met Gln Gly Cys Ser Thr Ile Ser Asp
    130                 135                 140

Met Gln Glu Cys Ile Lys Thr Leu Asp Val Lys Leu His Glu Asp Leu
145                 150                 155                 160

Gln Tyr Phe Thr Gln Ile Tyr Asn Tyr Ala Phe Asn Leu Ile Leu Asp
                165                 170                 175

Pro Asn Arg Lys Asp Ile Asp Thr Asp Glu Gly Ile Gln Tyr Trp Lys
            180                 185                 190

Leu Phe Phe Gln Pro Glu Tyr Pro Val Arg Met Glu Pro Asp Leu Leu
        195                 200                 205

Glu Ala Trp Phe Arg Phe Leu Arg Asp Glu Gly Lys Thr Thr Ile Ser
    210                 215                 220

Lys Asp Thr Trp Arg Met Leu Leu Leu Phe Phe Lys Arg Tyr Pro Thr
225                 230                 235                 240

Ile Gln Lys Ile Ile Ser Asp Tyr Asp Glu Thr Ala Ala Trp Pro Phe
                245                 250                 255

Ile Ile Asp Glu Phe Tyr Glu Cys Leu Gln Asp Gln Gln
            260                 265


<210> 240
<211> 335
<212> PRT
<213> Saccharomyces cerevisiae

<400> 240
Met Ser Glu Pro Ala Gln Lys Lys Gln Lys Val Ala Asn Asn Ser Leu
1               5                   10                  15

Glu Gln Leu Lys Ala Ser Gly Thr Val Val Val Ala Asp Thr Gly Asp
            20                  25                  30

Phe Gly Ser Ile Ala Lys Phe Gln Pro Gln Asp Ser Thr Thr Asn Pro
        35                  40                  45

Ser Leu Ile Leu Ala Ala Ala Lys Gln Pro Thr Tyr Ala Lys Leu Ile
    50                  55                  60

Asp Val Ala Val Glu Tyr Gly Lys Lys His Gly Lys Thr Thr Glu Glu
65                  70                  75                  80

Gln Val Glu Asn Ala Val Asp Arg Leu Leu Val Glu Phe Gly Lys Glu
                85                  90                  95

Ile Leu Lys Ile Val Pro Gly Arg Val Ser Thr Glu Val Asp Ala Arg
            100                 105                 110

Leu Ser Phe Asp Thr Gln Ala Thr Ile Glu Lys Ala Arg His Ile Ile
        115                 120                 125

Lys Leu Phe Glu Gln Glu Gly Val Ser Lys Glu Arg Val Leu Ile Lys
    130                 135                 140

Ile Ala Ser Thr Trp Glu Gly Ile Gln Ala Ala Lys Glu Leu Glu Glu
145                 150                 155                 160

Lys Asp Gly Ile His Cys Asn Leu Thr Leu Leu Phe Ser Phe Val Gln
                165                 170                 175

Ala Val Ala Cys Ala Glu Ala Gln Val Thr Leu Ile Ser Pro Phe Val
            180                 185                 190

Gly Arg Ile Leu Asp Trp Tyr Lys Ser Ser Thr Gly Lys Asp Tyr Lys
        195                 200                 205

Gly Glu Ala Asp Pro Gly Val Ile Ser Val Lys Lys Ile Tyr Asn Tyr
    210                 215                 220

Tyr Lys Lys Tyr Gly Tyr Lys Thr Ile Val Met Gly Ala Ser Phe Arg
225                 230                 235                 240

Ser Thr Asp Glu Ile Lys Asn Leu Ala Gly Val Asp Tyr Leu Thr Ile
                245                 250                 255

Ser Pro Ala Leu Leu Asp Lys Leu Met Asn Ser Thr Glu Pro Phe Pro
            260                 265                 270

Arg Val Leu Asp Pro Val Ser Ala Lys Lys Glu Ala Gly Asp Lys Ile
        275                 280                 285

Ser Tyr Ile Ser Asp Glu Ser Lys Phe Arg Phe Asp Leu Asn Glu Asp
    290                 295                 300

Ala Met Ala Thr Glu Lys Leu Ser Glu Gly Ile Arg Lys Phe Ser Ala
305                 310                 315                 320

Asp Ile Val Thr Leu Phe Asp Leu Ile Glu Lys Lys Val Thr Ala
                325                 330                 335

<210> 241
<211> 113
<212> PRT
<213> Saccharomyces cerevisiae

<400> 241
Met Ala Gly Leu Lys Asp Val Val Thr Arg Glu Tyr Thr Ile Asn Leu
1               5                   10                  15

His Lys Arg Leu His Gly Val Ser Phe Lys Lys Arg Ala Pro Arg Ala
            20                  25                  30

Val Lys Glu Ile Lys Lys Phe Ala Lys Leu His Met Gly Thr Glu Asp
        35                  40                  45

Val Arg Leu Ala Pro Glu Leu Asn Gln Ala Ile Trp Lys Arg Gly Val
    50                  55                  60

Lys Gly Val Glu Tyr Arg Leu Arg Leu Arg Ile Ser Arg Lys Arg Asn
65                  70                  75                  80

Glu Glu Glu Asp Ala Lys Asn Pro Leu Phe Ser Tyr Val Glu Pro Val
                85                  90                  95

Leu Val Ala Ser Ala Lys Gly Leu Gln Thr Val Val Val Glu Glu Asp
            100                 105                 110

Ala

<210> 242
<211> 263
<212> PRT
<213> Saccharomyces cerevisiae

<400> 242
Met Arg Asn Phe Phe Thr Leu Phe Phe Ala Ala Ile Phe Ser Leu Gly
1               5                   10                  15

Ala Leu Ile Leu Ala Ile Val Ala Cys Ala Gly Ser Thr Lys Asn Tyr
            20                  25                  30

Ser Pro Ile Asn Lys Ile Tyr Cys Ala Glu Leu Asp Leu Ser Gln Met
        35                  40                  45

Lys Val Ser Thr Val Leu Pro Ser Leu Ser Ser Ala Thr Leu Ser Ser
    50                  55                  60

Leu Gly Leu Pro Ser Tyr Ile Asn Ile Gly Leu Trp Ser Tyr Cys Thr
65                  70                  75                  80

Val Asp Ser Ser His Asn Ile Gln Ser Cys Ser Ser Pro His Gly Ile
                85                  90                  95

Gln Asn Phe Asn Leu Ser Ser Leu Val Tyr Asp Asn Ile Asn Asn Asn
            100                 105                 110

Glu Ala Leu Glu Leu Met Asp Ser Val Ala Ser Val Val Leu Pro Glu
        115                 120                 125

Lys Leu Lys Ser Lys Met Thr Tyr Tyr Asn Asn Leu Val Lys Cys Met
    130                 135                 140

Phe Ile Thr Ile Leu Ile Gly Ile Val Leu Thr Phe Val Asn Leu Val
145                 150                 155                 160

Phe Asn Val Leu Arg Trp Ile Ile His Ile Arg Pro Leu Thr Trp Phe
                165                 170                 175

Gly Ala Phe Phe Ser Phe Phe Ala Phe Ala Ala Leu Leu Val Ser Ile
            180                 185                 190

Gly Ser Cys Leu Gly Thr Tyr Ser Tyr Ile Lys Tyr Ile Leu Lys His
        195                 200                 205

Asn Tyr Ser Asp Tyr Gly Ile Ser Met Ser Ile Gly Arg Asn Tyr Gln
    210                 215                 220

Gly Leu Met Trp Gly Ala Val Val Gly Ala Leu Leu Asn Phe Ile Leu
225                 230                 235                 240

Trp Cys Ser Val Arg Ser Arg Pro Thr Val Ile Tyr Ala Asn Ala Pro
                245                 250                 255

Ile Glu Glu Lys Pro Leu Ile
            260

<210> 243
<211> 128
<212> PRT
<213> Saccharomyces cerevisiae

<400> 243
Met Leu Val Phe Ser Phe Leu Phe Val Val Val Ser Ile Asn Leu Asn
  1               5                  10                  15

Ala Leu Ile Phe Leu Cys Lys Lys Ser Trp Ala Ser Tyr Leu Phe Leu
             20                  25                  30

Tyr Leu Tyr Asn Phe Phe Phe Cys Glu Asp Glu Tyr Lys Leu Thr Lys
         35                  40                  45

Asn Cys Val Arg Val Glu Ala Ile Ala Pro Phe Met Met Cys Leu Gly
     50                  55                  60

Ser Leu Gly Ala Ile Leu Gly Lys Gln Arg Thr Ala Asn Phe Leu Leu
 65                  70                  75                  80

Leu Ser Tyr Asn Val Ile Asn Asn Pro Val Val Leu Val Tyr Tyr Val
                 85                  90                  95

Glu Asn Phe Ser Arg Ile Asn Phe Ile Lys His Thr Thr Lys Glu Lys
            100                 105                 110

Ser Val Ile Trp Thr Asn Glu Arg Gln Leu Asn Pro Trp Ile Cys Asn
        115                 120                 125

<210> 244
<211> 525
<212> PRT
<213> Saccharomyces cerevisiae

<400> 244
Met Ala Ala Gly Glu Gln Val Ser Asn Met Phe Asp Thr Ile Leu Val
1               5                   10                  15

Leu Asp Phe Gly Ser Gln Tyr Ser His Leu Ile Thr Arg Arg Leu Arg
            20                  25                  30

Glu Phe Asn Ile Tyr Ala Glu Met Leu Pro Cys Thr Gln Lys Ile Ser
        35                  40                  45

Glu Leu Gly Trp Thr Pro Lys Gly Val Ile Leu Ser Gly Gly Pro Tyr
    50                  55                  60

Ser Val Tyr Ala Glu Asp Ala Pro His Val Asp His Ala Ile Phe Asp
65                  70                  75                  80

Leu Asn Val Pro Ile Leu Gly Ile Cys Tyr Gly Met Gln Glu Leu Ala
                85                  90                  95

Trp Ile Asn Gly Lys Gln Val Gly Arg Gly Asp Lys Arg Glu Tyr Gly
            100                 105                 110

Pro Ala Thr Leu Lys Val Ile Asp Asp Ser Asn Ser Leu Phe Lys Gly
        115                 120                 125

Met Asn Asp Ser Thr Val Trp Met Ser His Gly Asp Lys Leu His Gly
    130                 135                 140

Leu Pro Thr Gly Tyr Lys Thr Ile Ala Thr Ser Asp Asn Ser Pro Tyr
145                 150                 155                 160

Cys Gly Ile Val His Glu Thr Lys Pro Ile Tyr Gly Ile Gln Phe His
                165                 170                 175

Pro Glu Val Thr His Ser Thr Gln Gly Lys Thr Leu Leu Lys Asn Phe
            180                 185                 190

Ala Val Asp Leu Cys His Ala Lys Gln Asn Trp Thr Met Glu Asn Phe
        195                 200                 205

Ile Asp Thr Glu Ile Asn Arg Ile Arg Lys Leu Val Gly Pro Thr Ala
    210                 215                 220

Glu Val Ile Gly Ala Val Ser Gly Gly Val Asp Ser Thr Val Ala Ser

225                 230                 235                 240

Lys Leu Met Thr Glu Ala Ile Gly Asp Arg Phe His Ala Ile Leu Val
                245                 250                 255

Asp Asn Gly Val Leu Arg Leu Asn Glu Ala Ala Asn Val Lys Lys Thr
            260                 265                 270

Leu Val Glu Gly Leu Gly Ile Asn Leu Met Val Val Asp Ala Ser Glu
        275                 280                 285

Glu Phe Leu Ser Lys Leu Lys Gly Val Thr Asp Pro Glu Lys Lys Arg
    290                 295                 300

Lys Ile Ile Gly Asn Thr Phe Ile His Val Phe Glu Arg Glu Ala Glu
305                 310                 315                 320

Lys Ile Lys Pro Lys Asp Gly Lys Glu Ile Gln Phe Leu Leu Gln Gly
                325                 330                 335

Thr Leu Tyr Pro Asp Val Ile Glu Ser Ile Ser Phe Lys Gly Pro Ser
            340                 345                 350

Gln Thr Ile Lys Thr His His Asn Val Gly Gly Leu Leu Glu Asn Met
        355                 360                 365

Lys Leu Lys Leu Ile Glu Pro Leu Arg Glu Leu Phe Lys Asp Glu Val
    370                 375                 380

Arg His Leu Gly Glu Leu Leu Gly Ile Pro His Asp Leu Val Trp Arg
385                 390                 395                 400

His Pro Phe Pro Gly Pro Gly Ile Ala Ile Arg Val Leu Gly Glu Val
                405                 410                 415

Thr Lys Glu Gln Val Glu Ile Ala Arg Lys Ala Asp Asn Ile Tyr Ile
            420                 425                 430

Glu Glu Ile Lys Lys Ala Gly Leu Tyr Asn Gln Ile Ser Gln Ala Phe
        435                 440                 445

Ala Cys Leu Leu Pro Val Lys Ser Val Gly Val Met Gly Asp Gln Arg
    450                 455                 460

Thr Tyr Asp Gln Val Ile Ala Leu Arg Ala Ile Glu Thr Thr Asp Phe
465                 470                 475                 480

Met Thr Ala Asp Trp Phe Pro Phe Glu His Ser Phe Leu Lys Lys Val
                485                 490                 495

Ala Ser Arg Ile Val Asn Glu Val Asp Gly Val Ala Arg Val Thr Tyr
            500                 505                 510

Asp Ile Thr Ser Lys Pro Pro Ala Thr Val Glu Trp Glu
        515                 520                 525

<210> 245
<211> 241
<212> PRT
<213> Saccharomyces cerevisiae

<400> 245
Met Val Lys Ala Val Ile Phe Thr Asp Phe Asp Gly Thr Val Thr Leu
1               5                   10                  15

Glu Asp Ser Asn Asp Tyr Leu Thr Asp Thr Leu Gly Phe Gly Lys Glu
            20                  25                  30

Lys Arg Leu Lys Val Phe Glu Gly Val Leu Asp Asp Thr Lys Ser Phe
        35                  40                  45

Arg Gln Gly Phe Met Glu Met Leu Glu Ser Ile His Thr Pro Phe Pro
    50                  55                  60

Glu Cys Ile Lys Ile Leu Glu Lys Lys Ile Arg Leu Asp Pro Gly Phe
65                  70                  75                  80

Lys Asp Thr Phe Glu Trp Ala Gln Glu Asn Asp Val Pro Val Ile Val
                85                  90                  95

Val Ser Ser Gly Met Lys Pro Ile Ile Lys Val Leu Leu Thr Arg Leu
            100                 105                 110

Val Gly Gln Glu Ser Ile His Lys Ile Asp Ile Val Ser Asn Glu Val
        115                 120                 125

Glu Ile Asp Ala His Asp Gln Trp Lys Ile Ile Tyr Lys Asp Glu Ser
    130                 135                 140

Pro Phe Gly His Asp Lys Ser Arg Ser Ile Asp Ala Tyr Lys Lys Lys
145                 150                 155                 160

Phe Glu Ser Thr Leu Lys Ala Gly Glu Gln Arg Pro Val Tyr Phe Tyr
                165                 170                 175

Cys Gly Asp Gly Val Ser Asp Leu Ser Ala Ala Lys Glu Cys Asp Leu
            180                 185                 190

Leu Phe Ala Lys Arg Gly Lys Asp Leu Val Thr Tyr Cys Lys Lys Gln
        195                 200                 205

Asn Val Pro Phe His Glu Phe Asp Thr Phe Lys Asp Ile Leu Ala Ser
    210                 215                 220

Met Lys Gln Val Leu Ala Gly Glu Lys Thr Val Ala Glu Leu Met Glu
225                 230                 235                 240

Asn

<210> 246
<211> 586
<212> PRT
<213> Saccharomyces cerevisiae

<400> 246
Met Ser Arg Ser Asn Ser Ile Tyr Thr Glu Asp Ile Glu Met Tyr Pro
1               5                   10                  15

Thr His Asn Glu Gln His Leu Thr Arg Glu Tyr Thr Lys Pro Asp Gly
            20                  25                  30

Gln Thr Lys Ser Glu Lys Leu Asn Phe Glu Gly Ala Tyr Ile Asn Ser
        35                  40                  45

His Gly Thr Leu Ser Lys Thr Thr Thr Arg Glu Ile Glu Gly Asp Leu
    50                  55                  60

Asp Ser Glu Thr Ser Ser His Ser Ser Asp Asp Lys Val Asp Pro Thr
65                  70                  75                  80

Gln Gln Ile Thr Ala Glu Thr Lys Ala Pro Tyr Thr Leu Leu Ser Tyr
                85                  90                  95

Gly Gln Lys Trp Gly Met Val Ala Ile Leu Thr Met Cys Gly Phe Trp
            100                 105                 110

Ser Ser Leu Gly Ser Pro Ile Tyr Tyr Pro Ala Leu Arg Gln Leu Glu
        115                 120                 125

Lys Gln Phe Asn Val Asp Glu Asn Met Val Asn Val Thr Val Val Val
    130                 135                 140

Tyr Leu Leu Phe Gln Gly Ile Ser Pro Thr Val Ser Gly Gly Leu Ala
145                 150                 155                 160

Asp Cys Phe Gly Arg Arg Pro Ile Ile Leu Ala Gly Met Leu Ile Tyr
                165                 170                 175

Val Ile Ala Ser Ile Gly Leu Ala Cys Ala Pro Ser Tyr Gly Val Ile
            180                 185                 190

Ile Phe Leu Arg Cys Ile Gln Ser Ile Gly Ile Ser Pro Thr Ile Ala
        195                 200                 205

Ile Ser Ser Gly Val Val Gly Asp Phe Thr Leu Lys His Glu Arg Gly
    210                 215                 220

Thr Phe Val Gly Ala Thr Ser Gly Phe Val Leu Leu Gly Gln Cys Phe
225                 230                 235                 240

Gly Ser Leu Ile Gly Ala Val Leu Thr Ala Arg Trp Asp Trp Arg Ala
                245                 250                 255

Ile Phe Trp Phe Leu Thr Ile Gly Cys Gly Ser Cys Phe Leu Ile Ala
            260                 265                 270

Phe Leu Ile Leu Pro Glu Thr Lys Arg Thr Ile Ala Gly Asn Leu Ser
        275                 280                 285

Ile Lys Pro Lys Arg Phe Ile Asn Arg Ala Pro Ile Phe Leu Leu Gly
    290                 295                 300

Pro Val Arg Arg Arg Phe Lys Tyr Asp Asn Pro Asp Tyr Glu Thr Leu
305                 310                 315                 320

Asp Pro Thr Ile Pro Lys Leu Asp Leu Ser Ser Ala Gly Lys Ile Leu
                325                 330                 335

Val Leu Pro Glu Ile Ile Leu Ser Leu Phe Pro Ser Gly Leu Leu Phe
            340                 345                 350

Ala Met Trp Thr Leu Met Leu Ser Ser Ile Ser Ser Gly Leu Ser Val
        355                 360                 365

Ala Pro Tyr Asn Tyr His Leu Val Ile Ile Gly Val Cys Tyr Leu Pro
    370                 375                 380

Gly Gly Ile Gly Gly Leu Met Gly Ser Phe Phe Thr Gly Arg Ile Ile
385                 390                 395                 400

Asp Met Tyr Phe Lys Arg Lys Ile Lys Lys Phe Glu Gln Asp Lys Ala
                405                 410                 415

Asn Gly Leu Ile Pro Gln Asp Ala Glu Ile Asn Met Phe Lys Val Arg
            420                 425                 430

Leu Val Cys Leu Leu Pro Gln Asn Phe Leu Ala Val Val Ala Tyr Leu
        435                 440                 445

Leu Phe Gly Trp Ser Ile Asp Lys Gly Trp Arg Ile Glu Ser Ile Leu
    450                 455                 460

Ile Thr Ser Phe Val Cys Ser Tyr Cys Ala Met Ser Thr Leu Ser Thr
465                 470                 475                 480

Ser Thr Thr Leu Leu Val Asp Leu Tyr Pro Thr Lys Ser Ser Thr Ala
                485                 490                 495

Ser Ser Cys Phe Asn Phe Val Arg Cys Ser Leu Ser Thr Ile Phe Met
            500                 505                 510

Gly Cys Phe Ala Lys Met Lys Ala Ala Met Thr Val Gly Gly Thr Phe
        515                 520                 525

Thr Phe Leu Cys Ala Leu Val Phe Phe Phe Asn Phe Leu Met Phe Ile
    530                 535                 540

Pro Met Lys Tyr Gly Met Lys Trp Arg Glu Asp Arg Leu Leu Lys Gln
545                 550                 555                 560

Gln Arg Gln Ser Trp Leu Asn Thr Leu Ala Val Lys Ala Lys Lys Gly

565                 570                 575

Thr Lys Arg Asp Gln Asn Asp Asn His Asn
            580                 585

<210> 247
<211> 455
<212> PRT
<213> Saccharomyces cerevisiae

<400> 247
Met Ser Lys Leu Phe Leu Asp Glu Leu Pro Glu Ser Leu Ser Arg Lys
1               5                   10                  15

Ile Gly Thr Val Val Arg Val Leu Pro Ser Ser Leu Glu Ile Phe Glu
            20                  25                  30

Glu Leu Tyr Lys Tyr Ala Leu Asn Glu Asn Ser Asn Asp Arg Ser Gly
        35                  40                  45

Arg His Lys Lys Pro Arg Ile Asp Val Ser Ser Asp Leu Leu Lys Thr
    50                  55                  60

Asp Glu Ile Ser Glu Thr Asn Thr Ile Phe Lys Leu Glu Gly Val Ser
65                  70                  75                  80

Val Leu Ser Pro Leu Arg Lys Lys Leu Asp Leu Val Phe Tyr Leu Ser
                85                  90                  95

Asn Val Asp Gly Ser Pro Val Ile Thr Leu Leu Lys Gly Asn Asp Arg
            100                 105                 110

Glu Leu Ser Ile Tyr Gln Leu Asn Lys Asn Ile Lys Met Ala Ser Phe
        115                 120                 125

Leu Pro Val Pro Glu Lys Pro Asn Leu Ile Tyr Leu Phe Met Thr Tyr
    130                 135                 140

Thr Ser Cys Glu Asp Asn Lys Phe Ser Glu Pro Val Val Met Thr Leu
145                 150                 155                 160

Asn Lys Glu Asn Thr Leu Asn Gln Phe Lys Asn Leu Gly Leu Leu Asp
                165                 170                 175

Ser Asn Val Thr Asp Phe Glu Lys Cys Val Glu Tyr Ile Arg Lys Gln
            180                 185                 190

Ala Ile Leu Thr Gly Phe Lys Ile Ser Asn Pro Phe Val Asn Ser Thr
        195                 200                 205

Leu Val Asp Thr Asp Ala Glu Lys Ile Asn Ser Phe His Leu Gln Cys
    210                 215                 220

His Arg Gly Thr Lys Glu Gly Thr Leu Tyr Phe Leu Pro Asp His Ile
225                 230                 235                 240

Ile Phe Gly Phe Lys Lys Pro Ile Leu Leu Phe Asp Ala Ser Asp Ile
                245                 250                 255

Glu Ser Ile Thr Tyr Ser Ser Ile Thr Arg Leu Thr Phe Asn Ala Ser
            260                 265                 270

Leu Val Thr Lys Asp Gly Glu Lys Tyr Glu Phe Ser Met Ile Asp Gln
        275                 280                 285

Thr Glu Tyr Ala Lys Ile Asp Asp Tyr Val Lys Arg Lys Gln Met Lys
    290                 295                 300

Asp Lys Ser Met Ser Glu Glu Leu Lys Ala Lys Ser Lys Ser Lys Gly
305                 310                 315                 320

Gln Ala Thr Asp Gly Thr Ala Asp Gln Pro Ser Ile Leu Gln Glu Ala
                325                 330                 335

Thr Arg Gln Met Gln Asp Glu Lys Lys Ala Gly Val Phe Ser Asp Asp
            340                 345                 350

Asp Glu Glu Asn Asp Gln Asn Phe Glu Ala Glu Ser Asp Leu Ser Asp
        355                 360                 365

Gly Ser Gly Gln Glu Ser Ser Asp Gly Ala Glu Asp Gly Glu Glu Ala
    370                 375                 380

Glu Glu Asp Asp Glu Glu Asp Asp Glu Glu Glu Asp Lys Lys Gly Gln
385                 390                 395                 400

Ser Ala Leu Asn Arg Asp Asn Ser Phe Ala Ser Ile Asn Gly Gln Pro
                405                 410                 415

Glu Gln Glu Leu Gln Tyr Lys Glu Phe Lys Glu Pro Leu Glu Leu Glu
            420                 425                 430

Asp Ile Pro Ile Glu Ile Asp Asn Asp Asp Asp Glu Asp Asp Glu Asp
        435                 440                 445

Gly Ser Gly Val Glu Tyr Asp
    450                 455

<210> 248
<211> 131
<212> PRT
<213> Saccharomyces cerevisiae

<400> 248
Met Cys Gly Ile Tyr Pro Tyr Gln Leu Val Thr Ser Ser Ala Ser Pro
  1               5                  10                  15

Lys Thr Asp Val Phe Val Ser Ser Leu Pro Thr Ser Pro Pro Gln Ala
             20                  25                  30

Leu Gly Ala Ala Ser Val Met Ala Leu Gln Leu Val Phe Lys Asn Cys
        35                  40                  45

Thr Ile Ser Leu Phe Ile Ser Leu Asn Ser Asn Arg Lys Ala Ser Trp
    50                  55                  60

Pro Ser Gly Asp Ser Ile Phe Ser Asn Cys Asp Phe Gly Met Cys Ser
65                  70                  75                  80

Ala Lys Asn Ser Cys Ser Glu Tyr Val Asn Ser Pro Ser Asp Trp Ile
                85                  90                  95

Pro Met Ile Arg Asp Gly Cys Trp Met Val Glu Arg Thr Ser Val Met
            100                 105                 110

Ser Ser Thr Leu Ser Leu Ala Lys Ser Cys Val Ser Ser Leu Arg Val
        115                 120                 125

Met Ala Met
    130

<210> 249
<211> 661
<212> PRT
<213> Saccharomyces cerevisiae

<400> 249
   Met Ser Gly Phe Lys Cys Tyr Leu Gln Leu Gly Asp Arg Leu Ser Gln
   1               5                   10                  15
   Ile Trp Leu Asn Lys Tyr Thr Leu Val Leu Leu Leu Ala Met Leu Lys
               20                  25                  30
   Leu Leu Phe Phe Ser Lys Ser Ile Gln His Ala Ile Glu Val Ser Glu
           35                  40                  45
   Thr Tyr Ile Leu Ser Asn Cys Tyr Ser Ile Asp Ser Leu Tyr Ser Lys
       50                  55                  60
   Met Thr Asp Asn Thr Pro His Tyr Leu Gly Ile Met Gly Asn Tyr Leu
   65                  70                  75                  80
   Ile Glu Lys Gly Met Glu Glu Thr Val Lys Ala Thr Leu Glu Thr Leu
                   85                  90                  95
   Ser Leu Ile Val Tyr Ala Ser Glu Gly Leu Val Asn Phe Ala Ile Asp
               100                 105                 110
   Leu Tyr Leu Gly Thr Tyr Ala Cys Leu Ile Val Ser Ala Val Asp Gly
           115                 120                 125
   Thr Val Asp Val Ala Thr Asn Ile Thr Glu Lys Leu Ile Ser Leu Val
       130                 135                 140
   Asn Asp Thr Val Ser Ser Val Ala Asn Glu Leu Asp Thr Gly Leu Asn
   145                 150                 155                 160
   Asp Ile Ser Lys Ile Ile Asn Lys Val Ile Lys Ala Ala Ser Lys Val
                   165                 170                 175
   Glu Asn Phe Phe Thr Gly Asp Asp Asp Asp Ser Asn Met Thr Ser Ser
               180                 185                 190
   Ile Lys Ser Val Asn Leu Thr Ile Ser Ala Leu His Asn Leu Tyr Ile
           195                 200                 205
   Pro Ser Ser Ile Asn Asp Lys Leu Glu Glu Leu Ser Ala Lys Thr Pro
       210                 215                 220
   Asp Phe Ala Gln Val Lys Asn Thr Thr Lys Asn Leu Ile Ser Val Pro
   225                 230                 235                 240
   Phe Asn Glu Val Arg Lys Asn Ile Lys Ala Val Asn Ala Ser Asn Ile
                   245                 250                 255
   Ile Gly Asp Thr Ser Val Leu Tyr Val Pro Pro Val Ser Leu Asp Asn
               260                 265                 270
   Ser Thr Gly Ile Cys Ser Ser Asn Gln Ser Glu Ile Leu Ala Phe Tyr
           275                 280                 285
   Ser Ile Leu Gly His Val Leu Lys Ile Ala Thr Val Val Cys Ile Thr
       290                 295                 300
   Val Leu Ile Cys Phe Ala Val Gly Ala Met Ala Pro Val Ala Trp Asn
   305                 310                 315                 320
   Glu Ile Lys Leu Trp Arg Arg Leu Cys Gly Met Arg Asp His Tyr Met
                   325                 330                 335
   Leu Ser Arg Gln Asp Ser Tyr Thr Ser Phe Ser Ser Glu Asn Thr His
               340                 345                 350
   Glu Leu Lys Asp Pro Phe Arg Asp Pro Pro Ile Gln Asn Gly Gln Tyr
           355                 360                 365
   Asp Val Ile Ala Ser Tyr Gln Gln Cys Phe Gln Thr Trp Asn Thr Arg
       370                 375                 380
   Ile Ala Gly Trp Met Thr Asn Leu Val Thr Phe Gly Lys Ser Pro Glu
   385                 390                 395                 400
   Asn Ile Asp Pro Lys Thr Lys Gln Lys Ile Glu Trp Val Val Ala Tyr
                   405                 410                 415
   Met Thr Ser Glu Arg Ala Leu Cys Val Leu Gly Ile Gly Leu Leu Gly
               420                 425                 430
   Ile Leu Val Cys Ile Cys Gln Phe Val Met Ile Ala Leu Leu Lys His
           435                 440                 445
   Lys Ile Ser His Ser Leu Thr Ser Asn Asp Gly Asp Gly Val Gln Asn
       450                 455                 460
   Leu Leu Lys Ser Ser Thr Ala Val Asp Ile Glu Asn Gln Met Ser Leu
   465                 470                 475                 480
   Trp Ser Val Gln Thr Asn Lys Tyr Ile Asn Thr Thr Glu Thr Asn Ile
                   485                 490                 495
   Asn Gln Glu Val Phe Gly Trp Ile Asn Thr Thr Thr Leu Ser Val Asn
               500                 505                 510
   Asn Thr Val Ala Thr Met Ile Ser Asp Ile Asp Thr Thr Leu Ala Asp
           515                 520                 525
   Val Phe Asn Gly Thr Leu Leu Tyr Asn Pro Met Lys Thr Val Val Gly
       530                 535                 540
   Cys Ala Ile Glu Asn Lys Leu Tyr Thr Ile Glu Lys Ala Met Thr Trp
   545                 550                 555                 560
   Ile His Asp Lys Ala Gln Leu His Ile Pro Arg Ile Asn Gly Thr Gln
                   565                 570                 575
   Ile Lys Gln Ala Leu Ala Lys Gln Thr Asp Asn Ser Thr Ile Pro Thr
               580                 585                 590
   Ala Ser Ser Thr Ser Ala Ala Thr Glu Asn Leu Leu Glu Asn Leu Val
           595                 600                 605
   Asn Asp Met Arg Glu Gly Leu Leu Lys Ile Leu Arg Ala Tyr His Arg
       610                 615                 620
   Ile Thr Leu Gly Glu Leu Thr Val Ala Leu Val Ile Leu Ala Val Trp
   625                 630                 635                 640
   Leu Val Gln Leu Pro Ile Ala Leu Val Ile Leu Arg Leu Arg Leu Arg
                   645                 650                 655
   Lys Ala Thr Phe Asp
               660


<210> 250
<211> 384
<212> PRT
<213> Saccharomyces cerevisiae

<400> 250
Met Val Thr Tyr Ala Gly Lys Leu Val Leu Ala Pro Met Val Arg Ala
  1               5                  10                  15

Gly Glu Leu Pro Thr Arg Leu Met Ala Leu Ala His Gly Ala Asp Leu
             20                  25                  30

Val Trp Ser Pro Glu Ile Ile Asp Lys Lys Leu Ile Gln Cys Val Arg

35                  40                  45

Lys Glu Asn Thr Ala Leu Gln Thr Val Asp Tyr Val Val Pro Ser Lys
    50                  55                  60

Val Gln Thr Arg Pro Glu Thr Leu Val Phe Arg Thr Tyr Pro Lys Leu
65                  70                  75                  80

Glu Ser Ser Lys Leu Ile Phe Gln Ile Gly Ser Ala Ser Pro Ala Leu
                85                  90                  95

Ala Thr Gln Ala Ala Leu Lys Val Ile Asn Asp Val Ser Gly Ile Asp
            100                 105                 110

Ile Asn Ala Gly Cys Pro Lys His Phe Ser Ile His Ser Gly Met Gly
        115                 120                 125

Ser Ala Leu Leu Arg Thr Pro Asp Thr Leu Cys Leu Ile Leu Lys Glu
    130                 135                 140

Leu Val Lys Asn Val Gly Asn Pro His Ser Lys Pro Ile Ser Val Lys
145                 150                 155                 160

Ile Arg Leu Leu Asp Thr Lys Gln Asp Thr Leu Gln Leu Val Lys Arg
                165                 170                 175

Leu Cys Ala Thr Gly Ile Thr Asn Leu Thr Val His Cys Arg Lys Thr
            180                 185                 190

Glu Met Arg Asn Arg Glu Gln Pro Ile Thr Asp Tyr Ile Ala Glu Ile
        195                 200                 205

Tyr Glu Ile Cys Gln Ala Asn Asn Val Ser Leu Ile Val Asn Gly Ala
    210                 215                 220

Ile Arg Asp Arg Ser His Phe His Asp Leu Gln Ala Asn His Trp Lys
225                 230                 235                 240

Asn Thr Asn Ile Gly Gly Met Ile Ala Glu Cys Ala Glu Arg Asp Pro
                245                 250                 255

Thr Val Phe Asp His Thr Ser Lys Pro Ser Glu Asp Gly Pro Ser Trp
            260                 265                 270

Val Val Ala Cys Arg Glu Phe Ile Gln Trp Ala Thr Lys Phe Asp Asn
        275                 280                 285

His Ile Gly Asn Thr Lys Tyr Met Leu Ser Arg Ile Val Pro Gly Lys
    290                 295                 300

Ser Val Phe Phe Gln Tyr Phe Ala Arg Cys Lys Ser Pro Glu Glu Val
305                 310                 315                 320

Ser Phe Val Leu Lys Gln Leu Asn Asp Asp Gly Ser Ala Gln Thr Asp
                325                 330                 335

Pro Ser Glu Tyr Leu Glu Asn Cys Arg Ala Gln Glu Lys Ala Leu Lys
            340                 345                 350

Asn Ala Asn Ala Ile Ala Lys Gln Lys Arg Lys Gln Thr Asp His Ile
        355                 360                 365

Gly Ser Asp Thr Lys Lys Gln Lys Val Val Pro Leu Pro Thr Asp Ile
    370                 375                 380

<210> 251
<211> 551
<212> PRT
<213> Saccharomyces cerevisiae

<400> 251
Met Thr Tyr Ser Thr Val Ser Ile Asn Thr Pro Pro Pro Tyr Leu Thr
  1               5                  10                  15

Leu Ala Cys Asn Glu Lys Leu Pro Thr Val Leu Ser Ile Ala Gly Thr
             20                  25                  30

Asp Pro Ser Gly Gly Ala Gly Ile Glu Ala Asp Val Lys Thr Ile Thr
         35                  40                  45

Ala His Arg Cys Tyr Ala Met Thr Cys Ile Thr Ala Leu Asn Ala Gln
     50                  55                  60

Thr Pro Val Lys Val Tyr Ser Ile Asn Asn Thr Pro Lys Glu Val Val
 65                  70                  75                  80

Phe Gln Thr Leu Glu Ser Asn Leu Lys Asp Met Lys Cys Asn Val Ile
                 85                  90                  95

Lys Thr Gly Met Leu Thr Ala Ala Ala Ile Glu Val Leu His Glu Lys
            100                 105                 110

Leu Leu Gln Leu Gly Glu Asn Arg Pro Lys Leu Val Val Asp Pro Val
        115                 120                 125

Leu Val Ala Thr Ser Gly Ser Ser Leu Ala Gly Lys Asp Ile Val Ser
    130                 135                 140

Leu Ile Thr Glu Lys Val Ala Pro Phe Ala Asp Ile Leu Thr Pro Asn
145                 150                 155                 160

Ile Pro Glu Cys Tyr Lys Leu Leu Gly Glu Glu Arg Lys Val Asn Gly
                165                 170                 175

Leu Gln Asp Ile Phe Gln Ile Ala Lys Asp Leu Ala Lys Ile Thr Lys
            180                 185                 190

Cys Ser Asn Ile Leu Val Lys Gly Gly His Ile Pro Trp Asn Asp Glu

195                 200                 205

Lys Glu Lys Tyr Ile Thr Asp Val Leu Phe Leu Gly Ala Glu Gln Lys
    210                 215                 220

Phe Ile Ile Phe Lys Gly Asn Phe Val Asn Thr Thr His Thr His Gly
225                 230                 235                 240

Thr Gly Cys Thr Leu Ala Ser Ala Ile Ala Ser Asn Leu Ala Arg Gly
                245                 250                 255

Tyr Ser Leu Pro Gln Ser Val Tyr Gly Gly Ile Glu Tyr Val Gln Asn
            260                 265                 270

Ala Val Ala Ile Gly Cys Asp Val Thr Lys Glu Thr Val Lys Asp Asn
        275                 280                 285

Gly Pro Ile Asn His Val Tyr Ala Val Glu Ile Pro Leu Glu Lys Met
    290                 295                 300

Leu Ser Asp Glu Cys Phe Thr Ala Ser Asp Val Ile Pro Lys Lys Pro
305                 310                 315                 320

Leu Lys Ser Ala Ala Asp Lys Ile Pro Gly Gly Asn Phe Tyr Glu Tyr
                325                 330                 335

Leu Ile Asn His Pro Lys Val Lys Pro His Trp Asp Ser Tyr Ile Asn
            340                 345                 350

His Glu Phe Val Lys Lys Val Ala Asp Gly Thr Leu Glu Arg Lys Lys
        355                 360                 365

Phe Gln Phe Phe Ile Glu Gln Asp Tyr Ala Tyr Leu Val Asp Tyr Ala
    370                 375                 380

Arg Val His Cys Ile Ala Gly Ser Lys Ala Pro Cys Leu Glu Asp Met
385                 390                 395                 400

Glu Lys Glu Leu Val Ile Val Gly Gly Val Arg Thr Glu Met Gly Gln
                405                 410                 415

His Glu Lys Arg Leu Lys Glu Val Phe Gly Val Lys Asp Pro Asp Tyr
            420                 425                 430

Phe Gln Lys Ile Lys Arg Gly Pro Ala Leu Arg Ala Tyr Ser Arg Tyr
        435                 440                 445

Phe Asn Asp Val Ser Arg Arg Gly Asn Trp Gln Glu Leu Val Ala Ser
    450                 455                 460

Leu Thr Pro Cys Leu Met Gly Tyr Gly Glu Ala Leu Thr Lys Met Lys
465                 470                 475                 480

Gly Lys Val Thr Ala Pro Glu Gly Ser Val Tyr His Glu Trp Cys Glu
                485                 490                 495

Thr Tyr Ala Ser Ser Trp Tyr Arg Glu Ala Met Asp Glu Gly Glu Lys
            500                 505                 510

Leu Leu Asn His Ile Leu Glu Thr Tyr Pro Pro Glu Gln Leu Asp Thr
        515                 520                 525

Leu Val Thr Ile Tyr Ala Glu Val Cys Glu Leu Glu Thr Asn Phe Trp
    530                 535                 540

Thr Ala Ala Leu Glu Tyr Glu
545                 550


<210> 252
<211> 483
<212> PRT
<213> Saccharomyces cerevisiae

<400> 252
Met Leu Ser Ala Thr Lys Gln Thr Phe Arg Ser Leu Gln Ile Arg Thr
  1               5                  10                  15

Met Ser Thr Asn Thr Lys His Tyr Asp Tyr Leu Val Ile Gly Gly Gly
             20                  25                  30

Ser Gly Gly Val Ala Ser Ala Arg Arg Ala Ala Ser Tyr Gly Ala Lys
         35                  40                  45

Thr Leu Leu Val Glu Ala Lys Ala Leu Gly Gly Thr Cys Val Asn Val
     50                  55                  60

Gly Cys Val Pro Lys Lys Val Met Trp Tyr Ala Ser Asp Leu Ala Thr
 65                  70                  75                  80

Arg Val Ser His Ala Asn Glu Tyr Gly Leu Tyr Gln Asn Leu Pro Leu
                 85                  90                  95

Asp Lys Glu His Leu Thr Phe Asn Trp Pro Glu Phe Lys Gln Lys Arg
            100                 105                 110

Asp Ala Tyr Val His Arg Leu Asn Gly Ile Tyr Gln Lys Asn Leu Glu
        115                 120                 125

Lys Glu Lys Val Asp Val Val Phe Gly Trp Ala Arg Phe Asn Lys Asp
    130                 135                 140

Gly Asn Val Glu Val Gln Lys Arg Asp Asn Thr Thr Glu Val Tyr Ser
145                 150                 155                 160

Ala Asn His Ile Leu Val Ala Thr Gly Gly Lys Ala Ile Phe Pro Glu
                165                 170                 175

Asn Ile Pro Gly Phe Glu Leu Gly Thr Asp Ser Asp Gly Phe Phe Arg
            180                 185                 190

Leu Glu Glu Gln Pro Lys Lys Val Val Val Val Gly Ala Gly Tyr Ile

195                 200                 205

Gly Ile Glu Leu Ala Gly Val Phe His Gly Leu Gly Ser Glu Thr His
    210                 215                 220

Leu Val Ile Arg Gly Glu Thr Val Leu Arg Lys Phe Asp Glu Cys Ile
225                 230                 235                 240

Gln Asn Thr Ile Thr Asp His Tyr Val Lys Glu Gly Ile Asn Val His
                245                 250                 255

Lys Leu Ser Lys Ile Val Lys Val Glu Lys Asn Val Glu Thr Asp Lys
            260                 265                 270

Leu Lys Ile His Met Asn Asp Ser Lys Ser Ile Asp Asp Val Asp Glu
        275                 280                 285

Leu Ile Trp Thr Ile Gly Arg Lys Ser His Leu Gly Met Gly Ser Glu
    290                 295                 300

Asn Val Gly Ile Lys Leu Asn Ser His Asp Gln Ile Ile Ala Asp Glu
305                 310                 315                 320

Tyr Gln Asn Thr Asn Val Pro Asn Ile Tyr Ser Leu Gly Asp Val Val
                325                 330                 335

Gly Lys Val Glu Leu Thr Pro Val Ala Ile Ala Ala Gly Arg Lys Leu
            340                 345                 350

Ser Asn Arg Leu Phe Gly Pro Glu Lys Phe Arg Asn Asp Lys Leu Asp
        355                 360                 365

Tyr Glu Asn Val Pro Ser Val Ile Phe Ser His Pro Glu Ala Gly Ser
    370                 375                 380

Ile Gly Ile Ser Glu Lys Glu Ala Ile Glu Lys Tyr Gly Lys Glu Asn
385                 390                 395                 400

Ile Lys Val Tyr Asn Ser Lys Phe Thr Ala Met Tyr Tyr Ala Met Leu
                405                 410                 415

Ser Glu Lys Ser Pro Thr Arg Tyr Lys Ile Val Cys Ala Gly Pro Asn
            420                 425                 430

Glu Lys Val Val Gly Leu His Ile Val Gly Asp Ser Ser Ala Glu Ile
        435                 440                 445

Leu Gln Gly Phe Gly Val Ala Ile Lys Met Gly Ala Thr Lys Ala Asp
    450                 455                 460

Phe Asp Asn Cys Val Ala Ile His Pro Thr Ser Ala Glu Glu Leu Val
465                 470                 475                 480

Thr Met Arg

<210> 253
<211> 451
<212> PRT
<213> Saccharomyces cerevisiae

<400> 253
Met Asp Gly Asn Asp His Lys Val Glu Asn Leu Gly Asp Val Asp Lys
1               5                   10                  15

Phe Tyr Ser Arg Ile Arg Trp Asn Asn Gln Phe Ser Tyr Met Ala Thr
            20                  25                  30

Leu Pro Pro His Leu Gln Ser Glu Met Glu Gly Gln Lys Ser Leu Leu
        35                  40                  45

Met Arg Tyr Asp Thr Tyr Arg Lys Glu Ser Ser Ser Phe Ser Gly Glu
    50                  55                  60

Gly Lys Lys Val Thr Leu Gln His Val Pro Thr Asp Phe Ser Glu Ala
65                  70                  75                  80

Ser Gln Ala Val Ile Ser Lys Lys Asp His Asp Thr His Ala Ser Ala
                85                  90                  95

Phe Val Asn Lys Ile Phe Gln Pro Glu Val Ala Glu Glu Leu Ile Val
            100                 105                 110

Asn Arg Tyr Glu Lys Leu Leu Ser Gln Arg Pro Glu Trp His Ala Pro
        115                 120                 125

Trp Lys Leu Ser Arg Val Ile Asn Gly His Leu Gly Trp Val Arg Cys
    130                 135                 140

Val Ala Ile Asp Pro Val Asp Asn Glu Trp Phe Ile Thr Gly Ser Asn
145                 150                 155                 160

Asp Thr Thr Met Lys Val Trp Asp Leu Ala Thr Gly Lys Leu Lys Thr
                165                 170                 175

Thr Leu Ala Gly His Val Met Thr Val Arg Asp Val Ala Val Ser Asp
            180                 185                 190

Arg His Pro Tyr Leu Phe Ser Val Ser Glu Asp Lys Thr Val Lys Cys
        195                 200                 205

Trp Asp Leu Glu Lys Asn Gln Ile Ile Arg Asp Tyr Tyr Gly His Leu
    210                 215                 220

Ser Gly Val Arg Thr Val Ser Ile His Pro Thr Leu Asp Leu Ile Ala
225                 230                 235                 240

Thr Ala Gly Arg Asp Ser Val Ile Lys Leu Trp Asp Met Arg Thr Arg
                245                 250                 255

Ile Pro Val Ile Thr Leu Val Gly His Lys Gly Pro Ile Asn Gln Val

260                 265                 270

Gln Cys Thr Pro Val Asp Pro Gln Val Val Ser Ser Ser Thr Asp Ala
        275                 280                 285

Thr Val Arg Leu Trp Asp Val Val Ala Gly Lys Thr Met Lys Val Leu
    290                 295                 300

Thr His His Lys Arg Ser Val Arg Ala Thr Ala Leu His Pro Lys Glu
305                 310                 315                 320

Phe Ser Val Ala Ser Ala Cys Thr Asp Asp Ile Arg Ser Trp Gly Leu
                325                 330                 335

Ala Glu Gly Ser Leu Leu Thr Asn Phe Glu Ser Glu Lys Thr Gly Ile
            340                 345                 350

Ile Asn Thr Leu Ser Ile Asn Gln Asp Asp Val Leu Phe Ala Gly Gly
        355                 360                 365

Asp Asn Gly Val Leu Ser Phe Tyr Asp Tyr Lys Ser Gly His Lys Tyr
    370                 375                 380

Gln Ser Leu Ala Thr Arg Glu Met Val Gly Ser Leu Glu Gly Glu Arg
385                 390                 395                 400

Ser Val Leu Cys Ser Thr Phe Asp Lys Thr Gly Leu Arg Leu Ile Thr
                405                 410                 415

Gly Glu Ala Asp Lys Ser Ile Lys Ile Trp Lys Gln Asp Glu Thr Ala
            420                 425                 430

Thr Lys Glu Ser Glu Pro Gly Leu Ala Trp Asn Pro Asn Leu Ser Ala
        435                 440                 445

Lys Arg Phe
    450

<210> 254
<211> 137
<212> PRT
<213> Saccharomyces cerevisiae

<400> 254
Met Lys Arg Asn Ile Ile Tyr Tyr Thr Leu Arg Leu Leu Pro His Gly
1               5                   10                  15

Ile Met Leu Val His Ser Leu Asn Gln Phe Ile Asp Glu Phe Phe Thr
            20                  25                  30

Val Thr Glu Arg Thr Thr Leu Asp Glu Met Leu Glu Leu Ser Trp Asp
        35                  40                  45

Thr Pro Thr Thr Arg Trp Val Gly Gln Leu Glu Trp Pro Gln Lys Val
    50                  55                  60

Val Ser Leu Leu Glu Val Trp Thr Asn Ser Asp Asp Phe Val Asn Gln
65                  70                  75                  80

Ile Val Asn Gly Gln Asp Thr Ile Leu Thr Gln Val Gly Phe Asp Asp
                85                  90                  95

Ser Ile Val Gly Gln Trp Asn Ser Leu Leu Val Asp Leu Thr Glu Thr
            100                 105                 110

Ser Leu Val Asp Gln Leu Ser Asn Ser Arg Val Gly Trp Val Thr Val
        115                 120                 125

Ser Asn Val Trp Phe Asn Gln Leu Gln
    130                 135

<210> 255
<211> 449
<212> PRT
<213> Saccharomyces cerevisiae

<400> 255
Met Leu Arg Ile Ser Ile Asp Ser Ile Lys Gln Phe Gly Ser Phe Val
1               5                   10                  15

Thr Gly Tyr Asn Asn Thr Ser Tyr His Ala Ala Gly Arg Ala Ile Arg
            20                  25                  30

Thr Ser Ser Leu Tyr Ser Thr Met Ile Ser Ala Asn Pro Arg Arg Cys
        35                  40                  45

Leu His Ser Ser Lys Leu Leu Asn Lys Glu Gly Gln Glu Glu Gly Tyr
    50                  55                  60

Asn Glu Gln Leu Ile Ser Lys Met Ser Ser Gln Asn Gly Ser Asn Ser
65                  70                  75                  80

Arg Gln Asn Glu Ser Glu Gly Lys Lys Glu Gly Lys Ala Ser Ser Val
                85                  90                  95

Lys Ser Leu Leu Gln His Thr His Ser His Ser His Thr His Met His
            100                 105                 110

Asp Asn Pro Leu Leu Ser Leu Asn Val Gln Gln Ile Lys Lys Asn Pro
        115                 120                 125

Gly Val Arg Ile Thr Trp Ile Gly Leu Ala Ser Asn Val Gly Met Ala
    130                 135                 140

Val Gly Lys Phe Val Gly Gly Ile Thr Phe His Ser Gln Ala Leu Leu
145                 150                 155                 160

Ala Asp Ser Val His Ala Leu Ser Asp Leu Val Ser Asp Phe Leu Thr
                165                 170                 175

Leu Phe Ser Val Gln Tyr Ala Ser Arg Lys Pro Thr Ser Glu Tyr Pro
            180                 185                 190

Tyr Gly Tyr Gly Lys Val Glu Thr Val Gly Ser Leu Ala Val Ser Thr
        195                 200                 205

Ile Leu Ala Met Ala Gly Ile Ser Ile Gly Trp Ser Ser Leu Cys Ala
    210                 215                 220

Ile Val Gly Pro Val Ile Pro His Ala Ile Leu Glu Ser Met Ala Gly
225                 230                 235                 240

Leu Ile Gly Glu Thr His Ser His Ser Gln Ser Leu Thr Gln Gln Ala
                245                 250                 255

Thr Asn Val Asn Ala Val Trp Ile Ala Ala Gly Ser Ile Leu Val Lys
            260                 265                 270

Glu Trp Val Phe Gln Ala Thr Lys Lys Val Ala Ile Gln Thr Asn Ser
        275                 280                 285

Asn Val Leu Met Ala Asn Ala Trp His His Arg Val Asp Ser Leu Thr
    290                 295                 300

Ser Leu Val Ala Leu Val Ala Ile Thr Ser Ser Tyr Phe Phe Asn Ile
305                 310                 315                 320

Gln Ser Leu Asp Asn Leu Gly Gly Leu Val Val Ser Gly Leu Ile Ile
                325                 330                 335

Lys Thr Gly Gly Gln Gly Ile Leu Ser Ser Leu Lys Glu Leu Val Asp
            340                 345                 350

Gln Ser Ile Pro Pro Thr Asp Pro Arg Tyr Leu Glu Ile Glu Ser Val
        355                 360                 365

Ile Lys Asp Ser Ile Gly Ser Leu Lys Thr Asp Leu Asp Leu Lys Gln
    370                 375                 380

Ser Leu His Val Arg Asp Leu Thr Ile Leu Ala Ser Gly Pro Asn Leu
385                 390                 395                 400

Arg Ala Thr Thr Thr Leu Glu Val Pro Val Leu His Ser Gly Gln Glu
                405                 410                 415

Val Gly Ile Arg Phe Leu Glu Asn Ala Ile Ser Thr Ile Arg Glu Asp
            420                 425                 430

Leu Arg Met Lys Val Pro Asn Val Gly Arg Trp Thr Ser Ser Leu Leu
        435                 440                 445

Met

<210> 256

<211> 1065
<212> PRT
<213> Saccharomyces cerevisiae

<400> 256
Met Asp Tyr Leu Lys Pro Ala Gln Lys Gly Arg Arg Arg Gly Leu Ser
1               5                   10                  15

Ile Asn Ser Leu Ser Glu Thr Gln Gln Ser Ala Met Asn Ser Ser Leu
            20                  25                  30

Asp His Leu Gln Asn Asp Leu Asn Arg Ile Asn Leu Gln Trp Asn Arg
        35                  40                  45

Ile Leu Ser Asp Asn Thr Asn Pro Leu Glu Leu Ala Leu Ala Phe Leu
    50                  55                  60

Asp Asp Thr Ser Val Gly Leu Gly His Arg Tyr Glu Glu Phe Asn Gln
65                  70                  75                  80

Leu Lys Ser Gln Ile Gly Ser His Leu Gln Asp Val Val Asn Glu His
                85                  90                  95

Ser Gln Val Phe Asn Thr Asn Val Ala Ser Tyr Gly Lys Ala Val Ser
            100                 105                 110

Ser Ile Met Gln Ala Gln Glu Gln Thr Leu Asn Leu Lys Asn Cys Leu
        115                 120                 125

Lys Glu Ala Asn Glu Lys Ile Thr Thr Asp Lys Gly Ser Leu Gln Glu
    130                 135                 140

Leu Asn Asp Asn Asn Leu Lys Tyr Thr Lys Met Ile Asp Val Leu Val
145                 150                 155                 160

Asn Ile Glu Glu Leu Leu Gln Ile Pro Glu Lys Ile Glu Glu Asn Ile
                165                 170                 175

Arg Lys Glu Asn Phe His Gln Val Gln Ile Leu Leu Glu Arg Gly Phe
            180                 185                 190

Ile Leu Met Asn Asn Lys Ser Leu Lys Thr Val Glu Ile Leu Lys Pro
        195                 200                 205

Ile Asn Gln Gln Leu Glu Leu Gln Glu His Leu Leu Phe Asn Asn Leu
    210                 215                 220

Ile Glu Glu Ile His Asp Ile Met Tyr Ser Lys Ser Asn Lys Thr Asn
225                 230                 235                 240

Phe Thr Arg Val Thr Asn Asn Asp Ile Phe Lys Ile Ile Ser Ile Ser
                245                 250                 255

His Asn Gly Phe Thr Ser Leu Glu Asn Tyr Leu Tyr Asn Ile Val Asn
            260                 265                 270

Ile Asp Ile Met Glu His Ser Lys Thr Ile Asn Lys Asn Leu Glu Gln
        275                 280                 285

Phe Ile His Asp Gln Ser Leu Asn Lys Gly Asn Ile Met Leu Gln Glu
    290                 295                 300

Asn Ala Ala Thr Gln Ala Pro Leu Ala Pro Ser Arg Asn Gln Glu Asn
305                 310                 315                 320

Glu Gly Phe Asn Arg Ile Gly Phe Leu Leu Lys Thr Ile Asn Asn Ile
                325                 330                 335

Asn Lys Leu Pro Val Ala Phe Asn Ile Ile Thr Glu Arg Ala Lys Glu
            340                 345                 350

Glu Ile His Asn Ile Ile Val Lys Ser Thr Glu Ser Ile Arg Ser Lys
        355                 360                 365

His Pro Ser Leu Leu Lys Met Ala Thr Ser Leu Lys Asn Asp Asn His
    370                 375                 380

Phe Gly Leu Pro Val Gln Asp Ile Leu Ser Ile Ile Leu Arg Glu Cys
385                 390                 395                 400

Phe Trp Glu Ile Phe Leu Lys Leu Leu Tyr Ala Ile Gln Cys His Arg
                405                 410                 415

Ala Ile Phe Glu Met Ser Asn Ile Leu Gln Pro Thr Ser Ser Ala Lys
            420                 425                 430

Pro Ala Phe Lys Phe Asn Lys Ile Trp Gly Lys Leu Leu Asp Glu Ile
        435                 440                 445

Glu Leu Leu Leu Val Arg Tyr Ile Asn Asp Pro Glu Leu Ile Ser Ser
    450                 455                 460

Asn Asn Gly Ser Ile Lys Pro Ile Asn Gly Ala Thr Asn Asn Ala Pro
465                 470                 475                 480

Thr Leu Pro Lys Arg Lys Asn Pro Lys Ile Phe Ser Leu Glu Tyr Asn
                485                 490                 495

Ile Glu Asp Asn Ser Ser Val Lys Asp Gln Ala Phe Glu Leu Lys Ala
            500                 505                 510

Leu Leu Lys Asp Ile Phe Pro Gly Phe Ser Val Ser Ser Asn Met Asp
        515                 520                 525

Leu Asp Ser Ile Tyr Val Lys Asp Glu Ser Phe Glu Gln Asp Glu Pro
    530                 535                 540

Leu Val Pro Pro Ser Val Phe Asn Met Lys Val Ile Leu Asp Pro Phe
545                 550                 555                 560

Leu Leu Phe Thr Gln Ser Thr Ser Thr Ile Val Pro Ser Val Leu Thr
                565                 570                 575

Gln Asn Thr Ile Ser Ser Leu Thr Phe Phe Asp Asp Tyr Met Asn Lys
            580                 585                 590

Ser Phe Leu Pro Lys Ile Gln Met Thr Met Asp Tyr Leu Phe Thr Val
        595                 600                 605

Glu Val Glu Ser Asn Asn Pro Tyr Ala Leu Glu Leu Ser Asp Glu Asn
    610                 615                 620

His Asn Ile Phe Lys Thr Ala Leu Asp Phe Gln Arg Leu Phe Tyr Asn
625                 630                 635                 640

Leu Leu Asn Val Phe Asn Thr Ala Asn Thr Phe Arg Glu Lys Ile Ser
                645                 650                 655

Tyr Cys Ile Leu Asp Leu Leu Asn His Phe Tyr Asn Tyr Tyr Leu Gly
            660                 665                 670

Leu Phe Asn Ser Leu Ile Gly Thr Ser Asp Arg His Leu Thr Arg Lys
        675                 680                 685

Ile Ile Thr Ala Trp Leu Gln Asn Gly Ile Leu Met Asp Gln Glu Gln
    690                 695                 700

Lys Ile Leu Asn Gly Asp Glu Thr Leu Phe His Glu Glu Ser Ile Glu
705                 710                 715                 720

Leu Phe Lys Glu Ile Pro His Phe Tyr Gln Ala Gly Lys Gly Leu Ser
                725                 730                 735

Lys Ser Asp Leu Phe Asn Asn Leu Thr Leu Asp Thr Ile Leu Gln Phe
            740                 745                 750

Ser Ala Ser Val Leu Trp Ile Leu Asn Trp Leu Pro Gly Leu Lys Lys
        755                 760                 765

Ala Ile Asn Ile Asp Glu Val Ser Gln Glu Pro Met Leu Asp Ala Asp
    770                 775                 780

Arg Leu Arg Ser Ser Trp Thr Phe Ser Glu Ser Met Asp Leu Asn Tyr
785                 790                 795                 800

Ser Asn Pro Ser Ser Ser Pro Asn Ser Leu Gly Asn Leu Lys Ile Leu
                805                 810                 815

Leu Asp Asp Lys Ala Ser Lys Lys Phe Asp Glu Thr Ile Asp Gly Phe
            820                 825                 830

Lys Thr Leu Lys Phe Lys Leu Ile Thr Ile Leu Arg Phe Asn Ile Arg
        835                 840                 845

Ala Leu Cys Ile Tyr Asp Ile Gly Ser Phe Phe Gln Asn Thr Lys Ile
    850                 855                 860

Trp Asn Met Asp Val Gly Ser Ile Glu Leu Asp Gln Asn Ile Ala Ser

865                 870                 875                 880

Leu Ile Ser Glu Leu Arg Arg Thr Glu Ser Lys Leu Lys Gln Gln Leu
                885                 890                 895

Pro Glu Lys Glu Lys Asn Ser Ile Phe Ile Gly Leu Asp Ile Val Asn
            900                 905                 910

Asn Tyr Ala Leu Ile Lys Gly Ala Lys Ser Ile Lys Val Leu Asn His
        915                 920                 925

Asn Gly Ile Lys Lys Met Leu Arg Asn Val Asn Val Leu Gln His Ala
    930                 935                 940

Tyr Arg Asn Leu Ser Ser Glu Pro Ser Lys Ile Asn Met Asn Val Thr
945                 950                 955                 960

Met Asn Phe Tyr Ser Leu Cys Gly Ser Ser Glu Ala Glu Leu Phe Glu
                965                 970                 975

Tyr Ile Lys Asp Asn Glu Leu Pro His Cys Ser Val Glu Asp Leu Lys
            980                 985                 990

Thr Ile Leu Arg Leu Gln Phe Ser Glu Glu Met His Arg Gln Leu Lys
        995                 1000                1005

Arg Gln Ser Thr Ser Ser Thr Lys Gly Ser Ile Lys Pro Ser Asn Lys
   1010                1015                1020

Arg Tyr Thr Glu Ala Leu Glu Lys Leu Ser Asn Leu Glu Lys Glu Gln
1025                1030                1035                1040

Ser Lys Glu Gly Ala Arg Thr Lys Ile Gly Lys Leu Lys Ser Lys Leu
                1045                1050                1055

Asn Ala Val His Thr Ala Asn Glu Lys
            1060                1065


<210> 257
<211> 22
<212> DNA
<213> Artificial Sequence

<220>
<223> Description of Artificial Sequence: Synthetic primer

<400> 257
atgaagctga tacgcggcat ac                                          22


<210> 258
<211> 23
<212> DNA
<213> Artificial Sequence

<220>
<223> Description of Artificial Sequence: Synthetic primer

<400> 258
atgttagaag atctcaaacg cca                                              23


<210> 259
<211> 26
<212> DNA
<213> Artificial Sequence

<220>
<223> Description of Artificial Sequence: Synthetic primer

<400> 259
atgtctaaaa aattaggttt tgccct                                           26


<210> 260
<211> 24
<212> DNA
<213> Artificial Sequence

<220>
<223> Description of Artificial Sequence: Synthetic primer

<400> 260
atgctcccgc cttatttttt gttc                                             24


<210> 261
<211> 23
<212> DNA
<213> Artificial Sequence

<220>
<223> Description of Artificial Sequence: Synthetic primer

<400> 261
atgctacaat gtggcgcgaa gaa                                              23


<210> 262
<211> 25
<212> DNA
<213> Artificial Sequence

<220>
<223> Description of Artificial Sequence: Synthetic primer

<400> 262
atgagtataa aaaatctacc tgccg                                            25

<210> 263
<211> 27
<212> DNA
<213> Artificial Sequence

<220>
<223> Description of Artificial Sequence: Synthetic primer

<400> 263
atgatgcagt tagttcactt atttatg                                          27


<210> 264
<211> 22
<212> DNA
<213> Artificial Sequence

<220>
<223> Description of Artificial Sequence: Synthetic primer

<400> 264
atggatagcg cacgcgccct ta                                               22


<210> 265
<211> 25
<212> DNA
<213> Artificial Sequence

<220>
<223> Description of Artificial Sequence: Synthetic primer

<400> 265
atgaaattgc cagtcagaga atttg                                            25


<210> 266
<211> 23
<212> DNA
<213> Artificial Sequence

<220>
<223> Description of Artificial Sequence: Synthetic primer

<400> 266
atgcagaaca gcgctttgaa agc                                              23


<210> 267
<211> 23
<212> DNA
<213> Artificial Sequence

<220>
<223> Description of Artificial Sequence: Synthetic primer

<400> 267
atggaaaagg gtactgttaa gtg                                              23

<210> 268
<211> 25
<212> DNA
<213> Artificial Sequence

<220>
<223> Description of Artificial Sequence: Synthetic primer

<400> 268
atgaatttat taaaatcgct ggccg                                            25

<210> 269
<211> 28
<212> DNA
<213> Artificial Sequence

<220>
<223> Description of Artificial Sequence: Synthetic primer

<400> 269
atgattaaat ttctctctgc attaattc                                         28

<210> 270
<211> 23
<212> DNA
<213> Artificial Sequence

<220>
<223> Description of Artificial Sequence: Synthetic primer

<400> 270
atgcgcgtac tggttgttga aga                                              23

<210> 271
<211> 20
<212> DNA
<213> Artificial Sequence

<220>
<223> Description of Artificial Sequence: Synthetic primer

<400> 271
atgagtaatc aggaaccggc                                                  20

<210> 272
<211> 24
<212> DNA
<213> Artificial Sequence

<220>
<223> Description of Artificial Sequence: Synthetic primer

<400> 272
atgagcacaa aaccactctt cctg                                          24


<210> 273
<211> 23
<212> DNA
<213> Artificial Sequence

<220>
<223> Description of Artificial Sequence: Synthetic primer

<400> 273
atgacaacgt ttcattcctt aac                                           23


<210> 274
<211> 27
<212> DNA
<213> Artificial Sequence

<220>
<223> Description of Artificial Sequence: Synthetic primer

<400> 274
atgtcaaata aaatgactgg tttagta                                       27


<210> 275
<211> 25
<212> DNA
<213> Artificial Sequence

<220>
<223> Description of Artificial Sequence: Synthetic primer

<400> 275
atgtcatctg aaaaactgta ttccc                                         25


<210> 276
<211> 24
<212> DNA
<213> Artificial Sequence

<220>
<223> Description of Artificial Sequence: Synthetic primer

<400> 276
atgccaaaaa ttaagaccgt acgc                                          24

<210> 277
<211> 25
<212> DNA
<213> Artificial Sequence

<220>
<223> Description of Artificial Sequence: Synthetic primer

<400> 277
atgactatca aagtaggtat caacg                                          25


<210> 278
<211> 25
<212> DNA
<213> Artificial Sequence

<220>
<223> Description of Artificial Sequence: Synthetic primer

<400> 278
atggatataa tgagaagtgt tgtgg                                          25


<210> 279
<211> 24
<212> DNA
<213> Artificial Sequence

<220>
<223> Description of Artificial Sequence: Synthetic primer

<400> 279
atgaggtttt tattgggcgt gctg                                           24


<210> 280
<211> 23
<212> DNA
<213> Artificial Sequence

<220>
<223> Description of Artificial Sequence: Synthetic primer

<400> 280
atgaatattc gccgtaaaaa ccg                                            23


<210> 281
<211> 23
<212> DNA
<213> Artificial Sequence

<220>
<223> Description of Artificial Sequence: Synthetic primer

<400> 281
atgagcgacc ttgcgagaga aat                                              23

<210> 282
<211> 24
<212> DNA
<213> Artificial Sequence

<220>
<223> Description of Artificial Sequence: Synthetic primer

<400> 282
atgacaacgg taacgcccac atat                                             24

<210> 283
<211> 23
<212> DNA
<213> Artificial Sequence

<220>
<223> Description of Artificial Sequence: Synthetic primer

<400> 283
atggattata cgctcacccg cat                                              23

<210> 284
<211> 27
<212> DNA
<213> Artificial Sequence

<220>
<223> Description of Artificial Sequence: Synthetic primer

<400> 284
atgctgaagt tactgaaaac tattatg                                          27

<210> 285
<211> 24
<212> DNA
<213> Artificial Sequence

<220>
<223> Description of Artificial Sequence: Synthetic primer

<400> 285
atggctatcg acgaaaacaa acag                                             24

<210> 286
<211> 23
<212> DNA
<213> Artificial Sequence

<220>
<223> Description of Artificial Sequence: Synthetic primer

<400> 286
atgagtgcaa tagaaaattt cga                                                23


<210> 287
<211> 28
<212> DNA
<213> Artificial Sequence

<220>
<223> Description of Artificial Sequence: Synthetic primer

<400> 287
atgatgaaat cgttaattat cgttaatc                                           28


<210> 288
<211> 22
<212> DNA
<213> Artificial Sequence

<220>
<223> Description of Artificial Sequence: Synthetic primer

<400> 288
atgtctgtaa ttaagatgac cg                                                 22


<210> 289
<211> 24
<212> DNA
<213> Artificial Sequence

<220>
<223> Description of Artificial Sequence: Synthetic primer

<400> 289
atgaaagatg aacgtcgccc tatt                                               24


<210> 290
<211> 26
<212> DNA
<213> Artificial Sequence

<220>
<223> Description of Artificial Sequence: Synthetic primer

<400> 290
atgctaaatt tatttgttgg ccttga                                             26

<210> 291
<211> 22
<212> DNA
<213> Artificial Sequence

<220>
<223> Description of Artificial Sequence: Synthetic primer

<400> 291
atgacgcaaa cttataacgc tg                                                22

<210> 292
<211> 21
<212> DNA
<213> Artificial Sequence

<220>
<223> Description of Artificial Sequence: Synthetic primer

<400> 292
atggctggac gaatcccacg c                                                 21

<210> 293
<211> 22
<212> DNA
<213> Artificial Sequence

<220>
<223> Description of Artificial Sequence: Synthetic primer

<400> 293
atggagcaaa acccgcagtc ac                                                22

<210> 294
<211> 24
<212> DNA
<213> Artificial Sequence

<220>
<223> Description of Artificial Sequence: Synthetic primer

<400> 294
atgtggcgca gactgattta tcac                                              24

<210> 295
<211> 24
<212> DNA
<213> Artificial Sequence

<220>
<223> Description of Artificial Sequence: Synthetic primer

<400> 295
atgattgttt tacccagacg cctg                                              24


<210> 296
<211> 24
<212> DNA
<213> Artificial Sequence

<220>
<223> Description of Artificial Sequence: Synthetic primer

<400> 296
atgcgtaact ttgatttatc cccg                                              24


<210> 297
<211> 24
<212> DNA
<213> Artificial Sequence

<220>
<223> Description of Artificial Sequence: Synthetic primer

<400> 297
atgaccgatt tacacaccga tgta                                              24


<210> 298
<211> 22
<212> DNA
<213> Artificial Sequence

<220>
<223> Description of Artificial Sequence: Synthetic primer

<400> 298
atgttgaccc cgttgacggc ct                                                22


<210> 299
<211> 25
<212> DNA
<213> Artificial Sequence

<220>
<223> Description of Artificial Sequence: Synthetic primer

<400> 299
atgaaaaaga ttgcatttgg ctgtg                                             25


<210> 300
<211> 24
<212> DNA
<213> Artificial Sequence

<220>
<223> Description of Artificial Sequence: Synthetic primer

<400> 300
atgacggttc ctacctatga caaa                                              24


<210> 301
<211> 25
<212> DNA
<213> Artificial Sequence

<220>
<223> Description of Artificial Sequence: Synthetic primer

<400> 301
atgtcaaaag ctgtcggtat tgatt                                             25


<210> 302
<211> 24
<212> DNA
<213> Artificial Sequence

<220>
<223> Description of Artificial Sequence: Synthetic primer

<400> 302
atgtccgacg aaattgtttg gcaa                                              24


<210> 303
<211> 24
<212> DNA
<213> Artificial Sequence

<220>
<223> Description of Artificial Sequence: Synthetic primer

<400> 303
atggtacgtc gatggattcc tagt                                              24


<210> 304
<211> 25
<212> DNA
<213> Artificial Sequence

<220>
<223> Description of Artificial Sequence: Synthetic primer

<400> 304
atgcaaacac cttcagaaaa taccg                                             25

<210> 305
<211> 24
<212> DNA
<213> Artificial Sequence

<220>
<223> Description of Artificial Sequence: Synthetic primer

<400> 305
atgccacaag tacagtcgtg gttt                                              24


<210> 306
<211> 24
<212> DNA
<213> Artificial Sequence

<220>
<223> Description of Artificial Sequence: Synthetic primer

<400> 306
atgaatacca acgagtccga acat                                              24


<210> 307
<211> 25
<212> DNA
<213> Artificial Sequence

<220>
<223> Description of Artificial Sequence: Synthetic primer

<400> 307
atgttgttca aaccaaaaac acgag                                             25


<210> 308
<211> 23
<212> DNA
<213> Artificial Sequence

<220>
<223> Description of Artificial Sequence: Synthetic primer

<400> 308
atgtccctac ggccttgtct aac                                               23


<210> 309
<211> 25
<212> DNA
<213> Artificial Sequence

<220>
<223> Description of Artificial Sequence: Synthetic primer

<400> 309
atgattgtgg ggttgacact ttatt                                          25

<210> 310
<211> 25
<212> DNA
<213> Artificial Sequence

<220>
<223> Description of Artificial Sequence: Synthetic primer

<400> 310
atggaaaagc tattacagtg gtcta                                          25

<210> 311
<211> 24
<212> DNA
<213> Artificial Sequence

<220>
<223> Description of Artificial Sequence: Synthetic primer

<400> 311
atgcattgcc caatttgcca ccat                                           24

<210> 312
<211> 28
<212> DNA
<213> Artificial Sequence

<220>
<223> Description of Artificial Sequence: Synthetic primer

<400> 312
atgaatataa tgaaaagaca attatgca                                       28

<210> 313
<211> 24
<212> DNA
<213> Artificial Sequence

<220>
<223> Description of Artificial Sequence: Synthetic primer

<400> 313
atgaatatgg cagaacgtgc agaa                                           24

<210> 314
<211> 24
<212> DNA
<213> Artificial Sequence

<220>
<223> Description of Artificial Sequence: Synthetic primer

<400> 314
atgttgcgac ttttttcact ggca                                              24


<210> 315
<211> 25
<212> DNA
<213> Artificial Sequence

<220>
<223> Description of Artificial Sequence: Synthetic primer

<400> 315
atgaaacctc cgttaaacat gtcac                                             25


<210> 316
<211> 26
<212> DNA
<213> Artificial Sequence

<220>
<223> Description of Artificial Sequence: Synthetic primer

<400> 316
atgttctcat attcagattt ctgttc                                            26


<210> 317
<211> 26
<212> DNA
<213> Artificial Sequence

<220>
<223> Description of Artificial Sequence: Synthetic primer

<400> 317
atgaatttag aagagagcca atcaaa                                            26


<210> 318
<211> 26
<212> DNA
<213> Artificial Sequence

<220>
<223> Description of Artificial Sequence: Synthetic primer

<400> 318
atgaaagaga atgaagtcaa agatga                                            26

<210> 319
<211> 26
<212> DNA
<213> Artificial Sequence

<220>
<223> Description of Artificial Sequence: Synthetic primer

<400> 319
atgactgtta taaagacaga accaac                                          26


<210> 320
<211> 27
<212> DNA
<213> Artificial Sequence

<220>
<223> Description of Artificial Sequence: Synthetic primer

<400> 320
atgatttata tgttggtttt tctggat                                         27


<210> 321
<211> 25
<212> DNA
<213> Artificial Sequence

<220>
<223> Description of Artificial Sequence: Synthetic primer

<400> 321
atgtgggatt cgttaattgt aagca                                           25


<210> 322
<211> 27
<212> DNA
<213> Artificial Sequence

<220>
<223> Description of Artificial Sequence: Synthetic primer

<400> 322
atggattttt atactactga tatcaac                                         27


<210> 323
<211> 23
<212> DNA
<213> Artificial Sequence

<220>
<223> Description of Artificial Sequence: Synthetic primer

<400> 323
atgccttgct tgttgccacc tac                                              23

<210> 324
<211> 25
<212> DNA
<213> Artificial Sequence

<220>
<223> Description of Artificial Sequence: Synthetic primer

<400> 324
atggacgcat tgaactccaa agaac                                            25

<210> 325
<211> 26
<212> DNA
<213> Artificial Sequence

<220>
<223> Description of Artificial Sequence: Synthetic primer

<400> 325
atgagtcttt ctttccttct cttttc                                           26

<210> 326
<211> 23
<212> DNA
<213> Artificial Sequence

<220>
<223> Description of Artificial Sequence: Synthetic primer

<400> 326
atgaaggaca gtgtagactg ccc                                              23

<210> 327
<211> 24
<212> DNA
<213> Artificial Sequence

<220>
<223> Description of Artificial Sequence: Synthetic primer

<400> 327
atgaatttca aggagccgct agtg                                             24

<210> 328
<211> 24
<212> DNA
<213> Artificial Sequence

<220>
<223> Description of Artificial Sequence: Synthetic primer

<400> 328
atggccgatg aggaacgttt aaag                                              24


<210> 329
<211> 25
<212> DNA
<213> Artificial Sequence

<220>
<223> Description of Artificial Sequence: Synthetic primer

<400> 329
atggcagatc aagtaccagt tacaa                                             25


<210> 330
<211> 23
<212> DNA
<213> Artificial Sequence

<220>
<223> Description of Artificial Sequence: Synthetic primer

<400> 330
atgtccacaa ctgattcggg gtt                                               23


<210> 331
<211> 26
<212> DNA
<213> Artificial Sequence

<220>
<223> Description of Artificial Sequence: Synthetic primer

<400> 331
atgaaactgg atagtataga cattac                                            26


<210> 332
<211> 25
<212> DNA
<213> Artificial Sequence

<220>
<223> Description of Artificial Sequence: Synthetic primer

<400> 332
atgaatagcg agtacgatta cctgt                                             25

<210> 333
<211> 26
<212> DNA
<213> Artificial Sequence

<220>
<223> Description of Artificial Sequence: Synthetic primer

<400> 333
atgatttcac ccataagctt tctatc                                          26


<210> 334
<211> 25
<212> DNA
<213> Artificial Sequence

<220>
<223> Description of Artificial Sequence: Synthetic primer

<400> 334
atgaataggg acaacatgga cacaa                                           25


<210> 335
<211> 25
<212> DNA
<213> Artificial Sequence

<220>
<223> Description of Artificial Sequence: Synthetic primer

<400> 335
atggaagaag tgaaagcagt caatt                                           25


<210> 336
<211> 25
<212> DNA
<213> Artificial Sequence

<220>
<223> Description of Artificial Sequence: Synthetic primer

<400> 336
atgagcattc tatcatccac acaat                                           25


<210> 337
<211> 24
<212> DNA
<213> Artificial Sequence

<220>
<223> Description of Artificial Sequence: Synthetic primer

<400> 337
atggacctag atctagccag tatc                                              24


<210> 338
<211> 26
<212> DNA
<213> Artificial Sequence

<220>
<223> Description of Artificial Sequence: Synthetic primer

<400> 338
atgaatagta atgaactgga tctaag                                            26


<210> 339
<211> 24
<212> DNA
<213> Artificial Sequence

<220>
<223> Description of Artificial Sequence: Synthetic primer

<400> 339
atgacagcct catcaaatga cgat                                              24


<210> 340
<211> 24
<212> DNA
<213> Artificial Sequence

<220>
<223> Description of Artificial Sequence: Synthetic primer

<400> 340
atgtctcgcc aaagcgcatt caaa                                              24


<210> 341
<211> 24
<212> DNA
<213> Artificial Sequence

<220>
<223> Description of Artificial Sequence: Synthetic primer

<400> 341
atgtctttct taggtttcgg tggt                                              24


<210> 342
<211> 25
<212> DNA
<213> Artificial Sequence

<220>
<223> Description of Artificial Sequence: Synthetic primer

<400> 342
atggcagact ttaaatcttt aggtc                                          25


<210> 343
<211> 24
<212> DNA
<213> Artificial Sequence

<220>
<223> Description of Artificial Sequence: Synthetic primer

<400> 343
atggctagag gaccgtatgt tgat                                           24


<210> 344
<211> 24
<212> DNA
<213> Artificial Sequence

<220>
<223> Description of Artificial Sequence: Synthetic primer

<400> 344
atgtgcctag gtaagctgta cttc                                           24


<210> 345
<211> 23
<212> DNA
<213> Artificial Sequence

<220>
<223> Description of Artificial Sequence: Synthetic primer

<400> 345
atgaggagag agaggcaaag gat                                            23


<210> 346
<211> 26
<212> DNA
<213> Artificial Sequence

<220>
<223> Description of Artificial Sequence: Synthetic primer

<400> 346
atgaatttct ccacagtttt tcaagc                                         26

<210> 347
<211> 24
<212> DNA
<213> Artificial Sequence

<220>
<223> Description of Artificial Sequence: Synthetic primer

<400> 347
atggccagga aattgaaggg taag                                              24


<210> 348
<211> 27
<212> DNA
<213> Artificial Sequence

<220>
<223> Description of Artificial Sequence: Synthetic primer

<400> 348
atgtatatat atgttgaagt gtgtaca                                           27


<210> 349
<211> 25
<212> DNA
<213> Artificial Sequence

<220>
<223> Description of Artificial Sequence: Synthetic primer

<400> 349
atgacaccag acgaactaaa ttcag                                             25


<210> 350
<211> 25
<212> DNA
<213> Artificial Sequence

<220>
<223> Description of Artificial Sequence: Synthetic primer

<400> 350
atgtccgtac aaaaagaaga atacg                                             25


<210> 351
<211> 25
<212> DNA
<213> Artificial Sequence

<220>
<223> Description of Artificial Sequence: Synthetic primer

<400> 351
atgactattt cttctgcaca tccag                                          25


<210> 352
<211> 25
<212> DNA
<213> Artificial Sequence

<220>
<223> Description of Artificial Sequence: Synthetic primer

<400> 352
atgggaaaga ctaaaacaag aggca                                          25


<210> 353
<211> 23
<212> DNA
<213> Artificial Sequence

<220>
<223> Description of Artificial Sequence: Synthetic primer

<400> 353
atgccaaata ctgctccgcc act                                            23


<210> 354
<211> 26
<212> DNA
<213> Artificial Sequence

<220>
<223> Description of Artificial Sequence: Synthetic primer

<400> 354
atgaggttcc agcttttcat atattt                                         26


<210> 355
<211> 25
<212> DNA
<213> Artificial Sequence

<220>
<223> Description of Artificial Sequence: Synthetic primer

<400> 355
atggacagag accatattaa tgacc                                          25


<210> 356
<211> 25
<212> DNA
<213> Artificial Sequence

<220>
<223> Description of Artificial Sequence: Synthetic primer

<400> 356
atggtagcta gttgtaaaga tcaga                                          25


<210> 357
<211> 24
<212> DNA
<213> Artificial Sequence

<220>
<223> Description of Artificial Sequence: Synthetic primer

<400> 357
atgaacaaca cttccataac cgga                                           24


<210> 358
<211> 28
<212> DNA
<213> Artificial Sequence

<220>
<223> Description of Artificial Sequence: Synthetic primer

<400> 358
atgatttctg ctaattcatt acttattt                                       28


<210> 359
<211> 27
<212> DNA
<213> Artificial Sequence

<220>
<223> Description of Artificial Sequence: Synthetic primer

<400> 359
atgcaagaat tttattcttt ttcacca                                        27


<210> 360
<211> 24
<212> DNA
<213> Artificial Sequence

<220>
<223> Description of Artificial Sequence: Synthetic primer

<400> 360
atgaattccg gaggtgaaga acca                                           24

<210> 361
<211> 25
<212> DNA
<213> Artificial Sequence

<220>
<223> Description of Artificial Sequence: Synthetic primer

<400> 361
atggctaaag atttattgcc caagc                                          25


<210> 362
<211> 26
<212> DNA
<213> Artificial Sequence

<220>
<223> Description of Artificial Sequence: Synthetic primer

<400> 362
atgtcccaag gtactttata cattaa                                         26


<210> 363
<211> 24
<212> DNA
<213> Artificial Sequence

<220>
<223> Description of Artificial Sequence: Synthetic primer

<400> 363
atgcaattct ctactgtcgc ttcc                                           24


<210> 364
<211> 24
<212> DNA
<213> Artificial Sequence

<220>
<223> Description of Artificial Sequence: Synthetic primer

<400> 364
atgagtcaaa gtcgctggag tatc                                           24


<210> 365
<211> 28
<212> DNA
<213> Artificial Sequence

<220>
<223> Description of Artificial Sequence: Synthetic primer

<400> 365
atggtatgtt tttaatttcg tttcttta                                          28


<210> 366
<211> 25
<212> DNA
<213> Artificial Sequence

<220>
<223> Description of Artificial Sequence: Synthetic primer

<400> 366
atgtctgaac cagctcaaaa gaaac                                             25


<210> 367
<211> 24
<212> DNA
<213> Artificial Sequence

<220>
<223> Description of Artificial Sequence: Synthetic primer

<400> 367
atggccggtt taaaagacgt tgtc                                              24


<210> 368
<211> 28
<212> DNA
<213> Artificial Sequence

<220>
<223> Description of Artificial Sequence: Synthetic primer

<400> 368
atgaggaatt ttttcacgtt attttttg                                          28


<210> 369
<211> 27
<212> DNA
<213> Artificial Sequence

<220>
<223> Description of Artificial Sequence: Synthetic primer

<400> 369
atgcttgttt tttcttttct gtttgtt                                           27


<210> 370
<211> 23
<212> DNA
<213> Artificial Sequence

<220>
<223> Description of Artificial Sequence: Synthetic primer

<400> 370
atggctgccg gtgaacaagt ttc                                              23


<210> 371
<211> 25
<212> DNA
<213> Artificial Sequence

<220>
<223> Description of Artificial Sequence: Synthetic primer

<400> 371
atggtcaaag ctgttatttt taccg                                            25


<210> 372
<211> 26
<212> DNA
<213> Artificial Sequence

<220>
<223> Description of Artificial Sequence: Synthetic primer

<400> 372
atgtcacgaa gtaacagtat atacac                                           26


<210> 373
<211> 27
<212> DNA
<213> Artificial Sequence

<220>
<223> Description of Artificial Sequence: Synthetic primer

<400> 373
atgtcaaaat tgttcttaga tgaactg                                          27


<210> 374
<211> 24
<212> DNA
<213> Artificial Sequence

<220>
<223> Description of Artificial Sequence: Synthetic primer

<400> 374
atgtgtggta tctatcccta ccag                                             24

<210> 375
<211> 25
<212> DNA
<213> Artificial Sequence

<220>
<223> Description of Artificial Sequence: Synthetic primer

<400> 375
atgagcggtt ttaaatgcta tttgc                                          25

<210> 376
<211> 25
<212> DNA
<213> Artificial Sequence

<220>
<223> Description of Artificial Sequence: Synthetic primer

<400> 376
atggttacat atgctggaaa actgg                                          25

<210> 377
<211> 25
<212> DNA
<213> Artificial Sequence

<220>
<223> Description of Artificial Sequence: Synthetic primer

<400> 377
atgacctatt ctacagttag catca                                          25

<210> 378
<211> 25
<212> DNA
<213> Artificial Sequence

<220>
<223> Description of Artificial Sequence: Synthetic primer

<400> 378
atgctttctg caaccaaaca aacat                                          25

<210> 379
<211> 24
<212> DNA
<213> Artificial Sequence

<220>
<223> Description of Artificial Sequence: Synthetic primer

<400> 379
atggacggaa atgatcacaa agtc                                              24


<210> 380
<211> 30
<212> DNA
<213> Artificial Sequence

<220>
<223> Description of Artificial Sequence: Synthetic primer

<400> 380
atgaagagaa atattattta ttatacattg                                        30


<210> 381
<211> 25
<212> DNA
<213> Artificial Sequence

<220>
<223> Description of Artificial Sequence: Synthetic primer

<400> 381
atgctacgga taagtattga ctcta                                             25


<210> 382
<211> 24
<212> DNA
<213> Artificial Sequence

<220>
<223> Description of Artificial Sequence: Synthetic primer

<400> 382
atggattacc taaaaccagc gcag                                              24


<210> 383
<211> 24
<212> DNA
<213> Artificial Sequence

<220>
<223> Description of Artificial Sequence: Synthetic primer

<400> 383
ttaagccggt tttgttagcc caaa                                              24


<210> 384
<211> 24
<212> DNA
<213> Artificial Sequence

<220>
<223> Description of Artificial Sequence: Synthetic primer

<400> 384
ttactgcccg taatatgcct tcgc                                              24


<210> 385
<211> 26
<212> DNA
<213> Artificial Sequence

<220>
<223> Description of Artificial Sequence: Synthetic primer

<400> 385
ttactgataa gtaatggtgt atgcag                                            26


<210> 386
<211> 26
<212> DNA
<213> Artificial Sequence

<220>
<223> Description of Artificial Sequence: Synthetic primer

<400> 386
ttagcatgac gcatttgaga atgttg                                            26


<210> 387
<211> 25
<212> DNA
<213> Artificial Sequence

<220>
<223> Description of Artificial Sequence: Synthetic primer

<400> 387
ttacgaatac tttttcgtcc gtcgc                                             25


<210> 388
<211> 23
<212> DNA
<213> Artificial Sequence

<220>
<223> Description of Artificial Sequence: Synthetic primer

<400> 388
ttatgcacct gcggcaatca gta                                               23

<210> 389
<211> 22
<212> DNA
<213> Artificial Sequence

<220>
<223> Description of Artificial Sequence: Synthetic primer

<400> 389
ttaccgctgc gtcacccctt cg                                                22

<210> 390
<211> 27
<212> DNA
<213> Artificial Sequence

<220>
<223> Description of Artificial Sequence: Synthetic primer

<400> 390
ttatatttcc agacatctgt tatcact                                           27

<210> 391
<211> 24
<212> DNA
<213> Artificial Sequence

<220>
<223> Description of Artificial Sequence: Synthetic primer

<400> 391
ttagtaagta cgaatcttcg gcgg                                              24

<210> 392
<211> 23
<212> DNA
<213> Artificial Sequence

<220>
<223> Description of Artificial Sequence: Synthetic primer

<400> 392
ttattcgacg ttcagcgcgt cat                                               23

<210> 393
<211> 24
<212> DNA
<213> Artificial Sequence

<220>
<223> Description of Artificial Sequence: Synthetic primer

<400> 393
ttatgcgact gccgcttcta cttc                                              24

<210> 394
<211> 19
<212> DNA
<213> Artificial Sequence

<220>
<223> Description of Artificial Sequence: Synthetic primer

<400> 394
ttacaccgtc cggcgggca                                                    19

<210> 395
<211> 25
<212> DNA
<213> Artificial Sequence

<220>
<223> Description of Artificial Sequence: Synthetic primer

<400> 395
ttagatgatt tccagttttg cccgc                                             25

<210> 396
<211> 25
<212> DNA
<213> Artificial Sequence

<220>
<223> Description of Artificial Sequence: Synthetic primer

<400> 396
ttagcgcaat tcgaacagat agccc                                             25

<210> 397
<211> 25
<212> DNA
<213> Artificial Sequence

<220>
<223> Description of Artificial Sequence: Synthetic primer

<400> 397
ttagaaaatg cgctcctgat gcacc                                             25

<210> 398
<211> 25
<212> DNA
<213> Artificial Sequence

<220>
<223> Description of Artificial Sequence: Synthetic primer

<400> 398
ttagtcattt gcatattcct tagcc                                          25


<210> 399
<211> 24
<212> DNA
<213> Artificial Sequence

<220>
<223> Description of Artificial Sequence: Synthetic primer

<400> 399
ttatgccatc cccttcgcgt caaa                                           24


<210> 400
<211> 26
<212> DNA
<213> Artificial Sequence

<220>
<223> Description of Artificial Sequence: Synthetic primer

<400> 400
ttaatcagta atgatgacat ttgctg                                         26


<210> 401
<211> 24
<212> DNA
<213> Artificial Sequence

<220>
<223> Description of Artificial Sequence: Synthetic primer

<400> 401
ttacaacgtc gggtaatcgg tata                                           24


<210> 402
<211> 21
<212> DNA
<213> Artificial Sequence

<220>
<223> Description of Artificial Sequence: Synthetic primer

<400> 402
ttatgcgtac ggcaggcacg c                                              21

<210> 403
<211> 24
<212> DNA
<213> Artificial Sequence

<220>
<223> Description of Artificial Sequence: Synthetic primer

<400> 403
ttatttggag atgtgagcga tcag                                              24


<210> 404
<211> 24
<212> DNA
<213> Artificial Sequence

<220>
<223> Description of Artificial Sequence: Synthetic primer

<400> 404
ttacgccaga ccaataaaga accc                                              24


<210> 405
<211> 23
<212> DNA
<213> Artificial Sequence

<220>
<223> Description of Artificial Sequence: Synthetic primer

<400> 405
ttatttactc tcctgcggcg aca                                               23


<210> 406
<211> 26
<212> DNA
<213> Artificial Sequence

<220>
<223> Description of Artificial Sequence: Synthetic primer

<400> 406
ttatgatgct gggtccttat aaacac                                            26


<210> 407
<211> 24
<212> DNA
<213> Artificial Sequence

<220>
<223> Description of Artificial Sequence: Synthetic primer

<400> 407
ttattcttct tctggctcgt cgtc                                              24

<210> 408
<211> 23
<212> DNA
<213> Artificial Sequence

<220>
<223> Description of Artificial Sequence: Synthetic primer

<400> 408
ttaatgatgt gccacgtcgg tct                                               23

<210> 409
<211> 24
<212> DNA
<213> Artificial Sequence

<220>
<223> Description of Artificial Sequence: Synthetic primer

<400> 409
ttaaatctcg tcaggtgtac gcag                                              24

<210> 410
<211> 24
<212> DNA
<213> Artificial Sequence

<220>
<223> Description of Artificial Sequence: Synthetic primer

<400> 410
ttatagctgc tccttagcca ccag                                              24

<210> 411
<211> 27
<212> DNA
<213> Artificial Sequence

<220>
<223> Description of Artificial Sequence: Synthetic primer

<400> 411
   ttaaaaatct tcgttagttt ctgctac                                            27

<210> 412
<211> 23
<212> DNA
<213> Artificial Sequence

<220>
<223> Description of Artificial Sequence: Synthetic primer

<400> 412
ttacaccagg ctcttcaagc gat                                              23


<210> 413
<211> 24
<212> DNA
<213> Artificial Sequence

<220>
<223> Description of Artificial Sequence: Synthetic primer

<400> 413
ttagagagga ttctcaccgc tggc                                             24


<210> 414
<211> 23
<212> DNA
<213> Artificial Sequence

<220>
<223> Description of Artificial Sequence: Synthetic primer

<400> 414
ttacttctta gcgcgctctt cga                                              23


<210> 415
<211> 24
<212> DNA
<213> Artificial Sequence

<220>
<223> Description of Artificial Sequence: Synthetic primer

<400> 415
ttaactcagg ttcatctcca gcgg                                             24


<210> 416
<211> 24
<212> DNA
<213> Artificial Sequence

<220>
<223> Description of Artificial Sequence: Synthetic primer

<400> 416
ttaaatcaac tgcaatgcta tcca                                             24

<210> 417
<211> 24
<212> DNA
<213> Artificial Sequence

<220>
<223> Description of Artificial Sequence: Synthetic primer

<400> 417
ttaaacctca atctccgcca tgtc                                              24


<210> 418
<211> 25
<212> DNA
<213> Artificial Sequence

<220>
<223> Description of Artificial Sequence: Synthetic primer

<400> 418
ttactttttc gccagctcct ggttt                                             25


<210> 419
<211> 23
<212> DNA
<213> Artificial Sequence

<220>
<223> Description of Artificial Sequence: Synthetic primer

<400> 419
ttaatcgtcc aggaagctac gca                                               23


<210> 420
<211> 22
<212> DNA
<213> Artificial Sequence

<220>
<223> Description of Artificial Sequence: Synthetic primer

<400> 420
ttacgccttc gaatcccgca ac                                                22


<210> 421
<211> 25
<212> DNA
<213> Artificial Sequence

<220>
<223> Description of Artificial Sequence: Synthetic primer

<400> 421
ttatgcgttt ttctccctcg aatgc                                          25

<210> 422
<211> 23
<212> DNA
<213> Artificial Sequence

<220>
<223> Description of Artificial Sequence: Synthetic primer

<400> 422
ttagttgatt tcgatacggc gcg                                            23

<210> 423
<211> 22
<212> DNA
<213> Artificial Sequence

<220>
<223> Description of Artificial Sequence: Synthetic primer

<400> 423
ttatttcccc cgtttggcgc gt                                             22

<210> 424
<211> 23
<212> DNA
<213> Artificial Sequence

<220>
<223> Description of Artificial Sequence: Synthetic primer

<400> 424
ttattcatcg cgcttcttcc ccc                                            23

<210> 425
<211> 25
<212> DNA
<213> Artificial Sequence

<220>
<223> Description of Artificial Sequence: Synthetic primer

<400> 425
ttaatttctc cgctgctcta ttgcc                                          25

<210> 426
<211> 28
<212> DNA
<213> Artificial Sequence

<220>
<223> Description of Artificial Sequence: Synthetic primer

<400> 426
ttactcaaaa tagtccatat ccagtttc                                          28


<210> 427
<211> 25
<212> DNA
<213> Artificial Sequence

<220>
<223> Description of Artificial Sequence: Synthetic primer

<400> 427
ttaatcaact tcttcaacgg ttgga                                             25


<210> 428
<211> 24
<212> DNA
<213> Artificial Sequence

<220>
<223> Description of Artificial Sequence: Synthetic primer

<400> 428
ttattgtgcc acttcttgct cagc                                              24


<210> 429
<211> 25
<212> DNA
<213> Artificial Sequence

<220>
<223> Description of Artificial Sequence: Synthetic primer

<400> 429
ttagggattg ttgattgata ggttg                                             25


<210> 430
<211> 25
<212> DNA
<213> Artificial Sequence

<220>
<223> Description of Artificial Sequence: Synthetic primer

<400> 430
tcaaggtaaa tcagcgtcag gatat                                             25

<210> 431
<211> 23
<212> DNA
<213> Artificial Sequence

<220>
<223> Description of Artificial Sequence: Synthetic primer

<400> 431
tcaaaccagc agccatacct tcg                                          23


<210> 432
<211> 22
<212> DNA
<213> Artificial Sequence

<220>
<223> Description of Artificial Sequence: Synthetic primer

<400> 432
tcaaggcacc tcttcgtcgt cc                                           22


<210> 433
<211> 25
<212> DNA
<213> Artificial Sequence

<220>
<223> Description of Artificial Sequence: Synthetic primer

<400> 433
tcagctctca taactatcgc aagaa                                        25


<210> 434
<211> 25
<212> DNA
<213> Artificial Sequence

<220>
<223> Description of Artificial Sequence: Synthetic primer

<400> 434
tcaatatgga gggtagaaca acagt                                        25


<210> 435
<211> 23
<212> DNA
<213> Artificial Sequence

<220>
<223> Description of Artificial Sequence: Synthetic primer

<400> 435
tcaggctggt ggtaaaaaag ctg                                              23

<210> 436
<211> 27
<212> DNA
<213> Artificial Sequence

<220>
<223> Description of Artificial Sequence: Synthetic primer

<400> 436
tcataataca tactttacgg ctaaata                                          27

<210> 437
<211> 22
<212> DNA
<213> Artificial Sequence

<220>
<223> Description of Artificial Sequence: Synthetic primer

<400> 437
ctagcctacc acgtaccatc gg                                               22

<210> 438
<211> 26
<212> DNA
<213> Artificial Sequence

<220>
<223> Description of Artificial Sequence: Synthetic primer

<400> 438
ttacttttcg ttaattgaag agagga                                           26

<210> 439
<211> 26
<212> DNA
<213> Artificial Sequence

<220>
<223> Description of Artificial Sequence: Synthetic primer

<400> 439
ctacaatttg gaattatcaa tcacct                                           26

<210> 440
<211> 24
<212> DNA
<213> Artificial Sequence

<220>
<223> Description of Artificial Sequence: Synthetic primer

<400> 440
tcaacgtact gtccataggt tgtc                                              24


<210> 441
<211> 22
<212> DNA
<213> Artificial Sequence

<220>
<223> Description of Artificial Sequence: Synthetic primer

<400> 441
ctaggcaact gccatgtctc ca                                                22


<210> 442
<211> 25
<212> DNA
<213> Artificial Sequence

<220>
<223> Description of Artificial Sequence: Synthetic primer

<400> 442
ctattttttg gcgttcttcc tcttt                                             25


<210> 443
<211> 25
<212> DNA
<213> Artificial Sequence

<220>
<223> Description of Artificial Sequence: Synthetic primer

<400> 443
tcaaattttg aagccgaagt tggta                                             25


<210> 444
<211> 27
<212> DNA
<213> Artificial Sequence

<220>
<223> Description of Artificial Sequence: Synthetic primer

<400> 444
tcatttcttc attaaagact cttcatt                                           27

<210> 445
<211> 28
<212> DNA
<213> Artificial Sequence

<220>
<223> Description of Artificial Sequence: Synthetic primer

<400> 445
ttatataata ttgagaattt cattagcc                                          28


<210> 446
<211> 26
<212> DNA
<213> Artificial Sequence

<220>
<223> Description of Artificial Sequence: Synthetic primer

<400> 446
tcaaatcaaa acaacaggat atcgac                                            26


<210> 447
<211> 24
<212> DNA
<213> Artificial Sequence

<220>
<223> Description of Artificial Sequence: Synthetic primer

<400> 447
ctaattaact gacccttcgc caag                                              24


<210> 448
<211> 27
<212> DNA
<213> Artificial Sequence

<220>
<223> Description of Artificial Sequence: Synthetic primer

<400> 448
ttattttttg tcctttttgg ccaattt                                           27


<210> 449
<211> 23
<212> DNA
<213> Artificial Sequence

<220>
<223> Description of Artificial Sequence: Synthetic primer

<400> 449
tcactctgcc accatcgtaa tgg                                              23


<210> 450
<211> 23
<212> DNA
<213> Artificial Sequence

<220>
<223> Description of Artificial Sequence: Synthetic primer

<400> 450
ttatcggccc aagccttgtc cca                                              23


<210> 451
<211> 23
<212> DNA
<213> Artificial Sequence

<220>
<223> Description of Artificial Sequence: Synthetic primer

<400> 451
ttaactgtag gctgtgccga cat                                              23


<210> 452
<211> 26
<212> DNA
<213> Artificial Sequence

<220>
<223> Description of Artificial Sequence: Synthetic primer

<400> 452
ctattgtggc aattctttca acttat                                           26


<210> 453
<211> 28
<212> DNA
<213> Artificial Sequence

<220>
<223> Description of Artificial Sequence: Synthetic primer

<400> 453
ttaattaata gtcttcttct ttcttatc                                         28


<210> 454
<211> 27
<212> DNA
<213> Artificial Sequence

<220>
<223> Description of Artificial Sequence: Synthetic primer

<400> 454
tcacattttt ctaaattcac ttagcac                                          27


<210> 455
<211> 22
<212> DNA
<213> Artificial Sequence

<220>
<223> Description of Artificial Sequence: Synthetic primer

<400> 455
tcaggcactc tcttcctccg gt                                               22


<210> 456
<211> 25
<212> DNA
<213> Artificial Sequence

<220>
<223> Description of Artificial Sequence: Synthetic primer

<400> 456
ctaattactt aacgtttgac actcc                                            25


<210> 457
<211> 26
<212> DNA
<213> Artificial Sequence

<220>
<223> Description of Artificial Sequence: Synthetic primer

<400> 457
ttatggcttt tctaattctt gcaaga                                           26


<210> 458
<211> 21
<212> DNA
<213> Artificial Sequence

<220>
<223> Description of Artificial Sequence: Synthetic primer

<400> 458
tcaacagcag cccccaccgg t                                                21

<210> 459
<211> 26
<212> DNA
<213> Artificial Sequence

<220>
<223> Description of Artificial Sequence: Synthetic primer

<400> 459
ttatggaaaa tattcaatca caccga                                          26


<210> 460
<211> 25
<212> DNA
<213> Artificial Sequence

<220>
<223> Description of Artificial Sequence: Synthetic primer

<400> 460
ttaacgacca aatttacgta gctct                                           25


<210> 461
<211> 26
<212> DNA
<213> Artificial Sequence

<220>
<223> Description of Artificial Sequence: Synthetic primer

<400> 461
ctaatttaat ggagtagttt cgttcc                                          26


<210> 462
<211> 26
<212> DNA
<213> Artificial Sequence

<220>
<223> Description of Artificial Sequence: Synthetic primer

<400> 462
ttaactactt gagttttctt tccagc                                          26


<210> 463
<211> 23
<212> DNA
<213> Artificial Sequence

<220>
<223> Description of Artificial Sequence: Synthetic primer

<400> 463
ttatgttagc agcattccgg cct                                              23


<210> 464
<211> 28
<212> DNA
<213> Artificial Sequence

<220>
<223> Description of Artificial Sequence: Synthetic primer

<400> 464
tcaaaaattt aacagttcga taatttca                                         28


<210> 465
<211> 27
<212> DNA
<213> Artificial Sequence

<220>
<223> Description of Artificial Sequence: Synthetic primer

<400> 465
ttaaaaccaa cagaatttgg tattgtt                                          27


<210> 466
<211> 24
<212> DNA
<213> Artificial Sequence

<220>
<223> Description of Artificial Sequence: Synthetic primer

<400> 466
tcaaaggctt acatctccag aatc                                             24


<210> 467
<211> 25
<212> DNA
<213> Artificial Sequence

<220>
<223> Description of Artificial Sequence: Synthetic primer

<400> 467
ctaaaactta ccggctgcgt taaat                                            25


<210> 468
<211> 23
<212> DNA
<213> Artificial Sequence

<220>
<223> Description of Artificial Sequence: Synthetic primer

<400> 468
tcaagagcgc aaacttttcc cgt                                              23


<210> 469
<211> 25
<212> DNA
<213> Artificial Sequence

<220>
<223> Description of Artificial Sequence: Synthetic primer

<400> 469
ttacaaacct tgttgagctc ttctt                                            25


<210> 470
<211> 23
<212> DNA
<213> Artificial Sequence

<220>
<223> Description of Artificial Sequence: Synthetic primer

<400> 470
ctacacacgt ttccaacctc tgc                                              23


<210> 471
<211> 26
<212> DNA
<213> Artificial Sequence

<220>
<223> Description of Artificial Sequence: Synthetic primer

<400> 471
ctaatcattg agatcgaatt tgtaca                                           26


<210> 472
<211> 24
<212> DNA
<213> Artificial Sequence

<220>
<223> Description of Artificial Sequence: Synthetic primer

<400> 472
tcacatacat acctgcggta gcaa                                             24

<210> 473
<211> 25
<212> DNA
<213> Artificial Sequence

<220>
<223> Description of Artificial Sequence: Synthetic primer

<400> 473
ttagtctgaa tcagaatctg tgtct                                          25


<210> 474
<211> 24
<212> DNA
<213> Artificial Sequence

<220>
<223> Description of Artificial Sequence: Synthetic primer

<400> 474
ttattcaccc tcacccctaa tacc                                           24


<210> 475
<211> 24
<212> DNA
<213> Artificial Sequence

<220>
<223> Description of Artificial Sequence: Synthetic primer

<400> 475
ttactcgaac gggatttggc ctaa                                           24


<210> 476
<211> 26
<212> DNA
<213> Artificial Sequence

<220>
<223> Description of Artificial Sequence: Synthetic primer

<400> 476
ctagtgaata aacctataat caagac                                         26


<210> 477
<211> 25
<212> DNA
<213> Artificial Sequence

<220>
<223> Description of Artificial Sequence: Synthetic primer

<400> 477
tcattcagat atgtaaattc tgccc                                          25

<210> 478
<211> 27
<212> DNA
<213> Artificial Sequence

<220>
<223> Description of Artificial Sequence: Synthetic primer

<400> 478
ttatctatag tgcttttgct ttttgaa                                        27

<210> 479
<211> 23
<212> DNA
<213> Artificial Sequence

<220>
<223> Description of Artificial Sequence: Synthetic primer

<400> 479
ttaccaacct acgtatcctc tcg                                            23

<210> 480
<211> 28
<212> DNA
<213> Artificial Sequence

<220>
<223> Description of Artificial Sequence: Synthetic primer

<400> 480
ttattgaata gcattatata ttagtgcc                                       28

<210> 481
<211> 22
<212> DNA
<213> Artificial Sequence

<220>
<223> Description of Artificial Sequence: Synthetic primer

<400> 481
ctaagtacgg ccggaagaga gc                                             22

<210> 482
<211> 23
<212> DNA
<213> Artificial Sequence

<220>
<223> Description of Artificial Sequence: Synthetic primer

<400> 482
tcagtccttc tgctggctgt tca                                              23


<210> 483
<211> 28
<212> DNA
<213> Artificial Sequence

<220>
<223> Description of Artificial Sequence: Synthetic primer

<400> 483
tcaattttta tcttcatttt taatgtcc                                         28


<210> 484
<211> 24
<212> DNA
<213> Artificial Sequence

<220>
<223> Description of Artificial Sequence: Synthetic primer

<400> 484
ttaacagctt gcaccagatc caga                                             24


<210> 485
<211> 25
<212> DNA
<213> Artificial Sequence

<220>
<223> Description of Artificial Sequence: Synthetic primer

<400> 485
ttaaatctgt tcttctggtg gttga                                            25


<210> 486
<211> 26
<212> DNA
<213> Artificial Sequence

<220>
<223> Description of Artificial Sequence: Synthetic primer

<400> 486
ttaacagaga tttcccaaac attctg                                           26

<210> 487
<211> 25
<212> DNA
<213> Artificial Sequence

<220>
<223> Description of Artificial Sequence: Synthetic primer

<400> 487
ttatatccat ttctgtcttg cagca                                          25


<210> 488
<211> 26
<212> DNA
<213> Artificial Sequence

<220>
<223> Description of Artificial Sequence: Synthetic primer

<400> 488
ttatttcaaa accttaccgt caacaa                                         26


<210> 489
<211> 25
<212> DNA
<213> Artificial Sequence

<220>
<223> Description of Artificial Sequence: Synthetic primer

<400> 489
ttataacaac atagcagcag cagct                                          25


<210> 490
<211> 26
<212> DNA
<213> Artificial Sequence

<220>
<223> Description of Artificial Sequence: Synthetic primer

<400> 490
tcaatggttt ttgtaagcat ctgata                                         26


<210> 491
<211> 25
<212> DNA
<213> Artificial Sequence

<220>
<223> Description of Artificial Sequence: Synthetic primer

<400> 491
ttattgctga tcctgtaagc actca                                          25


<210> 492
<211> 26
<212> DNA
<213> Artificial Sequence

<220>
<223> Description of Artificial Sequence: Synthetic primer

<400> 492
ttaagcggta actttctttt caatca                                         26


<210> 493
<211> 25
<212> DNA
<213> Artificial Sequence

<220>
<223> Description of Artificial Sequence: Synthetic primer

<400> 493
ttaagcatct tcttcaacga caacg                                          25


<210> 494
<211> 26
<212> DNA
<213> Artificial Sequence

<220>
<223> Description of Artificial Sequence: Synthetic primer

<400> 494
tcaaatcaat ggtttttcct caattg                                         26


<210> 495
<211> 27
<212> DNA
<213> Artificial Sequence

<220>
<223> Description of Artificial Sequence: Synthetic primer

<400> 495
tcaattacaa atccatggat taagttg                                        27


<210> 496
<211> 24
<212> DNA
<213> Artificial Sequence

<220>
<223> Description of Artificial Sequence: Synthetic primer

<400> 496
tcattcccat tcaacagttg cagg                                              24


<210> 497
<211> 25
<212> DNA
<213> Artificial Sequence

<220>
<223> Description of Artificial Sequence: Synthetic primer

<400> 497
ctaattttcc atcaattcag cgact                                             25


<210> 498
<211> 26
<212> DNA
<213> Artificial Sequence

<220>
<223> Description of Artificial Sequence: Synthetic primer

<400> 498
ttaattatga ttatcgttct ggtctc                                            26


<210> 499
<211> 24
<212> DNA
<213> Artificial Sequence

<220>
<223> Description of Artificial Sequence: Synthetic primer

<400> 499
tcaatcgtat tctactccgg atcc                                              24


<210> 500
<211> 25
<212> DNA
<213> Artificial Sequence

<220>
<223> Description of Artificial Sequence: Synthetic primer

<400> 500
ttacattgcc atcacacgta aactg                                             25

<210> 501
<211> 23
<212> DNA
<213> Artificial Sequence

<220>
<223> Description of Artificial Sequence: Synthetic primer

<400> 501
tcagtcaaag gtggctttgc gaa                                                23


<210> 502
<211> 25
<212> DNA
<213> Artificial Sequence

<220>
<223> Description of Artificial Sequence: Synthetic primer

<400> 502
ttatatatct gtgggaaggg gtaca                                              25


<210> 503
<211> 22
<212> DNA
<213> Artificial Sequence

<220>
<223> Description of Artificial Sequence: Synthetic primer

<400> 503
ctactcgtat tctagcgcag cg                                                 22


<210> 504
<211> 26
<212> DNA
<213> Artificial Sequence

<220>
<223> Description of Artificial Sequence: Synthetic primer

<400> 504
tcatctcata gtaaccaatt cttctg                                             26


<210> 505
<211> 25
<212> DNA
<213> Artificial Sequence

<220>
<223> Description of Artificial Sequence: Synthetic primer

<400> 505
ctaaaatctt ttggcgctta agttg                                          25

<210> 506
<211> 26
<212> DNA
<213> Artificial Sequence

<220>
<223> Description of Artificial Sequence: Synthetic primer

<400> 506
ctattgaagt tgattgaacc atacgt                                         26

<210> 507
<211> 24
<212> DNA
<213> Artificial Sequence

<220>
<223> Description of Artificial Sequence: Synthetic primer

<400> 507
ttacatcaac aaactcgacg tcca                                           24

<210> 508
<211> 25
<212> DNA
<213> Artificial Sequence

<220>
<223> Description of Artificial Sequence: Synthetic primer

<400> 508
tcatttttcg tttgcagtat ggaca                                          25

<210> 509
<211> 197
<212> PRT
<213> Brassica napus

<400> 509
Met Ser Ala Ser Arg Phe Ile Lys Cys Val Thr Val Gly Asp Gly Ala
  1               5                  10                  15

Val Gly Lys Thr Cys Leu Leu Ile Ser Tyr Thr Ser Asn Thr Phe Pro
             20                  25                  30

Thr Asp Tyr Val Pro Thr Val Phe Asp Asn Phe Ser Ala Asn Val Val
        35                  40                  45

Val Asn Gly Ala Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Ile Leu Ala Phe Ser Leu Ile Ser Lys Ala Ser Tyr Glu Asn Val
                85                  90                  95

Ser Lys Lys Trp Ile Pro Glu Leu Thr His Tyr Ala Pro Gly Val Pro
            100                 105                 110

Ile Val Leu Val Gly Thr Lys Leu Asp Leu Arg Asp Asp Lys Gln Phe
        115                 120                 125

Phe Val Asp His Pro Gly Ala Val Pro Ile Thr Thr Ala Gln Gly Glu
    130                 135                 140

Glu Leu Met Lys Leu Ile Gly Ala Pro Ser Tyr Ile Glu Cys Ser Ser
145                 150                 155                 160

Lys Ser Gln Lys Asn Val Lys Gly Val Phe Asp Ala Ala Ile Arg Val
                165                 170                 175

Val Leu Gln Pro Pro Lys Gln Lys Lys Lys Lys Gly Lys Val Gln Lys
            180                 185                 190

Ala Cys Ser Ile Leu
        195


<210> 510
<211> 196
<212> PRT
<213> Brassica napus

<400> 510
Met Ser Ala Ser Arg Phe Ile Lys Cys Val Thr Val Gly Asp Gly Ala
1               5                   10                  15

Val Gly Lys Thr Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Asn Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Ile Leu Ala Phe Ser Leu Ile Ser Lys Ala Ser Tyr Glu Asn Ile

85                 90                  95

Ala Lys Lys Trp Ile Pro Glu Leu Arg His Tyr Ala Pro Gly Val Pro
            100                 105                 110

Ile Ile Leu Val Gly Thr Lys Leu Asp Leu Arg Asp Asp Lys Gln Phe
        115                 120                 125

Phe Ile Asp His Pro Gly Ala Val Pro Ile Thr Thr Asn Gln Gly Glu
    130                 135                 140

Glu Leu Lys Lys Leu Ile Gly Ser Pro Val Tyr Ile Glu Cys Ser Ser
145                 150                 155                 160

Lys Thr Gln Gln Asn Val Lys Ala Val Phe Asp Ala Ala Ile Lys Val
                165                 170                 175

Val Leu Gln Pro Pro Lys Ser Lys Lys Lys Lys Lys Asn Lys Asn Arg
            180                 185                 190

Cys Val Phe Leu
        195

<210> 511
<211> 197
<212> PRT
<213> Brassica napus

<400> 511
Met Ser Ala Ser Arg Phe Ile Lys Cys Val Thr Val Gly Asp Gly Ala
  1               5                  10                  15

Val Gly Lys Thr Cys Leu Leu Ile Ser Tyr Thr Ser Asn Thr Phe Pro
             20                  25                  30

Thr Asp Tyr Val Pro Thr Val Phe Asp Asn Phe Ser Ala Asn Val Val
         35                  40                  45

Val Asn Gly Ala Thr Val Asn Leu Gly Leu Trp Asp Thr Ala Gly Gln
     50                  55                  60

Glu Asp Tyr Asn Arg Leu Arg Pro Leu Ser Tyr Arg Gly Ala Asp Val
 65                  70                  75                  80

Phe Ile Leu Ala Phe Ser Leu Ile Ser Lys Ala Ser Tyr Glu Asn Val
                 85                  90                  95

Ser Lys Lys Trp Ile Pro Glu Leu Thr His Tyr Ala Pro Gly Val Pro
            100                 105                 110

Ile Val Leu Val Gly Thr Lys Leu Asp Leu Arg Asp Asp Lys Gln Phe
        115                 120                 125

Phe Val Asp His Pro Gly Ala Val Pro Ile Thr Thr Ala Gln Gly Glu
    130                 135                 140

Glu Leu Met Lys Leu Ile Gly Ala Pro Ser Tyr Ile Glu Cys Ser Ser
145                 150                 155                 160

Lys Ser Gln Glu Asn Val Lys Gly Val Phe Asp Ala Ala Ile Arg Val
                165                 170                 175

Val Leu Gln Pro Pro Lys Gln Lys Lys Lys Lys Ser Lys Ala Gln Lys
            180                 185                 190

Ala Cys Ser Ile Leu
        195

<210> 512
<211> 201
<212> PRT
<213> Brassica napus

<400> 512
Met Ser Thr Ala Arg Phe Ile Lys Cys Val Thr Val Gly Asp Gly Ala
1               5                   10                  15

Val Gly Lys Thr Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Ser Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Ala Phe Ser Leu Ile Ser Lys Ala Ser Tyr Glu Asn Ile
                85                  90                  95

Tyr Lys Lys Trp Leu Pro Glu Leu Lys His Tyr Ala Pro Ser Ile Pro
            100                 105                 110

Ile Val Leu Val Gly Thr Lys Leu Asp Leu Arg Asp Asp Lys Gln Phe
        115                 120                 125

Leu Lys Asp His Pro Gly Ala Ala Ser Ile Thr Thr Ala Gln Gly Glu
    130                 135                 140

Glu Leu Arg Lys Met Ile Gly Ala Ile Lys Tyr Ile Glu Cys Ser Ser
145                 150                 155                 160

Lys Thr Gln Gln Asn Val Lys Ala Val Phe Asp Thr Ala Ile Arg Val
                165                 170                 175

Ala Leu Arg Pro Pro Lys Ala Lys Lys Lys Ile Lys Pro Leu Arg Thr
            180                 185                 190

Lys Arg Ser Arg Thr Cys Phe Phe Phe
        195                 200

<210> 513
<211> 211
<212> PRT
<213> Brassica napus

<400> 513
Met Ser Ala Ser Val Ala Ala Ala Ser Val Ser Thr Thr Thr Thr Ala
1               5                   10                  15

Ala Thr Thr Phe Ile Lys Cys Val Thr Val Gly Asp Gly Ala Val Gly
            20                  25                  30

Lys Thr Cys Leu Leu Ile Ser Tyr Thr Ser Asn Thr Phe Pro Thr Asp
        35                  40                  45

Tyr Val Pro Thr Val Phe Asp Asn Phe Ser Ala Asn Val Leu Val Asp
    50                  55                  60

Gly Lys Thr Val Asn Leu Gly Leu Trp Asp Thr Ala Gly Gln Glu Asp
65                  70                  75                  80

Tyr Asn Arg Leu Arg Pro Leu Ser Tyr Arg Gly Ala Asp Val Phe Ile
                85                  90                  95

Leu Ala Phe Ser Leu Ile Ser Arg Pro Ser Phe Glu Asn Ile Ala Lys
            100                 105                 110

Lys Trp Val Pro Glu Leu Arg His Tyr Ala Pro Asn Val Pro Ile Val
        115                 120                 125

Leu Val Gly Thr Lys Leu Asp Leu Arg Glu Asp Lys Lys Phe Pro Met
    130                 135                 140

Asn Tyr Pro Gly Ala Cys Thr Ile Ser Thr Glu Gln Gly Gln Glu Leu
145                 150                 155                 160

Arg Lys Glu Ile Gly Ala Leu Ala Tyr Ile Glu Cys Ser Ser Lys Thr
                165                 170                 175

Gln Gln Asn Val Lys Ala Val Phe Asp Ala Ala Ile Lys Val Val Leu
            180                 185                 190

Gln Pro Pro Thr Lys Ile Lys Lys Gln Lys Arg Arg Phe Arg Phe Cys
        195                 200                 205

His Ala Leu
    210

<210> 514
<211> 216
<212> PRT

<213> Brassica napus

<400> 514
Met Ala Ser Thr Ala Ser Lys Phe Ile Lys Cys Val Thr Val Gly Asp
1               5                   10                  15

Gly Ala Val Gly Lys Thr Cys Met Leu Ile Cys Tyr Thr Ser Asn Lys
            20                  25                  30

Phe Pro Thr Asp Tyr Ile Pro Thr Val Phe Asp Asn Phe Ser Ala Asn
        35                  40                  45

Val Val Val Glu Gly Thr Thr Val Asn Leu Gly Leu Trp Asp Thr Ala
    50                  55                  60

Gly Gln Glu Asp Tyr Asn Arg Leu Arg Pro Leu Ser Tyr Arg Gly Ala
65                  70                  75                  80

Asp Val Phe Val Leu Ser Phe Ser Leu Val Ser Arg Ala Ser Tyr Glu
                85                  90                  95

Asn Val Tyr Lys Lys Trp Ile Pro Glu Leu Gln His Phe Ala Pro Gly
            100                 105                 110

Val Pro Leu Val Leu Val Gly Thr Lys Leu Asp Leu Arg Glu Asp Asn
        115                 120                 125

Lys His Tyr Leu Ala Asp His Pro Gly Leu Ser Pro Val Thr Thr Ala
    130                 135                 140

Gln Gly Glu Glu Leu Arg Lys Leu Ile Gly Ala Thr Tyr Tyr Ile Glu
145                 150                 155                 160

Cys Ser Ser Lys Thr Gln Gln Asn Val Lys Ala Val Phe Asp Ser Ala
                165                 170                 175

Ile Lys Glu Val Ile Lys Pro Val Val Lys Gln Lys Glu Lys Thr Gln
            180                 185                 190

Lys Thr Lys Lys Gln Lys Ser Asn His Gly Cys Leu Ser Asn Val Leu
        195                 200                 205

Cys Gly Arg Ile Val Thr Arg His
    210                 215


<210> 515
<211> 216
<212> PRT
<213> Brassica napus

<400> 515
Met Ala Ser Ser Ala Ser Lys Phe Ile Lys Cys Val Thr Val Gly Asp
1               5                   10                  15

Gly Ala Val Gly Lys Thr Cys Met Leu Ile Cys Tyr Thr Ser Asn Lys

20                  25                  30

Phe Pro Thr Asp Tyr Val Pro Thr Val Phe Asp Asn Phe Ser Ala Asn
        35                  40                  45

Val Val Val Glu Gly Thr Thr Val Asn Leu Gly Leu Trp Asp Thr Ala
    50                  55                  60

Gly Gln Glu Asp Tyr Asn Arg Leu Arg Pro Leu Ser Tyr Arg Gly Ala
65                  70                  75                  80

Asp Val Phe Val Leu Ser Phe Ser Leu Val Ser Arg Ala Ser Tyr Glu
                85                  90                  95

Asn Val Phe Lys Lys Trp Ile Pro Glu Leu Gln His Phe Ala Pro Gly
            100                 105                 110

Val Pro Leu Val Leu Val Gly Thr Lys Leu Asp Leu Arg Glu Asp Lys
        115                 120                 125

His Tyr Leu Ala Asp His Pro Gly Leu Ser Pro Val Thr Thr Ala Gln
    130                 135                 140

Gly Glu Glu Leu Arg Lys Leu Ile Gly Ala Thr Tyr Tyr Ile Glu Cys
145                 150                 155                 160

Ser Ser Lys Thr Gln Gln Asn Val Lys Ala Val Phe Asp Ser Ala Ile
                165                 170                 175

Lys Glu Val Ile Lys Pro Val Leu Lys Gln Lys Gly Lys Thr Lys Lys
            180                 185                 190

Lys Lys Lys Gln Gln Ser Asn His His Gly Cys Leu Ser Asn Val Leu
        195                 200                 205

Cys Gly Arg Ile Val Thr Arg His
    210                 215


<210> 516
<211> 195
<212> PRT
<213> Brassica napus

<400> 516
Met Ser Ala Ser Arg Phe Ile Lys Cys Val Thr Val Gly Asp Gly Ala
1               5                   10                  15

Val Gly Lys Thr Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Asn Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Ile Leu Ala Phe Ser Leu Ile Ser Lys Ala Ser Tyr Glu Asn Ile
                85                  90                  95

Ala Lys Lys Trp Ile Pro Glu Leu Arg His Tyr Ala Pro Gly Val Pro
            100                 105                 110

Ile Ile Leu Val Gly Thr Lys Leu Asp Leu Arg Asp Asp Lys Gln Phe
        115                 120                 125

Phe Ile Asp His Pro Gly Ala Val Pro Ile Ser Thr Asn Gln Gly Glu
    130                 135                 140

Glu Leu Lys Lys Leu Ile Gly Ser Pro Ala Tyr Ile Glu Cys Ser Ser
145                 150                 155                 160

Lys Thr Gln Gln Asn Val Lys Ala Val Phe Asp Ala Ala Ile Lys Val
                165                 170                 175

Val Leu Gln Pro Pro Lys Gln Lys Lys Lys Lys Lys Lys Asn Gly Cys
            180                 185                 190

Val Phe Leu
        195

<210> 517
<211> 198
<212> PRT
<213> Brassica napus

<400> 517
Met Ser Ala Ser Arg Phe Ile Lys Cys Val Thr Val Gly Asp Gly Ala
1               5                   10                  15

Val Gly Lys Thr Cys Leu Leu Ile Ser Tyr Thr Ser Asn Thr Phe Pro
            20                  25                  30

Thr Asp Tyr Val Pro Thr Val Phe Asp Asn Phe Ser Ala Asn Val Ile
        35                  40                  45

Val Asp Gly Asn Thr Ile Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Ala Phe Ser Leu Val Ser Lys Ala Ser Tyr Glu Asn Val
                85                  90                  95

Ser Lys Lys Trp Val Pro Glu Leu Arg His Tyr Ala Pro Gly Val Pro
            100                 105                 110

Ile Ile Leu Val Gly Thr Lys Leu Asp Leu Arg Asp Asp Lys Gln Phe
        115                 120                 125

Phe Val Glu His Pro Gly Ala Val Pro Ile Ser Thr Ala Gln Gly Glu
    130                 135                 140

Glu Leu Lys Lys Val Ile Gly Ala Pro Ala Tyr Ile Glu Cys Ser Ala
145                 150                 155                 160

Lys Thr Gln Gln Asn Val Lys Ala Val Phe Asp Ala Ala Ile Lys Val
                165                 170                 175

Val Leu Gln Pro Pro Lys Asn Lys Lys Arg Lys Lys Arg Lys Ser Gln
            180                 185                 190

Lys Ala Cys Ser Ile Leu
        195

<210> 518
<211> 196
<212> PRT
<213> Glycine max

<400> 518
Met Ser Thr Ala Arg Phe Ile Lys Cys Val Thr Val Gly Asp Gly Ala
1               5                   10                  15

Val Gly Lys Thr Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Thr
        35                  40                  45

Val Asp Gly Ser Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Cys Tyr Ser Leu Ile Ser Lys Ala Ser Tyr Glu Asn Ile
                85                  90                  95

Ser Lys Lys Trp Ile Pro Glu Leu Arg His Tyr Ala Pro Asn Val Pro
            100                 105                 110

Ile Val Leu Val Gly Thr Lys Leu Asp Leu Arg Asp Asp Lys Gln Phe
        115                 120                 125

Leu Ile Asp His Pro Gly Ser Ala Arg Ile Thr Thr Ala Gln Gly Glu
    130                 135                 140

Glu Leu Lys Lys Met Ile Gly Ala Val Thr Tyr Ile Glu Cys Ser Ser
145                 150                 155                 160

Lys Thr Gln Leu Asn Val Lys Thr Val Phe Asp Ala Ala Ile Lys Val

165                 170                 175

Ala Leu Lys Pro Pro Lys Pro Lys Lys Lys Pro Arg Lys Lys Arg Thr
            180                 185                 190

Cys Thr Phe Leu
        195


<210> 519
<211> 197
<212> PRT
<213> Glycine max

<400> 519
Met Ser Thr Thr Arg Phe Ile Lys Cys Val Thr Val Gly Asp Gly Ala
1               5                   10                  15

Val Gly Lys Thr Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Ser Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Ala Phe Ser Leu Ile Ser Lys Ala Ser Tyr Glu Asn Ile
                85                  90                  95

Ser Lys Lys Trp Ile Pro Glu Leu Arg His Tyr Ala Pro Thr Val Pro
            100                 105                 110

Ile Val Leu Val Gly Thr Lys Leu Asp Leu Arg Glu Asp Arg Gln Tyr
        115                 120                 125

Leu Ile Asp His Pro Gly Thr Thr Ala Ile Ala Thr Ala Gln Gly Glu
    130                 135                 140

Glu Leu Lys Lys Ala Ile Gly Ala Ala Val Tyr Ile Glu Cys Ser Ser
145                 150                 155                 160

Lys Thr Gln Gln Asn Val Lys Ala Val Phe Asp Ala Ala Ile Lys Val
                165                 170                 175

Val Leu Gln Pro Pro Lys Ser Lys Lys Lys Gly Lys Lys Lys Asn Thr
            180                 185                 190

Pro Cys Val Phe Leu
        195


<210> 520

<211> 196
<212> PRT
<213> Glycine max

<400> 520
Met Ser Ala Ser Arg Phe Ile Lys Cys Val Thr Val Gly Asp Gly Ala
1               5                   10                  15

Val Gly Lys Thr Cys Leu Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Ser Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Ile Leu Ala Phe Ser Leu Ile Ser Lys Ala Ser Tyr Glu Asn Ile
                85                  90                  95

Ala Lys Lys Trp Ile Pro Glu Leu Arg His Tyr Ala Pro Gly Val Pro
            100                 105                 110

Ile Ile Leu Val Gly Thr Lys Leu Asp Leu Arg Asp Asp Lys Gln Phe
        115                 120                 125

Phe Met Asp His Pro Gly Ala Val Pro Ile Thr Thr Ala Gln Gly Glu
    130                 135                 140

Glu Leu Arg Lys Leu Ile Gly Ala Pro Ala Tyr Ile Glu Cys Ser Ser
145                 150                 155                 160

Lys Thr Gln Gln Asn Val Lys Ala Val Phe Asp Ala Ala Ile Lys Val
                165                 170                 175

Val Ile Gln Pro Pro Lys Leu Lys Lys Lys Arg Lys Thr Gln Lys Ala
            180                 185                 190

Cys Ser Ile Leu
        195


<210> 521
<211> 212
<212> PRT
<213> Glycine max

<400> 521
Met Ala Ser Ala Thr Ala Pro Arg Phe Ile Lys Cys Val Thr Val Gly
1               5                   10                  15

Asp Gly Ala Val Gly Lys Thr Cys Met Leu Ile Cys Tyr Thr Ser Asn
            20                  25                  30

Lys Phe Pro Thr Asp Tyr Ile Pro Thr Val Phe Asp Asn Phe Ser Ala
        35                  40                  45

Asn Val Val Val Glu Gly Ile Thr Val Asn Leu Gly Leu Trp Asp Thr
    50                  55                  60

Ala Gly Gln Glu Asp Tyr Asn Arg Leu Arg Pro Leu Ser Tyr Arg Gly
65                  70                  75                  80

Ala Asp Val Phe Val Leu Ala Phe Ser Leu Val Ser Lys Ala Ser Tyr
                85                  90                  95

Glu Asn Val Leu Lys Lys Trp Ile Pro Glu Leu Gln His Phe Ala Pro
            100                 105                 110

Gly Ile Pro Leu Val Leu Val Gly Thr Lys Leu Asp Leu Arg Glu Asp
        115                 120                 125

Lys His Tyr Met Ala Asp His Pro Ser Leu Val Pro Val Thr Thr Asp
    130                 135                 140

Gln Gly Glu Glu Leu Arg Lys His Ile Gly Ala Thr Tyr Tyr Ile Glu
145                 150                 155                 160

Cys Ser Ser Lys Thr Gln Gln Asn Val Lys Ala Val Phe Asp Ala Ala
                165                 170                 175

Ile Arg Met Val Ile Lys Pro Pro Gln Lys Gln Asn Glu Lys Arg Lys
            180                 185                 190

Lys Lys Pro Arg Gly Cys Phe Leu Asn Val Leu Cys Arg Arg Asn Ile
        195                 200                 205

Val Arg Leu Lys
    210


<210> 522
<211> 197
<212> PRT
<213> Glycine max

<400> 522
Met Ser Ala Ser Arg Phe Ile Lys Cys Val Thr Val Gly Asp Gly Ala
1               5                   10                  15

Val Gly Lys Thr Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Ser Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Ala Phe Ser Leu Ile Ser Arg Ala Ser Tyr Glu Asn Val
                85                  90                  95

Ala Lys Lys Trp Ile Pro Glu Leu Arg His Tyr Ala Pro Gly Val Pro
            100                 105                 110

Ile Ile Leu Val Gly Thr Lys Leu Asp Leu Arg Asp Asp Lys Gln Phe
        115                 120                 125

Phe Gln Asp His Pro Gly Ala Val Pro Ile Thr Thr Ala Gln Gly Glu
    130                 135                 140

Glu Leu Arg Lys Leu Ile Gly Ala Pro Ile Tyr Ile Glu Cys Ser Ser
145                 150                 155                 160

Lys Thr Gln Gln Asn Val Lys Ala Val Phe Asp Ala Ala Ile Lys Val
                165                 170                 175

Val Leu Gln Pro Pro Lys Gln Lys Lys Lys Lys Arg Lys Gly Gln Lys
            180                 185                 190

Ala Cys Ser Ile Leu
        195


<210> 523
<211> 197
<212> PRT
<213> Glycine max

<400> 523
Met Ser Ala Ser Arg Phe Ile Lys Cys Val Thr Val Gly Asp Gly Ala
  1               5                  10                  15

Val Gly Lys Thr Cys Leu Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asn Gly Ser Ile Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Ile Leu Ala Phe Ser Leu Ile Ser Lys Ala Ser Tyr Glu Asn Val
                85                  90                  95

Ser Lys Lys Trp Ile Pro Glu Leu Lys His Tyr Ala Pro Gly Val Pro
            100                 105                 110

Ile Ile Leu Val Gly Thr Lys Leu Asp Leu Arg Asp Asp Lys Gln Phe

115                 120                 125

Cys Ile Asp His Pro Gly Ala Val Pro Ile Thr Thr Ala Gln Gly Glu
    130                 135                 140

Glu Leu Arg Lys Leu Ile Asn Ala Pro Ala Tyr Ile Glu Cys Ser Ser
145                 150                 155                 160

Lys Thr Gln Glu Asn Val Lys Ala Val Phe Asp Ala Ala Ile Arg Val
                165                 170                 175

Val Leu Gln Pro Pro Lys Gln Lys Lys Lys Lys Gly Lys Ala Gln Lys
            180                 185                 190

Ala Cys Ser Ile Leu
        195

<210> 524
<211> 197
<212> PRT
<213> Glycine max

<400> 524
Met Ser Ala Ser Arg Phe Ile Lys Cys Val Thr Val Gly Asp Gly Ala
1               5                   10                  15

Val Gly Lys Thr Cys Met Leu Ile Ser Tyr Thr Ser Asn Thr Phe Pro
            20                  25                  30

Thr Asp Tyr Val Pro Thr Val Phe Asp Asn Phe Ser Ala Asn Val Val
        35                  40                  45

Val Asp Gly Ser Thr Val Asn Leu Gly Leu Trp Asp Thr Ala Gly Gln
    50                  55                  60

Glu Asp Tyr Asn Arg Leu Arg Pro Leu Ser Tyr Arg Gly Ala Asp Val
65                  70                  75                  80

Phe Leu Leu Ala Phe Ser Leu Ile Ser Arg Ala Ser Tyr Glu Asn Val
                85                  90                  95

Ala Lys Lys Trp Ile Pro Glu Leu Arg His Tyr Ala Pro Gly Val Pro
            100                 105                 110

Ile Ile Leu Val Gly Thr Lys Leu Asp Leu Arg Asp Asp Lys Gln Phe
        115                 120                 125

Phe Gln Asp His Pro Gly Ala Val Pro Ile Thr Thr Ala Gln Gly Glu
    130                 135                 140

Glu Leu Arg Lys Leu Ile Gly Ala Pro Ile Tyr Ile Glu Cys Ser Ser
145                 150                 155                 160

Lys Thr Gln Gln Asn Val Lys Ala Val Phe Asp Ala Ala Ile Lys Val
                165                 170                 175

Val Leu Gln Pro Pro Lys Gln Lys Lys Lys Lys Arg Lys Gly Gln Lys
            180                 185                 190

Ala Cys Ser Ile Leu
        195


<210> 525
<211> 214
<212> PRT
<213> Oryza sativa

<400> 525
Met Ser Gly Ala Thr Lys Phe Ile Lys Cys Val Thr Val Gly Asp Gly
1               5                   10                  15

Ala Val Gly Lys Thr Cys Met Leu Ile Cys Tyr Ile Ser Asn Lys Phe
            20                  25                  30

Pro Thr Asp Tyr Ile Pro Thr Val Phe Asp Asn Phe Ser Ala Asn Val
        35                  40                  45

Ser Val Asp Gly Asn Ile Val Asn Leu Gly Leu Trp Asp Thr Ala Gly
    50                  55                  60

Gln Glu Asp Tyr Ser Arg Leu Arg Pro Leu Ser Tyr Arg Gly Ala Asp
65                  70                  75                  80

Ile Phe Val Leu Ala Phe Ser Leu Ile Ser Arg Ala Ser Tyr Glu Asn
                85                  90                  95

Val Leu Lys Lys Trp Met Pro Glu Leu Arg Arg Phe Ala Pro Asn Val
            100                 105                 110

Pro Ile Val Leu Val Gly Thr Lys Leu Asp Leu Arg Asp His Arg Ser
        115                 120                 125

Tyr Leu Ala Asp His Pro Ala Ala Ser Ala Ile Thr Thr Ala Gln Gly
    130                 135                 140

Glu Glu Leu Arg Lys Gln Ile Gly Ala Ala Ala Tyr Ile Glu Cys Ser
145                 150                 155                 160

Ser Lys Thr Gln Gln Asn Ile Lys Ala Val Phe Asp Thr Ala Ile Lys
                165                 170                 175

Val Val Leu Gln Pro Pro Arg Arg Arg Gly Glu Thr Thr Met Ala Arg
            180                 185                 190

Lys Lys Thr Arg Arg Ser Thr Gly Cys Ser Leu Lys Asn Leu Met Cys
        195                 200                 205

Gly Ser Ala Cys Val Val
    210

<210> 526
<211> 199
<212> PRT
<213> Oryza sativa

<400> 526
Met Gly Cys Ser Ser Ser Val Pro Ala Arg Ser Thr Gly Gly Leu Asn
1               5                   10                  15

Asn Ile Ser Asn Asp Asn Ser Ala Thr Asp Ser Lys Asp Leu Arg Ala
            20                  25                  30

Lys Leu Val Leu Leu Gly Asp Ser Gly Val Gly Lys Ser Cys Ile Val
        35                  40                  45

Leu Arg Phe Val Arg Gly Gln Phe Asp Pro Thr Ser Lys Val Thr Val
    50                  55                  60

Gly Ala Ser Phe Leu Ser Gln Thr Leu Ala Leu Glu Asp Ser Thr Ile
65                  70                  75                  80

Val Lys Phe Glu Ile Trp Asp Thr Ala Gly Gln Glu Arg Tyr Ala Ala
                85                  90                  95

Leu Ala Pro Leu Tyr Tyr Arg Gly Ala Ala Ala Ala Val Val Val Tyr
            100                 105                 110

Asp Ile Thr Ser Pro Glu Ser Phe Ser Lys Ala Gln Tyr Trp Val Lys
        115                 120                 125

Glu Leu Gln Lys His Gly Ser Pro Asp Ile Ile Met Val Leu Val Gly
    130                 135                 140

Asn Lys Ala Asp Leu His Glu Asn Arg His Val Ser Ser Gln Glu Ala
145                 150                 155                 160

Gln Glu Tyr Ala Glu Lys Asn Asn Met Val Phe Ile Glu Thr Ser Ala
                165                 170                 175

Lys Thr Ala Asp Asn Ile Asn Gln Val Phe Glu Glu Ile Ala Lys Arg
            180                 185                 190

Leu Pro Arg Pro Thr Ala Ser
        195


<210> 527
<211> 215
<212> PRT
<213> Oryza sativa

<400> 527
Met Ala Ser Ser Ala Ser Arg Phe Ile Lys Cys Val Thr Val Gly Asp
1               5                   10                  15

Gly Ala Val Gly Lys Thr Cys Met Leu Ile Cys Tyr Thr Ser Asn Lys
            20                  25                  30

Phe Pro Thr Asp Tyr Val Pro Thr Val Phe Asp Asn Phe Ser Ala Asn
        35                  40                  45

Val Val Val Asp Gly Thr Thr Val Asn Leu Gly Leu Trp Asp Thr Ala
    50                  55                  60

Gly Gln Glu Asp Tyr Asn Arg Leu Arg Pro Leu Ser Tyr Arg Gly Ala
65                  70                  75                  80

Asp Val Phe Val Leu Ala Phe Ser Leu Val Ser Arg Ala Ser Tyr Glu
                85                  90                  95

Asn Val Met Lys Lys Trp Leu Pro Glu Leu Gln His Tyr Ala Pro Gly
            100                 105                 110

Val Pro Ile Val Leu Val Gly Thr Lys Leu Asp Leu Arg Glu Asp Lys
        115                 120                 125

His Tyr Leu Leu Asp His Pro Ser Leu Val Pro Val Thr Thr Ala Gln
    130                 135                 140

Gly Glu Glu Leu Arg Lys His Ile Gly Ala Thr Cys Tyr Ile Glu Cys
145                 150                 155                 160

Ser Ser Lys Thr Gln Gln Asn Val Lys Ala Val Phe Asp Ala Ala Ile
                165                 170                 175

Lys Val Val Ile Lys Pro Pro Thr Lys Gln Arg Asp Arg Lys Lys Lys
            180                 185                 190

Lys Thr Arg Arg Gly Cys Ser Phe Phe Cys Lys Gly Val Met Ser Arg
        195                 200                 205

Arg Arg Leu Val Cys Phe Lys
    210                 215


<210> 528
<211> 212
<212> PRT
<213> Oryza sativa

<400> 528
Met Gly Ser Lys Pro Pro Pro Pro Pro Gln Pro Ser Val Ser Phe Lys
1               5                   10                  15

Leu Val Leu Leu Gly Asp Gly Arg Val Gly Lys Thr Ser Leu Val Leu
            20                  25                  30

Arg Tyr Val Asn Asp Val Phe Ser Asp Lys Gln Glu Ala Thr Val Gln
        35                  40                  45

Ala Ser Tyr Leu Thr Lys Arg Leu Val Val Glu Gly Val Pro Ile Thr

50                  55                  60

Leu Ser Ile Trp Asp Thr Ala Gly Gln Glu Lys Phe His Ala Leu Gly
65                  70                  75                  80

Pro Ile Tyr Tyr Arg Asp Ala Asp Ala Ala Leu Leu Val Tyr Asp Ile
                85                  90                  95

Thr Asp Asn Asp Thr Phe Leu Arg Val Thr Lys Trp Val Lys Glu Leu
            100                 105                 110

Lys Gln Met Ala Asn Lys Asp Ile Val Met Ala Ile Ala Ala Asn Lys
        115                 120                 125

Ser Asp Leu Val Arg Ser Lys His Ile Asp Thr Asn Glu Ala Ala Ser
    130                 135                 140

Tyr Ala Glu Ser Ile Gly Ala Thr Leu Phe Val Thr Ser Ala Lys Ala
145                 150                 155                 160

Gly Thr Gly Ile Asp Asp Ile Phe Ser Asp Ile Ala Lys Arg Leu Leu
                165                 170                 175

Glu Arg Arg Lys Asn Ser Ser Asp Gly Leu Ser Leu Ala His Pro Lys
            180                 185                 190

Lys Gly Ile Leu Ile Val Asp Asp Glu Pro Glu Lys Glu Pro Pro Pro
        195                 200                 205

Lys Cys Cys Ser
    210


<210> 529
<211> 214
<212> PRT
<213> Oryza sativa

<400> 529
Met Ser Ser Ala Ala Ala Ala Thr Arg Phe Ile Lys Cys Val Thr Val
1               5                   10                  15

Gly Asp Gly Ala Val Gly Lys Thr Cys Met Leu Ile Cys Tyr Thr Cys
            20                  25                  30

Asn Lys Phe Pro Thr Asp Tyr Ile Pro Thr Val Phe Asp Asn Phe Ser
        35                  40                  45

Ala Asn Val Ser Val Asp Gly Ser Val Val Asn Leu Gly Leu Trp Asp
    50                  55                  60

Thr Ala Gly Gln Glu Asp Tyr Ser Arg Leu Arg Pro Leu Ser Tyr Arg
65                  70                  75                  80

Gly Ala Asp Val Phe Ile Leu Ser Phe Ser Leu Ile Ser Arg Ala Ser
                85                  90                  95

Tyr Glu Asn Val Gln Lys Lys Trp Met Pro Glu Leu Arg Arg Phe Ala
            100                 105                 110

Pro Gly Val Pro Val Val Leu Val Gly Thr Lys Leu Asp Leu Arg Glu
        115                 120                 125

Asp Arg Ala Tyr Leu Ala Asp His Pro Ala Ser Ser Ile Ile Thr Thr
    130                 135                 140

Glu Gln Gly Glu Glu Leu Arg Lys Leu Ile Gly Ala Val Ala Tyr Ile
145                 150                 155                 160

Glu Cys Ser Ser Lys Thr Gln Arg Asn Ile Lys Ala Val Phe Asp Thr
                165                 170                 175

Ala Ile Lys Val Val Leu Gln Pro Pro Arg His Lys Asp Val Thr Arg
            180                 185                 190

Lys Lys Leu Gln Ser Ser Ser Asn Arg Pro Val Arg Arg Tyr Phe Cys
        195                 200                 205

Gly Ser Ala Cys Phe Ala
    210

<210> 530
<211> 192
<212> PRT
<213> Saccharomyces cerevisiae

<400> 530
Met Ser Glu Lys Ala Val Arg Arg Lys Leu Val Ile Ile Gly Asp Gly
1               5                   10                  15

Ala Cys Gly Lys Thr Ser Leu Leu Tyr Val Phe Thr Leu Gly Lys Phe
            20                  25                  30

Pro Glu Gln Tyr His Pro Thr Val Phe Glu Asn Tyr Val Thr Asp Cys
        35                  40                  45

Arg Val Asp Gly Ile Lys Val Ser Leu Thr Leu Trp Asp Thr Ala Gly
    50                  55                  60

Gln Glu Glu Tyr Glu Arg Leu Arg Pro Phe Ser Tyr Ser Lys Ala Asp
65                  70                  75                  80

Ile Ile Leu Ile Gly Phe Ala Val Asp Asn Phe Glu Ser Leu Ile Asn
                85                  90                  95

Ala Arg Thr Lys Trp Ala Asp Glu Ala Leu Arg Tyr Cys Pro Asp Ala
            100                 105                 110

Pro Ile Val Leu Val Gly Leu Lys Lys Asp Leu Arg Gln Glu Ala His
        115                 120                 125

Phe Lys Glu Asn Ala Thr Asp Glu Met Val Pro Ile Glu Asp Ala Lys
    130                 135                 140

Gln Val Ala Arg Ala Ile Gly Ala Lys Lys Tyr Met Glu Cys Ser Ala
145                 150                 155                 160

Leu Thr Gly Glu Gly Val Asp Asp Val Phe Glu Val Ala Thr Arg Thr
                165                 170                 175

Ser Leu Leu Met Lys Lys Glu Pro Gly Ala Asn Cys Cys Ile Ile Leu
            180                 185                 190

<210> 531
<211> 4
<212> PRT
<213> Artificial Sequence

<220>
<223> Description of Artificial Sequence: Synthetic
      peptide

<400> 531
Lys Asp Glu Leu
  1

<210> 532
<211> 6
<212> PRT
<213> Artificial Sequence

<220>
<223> Description of Artificial Sequence: Synthetic
      peptide

<400> 532
Ser Glu Lys Asp Glu Leu
  1               5

<210> 533
<211> 38
<212> DNA
<213> Saccharomyces cerevisiae

<400> 533
ggaattccag ctgaccacca tggcaattcc cggggatc                          38

<210> 534
<211> 38
<212> DNA

<213> Escherichia coli

<400> 534
ttgctcttcc atggcaatga ttaattaacg aagagcaa                                38